# EUROPEAN PATENT APPLICATION

(11) **EP 3 949 731 A1**
(43) Date of publication of application: **09.02.2022**
(21) Application number: 20782936.7
(22) Date of filing: 01.04.2020
(51) Int. Cl.: A01M 21/04, C07D 405/04, C07D 405/14, C07D 409/04, C07D 409/14, A01P 13/00, A01N 47/02, A01N 47/06, A01N 43/58

(54) **PYRIDAZINONE COMPOUND AND HERBICIDE**

(30) Priority: 01.04.2019 JP 2019070172; 09.03.2020 JP 2020039961
(71) Applicant: Nissan Chemical Corporation, Tokyo 103-6119 (JP)
(72) Inventor: TANAKA, Yuuki, Funabashi-shi, Chiba 274-8507 (JP); IWASA, Motoyoshi, Funabashi-shi, Chiba 274-8507 (JP); INABA, Masamitsu, Funabashi-shi, Chiba 274-8507 (JP); TANIMA, Daisuke, Funabashi-shi, Chiba 274-8507 (JP); HADIAN, Permana, Shiraoka-shi, Saitama 349-0294 (JP); USUI, Yuto, Shiraoka-shi, Saitama 349-0294 (JP); FURUHASHI, Takamasa, Shiraoka-shi, Saitama 349-0294 (JP)
(74) Representative: Hoffmann Eitle
(86) International application number: PCT/JP2020/015122
(87) International publication number: WO 2020/204112

(57) **Abstract**

There is provided an agricultural chemical (in particular, a herbicide).

A pyridazinone compound of the following Formula (1): 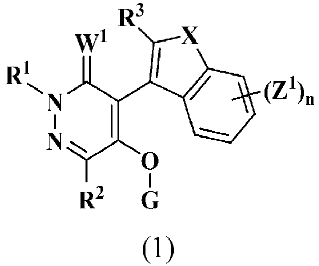 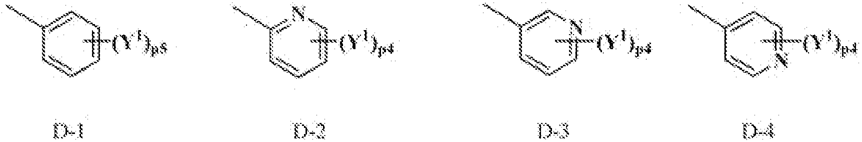 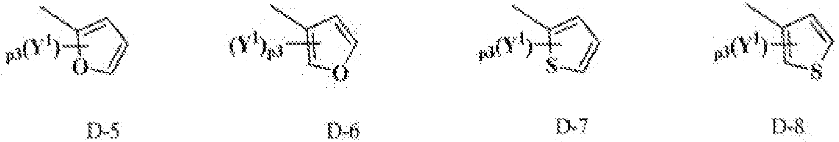 [wherein W¹ and X are each independently an oxygen atom or a sulfur atom; R¹ and R² are each independently such as C₁₋₆ alkyl; R³ is such as D-1 to D-8; Y¹ is such as a halogen atom; G is such as a hydrogen atom; Z¹ is such as C₁₋₆ alkyl; n is an integer of 0, 1, 2, 3, or 4; p5 is an integer of 0, 1, 2, 3, 4, or 5; p4 is an integer of 0, 1, 2, 3, or 4; and p3 is an integer of 0, 1, 2, or 3] and a herbicide comprising the same.

## Description

### TECHNICAL FIELD

The present invention relates to a novel pyridazinone compound or a salt thereof, and an agricultural chemical (in particular, a herbicide) containing the compound as an active ingredient.

### BACKGROUND ART

For example, Patent Documents 1 and 2 disclose a certain type of pyridazinone compound. However, the pyridazinone compound according to the present invention has not been disclosed at all.

### Prior Art Documents

### Patent Documents

Patent Document 1: International Publication WO 2017/074992
Patent Document 2: International Publication WO 2015/168010

### SUMMARY OF THE INVENTION

### Problems to be Solved by the Invention

An object of the present invention is to provide a chemical substance which reliably exhibits effects on various weeds even when applied in a small amount, which is less likely to cause problems (e.g., land pollution and adverse effects on succeeding crops), and which exhibits high safety and is useful as an active ingredient of a herbicide.

### Means for Solving the Problem

The present inventors have conducted extensive studies for solving the aforementioned problems, and as a result have found that a novel pyridazinone compound of Formula (1) described below is a very useful compound having excellent herbicidal activity as a herbicide and high safety against target crops, and exhibiting almost no adverse effects on non-target living organisms (e.g., such as mammals, fishes, beneficial insects, and natural enemies). The present invention has been accomplished on the basis of this finding.

Accordingly, the present invention is directed to the following [1] to [152].
[1] A pyridazinone compound of the following Formula (1) or a salt thereof: 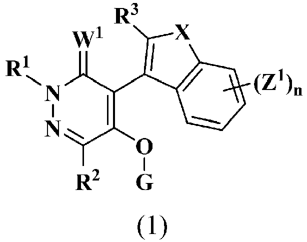 [wherein W¹ is an oxygen atom or a sulfur atom;
   X is an oxygen atom or a sulfur atom;
   Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, (C₃₋₆) cycloalkyl substituted with R⁴⁴, phenyl, phenyl substituted with (Z⁴)_{p5c}, Q-2, Q-3, Q-4, Q-5, Q-6, Q-7, Q-10, Q-11, Q-12, Q-13, -NR¹⁵R¹⁶, -OR³⁵, -S(O)ᵣ₁R³⁶, -CN, -NO₂, -C(O)OH, -C(=W³)R²⁰, or -N=C(C₆H₅)₂, and when n is an integer of 2 or more, each Z¹ is the same as or different from each other;
   G is a hydrogen atom, C₁₋₆ alkyl, (C₁₋₆) alkyl substituted with R⁴, -C(=W⁴)R⁵, or - S(O)₂R⁶;
   R¹ is a hydrogen atom, C₁₋₆ alkyl, C₃₋₆ alkenyl, C₃₋₆ alkynyl, or C₁₋₆ alkyl substituted with R³⁴;
   R² is a hydrogen atom, a halogen atom, C₁₋₆ alkyl, C₁₋₆ alkoxy, C₃₋₆ cycloalkyl, or - NR³⁰R³¹;
   R³ is D-1, D-2, D-3, D-4, D-5, D-6, D-7, D-8, D-9, D-10, D-11, D-12, D-13, D-14, D-15, D-16, D-17, D-18, D-19, D-20, D-21, D-22, D-23, D-24, D-25, D-26, D-27, D-28, D-29, D-30, D-31, D-32, D-33, D-34, D-35, D-36, D-37, D-38, D-39, D-40, D-41, D-42, D-43, D-44, D-45, D-46, D-47, D-48, D-49, D-50, D-51, D-52, D-53, D-54, D-55, or D-56;
   D-1, D-2, D-3, D-4, D-5, D-6, D-7, D-8, D-9, D-10, D-11, D-12, D-13, D-14, D-15, D-16, D-17, D-18, D-19, D-20, D-21, D-22, D-23, D-24, D-25, D-26, D-27, D-28, D-29, D-30, D-31, D-32, D-33, D-34, D-35, D-36, D-37, D-38, D-39, D-40, D-41, D-42, D-43, D-44, D-45, D-46, D-47, D-48, D-49, D-50, D-51, D-52, D-53, D-54, D-55, and D-56 are respectively the following structures: 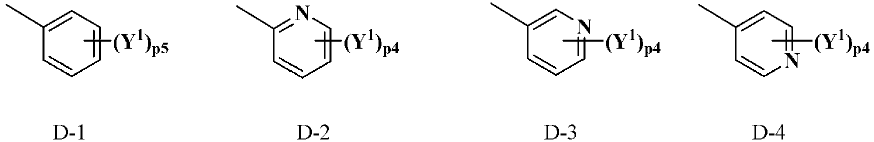 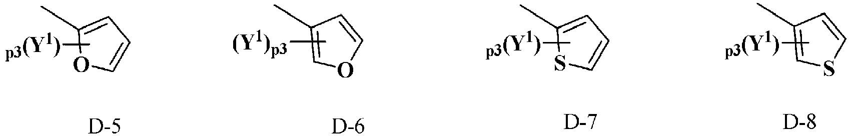 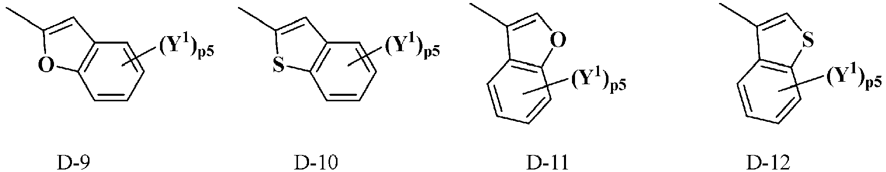 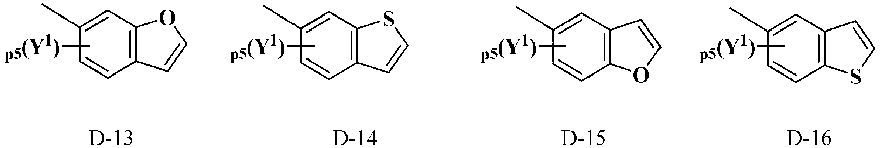 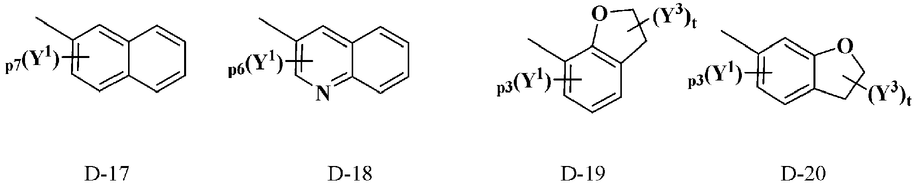 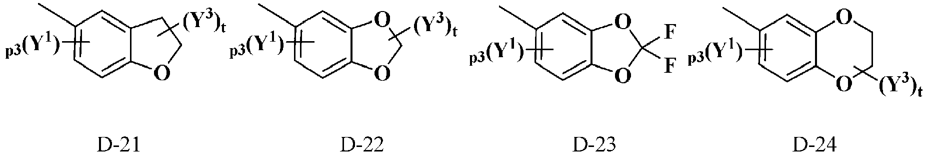 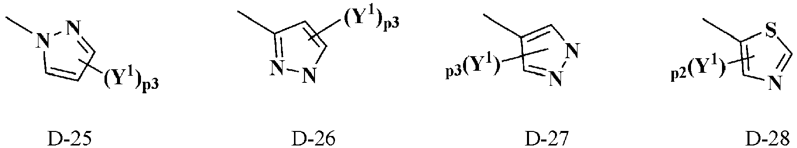 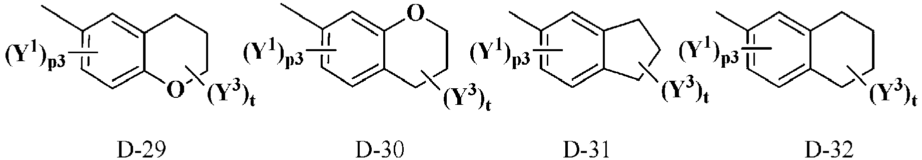 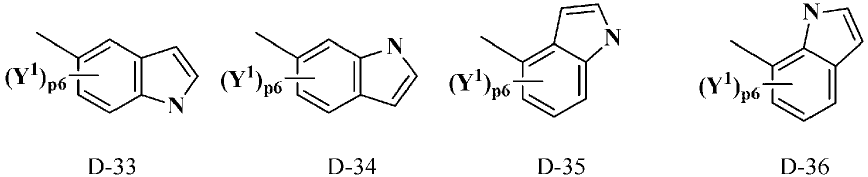 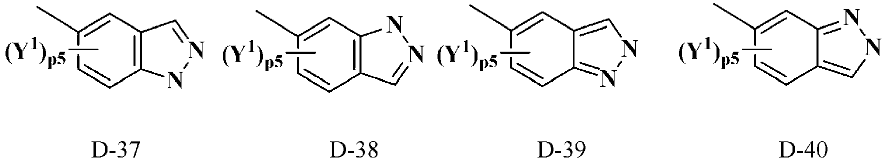 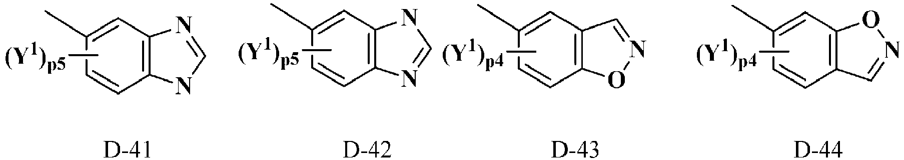 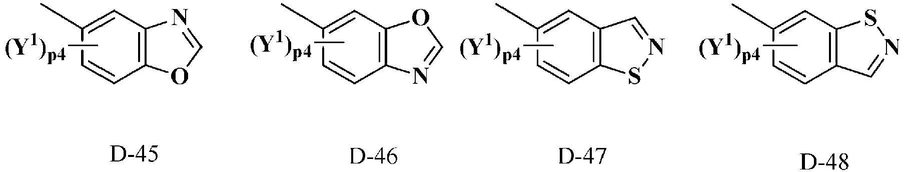 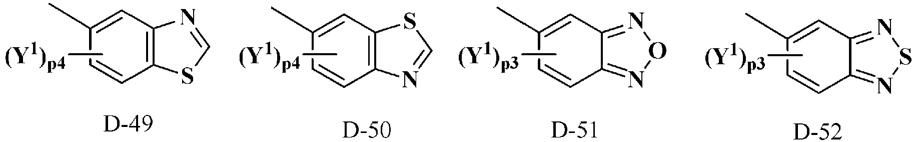 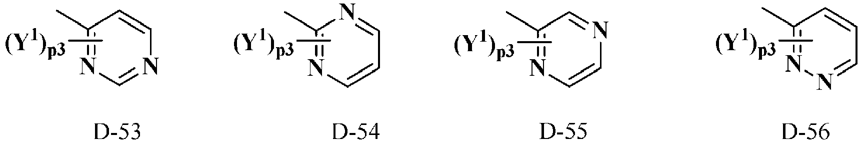
   Y¹ is substituted on the aromatic ring of each of D-1 to D-56, and Y³ is substituted on the aliphatic ring of D-19, D-20, D-21, D-22, D-24, D-29, D-30, D-31, or D-32;
   Y¹ is a hydrogen atom, a halogen atom, C₁₋₆ alkyl, C₂₋₆ alkenyl, C₂₋₆ alkynyl, C₃₋₆ cycloalkyl, C₂₋₆ haloalkenyl, C₂₋₆ haloalkynyl, (C₁₋₆) alkyl substituted with R¹⁰, C₃₋₆ cycloalkyl substituted with R⁴⁴, -OR¹¹, -S(O)ᵣ₂R³⁸, -NR⁸R⁹, -CN, -NO₂, -C(O)OH, - C(=W²)R¹³, phenyl, phenyl substituted with (Z⁴)_{p5c}, tri(C₁₋₆ alkyl)silyl, Q-6, Q-7, Q-10, Q-11, Q-12, or Q-13, and when p7, p6, p5, p4, p3, or p2 is an integer of 2 or more, each Y¹ is the same as or different from each other;
   R⁴ is a halogen atom, -CN, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁴, -OR²⁴, -S(O)ᵣ₄R⁵⁴, - C(O)R²³, phenyl, phenyl substituted with (Z⁴)_{p5c}, U-1, U-2, U-3, U-4, U-5, U-9, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, or Q-36;
   R⁵ is C₁₋₆ alkyl, (C₁₋₆) alkyl substituted with R²⁶, C₂₋₆ alkenyl, C₂₋₆ haloalkenyl, C₂₋₆ alkynyl, C₂₋₆ haloalkynyl, C₁₋₆ alkoxy, -OR⁵⁸, -SR⁵⁹, C₃₋₆ cycloalkyl, (C₃₋₆) cycloalkyl substituted with R⁴⁴, -NR⁵⁶R⁵⁷, U-1, U-2, U-3, U-4, U-5, U-6, U-7, U-8, U-9, U-10, U-11, U-12, U-13, U-14, U-15, U-16, U-17, U-18, U-19, U-20, U-21, U-22, U-23, U-24, U-25, U-26, U-27, U-28, U-29U-30, U-31, U-32, U-33, U-34, U-35, Q-1, Q-2, Q-3, Q-4, Q-5, Q-7, Q-8, Q-9, Q-10, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, Q-37, Q-38, Q-39, Q-40, phenyl, phenyl substituted with (Z²)ₚ₅ₐ, or -C(O)R⁵³;
   R⁶ is C₁₋₆ alkyl, C₁₋₆ haloalkyl, phenyl, phenyl substituted with (Z²)ₚ₅ₐ, U-6, U-7, U-8, Q-10, or -NR²⁸R²⁹;
   R⁷ is a hydrogen atom, C₁₋₆ alkyl, C₃₋₆ alkenyl, C₃₋₆ alkynyl, C₁₋₆ haloalkyl, C₁₋₆ alkoxy (C₁₋₂) alkyl, C₁₋₆ alkylthio (C₁₋₂) alkyl, C₃₋₆ cycloalkyl (C₁₋₂) alkyl, benzyl, or benzyl substituted with (Z⁴)_{p5c};
   R⁸ and R⁹ are each independently a hydrogen atom, or C₁₋₆ alkyl;
   R¹⁰ is a halogen atom, -OR⁴⁰, -S(O)ᵣ₃R⁴¹, -CN, C₃₋₆ cycloalkyl, (C₃₋₆) cycloalkyl substituted with R⁴⁴, Q-6, Q-7, Q-10, Q-11, Q-12, or Q-13;
   R¹¹ is a hydrogen atom, C₁₋₆ alkyl, (C₁₋₆) alkyl substituted with R¹², C₃₋₆ cycloalkyl, (C₃₋₆) cycloalkyl substituted with R⁴⁴, phenyl, phenyl substituted with (Z⁴)_{p5c}, tri(C₁₋₄ alkyl)silyl, C₁₋₆ alkylcarbonyl, C₁₋₆ alkoxycarbonyl, C₁₋₆ alkylsulfonyl, C₁₋₆ haloalkylsulfonyl, - C(=W²)R¹³, -S(O)ᵣ₅R⁴⁹, U-1, U-2, U-3, U-4, U-5, U-12, U-14, U-15, U-16, Q-17, Q-18, Q-19, or Q-20;
   R¹² is a halogen atom, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁵, -OR⁴⁸, -S(O)ᵣ₅R⁴⁹, -CN, phenyl, phenyl substituted with (Z³)_{p5b}, -C(=W²)R¹³, U-1, U-2, U-3, U-4, U-5, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, or - ON=CR⁴²R⁴³;
   Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, Q-37, Q-38, Q-39, and Q-40 are respectively the following structures: 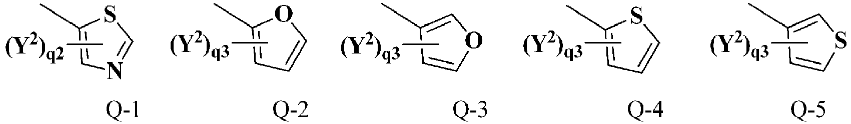 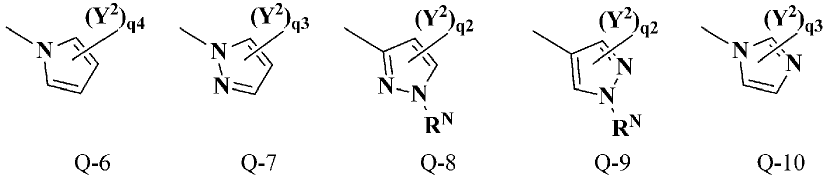 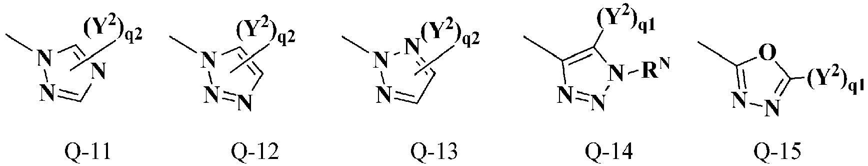 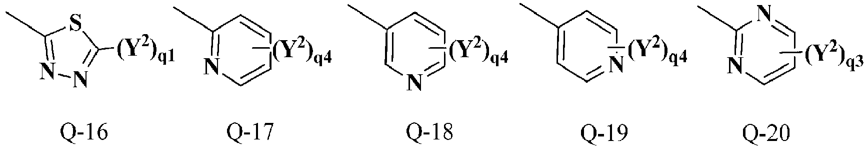 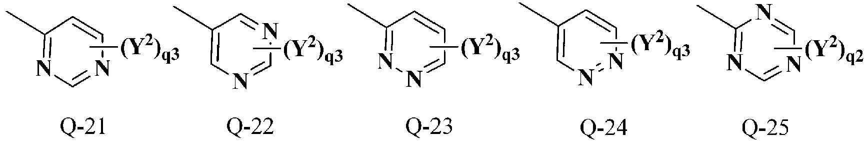 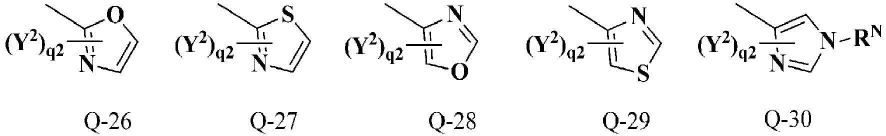 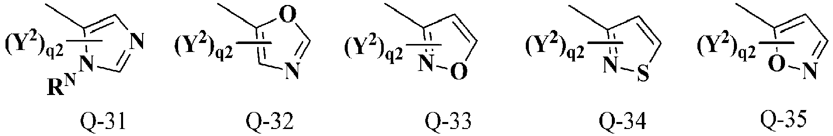 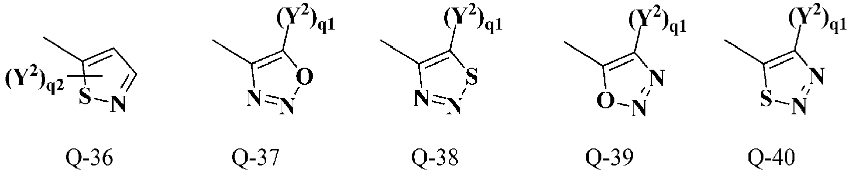
   Y² is substituted on the aromatic ring of each of Q-1 to Q-40;
   U-1, U-2, U-3, U-4, U-5, U-6, U-7, U-8, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, and U-35 are respectively the following structures: 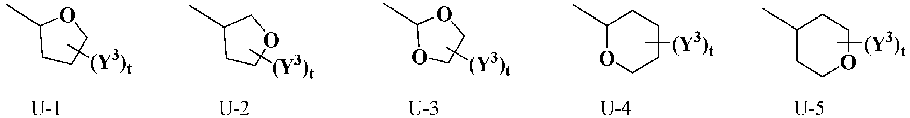 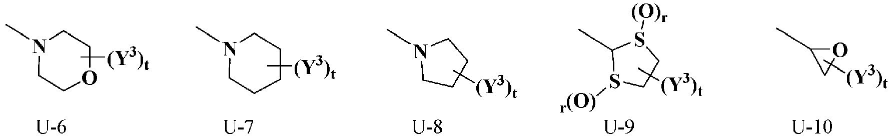 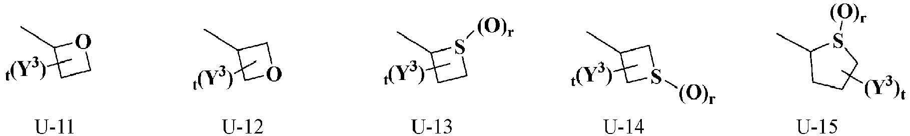 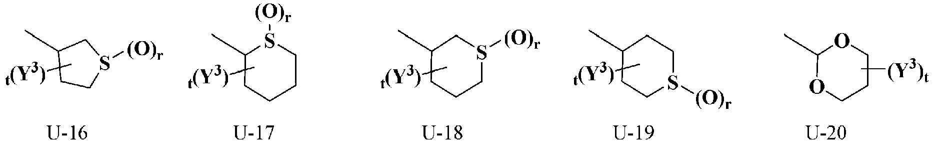 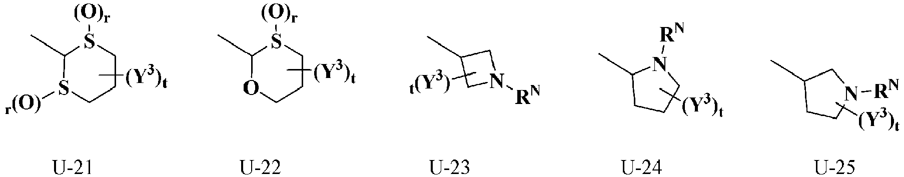 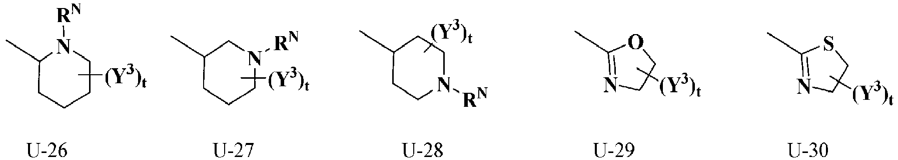 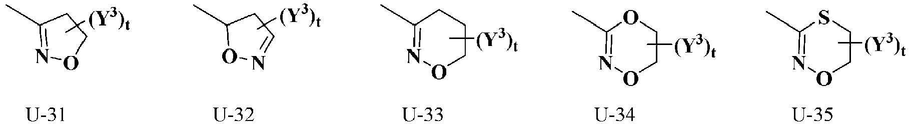
   R¹³ is a hydrogen atom, C₁₋₆ alkyl, C₁₋₆ alkoxy, C₁₋₆ alkylamino, di(C₁₋₆) alkylamino, C₁₋₆ haloalkylamino, C₁₋₆ alkylthio, C₁₋₆ haloalkylthio, or -NH₂;
   R¹⁴ is a halogen atom, C₂₋₆ alkenyl, C₂₋₆ alkynyl, C₃₋₆ cycloalkyl, C₂₋₆ haloalkenyl, C₂₋₆ haloalkynyl, (C₃₋₆) cycloalkyl substituted with R⁴⁵, C₁₋₆ alkoxy, C₁₋₆ alkylthio, C₁₋₆ alkylsulfinyl, C₁₋₆ alkylsulfonyl, C₁₋₆ haloalkoxy, C₁₋₆ haloalkylthio, C₁₋₆ haloalkylsulfinyl, C₁₋₆ haloalkylsulfonyl, phenyl, phenyl substituted with (Z⁴)_{p5c}, -CN, U-1, U-2, U-3, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, or Q-36;
   R¹⁵ and R¹⁶ are each independently a hydrogen atom, C₁₋₆ alkyl, -C(O)R¹⁷, or - S(O)₂R¹⁸;
   R¹⁷ is C₁₋₆ alkyl, C₁₋₆ alkoxy, or C₁₋₆ alkoxy (C₁₋₂) alkyl;
   R¹⁸ is C₁₋₆ alkyl or C₁₋₆ haloalkyl;
   R¹⁹ is C₃₋₆ cycloalkyl or tri(C₁₋₆ alkyl)silyl;
   R²⁰ is a hydrogen atom, C₁₋₆ alkyl, or C₁₋₆ alkoxy;
   R²¹ is a hydrogen atom, C₁₋₆ alkyl, C₁₋₆ haloalkyl, C₁₋₆ alkoxy (C₁₋₂) alkyl, or C₁₋₆ alkylthio (C₁₋₂) alkyl;
   R²² is a halogen atom, -OH, C₁₋₆ alkoxy, C₁₋₆ haloalkoxy, C₁₋₆ alkylthio, C₁₋₆ alkylsulfinyl, C₁₋₆ alkylsulfonyl, C₁₋₆ haloalkylthio, -CN, or Q-7;
   R²³ is C₁₋₆ alkyl, C₁₋₆ alkoxy, di(C₁₋₆) alkylamino, phenyl, phenyl substituted with (Z⁴)_{p5c}, U-6, U-7, or U-8;
   R²⁴ is C₁₋₆ alkyl, C₁₋₆ haloalkyl, C₃₋₆ alkenyl, C₃₋₆ haloalkenyl, C₃₋₆ alkynyl, C₃₋₆ haloalkynyl, (C₁₋₆) alkoxy (C₁₋₂) alkyl, -C(O)R²⁵, -S(O)₂R³³, phenyl, or phenyl substituted with (Z⁴)_{p5c};
   R²⁵ is C₁₋₆ alkyl, C₁₋₆ alkoxy, phenyl, phenyl substituted with (Z⁴)_{p5c}, di(C₁₋₆) alkylamino, U-6, U-7, or U-8;
   R²⁶ is a halogen atom, C₁₋₆ alkoxy, C₁₋₆ haloalkoxy, C₁₋₆ alkylthio, C₁₋₆ alkylsulfinyl, C₁₋₆ alkylsulfonyl, C₃₋₆ cycloalkyl, (C₃₋₆) cycloalkyl substituted with R⁴⁴, -CN, -OR³², phenyl, phenyl substituted with (Z⁴)_{p5c}, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, U-1, U-2, U-3, U-4, U-5, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, or U-35;
   R²⁷ is a halogen atom, C₂₋₆ alkenyl, C₂₋₆ alkynyl, C₁₋₆ alkoxy, C₁₋₆ haloalkoxy, C₃₋₆ cycloalkyl, C₃₋₆ halocycloalkyl, C₁₋₆ alkylthio, C₁₋₆ haloalkylthio, -CN, phenyl, phenyl substituted with (Z³)_{p5b}, 9-fluorenyl, Q-2, Q-3, Q-4, Q-5, Q-17, U-1, U-2, U-3, U-4, U-5, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, or U-35;
   R²⁸ and R²⁹ are each independently a hydrogen atom or C₁₋₆ alkyl;
   R³⁰ and R³¹ are each independently a hydrogen atom, C₁₋₆ alkyl, or benzyl;
   R³² is phenyl, phenyl substituted with (Z⁴)_{p5c}, or Q-17;
   R³³ is C₁₋₆ alkyl, C₁₋₆ haloalkyl, or di(C₁₋₆ alkyl)amino;
   R³⁴ is a halogen atom, C₁₋₆ alkoxy, phenyl, or -CN;
   R³⁵ is a hydrogen atom, C₁₋₆ alkyl, C₃₋₆ cycloalkyl, (C₁₋₆) alkyl substituted with R¹⁴, (C₁₋₆) cycloalkyl substituted with R⁴⁴, or -S(O)₂R³³;
   R³⁶ is C₁₋₆ alkyl, (C₁₋₆) alkyl substituted with R³⁷, C₃₋₆ cycloalkyl, or (C₁₋₆) cycloalkyl substituted with R⁴⁴;
   R³⁷ is a halogen atom, C₂₋₆ alkenyl, C₂₋₆ haloalkenyl, C₂₋₆ alkynyl, C₂₋₆ haloalkynyl, C₃₋₆ cycloalkyl, C₃₋₆ halocycloalkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, phenyl, phenyl substituted with (Z⁴)_{p5c}, -CN, U-1, U-2, U-3, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, or C₁₋₁₀ alkoxycarbonyl;
   R³⁸ is C₁₋₆ alkyl, C₃₋₆ alkenyl, C₃₋₆ alkynyl, C₃₋₆ cycloalkyl, C₃₋₆ haloalkenyl, C₃₋₆ haloalkynyl, (C₁₋₆) alkyl substituted with R³⁹, (C₃₋₆) cycloalkyl substituted with R⁴⁴, U-1, U-2, U-4, U-5, U-6, U-7, U-8, U-12, U-14, U-15, U-16, or -NR⁶⁰R⁶¹;
   R³⁹ is a halogen atom, C₃₋₆ cycloalkyl, (C₃₋₆) cycloalkyl substituted with R⁴⁴, -OR⁵¹, - S(O)ᵣ₆R⁵², -C(=W²)R¹³, -CN, phenyl, phenyl substituted with (Z³)_{p5b}, U-1, U-2, U-3, U-4, U-5, U-9, U-10, U-11, U-12, U-13, U-14, U-15, U-16, U-17, U-18, U-19, U-20, U-21, U-22, U-23, U24, U-25, U-26, U-27, U-28, U-29, U-30, U-31, U-32, U-33, U-34, U-35, Q-1, Q-2, Q-3, Q-4, Q-5, Q-8, Q-9, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, or - ON=CR⁴²R⁴³;
   R⁴⁰ is a hydrogen atom, C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, (C₁₋₆) alkyl substituted with R⁴⁶, (C₃₋₆) cycloalkyl substituted with R⁴⁴, or U-4;
   R⁴¹ is a hydrogen atom, C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, (C₃₋₆) cycloalkyl substituted with R⁴⁴, or (C₁₋₆) alkyl substituted with R⁴⁷;
   R⁴² and R⁴³ are each independently a hydrogen atom, C₁₋₆ alkyl, phenyl, or phenyl substituted with (Z⁴)_{p5c}, or R⁴² and R⁴³ form C₃₋₆ cycloalkyl together with the carbon atom to which R⁴² and R⁴³ are bonded;
   R⁴⁴ is a halogen atom, C₁₋₆ alkyl, or -CN;
   R⁴⁵ is a halogen atom, C₁₋₆ alkyl, or -CN;
   R⁴⁶ is C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₃₋₆ cycloalkyl, (C₃₋₆) cycloalkyl substituted with R⁴⁴, -CN, U-1, U-2, U-3, U-4, U-5, U-9, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, or Q-36;
   R⁴⁷ is C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₃₋₆ cycloalkyl, (C₃₋₆) cycloalkyl substituted with R⁴⁴, U-1, U-2, U-3, U-4, U-5, U-9, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, or -CN;
   R⁴⁸ is a hydrogen atom, C₁₋₆ alkyl, C₁₋₆ haloalkyl, C₃₋₆ alkenyl, C₃₋₆ haloalkenyl, C₃₋₆ alkynyl, C₃₋₆ haloalkynyl, C₃₋₆ cycloalkyl, (C₃₋₆) cycloalkyl substituted with R⁴⁴, -C(O)R⁵⁰, or -S(O)₂R³³;
   R⁴⁹ is C₁₋₆ alkyl, C₁₋₆ haloalkyl, C₃₋₆ alkenyl, C₃₋₆ haloalkenyl, C₃₋₆ alkynyl, C₃₋₆ haloalkynyl, C₃₋₆ cycloalkyl, or (C₃₋₆) cycloalkyl substituted with R⁴⁴;
   R⁵⁰ is C₁₋₆ alkyl, C₁₋₆ alkoxy, or di(C₁₋₆ alkyl)amimo;
   R⁵¹ is a hydrogen atom, C₁₋₆ alkyl, C₃₋₆ alkenyl, C₃₋₆ alkynyl, C₁₋₆ haloalkyl, C₃₋₆ haloalkenyl, or C₃₋₆ haloalkynyl;
   R⁵² is C₁₋₆ alkyl, C₃₋₆ alkenyl, C₃₋₆ alkynyl, C₁₋₆ haloalkyl, C₃₋₆ haloalkenyl, C₃₋₆ haloalkynyl, or C₃₋₆ cycloalkyl;
   R⁵³ is C₁₋₆ alkyl, C₁₋₆ alkoxy, phenyl, or phenyl substituted with (Z⁴)_{p5c};
   R⁵⁴ is C₁₋₆ alkyl, C₁₋₆ haloalkyl, C₃₋₆ alkenyl, C₃₋₆ alkynyl, phenyl, or phenyl substituted with (Z⁴)_{p5c};
   R⁵⁵ is C₁₋₆ alkyl, C₁₋₆ alkoxy, -OH, or NR⁵⁶R⁵⁷;
   R⁵⁶ and R⁵⁷ are each independently a hydrogen atom or C₁₋₆ alkyl;
   R⁵⁸ is (C₁₋₆) alkyl substituted with R²⁷, C₃₋₆ cycloalkyl, (C₃₋₆) cycloalkyl substituted with R⁴⁴, phenyl, or phenyl substituted with (Z³)_{p5b};
   R⁵⁹ is C₁₋₆ alkyl, C₁₋₆ haloalkyl, C₁₋₆ alkenyl, C₁₋₆ alkynyl, C₃₋₆ cycloalkyl, (C₃₋₆) cycloalkyl substituted with R⁴⁴, phenyl, or phenyl substituted with (Z³)_{p5b};
   R⁶⁰ and R⁶¹ are each independently a hydrogen atom, C₁₋₆ alkyl, C₁₋₆ haloalkyl, C₁₋₆ alkoxy (C₁₋₆) alkyl, phenyl, phenyl substituted with (Z⁴)_{p5c}, benzyl, or benzyl substituted with (Z⁴)_{p5c};
   R^{N} is a hydrogen atom or C₁₋₆ alkyl;
   Y² is a halogen atom, C₁₋₆ alkyl, C₁₋₆ haloalkyl, C₁₋₆ alkoxy, C₁₋₆ haloalkoxy, C₁₋₆ alkylthio, C₁₋₆ haloalkylthio, -CN, -NH₂, or -NO₂, and when q4, q3, or q2 is an integer of 2 or more, each Y² is the same as or different from each other;
   Y³ is a halogen atom, C₁₋₆ alkyl, C₁₋₆ haloalkyl, C₁₋₆ alkoxy, C₁₋₆ haloalkoxy, C₁₋₆ alkylthio, C₁₋₆ haloalkylthio, C₁₋₆ alkoxycarbonyl, -CN, -C(O)OH, -OH, or -NH₂, and when t is 2, each Y³ is the same as or different from each other;
   Z² is a halogen atom, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, - S(O)₂NR⁵⁶R⁵⁷, -OH, -NH₂, -CN, -NO₂, or -C(O)R⁵⁵, and when p5a is an integer of 2 or more, each Z² is the same as or different from each other;
   Z³ is a halogen atom, C₁₋₆ alkyl, C₁₋₆ haloalkyl, C₁₋₆ alkoxy, C₁₋₆ haloalkoxy, C₁₋₆ alkylthio, C₁₋₆ haloalkylthio, -CN, C₁₋₆ alkoxycarbonyl, or -NO₂, and when p5b is an integer of 2 or more, each Z³ is the same as or different from each other;
   Z⁴ is a halogen atom, C₁₋₆ alkyl, C₁₋₆ haloalkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, -CN, -NO₂, or C₁₋₆ alkoxycarbonyl, and when p5c is an integer of 2 or more, each Z⁴ is the same as or different from each other;
   W² is an oxygen atom or N-OR⁷;
   W³ is an oxygen atom or N-OR²¹;
   W⁴ is an oxygen atom or a sulfur atom;
   r1 is an integer of 0, 1, or 2;
   r2 is an integer of 0, 1, or 2;
   r3 is an integer of 0, 1, or 2;
   r4 is an integer of 0, 1, or 2;
   r5 is an integer of 0, 1, or 2;
   r6 is an integer of 0, 1, or 2;
   n is an integer of 0, 1, 2, 3, or 4;
   t is an integer of 0, 1, or 2;
   p2 is an integer of 0, 1, or 2;
   p3 is an integer of 0, 1, 2, or 3;
   p4 is an integer of 0, 1, 2, 3, or 4;
   p5 is an integer of 0, 1, 2, 3, 4, or 5;
   p6 is an integer of 0, 1, 2, 3, 4, 5, or 6;
   p7 is an integer of 0, 1, 2, 3, 4, 5, 6, or 7;
   p5a is an integer of 1, 2, 3, 4, or 5;
   p5b is an integer of 1, 2, 3, 4, or 5;
   p5c is an integer of 1, 2, 3, 4, or 5;
   q1 is an integer of 0 or 1;
   q2 is an integer of 0, 1, or 2;
   q3 is an integer of 0, 1, 2, or 3; and
   q4 is an integer of 0, 1, 2, 3, or 4].
[2] The pyridazinone compound and a salt thereof according to [1], wherein:
   W¹ is an oxygen atom;
   Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, (C₃₋₆) cycloalkyl substituted with R⁴⁴, phenyl, phenyl substituted with (Z⁴)_{p5c}, Q-3, -NR¹⁵R¹⁶, -OR³⁵, -S(O)ᵣ₁R³⁶, -CN, -NO₂, - C(O)OH, -C(=W³)R²⁰, or -N=C(C₆H₅)₂, and when n is an integer of 2 or more, each Z¹ is the same as or different from each other;
   R¹ is a hydrogen atom, C₁₋₆ alkyl, C₃₋₆ alkenyl, C₃₋₆ alkynyl, or C₁₋₆ alkyl substituted with R³⁴;
   R² is a hydrogen atom, a halogen atom, C₁₋₆ alkyl, C₁₋₆ alkoxy, or -NR³⁰R³¹;
   R³ is D-1, D-2, D-3, D-4, D-5, D-6, D-7, D-8, D-9, D-10, D-13, D-14, D-15, D-16, D-17, D-18, D-19, D-20, D-21, D-22, D-23, D-24, D-25, D-26, D-27, or D-28;
   Y¹ is a hydrogen atom, a halogen atom, C₁₋₆ alkyl, C₂₋₆ alkenyl, C₂₋₆ alkynyl, C₃₋₆ cycloalkyl, C₂₋₆ haloalkenyl, C₂₋₆ haloalkynyl, (C₁₋₆) alkyl substituted with R¹⁰, C₃₋₆ cycloalkyl substituted with R⁴⁴, -OR¹¹, -S(O)ᵣ₂R³¹, -NR⁸R⁹, -CN, -NO₂, -C(O)OH, - C(=W²)R¹³, phenyl, phenyl substituted with (Z⁴)_{p5c}, or tri(C₁₋₆ alkyl)silyl, and when p7, p6, p5, p4, p3, or p2 is an integer of 2 or more, each Y¹ is the same as or different from each other;
   R⁴ is a halogen atom, -CN, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁴, -OR²⁴, -S(O)ᵣ₄R⁵⁴, - C(O)R²³, phenyl, or phenyl substituted with (Z⁴)_{p5c};
   R⁵ is C₁₋₆ alkyl, (C₁₋₆) alkyl substituted with R²⁶, C₂₋₆ alkenyl, C₂₋₆ alkynyl, C₁₋₆ alkoxy, -OR⁵⁸, -SR⁵⁹, C₃₋₆ cycloalkyl, C₃₋₆ cycloalkyl substituted with R⁴⁴, -NR⁵⁶R⁵⁷, U-1, U-6, U-7, U-8, Q-1, Q-2, Q-3, Q-4, Q-5, Q-7, Q-8, Q-9, Q-10, Q-17, Q-18, Q-19, Q-20, phenyl, phenyl substituted with (Z²)ₚ₅ₐ, or -C(O)R⁵³;
   R⁶ is C₁₋₆ alkyl, C₁₋₆ haloalkyl, phenyl, phenyl substituted with (Z²)ₚ₅ₐ, or -NR²⁸R²⁹;
   R⁷ is a hydrogen atom, C₁₋₆ alkyl, C₃₋₆ alkenyl, C₃₋₆ alkynyl, C₁₋₆ haloalkyl, C₁₋₆ alkoxy (C₁₋₂) alkyl, or C₁₋₆ alkylthio (C₁₋₂) alkyl;
   R¹¹ is a hydrogen atom, C₁₋₆ alkyl, (C₁₋₆) alkyl substituted with R¹², C₃₋₆ cycloalkyl, (C₃₋₆) cycloalkyl substituted with R⁴⁴, phenyl, phenyl substituted with (Z⁴)_{p5c}, C₁₋₆ alkylsulfonyl, C₁₋₆ haloalkylsulfonyl, U-2, U-4, Q-17, Q-18, or Q-19;
   R¹² is a halogen atom, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁵, -OR⁴⁸, -S(O)ᵣ₅R⁴⁹, -CN, phenyl, phenyl substituted with (Z³)_{p5b}, -C(=W²)R¹³, U-1, U-2, U-3, Q-1, Q-2, Q-3, Q-4, Q-5, Q-18, or - ON=CR⁴²R⁴³;
   R¹⁴ is a halogen atom, C₂₋₆ alkenyl, C₂₋₆ alkynyl, C₃₋₆ cycloalkyl, C₂₋₆ haloalkenyl, C₂₋₆ haloalkynyl, (C₃₋₆) cycloalkyl substituted with R⁴⁵, C₁₋₆ alkoxy, C₁₋₆ alkylthio, C₁₋₆ alkylsulfinyl, C₁₋₆ alkylsulfonyl, phenyl, phenyl substituted with (Z⁴)_{p5c}, -CN, U-1, U-2, U-3, U-9, Q-17, Q-18, Q-19, or Q-20;
   R¹⁹ is tri(C₁₋₆ alkyl)silyl;
   R²² is a halogen atom, -OH, C₁₋₆ alkoxy, C₁₋₆ haloalkoxy, C₁₋₆ alkylthio, C₁₋₆ haloalkylthio, or -CN;
   R²³ is C₁₋₆ alkyl, C₁₋₆ alkoxy, di(C₁₋₆) alkylamino, phenyl, phenyl substituted with (Z⁴)_{p5c}, U-7, or U-8;
   R²⁴ is C₁₋₆ alkyl, C₁₋₆ haloalkyl, C₃₋₆ alkenyl, C₃₋₆ alkynyl, (C₁₋₆) alkoxy (C₁₋₂) alkyl, - C(O)R²⁵, or -S(O)₂R³³;
   R²⁵ is C₁₋₆ alkyl, C₁₋₆ alkoxy, or di(C₁₋₆) alkylamino;
   R²⁶ is a halogen atom, C₁₋₆ alkoxy, C₁₋₆ haloalkoxy, C₁₋₆ alkylthio, C₁₋₆ alkylsulfinyl, C₁₋₆ alkylsulfonyl, -OR³², phenyl, or phenyl substituted with (Z⁴)_{p5c};
   R²⁷ is a halogen atom, C₂₋₆ alkenyl, C₂₋₆ alkynyl, C₁₋₆ alkoxy, or C₁₋₆ alkylthio;
   R³⁶ is C₁₋₆ alkyl, (C₁₋₆) alkyl substituted with R³⁷, or C₃₋₆ cycloalkyl;
   R³⁷ is a halogen atom, C₂₋₆ alkenyl, C₂₋₆ alkynyl, C₃₋₆ cycloalkyl, C₁₋₆ alkoxy, C₁₋₆ alkylthio, or C₁₋₁₀ alkoxycarbonyl;
   R³⁸ is C₁₋₆ alkyl, C₃₋₆ alkenyl, C₃₋₆ alkynyl, C₃₋₆ cycloalkyl, C₃₋₆ haloalkenyl, C₃₋₆ haloalkynyl, (C₁₋₆) alkyl substituted with R³⁹, or (C₃₋₆) cycloalkyl substituted with R⁴⁴;
   R³⁹ is a halogen atom, C₃₋₆ cycloalkyl, -OR⁵¹, -S(O)ᵣ₆R⁵², -C(=W²)R¹³, -CN, phenyl, phenyl substituted with (Z³)_{p5b}, U-1, U-3, U-9, Q-1, or Q-18;
   R⁴⁰ is a hydrogen atom, C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, (C₁₋₆) alkyl substituted with R⁴⁶, or U-4;
   R⁴¹ is C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, or (C₁₋₆) alkyl substituted with R⁴⁷;
   R⁴⁶ is C₁₋₆ alkoxy, C₁₋₆ haloalkoxy, C₁₋₆ alkylthio, C₁₋₆ haloalkylthio, C₁₋₆ alkylsulfinyl, C₁₋₆ alkylsulfonyl, C₃₋₆ cycloalkyl, -CN, U-1, U-3, or U-9;
   R⁴⁷ is C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, or -CN;
   R⁴⁸ is a hydrogen atom, C₁₋₆ alkyl, C₁₋₆ haloalkyl, C₃₋₆ alkenyl, C₃₋₆ haloalkenyl, C₃₋₆ alkynyl, C₃₋₆ haloalkynyl, or C₃₋₆ cycloalkyl;
   R⁴⁹ is C₁₋₆ alkyl, C₁₋₆ haloalkyl, C₃₋₆ alkenyl, or C₃₋₆ alkynyl;
   R⁵¹ is C₁₋₆ alkyl, C₃₋₆ alkenyl, C₃₋₆ alkynyl, or C₁₋₆ haloalkyl;
   R⁵² is C₁₋₆ alkyl, C₃₋₆ alkenyl, or C₃₋₆ alkynyl;
   R⁵⁴ is C₁₋₆ alkyl or C₃₋₆ alkenyl;
   R⁵⁹ is C₁₋₆ alkyl, C₁₋₆ alkenyl, or phenyl;
   Y² is a halogen atom, C₁₋₆ alkyl, C₁₋₆ haloalkyl, C₁₋₆ alkoxy, C₁₋₆ haloalkoxy, C₁₋₆ alkylthio, or C₁₋₆ haloalkylthio; and
   Z² is a halogen atom, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, - OH, -NH₂, -CN, -NO₂, or -C(O)R⁵⁵, and when p5a is an integer of 2 or more, each Z² is the same as or different from each other.
[3] The pyridazinone compound and a salt thereof according to [2], wherein:
   Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, phenyl, phenyl substituted with (Z⁴)_{p5c}, Q-3, -NR¹⁵R¹⁶, -OR³⁵, -S(O)ᵣ₁R³⁶, -CN, -C(=W³)R²⁰, or -N=C(C₆H₅)₂, and when n is an integer of 2 or more, each Z¹ is the same as or different from each other;
   R¹ is a hydrogen atom, C₁₋₆ alkyl, or C₁₋₆ alkyl substituted with R³⁴;
   R³ is D-1, D-2, D-3, D-4, D-6, D-7, D-8, D-9, D-10, D-17, D-18, D-19, D-20, D-21, D-22, D-23, D-24, D-25, D-26, or D-28;
   Y¹ is a hydrogen atom, a halogen atom, C₁₋₆ alkyl, C₂₋₆ alkenyl, (C₁₋₆) alkyl substituted with R¹⁰, -C(O)OH, -OR¹¹, -S(O)ᵣ₂R³⁸, -NR⁸R⁹, -CN, -NO₂, -C(=W²)R¹³, phenyl, or tri(C₁₋₆ alkyl)silyl, and when p7, p6, p5, p4, p3, or p2 is an integer of 2 or more, each Y¹ is the same as or different from each other;
   R⁴ is a halogen atom, -CN, C₂₋₆ alkenyl, C₂₋₆ alkynyl, -OR²⁴, -S(O)ᵣ₄R⁵⁴, -C(O)R²³, or phenyl;
   R⁵ is C₁₋₆ alkyl, (C₁₋₆) alkyl substituted with R²⁶, C₂₋₆ alkenyl, C₁₋₆ alkoxy, -OR⁵⁸, -SR⁵⁹, C₃₋₆ cycloalkyl, -NR⁵⁶R⁵⁷, U-1, U-6, Q-2, Q-4, phenyl substituted with (Z²)ₚ₅ₐ, or -C(O)R⁵³;
   R⁶ is C₁₋₆ alkyl, phenyl substituted with (Z²)ₚ₅ₐ, or -NR²⁸R²⁹;
   R⁷ is a hydrogen atom or C₁₋₆ alkyl;
   R⁸ and R⁹ are each independently C₁₋₆ alkyl;
   R¹⁰ is a halogen atom, -OR⁴⁰, or -S(O)ᵣ₃R⁴¹;
   R¹¹ is a hydrogen atom, C₁₋₆ alkyl, (C₁₋₆) alkyl substituted with R¹², C₃₋₆ cycloalkyl, phenyl, C₁₋₆ alkylsulfonyl, C₁₋₆ haloalkylsulfonyl, U-2, U-4, or Q-17;
   R¹² is a halogen atom, C₂₋₆ alkenyl, C₂₋₆ haloalkenyl, C₂₋₆ alkynyl, C₃₋₆ cycloalkyl, (C₃₋₆) cycloalkyl substituted with R⁴⁵, -OR⁴⁸, -S(O)ᵣ₅R⁴⁹, -CN, phenyl, -C(=W²)R¹³, U-3, Q-1, or -ON=CR⁴²R⁴³;
   R¹³ is C₁₋₆ alkyl, C₁₋₆ alkoxy, or C₁₋₆ haloalkylamino;
   R¹⁴ is a halogen atom, C₂₋₆ alkenyl, C₃₋₆ cycloalkyl, (C₃₋₆) cycloalkyl substituted with R⁴⁵, C₁₋₆ alkoxy, C₁₋₆ alkylthio, C₁₋₆ alkylsulfonyl, phenyl, or U-1;
   R¹⁸ is C₁₋₆ alkyl;
   R²¹ is a hydrogen atom or C₁₋₆ alkyl;
   R²² is a halogen atom, -OH, or C₁₋₆ alkoxy;
   R²³ is C₁₋₆ alkyl, C₁₋₆ alkoxy, or phenyl;
   R²⁴ is C₁₋₆ alkyl, (C₁₋₆) alkoxy (C₁₋₂) alkyl, or -C(O)R²⁵;
   R²⁵ is C₁₋₆ alkoxy;
   R²⁶ is C₁₋₆ alkoxy, -OR³², or phenyl;
   R²⁷ is a halogen atom, C₂₋₆ alkenyl, or C₁₋₆ alkoxy;
   R²⁸ and R²⁹ are each independently C₁₋₆ alkyl;
   R³⁰ and R³¹ are each independently a hydrogen atom or benzyl;
   R³² is phenyl;
   R³³ is C₁₋₆ haloalkyl or di(C₁₋₆) alkylamino;
   R³⁴ is C₁₋₆ alkoxy, phenyl, or -CN;
   R³⁵ is a hydrogen atom, C₁₋₆ alkyl, C₃₋₆ cycloalkyl, (C₁₋₆) alkyl substituted with R¹⁴, or - S(O)₂R³³;
   R³⁶ is C₁₋₆ alkyl or (C₁₋₆) alkyl substituted with R³⁷;
   R³⁷ is a halogen atom or (C₁₋₁₀) alkoxycarbonyl;
   R³⁸ is C₁₋₆ alkyl, C₃₋₆ alkenyl, C₃₋₆ alkynyl, C₃₋₆ cycloalkyl, C₃₋₆ haloalkenyl, C₃₋₆ haloalkynyl, or (C₁₋₆) alkyl substituted with R³⁹;
   R³⁹ is a halogen atom, -OR⁵¹, -S(O)ᵣ₆R⁵², -C(=W²)R¹³, or -CN;
   R⁴⁰ is a hydrogen atom, C₁₋₆ alkyl, C₃₋₆ alkenyl, C₁₋₆ haloalkyl, or (C₁₋₆) alkyl substituted with R⁴⁶;
   R⁴¹ is C₁₋₆ alkyl, C₃₋₆ alkenyl, C₃₋₆ alkynyl, C₁₋₆ haloalkyl, or (C₁₋₆) alkyl substituted with R⁴⁷;
   R⁴² and R⁴³ are each independently C₁₋₆ alkyl;
   R⁴⁵ is a halogen atom;
   R⁴⁶ is C₁₋₆ alkoxy or C₁₋₆ alkylthio;
   R⁴⁷ is C₁₋₆ alkoxy or C₁₋₆ alkylthio;
   R⁴⁸ is C₁₋₆ alkyl or C₁₋₆ haloalkyl;
   R⁴⁹ is C₁₋₆ alkyl;
   R⁵¹ is C₁₋₆ alkyl;
   R⁵² is C₁₋₆ alkyl;
   R⁵³ is phenyl;
   R⁵⁴ is C₁₋₆ alkyl;
   R⁵⁶ and R⁵⁷ are each independently C₁₋₆ alkyl;
   R⁵⁸ is (C₁₋₆) alkyl substituted with R²⁷ or phenyl;
   R⁵⁹ is C₁₋₆ alkyl or phenyl;
   Y² is a halogen atom or C₁₋₆ haloalkyl;
   Z² is a halogen atom, C₁₋₆ alkyl, or C₁₋₆ alkoxy, and when p5a is an integer of 2 or more, each Z² is the same as or different from each other;
   Z⁴ is a halogen atom or C₁₋₆ alkoxy, and when p5c is an integer of 2 or more, each Z⁴ is the same as or different from each other; and
   t is 0.
[4] The pyridazinone compound and a salt thereof according to [3], wherein:
   Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, -NR¹⁵R¹⁶, -OR³⁵, -S(O)ᵣ₁R³⁶, or -CN, and when n is an integer of 2 or more, each Z¹ is the same as or different from each other;
   R² is a hydrogen atom, a halogen atom, C₁₋₆ alkyl, or C₁₋₆ alkoxy;
   R³ is D-1, D-2, D-3, D-4, D-6, D-7, D-8, D-9, D-10, D-17, D-18, D-20, D-21, D-22, D-23, D-24, or D-28;
   Y¹ is a halogen atom, C₁₋₆ alkyl, C₂₋₆ alkenyl, (C₁₋₆) alkyl substituted with R¹⁰, -OR¹¹,-S(O)ᵣ₂R³⁸, -NR⁸R⁹, -CN, -NO₂, or -C(=W²)R¹³, and when p7, p6, p5, p4, p3, or p2 is an integer of 2 or more, each Y¹ is the same as or different from each other;
   R¹¹ is a hydrogen atom, C₁₋₆ alkyl, (C₁₋₆) alkyl substituted with R¹², C₃₋₆ cycloalkyl, phenyl, C₁₋₆ alkylsulfonyl, C₁₋₆ haloalkylsulfonyl, U-2, or U-4; and
   R³⁷ is a halogen atom.
[5] The pyridazinone compound or a salt thereof according to [3] or [4], wherein X is a sulfur atom.
[6] The pyridazinone compound or a salt thereof according to [2], wherein:
   X is an oxygen atom;
   Z¹ is a halogen atom, C₁₋₆ alkyl, -OR³⁵, or -S(O)ᵣ₁R³⁶, and when n is an integer of 2 or more, each Z¹ is the same as or different from each other;
   R¹ is C₁₋₆ alkyl;
   R² is a hydrogen atom, a halogen atom, C₁₋₆ alkyl, or C₁₋₆ alkoxy;
   R³ is D-1, D-3, D-7, D-20, D-21, D-22, or D-24;
   Y¹ is a halogen atom, C₁₋₆ alkyl, (C₁₋₆) alkyl substituted with R¹⁰, -OR¹¹, or -S(O)ᵣ₂R³⁸, and when p7, p6, p5, p4, p3, or p2 is an integer of 2 or more, each Y¹ is the same as or different from each other;
   R⁴ is -OR²⁴ or -S(O)ᵣ₄R⁵⁴;
   R⁵ is C₁₋₆ alkyl, (C₁₋₆) alkyl substituted with R²⁶, or C₁₋₆ alkoxy;
   R⁶ is C₁₋₆ alkyl or -NR²⁸R²⁹;
   R¹⁰ is a halogen atom, -OR⁴⁰, or -S(O)ᵣ₃R⁴¹;
   R¹¹ is a hydrogen atom, C₁₋₆ alkyl, or (C₁₋₆) alkyl substituted with R¹²;
   R¹² is a halogen atom, C₂₋₆ alkenyl, -OR⁴⁸, -S(O)ᵣ₅R⁴⁹, phenyl, or -C(=W²)R¹³;
   R¹³ is a hydrogen atom or C₁₋₆ alkyl;
   R¹⁴ is a halogen atom, C₂₋₆ alkenyl, C₁₋₆ alkoxy, or C₁₋₆ alkylthio;
   R²⁴ is C₁₋₆ alkyl;
   R²⁶ is C₁₋₆ alkoxy;
   R²⁸ and R²⁹ are each independently C₁₋₆ alkyl;
   R³⁴ is C₁₋₆ alkoxy or -CN;
   R³⁵ is C₁₋₆ alkyl or (C₁₋₆) alkyl substituted with R¹⁴;
   R³⁶ is C₁₋₆ alkyl or (C₁₋₆) alkyl substituted with R³⁷;
   R³⁷ is a halogen atom, C₁₋₆ alkoxy, or C₁₋₆ alkylthio;
   R³⁸ is C₁₋₆ alkyl, C₃₋₆ alkenyl, C₃₋₆ alkynyl, or (C₁₋₆) alkyl substituted with R³⁹;
   R³⁹ is -OR⁵¹ or -S(O)ᵣ₆R⁵²;
   R⁴⁰ is C₁₋₆ alkyl, C₃₋₆ alkenyl, or C₃₋₆ alkynyl;
   R⁴¹ is C₁₋₆ alkyl, C₃₋₆ alkenyl, C₃₋₆ alkynyl, or (C₁₋₆) alkyl substituted with R⁴⁷;
   R⁴⁸ is C₁₋₆ alkyl;
   r1 is an integer of 0, 1, or 2;
   r4 is an integer of 0, 1, or 2;
   r5 is an integer of 0, 1, or 2;
   r6 is an integer of 0, 1, or 2;
   n is an integer of 0, 1, 2, 3, or 4;
   p3 is an integer of 0, 1, 2, or 3;
   p4 is an integer of 0, 1, 2, 3, or 4; and
   p5 is an integer of 0, 1, 2, 3, 4, or 5.
[7] The pyridazinone compound or a salt thereof according to [6], wherein:
   Z¹ is a halogen atom, C₁₋₆ alkyl, or -OR³⁵;
   R¹ is C₁₋₆ alkyl;
   R² is C₁₋₆ alkyl or C₁₋₆ alkoxy;
   R³ is D-1, D-7, or D-24;
   Y¹ is a halogen atom, C₁₋₆ alkyl, (C₁₋₆) alkyl substituted with R¹⁰, -OR¹¹, or -S(O)ᵣ₂R³⁸;
   G is a hydrogen atom, C₁₋₆ alkyl, or -C(=W⁴)R⁵;
   R⁵ is C₁₋₆ alkyl;
   R¹⁰ is a halogen atom or -OR⁴⁰;
   R¹² is a halogen atom, C₂₋₆ alkenyl, -OR⁴⁸, -S(O)ᵣ₅R⁴⁹, or phenyl;
   R³⁵ is C₁₋₆ alkyl;
   R³⁸ is C₁₋₆ alkyl;
   R⁴⁰ is C₁₋₆ alkyl;
   R⁴⁸ is C₁₋₆ alkyl; and
   R⁴⁹ is C₁₋₆ alkyl.
[8] The pyridazinone compound and a salt thereof according to [5], wherein:
   Z¹ is a halogen atom, C₁₋₆ alkyl, -OR³⁵, or -S(O)ᵣ₁R³⁶, and when n is an integer of 2 or more, each Z¹ is the same as or different from each other;
   R¹ is C₁₋₆ alkyl;
   R² is a hydrogen atom, a halogen atom, or C₁₋₆ alkyl;
   R³ is D-1 or D-3;
   R⁴ is -OR²⁴;
   Y¹ is a hydrogen atom, a halogen atom, C₁₋₆ alkyl, (C₁₋₆) alkyl substituted with R¹⁰, - OR¹¹, -S(O)ᵣ₂R³⁸, or -C(=W²)R¹³, and when p7, p6, p5, p4, p3, or p2 is an integer of 2 or more, each Y¹ is the same as or different from each other;
   R⁷ is C₁₋₆ alkyl;
   R¹¹ is C₁₋₆ alkyl, (C₁₋₆) alkyl substituted with R¹², or C₃₋₆ cycloalkyl;
   R¹² is a halogen atom, C₂₋₆ alkenyl, C₂₋₆ haloalkenyl, C₂₋₆ alkynyl, C₃₋₆ cycloalkyl, (C₃₋₆) cycloalkyl substituted with R⁴⁵, -OR⁴⁸, -S(O)ᵣ₅R⁴⁹, -CN, -C(=W²)R¹³, or -ON=CR⁴²R⁴³;
   R¹⁴ is a halogen atom, C₃₋₆ cycloalkyl, (C₃₋₆) cycloalkyl substituted with R⁴⁵, C₁₋₆ alkylthio, C₁₋₆ alkylsulfonyl, or U-1;
   R²⁴ is C₁₋₆ alkyl or (C₁₋₆) alkoxy (C₁₋₂) alkyl;
   R³⁵ is C₁₋₆ alkyl or (C₁₋₆) alkyl substituted with R¹⁴;
   R³⁸ is C₁₋₆ alkyl, C₃₋₆ cycloalkyl, or (C₁₋₆) alkyl substituted with R³⁹;
   R³⁹ is a halogen atom, -OR⁵¹, -S(O)ᵣ₆R⁵², -C(=W²)R¹³, or -CN;
   R⁴⁰ is a hydrogen atom, C₁₋₆ alkyl, C₃₋₆ alkenyl, C₁₋₆ haloalkyl, or (C₁₋₆) alkyl substituted with R⁴⁶;
   R⁴¹ is a hydrogen atom, C₁₋₆ alkyl, C₁₋₆ haloalkyl, or (C₁₋₆) alkyl substituted with R⁴⁷;
   R⁴⁶ is C₁₋₆ alkoxy or C₁₋₆ alkylthio; and
   W² is N-OR⁷.
[9] The pyridazinone compound and a salt thereof according to [8], wherein:
   G is a hydrogen atom;
   R¹¹ is C₁₋₆ alkyl or (C₁₋₆) alkyl substituted with R¹²;
   R¹² is a halogen atom, C₂₋₆ alkenyl, C₂₋₆ haloalkenyl, C₃₋₆ cycloalkyl, (C₃₋₆) cycloalkyl substituted with R⁴⁵, -OR⁴⁸, -S(O)ᵣ₅R⁴⁹, or -C(=W²)R¹³;
   R³⁶ is C₁₋₆ alkyl;
   R³⁸ is C₁₋₆ alkyl or (C₁₋₆) alkyl substituted with R³⁹; and
   n is an integer of 0 or 1.
[10] The pyridazinone compound and a salt thereof according to [3], wherein:
   W¹ is an oxygen atom;
   Z¹ is C₁₋₆ alkyl, (C₁₋₆) alkyl substituted with R²², phenyl substituted with (Z⁴)_{p5c},-NR¹⁵R¹⁶, -OR³⁵, -C(=W³)R²⁰, or -N=C(C₆H₅)₂, and when n is an integer of 2 or more, each Z¹ is the same as or different from each other;
   G is a hydrogen atom, C₁₋₆ alkyl, or (C₁₋₆) alkyl substituted with R⁴;
   R¹ is a hydrogen atom, C₁₋₆ alkyl, or C₁₋₆ alkyl substituted with R³⁴;
   R² is a hydrogen atom, C₁₋₆ alkyl, C₁₋₆ alkoxy, or -NR³⁰R³¹;
   R³ is D-1, D-3, D-4, or D-6;
   Y¹ is a hydrogen atom, a halogen atom, C₁₋₆ alkyl, (C₁₋₆) alkyl substituted with R¹⁰, - C(O)OH, -CN, -C(=W²)R¹³, phenyl, or tri(C₁₋₆ alkyl)silyl, and when p7, p6, p5, p4, p3, or p2 is an integer of 2 or more, each Y¹ is the same as or different from each other;
   R⁴ is a halogen atom, -CN, C₂₋₆ alkenyl, C₂₋₆ alkynyl, or phenyl;
   R⁷ is a hydrogen atom or C₁₋₆ alkyl;
   R⁸ and R⁹ are each independently C₁₋₆ alkyl;
   R¹⁰ is a halogen atom, -OR⁴⁰, or -S(O)ᵣ₃R⁴¹;
   R¹³ is C₁₋₆ alkyl or C₁₋₆ alkoxy;
   R¹⁴ is a halogen atom, C₂₋₆ alkenyl, C₁₋₆ alkoxy, C₁₋₆ alkylthio, or C₁₋₆ alkylsulfonyl;
   R¹⁵ and R¹⁶ are each independently a hydrogen atom or C₁₋₆ alkyl;
   R²⁰ is a hydrogen atom or C₁₋₆ alkyl;
   R²¹ is a hydrogen atom or C₁₋₆ alkyl;
   R²² is -OH or C₁₋₆ alkoxy;
   R²⁷ is a halogen atom or C₁₋₆ alkoxy;
   R³⁰ and R³¹ are a hydrogen atom;
   R³³ is C₁₋₆ haloalkyl or di(C₁₋₆) alkylamino;
   R³⁴ is C₁₋₆ alkoxy or -CN;
   R³⁵ is a hydrogen atom, C₁₋₆ alkyl, or (C₁₋₆) alkyl substituted with R¹⁴;
   R⁴⁰ is a hydrogen atom, C₁₋₆ alkyl, C₃₋₆ alkenyl, or C₁₋₆ haloalkyl;
   R⁴¹ is a hydrogen atom, C₁₋₆ alkyl, or C₁₋₆ haloalkyl;
   Z⁴ is a halogen atom or C₁₋₆ alkoxy, and when p5c is an integer of 2 or more, each Z⁴ is the same as or different from each other;
   W² is an oxygen atom or N-OR⁷; and
   W³ is an oxygen atom or N-OR²¹.
[11] The pyridazinone compound and a salt thereof according to [3], wherein:
   n is 0.
[12] The pyridazinone compound and a salt thereof according to [4], wherein:
   G is a hydrogen atom;
   R¹ is C₁₋₆ alkyl;
   R² is a halogen atom or C₁₋₆ alkyl;
   R³ is D-1;
   Y¹ is a hydrogen atom, a halogen atom, C₁₋₆ alkyl, (C₁₋₆) alkyl substituted with R¹⁰, - OR¹¹, or -S(O)ᵣ₂R³⁸, and when p7, p6, p5, p4, p3, or p2 is an integer of 2 or more, each Y¹ is the same as or different from each other;
   Z¹ is a halogen atom or -OR³⁵, and when n is an integer of 2 or more, each Z¹ is the same as or different from each other;
   R¹⁰ is a halogen atom;
   R¹¹ is C₁₋₆ alkyl or (C₁₋₆) alkyl substituted with R¹²;
   R¹² is a halogen atom, -OR⁴⁸, or S(O)ᵣ₅R⁴⁹;
   R³⁵ is C₁₋₆ alkyl or (C₁₋₆) alkyl substituted with R¹⁴;
   R³⁸ is C₁₋₆ alkyl or (C₁₋₆) alkyl substituted with R³⁹;
   R³⁹ is a halogen atom; and
   n is 0 or 1.
[13] The pyridazinone compound and a salt thereof according to [3], wherein:
   Z¹ is -OR³⁵, and when n is an integer of 2 or more, each Z¹ is the same as or different from each other;
   R¹ is C₁₋₆ alkyl;
   R² is a hydrogen atom, a halogen atom, or C₁₋₆ alkyl;
   R³ is D-1, D-3, or D-7;
   Y¹ is a hydrogen atom, a halogen atom, C₁₋₆ alkyl, (C₁₋₆) alkyl substituted with R¹⁰, - OR¹¹, -S(O)ᵣ₂R³⁸, -CN, or -C(=W²)R¹³, and when p7, p6, p5, p4, p3, or p2 is an integer of 2 or more, each Y¹ is the same as or different from each other;
   R⁷ is C₁₋₆ alkyl; and
   W² is N-OR⁷.
[14] The pyridazinone compound and a salt thereof according to [3], wherein:
   X is a sulfur atom;
   Z¹ is a halogen atom, and when n is an integer of 2 or more, each Z¹ is the same as or different from each other;
   R¹ is C₁₋₆ alkyl;
   R² is a hydrogen atom, a halogen atom, or C₁₋₆ alkyl;
   R³ is D-1;
   Y¹ is a hydrogen atom, a halogen atom, C₁₋₆ alkyl, (C₁₋₆) alkyl substituted with R¹⁰, - OR¹¹, -S(O)ᵣ₂R³⁸, -CN, or -C(=W²)R¹³, and when p7, p6, p5, p4, p3, or p2 is an integer of 2 or more, each Y¹ is the same as or different from each other;
   R⁷ is C₁₋₆ alkyl;
   R¹³ is C₁₋₆ alkyl; and
   W² is N-OR⁷.
[15] The pyridazinone compound and a salt thereof according to [3], wherein:
   Z¹ is C₃₋₆ cycloalkyl, and when n is an integer of 2 or more, each Z¹ is the same as or different from each other;
   R¹ is C₁₋₆ alkyl;
   R² is a hydrogen atom, a halogen atom, or C₁₋₆ alkyl;
   R³ is D-1 or D-3;
   Y¹ is a hydrogen atom, a halogen atom, C₁₋₆ alkyl, (C₁₋₆) alkyl substituted with R¹⁰, - OR¹¹, -S(O)ᵣ₂R³⁸, -CN, or -C(=W²)R¹³, and when p7, p6, p5, p4, p3, or p2 is an integer of 2 or more, each Y¹ is the same as or different from each other;
   R⁷ is C₁₋₆ alkyl; and
   W² is N-OR⁷.
[16] The pyridazinone compound and a salt thereof according to [7], wherein:
   R³ is D-1;
   R¹⁰ is a halogen atom; and
   n is an integer of 0 or 1.
[17] The pyridazinone compound and a salt thereof according to [9], wherein:
   Y¹ is a hydrogen atom, a halogen atom, or -OR¹¹, and when p7, p6, p5, p4, p3, or p2 is an integer of 2 or more, each Y¹ is the same as or different from each other;
   Z¹ is a halogen atom, and when n is an integer of 2 or more, each Z¹ is the same as or different from each other;
   R¹¹ is (C₁₋₆) alkyl substituted with R¹²;
   R¹² is a halogen atom; and
   n is 0 or 1.
[18] The pyridazinone compound and a salt thereof according to [9], wherein:
   Y¹ is a hydrogen atom, C₁₋₆ alkyl, or (C₁₋₆) alkyl substituted with R¹⁰, and when p7, p6, p5, p4, p3, or p2 is an integer of 2 or more, each Y¹ is the same as or different from each other;
   Z¹ is C₁₋₆ alkyl or -OR³⁵, and when n is an integer of 2 or more, each Z¹ is the same as or different from each other;
   R¹⁴ is a halogen atom or C₁₋₆ alkoxy;
   R³⁵ is a hydrogen atom, C₁₋₆ alkyl, or (C₁₋₆) alkyl substituted with R¹⁴; and
   n is 0 or 1.
[19] The pyridazinone compound or a salt thereof according to any of [1] to [3], wherein: 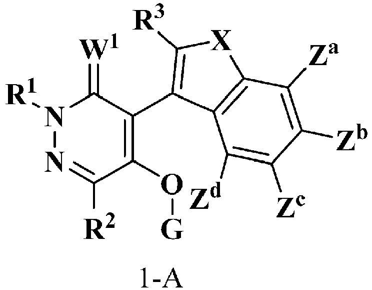
   Z^{a} is a hydrogen atom, a halogen atom, C₁₋₆ alkyl, or -OR³⁵;
   Z^{b} is a hydrogen atom, a halogen atom, C₁₋₆ alkyl, or (C₁₋₆) alkyl substituted with R²²;
   Z^{c} is a hydrogen atom, a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, phenyl, phenyl substituted with (Z⁴)_{p5c}, Q-3, -NR¹⁵R¹⁶, -OR³⁵, -S(O)ᵣ₁R³⁶, -CN, -C(=W³)R²⁰, or-N=C(C₆H₅)₂;
   Z^{d} is a hydrogen atom or a halogen atom;
   R¹⁴ is a halogen atom, C₂₋₆ alkenyl, C₃₋₆ cycloalkyl, (C₃₋₆) cycloalkyl substituted with R⁴⁵, C₁₋₆ alkoxy, C₁₋₆ alkylthio, C₁₋₆ alkylsulfonyl, phenyl, or U-1;
   R¹⁵ and R¹⁶ are each independently a hydrogen atom, C₁₋₆ alkyl, -C(O)R¹⁷, or-S(O)₂R¹⁸;
   R¹⁷ is C₁₋₆ alkyl, C₁₋₆ alkoxy, or C₁₋₆ alkoxy (C₁₋₂) alkyl;
   R¹⁸ is C₁₋₆ alkyl;
   R¹⁹ is tri(C₁₋₆ alkyl )silyl;
   R²⁰ is a hydrogen atom or C₁₋₆ alkyl;
   R²¹ is a hydrogen atom or C₁₋₆ alkyl;
   R²² is a halogen atom, -OH, or C₁₋₆ alkoxy;
   R³³ is di(C₁₋₆) alkylamino;
   R³⁵ is a hydrogen atom, C₁₋₆ alkyl, C₃₋₆ cycloalkyl, (C₁₋₆) alkyl substituted with R¹⁴, or - S(O)₂R³³;
   R³⁶ is C₁₋₆ alkyl or (C₁₋₆) alkyl substituted with R³⁷;
   R³⁷ is a halogen atom or (C₁₋₁₀) alkoxycarbonyl;
   R⁴⁵ is a halogen atom;
   Z⁴ is a halogen atom or C₁₋₆ alkoxy, and when p5c is an integer of 2 or more, each Z⁴ is the same as or different from each other;
   W³ is an oxygen atom or N-OR²¹;
   r1 is an integer of 0 or 2;
   p5c is an integer of 1 or 2; and
   q3 is 0.
[20] The pyridazinone compound or a salt thereof according to any of [1] to [19], wherein:
   W¹ is an oxygen atom.
[21] The pyridazinone compound or a salt thereof according to any of [1] to [19], wherein:
   W¹ is a sulfur atom.
[22] The pyridazinone compound or a salt thereof according to any of [1] to [21], wherein:
   Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, (C₃₋₆) cycloalkyl substituted with R⁴⁴, phenyl, phenyl substituted with (Z⁴)_{p5c}, Q-3, -NR¹⁵R¹⁶, -OR³⁵, -S(O)ᵣ₁R³⁶, -CN, -NO₂, - C(O)OH, -C(=W³)R²⁰, or -N=C(C₆H₅)₂, and when n is an integer of 2 or more, each Z¹ is the same as or different from each other.
[23] The pyridazinone compound or a salt thereof according to [22], wherein:
   Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, phenyl, phenyl substituted with (Z⁴)_{p5c}, Q-3, -NR¹⁵R¹⁶, -OR³⁵, -S(O)ᵣ₁R³⁶, -CN, -C(=W³)R²⁰, or -N=C(C₆H₅)₂, and when n is an integer of 2 or more, each Z¹ is the same as or different from each other.
[24] The pyridazinone compound or a salt thereof according to [22], wherein:
   Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, -NR¹⁵R¹⁶, -OR³⁵, -S(O)ᵣ₁R³⁶, or -CN, and when n is an integer of 2 or more, each Z¹ is the same as or different from each other.
[25] The pyridazinone compound or a salt thereof according to [22], wherein:
   Z¹ is a halogen atom, C₁₋₆ alkyl, -OR³⁵, or -S(O)ᵣ₁R³⁶, and when n is an integer of 2 or more, each Z¹ is the same as or different from each other.
[26] The pyridazinone compound or a salt thereof according to [22], wherein:
   Z¹ is a halogen atom, C₁₋₆ alkyl, or -OR³⁵, and when n is an integer of 2 or more, each Z¹ is the same as or different from each other.
[27] The pyridazinone compound or a salt thereof according to any of [1] to [26], wherein:
   G is a hydrogen atom, C₁₋₆ alkyl, (C₁₋₆) alkyl substituted with R⁴, -C(=W⁴)R⁵, or - S(O)₂R⁶.
[28] The pyridazinone compound or a salt thereof according to [27], wherein:
   G is a hydrogen atom, C₁₋₆ alkyl, or -C(=W⁴)R⁵.
[29] The pyridazinone compound or a salt thereof according to [27], wherein:
   G is a hydrogen atom or C₁₋₆ alkyl.
[30] The pyridazinone compound or a salt thereof according to any of [1] to [29], wherein:
   R¹ is C₁₋₆ alkyl, C₃₋₆ alkenyl, C₃₋₆ alkynyl, or C₁₋₆ alkyl substituted with R³⁴.
[31] The pyridazinone compound or a salt thereof according to [30], wherein:
   R¹ is a hydrogen atom, C₁₋₆ alkyl, or C₁₋₆ alkyl substituted with R³⁴.
[32] The pyridazinone compound or a salt thereof according to [30], wherein:
   R¹ is C₁₋₆ alkyl.
[33] The pyridazinone compound or a salt thereof according to any of [1] to [32], wherein:
   R² is a hydrogen atom, a halogen atom, C₁₋₆ alkyl, C₁₋₆ alkoxy, or -NR³⁰R³¹.
[34] The pyridazinone compound or a salt thereof according to [33], wherein:
   R² is a hydrogen atom, a halogen atom, C₁₋₆ alkyl, or C₁₋₆ alkoxy.
[35] The pyridazinone compound or a salt thereof according to [33], wherein:
   R² is C₁₋₆ alkyl or C₁₋₆ alkoxy.
[36] The pyridazinone compound or a salt thereof according to [33], wherein:
   R² is C₁₋₆ alkoxy, C₃₋₆ cycloalkyl, or -NR³⁰R³¹.
[37] The pyridazinone compound or a salt thereof according to [33], wherein:
   R² is C₃₋₆ cycloalkyl or -NR³⁰R³¹.
[38] The pyridazinone compound or a salt thereof according to any of [1] to [37], wherein:
   R³ is D-1, D-2, D-3, D-4, D-5, D-6, D-7, D-8, D-9, D-10, D-13, D-14, D-15, D-16, D-17, D-18, D-19, D-20, D-21, D-22, D-23, D-24, D-25, D-26, D-27, or D-28.
[39] The pyridazinone compound or a salt thereof according to [38], wherein:
   R³ is D-1, D-2, D-3, D-4, D-6, D-7, D-8, D-9, D-10, D-17, D-18, D-19, D-20, D-21, D-22, D-23, D-24, D-25, D-26, or D-28.
[40] The pyridazinone compound or a salt thereof according to [38], wherein:
   R³ is D-1, D-2, D-3, D-4, D-5, D-6, D-7, D-8, D-9, D-10, D-17, D-18, D-20, D-21, D-22, D-23, D-24, or D-28.
[41] The pyridazinone compound or a salt thereof according to [38], wherein:
   R³ is D-1, D-3, D-7, D-20, D-21, D-22, or D-24.
[42] The pyridazinone compound or a salt thereof according to [38], wherein:
   R³ is D-1, D-7, or D-24.
[43] The pyridazinone compound or a salt thereof according to [38], wherein:
   R³ is D-1.
[44] The pyridazinone compound or a salt thereof according to [38], wherein:
   R³ is D-3.
[45] The pyridazinone compound or a salt thereof according to [38], wherein:
   R³ is D-7.
[46] The pyridazinone compound or a salt thereof according to [38], wherein:
   R³ is D-24.
[47] The pyridazinone compound or a salt thereof according to any of [1] to [46], wherein:
   Y¹ is a hydrogen atom, a halogen atom, C₁₋₆ alkyl, C₂₋₆ alkenyl, C₂₋₆ alkynyl, C₃₋₆ cycloalkyl, C₂₋₆ haloalkenyl, C₂₋₆ haloalkynyl, (C₁₋₆) alkyl substituted with R¹⁰, C₃₋₆ cycloalkyl substituted with R⁴⁴, -OR¹¹, -S(O)ᵣ₂R³⁸, -NR⁸R⁹, -CN, -NO₂, -C(O)OH,-C(=W²)R¹³, phenyl, phenyl substituted with (Z⁴)_{p5c}, or tri(C₁₋₆ alkyl)silyl, and when p7, p6, p5, p4, p3, or p2 is an integer of 2 or more, each Y¹ is the same as or different from each other.
[48] The pyridazinone compound or a salt thereof according to [47], wherein:
   Y¹ is a hydrogen atom, a halogen atom, C₁₋₆ alkyl, C₂₋₆ alkenyl, (C₁₋₆) alkyl substituted with R¹⁰, -C(O)OH, -OR¹¹, -S(O)ᵣ₂R³⁸, -NR⁸R⁹, -CN, -NO₂, -C(=W²)R¹³, phenyl, or tri(C₁₋₆ alkyl)silyl, and when p7, p6, p5, p4, p3, or p2 is an integer of 2 or more, each Y¹ is the same as or different from each other.
[49] The pyridazinone compound or a salt thereof according to [47], wherein:
   Y¹ is a halogen atom, C₁₋₆ alkyl, C₂₋₆ alkenyl, (C₁₋₆) alkyl substituted with R¹⁰, -OR¹¹,-S(O)ᵣ₂R³⁸, -NR⁸R⁹, -CN, -NO₂, or -C(=W²)R¹³, and when p7, p6, p5, p4, p3, or p2 is an integer of 2 or more, each Y¹ is the same as or different from each other.
[50] The pyridazinone compound or a salt thereof according to [47], wherein:
   Y¹ is a halogen atom, C₁₋₆ alkyl, (C₁₋₆) alkyl substituted with R¹⁰, -OR¹¹, or -S(O)ᵣ₂R³⁸, and when p7, p6, p5, p4, p3, or p2 is an integer of 2 or more, each Y¹ is the same as or different from each other.
[51] The pyridazinone compound or a salt thereof according to [47], wherein:
   Y¹ is a halogen atom, C₁₋₆ alkyl, (C₁₋₆) alkyl substituted with R¹⁰, or -OR¹¹, and when p7, p6, p5, p4, p3, or p2 is an integer of 2 or more, each Y¹ is the same as or different from each other.
[52] The pyridazinone compound or a salt thereof according to [47], wherein:
   Y¹ is a hydrogen atom, a halogen atom, C₁₋₆ alkyl, (C₁₋₆) alkyl substituted with R¹⁰, - C(O)OH, -CN, -C(=W²)R¹³, phenyl, or tri(C₁₋₆ alkyl)silyl, and when p7, p6, p5, p4, p3, or p2 is an integer of 2 or more, each Y¹ is the same as or different from each other.
[53] The pyridazinone compound or a salt thereof according to [47], wherein:
   Y¹ is a hydrogen atom, C₁₋₆ alkyl, or (C₁₋₆) alkyl substituted with R¹⁰, and when p7, p6, p5, p4, p3, or p2 is an integer of 2 or more, each Y¹ is the same as or different from each other.
[54] The pyridazinone compound or a salt thereof according to [47], wherein:
   Y¹ is a hydrogen atom, a halogen atom, C₁₋₆ alkyl, phenyl, or tri(C₁₋₆ alkyl)silyl, and when p7, p6, p5, p4, p3, or p2 is an integer of 2 or more, each Y¹ is the same as or different from each other.
[55] The pyridazinone compound or a salt thereof according to [47], wherein:
   Y¹ is -OR¹¹ or -S(O)ᵣ₂R³⁸, and when p7, p6, p5, p4, p3, or p2 is an integer of 2 or more, each Y¹ is the same as or different from each other.
[56] The pyridazinone compound or a salt thereof according to [47], wherein:
   Y¹ is a hydrogen atom, a halogen atom, or -OR¹¹, and when p7, p6, p5, p4, p3, or p2 is an integer of 2 or more, each Y¹ is the same as or different from each other.
[57] The pyridazinone compound or a salt thereof according to any of [1] to [56], wherein:
   R⁴ is a halogen atom, -CN, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁴, -OR²⁴, -S(O)ᵣ₄R⁵⁴,-C(O)R²³, phenyl, or phenyl substituted with (Z⁴)_{p5c}.
[58] The pyridazinone compound or a salt thereof according to [57], wherein:
   R⁴ is a halogen atom, -CN, C₂₋₆ alkenyl, C₂₋₆ alkynyl, -OR²⁴, -S(O)ᵣ₄R⁵⁴, -C(O)R²³, or phenyl.
[59] The pyridazinone compound or a salt thereof according to [57], wherein:
   R⁴ is -OR²⁴ or -S(O)ᵣ₄R⁵⁴.
[60] The pyridazinone compound or a salt thereof according to any of [1] to [59], wherein:
   R⁵ is C₁₋₆ alkyl, (C₁₋₆) alkyl substituted with R²⁶, C₂₋₆ alkenyl, C₂₋₆ alkynyl, C₁₋₆ alkoxy, -OR⁵⁸, -SR⁵⁹, C₃₋₆ cycloalkyl, C₃₋₆ cycloalkyl substituted with R⁴⁴, -NR⁵⁶R⁵⁷, U-1, U-6, U-7, U-8, Q-1, Q-2, Q-3, Q-4, Q-5, Q-7, Q-8, Q-9, Q-10, Q-17, Q-18, Q-19, Q-20, phenyl, phenyl substituted with (Z²)ₚ₅ₐ, or -C(O)R⁵³.
[61] The pyridazinone compound or a salt thereof according to [60], wherein:
   R⁵ is C₁₋₆ alkyl, (C₁₋₆) alkyl substituted with R²⁶, C₂₋₆ alkenyl, C₂₋₆ alkynyl, C₁₋₆ alkoxy, -OR⁵⁸, -SR⁵⁹, C₃₋₆ cycloalkyl, -NR⁵⁶R⁵⁷, U-1, U-6, Q-2, Q-4, phenyl, phenyl substituted with (Z²)ₚ₅ₐ, or -C(O)R⁵³.
[62] The pyridazinone compound or a salt thereof according to [60], wherein:
   R⁵ is C₁₋₆ alkyl, (C₁₋₆) alkyl substituted with R²⁶, or C₁₋₆ alkoxy.
[63] The pyridazinone compound or a salt thereof according to [60], wherein:
   R⁵ is C₁₋₆ alkyl.
[64] The pyridazinone compound or a salt thereof according to any of [1] to [63], wherein:
   R⁶ is C₁₋₆ alkyl, C₁₋₆ haloalkyl, phenyl, phenyl substituted with (Z²)ₚ₅ₐ, or -NR²⁸R²⁹.
[65] The pyridazinone compound or a salt thereof according to [64], wherein:
   R⁶ is C₁₋₆ alkyl, phenyl substituted with (Z²)ₚ₅ₐ, or -NR²⁸R²⁹.
[66] The pyridazinone compound or a salt thereof according to [64], wherein:
   R⁶ is C₁₋₆ alkyl or -NR²⁸R²⁹.
[67] The pyridazinone compound or a salt thereof according to any of [1] to [66], wherein:
   R⁷ is a hydrogen atom, C₁₋₆ alkyl, C₃₋₆ alkenyl, C₃₋₆ alkynyl, C₁₋₆ haloalkyl, C₁₋₆ alkoxy (C₁₋₂) alkyl, or C₁₋₆ alkylthio (C₁₋₂) alkyl.
[68] The pyridazinone compound or a salt thereof according to [67], wherein:
   R⁷ is a hydrogen atom or C₁₋₆ alkyl.
[69] The pyridazinone compound or a salt thereof according to any of [1] to [68], wherein:
   R¹⁰ is a halogen atom, -OR⁴⁰, -S(O)ᵣ₃R⁴¹, C₃₋₆ cycloalkyl, or (C₃₋₆) cycloalkyl substituted with R⁴⁴.
[70] The pyridazinone compound or a salt thereof according to [69], wherein:
   R¹⁰ is a halogen atom, -OR⁴⁰, or -S(O)ᵣ₃R⁴¹.
[71] The pyridazinone compound or a salt thereof according to [69], wherein:
   R¹⁰ is a halogen atom or -OR⁴⁰.
[72] The pyridazinone compound or a salt thereof according to any of [1] to [71], wherein:
   R¹¹ is a hydrogen atom, C₁₋₆ alkyl, (C₁₋₆) alkyl substituted with R¹², C₃₋₆ cycloalkyl, (C₃₋₆) cycloalkyl substituted with R⁴⁴, phenyl, phenyl substituted with (Z⁴)_{p5c}, C₁₋₆ alkylsulfonyl, C₁₋₆ haloalkylsulfonyl, U-2, U-4, Q-17, Q-18, or Q-19.
[73] The pyridazinone compound or a salt thereof according to [72], wherein:
   R¹¹ is a hydrogen atom, C₁₋₆ alkyl, (C₁₋₆) alkyl substituted with R¹², C₃₋₆ cycloalkyl, phenyl, C₁₋₆ alkylsulfonyl, C₁₋₆ haloalkylsulfonyl, U-2, U-4, or Q-17.
[74] The pyridazinone compound or a salt thereof according to [72], wherein:
   R¹¹ is a hydrogen atom, C₁₋₆ alkyl, (C₁₋₆) alkyl substituted with R¹², C₃₋₆ cycloalkyl, phenyl, C₁₋₆ alkylsulfonyl, C₁₋₆ haloalkylsulfonyl, U-2, or U-4.
[75] The pyridazinone compound or a salt thereof according to [72], wherein:
   R¹¹ is C₁₋₆ alkyl or (C₁₋₆) alkyl substituted with R¹².
[76] The pyridazinone compound or a salt thereof according to any of [1] to [75], wherein:
   R¹² is a halogen atom, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁵, -OR⁴⁸, -S(O)ᵣ₅R⁴⁹, -CN, phenyl, phenyl substituted with (Z³)_{p5b}, -C(=W²)R¹³, U-1, U-2, U-3, Q-1, Q-2, Q-3, Q-4, Q-5, Q-18, or - ON=CR⁴²R⁴³.
[77] The pyridazinone compound or a salt thereof according to [76], wherein:
   R¹² is a halogen atom, C₂₋₆ alkenyl, C₂₋₆ haloalkenyl, C₂₋₆ alkynyl, C₃₋₆ cycloalkyl, (C₃₋₆) cycloalkyl substituted with R⁴⁵, -OR⁴⁸, -S(O)ᵣ₅R⁴⁹, -CN, phenyl, -C(=W²)R¹³, U-3, Q-1, or -ON=CR⁴²R⁴³.
[78] The pyridazinone compound or a salt thereof according to [76], wherein:
   R¹² is a halogen atom, C₂₋₆ alkenyl, -OR⁴⁸, -S(O)ᵣ₅R⁴⁹, or -C(=W²)R¹³.
[79] The pyridazinone compound or a salt thereof according to [76], wherein:
   R¹² is a halogen atom, C₂₋₆ alkenyl, -OR⁴⁸, or -S(O)ᵣ₅R⁴⁹.
[80] The pyridazinone compound or a salt thereof according to any of [1] to [79], wherein:
   R¹³ is C₁₋₆ alkyl, C₁₋₆ alkoxy, or C₁₋₆ haloalkylamino.
[81] The pyridazinone compound or a salt thereof according to [80], wherein:
   R¹³ is a hydrogen atom or C₁₋₆ alkyl.
[82] The pyridazinone compound or a salt thereof according to any of [1] to [81], wherein:
   R¹⁴ is a halogen atom, C₂₋₆ alkenyl, C₂₋₆ alkynyl, C₃₋₆ cycloalkyl, C₂₋₆ haloalkenyl, C₂₋₆ haloalkynyl, (C₃₋₆) cycloalkyl substituted with R⁴⁵, C₁₋₆ alkoxy, C₁₋₆ alkylthio, C₁₋₆ alkylsulfinyl, C₁₋₆ alkylsulfonyl, phenyl, phenyl substituted with (Z⁴)_{p5c}, -CN, U-1, U-2, U-3, U-9, Q-17, Q-18, Q-19, or Q-20.
[83] The pyridazinone compound or a salt thereof according to [82], wherein:
   R¹⁴ is a halogen atom, C₂₋₆ alkenyl, C₃₋₆ cycloalkyl, (C₃₋₆) cycloalkyl substituted with R⁴⁵, C₁₋₆ alkoxy, C₁₋₆ alkylthio, C₁₋₆ alkylsulfonyl, phenyl, or U-1.
[84] The pyridazinone compound or a salt thereof according to [82], wherein:
   R¹⁴ is a halogen atom, C₂₋₆ alkenyl, C₁₋₆ alkoxy, or C₁₋₆ alkylthio.
[85] The pyridazinone compound or a salt thereof according to any of [1] to [84], wherein:
   R¹⁵ and R¹⁶ are each independently a hydrogen atom, C₁₋₆ alkyl, -C(O)R¹⁷, or - S(O)₂R¹⁸.
[86] The pyridazinone compound or a salt thereof according to [85], wherein:
   R¹⁵ and R¹⁶ are each independently a hydrogen atom or C₁₋₆ alkyl.
[87] The pyridazinone compound or a salt thereof according to any of [1] to [86], wherein:
   R¹⁷ is C₁₋₆ alkyl, C₁₋₆ alkoxy, or C₁₋₆ alkoxy (C₁₋₂) alkyl.
[88] The pyridazinone compound or a salt thereof according to any of [1] to [87], wherein:
   R²² is a halogen atom, -OH, C₁₋₆ alkoxy, C₁₋₆ haloalkoxy, C₁₋₆ alkylthio, C₁₋₆ haloalkylthio, or -CN.
[89] The pyridazinone compound or a salt thereof according to [88], wherein:
   R²² is a halogen atom, -OH, or C₁₋₆ alkoxy.
[90] The pyridazinone compound or a salt thereof according to any of [1] to [89], wherein:
   R²³ is C₁₋₆ alkyl, C₁₋₆ alkoxy, di(C₁₋₆) alkylamino, phenyl, phenyl substituted with (Z⁴ )p_{5c}, U-7, or U-8.
[91] The pyridazinone compound or a salt thereof according to [90], wherein:
   R²³ is C₁₋₆ alkyl, C₁₋₆ alkoxy, or phenyl.
[92] The pyridazinone compound or a salt thereof according to any of [1] to [91], wherein:
   R²⁴ is C₁₋₆ alkyl, C₁₋₆ haloalkyl, C₃₋₆ alkenyl, C₃₋₆ alkynyl, (C₁₋₆) alkoxy (C₁₋₂) alkyl, - C(O)R²⁵, or -S(O)₂R³³.
[93] The pyridazinone compound or a salt thereof according to [92], wherein:
   R²⁴ is C₁₋₆ alkyl, (C₁₋₆) alkoxy (C₁₋₂) alkyl, or -C(O)R²⁵.
[94] The pyridazinone compound or a salt thereof according to [92], wherein:
   R²⁴ is C₁₋₆ alkyl.
[95] The pyridazinone compound or a salt thereof according to any of [1] to [94], wherein:
   R²⁵ is C₁₋₆ alkyl, C₁₋₆ alkoxy, or di(C₁₋₆) alkylamino.
[96] The pyridazinone compound or a salt thereof according to any of [1] to [95], wherein:
   R²⁶ is a halogen atom, C₁₋₆ alkoxy, C₁₋₆ haloalkoxy, C₁₋₆ alkylthio, C₁₋₆ alkylsulfinyl, C₁₋₆ alkylsulfonyl, -OR³², phenyl, or phenyl substituted with (Z⁴)_{p5c}.
[97] The pyridazinone compound or a salt thereof according to [96], wherein:
   R²⁶ is C₁₋₆ alkoxy, -OR³², or phenyl.
[98] The pyridazinone compound or a salt thereof according to [96], wherein:
   R²⁶ is C₁₋₆ alkoxy.
[99] The pyridazinone compound or a salt thereof according to any of [1] to [98], wherein:
   R²⁷ is a halogen atom, C₂₋₆ alkenyl, or C₁₋₆ alkoxy.
[100] The pyridazinone compound or a salt thereof according to any of [1] to [99], wherein:
   R³⁴ is C₁₋₆ alkoxy, phenyl, or -CN.
[101] The pyridazinone compound or a salt thereof according to [100], wherein:
   R³⁴ is C₁₋₆ alkoxy or -CN.
[102] The pyridazinone compound or a salt thereof according to any of [1] to [101], wherein:
   R³⁵ is a hydrogen atom, C₁₋₆ alkyl, C₃₋₆ cycloalkyl, (C₁₋₆) alkyl substituted with R¹⁴, or - S(O)₂R³³.
[103] The pyridazinone compound or a salt thereof according to [102], wherein:
   R³⁵ is C₁₋₆ alkyl or (C₁₋₆) alkyl substituted with R¹⁴.
[104] The pyridazinone compound or a salt thereof according to [102], wherein:
   R³⁵ is C₁₋₆ alkyl.
[105]
   The pyridazinone compound or a salt thereof according to any of [1] to [104], wherein:
   R³⁶ is C₁₋₆ alkyl, (C₁₋₆) alkyl substituted with R³⁷, or C₃₋₆ cycloalkyl.
[106] The pyridazinone compound or a salt thereof according to [105], wherein:
   R³⁶ is C₁₋₆ alkyl or (C₁₋₆) alkyl substituted with R³⁷.
[107]
   The pyridazinone compound or a salt thereof according to any of [1] to [106], wherein:
   R³⁷ is a halogen atom, C₂₋₆ alkenyl, C₂₋₆ alkynyl, C₃₋₆ cycloalkyl, C₁₋₆ alkoxy, C₁₋₆ alkylthio, or C₁₋₁₀ alkoxycarbonyl.
[108] The pyridazinone compound or a salt thereof according to [107], wherein:
   R³⁷ is a halogen atom or (C₁₋₁₀) alkoxycarbonyl.
[109] The pyridazinone compound or a salt thereof according to [107], wherein:
   R³⁷ is a halogen atom.
[110] The pyridazinone compound or a salt thereof according to any of [1] to [109], wherein:
   R³⁸ is C₁₋₆ alkyl, C₃₋₆ alkenyl, C₃₋₆ alkynyl, C₃₋₆ cycloalkyl, C₃₋₆ haloalkenyl, C₃₋₆ haloalkynyl, (C₁₋₆) alkyl substituted with R³⁹, or (C₃₋₆) cycloalkyl substituted with R⁴⁴.
[111] The pyridazinone compound or a salt thereof according to [110], wherein:
   R³⁸ is C₁₋₆ alkyl, C₃₋₆ alkenyl, C₃₋₆ alkynyl, or (C₁₋₆) alkyl substituted with R³⁹.
[112] The pyridazinone compound or a salt thereof according to [110], wherein:
   R³⁸ is C₁₋₆ alkyl.
[113] The pyridazinone compound or a salt thereof according to any of [1] to [112], wherein:
   R³⁹ is a halogen atom, C₃₋₆ cycloalkyl, -OR⁵¹, -S(O)ᵣ₆R⁵², -C(=W²)R¹³, -CN, phenyl, phenyl substituted with (Z³)_{p5b}, U-1, U-3, U-9, Q-1, or Q-18.
[114] The pyridazinone compound or a salt thereof according to [113], wherein:
   R³⁹ is a halogen atom, -OR⁵¹, -S(O)ᵣ₆R⁵², -C(=W²)R¹³, or -CN.
[115] The pyridazinone compound or a salt thereof according to [113], wherein:
   R³⁹ is -OR⁵¹ or -S(O)ᵣ₆R⁵².
[116] The pyridazinone compound or a salt thereof according to any of [1] to [115], wherein:
   R⁴⁰ is a hydrogen atom, C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, (C₁₋₆) alkyl substituted with R⁴⁶, or U-4.
[117] The pyridazinone compound or a salt thereof according to [116], wherein:
   R⁴⁰ is a hydrogen atom, C₁₋₆ alkyl, C₃₋₆ alkenyl, C₁₋₆ haloalkyl, or (C₁₋₆) alkyl substituted with R⁴⁶.
[118] The pyridazinone compound or a salt thereof according to [116], wherein:
   R⁴⁰ is C₁₋₆ alkyl, C₃₋₆ alkenyl, or C₃₋₆ alkynyl.
[119] The pyridazinone compound or a salt thereof according to [116], wherein:
   R⁴⁰ is C₁₋₆ alkyl.
[120] The pyridazinone compound or a salt thereof according to any of [1] to [119], wherein:
   R⁴¹ is C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, or (C₁₋₆) alkyl substituted with R⁴⁷.
[121] The pyridazinone compound or a salt thereof according to [120], wherein:
   R⁴¹ is a hydrogen atom, C₁₋₆ alkyl, C₃₋₆ alkenyl, C₃₋₆ alkynyl, C₁₋₆ haloalkyl, or (C₁₋₆) alkyl substituted with R⁴⁷.
[122] The pyridazinone compound or a salt thereof according to [120], wherein:
   R⁴¹ is C₁₋₆ alkyl, C₃₋₆ alkenyl, C₃₋₆ alkynyl, or (C₁₋₆) alkyl substituted with R⁴⁷.
[123] The pyridazinone compound or a salt thereof according to any of [1] to [122], wherein:
   R⁴⁸ is a hydrogen atom, C₁₋₆ alkyl, C₁₋₆ haloalkyl, C₃₋₆ alkenyl, C₃₋₆ haloalkenyl, C₃₋₆ alkynyl, C₃₋₆ haloalkynyl, or C₃₋₆ cycloalkyl.
[124] The pyridazinone compound or a salt thereof according to [123], wherein:
   R⁴⁸ is C₁₋₆ alkyl or C₁₋₆ haloalkyl.
[125] The pyridazinone compound or a salt thereof according to [123], wherein:
   R⁴⁸ is C₁₋₆ alkyl.
[126] The pyridazinone compound or a salt thereof according to any of [1] to [125], wherein:
   R⁴⁹ is C₁₋₆ alkyl, C₁₋₆ haloalkyl, C₃₋₆ alkenyl, or C₃₋₆ alkynyl.
[127] The pyridazinone compound or a salt thereof according to [126], wherein:
   R⁴⁹ is C₁₋₆ alkyl.
[128] The pyridazinone compound or a salt thereof according to any of [1] to [127], wherein:
   Y² is a halogen atom, C₁₋₆ alkyl, C₁₋₆ haloalkyl, C₁₋₆ alkoxy, C₁₋₆ haloalkoxy, C₁₋₆ alkylthio, or C₁₋₆ haloalkylthio.
[129] The pyridazinone compound or a salt thereof according to [128], wherein:
   Y² is a halogen atom or C₁₋₆ haloalkyl.
[130] The pyridazinone compound or a salt thereof according to any of [1] to [129], wherein:
   Z² is a halogen atom, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, - OH, -NH₂, -CN, -NO₂, or -C(O)R⁵⁵, and when p5a is an integer of 2 or more, each Z² is the same as or different from each other.
[131] The pyridazinone compound or a salt thereof according to [130], wherein:
   Z² is a halogen atom, C₁₋₆ alkyl, or C₁₋₆ alkoxy, and when p5a is an integer of 2 or more, each Z² is the same as or different from each other.
[132] The pyridazinone compound or a salt thereof according to any of [1] to [131], wherein:
   Z⁴ is a halogen atom, C₁₋₆ alkyl, or C₁₋₆ alkoxy, and when p5c is an integer of 2 or more, each Z³ is the same as or different from each other.
[133] The pyridazinone compound or a salt thereof according to [132], wherein:
   Z⁴ is a halogen atom or C₁₋₆ alkoxy, and when p5c is an integer of 2 or more, each Z³ is the same as or different from each other.
[134] The pyridazinone compound or a salt thereof according to any of [1] to [133], wherein:
   r1 is an integer of 0 or 2;
   r2 is an integer of 0, 1, or 2;
   r3 is an integer of 0, 1, or 2;
   r5 is an integer of 0, 1, or 2; and
   r6 is 0.
[135] The pyridazinone compound or a salt thereof according to any of [1] to [134], wherein:
   n is an integer of 0, 1, or 2.
[136] The pyridazinone compound or a salt thereof according to any of [1] to [135], wherein:
   p2 is an integer of 0 or 1;
   p3 is an integer of 0, 1, 2, or 3;
   p4 is an integer of 0, 1, or 2;
   p5 is an integer of 0, 1, 2, 3, or 4;
   p6 is an integer of 0, 1, or 2; and
   p7 is an integer of 0, 1, or 2.
[137] The pyridazinone compound or a salt thereof according to [136], wherein:
   p2 is an integer of 1;
   p3 is an integer of 0, 1, or 2;
   p4 is an integer of 1;
   p5 is an integer of 0, 1, 2, or 3;
   p6 is an integer of 0; and
   p7 is an integer of 0.
[138] The number of substituents (corresponding to Examples + α) The pyridazinone compound or a salt thereof according to any of [1] to [137], wherein:
   p5a is an integer of 1;
   p5b is an integer of 1 or 2; and
   p5c is an integer of 1 or 2.
[139] The pyridazinone compound or a salt thereof according to [138], wherein:
   p5a is an integer of 1; and
   p5c is an integer of 1 or 2.
[140] The pyridazinone compound or a salt thereof according to any of [1] to [139], wherein:
   q1 is an integer of 0 or 1;
   q2 is an integer of 0 or 1;
   q3 is an integer of 0 or 1; and
   q4 is an integer of 0 or 2.
[141] The pyridazinone compound or a salt thereof according to [140], wherein:
   q2 is an integer of 1;
   q3 is 0; and
   q4 is an integer of 2.
[142] A production intermediate of the pyridazinone compound or a salt thereof according to any of [1] to [141], the production intermediate being of the following formula A: 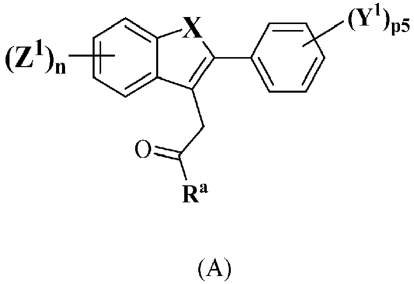 [wherein X is an oxygen atom or a sulfur atom;
   Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, phenyl, phenyl substituted with (Z⁴)_{p5c}, Q-3, -NR¹⁵R¹⁶, -OR³⁵, -S(O)ᵣ₁R³⁶, -CN, -C(=W³)R²⁰, or -N=C(C₆H₅)₂, and when n is an integer of 2 or more, each Z¹ is the same as or different fromeach other;
   Y¹ is a hydrogen atom, a halogen atom, C₁₋₆ alkyl, C₂₋₆ alkenyl, (C₁₋₆) alkyl substituted with R¹⁰, -C(O)OH, -OR¹¹, -S(O)ᵣ₂R³⁸, -NR⁸R⁹, -CN, -NO₂, -C(=W²)R¹³, phenyl, or tri(C₁₋₆ alkyl)silyl, and when p5 is an integer of 2 or more, each Y¹ is the same as or different from each other;
   R⁷ is a hydrogen atom or C₁₋₆ alkyl;
   R⁸ and R⁹ are each independently C₁₋₆ alkyl;
   R¹⁰ is a halogen atom, -OR⁴⁰, or -S(O)ᵣ₃R⁴¹;
   R¹¹ is a hydrogen atom, C₁₋₆ alkyl, (C₁₋₆) alkyl substituted with R¹², C₃₋₆ cycloalkyl, phenyl, C₁₋₆ alkylsulfonyl, C₁₋₆ haloalkylsulfonyl, U-2, U-4, or Q-17;
   R¹² is a halogen atom, C₂₋₆ alkenyl, C₂₋₆ haloalkenyl, C₂₋₆ alkynyl, C₃₋₆ cycloalkyl, (C₃₋₆) cycloalkyl substituted with R⁴⁵, -OR⁴⁸, -S(O)ᵣ₅R⁴⁹, -CN, phenyl, -C(=W²)R¹³, U-3, Q-1, or -ON=CR⁴²R⁴³;
   R¹³ is C₁₋₆ alkyl, C₁₋₆ alkoxy, or C₁₋₆ haloalkylamino;
   R¹⁴ is a halogen atom, C₂₋₆ alkenyl, C₃₋₆ cycloalkyl, (C₃₋₆) cycloalkyl substituted with R⁴⁵, C₁₋₆ alkoxy, C₁₋₆ alkylthio, C₁₋₆ alkylsulfonyl, phenyl, or U-1;
   R¹⁵ and R¹⁶ are each independently a hydrogen atom, C₁₋₆ alkyl, -C(O)R¹⁷, or - S(O)₂R¹⁸;
   R¹⁷ is C₁₋₆ alkyl, C₁₋₆ alkoxy, or C₁₋₆ alkoxy (C₁₋₂) alkyl;
   R¹⁸ is C₁₋₆ alkyl;
   R¹⁹ is tri(C₁₋₆ alkyl)silyl;
   R²⁰ is a hydrogen atom or C₁₋₆ alkyl;
   R²¹ is a hydrogen atom or C₁₋₆ alkyl;
   R²² is a halogen atom, -OH, or C₁₋₆ alkoxy;
   R³³ is C₁₋₆ haloalkyl or di(C₁₋₆) alkylamino;
   R³⁵ is a hydrogen atom, C₁₋₆ alkyl, C₃₋₆ cycloalkyl, (C₁₋₆) alkyl substituted with R¹⁴, or - S(O)₂R³³;
   R³⁶ is C₁₋₆ alkyl or (C₁₋₆) alkyl substituted with R³⁷;
   R³⁷ is a halogen atom or (C₁₋₁₀) alkoxycarbonyl;
   R³⁸ is C₁₋₆ alkyl, C₃₋₆ alkenyl, C₃₋₆ alkynyl, C₃₋₆ cycloalkyl, C₃₋₆ haloalkenyl, C₃₋₆ haloalkynyl, or (C₁₋₆) alkyl substituted with R³⁹;
   R³⁹ is a halogen atom, -OR⁵¹, -S(O)ᵣ₆R⁵², -C(=W²)R¹³, or -CN;
   R⁴⁰ is a hydrogen atom, C₁₋₆ alkyl, C₃₋₆ alkenyl, C₁₋₆ haloalkyl, or (C₁₋₆) alkyl substituted with R⁴⁶;
   R⁴¹ is a hydrogen atom, C₁₋₆ alkyl, C₃₋₆ alkenyl, C₃₋₆ alkynyl, C₁₋₆ haloalkyl, or (C₁₋₆) alkyl substituted with R⁴⁷;
   R⁴² and R⁴³ are each independently C₁₋₆ alkyl;
   R⁴⁴ is a halogen atom, C₁₋₆ alkyl, or -CN;
   R⁴⁵ is a halogen atom;
   R⁴⁶ is C₁₋₆ alkoxy or C₁₋₆ alkylthio;
   R⁴⁷ is C₁₋₆ alkoxy or C₁₋₆ alkylthio;
   R⁴⁸ is C₁₋₆ alkyl or C₁₋₆ haloalkyl;
   R⁴⁹ is C₁₋₆ alkyl;
   R⁵¹ is C₁₋₆ alkyl;
   R⁵² is C₁₋₆ alkyl;
   R^{a} is -OH, C₁₋₆ alkoxy, or -NR^{a1}R^{a2};
   R^{a1} is a hydrogen atom or C₁₋₆ alkyl;
   R^{a2} is a hydrogen atom, -NH₂, or -N=C(R^{a3})R^{a4};
   R^{a3} is a hydrogen atom or C₁₋₆ alkyl;
   R^{a4} is C₁₋₆ alkyl or C₁₋₆ alkoxycarbonyl;
   Y² is a halogen atom or C₁₋₆ haloalkyl;
   W² is an oxygen atom or N-OR⁷;
   W³ is an oxygen atom or N-OR²¹;
   Z⁴ is a halogen atom or C₁₋₆ alkoxy, and when p5c is an integer of 2 or more, each Z⁴ is the same as or different from each other;
   n is an integer of 0, 1, or 2;
   p5 is an integer of 0, 1, 2, 3, 4, or 5;
   q3 is 0; and
   p5c is an integer of 1 or 2].
[143] The production intermediate of the pyridazinone compound or a salt thereof according to [142], wherein:
   Z¹ is a halogen atom, C₁₋₆ alkyl, or -OR³⁵, and when n is an integer of 2 or more, each Z¹ is the same as or different from each other;
   Y¹ is a hydrogen atom, a halogen atom, or -OR¹¹, and when p5 is an integer of 2 or more, each Y¹ is the same as or different from each other;
   R¹¹ is C₁₋₆ alkyl or (C₁₋₆) alkyl substituted with R¹²;
   R¹² is a halogen atom;
   R¹⁴ is phenyl;
   R³⁵ is C₁₋₆ alkyl or (C₁₋₆) alkyl substituted with R¹⁴;
   R^{a} is -OH, C₁₋₆ alkoxy, or -NR^{a1}R^{a2};
   R^{a1} is C₁₋₆ alkyl;
   R^{a2} is -NH₂ or -N=C(R^{a3})R^{a4};
   R^{a3} is C₁₋₆ alkyl;
   R^{a4} is C₁₋₆ alkoxycarbonyl;
   p5 is an integer of 0, 1, or 2; and
   n is an integer of 0, 1, or 2.
[144] The production intermediate of the pyridazinone compound or a salt thereof according to [143], wherein:
   X is a sulfur atom; and
   R^{a} is -OH or C₁₋₆ alkoxy.
[145] The production intermediate of the pyridazinone compound or a salt thereof according to [143], wherein:
   X is a sulfur atom;
   R³⁵ is C₁₋₆ alkyl; and
   R^{a} is -NR^{a1}R^{a2}.
[146] The production intermediate of the pyridazinone compound or a salt thereof according to [143], wherein:
   X is an oxygen atom;
   Z¹ is -OR³⁵;
   Y¹ is -OR¹¹;
   R¹¹ is C₁₋₆ alkyl;
   R³⁵ is C₁₋₆ alkyl;
   R^{a} is -OH;
   p5 is an integer of 1; and
   n is an integer of 1.
[147] A pyridazinone compound of the following Formula (1) or a salt thereof: 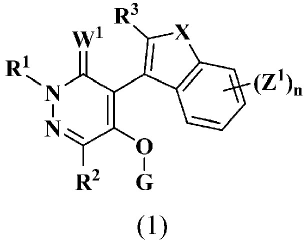 [wherein W¹ is an oxygen atom or a sulfur atom;
   X is an oxygen atom or a sulfur atom;
   Z¹ is a halogen atom, C₁₋₆ alkyl, C₁₋₆ haloalkyl, C₁₋₆ alkoxy, C₁₋₆ haloalkoxy, -OH, - NH₂, -CN, -NO₂, or -CO₂H, and when n is an integer of 2 or more, each Z¹ is the same as or different from each other;
   G is a hydrogen atom, C₁₋₆ alkyl, (C₁₋₆) alkyl substituted with R⁴, -C(O)R⁵, or -S(O)₂R⁶;
   R¹ is C₁₋₆ alkyl, C₃₋₆ alkenyl, or C₃₋₆ alkynyl;
   R² is a hydrogen atom, a halogen atom, C₁₋₆ alkyl, or C₁₋₆ alkoxy;
   R³ is D-1, D-2, D-3, D-4, D-5, D-6, D-7, D-8, D-9, D-10, D-17, D-20a, D-22a, D-24a, D-25, D-23, D-21a, orD-19a;
   D-1, D-2, D-3, D-4, D-5, D-6, D-7, D-8, D-9, D-10, D-17, D-20a, D-22a, D-24a, D-25, D-23, D-21a, and D-19a are respectively the following structures: 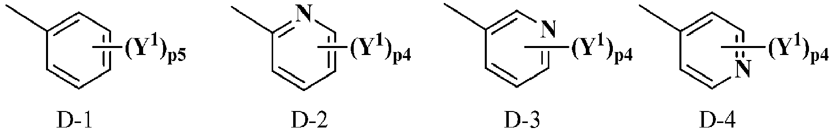 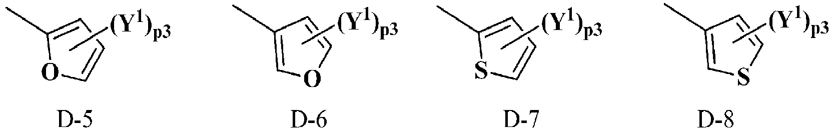 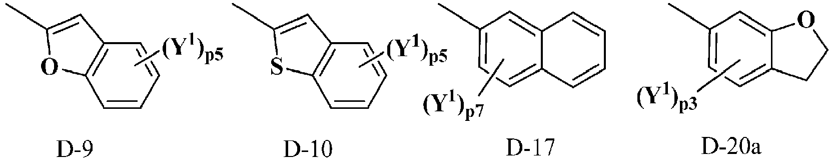 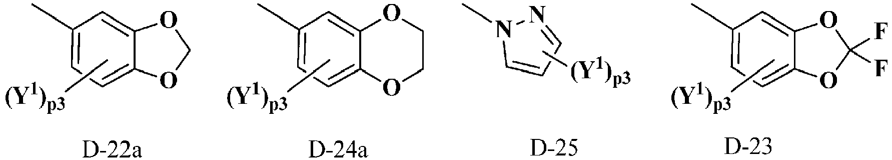 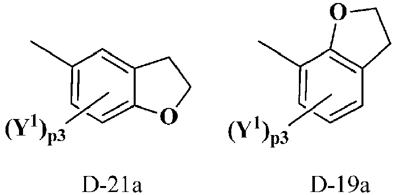
   Y¹ is substituted on the aromatic ring of each of D-1 to D-25;
   Y¹ is a halogen atom, C₁₋₆ alkyl, C₁₋₆ haloalkyl, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, -NR⁸R⁹, -CN, -NO₂, -CO₂H, -C(=W²)R¹³, phenyl, (C₁₋₆) alkyl substituted with R¹⁰, or -OR¹¹, and when p7, p5, p4, or p3 is an integer of 2 or more, each Y¹ is the same as or different from each other;
   W² is an oxygen atom or N-OR⁷;
   R⁴ is phenyl;
   R⁵ is C₁₋₆ alkyl, C₁₋₆ alkoxy, C₁₋₆ alkylthio, phenyl, or phenyl substituted with (Z²)ₚ₅ₐ;
   R⁶ is C₁₋₆ alkyl, C₁₋₆ haloalkyl, phenyl, or phenyl substituted with (Z²)ₚ₅ₐ;
   R⁷ is a hydrogen atom or C₁₋₆ alkyl;
   R⁸ and R⁹ are each independently a hydrogen atom or C₁₋₆ alkyl;
   R¹⁰ is C₁₋₆ alkoxy, C₁₋₆ alkylthio, C₁₋₆ alkylsulfinyl, C₁₋₆ alkylsulfonyl, or -CN;
   R¹¹ is a hydrogen atom, C₁₋₆ alkyl, (C₁₋₆) alkyl substituted with R¹², phenyl, tri(C₁₋₄ alkyl)silyl, C₁₋₆ alkylcarbonyl, or C₁₋₆ alkylsulfonyl;
   R¹² is a halogen atom, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₃₋₆ cycloalkyl, C₃₋₆ halocycloalkyl, C₁₋₆ alkylthio, C₁₋₆ alkylsulfinyl, C₁₋₆ alkylsulfonyl, -CN, phenyl, phenyl substituted with (Z³)_{p5b}, -C(=W²)R¹³, U-1a, U-2a, U-3a, or U-4a;
   U-1a, U-2a, U-3a, and U-4a are respectively the following structures: 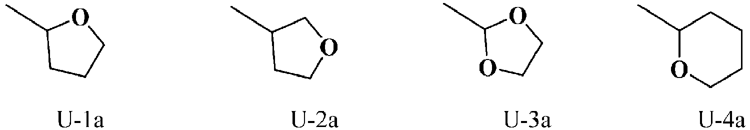
   R¹³ is a hydrogen atom or C₁₋₆ alkyl;
   Z² is a halogen atom, C₁₋₆ alkyl, or C₁₋₆ alkoxy, and when p5a is an integer of 2 or more, each Z² is the same as or different from each other;
   Z³ is a halogen atom, C₁₋₆ alkyl, C₁₋₆ haloalkyl, C₁₋₆ haloalkoxy, C₁₋₆ alkylthio, C₁₋₆ haloalkylthio, -CN, or -NO₂, and when p5b is an integer of 2 or more, each Z³ is the same as or different fromeach other;
   n is an integer of 0, 1, 2, 3, or 4;
   p3 is an integer of 0, 1, 2, or 3;
   p4 is an integer of 0, 1, 2, 3, or 4;
   p5 is an integer of 0, 1, 2, 3, 4, or 5;
   p7 is an integer of 0, 1, 2, 3, 4, 5, 6, or 7;
   p5a is an integer of 1, 2, 3, 4, or 5; and
   p5b is an integer of 1, 2, 3, 4, or 5].
[148] The pyridazinone compound or a salt thereof according to [147], wherein:
   W¹ is an oxygen atom;
   Z¹ is a halogen atom, C₁₋₆ alkyl, or C₁₋₆ alkoxy, and when n is an integer of 2 or more, each Z¹ is the same as or different from each other;
   G is a hydrogen atom, C₁₋₆ alkyl, -C(O)R⁵, or -S(O)₂R⁶;
   R¹ is C₁₋₆ alkyl;
   R² is a hydrogen atom, C₁₋₆ alkyl, or C₁₋₆ alkoxy;
   R³ is D-1, D-3, D-6, D-8, D-9, D-10, D-17, D-20a, D-22a, D-24a, D-25, D-23, D-21a, or D-19a;
   Y¹ is a halogen atom, C₁₋₆ alkyl, C₁₋₆ haloalkyl, C₁₋₆ alkylthio, C₁₋₆ alkylsulfonyl, - NR⁸R⁹, -CN, -NO₂, -C(=W²)R¹³, phenyl, or -OR¹¹, and when p7, p5, p4, or p3 is an integer of 2 or more, each Y¹ is the same as or different from each other;
   R⁵ is C₁₋₆ alkyl, C₁₋₆ alkoxy, C₁₋₆ alkylthio, or phenyl substituted with (Z²)ₚ₅ₐ;
   R⁶ is C₁₋₆ alkyl;
   R⁸ and R⁹ are each independently C₁₋₆ alkyl;
   R¹¹ is a hydrogen atom, C₁₋₆ alkyl, (C₁₋₆) alkyl substituted with R¹², or phenyl;
   R¹² is a halogen atom;
   R¹³ is C₁₋₆ alkyl;
   Z² is C₁₋₆ alkyl;
   n is an integer of 0, 1, or 2;
   p3 is an integer of 0 or 1;
   p4 is an integer of 0, 1, or 2;
   p5 is an integer of 0, 1, 2, or 3;
   p7 is an integer of 0; and
   p5a is an integer of 1.
[149] A pyridazinone compound of the following Formula (1) or a salt thereof: 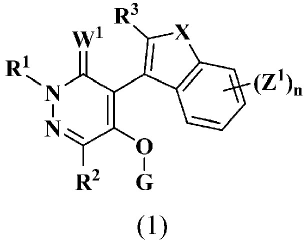 [wherein W¹ is an oxygen atom or a sulfur atom;
   X is an oxygen atom or a sulfur atom;
   Z¹ is a halogen atom, C₁₋₆ alkyl, (C₁₋₆) alkyl substituted with R²², C₂₋₆ alkenyl, C₂₋₆ alkynyl, (C₂₋₆) alkynyl substituted with R¹⁹, C₃₋₆ cycloalkyl, C₁₋₆ alkoxy, (C₁₋₆) alkoxy substituted with R¹⁴, phenyl, phenyl substituted with (Z⁴)_{p5c}, Q-3a, -OH, -NR¹⁵R¹⁶, -OR³⁵, - CN, -NO₂, -CO₂H, or -C(=W³)R²⁰, and when n is an integer of 2 or more, each Z¹ is the same as or different from each other;
   G is a hydrogen atom, C₁₋₆ alkyl, (C₁₋₆) alkyl substituted with R⁴, -C(O)R⁵, or -S(O)₂R⁶;
   R¹ is C₁₋₆ alkyl, C₃₋₆ alkenyl, or C₃₋₆ alkynyl;
   R² is a hydrogen atom, a halogen atom, C₁₋₆ alkyl, C₁₋₆ alkoxy, or -NR³⁰R³¹;
   R³ is D-1, D-2, D-3, D-4, D-5, D-6, D-7, D-8, D-9, D-10, D-17, D-20a, D-22a, D-24a, D-25, D-23, D-21a, D-19a, D-26, or D-18;
   D-1, D-2, D-3, D-4, D-5, D-6, D-7, D-8, D-9, D-10, D-17, D-20a, D-22a, D-24a, D-25, D-23, D-21a, D-19a, D-26, and D-18 are respectively the following structures: 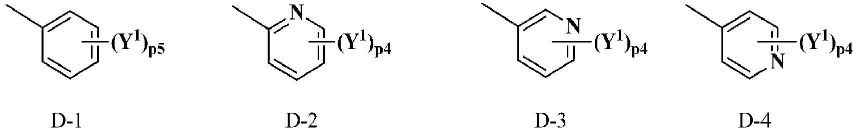 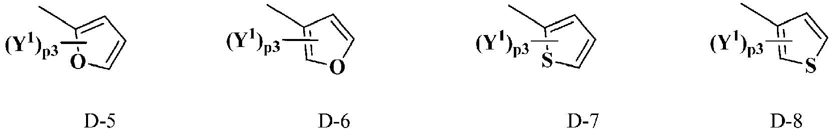 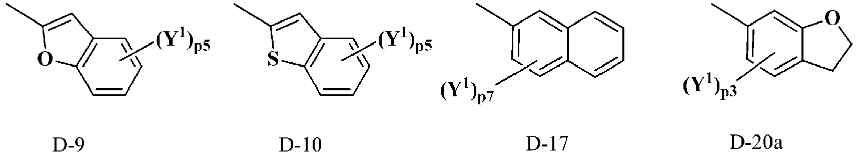 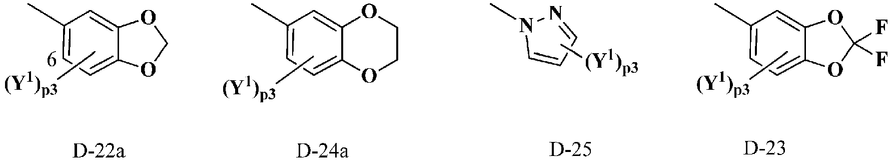 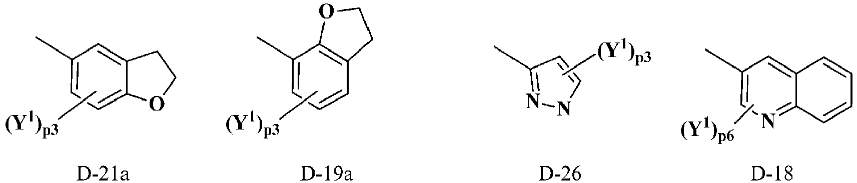
   Y¹ is substituted on the aromatic ring of each of D-1 to D-26;
   Y¹ is a hydrogen atom, a halogen atom, C₁₋₆ alkyl, C₁₋₆ haloalkyl, C₂₋₆ alkenyl, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, -NR⁸R⁹, -CN, -NO₂, -CO₂H, -C(=W²)R¹³, phenyl, (C₁₋₆) alkyl substituted with R¹⁰, or -OR¹¹, or tri(C₁₋₆ alkyl)silyl, and when p7, p6, p5, p4, or p3 is an integer of 2 or more, each Y¹ is the same as or different from each other;
   R⁴ is a halogen atom, -CN, C₂₋₆ alkenyl, C₂₋₆ alkynyl, C₁₋₆ alkoxy, C₁₋₆ alkylthio, - C(O)R²³, -OR²⁴, or phenyl;
   R⁵ is C₁₋₆ alkyl, C₁₋₆ alkyl substituted with R²⁶, C₁₋₆ alkoxy, (C₁₋₆) alkoxy substituted with R²⁷, C₁₋₆ alkylthio, U-6a, Q-2a, phenyl, or phenyl substituted with (Z²)ₚ₅ₐ;
   R⁶ is C₁₋₆ alkyl, C₁₋₆ haloalkyl, phenyl, phenyl substituted with (Z²)ₚ₅ₐ, or -NR²⁸R²⁹;
   W² is an oxygen atom or N-OR⁷;
   R⁷ is a hydrogen atom or C₁₋₆ alkyl;
   R⁸ and R⁹ are each independently a hydrogen atom, or C₁₋₆ alkyl;
   R¹⁰ is C₁₋₆ alkoxy, C₁₋₆ alkylthio, C₁₋₆ alkylsulfinyl, C₁₋₆ alkylsulfonyl, or -CN;
   R¹¹ is a hydrogen atom, C₁₋₆ alkyl, (C₁₋₆) alkyl substituted with R¹², C₃₋₆ cycloalkyl, phenyl, tri(C₁₋₄ alkyl)silyl, C₁₋₆ alkylcarbonyl, C₁₋₆ alkylsulfonyl, C₁₋₆ haloalkylsulfonyl, U-2a, or U-4a;
   R¹² is a halogen atom, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₃₋₆ cycloalkyl, C₃₋₆ halocycloalkyl, C₁₋₆ alkylthio, C₁₋₆ alkylsulfinyl, C₁₋₆ alkylsulfonyl, -CN, phenyl, phenyl substituted with (Z³)_{p5b}, -C(=W²)R¹³, U-1a, U-2a, U-3a, U-4a, U-5a, or Q-1a;
   U-1a, U-2a, U-3a, U-4a, U-5a, U-6a, Q-1a, Q-2a, and Q-3a are respectively the following structures: 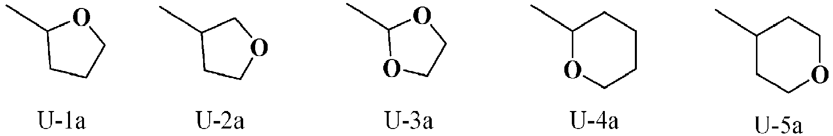 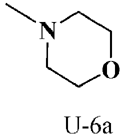 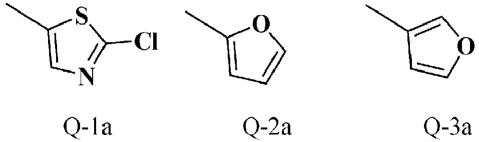
   R¹³ is a hydrogen atom, C₁₋₆ alkyl, C₁₋₆ alkoxy, or C₁₋₆ haloalkylamino;
   R¹⁴ is a halogen atom, C₃₋₆ cycloalkyl, C₃₋₆ halocycloalkyl, C₂₋₆ alkenyl, C₂₋₆ alkynyl, C₁₋₆ alkoxy, C₁₋₆ alkylthio, C₁₋₆ alkylsulfinyl, C₁₋₆ alkylsulfonyl, phenyl, or U-1a;
   R¹⁵ and R¹⁶ are each independently a hydrogen atom, C₁₋₆ alkyl, -C(O)R¹⁷, or - S(O)₂R¹⁸;
   R¹⁷ is C₁₋₆ alkyl, C₁₋₆ alkoxy, or C₁₋₆ alkoxy (C₁₋₂) alkyl;
   R¹⁸ is C₁₋₆ alkyl or C₁₋₆ haloalkyl;
   R¹⁹ is tri(C₁₋₆ alkyl)silyl;
   R²⁰ is a hydrogen atom or C₁₋₆ alkyl;
   W³ is an oxygen atom or N-OR²¹;
   R²¹ is a hydrogen atom or C₁₋₆ alkyl;
   R²² is a halogen atom, -OH, or C₁₋₆ alkoxy;
   R²³ is phenyl;
   R²⁴ is (C₁₋₆) alkoxy (C₁₋₂) alkyl or -C(O)R²⁵;
   R²⁵ is C₁₋₆ alkyl or C₁₋₆ alkoxy;
   R²⁶ is a halogen atom, (C₁₋₆) alkoxy, or -OR³²;
   R²⁷ is (C₁₋₆) alkoxy;
   R²⁸ and R²⁹ are each independently a hydrogen atom or C₁₋₆ alkyl;
   R³⁰ and R³¹ are each independently a hydrogen atom or benzyl;
   R³² is phenyl;
   R³³ is C₁₋₆ alkyl, C₁₋₆ haloalkyl, or di(C₁₋₆) alkylamino;
   R³⁵ is -SO₂R³³;
   Z² is a halogen atom, C₁₋₆ alkyl, or C₁₋₆ alkoxy, and when p5a is an integer of 2 or more, each Z² is the same as or different from each other;
   Z³ is a halogen atom, C₁₋₆ alkyl, C₁₋₆ haloalkyl, C₁₋₆ haloalkoxy, C₁₋₆ alkylthio, C₁₋₆ haloalkylthio, -CN, or -NO₂, and when p5b is an integer of 2 or more, each Z³ is the same as or different from each other;
   Z⁴ is a halogen atom, C₁₋₆ alkyl, or C₁₋₆ alkoxy, and when p5c is an integer of 2 or more, each Z³ is the same as or different from each other;
   n is an integer of 0, 1, 2, 3, or 4;
   p3 is an integer of 0, 1, 2, or 3;
   p4 is an integer of 0, 1, 2, 3, or 4;
   p5 is an integer of 0, 1, 2, 3, 4, or 5;
   p6 is an integer of 0, 1, 2, 3, 4, 5, or 6;
   p7 is an integer of 0, 1, 2, 3, 4, 5, 6, or 7;
   p5a is an integer of 1, 2, 3, 4, or 5;
   p5b is an integer of 1, 2, 3, 4, or 5; and
   p5c is an integer of 1, 2, 3, 4, or 5].
[150] The pyridazinone compound or a salt thereof according to [149], wherein:
   W¹ is an oxygen atom;
   Z¹ is a halogen atom, C₁₋₆ alkyl, (C₁₋₆) alkyl substituted with R²², C₂₋₆ alkenyl, C₂₋₆ alkynyl, (C₂₋₆) alkynyl substituted with R¹⁹, C₃₋₆ cycloalkyl, C₁₋₆ alkoxy, (C₁₋₆) alkoxy substituted with R¹⁴, phenyl, phenyl substituted with (Z⁴)_{p5c}, Q-3a, -OH, -NR¹⁵R¹⁶, -OR³⁵, - CN, or -C(=W³)R²⁰, and when n is an integer of 2 or more, each Z¹ is the same as or different from each other;
   R¹ is C₁₋₆ alkyl;
   R³ is D-1, D-3, D-6, D-8, D-9, D-10, D-17, D-20a, D-22a, D-24a, D-25, D-23, D-21a, D-19a, D-26, or D-18;
   Y¹ is a halogen atom, C₁₋₆ alkyl, C₁₋₆ haloalkyl, C₂₋₆ alkenyl, C₁₋₆ alkylthio, C₁₋₆ alkylsulfonyl, -NR⁸R⁹, -CN, -NO₂, -CO₂H, -C(=W²)R¹³, phenyl, (C₁₋₆) alkyl substituted with R¹⁰, -OR¹¹, or tri(C₁₋₆ alkyl)silyl, and when p7, p6, p5, p4, or p3 is an integer of 2 or more, each Y¹ is the same as or different from each other;
   R⁴ is C₂₋₆ alkynyl, C₁₋₆ alkoxy, -C(O)R²³, -OR²⁴, or phenyl;
   R⁵ is C₁₋₆ alkyl, (C₁₋₆) alkyl substituted with R²⁶, C₁₋₆ alkoxy, (C₁₋₆) alkoxy substituted with R²⁷, C₁₋₆ alkylthio, U-6a, Q-2a, or phenyl substituted with (Z²)ₚ₅ₐ;
   R⁶ is C₁₋₆ alkyl, phenyl substituted with (Z²)ₚ₅ₐ, or -NR²⁸R²⁹;
   R⁸ and R⁹ are each independently C₁₋₆ alkyl;
   R¹⁰ is C₁₋₆ alkoxy;
   R¹¹ is a hydrogen atom, C₁₋₆ alkyl, (C₁₋₆) alkyl substituted with R¹², C₃₋₆ cycloalkyl, phenyl, C₁₋₆ alkylsulfonyl, C₁₋₆ haloalkylsulfonyl, U-2a, or U-4a;
   R¹² is a halogen atom, C₂₋₆ alkenyl, C₂₋₆ alkynyl, C₁₋₆ alkoxy, C₃₋₆ cycloalkyl, C₃₋₆ halocycloalkyl, C₁₋₆ alkylthio, -CN, phenyl, -C(=W²)R¹³, or Q-1a;
   R¹³ is C₁₋₆ alkyl, C₁₋₆ alkoxy, or C₁₋₆ haloalkylamino;
   R¹⁴ is a halogen atom, C₃₋₆ cycloalkyl, C₃₋₆ halocycloalkyl, C₂₋₆ alkenyl, C₁₋₆ alkoxy, C₁₋₆ alkylthio, C₁₋₆ alkylsulfonyl, phenyl, or U-1a;
   R¹⁸ is C₁₋₆ alkyl;
   R²⁰ is C₁₋₆ alkyl;
   R²⁵ is C₁₋₆ alkoxy;
   R²⁶ is C₁₋₆ alkoxy, or -OR³²;
   R²⁸ and R²⁹ are C₁₋₆ alkyl;
   R³³ is di(C₁₋₆) alkylamino;
   R³⁵ is -SO₂R³³;
   Z² is a halogen atom or C₁₋₆ alkyl;
   Z⁴ is a halogen atom or C₁₋₆ alkoxy;
   n is an integer of 0, 1, or 2;
   p3 is an integer of 0 or 1;
   p4 is an integer of 0, 1, or 2;
   p5 is an integer of 0, 1, 2, or 3;
   p6 is an integer of 0;
   p7 is an integer of 0; and
   p5a is an integer of 1.
[151] An agricultural chemical comprising, as an active ingredient, one or more selected from the pyridazinone compound and a salt thereof according to any of [1] to [141].
[152] A herbicide comprising, as an active ingredient, one or more selected from the pyridazinone compound and a salt thereof according to any of [1] to [141].

### Effects of the Invention

The compound of the present invention exhibits excellent herbicidal activity against various weeds, and has high safety to target crops. Furthermore, the compound has almost no adverse effect on non-target living organisms, such as mammals, fishes, and beneficial insects, and has low environmental burden because of its low residual property.

Thus, the present invention can provide a herbicide useful in the agricultural and horticultural fields, such as paddy fields, farmlands, and orchards.

### MODES FOR CARRYING OUT THE INVENTION

The compound of the present invention may include geometrical isomers (i.e., E-form and Z-form) depending on the types of substituents. The present invention includes an E-form, a Z-form, and a mixture containing an E-form and a Z-form in any proportions.

The compound of the present invention may include, depending on the types of substituents, optically active substances attributed to the presence of one or more asymmetric carbon atoms. The present invention includes all optically active substances or racemates.

The compound of the present invention may include tautomers depending on the types of substituents. The present invention includes all tautomers or a mixture containing tautomers in any proportions. For example, among compounds of Formula (1) (hereinafter will be referred to "compounds (1)"), the compound of the following Formula (1-1a), in which G is a hydrogen atom and W¹ is an oxygen atom, may include a tautomer such as a compound of the following Formula (1-1a-1) or a compound of the following Formula (1-1a-2). Thus, in the case of introduction of the substituent G¹ (G¹ corresponds to structures represented by G (except for a hydrogen atom)) the enol structure (1-1a) is formed into a structure (1-1a-3), and the enol structure (1-1a-2) is formed into a structure (1-1a-4). The present invention includes all these structures.

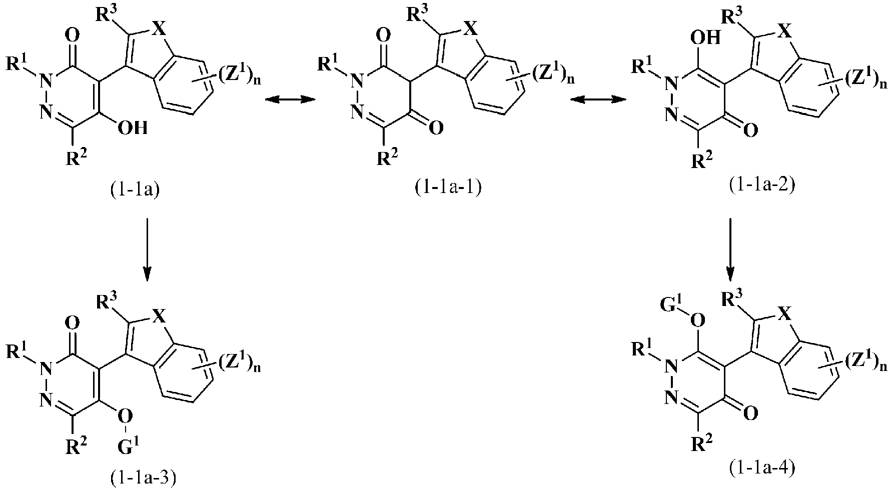

The compound of the present invention may include one or more rotational isomers attributed to limited bond rotation caused by the steric hindrance between substituents. The present invention includes all rotational isomers or a mixture containing diastereomers in any proportions.

The compound of the present invention may be formed into an acid addition salt by any common method. Examples of the acid addition salt include salts of hydrohalic acids such as hydrogen fluoride, hydrogen chloride, hydrogen bromide, and hydrogen iodide; salts of inorganic acids such as nitric acid, sulfuric acid, phosphoric acid, chloric acid, and perchloric acid; salts of sulfonic acids such as methanesulfonic acid, ethanesulfonic acid, trifluoromethanesulfonic acid, benzenesulfonic acid, and p-toluenesulfonic acid; salts of carboxylic acids such as formic acid, acetic acid, propionic acid, trifluoroacetic acid, fumaric acid, tartaric acid, oxalic acid, maleic acid, malic acid, succinic acid, benzoic acid, mandelic acid, ascorbic acid, lactic acid, gluconic acid, and citric acid; or salts of amino acids such as glutamic acid and aspartic acid.

Alternatively, the compound of the present invention may be formed into a metal salt by any common method. Examples of the metal salt include salts of alkali metals such as lithium, sodium, and potassium; salts of alkaline earth metals such as calcium, barium, and magnesium; salt of aluminum; or quaternary ammonium salts such as tetramethylammonium salt, tetrabutylammonium salt, and benzyltrimethylammonium salt.

The terms or phrases as used herein have the meanings or usages described below.

The term "agricultural chemical" as used herein refers to a insecticide, a miticide, a nematicide, a herbicide, and a fungicide in the agricultural and horticultural fields.

Examples of the "halogen atom" as used herein include a fluorine atom, a chlorine atom, a bromine atom, and an iodine atom. The term "halo" as used herein also refers to such a halogen atom.

In the specific description of substituents in the present specification, the term "n-" refers to "normal"; the term "i-" refers to "iso"; the term "sec-" refers to "secondary"; and the term "tert-" refers to "tertiary."

The expression "C_{a-b} alkyl" as used herein refers to a linear or branched hydrocarbon group having a carbon atom number of a to b. Specific examples of the Ca-b alkyl include methyl, ethyl, n-propyl, i-propyl, n-butyl, i-butyl, sec-butyl, tert-butyl, n-pentyl, 1-ethylpropyl, 2,2-dimethylpropyl, and n-hexyl. Each of these groups is selected within a range of the specified number of carbon atoms.

The expression "C_{a-b} haloalkyl" as used herein refers to a linear or branched hydrocarbon group having a carbon atom number of a to b wherein a hydrogen atom bonded to a carbon atom is optionally substituted with a halogen atom. When the hydrocarbon group is substituted with two or more halogen atoms, these halogen atoms may be identical to or different from one another. Specific examples of the Ca-b haloalkyl include fluoromethyl, chloromethyl, bromomethyl, iodomethyl, difluoromethyl, dichloromethyl, trifluoromethyl, chlorodifluoromethyl, trichloromethyl, bromodifluoromethyl, 1-fluoroethyl, 2-fluoroethyl, 2-chloroethyl, 2-bromoethyl, 1,1-difluoroethyl, 2,2-difluoroethyl, 2,2,2-trifluoroethyl, 2-chloro-2,2-difluoroethyl, 2,2,2-trichloroethyl, 2-bromo-2,2-difluoroethyl, 1,1,2,2-tetrafluoroethyl, 2-chloro-1,1,2-trifluoroethyl, pentafluoroethyl, 2,2-difluoropropyl, 3,3,3-trifluoropropyl, 3-bromo-3,3-difluoropropyl, 2,2,3,3-tetrafluoropropyl, 2,2,3,3,3-pentafluoropropyl, 1,1,2,3,3,3-hexafluoropropyl, heptafluoropropyl, 2,2,2-trifluoro-1-methylethyl, 2,2,2-trifluoro-1-(trifluoromethyl)ethyl, 1,2,2,2-tetrafluoro-1-(trifluoromethyl)ethyl, 2,2,2-trifluoro-1,1-dimethyl ethyl, 2,2,3,4,4,4-hexafluorobutyl, 2,2,3,3,4,4,4-heptafluorobutyl, and nonafluorobutyl. Each of these groups is selected within a range of the specified number of carbon atoms.

The expression "C_{a-b} alkenyl" as used herein refers to a linear or branched unsaturated hydrocarbon group having a carbon atom number of a to b and having one or more double bonds in the molecule. Specific examples of the Ca-b alkenyl include vinyl, 1-propenyl, 2-propenyl, 1-methylethenyl, 1-butenyl, 2-butenyl, 1-methyl-1-propenyl, 2-methyl-1-propenyl, 2-methyl-2-propenyl, and 3-methyl-3-butenyl. Each of these groups is selected within a range of the specified number of carbon atoms.

The expression "C_{a-b} haloalkenyl" as used herein refers to a linear or branched unsaturated hydrocarbon group having a carbon atom number of a to b and having one or more double bonds in the molecule wherein a hydrogen atom bonded to a carbon atom is optionally substituted with a halogen atom. When the hydrocarbon group is substituted with two or more halogen atoms, these halogen atoms may be identical to or different from one another. Specific examples of the Ca-b haloalkenyl include 2,2-difluorovinyl, 2,2-dichlorovinyl, 2-fluoro-2-propenyl, 2-chloro-2-propenyl, 3-chloro-2-propenyl, 2-bromo-2-propenyl, 3,3-difluoro-2-propenyl, 2,3-dichloro-2-propenyl, 3,3-dichloro-2-propenyl, 2,3,3-trifluoro-2-propenyl, 2,3,3-trichloro-2-propenyl, 1-(trifluoromethyl)ethenyl, 4,4-difluoro-3-butenyl, 3,4,4-trifluoro-3-butenyl, and 3-chloro-4,4,4-trifluoro-2-butenyl. Each of these groups is selected within a range of the specified number of carbon atoms.

The expression "C_{a-b} alkynyl" as used herein refers to a linear or branched unsaturated hydrocarbon group having a carbon atom number of a to b and having one or more triple bonds in the molecule. Specific examples of the C_{a-b} alkynyl include ethynyl, 1-propynyl, 2-propynyl, 1-butynyl, 2-butynyl, 3-butynyl, 1-methyl-2-propynyl, 1-pentynyl, 2-pentynyl, 1-hexynyl, 3-hexynyl, 3-methyl-1-pentynyl, 4-methyl-1-pentynyl, and 3,3-dimethyl-1-butynyl. Each of these groups is selected within a range of the specified number of carbon atoms.

The expression "C_{a-b} haloalkynyl" as used herein refers to a linear or branched unsaturated hydrocarbon group having a carbon atom number of a to b and having one or more triple bonds in the molecule wherein a hydrogen atom bonded to a carbon atom is optionally substituted with a halogen atom. When the hydrocarbon group is substituted with two or more halogen atoms, these halogen atoms may be identical to or different from one another. Specific examples of the C_{a-b} haloalkynyl include 2-chloroethynyl, 2-bromoethynyl, 2-iodoethynyl, 3-chloro-2-propynyl, 3-bromo-2-propynyl, and 3-iodo-2-propynyl. Each of these groups is selected within a range of the specified number of carbon atoms.

The expression "C_{a-b} alkoxy" as used herein refers to an alkyl-O- group wherein the alkyl has the same meaning as defined above and has a carbon atom number of a to b. Specific examples of the Ca-b alkoxy include methoxy, ethoxy, n-propyloxy, i-propyloxy, n-butyloxy, i-butyloxy, sec-butyloxy, tert-butyloxy, n-pentyloxy, and n-hexyloxy. Each of these groups is selected within a range of the specified number of carbon atoms.

The expression "C_{a-b} haloalkoxy" as used herein refers to a haloalkyl-O-group wherein the haloalkyl has the same meaning as defined above and has a carbon atom number of a to b. Specific examples of the Ca-b haloalkoxy include difluoromethoxy, trifluoromethoxy, chlorodifluoromethoxy, bromodifluoromethoxy, 2-fluoroethoxy, 2-chloroethoxy, 2,2,2-trifluoroethoxy, 1,1,2,2-tetrafluoroethoxy, 2-chloro-1,1,2-trifluoroethoxy, and 1,1,2,3,3,3-hexafluoropropyloxy. Each of these groups is selected within a range of the specified number of carbon atoms.

The expression "C_{a-b} alkylthio" as used herein refers to an alkyl-S- group wherein the alkyl has the same meaning as defined above and has a carbon atom number of a to b. Specific examples of the Ca-b alkylthio include methylthio, ethylthio, n-propylthio, i-propylthio, n-butylthio, i-butylthio, sec-butylthio, and tert-butylthio. Each of these groups is selected within a range of the specified number of carbon atoms.

The expression "C_{a-b} haloalkylthio" as used herein refers to a haloalkyl-S-group wherein the haloalkyl has the same meaning as defined above and has a carbon atom number of a to b. Specific examples of the Ca-b haloalkylthio include difluoromethylthio, trifluoromethylthio, chlorodifluoromethylthio, trichloromethylthio, bromodifluoromethylthio, 2,2,2-trifluoroethylthio, 1,1,2,2-tetrafluoroethylthio, 2-chloro-1,1,2-trifluoroethylthio, pentafluoroethylthio, 1,1,2,3,3,3-hexafluoropropylthio, heptafluoropropylthio, 1,2,2,2-tetrafluoro-1-(trifluoromethyl)ethylthio, and nonafluorobutylthio. Each of these groups is selected within a range of the specified number of carbon atoms.

The expression "C_{a-b} alkylsulfinyl" as used herein refers to an alkyl-S(O)-group wherein the alkyl has the same meaning as defined above and has a carbon atom number of a to b. Specific examples of the C_{a-b} alkylsulfinyl include methylsulfinyl, ethylsulfinyl, n-propylsulfinyl, i-propylsulfinyl, n-butylsulfinyl, i-butylsulfinyl, sec-butylsulfinyl, and tert-butylsulfinyl. Each of these groups is selected within a range of the specified number of carbon atoms.

The expression "C_{a-b} haloalkylsulfinyl" as used herein refers to a haloalkylS(O)- group wherein the haloalkyl has the same meaning as defined above and has a carbon atom number of a to b. Specific examples of the Ca-b haloalkylsulfinyl include difluoromethylsulfinyl, trifluoromethylsulfinyl, chlorodifluoromethylsulfinyl, bromodifluoromethylsulfinyl, 2,2,2-trifluoroethylsulfinyl, 1,2,2,2-tetrafluoro-1-(trifluoromethyl)ethylsulfinyl, and nonafluorobutylsulfinyl. Each of these groups is selected within a range of the specified number of carbon atoms.

The expression "C_{a-b} alkyl sulfonyl" as used herein refers to an alkyl-S(O)₂-group wherein the alkyl has the same meaning as defined above and has a carbon atom number of a to b. Specific examples of the C_{a-b} alkylsulfonyl include methylsulfonyl, ethylsulfonyl, n-propylsulfonyl, i-propylsulfonyl, n-butylsulfonyl, i-butylsulfonyl, sec-butylsulfonyl, and tert-butylsulfonyl. Each of these groups is selected within a range of the specified number of carbon atoms.

The expression "C_{a-b} haloalkylsulfonyl" as used herein refers to a haloalkylS(O)₂- group wherein the haloalkyl has the same meaning as defined above and has a carbon atom number of a to b. Specific examples of the C_{a-b} haloalkylsulfonyl include difluoromethylsulfonyl, trifluoromethyl sulfonyl, chlorodifluoromethyl sulfonyl, bromodifluoromethylsulfonyl, 2,2,2-trifluoroethylsulfonyl, 1,1,2,2-tetrafluoroethylsulfonyl, and 2-chloro-1,1,2-trifluoroethylsulfonyl. Each of these groups is selected within a range of the specified number of carbon atoms.

The expression "C_{a-b} cycloalkyl" as used herein refers to a cyclic hydrocarbon group having a carbon atom number of a to b, and the hydrocarbon group can form a monocyclic or multi-cyclic structure of a 3-membered to 10-membered ring. Each ring is optionally substituted with alkyl within a range of the specified number of carbon atoms. Specific examples of the C_{a-b} cycloalkyl include cyclopropyl, 1-methylcyclopropyl, 2-methylcyclopropyl, 2,2-dimethylcyclopropyl, cyclobutyl, cyclopentyl, and cyclohexyl. Each of these groups is selected within a range of the specified number of carbon atoms.

The expression "C_{a-b} halocycloalkyl" as used herein refers to a cyclic hydrocarbon group having a carbon atom number of a to b wherein a hydrogen atom bonded to a carbon atom is optionally substituted with a halogen atom, and the hydrocarbon group can form a monocyclic or multi-cyclic structure of a 3-membered to 10-membered ring. Each ring is optionally substituted with alkyl within a range of the specified number of carbon atoms, and the halogen atom may be substituted on a cyclic structure moiety, a side chain moiety, or both of these moieties. When the hydrocarbon group is substituted with two or more halogen atoms, these halogen atoms may be identical to or different from one another. Specific examples of the C_{a-b} halocycloalkyl include 2,2-difluorocyclopropyl, 2,2-dichlorocyclopropyl, 2,2-dibromocyclopropyl, 2,2-difluoro-1-methylcyclopropyl, 2,2-dichloro-1-methylcyclopropyl, 2,2-dibromo-1-methylcyclopropyl, and 2,2,3,3-tetrafluorocyclobutyl. Each of these groups is selected within a range of the specified number of carbon atoms.

The expression "benzyl" as used herein refers to "C₆H₅-CH₂-."

The expression "(C_{a-b}) alkyl substituted with R⁴", "(C_{a-b}) alkyl substituted with R¹⁰", "(C_{a-b}) alkyl substituted with R¹²", "(C_{a-b}) alkyl substituted with R¹⁴", "(C_{a-b}) alkyl substituted with R²²", "(C_{a-b}) alkyl substituted with R²⁶", "(C_{a-b}) alkyl substituted with R²⁷", "(C_{a-b}) alkyl substituted with R³⁴", "(C_{a-b}) alkyl substituted with R³⁷", "(C_{a-b}) alkyl substituted with R³⁹", "(C_{a-b}) alkyl substituted with R⁴⁶", or "(C_{a-b}) alkyl substituted with R⁴⁷" as used herein refers to an alkyl group having a carbon atom number of a to b wherein any hydrogen atom bonded to a carbon atom is partially or completely substituted with one or more substituents R⁴, R¹⁰, R¹², R¹⁴, R²², R²⁶, R²⁷, R³⁴, R³⁷, R³⁹, R⁴⁶, or R⁴⁷, and the alkyl has the same meaning as defined above. Each group is selected within a range of the specified number of carbon atoms. When two or more substituents R⁴, R¹⁰, R¹², R¹⁴, R²², R²⁶, R²⁷, R³⁴, R³⁷, R³⁹, R⁴⁶, or R⁴⁷ are present, the substituents R⁴, R¹⁰, R¹², R¹⁴, R²², R²⁶, R²⁷, R³⁴, R³⁷, R³⁹, R⁴⁶, or R⁴⁷ may be identical to or different from one another.

The expression "(C_{a-b}) alkynyl substituted with R¹⁹" as used herein refers to an alkynyl group having a carbon atom number of a to b wherein any hydrogen atom bonded to a carbon atom is partially or completely substituted with one or more substituents R¹⁹, and the alkynyl has the same meaning as defined above. Each group is selected within a range of the specified number of carbon atoms. When two or more substituents R¹⁹ are present, the substituents R¹⁹ may be identical to or different from one another.

The expression "(Ca-b) cycloalkyl substituted with R⁴⁴" or "(Ca-b) cycloalkyl substituted with R⁴⁵" as used herein refers to a cycloalkyl group having a carbon atom number of a to b wherein any hydrogen atom bonded to a carbon atom is partially or completely substituted with one or more substituents R⁴⁴ or R⁴⁵, and the cycloalkyl has the same meaning as defined above. Each group is selected within a range of the specified number of carbon atoms. When two or more substituents R⁴⁴ or R⁴⁵ are present, the substituents R⁴⁴ or R⁴⁵ may be identical to or different from one another.

The expression "C_{a-b} cycloalkyl (C_{d-e}) alkyl" as used herein refers to an alkyl group having a carbon atom number of d to e and the same meaning as defined above wherein a hydrogen atom bonded to a carbon atom is optionally substituted with a Ca-b cycloalkyl group having the same meaning as defined above. Each group is selected within a range of the specified number of carbon atoms.

The expression "C_{a-b} alkoxy (C_{d-e}) alkyl" as used herein refers to an alkyl group having a carbon atom number of d to e and the same meaning as defined above wherein a hydrogen atom bonded to a carbon atom is optionally substituted with a Ca-b alkoxy group having the same meaning as defined above. Each group is selected within a range of the specified number of carbon atoms.

The expression "tri(C_{a-b} alkyl)silyl" as used herein refers to a silyl group substituted with alkyl groups having a carbon atom number of a to b and having the same meaning as defined above wherein the alkyl groups may be identical to or different from one another. Specific examples of the tri(C_{a-b} alkyl)silyl include trimethylsilyl, triethylsilyl, tri(n-propyl)silyl, ethyldimethylsilyl, n-propyldimethylsilyl, n-butyldimethylsilyl, i-butyldimethylsilyl, and tert-butyldimethylsilyl. Each of these groups is selected within a range of the specified number of carbon atoms.

The expression "C_{a-b} alkylcarbonyl" as used herein refers to an alkyl-C(O)-group wherein the alkyl has the same meaning as defined above and has a carbon atom number of a to b. Specific examples of the Ca-b alkylcarbonyl include acetyl, n-propionyl, n-butyryl, i-butyryl, n-valeryl, i-valeryl, 2-methylbutanoyl, pivaloyl, n-hexanoyl, and n-heptanoyl. Each of these groups is selected within a range of the specified number of carbon atoms.

The expression "C_{a-b} alkoxycarbonyl" as used herein refers to an alkyl-O-C(O)- group wherein the alkyl has the same meaning as defined above and has a carbon atom number of a to b. Specific examples of the Ca-b alkoxycarbonyl include methoxycarbonyl, ethoxycarbonyl, n-propyloxycarbonyl, i-propyloxycarbonyl, n-butoxycarbonyl, i-butoxycarbonyl, sec-butoxycarbonyl, tert-butoxycarbonyl, and 2-ethylhexyloxycarbonyl. Each of these groups is selected within a range of the specified number of carbon atoms.

The expression "C_{a-b} alkylamino" as used herein refers to an amino group wherein one hydrogen atom is substituted with alkyl having the same meaning as defined above and having a carbon atom number of a to b. Specific examples of the Ca-b alkylamino include methylamino, ethylamino, n-propylamino, i-propylamino, n-butylamino, i-butylamino, and tert-butylamino. Each of these groups is selected within a range of the specified number of carbon atoms.

The expression "C_{a-b} haloalkylamino" as used herein refers to an amino group wherein one hydrogen atom is substituted with haloalkyl having the same meaning as defined above and having a carbon atom number of a to b. Specific examples of the C_{a-b} haloalkylamino include 2,2,2-trifluoroethylamino, 2-chloro-2,2-difluoroethylamino, and 3,3,3-trifluoropropylamino. Each of these groups is selected within a range of the specified number of carbon atoms.

The expression "di(Ca-b) alkylamino" as used herein refers to an amino group wherein both hydrogen atoms are substituted with alkyl groups which have the same meaning as defined above and have a carbon atom number of a to b and which may be identical to or different from each other. Specific examples of the di(Ca-b) alkylamino include dimethylamino, ethyl(methyl)amino, diethylamino, n-propyl(methyl)amino, i-propyl(methyl)amino, di(n-propyl)amino, and di(n-butyl)amino. Each of these groups is selected within a range of the specified number of carbon atoms.

The expression "C_{a-b} alkylthio (C_{d-e}) alkyl" as used herein refers to an alkyl group having the same meaning as defined above and having a carbon atom number of d to e wherein a hydrogen atom bonded to a carbon atom is optionally substituted with a Ca-b alkylthio group having the same meaning as defined above. Each group is selected within a range of the specified number of carbon atoms.

The expression "phenyl substituted with (Z²)ₚ₅ₐ", "phenyl substituted with (Z³)_{p5b}", or "phenyl substituted with (Z⁴)_{p5c}" as used herein refers to a phenyl group wherein p5a, p5b, or p5c hydrogen atoms bonded to carbon atoms of the benzene ring are substituted with substituents Z², Z³, or Z⁴ at any positions.

The expression "benzyl substituted with (Z⁴)_{p5c}" as used herein refers to a benzyl group (C₆H₅-CH₂-) wherein p5c hydrogen atoms bonded to carbon atoms of the benzene ring are substituted with substituents Z⁴ at any positions.

The production method of the present invention will next be described. In the following description, the symbol Ph denotes phenyl, and the symbol Bn denotes a benzyl group.

The compound of the present invention may include geometrical isomers (i.e., E-form and Z-form) depending on the types of substituents. In the following description, a mixture containing an E-form and a Z-form in any proportions is represented as a bond of a wavy line shown below.

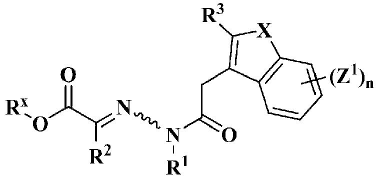

The compound of the present invention of Formula (1) can be produced by, for example, any of the methods described below.

### Production method A

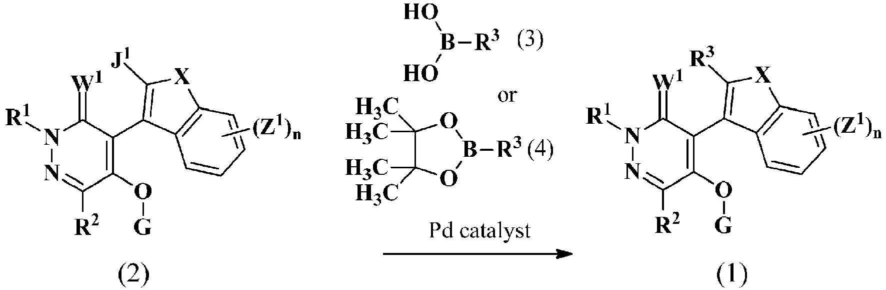

A compound of Formula (1) [wherein G, W¹, X, R¹, R², R³, Z¹, and n have the same meanings as defined above] [hereinafter the compound will be referred to as "compound (1)"] can be produced by reacting a compound of Formula (2) [wherein G, W¹, X, R¹, R², Z¹, and n have the same meanings as defined above, and J¹ is a chlorine atom, a bromine atom, or an iodine atom] [hereinafter the compound will be referred to as "compound (2)"] with a compound of Formula (3) [wherein R³ has the same meaning as defined above] [hereinafter the compound will be referred to as "compound (3)"] or a compound of Formula (4) [wherein R³ has the same meaning as defined above] [hereinafter the compound will be referred to as "compound (4)"] in the presence of a palladium catalyst (e.g., palladium(II) acetate, tetrakis(triphenylphosphine)palladium(0), or [1,1'-bis(diphenylphosphino)ferrocene]palladium(II) dichloride) in an amount of 0.001 to 0.5 equivalents by mole relative to compound (2) at a temperature ranging from room temperature to the reflux temperature of the reaction mixture for one hour to 48 hours, with optional use of a solvent (e.g., benzene, toluene, methanol, ethanol, propanol, 1,4-dioxane, tetrahydrofuran, dimethoxyethane, N,N-dimethylformamide, N,N-dimethylacetamide, dimethyl sulfoxide, water, or a mixture containing two or more of these in any proportions), with optional use of a base (e.g., sodium carbonate, potassium carbonate, cesium carbonate, potassium phosphate, sodium hydrogen carbonate, potassium hydrogen carbonate, triethylamine, or N,N-diisopropylethylamine) in an amount of 1 to 10 equivalents by mole relative to compound (2), and with optional addition of a ligand (e.g., triphenylphosphine, tricyclohexylphosphine, 2-dicyclohexylphosphino-2',6'-diisopropoxybiphenyl, 2-dicyclohexylphosphino-2',6'-dimethoxybiphenyl, or 1,1'-bis(diphenylphosphino)ferrocene) in an amount of 0.001 to 0.5 equivalents by mole relative to compound (2).

Some of compounds (3) and compounds (4) used herein are known compounds and are available as commercial products. The other compounds can also be synthesized by known methods described in literature, for example, methods described in Japanese Unexamined Patent Application Publication No. 2002-47292 (JP 2002-47292 A), Tetrahedron, 2006, Vol. 62, page 2831, and European Journal of Organic Chemistry, 2009, Vol. 2009, No. 23, page 3964.

### Production method B

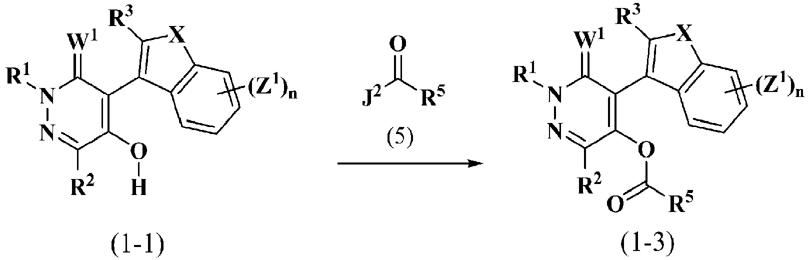

A compound of Formula (1-3) [wherein W¹, X, R¹, R², R³, R⁵, Z¹, and n have the same meanings as defined above] [hereinafter the compound will be referred to as "compound (1-3)"], which is a type of compound (1) wherein G is -C(O)R⁵, can be produced by reacting a compound of Formula (1-1) [wherein W¹, X, R¹, R², R³, Z¹, and n have the same meanings as defined above] [hereinafter the compound will be referred to as "compound (1-1)"], which is a type of compound (1) wherein G is a hydrogen atom, with a compound of Formula (5) [wherein R⁵ has the same meaning as defined above, and J² is a chlorine atom, a bromine atom, a C₁₋₄ alkylcarbonyloxy group (e.g., pivaloyl), a C₁₋₄ alkoxycarbonyloxy group (e.g., isobutyloxycarbonyloxy), or an azolyl group (e.g., imidazol-1-yl)] [hereinafter the compound will be referred to as "compound (5)"] at a temperature ranging from 0°C to the reflux temperature of the reaction mixture for 30 minutes to 24 hours, with optional use of a solvent (e.g., benzene, toluene, dichloromethane, chloroform, 1,2-dichloroethane, diethyl ether, tert-butyl methyl ether, tetrahydrofuran, 1,4-dioxane, ethyl acetate, N,N-dimethylformamide, N,N-dimethylacetamide, acetonitrile, water, or a mixture containing two or more of these in any proportions), and optionally in the presence of a base (e.g., sodium carbonate, potassium carbonate, sodium hydrogen carbonate, potassium hydrogen carbonate, triethylamine, N,N-diisopropylethylamine, pyridine, or 4-(dimethylamino)pyridine) in an amount of 1 to 3 equivalents by mole relative to compound (1-1).

Some of compounds (5) used herein are known compounds and are available as commercial products. The other compounds can also be synthesized by synthesis methods for known compounds.

### Production method C

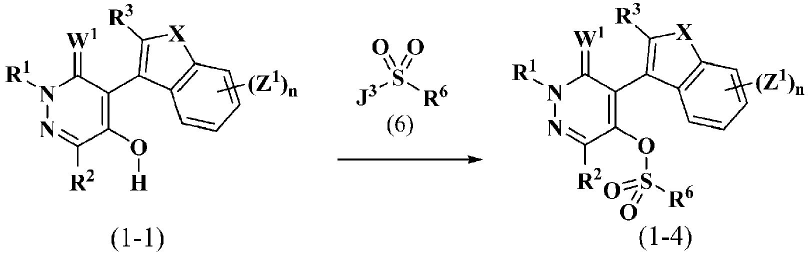

A compound of Formula (1-4) [wherein W¹, X, R¹, R², R³, R⁶, Z¹, and n have the same meanings as defined above] [hereinafter the compound will be referred to as "compound (1-4)"], which is a type of compound (1) wherein G is -S(O)₂R⁶, can be produced by reacting compound (1-1) with a compound of Formula (6) [wherein R⁶ has the same meaning as defined above, and J³ is a fluorine atom, a chlorine atom, a bromine atom, or -OS(O)₂R⁶] [hereinafter the compound will be referred to as "compound (6)"] at a temperature ranging from 0°C to the reflux temperature of the reaction mixture for 30 minutes to 24 hours, with optional use of a solvent (e.g., benzene, toluene, dichloromethane, chloroform, 1,2-dichloroethane, diethyl ether, tert-butyl methyl ether, tetrahydrofuran, 1,4-dioxane, ethyl acetate, N,N-dimethylformamide, N,N-dimethylacetamide, acetonitrile, water, or a mixture containing two or more of these in any proportions), and optionally in the presence of a base (e.g., sodium carbonate, potassium carbonate, sodium hydrogen carbonate, potassium hydrogen carbonate, triethylamine, N,N-diisopropylethylamine, pyridine, or 4-(dimethylamino)pyridine) in an amount of 1 to 3 equivalents by mole relative to compound (1-1).

Some of compounds (6) used herein are known compounds and are available as commercial products. The other compounds can also be synthesized by synthesis methods for known compounds.

### Production method D

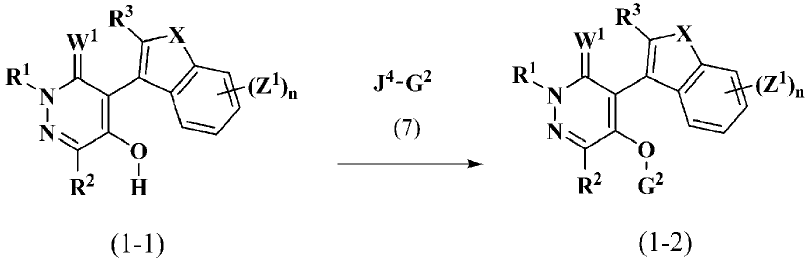

A compound of Formula (1-2) [wherein G², W¹, X, R¹, R², R³, Z¹, and n have the same meanings as defined above] [hereinafter the compound will be referred to as "compound (1-2)"], which is a type of compound (1) wherein G is G², can be produced by reacting compound (1-1) with a compound of Formula (7) [wherein G² is C₁₋₆ alkyl or (C₁₋₆) alkyl substituted with R⁴; J⁴ is a chlorine atom, a bromine atom, an iodine atom, C₁₋₆ alkylsulfonyloxy, or C₁₋₆ haloalkylsulfonyloxy; and R⁴ has the same meaning as defined above] [hereinafter the compound will be referred to as "compound (7)"] at a temperature ranging from -78°C to the reflux temperature of the reaction mixture for 30 minutes to 24 hours, with optional use of a solvent (e.g., benzene, toluene, diethyl ether, tert-butyl methyl ether, tetrahydrofuran, 1,4-dioxane, N,N-dimethylformamide, N,N-dimethylacetamide, acetonitrile, dimethyl sulfoxide, sulfolane, triamide hexamethylphosphate, water, or a mixture containing two or more of these in any proportions), and optionally in the presence of a base (e.g., sodium hydroxide, potassium hydroxide, lithium hydroxide, calcium hydroxide, sodium carbonate, potassium carbonate, cesium carbonate, sodium hydrogen carbonate, potassium hydrogen carbonate, sodium hydride, potassium hydride, potassium tert-butoxide, lithium diisopropylamide, sodium bis(trimethylsilyl)amide, or lithium bis(trimethylsilyl)amide) in an amount of 1 to 3 equivalents by mole relative to compound (1-1).

Some of compounds (7) used herein are known compounds and are available as commercial products. The other compounds can also be synthesized by synthesis methods for known compounds.

### Production method E

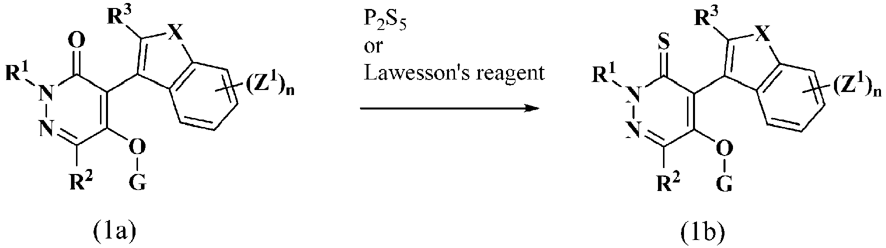

A compound of Formula (1b) [wherein G, X, R¹, R², R³, Z¹, and n have the same meanings as defined above] [hereinafter the compound will be referred to as "compound (1b)"], which is a type of compound (1) wherein W¹ is a sulfur atom, can be produced by reacting a compound of Formula (1a) [wherein G, X, R¹, R², R³, Z¹, and n have the same meanings as defined above] [hereinafter the compound will be referred to as "compound (1a)"], which is a type of compound (1) wherein W¹ is an oxygen atom, with diphosphorus pentasulfide or Lawesson's reagent: 2,4-bis(4-methoxyphenyl)-1,3-dithia-2,4-diphosphetane-2,4-disulfide] in an amount of 1 to 10 equivalents by mole relative to compound (1a) at a temperature ranging from room temperature to the reflux temperature of the reaction mixture for 30 minutes to 24 hours, with optional use of a solvent (e.g., benzene, toluene, xylene, pyridine, piperidine, diethyl ether, tert-butyl methyl ether, tetrahydrofuran, 1,4-dioxane, methanol, ethanol, water, or a mixture containing two or more of these in any proportions).

Compound (1-1a) can be produced by the method described below.

### Production method F

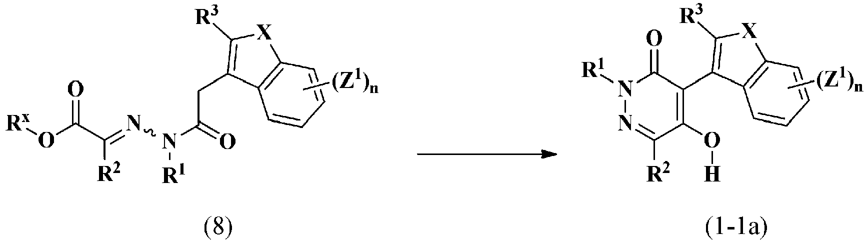

A compound of Formula (1-1a) [wherein X, R¹, R², R³, Z¹, and n have the same meanings as defined above] [hereinafter the compound will be referred to as "compound (1-1a)"], which is a type of compound (1-1) wherein W¹ is an oxygen atom, can be produced by reacting a compound of Formula (8) [wherein X, R¹, R², R³, Z¹, and n have the same meanings as defined above, and R^{X} is C₁₋₆ alkyl] [hereinafter the compound will be referred to as "compound (8)"] at a temperature ranging from room temperature to the reflux temperature of the reaction mixture for 30 minutes to 24 hours, with optional use of a solvent (e.g., hexane, heptane, benzene, toluene, diethyl ether, diisopropyl ether, tetrahydrofuran, 1,4-dioxane, dichloromethane, chloroform, 1,2-dichloroethane, N,N-dimethylformamide, N,N-dimethylacetamide, dimethyl sulfoxide, acetonitrile, acetone, methyl ethyl ketone, acetonitrile, water, or a mixture containing two or more of these in any proportions), and optionally in the presence of a base (e.g., triethylamine, tributylamine, diisopropylethylamine, pyridine, 4-(dimethylamino)pyridine, 1,8-diazabicyclo[5,4,0]-7-undecene, 1,4-diazabicyclo[2,2,2]octane, sodium hydroxide, potassium hydroxide, calcium hydroxide, sodium carbonate, potassium carbonate, sodium hydrogen carbonate, potassium hydrogen carbonate, sodium hydride, or potassium tert-butoxide) in an amount of 1 to 5 equivalents by mole relative to compound (8).

### Production method G

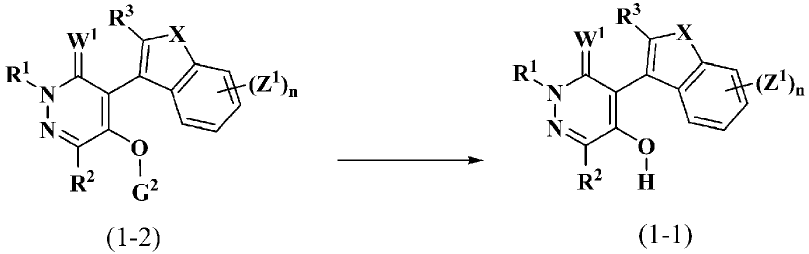

Compound (1-1) can be produced by reacting compound (1-2) with morpholine in an amount of 1 to 20 equivalents by mole relative to compound (1-2) at a temperature ranging from 0°C to the reflux temperature of the reaction mixture for one hour to 48 hours, with optional use of a solvent (e.g., benzene, toluene, xylene, N,N-dimethylformamide, N,N-dimethylacetamide, N-methyl-2-pyrrolidone, dimethyl sulfoxide, sulfolane, acetonitrile, water, or a mixture containing two or more of these in any proportions); or reacting compound (1-2) with a protonic acid (e.g., sulfuric acid or hydrochloric acid) or a Lewis acid (e.g., boron bromide, aluminum chloride, or zinc chloride) in an amount of 1 to 100 equivalents by mole relative to compound (1-2) at a temperature ranging from -78°C to the reflux temperature of the reaction mixture for one hour to 48 hours, with optional use of a solvent (e.g., dichloromethane, chloroform, 1,2-dichloroethane, carbon tetrachloride, or a mixture containing two or more of these in any proportions); or reacting compound (1-2) with a base (e.g., sodium hydroxide, potassium hydroxide, or lithium hydroxide) in an amount of 1 to 20 equivalents by mole relative to compound (1-2) at a temperature ranging from 0°C to the reflux temperature of the reaction mixture for one hour to 48 hours, with optional use of a solvent (e.g., methanol, ethanol, normal propanol, isopropanol, normal butanol, 1,4-dioxane, 1,2-dimethoxyethane, tetrahydrofuran, water, or a mixture containing two or more of these in any proportions).

### Production method H

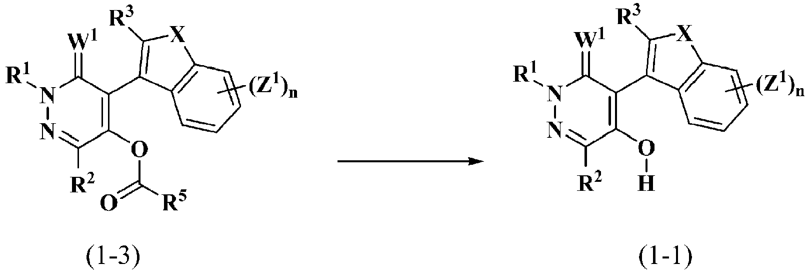

Compound (1-1) can be produced by reacting compound (1-3) with sodium hydroxide, potassium hydroxide, lithium hydroxide, sodium methoxide, or sodium ethoxide in an amount of 1 to 5 equivalents by mole relative to compound (1-3) at a temperature ranging from 0°C to the reflux temperature of the reaction mixture for one hour to 48 hours, with optional use of a solvent (e.g., methanol, ethanol, benzene, toluene, tetrahydrofuran, 1,4-dioxane, acetonitrile, acetone, water, or a mixture containing two or more of these in any proportions).

### Production method I

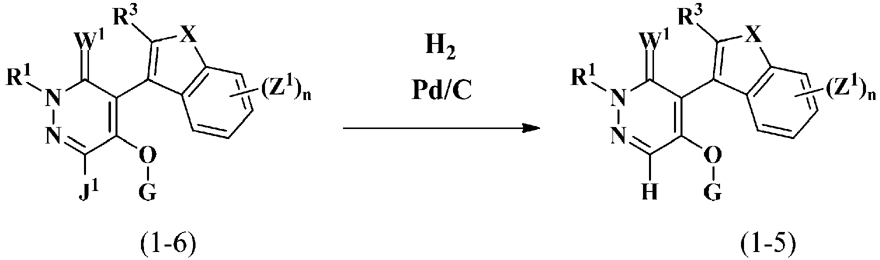

A compound of Formula (1-5) [wherein W¹, X, G, R¹, R³, Z¹, and n have the same meanings as defined above] [hereinafter the compound will be referred to as "compound (1-5)"], which is a type of compound (1) wherein R² is a hydrogen atom, can be produced by reacting a compound of Formula (1-6) [wherein W¹, X, G, R¹, R³, J¹, Z¹, and n have the same meanings as defined above] [hereinafter the compound will be referred to as "compound (1-6)"], which is a type of compound (1) wherein R² is J¹, in a hydrogen atmosphere at a temperature ranging from 0°C to the reflux temperature of the reaction mixture for 30 minutes to 50 hours in the presence of a palladium carbon catalyst in an amount of 0.1 to 3 parts by mass relative to compound (1-6), with use of a solvent (e.g., methanol, ethanol, benzene, toluene, 1,4-dioxane, tetrahydrofuran, dimethoxyethane, ethyl acetate, acetonitrile, N,N-dimethylformamide, acetic acid, water, or a mixture containing two or more of these in any proportions), and with optional use of an acid (e.g., hydrochloric acid, sulfuric acid, or methanesulfonic acid) or a base (e.g., triethylamine, N,N-diisopropylethylamine, or pyridine).

### Production method J

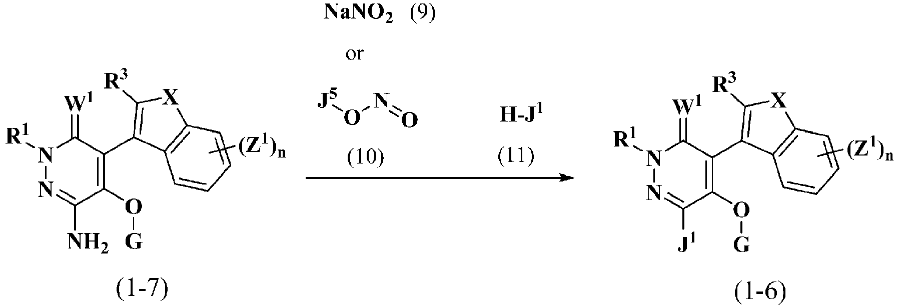

A compound of Formula (1-6) [wherein W¹, X, G, R¹, R³, Z¹, n, and J¹ have the same meanings as defined above] [hereinafter the compound will be referred to as "compound (1-6)"], which is a type of compound (1) wherein R² is J¹, can be produced by reacting a compound of Formula (1-7) [wherein W¹, X, G, R¹, R³, Z¹, and n have the same meanings as defined above] [hereinafter the compound will be referred to as "compound (1-7)"], which is a type of compound (1) wherein R² is -NH₂, with sodium nitrite (9) or a compound of Formula (10) [wherein J⁵ is C₃₋₆ alkyl] [hereinafter the compound will be referred to as "compound (10)"] in an amount of 0.5 to 5 equivalents by mole relative to compound (1-7) and a compound of Formula (11) [wherein J¹ has the same meaning as defined above] [hereinafter the compound will be referred to as "compound (11)"] at a temperature ranging from 0°C to the reflux temperature of the reaction mixture for 30 minutes to 24 hours, with optional use of, for example, copper chloride, copper bromide, copper iodide, or potassium iodide, and with use of a solvent (e.g., methanol, ethanol, 1,4-dioxane, tetrahydrofuran, acetonitrile, dichloromethane, N,N-dimethylformamide, water, or a mixture containing two or more of these in any proportions).

Compounds (9), (10), and (11) used herein are known compounds and some of them are available as commercial products. The other compounds can also be synthesized by synthesis methods for known compounds.

### Production method K

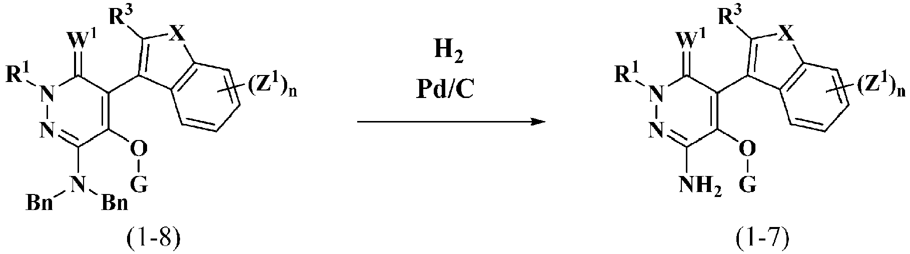

Compound (1-7) can be produced by reacting a compound of Formula (1-8) [wherein W¹, X, G, R¹, R³, Z¹, and n have the same meanings as defined above] [hereinafter the compound will be referred to as "compound (1-8)"], which is a type of compound (1) wherein R² is -N(Bn)₂, in a hydrogen atmosphere at a temperature ranging from 0°C to the reflux temperature of the reaction mixture for 30 minutes to 100 hours in the presence of a palladium carbon catalyst in an amount of 0.1 to 3 parts by mass relative to compound (1-8), with use of a solvent (e.g., methanol, ethanol, benzene, toluene, 1,4-dioxane, tetrahydrofuran, dimethoxyethane, ethyl acetate, acetonitrile, N,N-dimethylformamide, acetic acid, water, or a mixture containing two or more of these in any proportions), and with optional addition of an acid catalyst (e.g., hydrochloric acid, sulfuric acid, or methanesulfonic acid).

### Production method L

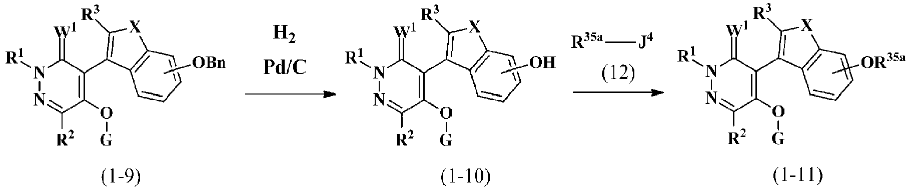

A compound of Formula (1-10) [wherein W¹, G, X, R¹, R², and R³ have the same meanings as defined above] [hereinafter the compound will be referred to as "compound (1-10)"], which is a type of compound (1) wherein Z¹ is a hydroxy group, can be produced by reacting a compound of Formula (1-9) [wherein W¹, G, X, R¹, R², and R³ have the same meanings as defined above] [hereinafter the compound will be referred to as "compound (1-9)"], which is a type of compound (1) wherein at least one Z¹ is -OBn, in a hydrogen atmosphere at a temperature ranging from 0°C to the reflux temperature of the reaction mixture for 30 minutes to 50 hours in the presence of a palladium carbon catalyst in an amount of 0.1 to 3 parts by mass relative to compound (1-9), with use of a solvent (e.g., methanol, ethanol, benzene, toluene, 1,4-dioxane, tetrahydrofuran, dimethoxyethane, ethyl acetate, acetonitrile, N,N-dimethylformamide, acetic acid, water, or a mixture containing two or more of these in any proportions), and with optional use of an acid (e.g., hydrochloric acid, sulfuric acid, or methanesulfonic acid) or a base (e.g., triethylamine, N,N-diisopropylethylamine, or pyridine). Subsequently, a compound of Formula (1-11) [wherein W¹, G, X, R¹, R², R³, and R^{35a} have the same meanings as defined above] [hereinafter the compound will be referred to as "compound (1-11)"] can be produced by reacting compound (1-10) with a compound of Formula (12) [wherein J⁴ has the same meaning as defined above, and R^{35a} corresponds to substituents represented by R³⁵ (except for a hydrogen atom)] [hereinafter the compound will be referred to as "compound (12)"] at a temperature ranging from room temperature to the reflux temperature of the reaction mixture for 30 minutes to 24 hours, with use of a solvent (e.g., tetrahydrofuran, 1,4-dioxane, dichloromethane, chloroform, 1,2-dichloroethane, N,N-dimethylformamide, N,N-dimethylacetamide, dimethyl sulfoxide, acetonitrile, acetone, methyl ethyl ketone, acetonitrile, water, or a mixture containing two or more of these in any proportions), and in the presence of a base (e.g., sodium hydroxide, potassium carbonate, cesium carbonate, sodium hydrogen carbonate, potassium hydrogen carbonate, sodium hydride, potassium hydride, potassium tert-butoxide, triethylamine, tributylamine, diisopropylethylamine, pyridine, 4-(dimethylamino)pyridine, or 1,8-diazabicyclo[5,4,0]-7-undecene) in an amount of 1 to 5 equivalents by mole relative to compound (1-10).

Some of compounds (12) used herein are known compounds and are available as commercial products. The other compounds can also be synthesized by synthesis methods for known compounds.

### Production method M

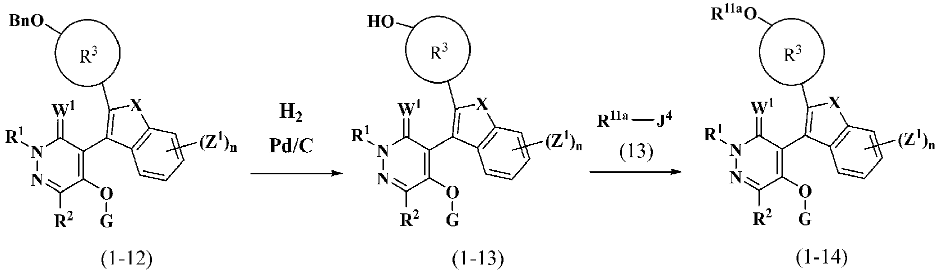

A compound of Formula (1-13) [wherein W¹, Z¹, X, G, n, R¹, R², and R³ have the same meanings as defined above] [hereinafter the compound will be referred to as "compound (1-13)"], which is a type of compound (1) wherein at least one Y¹ is a hydroxy group, can be produced by reacting a compound of Formula (1-12) [wherein W¹, Z¹, X, G, n, R¹, R², and R³ have the same meanings as defined above; note: BnO-, HO-, and R^{11a}O- are originally included in R³, but are shown in the Formulae separately from R³ for the sake of convenience and clarity] [hereinafter the compound will be referred to as "compound (1-12)"], which is a type of compound (1) wherein at least one Y¹ in R³ is -OBn, under the same conditions as in production method L. Subsequently, a compound of Formula (1-14) [wherein W¹, Z¹, X, G, n, R¹, R², R³, and R^{11a} have the same meanings as defined above] [hereinafter the compound will be referred to as "compound (1-14)"], which is a type of compound (1) wherein at least one Y¹ is -OR^{11a}, can be produced by reacting compound (1-13) with a compound of Formula (13) [wherein J⁴ has the same meaning as defined above, and R^{11a} corresponds to substituents represented by R¹¹ (except for a hydrogen atom)] [hereinafter the compound will be referred to as "compound (13)"] in the same manner as in production method L.

Some of compounds (13) used herein are known compounds and are available as commercial products. The other compounds can also be synthesized by synthesis methods for known compounds.

### Production method N

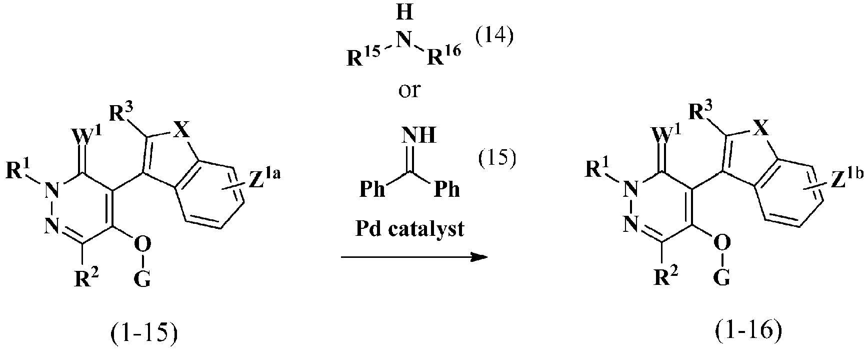

A compound of Formula (1-16) [wherein W¹, G, X, R¹, R², and R³ have the same meanings as defined above, and Z^{1b} is -NR¹⁵R¹⁶ or -N=C(C₆H₅)₂] [hereinafter the compound will be referred to as "compound (1-16)"], which is a type of compound (1) wherein at least one Z¹ is -NR¹⁵R¹⁶ or -N=C(C₆H₅)₂, can be produced by reacting a compound of Formula (1-15) [wherein W¹, X, G, R¹, R², and R³ have the same meanings as defined above , and Z^{1a} is a chlorine atom, a bromine atom, an iodine atom, C₁₋₆ alkylsulfonyloxy or C₁₋₆ haloalkylsulfonyloxy] [hereinafter the compound will be referred to as "compound (1-15)"], which is a type of compound (1) wherein at least one Z¹ is Z^{1a}, with a compound of Formula (14) [wherein R¹⁵ and R¹⁶ have the same meanings as defined above] [hereinafter the compound will be referred to as "compound (14)"] or benzophenoneimine (15) through a method described in, for example, International Publication WO 2013/033228 or WO 2016/102420 in the presence of a palladium catalyst.

Some of compounds (14) and (15) used herein are known compounds and are available as commercial products. The other compounds can also be synthesized by synthesis methods for known compounds.

### Production method O

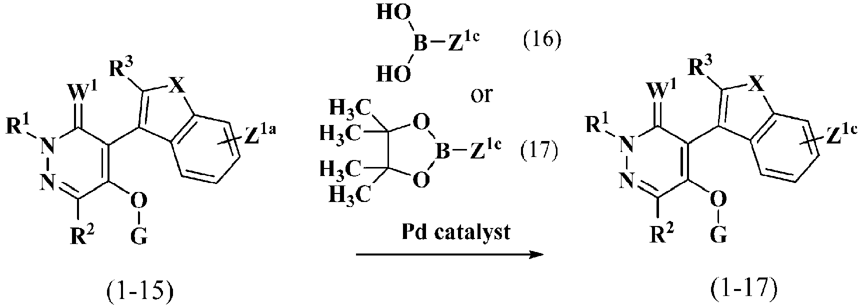

A compound of Formula (1-17) [wherein W¹, X, G, R¹, R², R³, and Z^{1c} have the same meanings as defined above], which is a type of compound (1) wherein at least one Z¹ is Z^{1c}, can be produced by reacting compound (1-15) with a compound of Formula (16) [wherein Z^{1c} is phenyl, phenyl substituted with (Z⁴)_{p5c}, Q-2, Q-3, Q-4, Q-5, or C₃₋₆ cycloalkyl] or a compound of Formula (17) [wherein Z^{1c} has the same meaning as defined above] through a method described in, for example, International Publication WO 2019/178129 or Angewandte Chemie, International Edition, 2018, Vol. 57, page 14198 in the presence of a palladium catalyst.

Some of compounds (16) and (17) used herein are known compounds and are available as commercial products. The other compounds can also be synthesized by synthesis methods for known compounds.

### Production method P

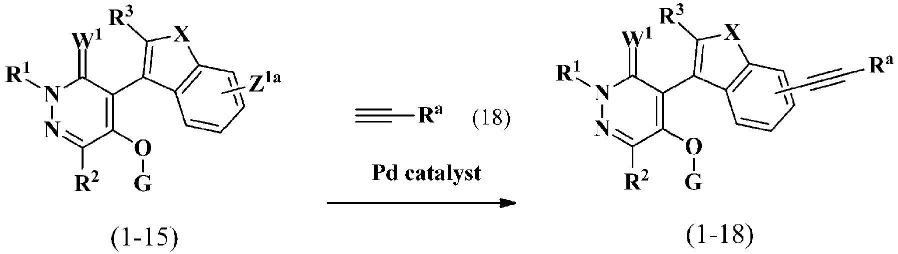

A compound of Formula (1-18) [wherein W¹, X, G, R¹, R², R³, and R^{a} have the same meanings as defined above] [hereinafter the compound will be referred to as "compound (1-18)"] can be produced by reacting compound (1-15) with a compound of Formula (18) [wherein R^{a} is C₁₋₄ alkyl, C₃₋₆ cycloalkyl, or tri(C₁₋₆ alkyl)silyl] [hereinafter the compound will be referred to as "compound (18)"] through a method described in, for example, The Journal of Organic Chemistry, 2007, Vol. 72, page 6672.

Some of compounds (18) used herein are known compounds and are available as commercial products. The other compounds can also be synthesized by synthesis methods for known compounds.

### Production method Q

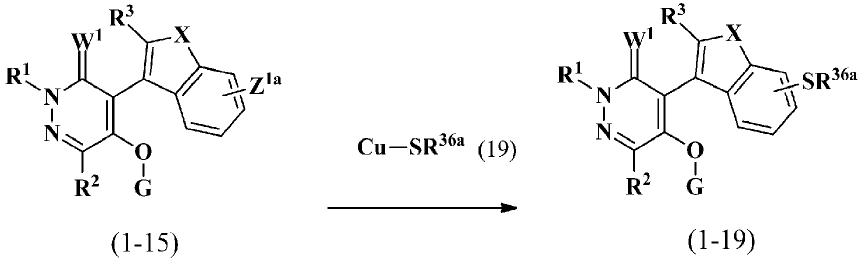

A compound of Formula (1-19) [wherein W¹, X, G, R¹, R², R³, and R^{36a} have the same meanings as defined above] [hereinafter the compound will be referred to as "compound (1-19)"], which is a type of compound (1) wherein at least one Z¹ is -SR^{36a}, can be produced by reacting compound (1-15) with a compound of Formula (19) [wherein R^{36a} is C₁₋₆ haloalkyl] [hereinafter the compound will be referred to as "compound (19)"] through a method described in, for example, U.S. Patent Application Publication No. 6215021.

Some of compounds (19) used herein are known compounds and are available as commercial products. The other compounds can also be synthesized by synthesis methods for known compounds.

### Production method R

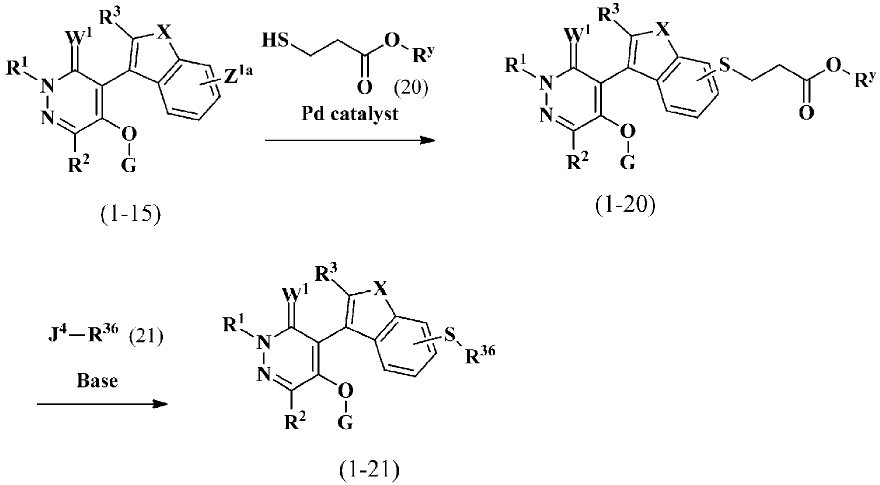

A compound of Formula (1-20) [wherein W¹, X, G, R¹, R², R³, and R^{y} have the same meanings as defined above] [hereinafter the compound will be referred to as "compound (1-20)"] is prepared by reacting compound (1-15) with a compound of Formula (20) [wherein R^{y} is C₁₋₁₀ alkyl] [hereinafter the compound will be referred to as "compound (20)"] through a method described in, for example, International Publication WO 2016/044770 in the presence of a palladium catalyst. Subsequently, a compound of Formula (1-21) [wherein W¹, X, G, R¹, R², R³, and R³⁶ have the same meanings as defined above] [hereinafter the compound will be referred to as "compound (1-21)"], which is a type of compound (1) wherein at least one Z¹ is -SR³⁶, can be produced by reacting compound (1-20) with a compound of Formula (20) [wherein R³⁶ and J⁴ have the same meanings defined above] [hereinafter the compound will be referred to as "compound (20)"] in the presence of a base (e.g., sodium methoxide, sodium ethoxide, or potassium tert-butoxide) in an amount of 1 to 20 equivalents by mole relative to compound (1-20).

Some of compounds (20) and (21) used herein are known compounds and are available as commercial products. The other compounds can also be synthesized by synthesis methods for known compounds.

In production methods A to R, the reaction mixture after completion of the reaction can be subjected to a common post-treatment (e.g., direct concentration; or dissolution in an organic solvent, washing with water, and subsequent concentration; or addition into ice water, extraction with an organic solvent, and subsequent concentration), to thereby yield a pyridazinone compound of interest. When purification is required, the reaction mixture can be purified through separation of impurities by any purification technique, such as recrystallization, column chromatography, thin-layer chromatography, or preparative liquid chromatography.

Compound (2) used in production method A can be synthesized as shown in, for example, reaction scheme 1.

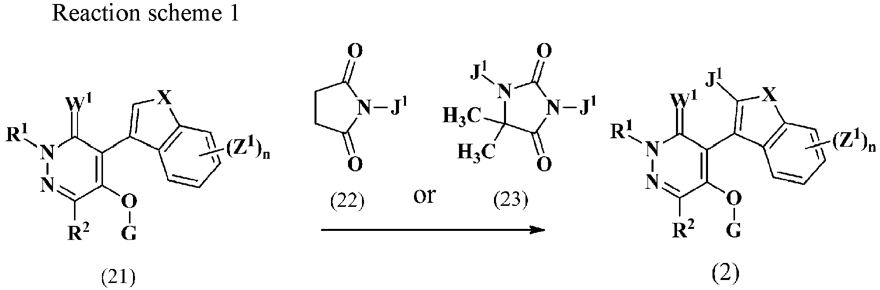

Compound (2) can be produced by reacting a compound of Formula (21) [wherein W¹, G, R¹, R², Z¹, and n have the same meanings as defined above] [hereinafter the compound will be referred to as "compound (21)"] with a compound of Formula (22) [wherein J¹ has the same meaning as defined above] [hereinafter the compound will be referred to as "compound (22)"] or a compound of Formula (23) [wherein J¹ has the same meaning as defined above] [hereinafter the compound will be referred to as "compound (23)"] through a method described in, for example, Chemistry of Heterocyclic Compounds, 2017, Vol. 53, page 1156.

Some of compounds (21) used in reaction scheme 1 are known compounds and can be synthesized by a method described in literature, for example, a method described in International Publication WO 2015/168010 or WO 2017/074992.

Compound (22) or (23) used in reaction scheme 1 is also a known compound and is available as a commercial product.

Compound (8) used in production method F can be synthesized as shown in reaction scheme 2 or 3, and compound (8-1a), which is a type of compound (8) wherein R¹ is R^{1a}, can be synthesized as shown in reaction scheme 4.

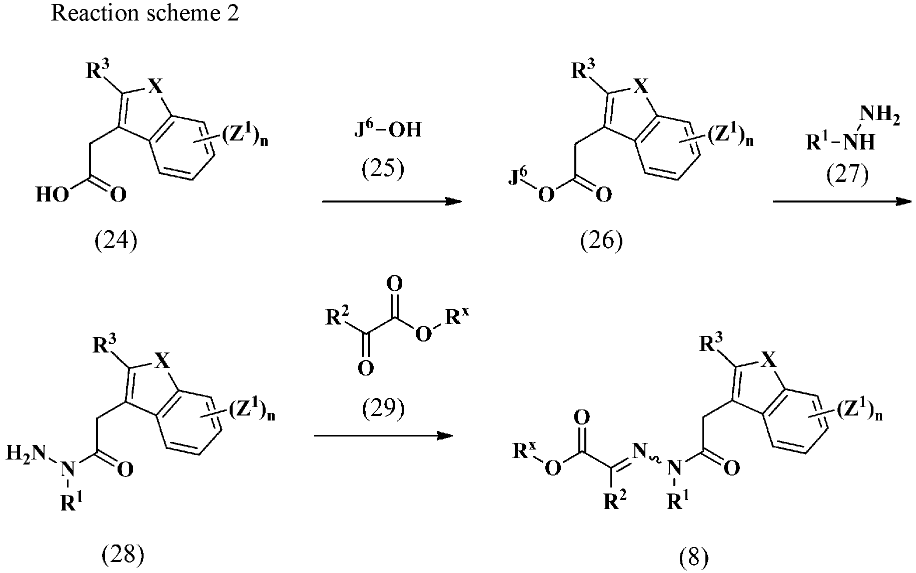

A compound of Formula (26) [wherein X, R³, Z¹, n, and J⁶ have the same meanings as defined above] [hereinafter the compound will be referred to as "compound (26)"] is prepared by reacting a compound of Formula (24) [wherein X, R³, Z¹, and n have the same meanings as defined above] [hereinafter the compound will be referred to as "compound (24)"] with a compound of Formula (25) [wherein J⁶ is pentafluorophenyl or 2,4,6-trichlorophenyl] [hereinafter the compound will be referred to as "compound (25)"] through a method described in, for example, International Publication WO 2017/074992 with use of a condensing agent such as N-(3-dimethylaminopropyl)-N'-ethylcarbodiimide hydrochloride. Subsequently, a compound of Formula (28) [wherein X, R¹, R³, Z¹, and n have the same meanings as defined above] [hereinafter the compound will be referred to as "compound (28)"] can be produced by reacting compound (26) with a hydrazine derivative of Formula (27) [wherein R¹ has the same meaning as defined above] or a salt thereof [e.g., hydrochloride, bromate, or sulfate, hereinafter referred to as "compound (27)"]. Subsequently, compound (8) can be produced by reacting compound (28) with a compound of Formula (29) [wherein R² and R^{x} have the same meanings as defined above] [hereinafter the compound will be referred to as "compound (29)"].

Some of compounds (25), (27), and (29) used in reaction scheme 2 are known compounds and are available as commercial products. The other compounds can also be synthesized by synthesis methods for known compounds.

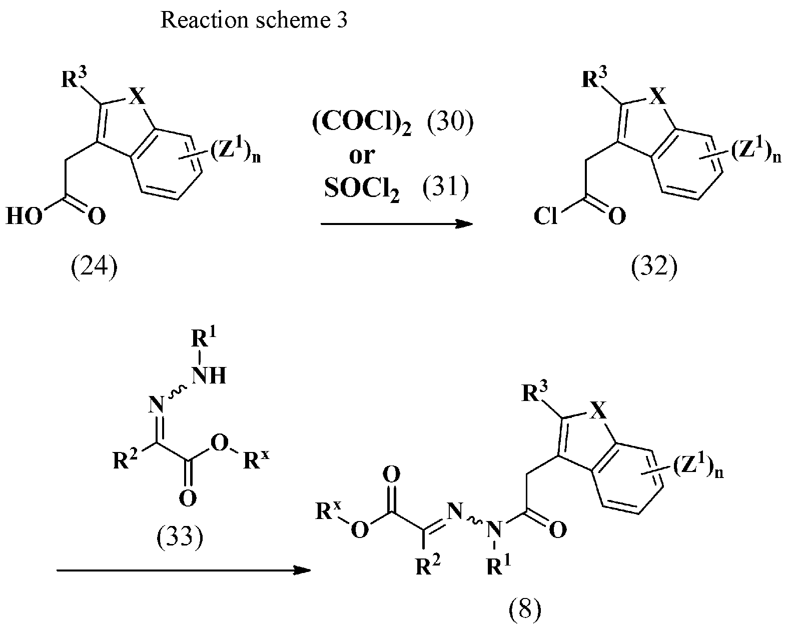

A compound of Formula (32) [wherein X, R³, Z¹, and n have the same meanings as defined above] [hereinafter the compound will be referred to as "compound (32)"] is prepared by reacting compound (24) with oxalyl chloride (30) or thionyl chloride (31) in an amount of 1 to 20 equivalents by mole relative to compound (24) at 0°C to the reflux temperature of the reaction mixture for 30 minutes to 24 hours, with optional use of a solvent (e.g., dichloromethane, chloroform, 1,2-dichloroethane, toluene, xylene, or a mixture containing two or more of these in any proportions), and with optional addition of N,N-dimethylformamide. Subsequently, compound (8) can be produced by reacting compound (32) with a compound of Formula (33) [wherein R¹, R², and R^{X} have the same meanings as defined above] [hereinafter the compound will be referred to as "compound (33)"] at 0°C to the reflux temperature of the reaction mixture for 30 minutes to 24 hours, with use of a solvent (e.g., dichloromethane, chloroform, 1,2-dichloroethane, pyridine, toluene, xylene, ethyl acetate, butyl acetate, heptane, 2-butanone, water, or a mixture containing two or more of these in any proportions), and optionally in the presence of a base (e.g., triethylamine, diisopropylethylamine, pyridine, 4-dimethylaminopyridine, sodium carbonate, sodium hydrogen carbonate, potassium carbonate, potassium hydrogen carbonate, cesium carbonate, sodium hydroxide, or potassium hydroxide).

Some of compounds (33) used herein are known compounds, and the other compounds can also be synthesized by synthesis methods for known compounds; for example, a method described in Nature Communications, 2017, Vol. 8, page 1 or International Publication WO 2012/091156.

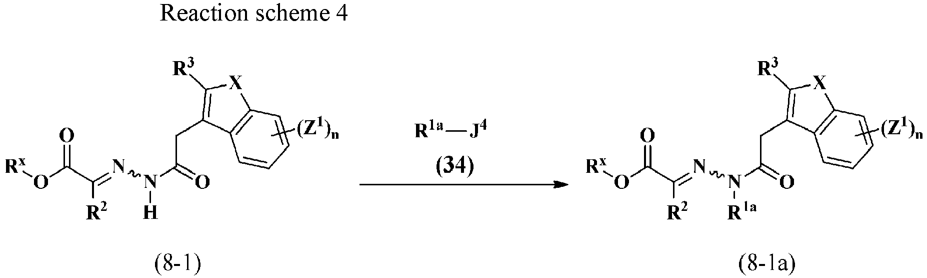

A compound of Formula (8-1a) [hereinafter the compound will be referred to as "compound (8-1a)"], which is a type of compound (8) wherein R¹ is R^{1a}, can be synthesized by reacting a compound of Formula (8-1) [hereinafter the compound will be referred to as "compound (8-1)"], which is a type of compound (8) wherein R¹ is a hydrogen atom, with a compound of Formula (34) [wherein R^{1a} is C₁₋₆ alkyl, C₁₋₆ alkenyl, C₁₋₆ alkynyl, or C₁₋₆ alkyl substituted with R³⁴, and R³⁴ and J⁴ have the same meanings as defined above] [hereinafter the compound will be referred to as "compound (34)"] through a method described in, for example, Advanced Synthesis & Catalysis, 2016, Vol. 358, page 276.

Compound (8-1) used herein can be synthesized by the method shown in reaction scheme 2 or 3. Some of compounds (34) are known compounds and are available as commercial products. The other compounds can also be synthesized by synthesis methods for known compounds.

Some of compounds (24) used in reaction schemes 2 and 3 are known compounds and are available as commercial products. The other compounds can be synthesized by a method shown in, for example, reaction scheme 5, 6, or 7.

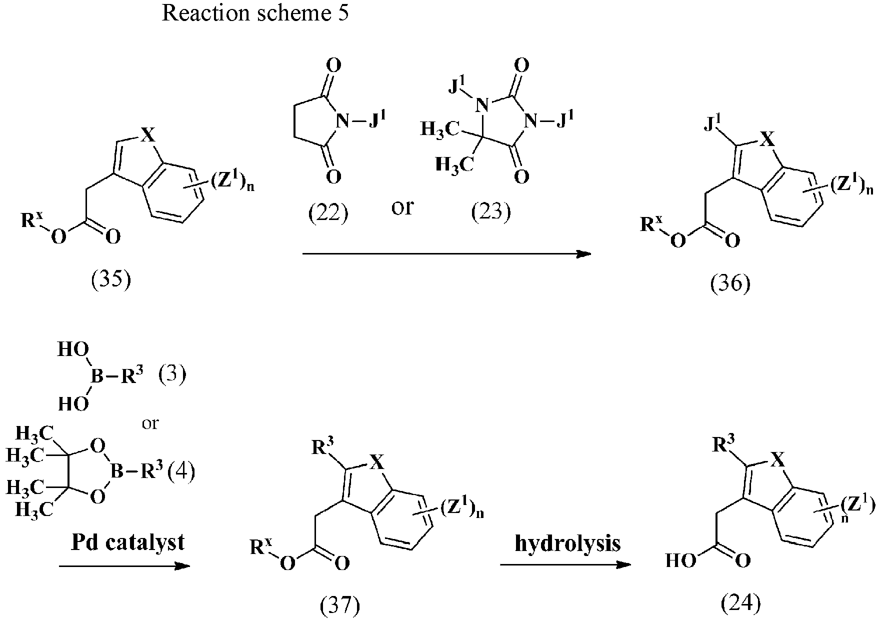

A compound of Formula (36) [wherein X, R^{X}, Z¹, J¹, and n have the same meanings as defined above] [hereinafter the compound will be referred to as "compound (36)"] can be produced by reacting a compound of Formula (35) [wherein X, R^{x}, Z¹, and n have the same meanings as defined above] [hereinafter the compound will be referred to as "compound (35)"] with compound (22) or compound (23) in the same manner as shown in reaction scheme 1. Subsequently, a compound of Formula (37) [wherein X, R³, R^{x}, Z¹, and n have the same meanings as defined above] [hereinafter the compound will be referred to as "compound (37)"] can be produced by reacting with compound (36) with compound (3) or compound (4) in the same manner as in production method A. Subsequently, compound (24) can be produced by reacting compound (37) with an aqueous solution of an alkali metal (e.g., sodium hydroxide, potassium hydroxide, or lithium hydroxide) through a method described in, for example, International Publication WO 2013/009259.

Some of compounds (35) used in reaction scheme 5 are known compounds and are available as commercial products. The other compounds can also be synthesized by synthesis methods for known compounds. The synthesis method for known compounds is described in, for example, Der Pharma Chemica, 2017, Vol. 9, page 85 and Organic Letters, 2008, Vol. 10, page 573.

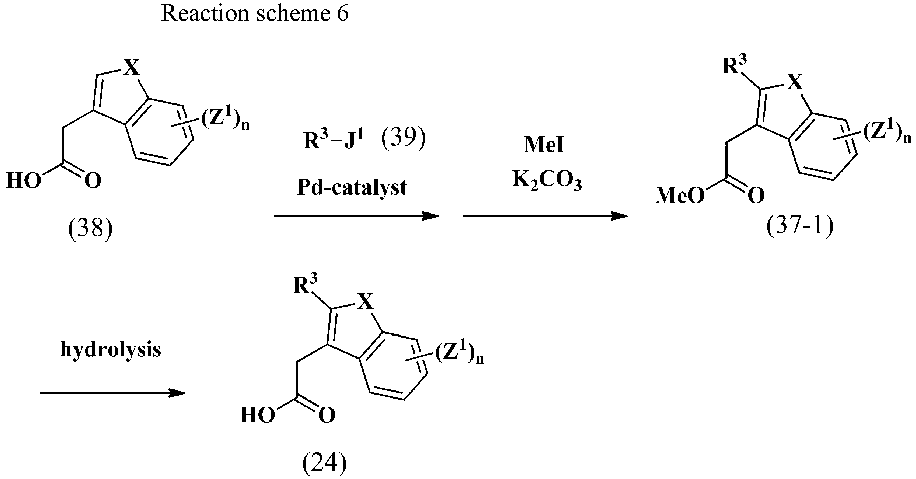

A compound of Formula (37-1) [wherein X, Z¹, R³, and n have the same meanings as defined above] [hereinafter the compound will be referred to as "compound (37-1)"], which is a type of compound (37) wherein R^{x} is methyl, can be produced by reacting a compound of Formula (38) [wherein X, Z¹, and n have the same meanings as defined above] [hereinafter the compound will be referred to as "compound (38)"] with a compound of Formula (39) [wherein R³ and J¹ have the same meanings as defined above] [hereinafter the compound will be referred to as "compound (39)"] in the presence of a palladium catalyst, and by treating the reaction mixture with methyl iodide and potassium carbonate through a synthesis method for a known compound; for example, a method described in Chemistry Letters, 2011, Vol. 40, page 1015. Subsequently, compound (24) can be produced through hydrolysis of compound (37-1) in the same manner as shown in reaction scheme 5.

Some of compounds (38) used in reaction scheme 6 are known compounds and are available as commercial products. The other compounds can also be synthesized by synthesis methods for known compounds. The synthesis method for known compounds is described in, for example, Heterocycles, 1995, Vol. 41, page 647.

Some of compounds (39) are known compounds and are available as commercial products. The other compounds can also be synthesized by synthesis methods for known compounds.

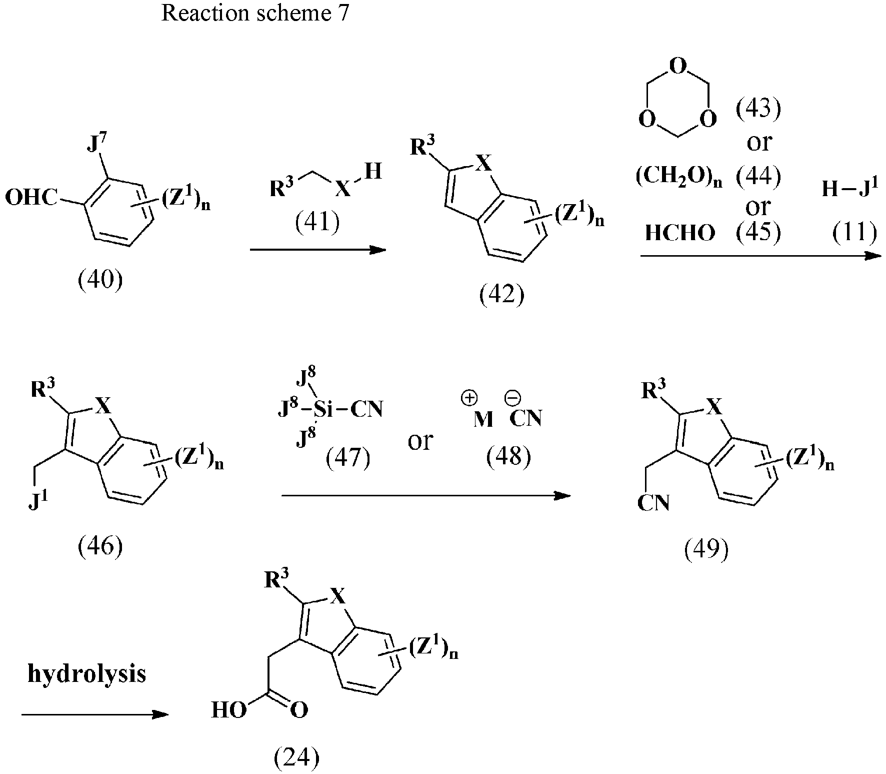

A compound of Formula (42) [wherein X, R³, Z¹, and n have the same meanings as defined above] [hereinafter the compound will be referred to as "compound (42)"] can be produced by reacting a compound of Formula (40) [wherein Z¹ and n have the same meanings as defined above, and J⁷ is a fluorine atom, a chlorine atom, a bromine atom, or an iodine atom] [hereinafter the compound will be referred to as "compound (40)"] with a compound of Formula (41) [wherein X and R³ have the same meanings as defined above] [hereinafter the compound will be referred to as "compound (41)"] in the presence of a base (e.g., sodium hydroxide, potassium hydroxide, sodium carbonate, or potassium carbonate) through a synthesis method for a known compound; for example, a method described in Tetrahedron Letters, 2003, Vol. 44, page 6665. Subsequently, a compound of Formula (46) [wherein X, R³, J¹, Z¹, and n have the same meanings as defined above] [hereinafter the compound will be referred to as "compound (46)"] can be produced by reacting compound (42) with a formaldehyde equivalent such as 1,3,5-trioxane (43), paraformaldehyde (44), or with formaldehyde (45) in the presence of compound (11) through a method described in, for example, Journal of the American Chemical Society, 1948, Vol. 70, page 3768. Subsequently, a compound of Formula (49) [wherein X, R³, Z¹, and n have the same meanings as defined above] [hereinafter the compound will be referred to as "compound (49)"] can be produced by reacting compound (46) with a compound of Formula (47) [wherein J⁸ is methyl or ethyl] [hereinafter the compound will be referred to as "compound (47)"] or a compound of Formula (48) [wherein M is an alkali metal such as sodium or potassium] [hereinafter the compound will be referred to as "compound (48)"] through a method described in, for example, Journal of Heterocyclic Chemistry, 1965, Vol. 2, page 231. Subsequently, compound (24) can be produced by reacting compound (49) with an aqueous solution of an alkali metal (e.g., sodium hydroxide, potassium hydroxide, or lithium hydroxide) or an acidic compound (e.g., hydrochloric acid, sulfuric acid, or acetic acid) through a method described in, for example, International Publication WO 2002/016353.

Some of compounds (40), (41), (47), and (48) used in reaction scheme 7 are known compounds and are available as commercial products. The other compounds can also be synthesized by synthesis methods for known compounds.

A production intermediate serving as a raw material compound for each of reaction schemes 1 to 7 can be produced by performing a common post-treatment after completion of the aforementioned reaction.

Each of the production intermediates produced by such a method can be used for the reaction in the subsequent step without isolation or purification.

Examples of the pyridazinone compound of Formula (1) of the present invention, which can be produced by any of the aforementioned methods, include compounds shown in Table 1. However, Table 1 is merely provided for illustration, and the pyridazinone compound of the present invention is not limited to examples shown in Table 1.

In Table 1, Me denotes methyl; Et, ethyl; n-Pr or Pr-n, normal propyl; i-Pr or Pr-i, isopropyl; c-Pr or Pr-c, cyclopropyl; n-Bu or Bu-n, normal butyl; i-Bu or Bu-i, isobutyl; sec-Bu or Bu-sec, secondary butyl; tert-Bu or Bu-tert, tertiary butyl; c-Bu or Bu-c, cyclobutyl; c-Pen or Pen-c, cyclopentyl; and c-Hex or Hex-c, cyclohexyl.

In Table 1, D-1 to D-56 correspond to the following structures.

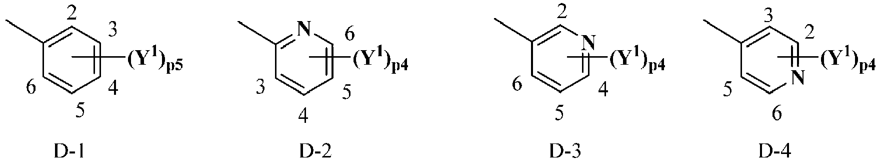

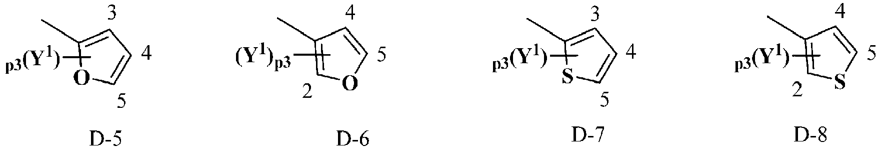

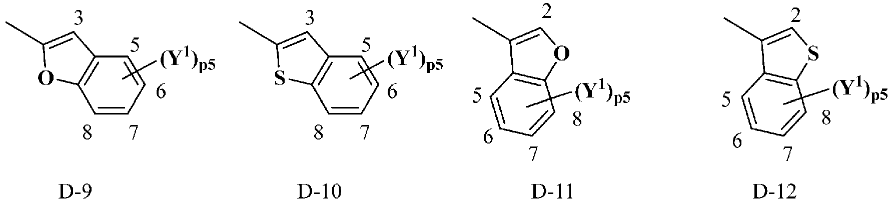

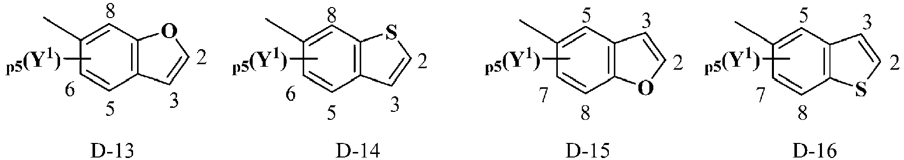

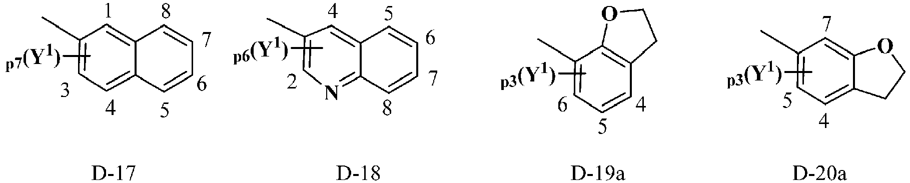

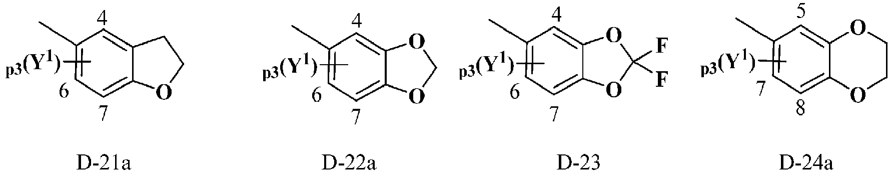

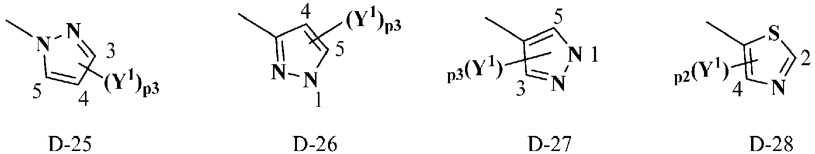

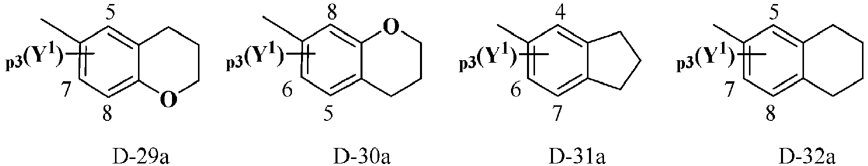

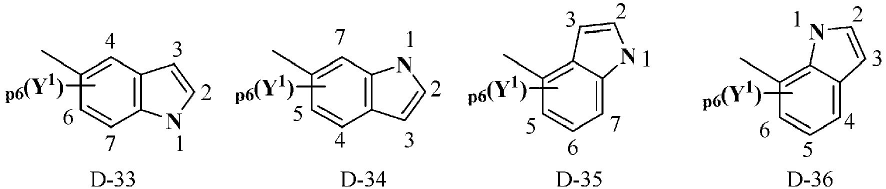

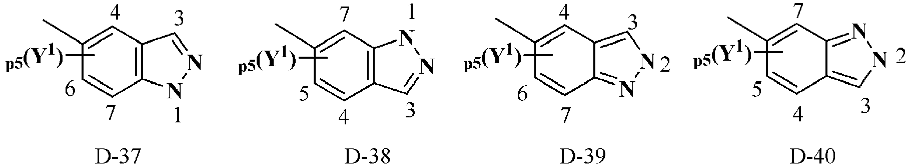

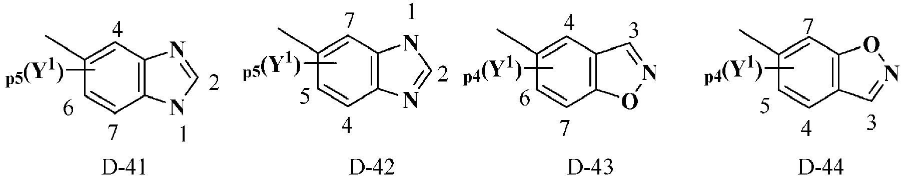

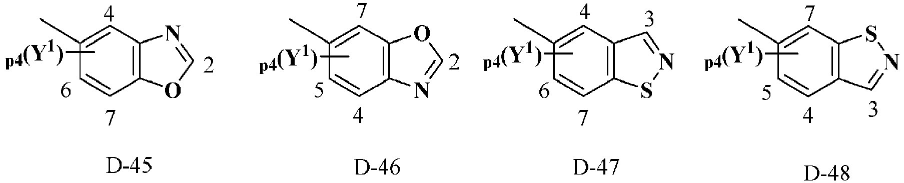

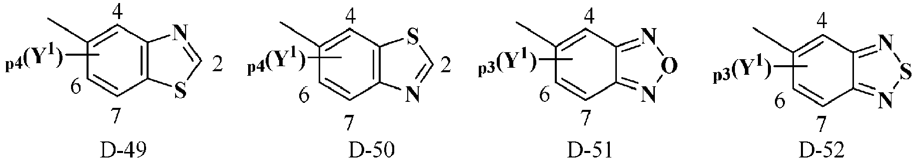

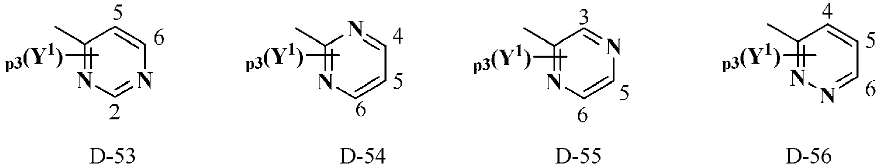

In Table 1, U-1a to U-32a, T-1-1 to T-1-5, and Q-1-1 to Q-35-1 correspond to the following structures.

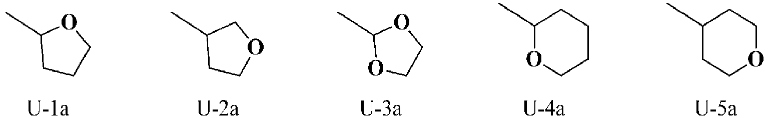

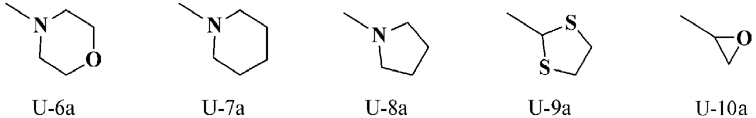

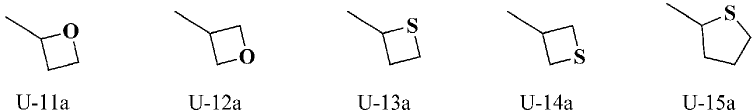

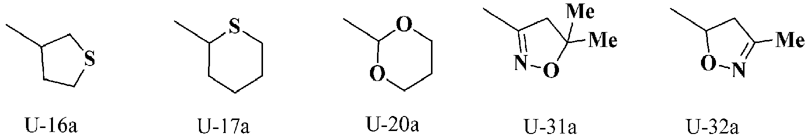

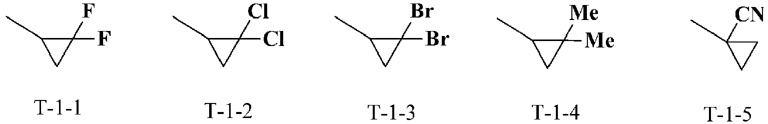

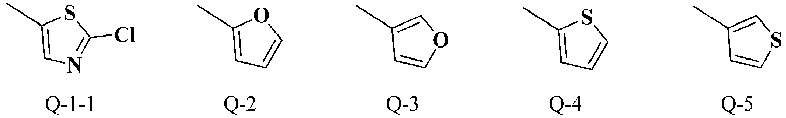

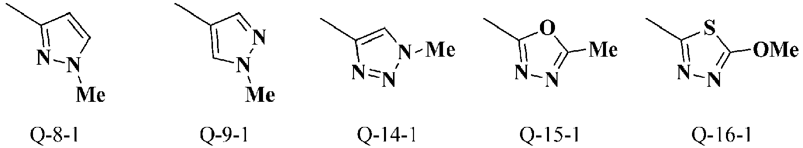

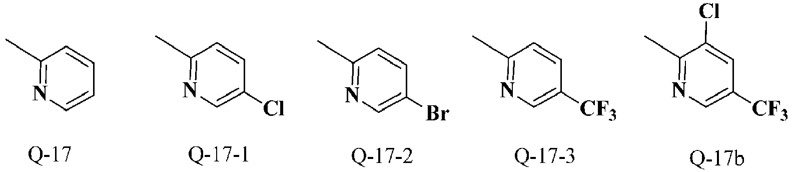

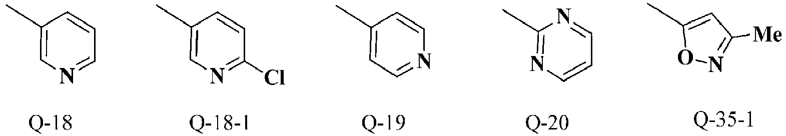

The expression "(Y¹)" in Table 1 refers to (Y¹)ₚ₇, (Y¹)ₚ₆, (Y¹)ₚ₅, (Y1)_{P4}, (Y¹)_{P3}, or (Y¹)_{P2} corresponding to each of the aforementioned structures of D-1 to D-56 specified in the column of R³. The substitution position number corresponds to the numbered position in each of the aforementioned structural formulae. The expression "-" in the column of (Z¹)ₙ and "(Y¹)" refers to no substitution.

**Table 1**

| | | | |
|---|---|---|---|
| 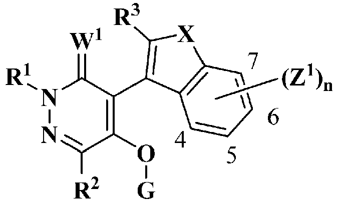 | | | |
| | R¹ is Me, R² is Me, W¹ is O, G is H, X is S, and (Z¹)ₙ is - (no substitution) | | |

| R³ | [(Y¹)] | R³ | [(Y¹)] |
|---|---|---|---|
| D-1 | [-] | D-1 | [2-F] |
| D-1 | [3-F] | D-1 | [4-F] |
| D-1 | [2-Cl] | D-1 | [3-Cl] |
| D-1 | [4-Cl] | D-1 | [2-Me] |
| D-1 | [3-Me] | D-1 | [4-Me] |
| D-1 | [2-OMe] | D-1 | [3-OMe] |
| D-1 | [4-OMe] | D-1 | [2,4-F_{2]} |
| D-1 | [2-F-4-Me] | D-1 | [2-F-4-OMe] |
| D-1 | [2-Cl-4-F] | D-1 | [2,4-Cl₂] |
| D-1 | [2-Cl-4-Me] | D-1 | [2-Cl-4-OMe] |
| D-2 | [3-Cl] | D-2 | [4-Cl] |
| D-2 | [5-Cl] | D-2 | [6-Cl] |
| D-2 | [3-OMe] | D-2 | [4-OMe] |
| D-2 | [5-OMe] | D-2 | [6-OMe] |
| D-3 | [2-Cl] | D-3 | [4-Cl] |
| D-3 | [5-Cl] | D-3 | [6-Cl] |
| D-3 | [2-OMe] | D-3 | [4-OMe] |
| D-3 | [5-OMe] | D-3 | [6-OMe] |
| D-4 | [2-Cl] | D-4 | [3-Cl] |
| D-4 | [2-OMe] | D-4 | [3-OMe] |
| D-1 | [2-Et] | D-1 | [2-Pr-n] |
| D-1 | [3-Et] | D-1 | [3-Pr-n] |
| D-1 | [4-Et] | D-1 | [4-Pr-n] |
| D-1 | [2-Pr-i] | D-1 | [2-Bu-n] |
| D-1 | [3-Pr-i] | D-1 | [3-Bu-n] |
| D-1 | [4-Pr-i] | D-1 | [4-Bu-n] |
| D-1 | [4-CH₂F] | D-1 | [2-CHF₂] |
| D-1 | [4-CH₂Cl] | D-1 | [3-CHF₂] |
| D-1 | [4-CH₂Br] | D-1 | [4-CHF₂] |
| D-1 | [2-CF₃] | D-1 | [2-CH=CH₂] |
| D-1 | [3-CF₃] | D-1 | [3-CH=CH₂] |
| D-1 | [4-CF₃] | D-1 | [4-CH=CH₂] |
| D-1 | [2-CH₂CH=CH₂] | D-1 | [2-CH=CF₂] |
| D-1 | [3-CH₂CH=CH₂] | D-1 | [3-CH=CF₂] |
| D-1 | [4-CH₂CH=CH₂] | D-1 | [4-CH=CF₂] |
| D-1 | [2-CH=CCl₂] | D-1 | [2-CH=CF₂] |
| D-1 | [3-CH=CCl₂] | D-1 | [3-CH=CF₂] |
| D-1 | [4-CH=CCl₂] | D-1 | [4-CH=CF₂] |
| D-1 | [4-C=CH] | D-1 | [4-C=CMe] |
| D-1 | [4-C=CBu-tert] | D-1 | [4-C≡CCl] |
| D-1 | [4-Pr-c] | D-1 | [4-(T-1-1)] |
| D-1 | [T-1-2] | D-1 | [4-(T-1-3)] |
| D-1 | [T-1-4] | D-1 | [4-(T-1-5)] |
| D-1 | [2-CH₂OH] | D-1 | [3-CH₂OH] |
| D-1 | [4-CH₂OH] | D-1 | [2-CH₂OMe] |
| D-1 | [3-CH₂OMe] | D-1 | [4-CH₂OMe] |
| D-1 | [4-CH₂OEt] | D-1 | [4-CH₂OPr-n] |
| D-1 | [4-CH₂OPr-i] | D-1 | [4-CH₂OBu-n] |
| D-1 | [4-CH₂OBu-i] | D-1 | [4-CH₂OBu-sec] |
| D-1 | [4-CH₂OCH₂CH=CH₂] | D-1 | [4-CH₂OCH₂C(Me)=CH₂] |
| D-1 | [4-CH₂OCH₂CH=CHMe] | D-1 | [4-CH₂OCH(Me)CH=CH₂] |
| D-1 | [4-CH₂OCH₂CH=C(Me)₂] | | D-1 [4-CH₂OC(Me)₂CH=CH₂] |
| D-1 | [4-CH₂OCH₂C≡CH] | D-1 | [4-CH₂OCH(Me)C≡CH] |
| D-1 | [4-CH₂OCH₂C≡CMe] | D-1 | [4-CH₂OC(Me)₂C≡CH] |
| D-1 | [4-CH₂OCH₂CH₂C≡CH] | D-1 | [4-CH₂OC(Me)(Et)C≡CH] |
| D-1 | [2-CH₂OCHF₂] | D-1 | [3-CH₂OCHF₂] |
| D-1 | [4-CH₂OCHF₂] | D-1 | [4-CH₂OCH₂CH₂F] |
| D-1 | [4-CH₂OCH₂CHF₂] | D-1 | [4-CH₂OCH₂CF₃] |
| D-1 | [4-CH₂OCH₂CH₂Cl] | D-1 | [4-CH₂OCH₂CF₂CHF₂] |
| D-1 | [4-CH₂OCH₂CH₂Br] | D-1 | [4-CH₂OCH₂CF₂CF₃] |
| D-1 | [4-CH₂OCH₂CH=CF₂] | D-1 | [4-CH₂OCH₂C(Br)=CH₂] |
| D-1 | [4-CH₂OCH₂CH=CHCl] | D-1 | [4-CH₂OCH₂CH₂CH=CF₂] |
| D-1 | [4-CH₂OCH₂CH=CCl₂] | D-1 | [4-CH₂OCH₂CH₂CF=CF₂] |
| D-1 | [4-CH₂OCH₂C≡CCl] | D-1 | [4-CH₂OCH₂CH₂C≡CCl] |
| D-1 | [4-CH₂OCH₂C≡CBr] | D-1 | [4-CH₂OCH₂CH₂C≡CBr] |
| D-1 | [4-CH₂OCH₂C≡CCl] | D-1 | [4-CH₂OCH₂CH₂C≡CI] |
| D-1 | [4-CH₂OPen-c] | D-1 | [2-CH₂OHex-c] |
| D-1 | [4-CH₂OCH₂CH₂OMe] | D-1 | [4-CH₂OCH(Me)CH₂OMe] |
| D-1 | [4-CH₂OCH₂CH₂OEt] | D-1 | [4-CH₂OCH₂CH(Me)OMe] |
| D-1 | [4-CH₂OCH₂CH₂SMe] | D-1 | [4-CH₂OCH(Me)CH₂SMe] |
| D-1 | [4-CH₂OCH₂CH₂SEt] | D-1 | [4-CH₂OCH₂CH(Me)SMe] |
| D-1 | [4-CH₂OCH₂CN] | D-1 | [4-CH₂OCH₂CH₂OCH₂CH₂F] |
| D-1 | [4-CH₂OCH₂CH₂CN] | D-1 | [4-CH₂OCH₂CH₂OCH₂CHF₂] |
| D-1 | [4-CH₂OCH(Me)CN] | D-1 | [4-CH₂OCH₂CH₂OCH₂CF₃] |
| D-1 | [4-CH₂OCH₂(U-1a)] | D-1 | [4-CH₂OCH₂CH₂OCH₂CH₂Cl] |
| D-1 | [4-CH₂OCH₂CH₂(U-1a)] | D-1 | [4-CH₂OCH₂CH₂OCH₂CHCl₂] |
| D-1 | [4-CH₂OCH₂(U-3a)] | D-1 | [4-CH₂OCH₂CH₂OCH₂CHCl₃] |
| D-1 | [4-CH₂OCH₂CH₂(U-3a)] | D-1 | [4-CH₂OCH₂CH(Me)S(O)Me] |
| D-1 | [4-CH₂OCH₂(U-9a)] | D-1 | [4-CH₂OCH₂CH(Me)S(O)₂Me] |
| D-1 | [4-CH₂OCH₂CH₂(U-9a)] | D-1 | [4-CH₂OCH(Me)CH₂S(O)Me] |
| D-1 | [4-CH₂OCH₂Pr-c] | D-1 | [4-CH₂OCH(Me)CH₂S(O)₂Me] |
| D-1 | [4-CH₂OCH₂Bu-c] | D-1 | [4-CH₂OCH₂CH₂S(O)Me] |
| D-1 | [4-CH₂OCH₂Pen-c] | D-1 | [4-CH₂OCH₂CH₂S(O)₂Me] |
| D-1 | [4-CH₂OCH₂Hex-c] | D-1 | [4-CH₂OCH₂CH₂SCH₂CH₂F] |
| D-1 | [4-CH₂OCH₂(T-1-1)] | D-1 | [4-CH₂OCH₂CH₂SCH₂CHF₂] |
| D-1 | [4-CH₂OCH₂(T-1-2)] | D-1 | [4-CH₂OCH₂CH₂SCH₂CF₃] |
| D-1 | [4-CH₂OCH₂(T-1-3)] | D-1 | [4-CH₂OCH₂CH₂SCH₂CH₂Cl] |
| D-1 | [4-CH₂OCH₂(T-1-4)] | D-1 | [4-CH₂OCH₂CH₂SCH₂CHCl₂] |
| D-1 | [4-CH₂OCH₂(T-1-5)] | D-1 | [4-CH₂OCH₂CH₂SCH₂CCl₃] |
| D-1 | [4-CH₂SH] | D-1 | [4-CH₂OCH₂CH₂S(O)CH₂CH₂F] |
| D-1 | [4-CH₂SMe)] | D-1 | [4-CH₂OCH₂CH₂S(O)CH₂CHF₂] |
| D-1 | [4-CH₂SEt] | D-1 | [4-CH₂OCH₂CH₂S(O)CH₂CF₃] |
| D-1 | [4-CH₂SPr-n] | D-1 | [4-CH₂OCH₂CH₂S(O)₂CH₂CH₂F] |
| D-1 | [4-CH₂SPr-i] | D-1 | [4-CH₂OCH₂CH₂S(O)₂CH₂CHF₂] |
| D-1 | [4-CH₂S(O)Me] | D-1 | [4-CH₂OCH₂CH₂S(O)₂CH₂CF₃] |
| D-1 | [4-CH₂S(O)Et] | D-1 | [4-CH₂SCH₂CH=CH₂] |
| D-1 | [4-CH₂S(O)Pr-n] | D-1 | [4-CH₂S(O)CH₂CH=CH₂] |
| D-1 | [4-CH₂S(O)Pr-i] | D-1 | [4-CH₂S(O)₂CH₂CH=CH₂] |
| D-1 | [4-CH₂S(O)₂Me] | D-1 | [4-CH₂SCH₂CH=CCl₂] |
| D-1 | [4-CH₂S(O)₂Et] | D-1 | [4-CH₂S(O)CH₂CH=CCl₂] |
| D-1 | [4-CH₂S(O)₂Pr-n] | D-1 | [4-CH₂S(O)₂CH₂CH=CCl₂] |
| D-1 | [4-CH₂S(O)₂Pr-i] | D-1 | [4-CH₂SCH₂C(Br)=CH₂] |
| D-1 | [4-SCH₂SCH₂C≡CH] | D-1 | [4-CH₂S(O)CH₂C(Br)=CH₂] |
| D-1 | [4-SCH₂S(O)CH₂C≡CH] | D-1 | [4-CH₂S(O)₂CH₂C(Br)=CH₂] |
| D-1 | [4-SCH₂SPen-c] | D-1 | [4-CH₂S(O)CH₂C≡CH] |
| D-1 | [4-SCH₂S(O)Pen-c] | D-1 | [4-CH₂S(O)₂CH₂C≡CH] |
| D-1 | [4-SCH₂S(O)₂Pen-c] | D-1 | [4-CH₂SCH₂C≡CCl] |
| D-1 | [4-SCH₂SHex-c] | D-1 | [4-CH₂S(O)CH₂C≡CCl] |
| D-1 | [4-SCH₂S(O)Hex-c] | D-1 | [4-CH₂S(O)₂CH₂C≡CCl] |
| D-1 | [4-SCH₂S(O)₂Hex-c] | D-1 | [4-CH₂S(T-1-5)] |
| D-1 | [4-SCH₂S(O)(T-1-5)] | D-1 | [4-CH₂S(O)₂(T-1-5)] |
| D-1 | [4-CH₂SCH₂CH₂OMe] | D-1 | [4-CH₂S(O)₂CH₂CH₂OMe] |
| D-1 | [4-CH₂SCH₂CH₂OEt] | D-1 | [4-CH₂S(O)₂CH₂CH₂OEt] |
| D-1 | [4-CH₂S(O)CH₂CH₂OMe] | D-1 | [4-CH₂S(O)CH(Me)CH₂OMe] |
| D-1 | [4-CH₂S(O)CH₂CH₂OEt] | D-1 | [4-CH₂S(O)₂CH(Me)CH₂OMe] |
| D-1 | [4-CH₂SCH(Me)CH₂OMe] | D-1 | [4-CH₂S(O)CH₂CH(Me)OMe] |
| D-1 | [4-CH₂SCH₂CH(Me)OMe] | D-1 | [4-CH₂S(O)₂CH₂CH(Me)OMe] |
| D-1 | [4-CH₂SCH₂CH₂SMe] | D-1 | [4-CH₂S(O)₂CH₂CH₂SMe] |
| D-1 | [4-CH₂S(O)CH₂CH₂SMe] | D-1 | [4-CH₂SCH₂CH₂S(O)₂Me] |
| D-1 | [4-CH₂SCH₂CH₂S(O)Me] | D-1 | [4-CH₂S(O)₂CH₂CH₂S(O)₂Me] |
| D-1 | [4-CH₂SCH₂(U-1a)] | D-1 | [4-CH₂SCH₂CH₂SCH₂CF₃] |
| D-1 | [4-CH₂S(O)CH₂(U-1a)] | D-1 | [4-CH₂SCH₂CH₂S(O)CH₂CF₃] |
| D-1 | [4-CH₂S(O)₂CH₂(U-1a)] | D-1 | [4-CH₂SCH₂CH₂S(O)₂CH₂CF₃] |
| D-1 | [4-CH₂SCH₂(U-3a)] | D-1 | [4-CH₂S(O)CH₂CH₂SCH₂CF₃] |
| D-1 | [4-CH₂S(O)CH₂(U-3a)] | D-1 | [4-CH₂S(O)CH₂CH₂S(O)CH₂CF₃] |
| D-1 | [4-CH₂S(O)₂CH₂(U-3a)] | D-1 | [4-CH₂S(O)CH₂CH₂S(O)₂CH₂CF₃] |
| D-1 | [4-CH₂SCH₂(U-9a)] | D-1 | [4-CH₂S(O)₂CH₂CH₂SCH₂CF₃] |
| D-1 | [4-CH₂S(O)CH₂(U-9a)] | D-1 | [4-CH₂S(O)₂CH₂CH₂S(O)CH₂CF₃] |
| D-1 | [4-CH₂S(O)₂CH₂(U-9a)] | D-1 | [4-CH₂S(O)₂CH₂CH₂S(O)₂CH₂CF₃] |
| D-1 | [4-CH₂SCH₂Pr-c] | D-1 | [4-CH₂SCH₂CN] |
| D-1 | [4-CH₂S(O)CH₂Pr-c] | D-1 | [4-CH₂S(O)CH₂CN] |
| D-1 | [4-CH₂S(O)₂CH₂Pr-c] | D-1 | [4-CH₂S(O)₂CH₂CN] |
| D-1 | [4-CH₂SCH₂Bu-c] | D-1 | [4-CH₂SCH₂CH₂CN] |
| D-1 | [4-CH₂S(O)CH₂Bu-c] | D-1 | [4-CH₂S(O)(CH₂CH₂CN] |
| D-1 | [4-CH₂S(O)₂CH₂Bu-c] | D-1 | [4-CH₂S(O)₂CH₂CH₂CN] |
| D-1 | [4-CH₂SCH₂Pen-c] | D-1 | [4-CH₂SCH₂(T-1-1)] |
| D-1 | [4-CH₂S(O)CH₂Pen-c] | D-1 | [4-CH₂(O)CH₂(T-1-1)] |
| D-1 | [4-CH₂S(O)₂CH₂Pen-c] | D-1 | [4-CH₂S(O)₂CH₂(T-1-1)] |
| D-1 | [4-CH₂SCH₂Hex-c] | D-1 | [4-CH₂SCH₂(T-1-2)] |
| D-1 | [4-CH₂S(O)CH₂Hex-c] | D-1 | [4-CH₂S(O)CH₂(T-1-2)] |
| D-1 | [4-CH₂S(O)₂CH₂Hex-c] | D-1 | [4-CH₂S(O)₂CH₂(T-1-2)] |
| D-1 | [2-OH] | D-1 | [2-OEt] |
| D-1 | [3-OH] | D-1 | [3-OEt] |
| D-1 | [4-OH] | D-1 | [4-OEt] |
| D-1 | [2-OPr-n] | D-1 | [2-OPr-i] |
| D-1 | [3-OPr-n] | D-1 | [3-OPr-i] |
| D-1 | [4-OPr-n] | D-1 | [4-OPr-i] |
| D-1 | [2-OBu-n] | D-1 | [2-OBu-i] |
| D-1 | [3-OBu-n] | D-1 | [3-OBu-i] |
| D-1 | [4-OBu-n] | D-1 | [4-OBu-i] |
| D-1 | [2-OBu-sec] | D-1 | [2-OPen-n] |
| D-1 | [3-OBu-sec] | D-1 | [3-OPen-n] |
| D-1 | [4-OBu-sec] | D-1 | [4-OPen-n] |
| D-1 | [2-OHex-n] | D-1 | [4-OCH₂Bu-tert] |
| D-1 | [3-OHex-n] | D-1 | [4-OCH(Et)_{2]} |
| D-1 | [4-OHex-n] | D-1 | [4-OCH(Me)Pr-i] |
| D-1 | [4-OCH(Me)Bu-tert] | D-1 | [4-OBu-tert] |
| D-1 | [2-OCHF₂] | D-1 | [2-OCF₃] |
| D-1 | [3-OCHF₂] | D-1 | [3-OCF₃] |
| D-1 | [4-OCHF₂] | D-1 | [4-OCF₃] |
| D-1 | [2-OCF₂Br] | D-1 | [4-OCH₂CH₂F] |
| D-1 | [3-OCF₂Br] | D-1 | [4-OCH₂CH₂Cl] |
| D-1 | [4-OCF₂Br] | D-1 | [4-OCH₂CH₂Br] |
| D-1 | [4-OCH₂CHF₂] | D-1 | [4-OCH₂CH₂CHF] |
| D-1 | [4-OCH₂CHCl₂] | D-1 | [4-OCH₂CH₂CHCl] |
| D-1 | [4-OCH₂CF₃] | D-1 | [4-OCH₂CH₂CH₂Br] |
| D-1 | [4-OCH₂CF₂CHF₂] | D-1 | [4-OCH₂HCF₂CF₃] |
| D-1 | [4-OCH₂CH=CH₂] | D-1 | [4-OCH₂C(Me)=CH₂] |
| D-1 | [4-OCH₂CH=C(Me)₂] | D-1 | [4-OCH₂CH₂CH=CH₂] |
| D-1 | [4-OCH(Me)CH=CH₂] | D-1 | [4-OC(Me)₂CH=CH₂] |
| D-1 | [4-OCH₂CH=CF₂] | D-1 | [4-OCH₂C(Br)=CH₂] |
| D-1 | [4-OCH₂CH=CCl₂] | D-1 | [4-OCH₂CCl=CHCl] |
| D-1 | [4-OCH₂CH₂CH=CF₂] | D-1 | [4-OCH₂CH₂CF=CF₂] |
| D-1 | [4-OCH₂C≡CH] | D-1 | [4-OCH₂C≡CMe] |
| D-1 | [4-OCH(Me)C≡CH] | D-1 | [4-OC(Me)₂C≡CH] |
| D-1 | [4-OCH₂CH₂C≡CH] | D-1 | [4-OCH₂C≡CEt] |
| D-1 | [4-OCH₂CH₂C≡CMe] | D-1 | [4-OCH₂C≡CPr-n] |
| D-1 | [4-OCH₂C≡CCl] | D-1 | [4-OCH₂C≡CBr] |
| D-1 | [4-OCH₂Pr-c] | D-1 | [4-OCH₂CBu-c] |
| D-1 | [4-OCH₂Pec-c] | D-1 | [4-OCH₂CHex-c] |
| D-1 | [2-OCH₂(T-1-1)] | D-1 | [2-OCH₂(T-1-2)] |
| D-1 | [3-OCH₂(T-1-1)] | D-1 | [3-OCH₂(T-1-2)] |
| D-1 | [4-OCH₂(T-1-1)] | D-1 | [4-OCH₂(T-1-2)] |
| D-1 | [2-OCH₂(T-1-3)] | D-1 | [2-OCH₂(T-1-4)] |
| D-1 | [3-OCH₂(T-1-3)] | D-1 | [3-OCH₂(T-1-4)] |
| D-1 | [4-OCH₂(T-1-3)] | D-1 | [4-OCH₂(T-1-4)] |
| D-1 | [4-OCH₂OMe] | D-1 | [4-OCH₂OEt] |
| D-1 | [4-OCH₂OPr-n] | D-1 | [2-OCH₂CH₂OH] |
| D-1 | [3-OCH₂CH₂OH] | D-1 | [4-OCH₂CH₂OH] |
| D-1 | [4-OCH₂CH₂OMe] | D-1 | [4-OCH₂CH(OMe)₂] |
| D-1 | [4-OCH₂CH₂OEt] | D-1 | [4-OCH(Me)CH₂OMe] |
| D-1 | [4-OCH₂CH₂OPr-n] | D-1 | [4-OCH₂CH(Me)OMe] |
| D-1 | [4-OCH₂OCH₂CH₂F] | D-1 | [4-OCH₂OCH₂CH₂Cl] |
| D-1 | [4-OCH₂CH₂OCH₂CH₂F] | D-1 | [4-OCH₂CH₂CH₂OCH₂CH₂Cl] |
| D-1 | [4-OCH₂CH₂OCH₂CHF₂] | D-1 | [4-OCH₂CH₂CH₂OCH₂CHCl₂] |
| D-1 | [4-OCH₂CH₂OCH₂CF₃] | D-1 | [4-OCH₂CH₂CH₂OCH₂CCl₃] |
| D-1 | [4-OCH₂CH₂OCH₂CH=CH₂] | D-1 | [4-OCH₂CH₂OCH₂CH₂CH=CH₂] |
| D-1 | [4-OCH₂CH₂OCH₂CH=CF₂] | D-1 | [4-OCH₂CH₂OCH₂CH=C(Me)₂] |
| D-1 | [4-OCH₂CH₂OCH₂CH=CCl₂] | D-1 | [4-OCH₂CH₂OCH(Me)CH=CH₂] |
| D-1 | [4-OCH₂CH₂OCH₂CF=CF₂] | D-1 | [4-OCH₂CH₂OC(Me)₂CH=CH₂] |
| D-1 | [4-OCH₂CH₂OCH₂C≡CH] | D-1 | [4-OCH₂CH₂OCH₂C≡CMe] |
| D-1 | [4-OCH₂CH₂OCH₂C≡CCl] | D-1 | [4-OCH₂CH₂OCH₂C≡CBr] |
| D-1 | [4-OCH₂CH₂OPen-c] | D-1 | [4-OCH₂CH₂OHex-c] |
| D-1 | [4-OCH₂CH₂OC(O)Me | D-1 | [4-OCH₂CH₂OC(O)Et] |
| D-1 | [4-OCH₂CH₂OC(O)Pr-i | D-1 | [4-OCH₂CH₂OC(O)Bu-tert] |
| D-1 | [4-OCH₂CH₂OC(O)OMe | D-1 | [4-OCH₂CH₂OC(O)OEt] |
| D-1 | [4-OCH₂CH₂OC(O)OPr-i | D-1 | [4-OCH₂CH₂OC(O)OBu-tert] |
| D-1 | [4-OCH₂CH₂OC(O)N(Me)₂ | D-1 | [4-OCH₂CH₂OS(O)₂Me] |
| D-1 | [4-OCH₂CH₂OS(O)₂Et | D-1 | [4-OCH₂CH₂OS(O)₂CH₂Cl] |
| D-1 | [4-OCH₂CH₂OS(O)₂CF₃ | D-1 | [4-OCH₂CH₂OS(O)₂N(Me)₂] |
| D-1 | [4-OCH₂SMe] | D-1 | [4-OCH₂SEt] |
| D-1 | [4-OCH₂CH₂SMe] | D-1 | [4-OCH(Me)CH₂SMe] |
| D-1 | [4-OCH₂CH₂S(O)Me] | D-1 | [4-OCH(Me)CH₂S(O)Me] |
| D-1 | [4-OCH₂CH₂S(O)₂Me] | D-1 | [4-OCH(Me)CH₂S(O)₂Me] |
| D-1 | [4-OCH₂CH(Me)SMe] | D-1 | [4-OCH(Me)CH₂SMe] |
| D-1 | [4-OCH₂CH(Me)S(O)Me] | D-1 | [4-OCH(Me)CH₂S(O)Me] |
| D-1 | [4-OCH₂CH(Me)S(O)₂Me] | D-1 | [4-OCH(Me)CH₂S(O)₂Me] |
| D-1 | [4-OCH₂CH₂SCH₂CF₃] | D-1 | [4-OCH₂CH₂SCH₂CHF₂] |
| D-1 | [4-OCH₂CH₂S(O)CH₂CF₃] | D-1 | [4-OCH₂CH₂S(O)CH₂CHF₂] |
| D-1 | [4-OCH₂CH₂S(O)₂CH₂CF₃] | D-1 | [4-OCH₂CH₂S(O)₂CH₂CHF₂] |
| D-1 | [4-OCH₂CH₂SCH₂CH=CH₂] | D-1 | [4-OCH₂CH₂S(O)CH₂CH=CH₂] |
| D-1 | [4-OCH₂CH₂SCH₂CH=C(Me)₂] | D-1 | [4-OCH₂CH₂S(O)₂CH₂CH=CH₂] |
| D-1 | [4-OCH₂CH₂SCH₂CH=CCl₂] | D-1 | [4-OCH₂CH₂S(O)CH₂CH=CCl₂] |
| D-1 | [4-OCH₂CH₂SCH₂C≡CH] | D-1 | [4-OCH₂CH₂S(O)CH₂C≡CH] |
| D-1 | [4-OCH₂CH₂SCH₂C≡CMe] | D-1 | [4-OCH₂CH₂S(O)₂CH₂C≡CH] |
| D-1 | [4-OCH₂CH₂SCH₂C≡CCl] | D-1 | [4-OCH₂CH₂S(O)CH₂C≡CMe] |
| D-1 | [4-OCH₂CH₂SCH₂C≡CBr] | D-1 | [4-OCH₂CH₂S(O)₂CH₂C≡CMe] |
| D-1 | [4-OCH₂CH₂SPen-c] | D-1 | [4-OCH₂CH₂S(O)CH₂C≡CCl] |
| D-1 | [4-OCH₂CH₂S(O)Pen-c] | D-1 | [4-OCH₂CH₂S(O)₂CH₂C≡CCl] |
| D-1 | [4-OCH₂CH₂S(O)₂Pen-c] | D-1 | [4-OCH₂CH₂S(O)CH₂C≡CBr] |
| D-1 | [4-OCH₂CH₂SHex-c] | D-1 | [4-OCH₂CH₂S(O)₂CH₂C≡CBr] |
| D-1 | [4-OCH₂CH₂S(O)Hex-c] | D-1 | [4-OCH₂CH₂S(O)₂Hex-c] |
| D-1 | [2-OCH₂Ph] | D-1 | [2-OCH₂CN] |
| D-1 | [3-OCH₂Ph] | D-1 | [3-OCH₂CN] |
| D-1 | [4-OCH₂Ph] | D-1 | [4-OCH₂CN] |
| D-1 | [2-OCH(Me)CN] | D-1 | [2-OCH₂CH₂CN] |
| D-1 | [3-OCH(Me)CN] | D-1 | [3-OCH₂CH₂CN] |
| D-1 | [4-OCH(Me)CN] | D-1 | [4-OCH₂CH₂CN] |
| D-1 | [2-OCH₂Ph(2-F)] | D-1 | [4-OCH₂Ph(2-F)] |
| D-1 | [2-OCH₂Ph(3-F)] | D-1 | [4-OCH₂Ph(3-F)] |
| D-1 | [2-OCH₂Ph(4-F)] | D-1 | [4-OCH₂Ph(4-F)] |
| D-1 | [2-OCH₂Ph(2-Cl)] | D-1 | [4-OCH₂Ph(2-Cl)] |
| D-1 | [2-OCH₂Ph(3-Cl)] | D-1 | [4-OCH₂Ph(3-Cl)] |
| D-1 | [2-OCH₂Ph(4-Cl)] | D-1 | [4-OCH₂Ph(4-Cl)] |
| D-1 | [2-OCH₂Ph(2-Me)] | D-1 | [4-OCH₂Ph(2-Me)] |
| D-1 | [2-OCH₂Ph(3-Me)] | D-1 | [4-OCH₂Ph(3-Me)] |
| D-1 | [2-OCH₂Ph(4-Me)] | D-1 | [4-OCH₂Ph(4-Me)] |
| D-1 | [2-OCH₂Ph(2-OMe)] | D-1 | [4-OCH₂Ph(2-OMe)] |
| D-1 | [2-OCH₂Ph(3-OMe)] | D-1 | [4-OCH₂Ph(3-OMe)] |
| D-1 | [2-OCH₂Ph(4-OMe)] | D-1 | [4-OCH₂Ph(4-OMe)] |
| D-1 | [4-OCH₂C(=NOH)H] | D-1 | [4-OCH₂C(=NOCH₂CH=CH₂)Me] |
| D-1 | [4-OCH₂CH₂C(=NOH)Me] | D-1 | [4-OCH₂C(=NOCH₂C=CH)Me] |
| D-1 | [4-OCH₂C(=NOMe)H] | D-1 | [4-OCH₂C(=NOCH₂CH₂F)Me] |
| D-1 | [4-OCH₂CH₂C(=NOMe)Me] | D-1 | [4-OCH₂C(=NOCH₂CH₂Cl)Me] |
| D-1 | [4-OCH₂C(=NOMe)OMe] | D-1 | [4-OCH₂C(=NOCH₂CHF₂)Me] |
| D-1 | [4-OCH₂C(=NOEt)Me] | D-1 | [4-OCH₂C(=NOCH₂CHCl₂)Me] |
| D-1 | [4-OCH₂C(=NOPr-n)Me] | D-1 | [4-OCH₂C(=NOCH₂CF₃)Me] |
| D-1 | [4-OCH₂C(=NOPr-i)Me] | D-1 | [4-OCH₂C(=NOCH₂CH₂CH₂Cl)Me] |
| D-1 | [4-OCH₂C(=NOH)Me] | D-1 | [4-OCH₂C(=NOCH₂CH₂OMe)Me] |
| D-1 | [4-OCH₂C(=NOH)NH₂] | D-1 | [4-OCH₂C(=NOCH₂CH₂SMe)Me] |
| D-1 | [4-OCH₂C(=NOMe)Me] | D-1 | [4-OCH₂C(=NOCH₂Pr-c)Me] |
| D-1 | [4-OCH₂C(=NOMe)NH₂] | D-1 | [4-OCH₂C(=NOCH₂(Ph-2-F))Me] |
| D-1 | [4-OCH₂C(=NOMe)SMe] | D-1 | [4-OCH₂C(=NOCH₂(Ph-3-F))Me] |
| D-1 | [4-OCH₂C(=NOMe)OMe] | D-1 | [4-OCH₂C(=NOCH₂(Ph-4-F))Me] |
| D-1 | [4-OCH₂CH₂C(=NOH)Me] | D-1 | [4-OCH₂C(=NOCH₂(Ph-2-OMe))Me] |
| D-1 | [4-OCH₂CH₂C(=NOMe)Me] | D-1 | [4-OCH₂C(=NOCH₂(Ph-3-OMe))Me] |
| D-1 | [4-OCH₂CH₂C(=NOEt)Me] | D-1 | [4-OCH₂C(=NOCH₂(Ph-4-OMe))Me] |
| D-1 | [4-OCH₂(U-1a)] | D-1 | [4-OCH₂C(=NOCH₂(Ph-2-Cl))Me] |
| D-1 | [4-OCH₂(U-2a)] | D-1 | [4-OCH₂C(=NOCH₂(Ph-3-Cl))Me] |
| D-1 | [4-OCH₂(U-3a)] | D-1 | [4-OCH₂C(=NOCH₂(Ph-4-Cl))Me] |
| D-1 | [4-OCH₂(U-4a)] | D-1 | [4-OCH₂C(=NOCH₂(Ph-2-Me))Me] |
| D-1 | [4-OCH₂(U-5a)] | D-1 | [4-OCH₂C(=NOCH₂(Ph-3-Me))Me] |
| D-1 | [4-OCH₂(U-9a)] | D-1 | [4-OCH₂C(=NOCH₂(Ph-4-Me))Me] |
| D-1 | [4-OCH₂(U-10a)] | D-1 | [4-OCH₂(U-11a)] |
| D-1 | [4-OCH₂(U-12a)] | D-1 | [4-OCH₂(U-13a)] |
| D-1 | [4-OCH₂(U-14a)] | D-1 | [4-OCH₂(U-15a)] |
| D-1 | [4-OCH₂(U-16a)] | D-1 | [4-OCH₂(U-17a)] |
| D-1 | [4-OCH₂(U-20a)] | D-1 | [4-OCH₂(U-31a)] |
| D-1 | [4-OCH₂(U-32a)] | D-1 | [4-OCH₂(Q-1-1)] |
| D-1 | [4-OCH₂(Q-2)] | D-1 | [4-OCH₂(Q-3)] |
| D-1 | [4-OCH₂(Q-4)] | D-1 | [4-OCH₂(Q-5)] |
| D-1 | [4-OCH₂(Q-8-1)] | D-1 | [4-OCH₂(Q-9-1)] |
| D-1 | [4-OCH₂(Q-14-1)] | D-1 | [4-OCH₂(Q-15-1)] |
| D-1 | [4-OCH₂(Q-16-1)] | D-1 | [4-OCH₂(Q-17)] |
| D-1 | [4-OCH₂(Q-18)] | D-1 | [4-OCH₂(Q-18-1)] |
| D-1 | [4-OCH₂(Q-19)] | D-1 | [4-OCH₂(Q-20)] |
| D-1 | [4-OCH₂(Q-35-1)] | D-1 | [4-OCH₂ON=C(Me)H] |
| D-1 | [4-OCH₂ON=C(Me)₂] | D-1 | [4-OCH₂ON=C(Me)Et] |
| D-1 | [4-OCH₂ON=C(CH₂)₄] | D-1 | [4-OCH₂ON=C(CH₂)₅] |
| D-1 | [4-OPr-c] | D-1 | [4-OBu-c)] |
| D-1 | [4-OPen-c] | D-1 | [4-OHex-c)] |
| D-1 | [2-O(T-1-5)] | D-1 | [4-O(T-1-5)] |
| D-1 | [2-OPh] | D-1 | [4-O(Ph)] |
| D-1 | [4-O(Ph-2-F)] | D-1 | [4-O(Ph-3-F)] |
| D-1 | [4-0(Ph-4-F)] | D-1 | [4-O(Ph-2-Cl)] |
| D-1 | [4-O(Ph-3-Cl)] | D-1 | [4-O(Ph-4-Cl)] |
| D-1 | [4-O(Ph-2-Me)] | D-1 | [4-O(Ph-3-Me)] |
| D-1 | [4-O(Ph-4-Me)] | D-1 | [4-O(Ph-2-OMe)] |
| D-1 | [4-O(Ph-3-OMe)] | D-1 | [4-O(Ph-4-OMe)] |
| D-1 | [4-O(Ph-2-CF₃)] | D-1 | [4-O(Ph-3-CF₃)] |
| D-1 | [4-O(Ph-4-CF₃)] | D-1 | [4-O(Ph-2-SMe)] |
| D-1 | [4-O(Ph-3-SMe)] | D-1 | [4-O(Ph-4-SMe)] |
| D-1 | [4-O(Ph-4-CN)] | D-1 | [4-O(Ph-4-NO₂)] |
| D-1 | [4-O(Ph-2-Cl-4-CF₃)] | D-1 | [4-O(Ph-2,4-Cl₂)] |
| D-1 | [4-OC(O)Me] | D-1 | [4-OC(O)Et] |
| D-1 | [4-OC(O)Pr-n] | D-1 | [4-OC(O)Pr-i] |
| D-1 | [4-OC(O)Bu-tert] | D-1 | [4-OC(O)OMe] |
| D-1 | [4-OC(O)OEt] | D-1 | [4-OC(O)OBu-tert] |
| D-1 | [4-OS(O)₂Me] | D-1 | [4-OS(O)₂Et] |
| D-1 | [4-OS(O)₂CH₂Cl] | D-1 | [4-OS(O)₂CF₃] |
| D-1 | [4-O(U-1a)] | D-1 | [4-O(U-2a)] |
| D-1 | [4-O(U-3a)] | D-1 | [4-O(U-4a)] |
| D-1 | [4-O(U-5a)] | D-1 | [4-O(U-12a)] |
| D-1 | [4-O(U-14a)] | D-1 | [4-O(U-15a)] |
| D-1 | [4-O(U-16a)] | D-1 | [4-O(Q-17-1)] |
| D-1 | [4-O(Q-17-2)] | D-1 | [4-O(Q-17-3)] |
| D-1 | [4-O(Q-17b)] | D-1 | [4-O(Q-18)] |
| D-1 | [4-O(Q-19)] | D-1 | [4-O(Q-20)] |
| D-1 | [2-SMe] | D-1 | [2-S(O)Me] |
| D-1 | [3-SMe] | D-1 | [3-S(O)Me] |
| D-1 | [4-SMe] | D-1 | [4-S(O)Me] |
| D-1 | [2-S(O)₂Me] | D-1 | [2-SEt] |
| D-1 | [3-S(O)₂Me] | D-1 | [3-SEt] |
| D-1 | [4-S(O)₂Me] | D-1 | [4-SEt] |
| D-1 | [2-S(O)Et] | D-1 | [2-S(O)₂Et] |
| D-1 | [3-S(O)Et] | D-1 | [3-S(O)₂Et] |
| D-1 | [4-S(O)Et] | D-1 | [4-S(O)₂Et] |
| D-1 | [2-SPr-n] | D-1 | [2-S(O)Pr-n] |
| D-1 | [3-SPr-n] | D-1 | [3-S(O)Pr-n] |
| D-1 | [4-SPr-n] | D-1 | [4-S(O)Pr-n] |
| D-1 | [2-S(O)₂Pr-n] | D-1 | [2-SPr-i] |
| D-1 | [3-S(O)₂Pr-n] | D-1 | [3-SPr-i] |
| D-1 | [4-S(O)₂Pr-n] | D-1 | [4-SPr-i] |
| D-1 | [2-S(O)Pr-i] | D-1 | [2-S(O)₂Pr-i] |
| D-1 | [3-S(O)Pr-i] | D-1 | [3-S(O)₂Pr-i] |
| D-1 | [4-S(O)Pr-i] | D-1 | [4-S(O)₂Pr-i] |
| D-1 | [2-SBu-n] | D-1 | [2-S(O)Bu-n] |
| D-1 | [3-SBu-n] | D-1 | [3-S(O)Bu-n] |
| D-1 | [4-SBu-n] | D-1 | [4-S(O)Bu-n] |
| D-1 | [2-S(O)₂Bu-n] | D-1 | [2-SBu-i] |
| D-1 | [3-S(O)₂Bu-n] | D-1 | [3-SBu-i] |
| D-1 | [4-S(O)₂Bu-n] | D-1 | [4-SBu-i] |
| D-1 | [2-S(O)Bu-i] | D-1 | [2-S(O)₂Bu-i] |
| D-1 | [3-S(O)Bu-i] | D-1 | [3-S(O)₂Bu-i] |
| D-1 | [4-S(O)Bu-i] | D-1 | [4-S(O)₂Bu-i] |
| D-1 | [2-SBu-sec] | D-1 | [2-S(O)Bu-sec] |
| D-1 | [3-SBu-sec] | D-1 | [3-S(O)Bu-sec] |
| D-1 | [4-SBu-sec] | D-1 | [4-S(O)Bu-sec] |
| D-1 | [2-S(O)₂Bu-sec] | D-1 | [2-SBu-tert] |
| D-1 | [3-S(O)₂Bu-sec] | D-1 | [3-SBu-tert] |
| D-1 | [4-S(O)₂Bu-sec] | D-1 | [4-SBu-tert] |
| D-1 | [2-S(O)Bu-tert] | D-1 | [2-S(O)₂Bu-tert] |
| D-1 | [3-S(O)Bu-tert] | D-1 | [3-S(O)₂Bu-tert] |
| D-1 | [4-S(O)Bu-tert] | D-1 | [4-S(O)₂Bu-tert] |
| D-1 | [2-SPen-n] | D-1 | [2-S(O)Pen-n] |
| D-1 | [3-SPen-n] | D-1 | [3-S(O)Pen-n] |
| D-1 | [4-SPen-n] | D-1 | [4-S(O)Pen-n] |
| D-1 | [2-S(O)₂Pen-n] | D-1 | [2-SHex-n] |
| D-1 | [3-S(O)₂Pen-n] | D-1 | [3-SHex-n] |
| D-1 | [4-S(O)₂Pen-n] | D-1 | [4-SHex-n] |
| D-1 | [2-S(O)Hex-n] | D-1 | [2-S(O)₂Hex-n] |
| D-1 | [3-S(O)Hex-n] | D-1 | [3-S(O)₂Hex-n] |
| D-1 | [4-S(O)Hex-n] | D-1 | [4-S(O)₂Hex-n] |
| D-1 | [4-SCH₂Bu-tert] | D-1 | [4-SCH(Et)₂] |
| D-1 | [4-S(O)CH₂Bu-tert] | D-1 | [4-S(O)CH(Et)₂] |
| D-1 | [4-S(O)₂CH₂Bu-tert] | D-1 | [4-S(O)₂CH(Et)₂] |
| D-1 | [4-SCH(Me)Pr-i] | D-1 | [4-SCH(Me)Bu-tert] |
| D-1 | [4-S(O)CH(Me)Pr-i] | D-1 | [4-S(O)CH(Me)Bu-tert] |
| D-1 | [4-S(O)₂CH(Me)Pr-i] | D-1 | [4-S(O)₂CH(Me)Bu-tert] |
| D-1 | [4-SCH₂CH=CH₂] | D-1 | [4-SCH₂C(Me)=CH₂] |
| D-1 | [4-S(O)CH₂CH=CH₂] | D-1 | [4-S(O)CH₂C(Me)=CH₂] |
| D-1 | [4-S(O)₂CH₂CH=CH₂] | D-1 | [4-S(O)₂CH₂C(Me)=CH₂] |
| D-1 | [4-SCH₂CH=C(Me)₂] | D-1 | [4-SCH₂CH₂CH=CH₂] |
| D-1 | [4-S(O)CH₂CH=C(Me)₂] | D-1 | [4-S(O)CH₂CH₂CH=CH₂] |
| D-1 | [4-S(O)₂CH₂CH=C(Me)₂] | D-1 | [4-S(O)₂CH₂CH₂CH=CH₂] |
| D-1 | [4-SCH(Me)CH=CH₂] | D-1 | [4-SC(Me)₂CH=CH₂] |
| D-1 | [4-S(O)CH(Me)CH=CH₂] | D-1 | [4-S(O)C(Me)₂CH=CH₂] |
| D-1 | [4-S(O)₂CH(Me)CH=CH₂] | D-1 | [4-S(O)₂C(Me)₂CH=CH₂] |
| D-1 | [2-SCHF₂] | D-1 | [2-SCF₃] |
| D-1 | [3-SCHF₂] | D-1 | [3-SCF₃] |
| D-1 | [4-SCHF₂] | D-1 | [4-SCF₃] |
| D-1 | [2-S(O)CHF₂] | D-1 | [2-S(O)CF₃] |
| D-1 | [3-S(O)CHF₂] | D-1 | [3-S(O)CF₃] |
| D-1 | [4-S(O)CHF₂] | D-1 | [4-S(O)CF₃] |
| D-1 | [2-S(O)₂CHF₂] | D-1 | [2-S(O)₂CF₃] |
| D-1 | [3-S(O)₂CHF₂] | D-1 | [3-S(O)₂CF₃] |
| D-1 | [4-S(O)₂CHF₂] | D-1 | [4-S(O)₂CF₃] |
| D-1 | [2-SCF₂Br] | D-1 | [4-SCH₂CH₂F] |
| D-1 | [3-SCF₂Br] | D-1 | [4-SCH₂CH₂Cl] |
| D-1 | [4-SCF₂Br] | D-1 | [4-SCH₂CH₂Br] |
| D-1 | [2-S(O)CF₂Br] | D-1 | [4-S(O)CH₂CH₂F] |
| D-1 | [3-S(O)CF₂Br] | D-1 | [4-S(O)CH₂CH₂Cl] |
| D-1 | [4-S(O)CF₂Br] | D-1 | [4-S(O)CH₂CH₂Br] |
| D-1 | [2-S(O)₂CF₂Br] | D-1 | [4-S(O)₂CH₂CH₂F] |
| D-1 | [3-S(O)₂CF₂Br] | D-1 | [4-S(O)₂CH₂CH₂Cl] |
| D-1 | [4-S(O)₂CF₂Br] | D-1 | [4-S(O)₂CH₂CH₂Br] |
| D-1 | [4-SCH₂CHF₂] | D-1 | [4-SCH₂CH₂CHF] |
| D-1 | [4-SCH₂CHCl₂] | D-1 | [4-SCH₂CH₂CHCl] |
| D-1 | [4-SCH₂CF₃] | D-1 | [4-SCH₂CH₂CH₂Br] |
| D-1 | [4-SCH₂CF₂CHF₂] | D-1 | [4-SCH₂HCF₂CF₃] |
| D-1 | [4-S(O)CH₂CHF₂] | D-1 | [4-S(O)CH₂CH₂CHF] |
| D-1 | [4-S(O)CH₂CHCl₂] | D-1 | [4-S(O)CH₂CH₂CHCl] |
| D-1 | [4-S(O)CH₂CF₃] | D-1 | [4-S(O)CH₂CH₂CH₂Br] |
| D-1 | [4-S(O)CH₂CF₂CHF₂] | D-1 | [4-S(O)CH₂HCF₂CF₃] |
| D-1 | [4-S(O)₂CH₂CHF₂] | D-1 | [4-S(O)₂CH₂CH₂CHF] |
| D-1 | [4-S(O)₂CH₂CHCl₂] | D-1 | [4-S(O)₂CH₂CH₂CHCl] |
| D-1 | [4-S(O)₂CH₂CF₃] | D-1 | [4-S(O)₂CH₂CH₂CH₂Br] |
| D-1 | [4-S(O)₂CH₂CF₂CHF₂] | D-1 | [4-S(O)₂CH₂HCF₂CF₃] |
| D-1 | [4-SCH₂CH=CF₂] | D-1 | [4-SCH₂C(Br)=CH₂] |
| D-1 | [4-SCH₂CH=CCl₂] | D-1 | [4-SCH₂CCl=CHCl] |
| D-1 | [4-SCH₂CH₂CH=CF₂] | D-1 | [4-SCH₂CH₂CF=CF₂] |
| D-1 | [4-S(O)CH₂CH=CF₂] | D-1 | [4-S(O)CH₂C(Br)=CH₂] |
| D-1 | [4-S(O)CH₂CH=CCl₂] | D-1 | [4-S(O)CH₂CCl=CHCl] |
| D-1 | [4-S(O)CH₂CH₂CH=CF₂] | D-1 | [4-S(O)CH₂CH₂CF=CF₂] |
| D-1 | [4-S(O)₂CH₂CH=CF₂] | D-1 | [4-S(O)₂CH₂C(Br)=CH₂] |
| D-1 | [4-S(O)₂CH₂CH=CCl₂] | D-1 | [4-S(O)₂CH₂CCl=CHCl] |
| D-1 | [4-S(O)₂CH₂CH₂CH=CF₂] | D-1 | [4-S(O)₂CH₂CH₂CF=CF₂] |
| D-1 | [4-SCH₂C≡CH] | D-1 | [4-SCH₂C≡CMe] |
| D-1 | [4-SCH(Me)C=CH] | D-1 | [4-SC(Me)₂C≡CH] |
| D-1 | [4-SCH₂CH₂C≡CH] | D-1 | [4-SCH₂C≡CEt] |
| D-1 | [4-SCH₂CH₂C≡CMe] | D-1 | [4-SCH₂C≡CPr-n] |
| D-1 | [4-S(O)CH₂C≡CH] | D-1 | [4-S(O)CH₂C≡CMe] |
| D-1 | [4-S(O)CH(Me)C≡CH] | D-1 | [4-S(O)C(Me)₂C≡CH] |
| D-1 | [4-S(O)CH₂CH₂C≡CH] | D-1 | [4-S(O)CH₂C≡CEt] |
| D-1 | [4-S(O)CH₂CH₂C≡CMe] | D-1 | [4-S(O)CH₂C≡CPr-n] |
| D-1 | [4-S(O)₂CH₂C≡CH] | D-1 | [4-S(O)₂CH₂C≡CMe] |
| D-1 | [4-S(O)₂CH(Me)C≡CH] | D-1 | [4-S(O)₂C(Me)₂C≡CH] |
| D-1 | [4-S(O)₂CH₂CH₂C≡CH] | D-1 | [4-S(O)₂CH₂C≡CEt] |
| D-1 | [4-S(O)₂CH₂CH₂C≡CMe] | D-1 | [4-S(O)₂CH₂C≡CPr-n] |
| D-1 | [4-SCH₂C≡CCl] | D-1 | [4-SCH₂C≡CBr] |
| D-1 | [4-S(O)CH₂C≡CCl] | D-1 | [4-S(O)CH₂C≡CBr] |
| D-1 | [4-S(O)₂CH₂C≡CCl] | D-1 | [4-S(O)₂CH₂C≡CBr] |
| D-1 | [4-SCH₂Pr-c] | D-1 | [4-SCH₂CBu-c] |
| D-1 | [4-SCH₂Pen-c] | D-1 | [4-SCH₂CHex-c] |
| D-1 | [4-S(O)CH₂Pr-c] | D-1 | [4-S(O)CH₂CBu-c] |
| D-1 | [4-S(O)CH₂Pen-c] | D-1 | [4-S(O)CH₂CHex-c] |
| D-1 | [4-S(O)₂CH₂Pr-c] | D-1 | [4-S(O)₂CH₂CBu-c] |
| D-1 | [4-S(O)₂CH₂Pen-c] | D-1 | [4-S(O)₂CH₂CHex-c] |
| D-1 | [2-SCH₂(T-1-1)] | D-1 | [2-SCH₂(T-1-2)] |
| D-1 | [3-SCH₂(T-1-1)] | D-1 | [3-SCH₂(T-1-2)] |
| D-1 | [4-SCH₂(T-1-1)] | D-1 | [4-SCH₂(T-1-2)] |
| D-1 | [2-SCH₂(T-1-3)] | D-1 | [2-SCH₂(T-1-4)] |
| D-1 | [3-SCH₂(T-1-3)] | D-1 | [3-SCH₂(T-1-4)] |
| D-1 | [4-SCH₂(T-1-3)] | D-1 | [4-SCH₂(T-1-4)] |
| D-1 | [2-S(O)CH₂(T-1-1)] | D-1 | [2-S(O)CH₂(T-1-2)] |
| D-1 | [3-S(O)CH₂(T-1-1)] | D-1 | [3-S(O)CH₂(T-1-2)] |
| D-1 | [4-S(O)CH₂(T-1-1)] | D-1 | [4-S(O)CH₂(T-1-2)] |
| D-1 | [2-S(O)CH₂(T-1-3)] | D-1 | [2-S(O)CH₂(T-1-4)] |
| D-1 | [3-S(O)CH₂(T-1-3)] | D-1 | [3-S(O)CH₂(T-1-4)] |
| D-1 | [4-S(O)CH₂(T-1-3)] | D-1 | [4-S(O)CH₂(T-1-4)] |
| D-1 | [2-S(O)₂CH₂(T-1-1)] | D-1 | [2-S(O)₂CH₂(T-1-2)] |
| D-1 | [3-S(O)₂CH₂(T-1-1)] | D-1 | [3-S(O)₂CH₂(T-1-2)] |
| D-1 | [4-S(O)₂CH₂(T-1-1)] | D-1 | [4-S(O)₂CH₂(T-1-2)] |
| D-1 | [2-S(O)₂CH₂(T-1-3)] | D-1 | [2-S(O)₂CH₂(T-1-4)] |
| D-1 | [3-S(O)₂CH₂(T-1-3)] | D-1 | [3-S(O)₂CH₂(T-1-4)] |
| D-1 | [4-S(O)₂CH₂(T-1-3)] | D-1 | [4-S(O)₂CH₂(T-1-4)] |
| D-1 | [4-SCH₂OMe] | D-1 | [4-SCH₂OEt] |
| D-1 | [4-SCH₂OPr-n] | D-1 | [2-SCH₂CH₂OH] |
| D-1 | [3-SCH₂CH₂OH] | D-1 | [4-SCH₂CH₂OH] |
| D-1 | [4-SCH₂CH₂OMe] | D-1 | [4-SCH₂CH(OMe)₂] |
| D-1 | [4-SCH₂CH₂OEt] | D-1 | [4-SCH(Me)CH₂OMe] |
| D-1 | [4-SCH₂CH₂OPr-n] | D-1 | [4-SCH₂CH(Me)OMe] |
| D-1 | [4-S(O)CH₂OMe] | D-1 | [4-S(O)CH₂OEt] |
| D-1 | [4-S(O)CH₂OPr-n] | D-1 | [2-S(O)CH₂CH₂OH] |
| D-1 | [3-S(O)CH₂CH₂OH] | D-1 | [4-S(O)CH₂CH₂OH] |
| D-1 | [4-S(O)CH₂CH₂OMe] | D-1 | [4-S(O)CH₂CH(OMe)₂] |
| D-1 | [4-S(O)CH₂CH₂OEt] | D-1 | [4-S(O)CH(Me)CH₂OMe] |
| D-1 | [4-S(O)CH₂CH₂OPr-n] | D-1 | [4-S(O)CH₂CH(Me)OMe] |
| D-1 | [4-S(O)₂CH₂OMe] | D-1 | [4-S(O)₂CH₂OEt] |
| D-1 | [4-S(O)₂CH₂OPr-n] | D-1 | [2-S(O)₂CH₂CH₂OH] |
| D-1 | [3-S(O)₂CH₂CH₂OH] | D-1 | [4-S(O)₂CH₂CH₂OH] |
| D-1 | [4-S(O)₂CH₂CH₂OMe] | D-1 | [4-S(O)₂CH₂CH(OMe)₂] |
| D-1 | [4-S(O)₂CH₂CH₂OEt] | D-1 | [4-S(O)₂CH(Me)CH₂OMe] |
| D-1 | [4-S(O)₂CH₂CH₂OPr-n] | D-1 | [4-S(O)₂CH₂CH(Me)OMe] |
| D-1 | [4-SCH₂OCH₂CH₂F] | D-1 | [4-SCH₂OCH₂CH₂Cl] |
| D-1 | [4-SCH₂CH₂OCH₂CH₂F] | D-1 | [4-SCH₂CH₂CH₂OCH₂CH₂Cl] |
| D-1 | [4-SCH₂CH₂OCH₂CHF₂] | D-1 | [4-SCH₂CH₂CH₂OCH₂CHCl₂] |
| D-1 | [4-SCH₂CH₂OCH₂CF₃] | D-1 | [4-SCH₂CH₂CH₂OCH₂CCl₃] |
| D-1 | [4-S(O)CH₂OCH₂CH₂F] | D-1 | [4-S(O)CH₂OCH₂CH₂Cl] |
| D-1 | [4-S(O)CH₂CH₂OCH₂CH₂F] | D-1 | [4-S(O)CH₂CH₂CH₂OCH₂CH₂Cl] |
| D-1 | [4-S(O)CH₂CH₂OCH₂CHF₂] | D-1 | [4-S(O)CH₂CH₂CH₂OCH₂CHCl₂] |
| D-1 | [4-S(O)CH₂CH₂OCH₂CF₃] | D-1 | [4-S(O)CH₂CH₂CH₂OCH₂CCl₃] |
| D-1 | [4-S(O)₂CH₂OCH₂CH₂F] | D-1 | [4-S(O)₂CH₂OCH₂CH₂Cl] |
| D-1 | [4-S(O)₂CH₂CH₂OCH₂CH₂F] | D-1 | [4-S(O)₂CH₂CH₂CH₂OCH₂CH₂Cl] |
| D-1 | [4-S(O)₂CH₂CH₂OCH₂CHF₂] | D-1 | [4-S(O)₂CH₂CH₂CH₂OCH₂CHCl₂] |
| D-1 | [4-S(O)₂CH₂CH₂OCH₂CF₃] | D-1 | [4-S(O)₂CH₂CH₂CH₂OCH₂CCl₃] |
| D-1 | [4-SCH₂CH₂OCH₂CH=CH₂] | D-1 | [4-SCH₂CH₂OCH₂CH₂CH=CH₂] |
| D-1 | [4-SCH₂CH₂OCH₂CH=CF₂] | D-1 | [4-SCH₂CH₂OCH₂CH=C(Me)₂] |
| D-1 | [4-S(O)CH₂CH₂OCH₂CH=CH₂] | D-1 | [4-S(O)CH₂CH₂OCH₂CH₂CH=CH₂] |
| D-1 | [4-S(O)CH₂CH₂OCH₂CH=CF₂] | D-1 | [4-S(O)CH₂CH₂OCH₂CH=C(Me)₂] |
| D-1 | [4-S(O)₂CH₂CH₂OCH₂CH=CH₂] | D-1 | [4-S(O)₂CH₂CH₂OCH₂CH₂CH=CH₂] |
| D-1 | [4-S(O)₂CH₂CH₂OCH₂CH=CF₂] | D-1 | [4-S(O)₂CH₂CH₂OCH₂CH=C(Me)₂] |
| D-1 | [4-SCH₂CH₂OCH₂CH=CCl₂] | D-1 | [4-SCH₂CH₂OCH(Me)CH=CH₂] |
| D-1 | [4-SCH₂CH₂OCH₂CF=CF₂] | D-1 | [4-SCH₂CH₂OC(Me)₂CH=CH₂] |
| D-1 | [4-S(O)CH₂CH₂OCH₂CH=CCl₂] | D-1 | [4-S(O)CH₂CH₂OCH(Me)CH=CH₂] |
| D-1 | [4-S(O)CH₂CH₂OCH₂CF=CF₂] | D-1 | [4-S(O)CH₂CH₂OC(Me)₂CH=CH₂] |
| D-1 | [4-S(O)₂CH₂CH₂OCH₂CH=CCl₂] | D-1 | [4-S(O)₂CH₂CH₂OCH(Me)CH=CH₂] |
| D-1 | [4-S(O)₂CH₂CH₂OCH₂CF=CF₂] | D-1 | [4-S(O)₂CH₂CH₂OC(Me)₂CH=CH₂] |
| D-1 | [4-SCH₂CH₂OCH₂C=CH] | D-1 | [4-SCH₂CH₂OCH₂C=CMe] |
| D-1 | [4-SCH₂CH₂OCH₂C=CCl] | D-1 | [4-SCH₂CH₂OCH₂C=CBr] |
| D-1 | [4-S(O)CH₂CH₂OCH₂C=CH] | D-1 | [4-S(O)CH₂CH₂OCH₂C=CMe] |
| D-1 | [4-S(O)CH₂CH₂OCH₂C=CCl] | D-1 | [4-S(O)CH₂CH₂OCH₂C=CBr] |
| D-1 | [4-S(O)₂CH₂CH₂OCH₂C=CH] | D-1 | [4-S(O)₂CH₂CH₂OCH₂C=CMe] |
| D-1 | [4-S(O)₂CH₂CH₂OCH₂C=CCl] | D-1 | [4-S(O)₂CH₂CH₂OCH₂C=CBr] |
| D-1 | [4-SCH₂SMe] | D-1 | [4-SCH₂SEt] |
| D-1 | [4-SCH₂CH₂SMe] | D-1 | [4-SCH(Me)CH₂SMe] |
| D-1 | [4-SCH₂CH₂S(O)Me] | D-1 | [4-SCH(Me)CH₂S(O)Me] |
| D-1 | [4-SCH₂CH₂S(O)₂Me] | D-1 | [4-SCH(Me)CH₂S(O)₂Me] |
| D-1 | [4-SCH₂CH(Me)SMe] | D-1 | [4-SCH(Me)CH₂SMe] |
| D-1 | [4-SCH₂CH(Me)S(O)Me] | D-1 | [4-SCH(Me)CH₂S(O)Me] |
| D-1 | [4-SCH₂CH(Me)S(O)₂Me] | D-1 | [4-SCH(Me)CH₂S(O)₂Me] |
| D-1 | [4-S(O)CH₂SMe] | D-1 | [4-S(O)CH₂SEt] |
| D-1 | [4-S(O)CH₂CH₂SMe] | D-1 | [4-S(O)CH(Me)CH₂SMe] |
| D-1 | [4-S(O)CH₂CH₂S(O)Me] | D-1 | [4-S(O)CH(Me)CH₂S(O)Me] |
| D-1 | [4-S(O)CH₂CH₂S(O)₂Me] | D-1 | [4-S(O)CH(Me)CH₂S(O)₂Me] |
| D-1 | [4-S(O)CH₂CH(Me)SMe] | D-1 | [4-S(O)CH(Me)CH₂SMe] |
| D-1 | [4-S(O)CH₂CH(Me)S(O)Me] | D-1 | [4-S(O)CH(Me)CH₂S(O)Me] |
| D-1 | [4-S(O)CH₂CH(Me)S(O)₂Me] | D-1 | [4-S(O)CH(Me)CH₂S(O)₂Me] |
| D-1 | [4-S(O)₂CH₂SMe] | D-1 | [4-S(O)₂CH₂SEt] |
| D-1 | [4-S(O)₂CH₂CH₂SMe] | D-1 | [4-S(O)₂CH(Me)CH₂SMe] |
| D-1 | [4-S(O)₂CH₂CH₂S(O)Me] | D-1 | [4-S(O)₂CH(Me)CH₂S(O)Me] |
| D-1 | [4-S(O)₂CH₂CH₂S(O)₂Me] | D-1 | [4-S(O)₂CH(Me)CH₂S(O)₂Me] |
| D-1 | [4-S(O)₂CH₂CH(Me)SMe] | D-1 | [4-S(O)₂CH(Me)CH₂SMe] |
| D-1 | [4-S(O)₂CH₂CH(Me)S(O)Me] | D-1 | [4-S(O)₂CH(Me)CH₂S(O)Me] |
| D-1 | [4-S(O)₂CH₂CH(Me)S(O)₂Me] | D-1 | [4-S(O)₂CH(Me)CH₂S(O)₂Me] |
| D-1 | [4-SCH₂CH₂SCH₂CF₃] | D-1 | [4-SCH₂CH₂SCH₂CHF₂] |
| D-1 | [4-SCH₂CH₂S(O)CH₂CF₃] | D-1 | [4-SCH₂CH₂S(O)CH₂CHF₂] |
| D-1 | [4-SCH₂CH₂S(O)₂CH₂CF₃] | D-1 | [4-SCH₂CH₂S(O)₂CH₂CHF₂] |
| D-1 | [4-S(O)CH₂CH₂SCH₂CF₃] | D-1 | [4-S(O)CH₂CH₂SCH₂CHF₂] |
| D-1 | [4-S(O)CH₂CH₂S(O)CH₂CF₃] | D-1 | [4-S(O)CH₂CH₂S(O)CH₂CHF₂] |
| D-1 | [4-S(O)CH₂CH₂S(O)₂CH₂CF₃] | D-1 | [4-S(O)CH₂CH₂S(O)₂CH₂CHF₂] |
| D-1 | [4-S(O)₂CH₂CH₂SCH₂CF₃] | D-1 | [4-S(O)₂CH₂CH₂SCH₂CHF₂] |
| D-1 | [4-S(O)₂(O)₂CH₂CH₂S(O)CH₂CF₃] | D-1 | [4-S(O)₂CH₂CH₂S(O)CH₂CHF₂] |
| D-1 | [4-S(O)₂CH₂CH₂S(O)₂CH₂CF₃] | D-1 | [4-S(O)₂CH₂CH₂S(O)₂CH₂CHF₂] |
| D-1 | [4-SCH₂CH₂SCH₂CH=CH₂] | D-1 | [4-SCH₂CH₂S(O)CH₂CH=CH₂] |
| D-1 | [4-SCH₂CH₂SCH₂CH=C(Me)₂] | D-1 | [4-SCH₂CH₂S(O)₂CH₂CH=CH₂] |
| D-1 | [4-SCH₂CH₂SCH₂CH=CCl₂] | D-1 | [4-SCH₂CH₂S(O)CH₂CH=CCl₂] |
| D-1 | [4-S(O)CH₂CH₂SCH₂CH=CH₂] | D-1 | [4-S(O)CH₂CH₂S(O)CH₂CH=CH₂] |
| D-1 | [4-S(O)CH₂CH₂SCH₂CH=C(Me)₂] | D-1 | [4-S(O)CH₂CH₂S(O)₂CH₂CH=CH₂] |
| D-1 | [4-S(O)CH₂CH₂SCH₂CH=CCl₂] | D-1 | [4-S(O)CH₂CH₂S(O)CH₂CH=CCl₂] |
| D-1 | [4-S(O)₂CH₂CH₂SCH₂CH=CH₂] | D-1 | [4-S(O)₂CH₂CH₂S(O)CH₂CH=CH₂] |
| D-1 | [4-S(O)₂CH₂CH₂SCH₂CH=C(Me)₂] | D-1 | [4-S(O)₂CH₂CH₂S(O)₂CH₂CH=CH₂] |
| D-1 | [4-S(O)₂CH₂CH₂SCH₂CH=CCl₂] | D-1 | [4-S(O)₂CH₂CH₂S(O)CH₂CH=CCl₂] |
| D-1 | [4-SCH₂CH₂SCH₂C=CH] | D-1 | [4-SCH₂CH₂SCH₂C=CMe] |
| D-1 | [4-SCH₂CH₂S(O)CH₂C=CH] | D-1 | [4-SCH₂CH₂S(O)CH₂C=CMe] |
| D-1 | [4-SCH₂CH₂S(O)₂CH₂C=CH] | D-1 | [4-SCH₂CH₂S(O)₂CH₂C=CMe] |
| D-1 | [4-SCH₂CH₂SCH₂C=CCl] | D-1 | [4-SCH₂CH₂SCH₂C=CBr] |
| D-1 | [4-SCH₂CH₂S(O)CH₂C=CCl] | D-1 | [4-SCH₂CH₂S(O)CH₂C=CBr] |
| D-1 | [4-SCH₂CH₂SC(O)₂CH₂C=CCl] | D-1 | [4-SCH₂CH₂S(O)₂CH₂C=CBr] |
| D-1 | [4-S(O)CH₂CH₂SCH₂C=CH] | D-1 | [4-S(O)CH₂CH₂SCH₂C=CMe] |
| D-1 | [4-S(O)CH₂CH₂S(O)CH₂C=CH] | D-1 | [4-S(O)CH₂CH₂S(O)CH₂C=CMe] |
| D-1 | [4-S(O)CH₂CH₂S(O)₂CH₂C=CH] | D-1 | [4-S(O)CH₂CH₂S(O)₂CH₂C=CMe] |
| D-1 | [4-SCH₂CH₂SPen-c] | D-1 | [4-S(O)CH₂CH₂SCH₂C=CBr] |
| D-1 | [4-SCH₂CH₂S(O)Pen-c] | D-1 | [4-S(O)CH₂CH₂S(O)CH₂C=CBr] |
| D-1 | [4-SCH₂CH₂S(O)₂Pen-c] | D-1 | [4-S(O)CH₂CH₂S(O)₂CH₂C=CBr] |
| D-1 | [4-S(O)CH₂CH₂SPen-c] | D-1 | [4-S(O)₂CH₂CH₂SCH₂C=CMe] |
| D-1 | [4-S(O)CH₂CH₂S(O)Pen-c] | D-1 | [4-S(O)₂CH₂CH₂S(O)CH₂C=CMe] |
| D-1 | [4-S(O)SCH₂CH₂S(O)₂Pen-c] | D-1 | [4-S(O)₂CH₂CH₂S(O)₂CH₂C=CMe] |
| D-1 | [4-S(O)₂CH₂CH₂SPen-c] | D-1 | [4-S(O)₂CH₂CH₂SCH₂C=CBr] |
| D-1 | [4-S(O)₂CH₂CH₂S(O)Pen-c] | D-1 | [4-S(O)₂CH₂CH₂S(O)CH₂C=CBr] |
| D-1 | [4-S(O)₂SCH₂CH₂S(O)₂Pen-c] | D-1 | [4-S(O)₂CH₂CH₂S(O)₂CH₂C=CBr] |
| D-1 | [4-SCH₂CH₂SHex-c ] | D-1 | [4-S(O)₂CH₂CH₂SCH₂C=CH] |
| D-1 | [4-SCH₂CH₂S(O)Hex-c] | D-1 | [S(O)₂CH₂CH₂S(O)CH₂C=CH] |
| D-1 | [4-SCH₂CH₂S(O)₂Hex-c] | D-1 | [S(O)₂CH₂CH₂S(O)₂CH₂C=CH] |
| D-1 | [4-S(O)CH₂CH₂SHex-c] | D-1 | [4-S(O)CH₂CH₂SCH₂C=CCl] |
| D-1 | [4-S(O)CH₂CH₂S(O)Hex-c] | D-1 | [4-S(O)CH₂CH₂S(O)CH₂C=CCl] |
| D-1 | [4-S(O)CH₂CH₂S(O)₂Hex-c] | D-1 | [4-S(O)CH₂CH₂SC(O)₂CH₂C=CCl] |
| D-1 | [4-S(O)₂CH₂CH₂SHex-c] | D-1 | [4-S(O)₂CH₂CH₂SCH₂C=CCl] |
| D-1 | [4-S(O)₂CH₂CH₂S(O)Hex-c] | D-1 | [4-S(O)₂CH₂CH₂S(O)CH₂C=CCl] |
| D-1 | [4-S(O)₂CH₂CH₂S(O)₂Hex-c] | D-1 | [4-S(O)₂CH₂CH₂SC(O)₂CH₂C=CCl] |
| D-1 | [2-SCH₂Ph] | D-1 | [2-SCH₂CN] |
| D-1 | [3-SCH₂Ph] | D-1 | [3-SCH₂CN] |
| D-1 | [4-SCH₂Ph] | D-1 | [4-SCH₂CN] |
| D-1 | [2-SCH(Me)CN] | D-1 | [2-SCH₂CH₂CN] |
| D-1 | [3-SCH(Me)CN] | D-1 | [3-SCH₂CH₂CN] |
| D-1 | [4-SCH(Me)CN] | D-1 | [4-SCH₂CH₂CN] |
| D-1 | [2-S(O)CH₂Ph] | D-1 | [2-S(O)CH₂CN] |
| D-1 | [3-S(O)CH₂Ph] | D-1 | [3-S(O)CH₂CN] |
| D-1 | [4-S(O)CH₂Ph] | D-1 | [4-S(O)CH₂CN] |
| D-1 | [2-S(O)CH(Me)CN] | D-1 | [2-S(O)CH₂CH₂CN] |
| D-1 | [3-S(O)CH(Me)CN] | D-1 | [3-S(O)CH₂CH₂CN] |
| D-1 | [4-S(O)CH(Me)CN] | D-1 | [4-S(O)CH₂CH₂CN] |
| D-1 | [2-S(O)₂CH₂Ph] | D-1 | [2-S(O)₂CH₂CN] |
| D-1 | [3-S(O)₂CH₂Ph] | D-1 | [3-S(O)₂CH₂CN] |
| D-1 | [4-S(O)₂CH₂Ph] | D-1 | [4-S(O)₂CH₂CN] |
| D-1 | [2-S(O)₂CH(Me)CN] | D-1 | [2-S(O)₂CH₂CH₂CN] |
| D-1 | [3-S(O)₂CH(Me)CN] | D-1 | [3-S(O)₂CH₂CH₂CN] |
| D-1 | [4-S(O)₂CH(Me)CN] | D-1 | [4-S(O)₂CH₂CH₂CN] |
| D-1 | [2-SCH₂Ph(2-F)] | D-1 | [4-SCH₂Ph(2-F)] |
| D-1 | [2-SCH₂Ph(3-F)] | D-1 | [4-SCH₂Ph(3-F)] |
| D-1 | [2-SCH₂Ph(4-F)] | D-1 | [4-SCH₂Ph(4-F)] |
| D-1 | [2-SCH₂Ph(2-Cl)] | D-1 | [4-SCH₂Ph(2-Cl)] |
| D-1 | [2-SCH₂Ph(3-Cl)] | D-1 | [4-SCH₂Ph(3-Cl)] |
| D-1 | [2-SCH₂Ph(4-Cl)] | D-1 | [4-SCH₂Ph(4-Cl)] |
| D-1 | [2-SCH₂Ph(2-Me)] | D-1 | [4-SCH₂Ph(2-Me)] |
| D-1 | [2-SCH₂Ph(3-Me)] | D-1 | [4-SCH₂Ph(3-Me)] |
| D-1 | [2-SCH₂Ph(4-Me)] | D-1 | [4-SCH₂Ph(4-Me)] |
| D-1 | [2-SCH₂Ph(2-OMe)] | D-1 | [4-SCH₂Ph(2-OMe)] |
| D-1 | [2-SCH₂Ph(3-OMe)] | D-1 | [4-SCH₂Ph(3-OMe)] |
| D-1 | [2-SCH₂Ph(4-OMe)] | D-1 | [4-SCH₂Ph(4-OMe)] |
| D-1 | [2-S(O)CH₂Ph(2-F)] | D-1 | [4-S(O)CH₂Ph(2-F)] |
| D-1 | [2-S(O)CH₂Ph(3-F)] | D-1 | [4-S(O)CH₂Ph(3-F)] |
| D-1 | [2-S(O)CH₂Ph(4-F)] | D-1 | [4-S(O)CH₂Ph(4-F)] |
| D-1 | [2-S(O)CH₂Ph(2-Cl)] | D-1 | [4-S(O)CH₂Ph(2-Cl)] |
| D-1 | [2-S(O)CH₂Ph(3-Cl)] | D-1 | [4-S(O)CH₂Ph(3-Cl)] |
| D-1 | [2-S(O)CH₂Ph(4-Cl)] | D-1 | [4-S(O)CH₂Ph(4-Cl)] |
| D-1 | [2-S(O)CH₂Ph(2-Me)] | D-1 | [4-S(O)CH₂Ph(2-Me)] |
| D-1 | [2-S(O)CH₂Ph(3-Me)] | D-1 | [4-S(O)CH₂Ph(3-Me)] |
| D-1 | [2-S(O)CH₂Ph(4-Me)] | D-1 | [4-S(O)CH₂Ph(4-Me)] |
| D-1 | [2-S(O)CH₂Ph(2-OMe)] | D-1 | [4-S(O)CH₂Ph(2-OMe)] |
| D-1 | [2-S(O)CH₂Ph(3-OMe)] | D-1 | [4-S(O)CH₂Ph(3-OMe)] |
| D-1 | [2-S(O)CH₂Ph(4-OMe)] | D-1 | [4-S(O)CH₂Ph(4-OMe)] |
| D-1 | [2-S(O)₂CH₂Ph(2-F)] | D-1 | [4-S(O)₂CH₂Ph(2-F)] |
| D-1 | [2-S(O)₂CH₂Ph(3-F)] | D-1 | [4-S(O)₂CH₂Ph(3-F)] |
| D-1 | [2-S(O)₂CH₂Ph(4-F)] | D-1 | [4-S(O)₂CH₂Ph(4-F)] |
| D-1 | [2-S(O)₂CH₂Ph(2-Cl)] | D-1 | [4-S(O)₂CH₂Ph(2-Cl)] |
| D-1 | [2-S(O)₂CH₂Ph(3-Cl)] | D-1 | [4-S(O)₂CH₂Ph(3-Cl)] |
| D-1 | [2-S(O)₂CH₂Ph(4-Cl)] | D-1 | [4-S(O)₂CH₂Ph(4-Cl)] |
| D-1 | [2-S(O)₂CH₂Ph(2-Me)] | D-1 | [4-S(O)₂CH₂Ph(2-Me)] |
| D-1 | [2-S(O)₂CH₂Ph(3-Me)] | D-1 | [4-S(O)₂CH₂Ph(3-Me)] |
| D-1 | [2-S(O)₂CH₂Ph(4-Me)] | D-1 | [4-S(O)₂CH₂Ph(4-Me)] |
| D-1 | [2-S(O)₂CH₂Ph(2-OMe)] | D-1 | [4-S(O)₂CH₂Ph(2-OMe)] |
| D-1 | [2-S(O)₂CH₂Ph(3-OMe)] | D-1 | [4-S(O)₂CH₂Ph(3-OMe)] |
| D-1 | [2-S(O)₂CH₂Ph(4-OMe)] | D-1 | [4-S(O)₂CH₂Ph(4-OMe)] |
| D-1 | [4-SCH₂C(=NOH)H] | D-1 | [4-SCH₂C(=NOCH₂CH=CH₂)Me] |
| D-1 | [4-SCH₂CH₂C(=NOH)Me] | D-1 | [4-SCH₂C(=NOCH₂C≡CH)Me] |
| D-1 | [4-SCH₂C(=NOMe)H] | D-1 | [4-SCH₂C(=NOCH₂CH₂F)Me] |
| D-1 | [4-SCH₂CH₂C(=NOMe)Me] | D-1 | [4-SCH₂C(=NOCH₂CH₂Cl)Me] |
| D-1 | [4-SCH₂C(=NOMe)OMe] | D-1 | [4-SCH₂C(=NOCH₂CHF₂)Me] |
| D-1 | [4-SCH₂C(=NOEt)Me] | D-1 | [4-SCH₂C(=NOCH₂CHCl₂)Me] |
| D-1 | [4-SCH₂C(=NOPr-n)Me] | D-1 | [4-SCH₂C(=NOCH₂CF₃)Me] |
| D-1 | [4-SCH₂C(=NOPr-i)Me] | D-1 | [4-SCH₂C(=NOCH₂CH₂CH₂Cl)Me] |
| D-1 | [4-SCH₂C(=NOH)Me] | D-1 | [4-SCH₂C(=NOCH₂CH₂OMe)Me] |
| D-1 | [4-SCH₂C(=NOH)NH₂] | D-1 | [4-SCH₂C(=NOCH₂CH₂SMe)Me] |
| D-1 | [4-SCH₂C(=NOMe)Me] | D-1 | [4-SCH₂C(=NOCH₂Pr-c)Me] |
| D-1 | [4-SCH₂C(=NOMe)NH₂] | D-1 | [4-SCH₂C(=NOCH₂(Ph-2-F))Me] |
| D-1 | [4-SCH₂C(=NOMe)SMe] | D-1 | [4-SCH₂C(=NOCH₂(Ph-3-F))Me] |
| D-1 | [4-SCH₂C(=NOMe)OMe] | D-1 | [4-SCH₂C(=NOCH₂(Ph-4-F))Me] |
| D-1 | [4-SCH₂CH₂C(=NOH)Me] | D-1 | [4-SCH₂C(=NOCH₂(Ph-2-OMe))Me] |
| D-1 | [4-SCH₂CH₂C(=NOMe)Me] | D-1 | [4-SCH₂C(=NOCH₂(Ph-3-OMe))Me] |
| D-1 | [4-SCH₂CH₂C(=NOEt)Me] | D-1 | [4-SCH₂C(=NOCH₂(Ph-4-OMe))Me] |
| D-1 | [4-SCH₂C(=NOCH₂(Ph-2-Cl))Me] | D-1 | [4-SCH₂C(=NOCH₂(Ph-2-Me))Me] |
| D-1 | [4-SCH₂C(=NOCH₂(Ph-3-Cl))Me] | D-1 | [4-SCH₂C(=NOCH₂(Ph-3-Me))Me] |
| D-1 | [4-SCH₂C(=NOCH₂(Ph-4-Cl))Me] | D-1 | [4-SCH₂C(=NOCH₂(Ph-4-Me))Me] |
| D-1 | [4-S(O)CH₂C(=NOH)H] | D-1 | [4-S(O)CH₂C(=NOCH₂CH=CH₂)Me] |
| D-1 | [4-S(O)CH₂CH₂C(=NOH)Me] | D-1 | [4-S(O)CH₂C(=NOCH₂C=CH)Me] |
| D-1 | [4-S(O)CH₂C(=NOMe)H] | D-1 | [4-S(O)CH₂C(=NOCH₂CH₂F)Me] |
| D-1 | [4-S(O)CH₂CH₂C(=NOMe)Me] | D-1 | [4-S(O)CH₂C(=NOCH₂CH₂Cl)Me] |
| D-1 | [4-S(O)CH₂C(=NOMe)OMe] | D-1 | [4-S(O)CH₂C(=NOCH₂CHF₂)Me] |
| D-1 | [4-S(O)CH₂C(=NOEt)Me] | D-1 | [4-S(O)CH₂C(=NOCH₂CHCl₂)Me] |
| D-1 | [4-S(O)CH₂C(=NOPr-n)Me] | D-1 | [4-S(O)CH₂C(=NOCH₂CF₃)Me] |
| D-1 | [4-S(O)CH₂C(=NOPr-i)Me] | D-1 | [4-S(O)CH₂C(=NOCH₂CH₂CH₂Cl)Me] |
| D-1 | [4-S(O)CH₂C(=NOMe)NH₂] | D-1 | [4-S(O)CH₂C(=NOCH₂(Ph-2-F))Me] |
| D-1 | [4-S(O)CH₂C(=NOMe)SMe] | D-1 | [4-S(O)CH₂C(=NOCH₂(Ph-3-F))Me] |
| D-1 | [4-S(O)CH₂C(=NOMe)OMe] | D-1 | [4-S(O)CH₂C(=NOCH₂(Ph-4-F))Me] |
| D-1 | [4-S(O)CH₂CH₂C(=NOH)Me] | D-1 | [4-S(O)CH₂C(=NOCH₂(Ph-2-OMe))Me] |
| D-1 | [4-S(O)CH₂CH₂C(=NOMe)Me] | D-1 | [4-S(O)CH₂C(=NOCH₂(Ph-3-OMe))Me] |
| D-1 | [4-S(O)CH₂CH₂C(=NOEt)Me] | D-1 | [4-S(O)CH₂C(=NOCH₂(Ph-4-OMe))Me] |
| D-1 | [4-S(O)CH₂C(=NOH)Me] | D-1 | [4-S(O)CH₂C(=NOCH₂(Ph-2-Cl))Me] |
| D-1 | [4-S(O)CH₂C(=NOCH₂CH₂OMe)Me] | D-1 | [4-S(O)CH₂C(=NOCH₂(Ph-3-Cl))Me] |
| D-1 | [4-S(O)CH₂C(=NOH)NH₂] | D-1 | [4-S(O)CH₂C(=NOCH₂(Ph-4-Cl))Me] |
| D-1 | [4-S(O)CH₂C(=NOCH₂CH₂SMe)Me] | D-1 | [4-S(O)CH₂C(=NOCH₂(Ph-2-Me))Me] |
| D-1 | [4-S(O)CH₂C(=NOMe)Me] | D-1 | [4-S(O)CH₂C(=NOCH₂(Ph-2-Me))Me] |
| D-1 | [4-S(O)CH₂C(=NOCH₂Pr-c)Me] | D-1 | [4-S(O)CH₂C(=NOCH₂(Ph-4-Me))Me] |
| D-1 | [4-S(O)₂CH₂C(=NOH)H] | D-1 | [4-S(O)₂CH₂C(=NOCH₂CH=CH₂)Me] |
| D-1 | [4-S(O)₂CH₂CH₂C(=NOH)Me] | D-1 | [4-S(O)₂CH₂C(=NOCH₂C=CH)Me] |
| D-1 | [4-S(O)₂CH₂C(=NOMe)H] | D-1 | [4-S(O)₂CH₂C(=NOCH₂CH₂F)Me] |
| D-1 | [4-S(O)₂CH₂CH₂C(=NOMe)Me] | D-1 | [4-S(O)₂CH₂C(=NOCH₂CH₂Cl)Me] |
| D-1 | [4-S(O)₂CH₂C(=NOMe)OMe] | D-1 | [4-S(O)₂CH₂C(=NOCH₂CHF₂)Me] |
| D-1 | [4-S(O)₂CH₂C(=NOEt)Me] | D-1 | [4-S(O)₂CH₂C(=NOCH₂CHCl₂)Me] |
| D-1 | [4-S(O)₂CH₂C(=NOPr-n)Me] | D-1 | [4-S(O)₂CH₂C(=NOCH₂CF₃)Me] |
| D-1 | [4-S(O)₂CH₂C(=NOPr-i)Me] | D-1 | [4-S(O)₂CH₂C(=NOCH₂CH₂CH₂Cl)Me] |
| D-1 | [4-S(O)₂CH₂C(=NOH)Me] | D-1 | [4-S(O)₂CH₂C(=NOCH₂CH₂OMe)Me] |
| D-1 | [4-S(O)₂CH₂C(=NOH)NH₂] | D-1 | [4-S(O)₂CH₂C(=NOCH₂CH₂SMe)Me] |
| D-1 | [4-S(O)₂CH₂C(=NOMe)Me] | D-1 | [4-S(O)₂CH₂C(=NOCH₂Pr-c)Me] |
| D-1 | [4-S(O)₂CH₂C(=NOMe)NH₂] | D-1 | [4-S(O)₂CH₂C(=NOCH₂(Ph-2-F))Me] |
| D-1 | [4-S(O)₂CH₂C(=NOMe)SMe] | D-1 | [4-S(O)₂CH₂C(=NOCH₂(Ph-3-F))Me] |
| D-1 | [4-S(O)₂CH₂C(=NOMe)OMe] | D-1 | [4-S(O)₂CH₂C(=NOCH₂(Ph-4-F))Me] |
| D-1 | [4-S(O)₂CH₂CH₂C(=NOH)Me] | D-1 | [4-S(O)₂CH₂C(=NOCH₂(Ph-2-OMe))Me] |
| D-1 | [4-S(O)₂CH₂CH₂C(=NOMe)Me] | D-1 | [4-S(o)₂CH₂C(=NOCH₂(Ph-3-OMe))Me] |
| D-1 | [4-S(O)₂CH₂CH₂C(=NOEt)Me] | D-1 | [4-S(O)₂CH₂C(=NOCH₂(Ph-4-OMe))Me] |
| D-1 | [4-SCH₂(U-1a)] | D-1 | [4-S(O)₂CH₂C(=NOCH₂(Ph-2-Me))Me] |
| D-1 | [4-SCH₂(U-2a)] | D-1 | [4-S(O)₂CH₂C(=NOCH₂(Ph-3-Me))Me] |
| D-1 | [4-SCH₂(U-3a)] | D-1 | [4-S(O)₂CH₂C(=NOCH₂(Ph-4-Me))Me] |
| D-1 | [4-SCH₂(U-4a)] | D-1 | [4-S(O)₂CH₂C(=NOCH₂(Ph-2-Cl))Me] |
| D-1 | [4-SCH₂(U-5a) ] | D-1 | [4-S(O)₂CH₂C(=NOCH₂(Ph-3-Cl))Me] |
| D-1 | [4-SCH₂(U-9a)] | D-1 | [4-S(O)₂CH₂C(=NOCH₂(Ph-4-Cl))Me] |
| D-1 | [4-SCH₂(U-1a)] | D-1 | [4-S(O)₂CH₂C(=NOCH₂(Ph-2-Me))Me] |
| D-1 | [4-SCH₂(U-2a)] | D-1 | [4-S(O)₂CH₂C(=NOCH₂(Ph-3-Me))Me] |
| D-1 | [4-SCH₂(U-3a)] | D-1 | [4-S(O)₂CH₂C(=NOCH₂(Ph-4-Me))Me] |
| D-1 | [4-SCH₂(U-4a)] | D-1 | [4-S(O)₂CH₂C(=NOCH₂(Ph-2-Cl))Me] |
| D-1 | [4-SCH₂(U-5a) ] | D-1 | [4-S(O)₂CH₂C(=NOCH₂(Ph-3-Cl))Me] |
| D-1 | [4-SCH₂(U-9a)] | D-1 | [4-S(O)₂CH₂C(=NOCH₂(Ph-4-Cl))Me] |
| D-1 | [4-SCH₂(U-11a)] | D-1 | [4-SCH₂(U-12a)] |
| D-1 | [4-SCH₂(U-13a)] | D-1 | [4-SCH₂(U-14a)] |
| D-1 | [4-SCH₂(U-15a)] | D-1 | [4-SCH₂(U-16a)] |
| D-1 | [4-SCH₂(U-17a)] | D-1 | [4-SCH₂(U-20a)] |
| D-1 | [4-SCH₂(U-31a)] | D-1 | [4-SCH₂(U-32a)] |
| D-1 | [4-S(O)CH₂(U-1a)] | D-1 | [4-S(O)CH₂(U-2a)] |
| D-1 | [4-S(O)CH₂(U-3a)] | D-1 | [4-S(O)CH₂(U-4a)] |
| D-1 | [4-S(O)CH₂(U-5a)] | D-1 | [4-S(O)CH₂(U-9a)] |
| D-1 | [4-S(O)CH₂(U-11a)] | D-1 | [4-S(O)CH₂(U-12a)] |
| D-1 | [4-S(O)CH₂(U-13a)] | D-1 | [4-S(O)CH₂(U-14a)] |
| D-1 | [4-S(O)CH₂(U-15a)] | D-1 | [4-S(O)CH₂(U-16a)] |
| D-1 | [4-S(O)CH₂(U-17a)] | D-1 | [4-S(O)CH₂(U-20a)] |
| D-1 | [4-S(O)CH₂(U-31a)] | D-1 | [4-S(O)CH₂(U-32a)] |
| D-1 | [4-S(O)₂CH₂(U-1a)] | D-1 | [4-S(O)₂CH₂(U-2a)] |
| D-1 | [4-S(O)₂CH₂(U-3a)] | D-1 | [4-S(O)₂CH₂(U-4a)] |
| D-1 | [4-S(O)₂CH₂(U-5a)] | D-1 | [4-S(O)₂CH₂(U-9a)] |
| D-1 | [4-S(O)₂CH₂(U-11a)] | D-1 | [4-S(O)₂CH₂(U-12a)] |
| D-1 | [4-S(O)₂CH₂(U-13a)] | D-1 | [4-S(O)₂CH₂(U-14a)] |
| D-1 | [4-S(O)₂CH₂(U-15a)] | D-1 | [4-S(O)₂CH₂(U-16a)] |
| D-1 | [4-S(O)₂CH₂(U-17a)] | D-1 | [4-S(O)₂CH₂(U-20a)] |
| D-1 | [4-S(O)₂CH₂(U-31a)] | D-1 | [4-S(O)₂CH₂(U-32a)] |
| D-1 | [4-SCH₂(Q-1-1)] | D-1 | [4-SCH₂(Q-2)] |
| D-1 | [4-SCH₂(Q-3)] | D-1 | [4-SCH₂(Q-4)] |
| D-1 | [4-SCH₂(Q-5)] | D-1 | [4-SCH₂(Q-8-1)] |
| D-1 | [4-SCH₂(Q-9-1)] | D-1 | [4-SCH₂(Q-14-1)] |
| D-1 | [4-SCH₂(Q-15-1)] | D-1 | [4-SCH₂(Q-16-1)] |
| D-1 | [4-SCH₂(Q-17)] | D-1 | [4-SCH₂(Q-18)] |
| D-1 | [4-SCH₂(Q-18-1)] | D-1 | [4-SCH₂(Q-19)] |
| D-1 | [4-SCH₂(Q-20)] | D-1 | [4-SCH₂ON=C(Me)H] |
| D-1 | [4-SCH₂ON=C(Me)₂] | D-1 | [4-SCH₂ON=C(Me)Et] |
| D-1 | [4-SCH₂ON=C(CH₂)₄] | D-1 | [4-SCH₂ON=C(CH₂)₅] |
| D-1 | [2-S(T-1-5)] | D-1 | [4-S(T-1-5)] |
| D-1 | [4-SPr-c] | D-1 | [4-SBu-c)] |
| D-1 | [4-SPen-c] | D-1 | [4-SHex-c)] |
| D-1 | [4-S(U-1a)] | D-1 | [4-S(U-2a)] |
| D-1 | [4-S(U-4a)] | D-1 | [4-S(U-5a)] |
| D-1 | [4-S(U-12a)] | D-1 | [4-S(U-14a)] |
| D-1 | [4-S(U-15a)] | D-1 | [4-S(U-16a)] |
| D-1 | [4-S(O)CH₂(Q-1-1)] | D-1 | [4-S(O)CH₂(Q-2)] |
| D-1 | [4-S(O)CH₂(Q-3)] | D-1 | [4-S(O)CH₂(Q-4)] |
| D-1 | [4-S(O)CH₂(Q-5)] | D-1 | [4-S(O)CH₂(Q-8-1)] |
| D-1 | [4-S(O)CH₂(Q-9-1)] | D-1 | [4-S(O)CH₂(Q-14-1)] |
| D-1 | [4-S(O)CH₂(Q-15-1)] | D-1 | [4-S(O)CH₂(Q-16-1)] |
| D-1 | [4-S(O)CH₂(Q-17)] | D-1 | [4-S(O)CH₂(Q-18)] |
| D-1 | [4-S(O)CH₂(Q-18-1)] | D-1 | [4-S(O)CH₂(Q-19)] |
| D-1 | [4-S(O)CH₂(Q-20)] | D-1 | [4-S(O)CH₂ON=C(Me)H] |
| D-1 | [4-S(O)CH₂ON=C(Me)₂] | D-1 | [4-S(O)CH₂ON=C(Me)Et] |
| D-1 | [4-S(O)CH₂ON=C(CH₂)₄] | D-1 | [4-S(O)CH₂ON=C(CH₂)₅] |
| D-1 | [2-S(O)(T-1-5)] | D-1 | [4-S(O)(T-1-5)] |
| D-1 | [4-S(O)Pr-c] | D-1 | [4-S(O)Bu-c] |
| D-1 | [4-S(O)Pen-c] | D-1 | [4-S(O)Hex-c] |
| D-1 | [4-S(O)(U-1a)] | D-1 | [4-S(O)(U-2a)] |
| D-1 | [4-S(O)(U-4a)] | D-1 | [4-S(O)(U-5a)] |
| D-1 | [4-S(O)(U-12a)] | D-1 | [4-S(O)(U-14a)] |
| D-1 | [4-S(O)(U-15a)] | D-1 | [4-S(O)(U-16a)] |
| D-1 | [4-S(O)₂CH₂(Q-1-1)] | D-1 | [4-S(O)₂CH₂(Q-2)] |
| D-1 | [4-S(O)₂CH₂(Q-3)] | D-1 | [4-S(O)₂CH₂(Q-4)] |
| D-1 | [4-S(O)₂CH₂(Q-5)] | D-1 | [4-S(O)₂CH₂(Q-8-1)] |
| D-1 | [4-S(O)₂CH₂(Q-9-1)] | D-1 | [4-S(O)₂CH₂(Q-14-1)] |
| D-1 | [4-S(O)₂CH₂(Q-15-1)] | D-1 | [4-S(O)₂CH₂(Q-16-1)] |
| D-1 | [4-S(O)₂CH₂(Q-17)] | D-1 | [4-S(O)₂CH₂(Q-18)] |
| D-1 | [4-S(O)₂CH₂(Q-18-1)] | D-1 | [4-S(O)₂CH₂(Q-19)] |
| D-1 | [4-S(O)₂CH₂(Q-20)] | D-1 | [4-S(O)₂CH₂ON=C(Me)H] |
| D-1 | [4-S(O)₂CH₂ON=C(Me)₂] | D-1 | [4-S(O)₂CH₂ON=C(Me)Et] |
| D-1 | [4-S(O)₂CH₂ON=C(CH₂)₄] | D-1 | [4-S(O)₂CH₂ON=C(CH₂)₅] |
| D-1 | [2-S(O)₂(T-1-5)] | D-1 | [4-S(O)₂ (T-1-5)] |
| D-1 | [4-S(O)₂Pr-c] | D-1 | [4-S(O)₂Bu-c] |
| D-1 | [4-S(O)₂Pen-c] | D-1 | [4-S(O)₂Hex-c] |
| D-1 | [4-S(O)₂(U-1a)] | D-1 | [4-S(O)₂(U-2a)] |
| D-1 | [4-S(O)₂(U-4a)] | D-1 | [4-S(O)₂(U-5a)] |
| D-1 | [4-S(O)₂(U-12a)] | D-1 | [4-S(O)₂(U-14a)] |
| D-1 | [4-S(O)₂(U-15a)] | D-1 | [4-S(O)₂(U-16a)] |
| D-1 | [4-NH₂] | D-1 | [4-NHMe] |
| D-1 | [4-NHEt] | D-1 | [4-NHPr-n] |
| D-1 | [4-NHPr-i] | D-1 | [4-NHBu-tert] |
| D-1 | [4-N(Me)Me] | D-1 | [4-N(Me)Et] |
| D-1 | [4-N(Me)Pr-n] | D-1 | [4-N(Me)Pr-i] |
| D-1 | [4-N(Me)Bu-tert] | D-1 | [4-N(Et)Et] |
| D-1 | [2-CN] | D-1 | [3-CN] |
| D-1 | [4-CN] | D-1 | [2-NO₂] |
| D-1 | [3-NO₂] | D-1 | [4-NO₂] |
| D-1 | [2-C(O)OH] | D-1 | [2-C(O)H] |
| D-1 | [3-C(O)OH] | D-1 | [3-C(O)H] |
| D-1 | [4-C(O)OH] | D-1 | [4-C(O)H] |
| D-1 | [2-C(O)Me] | D-1 | [2-C(O)Et] |
| D-1 | [3-C(O)Me] | D-1 | [3-C(O)Et] |
| D-1 | [4-C(O)Me] | D-1 | [4-C(O)Et] |
| D-1 | [2-C(O)NH₂] | D-1 | [2-C(O)NHMe] |
| D-1 | [3-C(O)NH₂] | D-1 | [3-C(O)NHMe] |
| D-1 | [4-C(O)NH₂] | D-1 | [4-C(O)NHMe] |
| D-1 | [2-C(O)N(Me)₂] | D-1 | [2-C(O)NHEt] |
| D-1 | [3-C(O)N(Me)₂] | D-1 | [3-C(O)NHEt] |
| D-1 | [4-C(O)N(Me)₂] | D-1 | [4-C(O)NHEt] |
| D-1 | [2-C(O)N(Me)Et] | D-1 | [2-C(O)NHPr-n] |
| D-1 | [3-C(O)N(Me)Et] | D-1 | [3-C(O)NHPr-n] |
| D-1 | [4-C(O)N(Me)Et] | D-1 | [4-C(O)NHPr-n] |
| D-1 | [2-C(O)N(Et)₂] | D-1 | [2-C(O)NHPr-i] |
| D-1 | [3-C(O)N(Et)₂] | D-1 | [3-C(O)NHPr-i] |
| D-1 | [4-C(O)N(Et)₂] | D-1 | [4-C(O)NHPr-i] |
| D-1 | [2-C(O)NHCH₂CF₃] | D-1 | [2-C(O)N(Me)CH₂CF₃] |
| D-1 | [3-C(O)NHCH₂CF₃] | D-1 | [3-C(O)N(Me)CH₂CF₃] |
| D-1 | [4-C(O)NHCH₂CF₃] | D-1 | [4-C(O)N(Me)CH₂CF₃] |
| D-1 | [4-C(=NOH)H] | D-1 | [4-C(=NOCH₂CH=CH₂)Me] |
| D-1 | [4-C(=NOH)Me] | D-1 | [4-C(=NOCH₂C=CH)Me] |
| D-1 | [4-C(=NOMe)H] | D-1 | [4-C(=NOCH₂CH₂F)Me] |
| D-1 | [4-C(=NOMe)Me] | D-1 | [4-C(=NOCH₂CH₂Cl)Me] |
| D-1 | [4-OCH₂C(=NOMe)OMe] | D-1 | [4-C(=NOCH₂CHF₂)Me] |
| D-1 | [4-OCH₂C(=NOEt)Me] | D-1 | [4-C(=NOCH₂CHCl₂)Me] |
| D-1 | [4-OCH₂C(=NOPr-n)Me] | D-1 | [4-C(=NOCH₂CF₃)Me] |
| D-1 | [4-OCH₂C(=NOPr-i)Me] | D-1 | [4-C(=NOCH₂CH₂CH₂Cl)Me] |
| D-1 | [4-OCH₂C(=NOH)Me] | D-1 | [4-C(=NOCH₂CH₂OMe)Me] |
| D-1 | [4-OCH₂C(=NOH)NH₂] | D-1 | [4-C(=NOCH₂CH₂SMe)Me] |
| D-1 | [4-OCH₂C(=NOMe)Me] | D-1 | [4-C(=NOCH₂Pr-c)Me] |
| D-1 | [4-OCH₂C(=NOMe)NH₂] | D-1 | [4-C(=NOCH₂(Ph-2-F))Me] |
| D-1 | [4-OCH₂C(=NOMe)SMe] | D-1 | [4-C(=NOCH₂(Ph-3-F))Me] |
| D-1 | [4-OCH₂C(=NOMe)OMe] | D-1 | [4-C(=NOCH₂(Ph-4-F))Me] |
| D-1 | [4-OCH₂CH₂C(=NOH)Me] | D-1 | [4-C(=NOCH₂(Ph-2-OMe))Me] |
| D-1 | [4-OCH₂CH₂C(=NOMe)Me] | D-1 | [4-C(=NOCH₂(Ph-3-OMe))Me] |
| D-1 | [4-OCH₂CH₂C(=NOEt)Me] | D-1 | [4-C(=NOCH₂(Ph-4-OMe))Me] |
| D-1 | [4-C(=NOCH₂(Ph-2-Cl))Me ] | D-1 | [4-C(=NOCH₂(Ph-2-Me))Me] |
| D-1 | [4-C(=NOCH₂(Ph-3-Cl))Me] | D-1 | [4-C(=NOCH₂(Ph-3-Me))Me] |
| D-1 | [4-C(=NOCH₂(Ph-4-Cl))Me] | D-1 | [4-C(=NOCH₂(Ph-4-Me))Me] |
| D-1 | [3-Ph] | D-1 | [4-Ph] |
| D-1 | [4-Ph(2-F)] | D-1 | [4-Ph(3-F)] |
| D-1 | [4-Ph(4-F)] | D-1 | [4-Ph(2-Cl)] |
| D-1 | [4-Ph(3-Cl)] | D-1 | [4-Ph(4-Cl)] |
| D-1 | [4-Ph(2-Me)] | D-1 | [4-Ph(3-Me)] |
| D-1 | [4-Ph(4-Me)] | D-1 | [4-Ph(2-OMe)] |
| D-1 | [4-Ph(3-OMe)] | D-1 | [4-Ph(4-OMe)] |
| D-1 | [2-Br] | D-1 | [4-1] |
| D-1 | [3-Br] | D-1 | [4-1] |
| D-1 | [4-Br] | D-1 | [4-1] |
| D-1 | [2,3-F₂] | D-1 | [2,5-F₂] |
| D-1 | [2,6-F₂] | D-1 | [3,4-F₂] |
| D-1 | [3,5-F₂] | D-1 | [2,3-Cl₂] |
| D-1 | [2,5-Cl₂] | D-1 | [2,6-Cl₂] |
| D-1 | [3,4-Cl₂] | D-1 | [3,5-Cl₂] |
| D-1 | [2,3-(Me)₂] | D-1 | [2,5-(Me)₂] |
| D-1 | [2,6-(Me)₂] | D-1 | [3,4-(Me)₂] |
| D-1 | [3,5-(Me)₂] | D-1 | [2,4-(Me)₂] |
| D-1 | [2,3-(OMe)₂] | D-1 | [2,4-(OMe)₂] |
| D-1 | [2,5-(OMe)₂] | D-1 | [2,6-(OMe)₂] |
| D-1 | [3,4-(OMe)₂] | D-1 | [3,5-(OMe)₂] |
| D-1 | [2-F-3-Cl] | D-1 | [2-F-4-Cl] |
| D-1 | [2-F-5-Cl] | D-1 | [2-F-6-Cl] |
| D-1 | [2-F-3-Me] | D-1 | [2-F-5-Me] |
| D-1 | [2-F-6-Me] | D-1 | [2-F-3-OMe] |
| D-1 | [2-F-5-OMe] | D-1 | [2-F-6-OMe] |
| D-1 | [2-Cl-3-Me] | D-1 | [2-Cl-5-Me] |
| D-1 | [2-Cl-6-Me] | D-1 | [2-Cl-3-OMe] |
| D-1 | [2-Cl-5-OMe] | D-1 | [2-Cl-6-OMe] |
| D-1 | [2-Me-3-F] | D-1 | [2-Me-4-F] |
| D-1 | [2-Me-5-F] | D-1 | [2-Me-3-Cl] |
| D-1 | [2-Me-4-Cl] | D-1 | [2-Me-5-Cl] |
| D-1 | [3-F-5-Cl] | D-1 | [3-F-5-Me] |
| D-1 | [3-F-5-OMe] | D-1 | [3-F-5-CF₃] |
| D-1 | [3-F-5-OCF₃] | D-1 | [3-F-5-SMe] |
| D-1 | [3-Cl-5-Me] | D-1 | [3-Cl-5-OMe] |
| D-1 | [3-Cl-5-CF₃] | D-1 | [3-Cl-5-OCF₃] |
| D-1 | [3-Cl-5-SMe] | D-1 | [3-Cl-5-S(O)Me] |
| D-1 | [2,4,6-F₃] | D-1 | [2,4,5-F₃] |
| D-1 | [3,4,5-F₃] | D-1 | [2,4,6-(Me)₃] |
| D-1 | [3,5-F₂-4-OMe] | D-1 | [2-F-4-OEt] |
| D-1 | [2-F-4-OPr-n] | D-1 | [2-F-4-OPr-i] |
| D-1 | [2-F-4-OPr-c] | D-1 | [2-F-4-OBu-n] |
| D-1 | [2-F-4-OBu-i] | D-1 | [2-F-4-OBu-sec] |
| D-1 | [2-F-4-OBu-tert] | D-1 | [2-F-4-OBu-c] |
| D-1 | [2-F-4-OPen-c] | D-1 | [2-F-4-OHex-c] |
| D-1 | [2-F-4-OCH₂CH=CH₂] | D-1 | [2-F-4-OCH₂C=CH] |
| D-1 | [2-F-4-OCH₂CH=C(Me)₂] | D-1 | [2-F-4-OCH₂C=CMe] |
| D-1 | [2-F-4-OCHF₂] | D-1 | [2-F-4-OCF₃] |
| D-1 | [2-F-4-OCH₂CH₂F] | D-1 | [2-F-4-OCH₂CH₂Cl] |
| D-1 | [2-F-4-OCH₂CHF₂] | D-1 | [2-F-4-OCH₂CH₂CF₃] |
| D-1 | [2-F-4-OCH₂CF₂CHF₂] | D-1 | [2-F-4-OCH₂CF₂CF₃] |
| D-1 | [2-F-4-OCH₂CH=CF₂] | D-1 | [2-F-4-OCH₂CH=CCl₂] |
| D-1 | [2-F-4-OCH₂CH₂CF=CF₂] | D-1 | [2-F-4-OCH₂CH₂CH=CF₂] |
| D-1 | [2-F-4-OCH₂C=CCl] | D-1 | [2-F-4-OCH₂C=CBr] |
| D-1 | [2-F-4-OCH₂CH₂OMe] | D-1 | [2-F-4-OCH₂CH₂SMe] |
| D-1 | [3-F-4-OMe] | D-1 | [3-F-4-OEt] |
| D-1 | [3-F-4-OPr-n] | D-1 | [3-F-4-OPr-i] |
| D-1 | [3-F-4-OPr-c] | D-1 | [3-F-4-OBu-n] |
| D-1 | [3-F-4-OBu-i] | D-1 | [3-F-4-OBu-sec] |
| D-1 | [3-F-4-OBu-tert] | D-1 | [3-F-4-OBu-c] |
| D-1 | [3-F-4-OPen-c] | D-1 | [3-F-4-OHex-c] |
| D-1 | [3-F-4-OCH₂CH=CH₂] | D-1 | [3-F-4-OCH₂C≡CH] |
| D-1 | [3-F-4-OCH₂CH=C(Me)₂] | D-1 | [3-F-4-OCH₂C≡CMe] |
| D-1 | [3-F-4-OCHF₂] | D-1 | [3-F-4-OCF₃] |
| D-1 | [3-F-4-OCH₂CH₂F] | D-1 | [3-F-4-OCH₂CH₂Cl] |
| D-1 | [3-F-4-OCH₂CHF₂] | D-1 | [3-F-4-OCH₂CH₂CF₃] |
| D-1 | [3-F-4-OCH₂CF₂CHF₂] | D-1 | [3-F-4-OCH₂CF₂CF₃] |
| D-1 | [3-F-4-OCH₂CH=CF₂] | D-1 | [3-F-4-OCH₂CH=CCl₂] |
| D-1 | [3-F-4-OCH₂CH₂CF=CF₂] | D-1 | [3-F-4-OCH₂CH₂CH=CF₂] |
| D-1 | [3-F-4-OCH₂C≡CCl] | D-1 | [3-F-4-OCH₂C≡CBr] |
| D-1 | [3-F-4-OCH₂CH₂OMe] | D-1 | [3-F-4-OCH₂CH₂SMe] |
| D-1 | [3,5-Cl₂-4-OMe] | D-1 | [2-Cl-4-OEt] |
| D-1 | [2-Cl-4-OPr-n] | D-1 | [2-Cl-4-OPr-i] |
| D-1 | [2-Cl-4-OPr-c] | D-1 | [2-Cl-4-OBu-n] |
| D-1 | [2-Cl-4-OBu-i] | D-1 | [2-Cl-4-OBu-sec] |
| D-1 | [2-Cl-4-OBu-tert] | D-1 | [2-Cl-4-OBu-c] |
| D-1 | [2-Cl-4-OPen-c] | D-1 | [2-Cl-4-OHex-c] |
| D-1 | [2-Cl-4-OCH₂CH=CH₂] | D-1 | [2-Cl-4-OCH₂C≡CH] |
| D-1 | [2-Cl-4-OCH₂CH=C(Me)₂] | D-1 | [2-Cl-4-OCH₂C≡CMe] |
| D-1 | [2-Cl-4-OCHF₂] | D-1 | [2-Cl-4-OCF₃] |
| D-1 | [2-Cl-4-OCH₂CH₂F] | D-1 | [2-Cl-4-OCH₂CH₂Cl] |
| D-1 | [2-Cl-4-OCH₂CHF₂] | D-1 | [2-Cl-4-OCH₂CH₂CF₃] |
| D-1 | [2-Cl-4-OCH₂CF₂CHF₂] | D-1 | [2-Cl-4-OCH₂CF₂CF₃] |
| D-1 | [2-Cl-4-OCH₂CH=CF₂] | D-1 | [2-Cl-4-OCH₂CH=CCl₂] |
| D-1 | [2-Cl-4-OCH₂CH₂CF=CF₂] | D-1 | [2-Cl-4-OCH₂CH₂CH=CF₂] |
| D-1 | [2-Cl-4-OCH₂C≡CCl] | D-1 | [2-Cl-4-OCH₂C≡CBr] |
| D-1 | [2-Cl-4-OCH₂CH₂OMe] | D-1 | [2-Cl-4-OCH₂CH₂SMe] |
| D-1 | [3-Cl-4-OMe] | D-1 | [3-Cl-4-OEt] |
| D-1 | [3-Cl-4-OPr-n] | D-1 | [3-Cl-4-OPr-i] |
| D-1 | [3-Cl-4-OPr-c] | D-1 | [3-Cl-4-OBu-n] |
| D-1 | [3-Cl-4-OBu-i] | D-1 | [3-Cl-4-OBu-sec] |
| D-1 | [3-Cl-4-OBu-tert] | D-1 | [3-Cl-4-OBu-c] |
| D-1 | [3-Cl-4-OPen-c] | D-1 | [3-Cl-4-OHex-c] |
| D-1 | [3-Cl-4-OCH₂CH=CH₂] | D-1 | [3-Cl-4-OCH₂C≡CH] |
| D-1 | [3-Cl-4-OCH₂CH=C(Me)₂] | D-1 | [3-Cl-4-OCH₂C≡CMe] |
| D-1 | [3-Cl-4-OCHF₂] | D-1 | [3-Cl-4-OCF₃] |
| D-1 | [3-Cl-4-OCH₂CH₂F] | D-1 | [3-Cl-4-OCH₂CH₂Cl] |
| D-1 | [3-Cl-4-OCH₂CHF₂] | D-1 | [3-Cl-4-OCH₂CH₂CF₃] |
| D-1 | [3-Cl-4-OCH₂CF₂CHF₂] | D-1 | [3-Cl-4-OCH₂CF₂CF₃] |
| D-1 | [3-Cl-4-OCH₂CH=CF₂] | D-1 | [3-Cl-4-OCH₂CH=CCl₂] |
| D-1 | [3-Cl-4-OCH₂CH₂CF=CF₂] | D-1 | [3-Cl-4-OCH₂CH₂CH=CF₂] |
| D-1 | [3-Cl-4-OCH₂C≡CCl] | D-1 | [3-Cl-4-OCH₂C≡CBr] |
| D-1 | [3-Cl-4-OCH₂CH₂OMe] | D-1 | [3-Cl-4-OCH₂CH₂SMe] |
| D-1 | [2-F-4-SMe] | D-1 | [2-F-4-SEt] |
| D-1 | [2-F-4-SPr-n] | D-1 | [2-F-4-SPr-i] |
| D-1 | [2-F-4-SPr-c] | D-1 | [2-F-4-SBu-n] |
| D-1 | [2-F-4-SBu-i] | D-1 | [2-F-4-SBu-sec] |
| D-1 | [2-F-4-SBu-tert] | D-1 | [2-F-4-SBu-c] |
| D-1 | [2-F-4-SPen-c] | D-1 | [2-F-4-SHex-c] |
| D-1 | [2-F-4-SCH₂CH=CH₂] | D-1 | [2-F-4-SCH₂C≡CH] |
| D-1 | [2-F-4-SCH₂CH=C(Me)₂] | D-1 | [2-F-4-SCH₂C≡CMe] |
| D-1 | [2-F-4-SCHF₂] | D-1 | [2-F-4-SCF₃] |
| D-1 | [2-F-4-SCH₂CH₂F] | D-1 | [2-F-4-SCH₂CH₂Cl] |
| D-1 | [2-F-4-SCH₂CHF₂] | D-1 | [2-F-4-SCH₂CH₂CF₃] |
| D-1 | [2-F-4-SCH₂CF₂CHF₂] | D-1 | [2-F-4-SCH₂CF₂CF₃] |
| D-1 | [2-F-4-SCH₂CH=CF₂] | D-1 | [2-F-4-SCH₂CH=CCl₂] |
| D-1 | [2-F-4-SCH₂CH₂CF=CF₂] | D-1 | [2-F-4-SCH₂CH₂CH=CF₂] |
| D-1 | [2-F-4-SCH₂C≡CCl] | D-1 | [2-F-4-SCH₂C≡CBr] |
| D-1 | [2-F-4-SCH₂CH₂OMe] | D-1 | [2-F-4-SCH₂CH₂SMe] |
| D-1 | [3-F-4-SMe] | D-1 | [3-F-4-SEt] |
| D-1 | [3-F-4-SPr-n] | D-1 | [3-F-4-SPr-i] |
| D-1 | [3-F-4-SPr-c] | D-1 | [3-F-4-SBu-n] |
| D-1 | [3-F-4-SBu-i] | D-1 | [3-F-4-SBu-sec] |
| D-1 | [3-F-4-SBu-tert] | D-1 | [3-F-4-SBu-c] |
| D-1 | [3-F-4-SPen-c] | D-1 | [3-F-4-SHex-c] |
| D-1 | [3-F-4-SCH₂CH=CH₂] | D-1 | [3-F-4-SCH₂C≡CH] |
| D-1 | [3-F-4-SCH₂CH=C(Me)₂] | D-1 | [3-F-4-SCH₂C≡CMe] |
| D-1 | [3-F-4-SCHF₂] | D-1 | [3-F-4-SCF₃] |
| D-1 | [3-F-4-SCH₂CH₂F] | D-1 | [3-F-4-SCH₂CH₂Cl] |
| D-1 | [3-F-4-SCH₂CHF₂] | D-1 | [3-F-4-SCH₂CH₂CF₃] |
| D-1 | [3-F-4-SCH₂CF₂CHF₂] | D-1 | [3-F-4-SCH₂CF₂CF₃] |
| D-1 | [3-F-4-SCH₂CH=CF₂] | D-1 | [3-F-4-SCH₂CH=CCl₂] |
| D-1 | [3-F-4-SCH₂CH₂CF=CF₂] | D-1 | [3-F-4-SCH₂CH₂CH=CF₂] |
| D-1 | [3-F-4-SCH₂C≡CCl] | D-1 | [3-F-4-SCH₂C≡CBr] |
| D-1 | [3-F-4-SCH₂CH₂OMe] | D-1 | [3-F-4-SCH₂CH₂SMe] |
| D-1 | [2-Cl-4-SMe] | D-1 | [2-Cl-4-SEt] |
| D-1 | [2-Cl-4-SPr-n] | D-1 | [2-Cl-4-SPr-i] |
| D-1 | [2-Cl-4-SPr-c] | D-1 | [2-Cl-4-SBu-n] |
| D-1 | [2-Cl-4-SBu-i] | D-1 | [2-Cl-4-SBu-sec] |
| D-1 | [2-Cl-4-SBu-tert] | D-1 | [2-Cl-4-SBu-c] |
| D-1 | [2-Cl-4-SPen-c] | D-1 | [2-Cl-4-SHex-c] |
| D-1 | [2-Cl-4-SCH₂CH=CH₂] | D-1 | [2-Cl-4-SCH₂C≡CH] |
| D-1 | [2-Cl-4-SCH₂CH=C(Me)₂] | D-1 | [2-Cl-4-SCH₂C≡CMe] |
| D-1 | [2-Cl-4-SCHF₂] | D-1 | [2-Cl-4-SCF₃] |
| D-1 | [2-Cl-4-SCH₂CH₂F] | D-1 | [2-Cl-4-SCH₂CH₂Cl] |
| D-1 | [2-Cl-4-SCH₂CHF₂] | D-1 | [2-Cl-4-SCH₂CH₂CF₃] |
| D-1 | [2-Cl-4-SCH₂CF₂CHF₂] | D-1 | [2-Cl-4-SCH₂CF₂CF₃] |
| D-1 | [2-Cl-4-SCH₂CH=CF₂] | D-1 | [2-Cl-4-SCH₂CH=CCl₂] |
| D-1 | [2-Cl-4-SCH₂CH₂CF=CF₂] | D-1 | [2-Cl-4-SCH₂CH₂CH=CF₂] |
| D-1 | [2-Cl-4-SCH₂C≡CCl] | D-1 | [2-Cl-4-SCH₂C≡CBr] |
| D-1 | [2-Cl-4-SCH₂CH₂OMe] | D-1 | [2-Cl-4-SCH₂CH₂SMe] |
| D-1 | [3-Cl-4-SMe] | D-1 | [3-Cl-4-SEt] |
| D-1 | [3-Cl-4-SPr-n] | D-1 | [3-Cl-4-SPr-i] |
| D-1 | [3-Cl-4-SPr-c] | D-1 | [3-Cl-4-SBu-n] |
| D-1 | [3-Cl-4-SBu-i] | D-1 | [3-Cl-4-SBu-sec] |
| D-1 | [3-Cl-4-SBu-tert] | D-1 | [3-Cl-4-SBu-c] |
| D-1 | [3-Cl-4-SPen-c] | D-1 | [3-Cl-4-SHex-c] |
| D-1 | [3-Cl-4-SCH₂CH=CH₂] | D-1 | [3-Cl-4-SCH₂C≡CH] |
| D-1 | [3-Cl-4-SCH₂CH=C(Me)₂] | D-1 | [3-Cl-4-SCH₂C≡CMe] |
| D-1 | [3-Cl-4-SCHF₂] | D-1 | [3-Cl-4-SCF₃] |
| D-1 | [3-Cl-4-SCH₂CH₂F] | D-1 | [3-Cl-4-SCH₂CH₂Cl] |
| D-1 | [3-Cl-4-SCH₂CHF₂] | D-1 | [3-Cl-4-SCH₂CH₂CF₃] |
| D-1 | [3-Cl-4-SCH₂CF₂CHF₂] | D-1 | [3-Cl-4-SCH₂CF₂CF₃] |
| D-1 | [3-Cl-4-SCH₂CH=CF₂] | D-1 | [3-Cl-4-SCH₂CH=CCl₂] |
| D-1 | [3-Cl-4-SCH₂CH₂CF=CF₂] | D-1 | [3-Cl-4-SCH₂CH₂CH=CF₂] |
| D-1 | [3-Cl-4-SCH₂C≡CCl] | D-1 | [3-Cl-4-SCH₂C≡CBr] |
| D-1 | [3-Cl-4-SCH₂CH₂OMe] | D-1 | [3-Cl-4-SCH₂CH₂SMe] |
| D-1 | [2-F-4-S(O)Me] | D-1 | [2-F-4-S(O)Et] |
| D-1 | [2-F-4-S(O)Pr-n] | D-1 | [2-F-4-S(O)Pr-i] |
| D-1 | [2-F-4-S(O)Pr-c] | D-1 | [2-F-4-S(O)Bu-n] |
| D-1 | [2-F-4-S(O)Bu-i] | D-1 | [2-F-4-S(O)Bu-sec] |
| D-1 | [2-F-4-S(O)Bu-tert] | D-1 | [2-F-4-S(O)Bu-c] |
| D-1 | [2-F-4-S(O)Pen-c] | D-1 | [2-F-4-S(O)Hex-c] |
| D-1 | [2-F-4-S(O)CH₂CH=CH₂] | D-1 | [2-F-4-S(O)CH₂C≡CH] |
| D-1 | [2-F-4-S(O)CH₂CH=C(Me)₂] | D-1 | [2-F-4-S(O)CH₂C≡CMe] |
| D-1 | [2-F-4-S(O)CHF₂] | D-1 | [2-F-4-S(O)CF₃] |
| D-1 | [2-F-4-S(O)CH₂CH₂F] | D-1 | [2-F-4-S(O)CH₂CH₂Cl] |
| D-1 | [2-F-4-S(O)CH₂CHF₂] | D-1 | [2-F-4-S(O)CH₂CH₂CF₃] |
| D-1 | [2-F-4-S(O)CH₂CF₂CHF₂] | D-1 | [2-F-4-S(O)CH₂CF₂CF₃] |
| D-1 | [2-F-4-S(O)CH₂CH=CF₂] | D-1 | [2-F-4-S(O)CH₂CH=CCl₂] |
| D-1 | [2-F-4-S(O)CH₂CH₂CF=CF₂] | D-1 | [2-F-4-S(O)CH₂CH₂CH=CF₂] |
| D-1 | [2-F-4-S(O)CH₂C≡CCl] | D-1 | [2-F-4-S(O)CH₂C≡CBr] |
| D-1 | [2-F-4-S(O)CH₂CH₂OMe] | D-1 | [2-F-4-S(O)CH₂CH₂SMe] |
| D-1 | [3-F-4-S(O)Me] | D-1 | [3-F-4-S(O)Et] |
| D-1 | [3-F-4-S(O)Pr-n] | D-1 | [3-F-4-S(O)Pr-i] |
| D-1 | [3-F-4-S(O)Pr-c] | D-1 | [3-F-4-S(O)Bu-n] |
| D-1 | [3-F-4-S(O)Bu-i] | D-1 | [3-F-4-S(O)Bu-sec] |
| D-1 | [3-F-4-S(O)Bu-tert] | D-1 | [3-F-4-S(O)Bu-c] |
| D-1 | [3-F-4-S(O)Pen-c] | D-1 | [3-F-4-S(O)Hex-c] |
| D-1 | [3-F-4-S(O)CH₂CH=CH₂] | D-1 | [3-F-4-S(O)CH₂C≡CH] |
| D-1 | [3-F-4-S(O)CH₂CH=C(Me)₂] | D-1 | [3-F-4-S(O)CH₂C≡CMe] |
| D-1 | [3-F-4-S(O)CHF₂] | D-1 | [3-F-4-S(O)CF₃] |
| D-1 | [3-F-4-S(O)CH₂CH₂F] | D-1 | [3-F-4-S(O)CH₂CH₂Cl] |
| D-1 | [3-F-4-S(O)CH₂CHF₂] | D-1 | [3-F-4-S(O)CH₂CH₂CF₃] |
| D-1 | [3-F-4-S(O)CH₂CF₂CHF₂] | D-1 | [3-F-4-S(O)CH₂CF₂CF₃] |
| D-1 | [3-F-4-S(O)CH₂CH=CF₂] | D-1 | [3-F-4-S(O)CH₂CH=CCl₂] |
| D-1 | [3-F-4-S(O)CH₂CH₂CF=CF₂] | D-1 | [3-F-4-S(O)CH₂CH₂CH=CF₂] |
| D-1 | [3-F-4-S(O)CH₂C≡CCl] | D-1 | [3-F-4-S(O)CH₂C≡CBr] |
| D-1 | [3-F-4-S(O)CH₂CH₂OMe] | D-1 | [3-F-4-S(O)CH₂CH₂SMe] |
| D-1 | [2-Cl-4-S(O)Me] | D-1 | [2-Cl-4-S(O)Et] |
| D-1 | [2-Cl-4-S(O)Pr-n] | D-1 | [2-Cl-4-S(O)Pr-i] |
| D-1 | [2-Cl-4-S(O)Pr-c] | D-1 | [2-Cl-4-S(O)Bu-n] |
| D-1 | [2-Cl-4-S(O)Bu-i] | D-1 | [2-Cl-4-S(O)Bu-sec] |
| D-1 | [2-Cl-4-S(O)Bu-tert] | D-1 | [2-Cl-4-S(O)Bu-c] |
| D-1 | [2-Cl-4-S(O)Pen-c] | D-1 | [2-Cl-4-S(O)Hex-c] |
| D-1 | [2-Cl-4-S(O)CH₂CH=CH₂] | D-1 | [2-Cl-4-S(O)CH₂C≡CH] |
| D-1 | [2-Cl-4-S(O)CH₂CH=C(Me)₂] | D-1 | [2-Cl-4-S(O)CH₂C≡CMe] |
| D-1 | [2-Cl-4-S(O)CHF₂] | D-1 | [2-Cl-4-S(O)CF₃] |
| D-1 | [2-Cl-4-S(O)CH₂CH₂F] | D-1 | [2-Cl-4-S(O)CH₂CH₂Cl] |
| D-1 | [2-Cl-4-S(O)CH₂CHF₂] | D-1 | [2-Cl-4-S(O)CH₂CH₂CF₃] |
| D-1 | [2-Cl-4-S(O)CH₂CF₂CHF₂] | D-1 | [2-Cl-4-S(O)CH₂CF₂CF₃] |
| D-1 | [2-Cl-4-S(O)CH₂CH=CF₂] | D-1 | [2-Cl-4-S(O)CH₂CH=CCl₂] |
| D-1 | [2-Cl-4-S(O)CH₂CH₂CF=CF₂] | D-1 | [2-Cl-4-S(O)CH₂CH₂CH=CF₂] |
| D-1 | [2-Cl-4-S(O)CH₂C≡CCl] | D-1 | [2-Cl-4-S(O)CH₂C≡CBr] |
| D-1 | [2-Cl-4-S(O)CH₂CH₂OMe] | D-1 | [2-Cl-4-S(O)CH₂CH₂SMe] |
| D-1 | [3-Cl-4-S(O)Me] | D-1 | [3-Cl-4-S(O)Et] |
| D-1 | [3-Cl-4-S(O)Pr-n] | D-1 | [3-Cl-4-S(O)Pr-i] |
| D-1 | [3-Cl-4-S(O)Pr-c] | D-1 | [3-Cl-4-S(O)Bu-n] |
| D-1 | [3-Cl-4-S(O)Bu-i] | D-1 | [3-Cl-4-S(O)Bu-sec] |
| D-1 | [3-Cl-4-S(O)Bu-tert] | D-1 | [3-Cl-4-S(O)Bu-c] |
| D-1 | [3-Cl-4-S(O)Pen-c] | D-1 | [3-Cl-4-S(O)Hex-c] |
| D-1 | [3-Cl-4-S(O)CH₂CH=CH₂] | D-1 | [3-Cl-4-S(O)CH₂C≡CH] |
| D-1 | [3-Cl-4-S(O)CH₂CH=C(Me)₂] | D-1 | [3-Cl-4-S(O)CH₂C≡CMe] |
| D-1 | [3-Cl-4-S(O)CHF₂] | D-1 | [3-Cl-4-S(O)CF₃] |
| D-1 | [3-Cl-4-S(O)CH₂CH₂F] | D-1 | [3-Cl-4-S(O)CH₂CH₂Cl] |
| D-1 | [3-Cl-4-S(O)CH₂CHF₂] | D-1 | [3-Cl-4-S(O)CH₂CH₂CF₃] |
| D-1 | [3-Cl-4-S(O)CH₂CF₂CHF₂] | D-1 | [3-Cl-4-S(O)CH₂CF₂CF₃] |
| D-1 | [3-Cl-4-S(O)CH₂CH=CF₂] | D-1 | [3-Cl-4-S(O)CH₂CH=CCl₂] |
| D-1 | [3-Cl-4-S(O)CH₂CH₂CF=CF₂] | D-1 | [3-Cl-4-S(O)CH₂CH₂CH=CF₂] |
| D-1 | [3-Cl-4-S(O)CH₂C≡CCl] | D-1 | [3-Cl-4-S(O)CH₂C≡CBr] |
| D-1 | [3-Cl-4-S(O)CH₂CH₂OMe] | D-1 | [3-Cl-4-S(O)CH₂CH₂SMe] |
| D-1 | [2-F-4-S(O)₂Me] | D-1 | [2-F-4-S(O)₂Et] |
| D-1 | [2-F-4-S(O)₂Pr-n] | D-1 | [2-F-4-S(O)₂Pr-i] |
| D-1 | [2-F-4-S(O)₂Pr-c] | D-1 | [2-F-4-S(O)₂Bu-n] |
| D-1 | [2-F-4-S(O)₂Bu-i] | D-1 | [2-F-4-S(O)₂Bu-sec] |
| D-1 | [2-F-4-S(O)₂Bu-tert] | D-1 | [2-F-4-S(O)₂Bu-c] |
| D-1 | [2-F-4-S(O)₂Pen-c] | D-1 | [2-F-4-S(O)₂Hex-c] |
| D-1 | [2-F-4-S(O)₂CH₂CH=CH₂] | D-1 | [2-F-4-S(O)₂CH₂C≡CH] |
| D-1 | [2-F-4-S(O)₂CH₂CH=C(Me)₂] | D-1 | [2-F-4-S(O)₂CH₂C≡CMe] |
| D-1 | [2-F-4-S(O)₂CHF₂] | D-1 | [2-F-4-S(O)₂CF₃] |
| D-1 | [2-F-4-S(O)₂CH₂CH₂F] | D-1 | [2-F-4-S(O)₂CH₂CH₂Cl] |
| D-1 | [2-F-4-S(O)₂CH₂CHF₂] | D-1 | [2-F-4-S(O)₂CH₂CH₂CF₃] |
| D-1 | [2-F-4-S(O)₂CH₂CF₂CHF₂] | D-1 | [2-F-4-S(O)₂CH₂CF₂CF₃] |
| D-1 | [2-F-4-S(O)₂CH₂CH=CF₂] | D-1 | [2-F-4-S(O)₂CH₂CH=CCl₂] |
| D-1 | [2-F-4-S(O)₂CH₂CH₂CF=CF₂] | D-1 | [2-F-4-S(O)₂CH₂CH₂CH=CF₂] |
| D-1 | [2-F-4-S(O)₂CH₂C≡CCl] | D-1 | [2-F-4-S(O)₂CH₂C≡CBr] |
| D-1 | [2-F-4-S(O)₂CH₂CH₂OMe] | D-1 | [2-F-4-S(O)₂CH₂CH₂SMe] |
| D-1 | [3-F-4-S(O)₂Me] | D-1 | [3-F-4-S(O)₂Et] |
| D-1 | [3-F-4-S(O)₂Pr-n] | D-1 | [3-F-4-S(O)₂Pr-i] |
| D-1 | [3-F-4-S(O)₂Pr-c] | D-1 | [3-F-4-S(O)₂Bu-n] |
| D-1 | [3-F-4-S(O)₂Bu-i] | D-1 | [3-F-4-S(O)₂Bu-sec] |
| D-1 | [3-F-4-S(O)₂Bu-tert] | D-1 | [3-F-4-S(O)₂Bu-c] |
| D-1 | [3-F-4-S(O)₂Pen-c] | D-1 | [3-F-4-S(O)₂Hex-c] |
| D-1 | [3-F-4-S(O)₂CH₂CH=CH₂] | D-1 | [3-F-4-S(O)₂CH₂C≡CH] |
| D-1 | [3-F-4-S(O)₂CH₂CH=C(Me)₂] | D-1 | [3-F-4-S(O)₂CH₂C≡CMe] |
| D-1 | [3-F-4-S(O)₂CHF₂] | D-1 | [3-F-4-S(O)₂CF₃] |
| D-1 | [3-F-4-S(O)₂CH₂CH₂F] | D-1 | [3-F-4-S(O)₂CH₂CH₂Cl] |
| D-1 | [3-F-4-S(O)₂CH₂CHF₂] | D-1 | [3-F-4-S(O)₂CH₂CH₂CF₃] |
| D-1 | [3-F-4-S(O)₂CH₂CF₂CHF₂] | D-1 | [3-F-4-S(O)₂CH₂CF₂CF₃] |
| D-1 | [3-F-4-S(O)₂CH₂CH=CF₂] | D-1 | [3-F-4-S(O)₂CH₂CH=CCl₂] |
| D-1 | [3-F-4-S(O)₂CH₂CH₂CF=CF₂] | D-1 | [3-F-4-S(O)₂CH₂CH₂CH=CF₂] |
| D-1 | [3-F-4-S(O)₂CH₂C≡CCl] | D-1 | [3-F-4-S(O)₂CH₂C≡CBr] |
| D-1 | [3-F-4-S(O)₂CH₂CH₂OMe] | D-1 | [3-F-4-S(O)₂CH₂CH₂SMe] |
| D-1 | [2-Cl-4-S(O)₂Me] | D-1 | [2-Cl-4-S(O)₂Et] |
| D-1 | [2-Cl-4-S(O)₂Pr-n] | D-1 | [2-Cl-4-S(O)₂Pr-i] |
| D-1 | [2-Cl-4-S(O)₂Pr-c] | D-1 | [2-Cl-4-S(O)₂Bu-n] |
| D-1 | [2-Cl-4-S(O)₂Bu-i] | D-1 | [2-Cl-4-S(O)₂Bu-sec] |
| D-1 | [2-Cl-4-S(O)₂Bu-tert] | D-1 | [2-Cl-4-S(O)₂Bu-c] |
| D-1 | [2-Cl-4-S(O)₂Pen-c] | D-1 | [2-Cl-4-S(O)₂Hex-c] |
| D-1 | [2-Cl-4-S(O)₂CH₂CH=CH₂] | D-1 | [2-Cl-4-S(O)₂CH₂C≡CH] |
| D-1 | [2-Cl-4-S(O)₂CH₂CH=C(Me)₂] | D-1 | [2-Cl-4-S(O)₂CH₂C≡CMe] |
| D-1 | [2-Cl-4-S(O)₂CHF₂] | D-1 | [2-Cl-4-S(O)₂CF₃] |
| D-1 | [2-Cl-4-S(O)₂CH₂CH₂F] | D-1 | [2-Cl-4-S(O)₂CH₂CH₂Cl] |
| D-1 | [2-Cl-4-S(O)₂CH₂CHF₂] | D-1 | [2-Cl-4-S(O)₂CH₂CH₂CF₃] |
| D-1 | [2-Cl-4-S(O)₂CH₂CF₂CHF₂] | D-1 | [2-Cl-4-S(O)₂CH₂CF₂CF₃] |
| D-1 | [2-Cl-4-S(O)₂CH₂CH=CF₂] | D-1 | [2-Cl-4-S(O)₂CH₂CH=CCl₂] |
| D-1 | [2-Cl-4-S(O)₂CH₂CH₂CF=CF₂] | D-1 | [2-Cl-4-S(O)₂CH₂CH₂CH=CF₂] |
| D-1 | [2-Cl-4-S(O)₂CH₂C≡CCl] | D-1 | [2-Cl-4-S(O)₂CH₂C≡CBr] |
| D-1 | [2-Cl-4-S(O)₂CH₂CH₂OMe] | D-1 | [2-Cl-4-S(O)₂CH₂CH₂SMe] |
| D-1 | [3-Cl-4-S(O)₂Me] | D-1 | [3-Cl-4-S(O)₂Et] |
| D-1 | [3-Cl-4-S(O)₂Pr-n] | D-1 | [3-Cl-4-S(O)₂Pr-i] |
| D-1 | [3-Cl-4-S(O)₂Pr-c] | D-1 | [3-Cl-4-S(O)₂Bu-n] |
| D-1 | [3-Cl-4-S(O)₂Bu-i] | D-1 | [3-Cl-4-S(O)₂Bu-sec] |
| D-1 | [3-Cl-4-S(O)₂Bu-tert] | D-1 | [3-Cl-4-S(O)₂Bu-c] |
| D-1 | [3-Cl-4-S(O)₂Pen-c] | D-1 | [3-Cl-4-S(O)₂Hex-c] |
| D-1 | [3-Cl-4-S(O)₂CH₂CH=CH₂] | D-1 | [3-Cl-4-S(O)₂CH₂C≡CH] |
| D-1 | [3-Cl-4-S(O)₂CH₂CH=C(Me)₂] | D-1 | [3-Cl-4-S(O)₂CH₂C≡CMe] |
| D-1 | [3-Cl-4-S(O)₂CHF₂] | D-1 | [3-Cl-4-S(O)₂CF₃] |
| D-1 | [3-Cl-4-S(O)₂CH₂CH₂F] | D-1 | [3-Cl-4-S(O)₂CH₂CH₂Cl] |
| D-1 | [3-Cl-4-S(O)₂CH₂CHF₂] | D-1 | [3-Cl-4-S(O)₂CH₂CH₂CF₃] |
| D-1 | [3-Cl-4-S(O)₂CH₂CF₂CHF₂] | D-1 | [3-Cl-4-S(O)₂CH₂CF₂CF₃] |
| D-1 | [3-Cl-4-S(O)₂CH₂CH=CF₂] | D-1 | [3-Cl-4-S(O)₂CH₂CH=CCl₂] |
| D-1 | [3-Cl-4-S(O)₂CH₂CH₂CF=CF₂] | D-1 | [3-Cl-4-S(O)₂CH₂CH₂CH=CF₂] |
| D-1 | [3-Cl-4-S(O)₂CH₂C≡CCl] | D-1 | [3-Cl-4-S(O)₂CH₂C≡CBr] |
| D-1 | [3-Cl-4-S(O)₂CH₂CH₂OMe] | D-1 | [3-Cl-4-S(O)₂CH₂CH₂SMe] |
| D-1 | [2-OMe-3-F] | D-1 | [2-OMe-4-F] |
| D-1 | [2-OMe-5-F] | D-1 | [2-OMe-3-Cl] |
| D-1 | [2-OMe-4-Cl] | D-1 | [2-OMe-5-Cl] |
| D-1 | [2-OMe-3-Me] | D-1 | [2-OMe-4-Me] |
| D-1 | [2-OMe-5-Me] | D-1 | [2-OMe-6-Me] |
| D-1 | [2-OMe-3-CF₃] | D-1 | [2-OMe-4-CF₃] |
| D-1 | [2-OMe-5-CF₃] | D-1 | [2-OMe-6-CF₃] |
| D-1 | [2-OMe-3-OCHF₂] | D-1 | [2-OMe-4-OCHF₂] |
| D-1 | [2-OMe-5-OCHF₂] | D-1 | [2-OMe-6-OCHF₂] |
| D-1 | [2-OMe-3-OCF₃] | D-1 | [2-OMe-4-OCF₃] |
| D-1 | [2-OMe-5-OCF₃] | D-1 | [2-OMe-6-OCF₃] |
| D-1 | [2-OMe-3-CN] | D-1 | [2-OMe-4-CN] |
| D-1 | [2-OMe-5-CN] | D-1 | [2-OMe-6-CN] |
| D-1 | [2-OMe-3-NO₂] | D-1 | [2-OMe-4-NO₂] |
| D-1 | [2-OMe-5-NO₂] | D-1 | [2-OMe-6-NO₂] |
| D-1 | [2-OMe-3-C(O)Me] | D-1 | [2-OMe-4-C(O)Me] |
| D-1 | [2-OMe-5-C(O)Me] | D-1 | [2-OMe-6-C(O)Me] |
| D-1 | [2-OMe-3-C(=NOMe)Me] | D-1 | [2-OMe-4-C(=NOMe)Me] |
| D-1 | [2-OMe-5-C(=NOMe)Me] | D-1 | [2-OMe-6-C(=NOMe)Me] |
| D-1 | [2-OEt-3-F] | D-1 | [2-OEt-4-F] |
| D-1 | [2-OEt-5-F] | D-1 | [2-OEt-4-F] |
| D-1 | [2-OEt-3-Cl] | D-1 | [2-OEt-4-Cl] |
| D-1 | [2-OEt-5-Cl] | D-1 | [2-OEt-4-Cl] |
| D-1 | [2-OEt-3-Me] | D-1 | [2-OEt-4-Me] |
| D-1 | [2-OEt-5-Me] | D-1 | [2-OEt-6-Me] |
| D-1 | [2-OEt-3-CF₃] | D-1 | [2-OEt-4-CF₃] |
| D-1 | [2-OEt-5-CF₃] | D-1 | [2-OEt-6-CF₃] |
| D-1 | [2-OEt-3-OCHF₂] | D-1 | [2-OEt-4-OCHF₂] |
| D-1 | [2-OEt-5-OCHF₂] | D-1 | [2-OEt-6-OCHF₂] |
| D-1 | [2-OEt-3-OCF₃] | D-1 | [2-OEt-4-OCF₃] |
| D-1 | [2-OEt-5-OCF₃] | D-1 | [2-OEt-6-OCF₃] |
| D-1 | [2-OEt-3-CN] | D-1 | [2-OEt-4-CN] |
| D-1 | [2-OEt-5-CN] | D-1 | [2-OEt-6-CN] |
| D-1 | [2-OEt-3-NO₂] | D-1 | [2-OEt-4-NO₂] |
| D-1 | [2-OEt-5-NO₂] | D-1 | [2-OEt-6-NO₂] |
| D-1 | [2-OEt-3-C(O)Me] | D-1 | [2-OEt-4-C(O)Me] |
| D-1 | [2-OEt-5-C(O)Me] | D-1 | [2-OEt-6-C(O)Me] |
| D-1 | [2-OEt-3-C(=NOMe)Me] | D-1 | [2-OEt-4-C(=NOMe)Me] |
| D-1 | [2-OEt-5-C(=NOMe)Me] | D-1 | [2-OEt-6-C(=NOMe)Me] |
| D-1 | [2-OPr-n-3-F] | D-1 | [2-OPr-n-4-F] |
| D-1 | [2-OPr-n-5-F] | D-1 | [2-OPr-n-3-Cl] |
| D-1 | [2-OPr-n-4-Cl] | D-1 | [2-OPr-n-5-Cl] |
| D-1 | [2-OPr-n-3-Me] | D-1 | [2-OPr-n-4-Me] |
| D-1 | [2-OPr-n-5-Me] | D-1 | [2-OPr-n-6-Me] |
| D-1 | [2-OPr-n-3-CF₃] | D-1 | [2-OPr-n-4-CF₃] |
| D-1 | [2-OPr-n-5-CF₃] | D-1 | [2-OPr-n-6-CF₃] |
| D-1 | [2-OPr-n-3-OCHF₂] | D-1 | [2-OPr-n-4-OCHF₂] |
| D-1 | [2-OPr-n-5-OCHF₂] | D-1 | [2-OPr-n-6-OCHF₂] |
| D-1 | [2-OPr-n-3-OCF₃] | D-1 | [2-OPr-n-4-OCF₃] |
| D-1 | [2-OPr-n-5-OCF₃] | D-1 | [2-OPr-n-6-OCF₃] |
| D-1 | [2-OPr-n-3-CN] | D-1 | [2-OPr-n-4-CN] |
| D-1 | [2-OPr-n-5-CN] | D-1 | [2-OPr-n-6-CN] |
| D-1 | [2-OPr-n-3-NO₂] | D-1 | [2-OPr-n-4-NO₂] |
| D-1 | [2-OPr-n-5-NO₂] | D-1 | [2-OPr-n-6-NO₂] |
| D-1 | [2-OPr-n-3-C(O)Me] | D-1 | [2-OPr-n-4-C(O)Me] |
| D-1 | [2-OPr-n-5-C(O)Me] | D-1 | [2-OPr-n-6-C(O)Me] |
| D-1 | [2-OPr-n-3-C(=NOMe)Me] | D-1 | [2-OPr-n-4-C(=NOMe)Me] |
| D-1 | [2-OPr-n-5-C(=NOMe]Me] | D-1 | [2-OPr-n-6-C(=NOMe]Me] |
| D-1 | [2-OPr-i-3-F] | D-1 | [2-OPr-i-4-F] |
| D-1 | [2-OPr-i-5-F] | D-1 | [2-OPr-i-3-Cl] |
| D-1 | [2-OPr-i-4-Cl] | D-1 | [2-OPr-i-5-Cl] |
| D-1 | [2-OPr-i-3-Me] | D-1 | [2-OPr-i-4-Me] |
| D-1 | [2-OPr-i-5-Me] | D-1 | [2-OPr-i-6-Me] |
| D-1 | [2-OPr-i-3-CF₃] | D-1 | [2-OPr-i-4-CF₃] |
| D-1 | [2-OPr-i-5-CF₃] | D-1 | [2-OPr-i-6-CF₃] |
| D-1 | [2-OPr-i-3-OCHF₂] | D-1 | [2-OPr-i-4-OCHF₂] |
| D-1 | [2-OPr-i-5-OCHF₂] | D-1 | [2-OPr-i-6-OCHF₂] |
| D-1 | [2-OPr-i-3-OCF₃] | D-1 | [2-OPr-i-4-OCF₃] |
| D-1 | [2-OPr-i-5-OCF₃] | D-1 | [2-OPr-i-6-OCF₃] |
| D-1 | [2-OPr-i-3-CN] | D-1 | [2-OPr-i-4-CN] |
| D-1 | [2-OPr-i-5-CN] | D-1 | [2-OPr-i-6-CN] |
| D-1 | [2-OPr-i-3-NO₂] | D-1 | [2-OPr-i-4-NO₂] |
| D-1 | [2-OPr-i-5-NO₂] | D-1 | [2-OPr-i-6-NO₂] |
| D-1 | [2-OPr-i-3-C(O)Me] | D-1 | [2-OPr-i-4-C(O)Me] |
| D-1 | [2-OPr-i-5-C(O)Me] | D-1 | [2-OPr-i-6-C(O)Me] |
| D-1 | [2-OPr-i-3-C(=NOMe)Me] | D-1 | [2-OPr-i-4-C(=NOMe)Me] |
| D-1 | [2-OPr-i-5-C(=NOMe)Me] | D-1 | [2-OPr-i-6-C(=NOMe)Me] |
| D-1 | [2-OBu-n-3-F] | D-1 | [2-OBu-n-4-F] |
| D-1 | [2-OBu-n-5-F] | D-1 | [2-OBu-n-3-Cl] |
| D-1 | [2-OBu-n-4-Cl] | D-1 | [2-OBu-n-5-Cl] |
| D-1 | [2-OBu-n-3-Me] | D-1 | [2-OBu-n-4-Me] |
| D-1 | [2-OBu-n-5-Me] | D-1 | [2-OBu-n-6-Me] |
| D-1 | [2-OBu-n-3-CF₃] | D-1 | [2-OBu-n-4-CF₃] |
| D-1 | [2-OBu-n-5-CF₃] | D-1 | [2-OBu-n-6-CF₃] |
| D-1 | [2-OBu-n-3-OCHF₂] | D-1 | [2-OBu-n-4-OCHF₂] |
| D-1 | [2-OBu-n-5-OCHF₂] | D-1 | [2-OBu-n-6-OCHF₂] |
| D-1 | [2-OBu-n-3-OCF₃] | D-1 | [2-OBu-n-4-OCF₃] |
| D-1 | [2-OBu-n-5-OCF₃] | D-1 | [2-OBu-n-6-OCF₃] |
| D-1 | [2-OBu-n-3-CN] | D-1 | [2-OBu-n-4-CN] |
| D-1 | [2-OBu-n-5-CN] | D-1 | [2-OBu-n-6-CN] |
| D-1 | [2-OBu-n-3-NO₂] | D-1 | [2-OBu-n-4-NO₂] |
| D-1 | [2-OBu-n-5-NO₂] | D-1 | [2-OBu-n-6-NO₂] |
| D-1 | [2-OBu-n-3-C(O)Me] | D-1 | [2-OBu-n-4-C(O)Me] |
| D-1 | [2-OBu-n-5-C(O)Me] | D-1 | [2-OBu-n-6-C(O)Me] |
| D-1 | [2-OBu-n-3-C(=NOMe)Me] | D-1 | [2-OBu-n-4-C(=NOMe)Me] |
| D-1 | [2-OBu-n-5-C(=NOMe)Me] | D-1 | [2-OBu-n-6-C(=NOMe)Me] |
| D-1 | [2-OBu-i-3-F] | D-1 | [2-OBu-i-4-F] |
| D-1 | [2-OBu-i-5-F] | D-1 | [2-OBu-i-3-Cl] |
| D-1 | [2-OBu-i-4-Cl] | D-1 | [2-OBu-i-5-Cl] |
| D-1 | [2-OBu-i-3-Me] | D-1 | [2-OBu-i-4-Me] |
| D-1 | [2-OBu-i-5-Me] | D-1 | [2-OBu-i-6-Me] |
| D-1 | [2-OBu-i-3-CF₃] | D-1 | [2-OBu-i-4-CF₃] |
| D-1 | [2-OBu-i-5-CF₃] | D-1 | [2-OBu-i-6-CF₃] |
| D-1 | [2-OBu-i-3-OCHF₂] | D-1 | [2-OBu-i-4-OCHF₂] |
| D-1 | [2-OBu-i-5-OCHF₂] | D-1 | [2-OBu-i-6-OCHF₂] |
| D-1 | [2-OBu-i-3-OCF₃] | D-1 | [2-OBu-i-4-OCF₃] |
| D-1 | [2-OBu-i-5-OCF₃] | D-1 | [2-OBu-i-6-OCF₃] |
| D-1 | [2-OBu-i-3-CN] | D-1 | [2-OBu-i-4-CN] |
| D-1 | [2-OBu-i-5-CN] | D-1 | [2-OBu-i-6-CN] |
| D-1 | [2-OBu-i-3-NO₂] | D-1 | [2-OBu-i-4-NO₂] |
| D-1 | [2-OBu-i-5-NO₂] | D-1 | [2-OBu-i-6-NO₂] |
| D-1 | [2-OBu-i-3-C(O)Me] | D-1 | [2-OBu-i-4-C(O)Me] |
| D-1 | [2-OBu-i-5-C(O)Me] | D-1 | [2-OBu-i-6-C(O)Me] |
| D-1 | [2-OBu-i-3-C(=NOMe)Me] | D-1 | [2-OBu-i-4-C(=NOMe)Me] |
| D-1 | [2-OBu-i-5-C(=NOMe)Me] | D-1 | [2-OBu-i-6-C(=NOMe)Me] |
| D-1 | [2-OBu-t-3-F] | D-1 | [2-OBu-t-4-F] |
| D-1 | [2-OBu-tert-5-F] | D-1 | [2-OBu-tert-3-Cl] |
| D-1 | [2-OBu-tert-4-Cl] | D-1 | [2-OBu-tert-5-Cl] |
| D-1 | [2-OBu-tert-3-Me] | D-1 | [2-OBu-tert-4-Me] |
| D-1 | [2-OBu-tert-5-Me] | D-1 | [2-OBu-ertt-6-Me] |
| D-1 | [2-OBu-tert-3-CF₃] | D-1 | [2-OBu-tert-4-CF₃] |
| D-1 | [2-OBu-tert-5-CF₃] | D-1 | [2-OBu-tert-6-CF₃] |
| D-1 | [2-OBu-tert-3-OCHF₂] | D-1 | [2-OBu-tert-4-OCHF₂] |
| D-1 | [2-OBu-tert-5-OCHF₂] | D-1 | [2-OBu-tert-6-OCHF₂] |
| D-1 | [2-OBu-tert-3-OCF₃] | D-1 | [2-OBu-tert-4-OCF₃] |
| D-1 | [2-OBu-tert-5-OCF₃] | D-1 | [2-OBu-tert-6-OCF₃] |
| D-1 | [2-OBu-tert-3-CN] | D-1 | [2-OBu-tert-4-CN] |
| D-1 | [2-OBu-tert-5-CN] | D-1 | [2-OBu-tert-6-CN] |
| D-1 | [2-OBu-tert-3-NO₂] | D-1 | [2-OBu-tert-4-NO₂] |
| D-1 | [2-OBu-tert-5-NO₂] | D-1 | [2-OBu-tert-6-NO₂] |
| D-1 | [2-OBu-tert-3-C(O)Me] | D-1 | [2-OBu-tert-4-C(O)Me] |
| D-1 | [2-OBu-tert-5-C(O)Me] | D-1 | [2-OBu-tert-6-C(O)Me] |
| D-1 | [2-OBu-tert-3-C(=NOMe)Me] | D-1 | [2-OBu-tert-4-C(=NOMe)Me] |
| D-1 | [2-OBu-tert-5-C(=NOMe)Me] | D-1 | [2-OBu-tert-6-C(=NOMe)Me] |
| D-1 | [2-OCH₂CH=CH₂-3-F] | D-1 | [2-OCH₂CH=CH₂-4-F] |
| D-1 | [2-OCH₂CH=CH₂-5-F] | D-1 | [2-OCH₂CH=CH₂-3-Cl] |
| D-1 | [2-OCH₂CH=CH₂-4-Cl] | D-1 | [2-OCH₂CH=CH₂-5-Cl] |
| D-1 | [2-OCH₂CH=CH₂-3-Me] | D-1 | [2-OCH₂CH=CH₂-4-Me] |
| D-1 | [2-OCH₂CH=CH₂-5-Me] | D-1 | [2-OCH₂CH=CH₂-6-Me] |
| D-1 | [2-OCH₂CH=CH₂-3-CF₃] | D-1 | [2-OCH₂CH=CH₂-4-CF₃] |
| D-1 | [2-OCH₂CH=CH₂-5-CF₃] | D-1 | [2-OCH₂CH=CH₂-6-CF₃] |
| D-1 | [2-OCH₂CH=CH₂-3-OCHF₂] | D-1 | [2-OCH₂CH=CH₂-4-OCHF₂] |
| D-1 | [2-OCH₂CH=CH₂-5-OCHF₂] | D-1 | [2-OCH₂CH=CH₂-6-OCHF₂] |
| D-1 | [2-OCH₂CH=CH₂-3-OCF₃] | D-1 | [2-OCH₂CH=CH₂-4-OCF₃] |
| D-1 | [2-OCH₂CH=CH₂-5-OCF₃] | D-1 | [2-OCH₂CH=CH₂-6-OCF₃] |
| D-1 | [2-OCH₂CH=CH₂-3-CN] | D-1 | [2-OCH₂CH=CH₂-4-CN] |
| D-1 | [2-OCH₂CH=CH₂-5-CN] | D-1 | [2-OCH₂CH=CH₂-6-CN] |
| D-1 | [2-OCH₂CH=CH₂-3-NO₂] | D-1 | [2-OCH₂CH=CH₂-4-NO₂] |
| D-1 | [2-OCH₂CH=CH₂-5-NO₂] | D-1 | [2-OCH₂CH=CH₂-6-NO₂] |
| D-1 | [2-OCH₂CH=CH₂-3-C(O)Me] | D-1 | [2-OCH₂CH=CH₂-4-C(O)Me] |
| D-1 | [2-OCH₂CH=CH₂-5-C(O)Me] | D-1 | [2-OCH₂CH=CH₂-6-C(O)Me] |
| D-1 | [2-OCH₂CH=CH₂-3-C(=NOMe)Me] | D-1 | [2-OCH₂CH=CH₂-4-C(=NOMe)Me] |
| D-1 | [2-OCH₂CH=CH₂-5-C(=NOMe)Me] | D-1 | [2-OCH₂CH=CH₂-6-C(=NOMe)Me] |
| D-1 | [2-OCH₂C≡CH-3-F] | D-1 | [2-OCH₂C≡CH-4-F] |
| D-1 | [2-OCH₂C≡CH-5-F] | D-1 | [2-OCH₂C≡CH-3-Cl] |
| D-1 | [2-OCH₂C≡CH-4-Cl] | D-1 | [2-OCH₂C≡CH-5-Cl] |
| D-1 | [2-OCH₂C≡CH-3-Me] | D-1 | [2-OCH₂C≡CH-4-Me] |
| D-1 | [2-OCH₂C≡CH-5-Me] | D-1 | [2-OCH₂C≡CH-6-Me] |
| D-1 | [2-OCH₂C≡CH-3-CF₃] | D-1 | [2-OCH₂C≡CH-4-CF₃] |
| D-1 | [2-OCH₂C≡CH-5-CF₃] | D-1 | [2-OCH₂C≡CH-6-CF₃] |
| D-1 | [2-OCH₂C≡CH-3-OCHF₂] | D-1 | [2-OCH₂C≡CH-4-OCHF₂] |
| D-1 | [2-OCH₂C≡CH-5-OCHF₂] | D-1 | [2-OCH₂C≡CH-6-OCHF₂] |
| D-1 | [2-OCH₂C≡CH-3-OCF₃] | D-1 | [2-OCH₂C≡CH-4-OCF₃] |
| D-1 | [2-OCH₂C≡CH-5-OCF₃] | D-1 | [2-OCH₂C≡CH-6-OCF₃] |
| D-1 | [2-OCH₂C≡CH-3-CN] | D-1 | [2-OCH₂C≡CH-4-CN] |
| D-1 | [2-OCH₂C≡CH-5-CN] | D-1 | [2-OCH₂C≡CH-6-CN] |
| D-1 | [2-OCH₂C≡CH-3-NO₂] | D-1 | [2-OCH₂C≡CH-4-NO₂] |
| D-1 | [2-OCH₂C≡CH-5-NO₂] | D-1 | [2-OCH₂C≡CH-6-NO₂] |
| D-1 | [2-OCH₂C≡CH-3-C(O)Me] | D-1 | [2-OCH₂C≡CH-4-C(O)Me] |
| D-1 | [2-OCH₂C≡CH-5-C(O)Me] | D-1 | [2-OCH₂C≡CH-6-C(O)Me] |
| D-1 | [2-OCH₂C≡CH-3-C(=NOMe)Me] | D-1 | [2-OCH₂C≡CH-4-C(=NOMe)Me] |
| D-1 | [2-OCH₂C≡CH-5-C(=NOMe)Me] | D-1 | [2-OCH₂C≡CH-6-C(=NOMe)Me] |
| D-1 | [2-OCH₂CN-3-F] | D-1 | [2-OCH₂CN-4-F] |
| D-1 | [2-OCH₂CN-5-F] | D-1 | [2-OCH₂CN-3-Cl] |
| D-1 | [2-OCH₂CN-4-Cl] | D-1 | [2-OCH₂CN-5-Cl] |
| D-1 | [2-OCH₂CN-3-Me] | D-1 | [2-OCH₂CN-4-Me] |
| D-1 | [2-OCH₂CN-5-Me] | D-1 | [2-OCH₂CN-6-Me] |
| D-1 | [2-OCH₂CN-3-CF₃] | D-1 | [2-OCH₂CN-4-CF₃] |
| D-1 | [2-OCH₂CN-5-CF₃] | D-1 | [2-OCH₂CN-6-CF₃] |
| D-1 | [2-OCH₂CN-3-OCHF₂] | D-1 | [2-OCH₂CN-4-OCHF₂] |
| D-1 | [2-OCH₂CN-5-OCHF₂] | D-1 | [2-OCH₂CN-6-OCHF₂] |
| D-1 | [2-OCH₂CN-3-OCF₃] | D-1 | [2-OCH₂CN-4-OCF₃] |
| D-1 | [2-OCH₂CN-5-OCF₃] | D-1 | [2-OCH₂CN-6-OCF₃] |
| D-1 | [2-OCH₂CN-3-CN] | D-1 | [2-OCH₂CN-4-CN] |
| D-1 | [2-OCH₂CN-5-CN] | D-1 | [2-OCH₂CN-6-CN] |
| D-1 | [2-OCH₂CN-3-NO₂] | D-1 | [2-OCH₂CN-4-NO₂] |
| D-1 | [2-OCH₂CN-5-NO₂] | D-1 | [2-OCH₂CN-6-NO₂] |
| D-1 | [2-OCH₂CN-3-C(O)Me] | D-1 | [2-OCH₂CN-4-C(O)Me] |
| D-1 | [2-OCH₂CN-5-C(O)Me] | D-1 | [2-OCH₂CN-6-C(O)Me] |
| D-1 | [2-OCH₂CN-3-C(=NOMe)Me] | D-1 | [2-OCH₂CN-4-C(=NOMe)Me] |
| D-1 | [2-OCH₂CN-5-C(=NOMe)Me] | D-1 | [2-OCH₂CN-6-C(=NOMe)Me] |
| D-1 | [2-OCF₃-3-F] | D-1 | [2-OCF₃-4-F] |
| D-1 | [2-OCF₃-5-F] | D-1 | [2-OCF₃-3-Cl] |
| D-1 | [2-OCF₃-4-Cl] | D-1 | [2-OCF₃-5-Cl] |
| D-1 | [2-OCF₃-3-Me] | D-1 | [2-OCF₃-4-Me] |
| D-1 | [2-OCF₃-5-Me] | D-1 | [2-OCF₃-6-Me] |
| D-1 | [2-OCF₃-3-CF₃] | D-1 | [2-OCF₃-4-CF₃] |
| D-1 | [2-OCF₃-5-CF₃] | D-1 | [2-OCF₃-6-CF₃] |
| D-1 | [2-OCF₃-3-OCHF₂] | D-1 | [2-OCF₃-4-OCHF₂] |
| D-1 | [2-OCF₃-5-OCHF₂] | D-1 | [2-OCF₃-6-OCHF₂] |
| D-1 | [2-OCF₃-3-OCF₃] | D-1 | [2-OCF₃-4-OCF₃] |
| D-1 | [2-OCF₃-5-OCF₃] | D-1 | [2-OCF₃-6-OCF₃] |
| D-1 | [2-OCF₃-3-CN] | D-1 | [2-OCF₃-4-CN] |
| D-1 | [2-OCF₃-5-CN] | D-1 | [2-OCF₃-6-CN] |
| D-1 | [2-OCF₃-3-NO₂] | D-1 | [2-OCF₃-4-NO₂] |
| D-1 | [2-OCF₃-5-NO₂] | D-1 | [2-OCF₃-6-NO₂] |
| D-1 | [2-OCF₃-3-C(O)Me] | D-1 | [2-OCF₃-4-C(O)Me] |
| D-1 | [2-OCF₃-5-C(O)Me] | D-1 | [2-OCF₃-6-C(O)Me] |
| D-1 | [2-OCF₃-3-C(=NOMe)Me] | D-1 | [2-OCF₃-4-C(=NOMe)Me] |
| D-1 | [2-OCF₃-5-C(=NOMe)Me] | D-1 | [2-OCF₃-6-C(=NOMe)Me] |
| D-1 | [2-OCH₂CHF₂-3-F] | D-1 | [2-OCH₂CHF₂-4-F] |
| D-1 | [2-OCH₂CHF₂-5-F] | D-1 | [2-OCH₂CHF₂-3-Cl] |
| D-1 | [2-OCH₂CHF₂-4-Cl] | D-1 | [2-OCH₂CHF₂-5-Cl] |
| D-1 | [2-OCH₂CHF₂-3-Me] | D-1 | [2-OCH₂CHF₂-4-Me] |
| D-1 | [2-OCH₂CHF₂-5-Me] | D-1 | [2-OCH₂CHF₂-6-Me] |
| D-1 | [2-OCH₂CHF₂-3-CF₃] | D-1 | [2-OCH₂CHF₂-4-CF₃] |
| D-1 | [2-OCH₂CHF₂-5-CF₃] | D-1 | [2-OCH₂CHF₂-6-CF₃] |
| D-1 | [2-OCH₂CHF₂-3-OCHF₂] | D-1 | [2-OCH₂CHF₂-4-OCHF₂] |
| D-1 | [2-OCH₂CHF₂-5-OCHF₂] | D-1 | [2-OCH₂CHF₂-6-OCHF₂] |
| D-1 | [2-OCH₂CHF₂-3-OCF₃] | D-1 | [2-OCH₂CHF₂-4-OCF₃] |
| D-1 | [2-OCH₂CHF₂-5-OCF₃] | D-1 | [2-OCH₂CHF₂-6-OCF₃] |
| D-1 | [2-OCH₂CHF₂-3-CN] | D-1 | [2-OCH₂CHF₂-4-CN] |
| D-1 | [2-OCH₂CHF₂-5-CN] | D-1 | [2-OCH₂CHF₂-6-CN] |
| D-1 | [2-OCH₂CHF₂-3-NO₂] | D-1 | [2-OCH₂CHF₂-4-NO₂] |
| D-1 | [2-OCH₂CHF₂-5-NO₂] | D-1 | [2-OCH₂CHF₂-6-NO₂] |
| D-1 | [2-OCH₂CHF₂-3-C(O)Me] | D-1 | [2-OCH₂CHF₂-4-C(O)Me] |
| D-1 | [2-OCH₂CHF₂-5-C(O)Me] | D-1 | [2-OCH₂CHF₂-6-C(O)Me] |
| D-1 | [2-OCH₂CHF₂-3-C(=NOMe)Me] | D-1 | [2-OCH₂CHF₂-4-C(=NOMe)Me] |
| D-1 | [2-OCH₂CHF₂-5-C(=NOMe)Me] | D-1 | [2-OCH₂CHF₂-6-C(=NOMe)Me] |
| D-1 | [2-OCH₂CF₃-3-F] | D-1 | [2-OCH₂CF₃-4-F] |
| D-1 | [2-OCH₂CF₃-5-F] | D-1 | [2-OCH₂CF₃-3-Cl] |
| D-1 | [2-OCH₂CF₃-4-Cl] | D-1 | [2-OCH₂CF₃-5-Cl] |
| D-1 | [2-OCH₂CF₃-3-Me] | D-1 | [2-OCH₂CF₃-4-Me] |
| D-1 | [2-OCH₂CF₃-5-Me] | D-1 | [2-OCH₂CF₃-6-Me] |
| D-1 | [2-OCH₂CF₃-3-CF₃] | D-1 | [2-OCH₂CF₃-4-CF₃] |
| D-1 | [2-OCH₂CF₃-5-CF₃] | D-1 | [2-OCH₂CF₃-6-CF₃] |
| D-1 | [2-OCH₂CF₃-3-OCHF₂] | D-1 | [2-OCH₂CF₃-4-OCHF₂] |
| D-1 | [2-OCH₂CF₃-5-OCHF₂] | D-1 | [2-OCH₂CF₃-6-OCHF₂] |
| D-1 | [2-OCH₂CF₃-3-OCF₃] | D-1 | [2-OCH₂CF₃-4-OCF₃] |
| D-1 | [2-OCH₂CF₃-5-OCF₃] | D-1 | [2-OCH₂CF₃-6-OCF₃] |
| D-1 | [2-OCH₂CF₃-3-CN] | D-1 | [2-OCH₂CF₃-4-CN] |
| D-1 | [2-OCH₂CF₃-5-CN] | D-1 | [2-OCH₂CF₃-6-CN] |
| D-1 | [2-OCH₂CF₃-3-NO₂] | D-1 | [2-OCH₂CF₃-4-NO₂] |
| D-1 | [2-OCH₂CF₃-5-NO₂] | D-1 | [2-OCH₂CF₃-6-NO₂] |
| D-1 | [2-OCH₂CF₃-3-C(O)Me] | D-1 | [2-OCH₂CF₃-4-C(O)Me] |
| D-1 | [2-OCH₂CF₃-5-C(O)Me] | D-1 | [2-OCH₂CF₃-6-C(O)Me] |
| D-1 | [2-OCH₂CF₃-3-C(=NOMe)Me] | D-1 | [2-OCH₂CF₃-4-C(=NOMe)Me] |
| D-1 | [2-OCH₂CF₃-5-C(=NOMe)Me] | D-1 | [2-OCH₂CF₃-6-C(=NOMe)Me] |
| D-1 | [2-SMe-3-F] | D-1 | [2-SMe-4-F] |
| D-1 | [2-SMe-5-F] | D-1 | [2-SMe-3-Cl] |
| D-1 | [2-SMe-4-Cl] | D-1 | [2-SMe-5-Cl] |
| D-1 | [2-SMe-3-Me] | D-1 | [2-SMe-4-Me] |
| D-1 | [2-SMe-5-Me] | D-1 | [2-SMe-6-Me] |
| D-1 | [2-SMe-3-CF₃] | D-1 | [2-SMe-4-CF₃] |
| D-1 | [2-SMe-5-CF₃] | D-1 | [2-SMe-6-CF₃] |
| D-1 | [2-SMe-3-OCHF₂] | D-1 | [2-SMe-4-OCHF₂] |
| D-1 | [2-SMe-5-OCHF₂] | D-1 | [2-SMe-6-OCHF₂] |
| D-1 | [2-SMe-3-OCF₃] | D-1 | [2-SMe-4-OCF₃] |
| D-1 | [2-SMe-5-OCF₃] | D-1 | [2-SMe-6-OCF₃] |
| D-1 | [2-SMe-3-CN] | D-1 | [2-SMe-4-CN] |
| D-1 | [2-SMe-5-CN] | D-1 | [2-SMe-6-CN] |
| D-1 | [2-SMe-3-NO₂] | D-1 | [2-SMe-4-NO₂] |
| D-1 | [2-SMe-5-NO₂] | D-1 | [2-SMe-6-NO₂] |
| D-1 | [2-SMe-3-C(O)Me] | D-1 | [2-SMe-4-C(O)Me] |
| D-1 | [2-SMe-5-C(O)Me] | D-1 | [2-SMe-6-C(O)Me] |
| D-1 | [2-SMe-3-C(=NOMe)Me] | D-1 | [2-SMe-4-C(=NOMe)Me] |
| D-1 | [2-SMe-5-C(=NOMe)Me] | D-1 | [2-SMe-6-C(=NOMe)Me] |
| D-1 | [2-S(O)Me-3-F] | D-1 | [2-S(O)Me-4-F] |
| D-1 | [2-S(O)Me-5-F] | D-1 | [2-S(O)Me-3-Cl] |
| D-1 | [2-S(O)Me-4-Cl] | D-1 | [2-S(O)Me-5-Cl] |
| D-1 | [2-S(O)Me-3-Me] | D-1 | [2-S(O)Me-4-Me] |
| D-1 | [2-S(O)Me-5-Me] | D-1 | [2-S(O)Me-6-Me] |
| D-1 | [2-S(O)Me-3-CF₃] | D-1 | [2-S(O)Me-4-CF₃] |
| D-1 | [2-S(O)Me-5-CF₃] | D-1 | [2-S(O)Me-6-CF₃] |
| D-1 | [2-S(O)Me-3-OCHF₂] | D-1 | [2-S(O)Me-4-OCHF₂] |
| D-1 | [2-S(O)Me-5-OCHF₂] | D-1 | [2-S(O)Me-6-OCHF₂] |
| D-1 | [2-S(O)Me-3-OCF₃] | D-1 | [2-S(O)Me-4-OCF₃] |
| D-1 | [2-S(O)Me-5-OCF₃] | D-1 | [2-S(O)Me-6-OCF₃] |
| D-1 | [2-S(O)Me-3-CN] | D-1 | [2-S(O)Me-4-CN] |
| D-1 | [2-S(O)Me-5-CN] | D-1 | [2-S(O)Me-6-CN] |
| D-1 | [2-S(O)Me-3-NO₂] | D-1 | [2-S(O)Me-4-NO₂] |
| D-1 | [2-S(O)Me-5-NO₂] | D-1 | [2-S(O)Me-6-NO₂] |
| D-1 | [2-S(O)Me-3-C(O)Me] | D-1 | [2-S(O)Me-4-C(O)Me] |
| D-1 | [2-S(O)Me-5-C(O)Me] | D-1 | [2-S(O)Me-6-C(O)Me] |
| D-1 | [2-S(O)Me-3-C(=NOMe)Me] | D-1 | [2-S(O)Me-4-C(=NOMe)Me] |
| D-1 | [2-S(O)Me-5-C(=NOMe)Me] | D-1 | [2-S(O)Me-6-C(=NOMe)Me] |
| D-1 | [2-S(O)₂Me-3-F] | D-1 | [2-S(O)₂Me-4-F] |
| D-1 | [2-S(O)₂Me-5-F] | D-1 | [2-S(O)₂Me-3-Cl] |
| D-1 | [2-S(O)₂Me-4-Cl] | D-1 | [2-S(O)₂Me-5-Cl] |
| D-1 | [2-S(O)₂Me-3-Me] | D-1 | [2-S(O)₂Me-4-Me] |
| D-1 | [2-S(O)₂Me-5-Me] | D-1 | [2-S(O)₂Me-6-Me] |
| D-1 | [2-S(O)₂Me-3-CF₃] | D-1 | [2-S(O)₂Me-4-CF₃] |
| D-1 | [2-S(O)₂Me-5-CF₃] | D-1 | [2-S(O)₂Me-6-CF₃] |
| D-1 | [2-S(O)₂Me-3-OCHF₂] | D-1 | [2-S(O)₂Me-4-OCHF₂] |
| D-1 | [2-S(O)₂Me-5-OCHF₂] | D-1 | [2-S(O)₂Me-6-OCHF₂] |
| D-1 | [2-S(O)₂Me-3-OCF₃] | D-1 | [2-S(O)₂Me-4-OCF₃] |
| D-1 | [2-S(O)₂Me-5-OCF₃] | D-1 | [2-S(O)₂Me-6-OCF₃] |
| D-1 | [2-S(O)₂Me-3-CN] | D-1 | [2-S(O)₂Me-4-CN] |
| D-1 | [2-S(O)₂Me-5-CN] | D-1 | [2-S(O)₂Me-6-CN] |
| D-1 | [2-S(O)₂Me-3-NO₂] | D-1 | [2-S(O)₂Me-4-NO₂] |
| D-1 | [2-S(O)₂Me-5-NO₂] | D-1 | [2-S(O)₂Me-6-NO₂] |
| D-1 | [2-S(O)₂Me-3-C(O)Me] | D-1 | [2-S(O)₂Me-4-C(O)Me] |
| D-1 | [2-S(O)₂Me-5-C(O)Me] | D-1 | [2-S(O)₂Me-6-C(O)Me] |
| D-1 | [2-S(O)₂Me-3-C(=NOMe)Me] | D-1 | [2-S(O)₂Me-4-C(=NOMe)Me] |
| D-1 | [2-S(O)₂Me-5-C(=NOMe)Me] | D-1 | [2-S(O)₂Me-6-C(=NOMe)Me] |
| D-1 | [2-SEt-3-F] | D-1 | [2-SEt-4-F] |
| D-1 | [2-SEt-5-F] | D-1 | [2-SEt-3-Cl] |
| D-1 | [2-SEt-4-Cl] | D-1 | [2-SEt-5-Cl] |
| D-1 | [2-SEt-3-Me] | D-1 | [2-SEt-4-Me] |
| D-1 | [2-SEt-5-Me] | D-1 | [2-SEt-6-Me] |
| D-1 | [2-SEt-3-CF₃] | D-1 | [2-SEt-4-CF₃] |
| D-1 | [2-SEt-5-CF₃] | D-1 | [2-SEt-6-CF₃] |
| D-1 | [2-SEt-3-OCHF₂] | D-1 | [2-SEt-4-OCHF₂] |
| D-1 | [2-SEt-5-OCHF₂] | D-1 | [2-SEt-6-OCHF₂] |
| D-1 | [2-SEt-3-OCF₃] | D-1 | [2-SEt-4-OCF₃] |
| D-1 | [2-SEt-5-OCF₃] | D-1 | [2-SEt-6-OCF₃] |
| D-1 | [2-SEt-3-CN] | D-1 | [2-SEt-4-CN] |
| D-1 | [2-SEt-5-CN] | D-1 | [2-SEt-6-CN] |
| D-1 | [2-SEt-3-NO₂] | D-1 | [2-SEt-4-NO₂] |
| D-1 | [2-SEt-5-NO₂] | D-1 | [2-SEt-6-NO₂] |
| D-1 | [2-SEt-3-C(O)Me] | D-1 | [2-SEt-4-C(O)Me] |
| D-1 | [2-SEt-5-C(O)Me] | D-1 | [2-SEt-6-C(O)Me] |
| D-1 | [2-SEt-3-C(=NOMe)Me] | D-1 | [2-SEt-4-C(=NOMe)Me] |
| D-1 | [2-SEt-5-C(=NOMe)Me] | D-1 | [2-SEt-6-C(=NOMe)Me] |
| D-1 | [2-S(O)Et-3-F] | D-1 | [2-S(O)Et-4-F] |
| D-1 | [2-S(O)Et-5-F] | D-1 | [2-S(O)Et-3-Cl] |
| D-1 | [2-S(O)Et-4-Cl] | D-1 | [2-S(O)Et-5-Cl] |
| D-1 | [2-S(O)Et-3-Me] | D-1 | [2-S(O)Et-4-Me] |
| D-1 | [2-S(O)Et-5-Me] | D-1 | [2-S(O)Et-6-Me] |
| D-1 | [2-S(O)Et-3-CF₃] | D-1 | [2-S(O)Et-4-CF₃] |
| D-1 | [2-S(O)Et-5-CF₃] | D-1 | [2-S(O)Et-6-CF₃] |
| D-1 | [2-S(O)Et-3-OCHF₂] | D-1 | [2-S(O)Et-4-OCHF₂] |
| D-1 | [2-S(O)Et-5-OCHF₂] | D-1 | [2-S(O)Et-6-OCHF₂] |
| D-1 | [2-S(O)Et-3-OCF₃] | D-1 | [2-S(O)Et-4-OCF₃] |
| D-1 | [2-S(O)Et-5-OCF₃] | D-1 | [2-S(O)Et-6-OCF₃] |
| D-1 | [2-S(O)Et-3-CN] | D-1 | [2-S(O)Et-4-CN] |
| D-1 | [2-S(O)Et-5-CN] | D-1 | [2-S(O)Et-6-CN] |
| D-1 | [2-S(O)Et-3-NO₂] | D-1 | [2-S(O)Et-4-NO₂] |
| D-1 | [2-S(O)Et-5-NO₂] | D-1 | [2-S(O)Et-6-NO₂] |
| D-1 | [2-S(O)Et-3-C(O)Me] | D-1 | [2-S(O)Et-4-C(O)Me] |
| D-1 | [2-S(O)Et-5-C(O)Me] | D-1 | [2-S(O)Et-6-C(O)Me] |
| D-1 | [2-S(O)Et-3-C(=NOMe)Me] | D-1 | [2-S(O)Et-4-C(=NOMe)Me] |
| D-1 | [2-S(O)Et-5-C(=NOMe)Me] | D-1 | [2-S(O)Et-6-C(=NOMe)Me] |
| D-1 | [2-S(O)₂Et-3-F] | D-1 | [2-S(O)₂Et-4-F] |
| D-1 | [2-S(O)₂Et-5-F] | D-1 | [2-S(O)₂Et-3-Cl] |
| D-1 | [2-S(O)₂Et-4-Cl] | D-1 | [2-S(O)₂Et-5-Cl] |
| D-1 | [2-S(O)₂Et-3-Me] | D-1 | [2-S(O)₂Et-4-Me] |
| D-1 | [2-S(O)₂Et-5-Me] | D-1 | [2-S(O)₂Et-6-Me] |
| D-1 | [2-S(O)₂Et-3-CF₃] | D-1 | [2-S(O)₂Et-4-CF₃] |
| D-1 | [2-S(O)₂Et-5-CF₃] | D-1 | [2-S(O)₂Et-6-CF₃] |
| D-1 | [2-S(O)₂Et-3-OCHF₂] | D-1 | [2-S(O)₂Et-4-OCHF₂] |
| D-1 | [2-S(O)₂Et-5-OCHF₂] | D-1 | [2-S(O)₂Et-6-OCHF₂] |
| D-1 | [2-S(O)₂Et-3-OCF₃] | D-1 | [2-S(O)₂Et-4-OCF₃] |
| D-1 | [2-S(O)₂Et-5-OCF₃] | D-1 | [2-S(O)₂Et-6-OCF₃] |
| D-1 | [2-S(O)₂Et-3-CN] | D-1 | [2-S(O)₂Et-4-CN] |
| D-1 | [2-S(O)₂Et-5-CN] | D-1 | [2-S(O)₂Et-6-CN] |
| D-1 | [2-S(O)₂Et-3-NO₂] | D-1 | [2-S(O)₂Et-4-NO₂] |
| D-1 | [2-S(O)₂Et-5-NO₂] | D-1 | [2-S(O)₂Et-6-NO₂] |
| D-1 | [2-S(O)₂Et-3-C(O)Me] | D-1 | [2-S(O)₂Et-4-C(O)Me] |
| D-1 | [2-S(O)₂Et-5-C(O)Me] | D-1 | [2-S(O)₂Et-6-C(O)Me] |
| D-1 | [2-S(O)₂Et-3-C(=NOMe)Me] | D-1 | [2-S(O)₂Et-4-C(=NOMe)Me] |
| D-1 | [2-S(O)₂Et-5-C(=NOMe)Me] | D-1 | [2-S(O)₂Et-6-C(=NOMe)Me] |
| D-2 | [3-F] | D-2 | [4-F] |
| D-2 | [5-F] | D-2 | [6-F] |
| D-2 | [3-Br] | D-2 | [4-Br] |
| D-2 | [5-Br] | D-2 | [6-Br] |
| D-2 | [3-Me] | D-2 | [4-Me] |
| D-2 | [5-Me] | D-2 | [6-Me] |
| D-2 | [3-SMe] | D-2 | [4-SMe] |
| D-2 | [5-SMe] | D-2 | [6-SMe] |
| D-2 | [3-CF₃] | D-2 | [4-CF₃] |
| D-2 | [5-CF₃] | D-2 | [6-CF₃] |
| D-2 | [3,5-Cl₂] | D-2 | [3-Cl-5-CF₃] |
| D-2 | [3,6-Cl₂] | D-2 | [3,5,6-Cl₃] |
| D-2 | [3-OEt] | D-2 | [3-OPr-n] |
| D-2 | [3-OPr-i] | D-2 | [3-OBu-tert] |
| D-2 | [5-OEt] | D-2 | [5-OPr-n] |
| D-2 | [5-OPr-i] | D-2 | [5-OBu-i] |
| D-2 | [5-OPr-c] | D-2 | [5-OBu-n] |
| D-2 | [5-OBu-i] | D-2 | [5-OBu-sec] |
| D-2 | [5-OBu-tert] | D-2 | [5-OBu-c] |
| D-2 | [5-OPen-c] | D-2 | [5-OHex-c] |
| D-2 | [5-OCH₂CH=CH₂] | D-2 | [5-OCH₂C≡CH] |
| D-2 | [5-OCH₂CH=C(Me)₂] | D-2 | [5-OCH₂C≡CMe] |
| D-2 | [5-OCHF₂] | D-2 | [5-OCF₃] |
| D-2 | [5-OCH₂CH₂F] | D-2 | [5-OCH₂CH₂Cl] |
| D-2 | [5-OCH₂CHF₂] | D-2 | [5-OCH₂CH₂CF₃] |
| D-2 | [5-OCH₂CF₂CHF₂] | D-2 | [5-OCH₂CF₂CF₃] |
| D-2 | [5-OCH₂CH=CF₂] | D-2 | [5-OCH₂CH=CCl₂] |
| D-2 | [5-OCH₂CH₂CF=CF₂] | D-2 | [5-OCH₂CH₂CH=CF₂] |
| D-2 | [5-OCH₂C≡CCl] | D-2 | [5-OCH₂C≡CBr] |
| D-2 | [5-OCH₂CH₂OMe] | D-2 | [5-OCH₂CH₂SMe] |
| D-2 | [5-OCH₂CH₂S(O)Me] | D-2 | [5-OCH₂CH₂S(O)₂Me] |
| D-2 | [5-SEt] | D-2 | [5-SPr-n] |
| D-2 | [5-SPr-i] | D-2 | [5-SBu-i] |
| D-2 | [5-SPr-c] | D-2 | [5-SBu-n] |
| D-2 | [5-SBu-i] | D-2 | [5-SBu-sec] |
| D-2 | [5-SBu-tert] | D-2 | [5-SBu-c] |
| D-2 | [5-SPen-c] | D-2 | [5-SHex-c] |
| D-2 | [5-SCH₂CH=CH₂] | D-2 | [5-SCH₂C≡CH] |
| D-2 | [5-SCH₂CH=C(Me)₂] | D-2 | [5-SCH₂C≡CMe] |
| D-2 | [5-SCHF₂] | D-2 | [5-SCF₃] |
| D-2 | [5-SCH₂CH₂F] | D-2 | [5-SCH₂CH₂Cl] |
| D-2 | [5-SCH₂CHF₂] | D-2 | [5-SCH₂CH₂CF₃] |
| D-2 | [5-SCH₂CF₂CHF₂] | D-2 | [5-SCH₂CF₂CF₃] |
| D-2 | [5-SCH₂CH=CF₂] | D-2 | [5-SCH₂CH=CCl₂] |
| D-2 | [5-SCH₂CH₂CF=CF₂] | D-2 | [5-SCH₂CH₂CH=CF₂] |
| D-2 | [5-SCH₂C≡CCl] | D-2 | [5-SCH₂C≡CBr] |
| D-2 | [5-SCH₂CH₂OMe] | D-2 | [5-SCH₂CH₂SMe] |
| D-2 | [5-S(O)Et] | D-2 | [5-S(O)Pr-n] |
| D-2 | [5-S(O)Pr-i] | D-2 | [5-S(O)Bu-i] |
| D-2 | [5-S(O)Pr-c] | D-2 | [5-S(O)Bu-n] |
| D-2 | [5-S(O)Bu-i] | D-2 | [5-S(O)Bu-sec] |
| D-2 | [5-S(O)Bu-tert] | D-2 | [5-S(O)Bu-c] |
| D-2 | [5-S(O)Pen-c] | D-2 | [5-S(O)Hex-c] |
| D-2 | [5-S(O)CH₂CH=CH₂] | D-2 | [5-S(O)CH₂C≡CH] |
| D-2 | [5-S(O)CH₂CH=C(Me)₂] | D-2 | [5-S(O)CH₂C≡CMe] |
| D-2 | [5-S(O)CHF₂] | D-2 | [5-S(O)CF₃] |
| D-2 | [5-S(O)CH₂CH₂F] | D-2 | [5-S(O)CH₂CH₂Cl] |
| D-2 | [5-S(O)CH₂CHF₂] | D-2 | [5-S(O)CH₂CH₂CF₃] |
| D-2 | [5-S(O)CH₂CF₂CHF₂] | D-2 | [5-S(O)CH₂CF₂CF₃] |
| D-2 | [5-S(O)CH₂CH=CF₂] | D-2 | [5-S(O)CH₂CH=CCl₂] |
| D-2 | [5-S(O)CH₂CH₂CF=CF₂] | D-2 | [5-S(O)CH₂CH₂CH=CF₂] |
| D-2 | [5-S(O)CH₂C≡CCl] | D-2 | [5-S(O)CH₂C≡CBr] |
| D-2 | [5-S(O)CH₂CH₂OMe] | D-2 | [5-S(O)CH₂CH₂SMe] |
| D-2 | [5-S(O)₂Et] | D-2 | [5-S(O)₂Pr-n] |
| D-2 | [5-S(O)₂Pr-i] | D-2 | [5-S(O)₂Bu-i] |
| D-2 | [5-S(O)₂Pr-c] | D-2 | [5-S(O)₂Bu-n] |
| D-2 | [5-S(O)₂Bu-i] | D-2 | [5-S(O)₂Bu-sec] |
| D-2 | [5-S(O)₂Bu-tert] | D-2 | [5-S(O)₂Bu-c] |
| D-2 | [5-S(O)₂Pen-c] | D-2 | [5-S(O)₂Hex-c] |
| D-2 | [5-S(O)₂CH₂CH=CH₂] | D-2 | [5-S(O)₂CH₂C≡CH] |
| D-2 | [5-S(O)₂CH₂CH=C(Me)₂] | D-2 | [5-S(O)₂CH₂C≡CMe] |
| D-2 | [5-S(O)₂CHF₂] | D-2 | [5-S(O)₂CF₃] |
| D-2 | [5-S(O)₂CH₂CH₂F] | D-2 | [5-S(O)₂CH₂CH₂Cl] |
| D-2 | [5-S(O)₂CH₂CHF₂] | D-2 | [5-S(O)₂CH₂CH₂CF₃] |
| D-2 | [5-S(O)₂CH₂CF₂CHF₂] | D-2 | [5-S(O)₂CH₂CF₂CF₃] |
| D-2 | [5-S(O)₂CH₂CH=CF₂] | D-2 | [5-S(O)₂CH₂CH=CCl₂] |
| D-2 | [5-S(O)₂CH₂CH₂CF=CF₂] | D-2 | [5-S(O)₂CH₂CH₂CH=CF₂] |
| D-2 | [5-S(O)₂CH₂C-CCl] | D-2 | [5-S(O)₂CH₂C≡CBr] |
| D-2 | [5-S(O)₂CH₂CH₂OMe] | D-2 | [5-S(O)₂CH₂CH₂SMe] |
| D-2 | [5-S(O)₂CH₂CH₂S(O)Me] | D-2 | [5-S(O)₂CH₂CH₂S(O)₂Me] |
| D-3 | [2-F] | D-3 | [4-F] |
| D-3 | [5-F] | D-3 | [6-F] |
| D-3 | [2-Br] | D-3 | [4-Br] |
| D-3 | [5-Br] | D-3 | [6-Br] |
| D-3 | [2-Me] | D-3 | [4-Me] |
| D-3 | [5-Me] | D-3 | [6-Me] |
| D-3 | [2-SMe] | D-3 | [4-SMe] |
| D-3 | [5-SMe] | D-3 | [6-SMe] |
| D-3 | [2-CF₃] | D-3 | [4-CF₃] |
| D-3 | [5-CF₃] | D-3 | [6-CF₃] |
| D-3 | [2,4-Cl₂] | D-3 | [2-Cl-4-CF₃] |
| D-3 | [2-OCHF₂] | D-3 | [2-OCF₃] |
| D-3 | [2-OEt] | D-3 | [2-OPr-n] |
| D-3 | [2-OPr-i] | D-3 | [2-OBu-tert] |
| D-3 | [2-OEt] | D-3 | [2-OPr-n] |
| D-3 | [2-OPr-i] | D-3 | [2-OBu-i] |
| D-3 | [2-OPr-c] | D-3 | [2-OBu-n] |
| D-3 | [2-OBu-i] | D-3 | [2-OBu-sec] |
| D-3 | [2-OBu-tert] | D-3 | [2-OBu-c] |
| D-3 | [2-OPen-c] | D-3 | [2-OHex-c] |
| D-3 | [2-OCH₂CH=CH₂] | D-3 | [2-OCH₂C≡CH] |
| D-3 | [2-OCH₂CH=C(Me)₂] | D-3 | [2-OCH₂C≡CMe] |
| D-3 | [2-OCHF₂] | D-3 | [2-OCF₃] |
| D-3 | [2-OCH₂CH₂F] | D-3 | [2-OCH₂CH₂Cl] |
| D-3 | [2-OCH₂CHF₂] | D-3 | [2-OCH₂CH₂CF₃] |
| D-3 | [2-OCH₂CF₂CHF₂] | D-3 | [2-OCH₂CF₂CF₃] |
| D-3 | [2-OCH₂CH=CF₂] | D-3 | [2-OCH₂CH=CCl₂] |
| D-3 | [2-OCH₂CH₂CF=CF₂] | D-3 | [2-OCH₂CH₂CH=CF₂] |
| D-3 | [2-OCH₂C-CCl] | D-3 | [2-OCH₂C≡CBr] |
| D-3 | [2-OCH₂CH₂OMe] | D-3 | [2-OCH₂CH₂SMe] |
| D-3 | [2-OCH₂CH₂S(O)Me] | D-3 | [2-OCH₂CH₂S(O)₂Me] |
| D-3 | [2-SEt] | D-3 | [2-SPr-n] |
| D-3 | [2-SPr-i] | D-3 | [2-SBu-tert] |
| D-3 | [2-SEt] | D-3 | [2-SPr-n] |
| D-3 | [2-SPr-i] | D-3 | [2-SBu-i] |
| D-3 | [2-SPr-c] | D-3 | [2-SBu-n] |
| D-3 | [2-SBu-i] | D-3 | [2-SBu-sec] |
| D-3 | [2-SBu-tert] | D-3 | [2-SBu-c] |
| D-3 | [2-SPen-c] | D-3 | [2-SHex-c] |
| D-3 | [2-SCH₂CH=CH₂] | D-3 | [2-SCH₂C≡CH] |
| D-3 | [2-SCH₂CH=C(Me)₂] | D-3 | [2-SCH₂C≡CMe] |
| D-3 | [2-SCHF₂] | D-3 | [2-SCF₃] |
| D-3 | [2-SCH₂CH₂F] | D-3 | [2-SCH₂CH₂Cl] |
| D-3 | [2-SCH₂CHF₂] | D-3 | [2-SCH₂CH₂CF₃] |
| D-3 | [2-SCH₂CF₂CHF₂] | D-3 | [2-SCH₂CF₂CF₃] |
| D-3 | [2-SCH₂CH=CF₂] | D-3 | [2-SCH₂CH=CCl₂] |
| D-3 | [2-SCH₂CH₂CF=CF₂] | D-3 | [2-SCH₂CH₂CH=CF₂] |
| D-3 | [2-SCH₂C≡CCl] | D-3 | [2-SCH₂C≡CBr] |
| D-3 | [2-SCH₂CH₂OMe] | D-3 | [2-SCH₂CH₂SMe] |
| D-3 | [2-SCH₂CH₂S(O)Me] | D-3 | [2-SCH₂CH₂S(O)₂Me] |
| D-3 | [2-S(O)Et] | D-3 | [2-S(O)Pr-n] |
| D-3 | [2-S(O)Pr-i] | D-3 | [2-S(O)Bu-tert] |
| D-3 | [2-S(O)Et] | D-3 | [2-S(O)Pr-n] |
| D-3 | [2-S(O)Pr-i] | D-3 | [2-S(O)Bu-i] |
| D-3 | [2-S(O)Pr-c] | D-3 | [2-S(O)Bu-n] |
| D-3 | [2-S(O)Bu-i] | D-3 | [2-S(O)Bu-sec] |
| D-3 | [2-S(O)Bu-tert] | D-3 | [2-S(O)Bu-c] |
| D-3 | [2-S(O)Pen-c] | D-3 | [2-S(O)Hex-c] |
| D-3 | [2-S(O)CH₂CH=CH₂] | D-3 | [2-S(O)CH₂C≡CH] |
| D-3 | [2-S(O)CH₂CH=C(Me)₂] | D-3 | [2-S(O)CH₂C≡CMe] |
| D-3 | [2-S(O)CHF₂] | D-3 | [2-S(O)CF₃] |
| D-3 | [2-S(O)CH₂CH₂F] | D-3 | [2-S(O)CH₂CH₂Cl] |
| D-3 | [2-S(O)CH₂CHF₂] | D-3 | [2-S(O)CH₂CH₂CF₃] |
| D-3 | [2-S(O)CH₂CF₂CHF₂] | D-3 | [2-S(O)CH₂CF₂CF₃] |
| D-3 | [2-S(O)CH₂CH=CF₂] | D-3 | [2-S(O)CH₂CH=CCl₂] |
| D-3 | [2-S(O)CH₂CH₂CF=CF₂] | D-3 | [2-S(O)CH₂CH₂CH=CF₂] |
| D-3 | [2-S(O)CH₂C≡CCl] | D-3 | [2-S(O)CH₂C≡CBr] |
| D-3 | [2-S(O)CH₂CH₂OMe] | D-3 | [2-S(O)CH₂CH₂SMe] |
| D-3 | [2-S(O)CH₂CH₂S(O)Me] | D-3 | [2-S(O)CH₂CH₂S(O)₂Me] |
| D-3 | [2-S(O)₂Et] | D-3 | [2-S(O)₂Pr-n] |
| D-3 | [2-S(O)₂Pr-i] | D-3 | [2-S(O)₂Bu-tert] |
| D-3 | [2-S(O)₂Et] | D-3 | [2-S(O)₂Pr-n] |
| D-3 | [2-S(O)₂Pr-i] | D-3 | [2-S(O)₂Bu-i] |
| D-3 | [2-S(O)₂Pr-c] | D-3 | [2-S(O)₂Bu-n] |
| D-3 | [2-S(O)₂Bu-i] | D-3 | [2-S(O)₂Bu-sec] |
| D-3 | [2-S(O)₂Bu-tert] | D-3 | [2-S(O)₂Bu-c] |
| D-3 | [2-S(O)₂Pen-c] | D-3 | [2-S(O)₂Hex-c] |
| D-3 | [2-S(O)₂CH₂CH=CH₂] | D-3 | [2-S(O)₂CH₂C≡CH] |
| D-3 | [2-S(O)₂CH₂CH=C(Me)₂] | D-3 | [2-S(O)₂CH₂C≡CMe] |
| D-3 | [2-S(O)₂CHF₂] | D-3 | [2-S(O)₂CF₃] |
| D-3 | [2-S(O)₂CH₂CH₂F] | D-3 | [2-S(O)₂CH₂CH₂Cl] |
| D-3 | [2-S(O)₂CH₂CHF₂] | D-3 | [2-S(O)₂CH₂CH₂CF₃] |
| D-3 | [2-S(O)₂CH₂CF₂CHF₂] | D-3 | [2-S(O)₂CH₂CF₂CF₃] |
| D-3 | [2-S(O)₂CH₂CH=CF₂] | D-3 | [2-S(O)₂CH₂CH=CCl₂] |
| D-3 | [2-S(O)₂CH₂CH₂CF=CF₂] | D-3 | [2-S(O)₂CH₂CH₂CH=CF₂] |
| D-3 | [2-S(O)₂CH₂C≡CCl] | D-3 | [2-S(O)₂CH₂C≡CBr] |
| D-3 | [2-S(O)₂CH₂CH₂OMe] | D-3 | [2-S(O)₂CH₂CH₂SMe] |
| D-3 | [2-S(O)₂CH₂CH₂S(O)Me] | D-3 | [2-S(O)₂CH₂CH₂S(O)₂Me] |
| D-3 | [4-OEt] | D-3 | [4-OPr-n] |
| D-3 | [4-OPr-i] | D-3 | [4-OBu-t] |
| D-3 | [4-OEt] | D-3 | [4-OPr-n] |
| D-3 | [4-OPr-i] | D-3 | [4-OBu-i] |
| D-3 | [4-OPr-c] | D-3 | [4-OBu-n] |
| D-3 | [4-OBu-i] | D-3 | [4-OBu-sec] |
| D-3 | [4-OBu-tert] | D-3 | [4-OBu-c] |
| D-3 | [4-OPen-c] | D-3 | [4-OHex-c] |
| D-3 | [4-OCH₂CH=CH₂] | D-3 | [4-OCH₂C≡CH] |
| D-3 | [4-OCH₂CH=C(Me)₂] | D-3 | [4-OCH₂C≡CMe] |
| D-3 | [4-OCHF₂] | D-3 | [4-OCF₃] |
| D-3 | [4-OCH₂CH₂F] | D-3 | [4-OCH₂CH₂Cl] |
| D-3 | [4-OCH₂CHF₂] | D-3 | [4-OCH₂CH₂CF₃] |
| D-3 | [4-OCH₂CF₂CHF₂] | D-3 | [4-OCH₂CF₂CF₃] |
| D-3 | [4-OCH₂CH=CF₂] | D-3 | [4-OCH₂CH=CCl₂] |
| D-3 | [4-OCH₂CH₂CF=CF₂] | D-3 | [4-OCH₂CH₂CH=CF₂] |
| D-3 | [4-OCH₂C≡CCl] | D-3 | [4-OCH₂C≡CBr] |
| D-3 | [4-OCH₂CH₂OMe] | D-3 | [4-OCH₂CH₂SMe] |
| D-3 | [4-SEt] | D-3 | [4-SPr-n] |
| D-3 | [4-SPr-i] | D-3 | [4-SBu-tert] |
| D-3 | [4-SEt] | D-3 | [4-SPr-n] |
| D-3 | [4-SPr-i] | D-3 | [4-SBu-i] |
| D-3 | [4-SPr-c] | D-3 | [4-SBu-n] |
| D-3 | [4-SBu-i] | D-3 | [4-SBu-sec] |
| D-3 | [4-SBu-tert] | D-3 | [4-SBu-c] |
| D-3 | [4-SPen-c] | D-3 | [4-SHex-c] |
| D-3 | [4-SCH₂CH=CH₂] | D-3 | [4-SCH₂C≡CH] |
| D-3 | [4-SCH₂CH=C(Me)₂] | D-3 | [4-SCH₂C≡CMe] |
| D-3 | [4-SCHF₂] | D-3 | [4-SCF₃] |
| D-3 | [4-SCH₂CH₂F] | D-3 | [4-SCH₂CH₂Cl] |
| D-3 | [4-SCH₂CHF₂] | D-3 | [4-SCH₂CH₂CF₃] |
| D-3 | [4-SCH₂CF₂CHF₂] | D-3 | [4-SCH₂CF₂CF₃] |
| D-3 | [4-SCH₂CH=CF₂] | D-3 | [4-SCH₂CH=CCl₂] |
| D-3 | [4-SCH₂CH₂CF=CF₂] | D-3 | [4-SCH₂CH₂CH=CF₂] |
| D-3 | [4-SCH₂C≡CCl] | D-3 | [4-SCH₂C≡CBr] |
| D-3 | [4-SCH₂CH₂OMe] | D-3 | [4-SCH₂CH₂SMe] |
| D-3 | [4-SCH₂CH₂S(O)Me] | D-3 | [4-SCH₂CH₂S(O)₂Me] |
| D-3 | [4-S(O)Et] | D-3 | [4-S(O)Pr-n] |
| D-3 | [4-S(O)Pr-i] | D-3 | [4-S(O)Bu-tert] |
| D-3 | [4-S(O)Et] | D-3 | [4-S(O)Pr-n] |
| D-3 | [4-S(O)Pr-i] | D-3 | [4-S(O)Bu-i] |
| D-3 | [4-S(O)Pr-c] | D-3 | [4-S(O)Bu-n] |
| D-3 | [4-S(O)Bu-i] | D-3 | [4-S(O)Bu-sec] |
| D-3 | [4-S(O)Bu-tert] | D-3 | [4-S(O)Bu-c] |
| D-3 | [4-S(O)Pen-c] | D-3 | [4-S(O)Hex-c] |
| D-3 | [4-S(O)CH₂CH=CH₂] | D-3 | [4-S(O)CH₂C≡CH] |
| D-3 | [4-S(O)CH₂CH=C(Me)₂] | D-3 | [4-S(O)CH₂C≡CMe] |
| D-3 | [4-S(O)CHF₂] | D-3 | [4-S(O)CF₃] |
| D-3 | [4-S(O)CH₂CH₂F] | D-3 | [4-S(O)CH₂CH₂Cl] |
| D-3 | [4-S(O)CH₂CHF₂] | D-3 | [4-S(O)CH₂CH₂CF₃] |
| D-3 | [4-S(O)CH₂CF₂CHF₂] | D-3 | [4-S(O)CH₂CF₂CF₃] |
| D-3 | [4-S(O)CH₂CH=CF₂] | D-3 | [4-S(O)CH₂CH=CCl₂] |
| D-3 | [4-S(O)CH₂CH₂CF=CF₂] | D-3 | [4-S(O)CH₂CH₂CH=CF₂] |
| D-3 | [4-S(O)CH₂C≡CCl] | D-3 | [4-S(O)CH₂C≡CBr] |
| D-3 | [4-S(O)CH₂CH₂OMe] | D-3 | [4-S(O)CH₂CH₂SMe] |
| D-3 | [4-S(O)CH₂CH₂S(O)Me] | D-3 | [4-S(O)CH₂CH₂S(O)₂Me] |
| D-3 | [4-S(O)₂Et] | D-3 | [4-S(O)₂Pr-n] |
| D-3 | [4-S(O)₂Pr-i] | D-3 | [4-S(O)₂Bu-tert] |
| D-3 | [4-S(O)₂Et] | D-3 | [4-S(O)₂Pr-n] |
| D-3 | [4-S(O)₂Pr-i] | D-3 | [4-S(O)₂Bu-i] |
| D-3 | [4-S(O)₂Pr-c] | D-3 | [4-S(O)₂Bu-n] |
| D-3 | [4-S(O)₂Bu-i] | D-3 | [4-S(O)₂Bu-sec] |
| D-3 | [4-S(O)₂Bu-tert] | D-3 | [4-S(O)₂Bu-c] |
| D-3 | [4-S(O)₂Pen-c] | D-3 | [4-S(O)₂Hex-c] |
| D-3 | [4-S(O)₂CH₂CH=CH₂] | D-3 | [4-S(O)₂CH₂C≡CH] |
| D-3 | [4-S(O)₂CH₂CH=C(Me)₂] | D-3 | [4-S(O)₂CH₂C≡CMe] |
| D-3 | [4-S(O)₂CHF₂] | D-3 | [4-S(O)₂CF₃] |
| D-3 | [4-S(O)₂CH₂CH₂F] | D-3 | [4-S(O)₂CH₂CH₂Cl] |
| D-3 | [4-S(O)₂CH₂CHF₂] | D-3 | [4-S(O)₂CH₂CH₂CF₃] |
| D-3 | [4-S(O)₂CH₂CF₂CHF₂] | D-3 | [4-S(O)₂CH₂CF₂CF₃] |
| D-3 | [4-S(O)₂CH₂CH=CF₂] | D-3 | [4-S(O)₂CH₂CH=CCl₂] |
| D-3 | [4-S(O)₂CH₂CH₂CF=CF₂] | D-3 | [4-S(O)₂CH₂CH₂CH=CF₂] |
| D-3 | [4-S(O)₂CH₂C≡CCl] | D-3 | [4-S(O)₂CH₂C≡CBr] |
| D-3 | [4-S(O)₂CH₂CH₂OMe] | D-3 | [4-S(O)₂CH₂CH₂SMe] |
| D-3 | [4-S(O)₂CH₂CH₂S(O)Me] | D-3 | [4-S(O)₂CH₂CH₂S(O)₂Me] |
| D-4 | [2-F] | D-4 | [3-F] |
| D-4 | [2-Br] | D-4 | [3-Br] |
| D-4 | [2-Me] | D-4 | [3-Me] |
| D-4 | [2,6-F₂] | D-4 | [2,6-Cl₂] |
| D-4 | [2,6-(Me)₂] | D-4 | [2,3,5,6-F₄] |
| D-4 | [2,3,5,6-Cl₄] | D-4 | [2-N(Me)₂] |
| D-4 | [2-CHF₂] | D-4 | [2-CF₃] |
| D-4 | [2-OEt] | D-4 | [2-OPr-n] |
| D-4 | [2-OPr-i] | D-4 | [2-OBu-tert] |
| D-4 | [2-OEt] | D-4 | [2-OPr-n] |
| D-4 | [2-OPr-i] | D-4 | [2-OBu-i] |
| D-4 | [2-OPr-c] | D-4 | [2-OBu-n] |
| D-4 | [2-OBu-i] | D-4 | [2-OBu-sec] |
| D-4 | [2-OBu-tert] | D-4 | [2-OBu-c] |
| D-4 | [2-OPen-c] | D-4 | [2-OHex-c] |
| D-4 | [2-OCH₂CH=CH₂] | D-4 | [2-OCH₂C≡CH] |
| D-4 | [2-OCH₂CH=C(Me)₂] | D-4 | [2-OCH₂C≡CMe] |
| D-4 | [2-OCHF₂] | D-4 | [2-OCF₃] |
| D-4 | [2-OCH₂CH₂F] | D-4 | [2-OCH₂CH₂Cl] |
| D-4 | [2-OCH₂CHF₂] | D-4 | [2-OCH₂CH₂CF₃] |
| D-4 | [2-OCH₂CF₂CHF₂] | D-4 | [2-OCH₂CF₂CF₃] |
| D-4 | [2-OCH₂CH=CF₂] | D-4 | [2-OCH₂CH=CCl₂] |
| D-4 | [2-OCH₂CH₂CF=CF₂] | D-4 | [2-OCH₂CH₂CH=CF₂] |
| D-4 | [2-OCH₂C≡CCl] | D-4 | [2-OCH₂C≡CBr] |
| D-4 | [2-OCH₂CH₂OMe] | D-4 | [2-OCH₂CH₂SMe] |
| D-4 | [2-OCH₂CH₂S(O)Me] | D-4 | [2-OCH₂CH₂S(O)₂Me] |
| D-4 | [2-SEt] | D-4 | [2-SPr-n] |
| D-4 | [2-SPr-i] | D-4 | [2-SBu-tert] |
| D-4 | [2-SEt] | D-4 | [2-SPr-n] |
| D-4 | [2-SPr-i] | D-4 | [2-SBu-i] |
| D-4 | [2-SPr-c] | D-4 | [2-SBu-n] |
| D-4 | [2-SBu-i] | D-4 | [2-SBu-sec] |
| D-4 | [2-SBu-tert] | D-4 | [2-SBu-c] |
| D-4 | [2-SPen-c] | D-4 | [2-SHex-c] |
| D-4 | [2-SCH₂CH=CH₂] | D-4 | [2-SCH₂C≡CH] |
| D-4 | [2-SCH₂CH=C(Me)₂] | D-4 | [2-SCH₂C≡CMe] |
| D-4 | [2-SCHF₂] | D-4 | [2-SCF₃] |
| D-4 | [2-SCH₂CH₂F] | D-4 | [2-SCH₂CH₂Cl] |
| D-4 | [2-SCH₂CHF₂] | D-4 | [2-SCH₂CH₂CF₃] |
| D-4 | [2-SCH₂CF₂CHF₂] | D-4 | [2-SCH₂CF₂CF₃] |
| D-4 | [2-SCH₂CH=CF₂] | D-4 | [2-SCH₂CH=CCl₂] |
| D-4 | [2-SCH₂CH₂CF=CF₂] | D-4 | [2-SCH₂CH₂CH=CF₂] |
| D-4 | [2-SCH₂C≡CCl] | D-4 | [2-SCH₂C≡CBr] |
| D-4 | [2-SCH₂CH₂OMe] | D-4 | [2-SCH₂CH₂SMe] |
| D-4 | [2-SCH₂CH₂S(O)Me] | D-4 | [2-SCH₂CH₂S(O)₂Me] |
| D-4 | [2-S(O)Et] | D-4 | [2-S(O)Pr-n] |
| D-4 | [2-S(O)Pr-i] | D-4 | [2-S(O)Bu-tert] |
| D-4 | [2-S(O)Et] | D-4 | [2-S(O)Pr-n] |
| D-4 | [2-S(O)Pr-i] | D-4 | [2-S(O)Bu-i] |
| D-4 | [2-S(O)Pr-c] | D-4 | [2-S(O)Bu-n] |
| D-4 | [2-S(O)Bu-i] | D-4 | [2-S(O)Bu-sec] |
| D-4 | [2-S(O)Bu-tert] | D-4 | [2-S(O)Bu-c] |
| D-4 | [2-S(O)Pen-c] | D-4 | [2-S(O)Hex-c] |
| D-4 | [2-S(O)CH₂CH=CH₂] | D-4 | [2-S(O)CH₂C≡CH] |
| D-4 | [2-S(O)CH₂CH=C(Me)₂] | D-4 | [2-S(O)CH₂C≡CMe] |
| D-4 | [2-S(O)CHF₂] | D-4 | [2-S(O)CF₃] |
| D-4 | [2-S(O)CH₂CH₂F] | D-4 | [2-S(O)CH₂CH₂Cl] |
| D-4 | [2-S(O)CH₂CHF₂] | D-4 | [2-S(O)CH₂CH₂CF₃] |
| D-4 | [2-S(O)CH₂CF₂CHF₂] | D-4 | [2-S(O)CH₂CF₂CF₃] |
| D-4 | [2-S(O)CH₂CH=CF₂] | D-4 | [2-S(O)CH₂CH=CCl₂] |
| D-4 | [2-S(O)CH₂CH₂CF=CF₂] | D-4 | [2-S(O)CH₂CH₂CH=CF₂] |
| D-4 | [2-S(O)CH₂C≡CCl] | D-4 | [2-S(O)CH₂C≡CBr] |
| D-4 | [2-S(O)CH₂CH₂OMe] | D-4 | [2-S(O)CH₂CH₂SMe] |
| D-4 | [2-S(O)CH₂CH₂S(O)Me] | D-4 | [2-S(O)CH₂CH₂S(O)₂Me] |
| D-4 | [2-S(O)₂Et] | D-4 | [2-S(O)₂Pr-n] |
| D-4 | [2-S(O)₂Pr-i] | D-4 | [2-S(O)₂Bu-tert] |
| D-4 | [2-S(O)₂Et] | D-4 | [2-S(O)₂Pr-n] |
| D-4 | [2-S(O)₂Pr-i] | D-4 | [2-S(O)₂Bu-i] |
| D-4 | [2-S(O)₂Pr-c] | D-4 | [2-S(O)₂Bu-n] |
| D-4 | [2-S(O)₂Bu-i] | D-4 | [2-S(O)₂Bu-sec] |
| D-4 | [2-S(O)₂Bu-tert] | D-4 | [2-S(O)₂Bu-c] |
| D-4 | [2-S(O)₂Pen-c] | D-4 | [2-S(O)₂Hex-c] |
| D-4 | [2-S(O)₂CH₂CH=CH₂] | D-4 | [2-S(O)₂CH₂C≡CH] |
| D-4 | [2-S(O)₂CH₂CH=C(Me)₂] | D-4 | [2-S(O)₂CH₂C≡CMe] |
| D-4 | [2-S(O)₂CHF₂] | D-4 | [2-S(O)₂CF₃] |
| D-4 | [2-S(O)₂CH₂CH₂F] | D-4 | [2-S(O)₂CH₂CH₂Cl] |
| D-4 | [2-S(O)₂CH₂CHF₂] | D-4 | [2-S(O)₂CH₂CH₂CF₃] |
| D-4 | [2-S(O)₂CH₂CF₂CHF₂] | D-4 | [2-S(O)₂CH₂CF₂CF₃] |
| D-4 | [2-S(O)₂CH₂CH=CF₂] | D-4 | [2-S(O)₂CH₂CH=CCl₂] |
| D-4 | [2-S(O)₂CH₂CH₂CF=CF₂] | D-4 | [2-S(O)₂CH₂CH₂CH=CF₂] |
| D-4 | [2-S(O)₂CH₂C≡CCl] | D-4 | [2-S(O)₂CH₂C≡CBr] |
| D-4 | [2-S(O)₂CH₂CH₂OMe] | D-4 | [2-S(O)₂CH₂CH₂SMe] |
| D-4 | [2-S(O)₂CH₂CH₂S(O)Me] | D-4 | [2-S(O)₂CH₂CH₂S(O)₂Me] |
| D-4 | [3-OEt] | D-4 | [3-OPr-n] |
| D-4 | [3-OPr-i] | D-4 | [3-OBu-tert] |
| D-4 | [3-OEt] | D-4 | [3-OPr-n] |
| D-4 | [3-OPr-i] | D-4 | [3-OBu-i] |
| D-4 | [3-OPr-c] | D-4 | [3-OBu-n] |
| D-4 | [3-OBu-i] | D-4 | [3-OBu-sec] |
| D-4 | [3-OBu-tert] | D-4 | [3-OBu-c] |
| D-4 | [3-OPen-c] | D-4 | [3-OHex-c] |
| D-4 | [3-OCH₂CH=CH₂] | D-4 | [3-OCH₂C≡CH] |
| D-4 | [3-OCH₂CH=C(Me)₂] | D-4 | [3-OCH₂C≡CMe] |
| D-4 | [3-OCHF₂] | D-4 | [3-OCF₃] |
| D-4 | [3-OCH₂CH₂F] | D-4 | [3-OCH₂CH₂Cl] |
| D-4 | [3-OCH₂CHF₂] | D-4 | [3-OCH₂CH₂CF₃] |
| D-4 | [3-OCH₂CF₂CHF₂] | D-4 | [3-OCH₂CF₂CF₃] |
| D-4 | [3-OCH₂CH=CF₂] | D-4 | [3-OCH₂CH=CCl₂] |
| D-4 | [3-OCH₂CH₂CF=CF₂] | D-4 | [3-OCH₂CH₂CH=CF₂] |
| D-4 | [3-OCH₂C≡CCl] | D-4 | [3-OCH₂C≡CBr] |
| D-4 | [3-OCH₂CH₂OMe] | D-4 | [3-OCH₂CH₂SMe] |
| D-4 | [3-OCH₂CH₂S(O)Me] | D-4 | [3-OCH₂CH₂S(O)₂Me] |
| D-4 | [3-SEt] | D-4 | [3-SPr-n] |
| D-4 | [3-SPr-i] | D-4 | [3-SBu-tert] |
| D-4 | [3-SEt] | D-4 | [3-SPr-n] |
| D-4 | [3-SPr-i] | D-4 | [3-SBu-i] |
| D-4 | [3-SPr-c] | D-4 | [3-SBu-n] |
| D-4 | [3-SBu-i] | D-4 | [3-SBu-sec] |
| D-4 | [3-SBu-tert] | D-4 | [3-SBu-c] |
| D-4 | [3-SPen-c] | D-4 | [3-SHex-c] |
| D-4 | [3-SCH₂CH=CH₂] | D-4 | [3-SCH₂C≡CH] |
| D-4 | [3-SCH₂CH=C(Me)₂] | D-4 | [3-SCH₂C≡CMe] |
| D-4 | [3-SCHF₂] | D-4 | [3-SCF₃] |
| D-4 | [3-SCH₂CH₂F] | D-4 | [3-SCH₂CH₂Cl] |
| D-4 | [3-SCH₂CHF₂] | D-4 | [3-SCH₂CH₂CF₃] |
| D-4 | [3-SCH₂CF₂CHF₂] | D-4 | [3-SCH₂CF₂CF₃] |
| D-4 | [3-SCH₂CH=CF₂] | D-4 | [3-SCH₂CH=CCl₂] |
| D-4 | [3-SCH₂CH₂CF=CF₂] | D-4 | [3-SCH₂CH₂CH=CF₂] |
| D-4 | [3-SCH₂C≡CCl] | D-4 | [3-SCH₂C≡CBr] |
| D-4 | [3-SCH₂CH₂OMe] | D-4 | [3-SCH₂CH₂SMe] |
| D-4 | [3-SCH₂CH₂S(O)Me] | D-4 | [3-SCH₂CH₂S(O)₂Me] |
| D-4 | [3-S(O)Et] | D-4 | [3-S(O)Pr-n] |
| D-4 | [3-S(O)Pr-i] | D-4 | [3-S(O)Bu-tert] |
| D-4 | [3-S(O)Et] | D-4 | [3-S(O)Pr-n] |
| D-4 | [3-S(O)Pr-i] | D-4 | [3-S(O)Bu-i] |
| D-4 | [3-S(O)Pr-c] | D-4 | [3-S(O)Bu-n] |
| D-4 | [3-S(O)Bu-i] | D-4 | [3-S(O)Bu-sec] |
| D-4 | [3-S(O)Bu-tert] | D-4 | [3-S(O)Bu-c] |
| D-4 | [3-S(O)Pen-c] | D-4 | [3-S(O)Hex-c] |
| D-4 | [3-S(O)CH₂CH=CH₂] | D-4 | [3-S(O)CH₂C≡CH] |
| D-4 | [3-S(O)CH₂CH=C(Me)₂] | D-4 | [3-S(O)CH₂C≡CMe] |
| D-4 | [3-S(O)CHF₂] | D-4 | [3-S(O)CF₃] |
| D-4 | [3-S(O)CH₂CH₂F] | D-4 | [3-S(O)CH₂CH₂Cl] |
| D-4 | [3-S(O)CH₂CHF₂] | D-4 | [3-S(O)CH₂CH₂CF₃] |
| D-4 | [3-S(O)CH₂CF₂CHF₂] | D-4 | [3-S(O)CH₂CF₂CF₃] |
| D-4 | [3-S(O)CH₂CH=CF₂] | D-4 | [3-S(O)CH₂CH=CCl₂] |
| D-4 | [3-S(O)CH₂CH₂CF=CF₂] | D-4 | [3-S(O)CH₂CH₂CH=CF₂] |
| D-4 | [3-S(O)CH₂C≡CCl] | D-4 | [3-S(O)CH₂C≡CBr] |
| D-4 | [3-S(O)CH₂CH₂OMe] | D-4 | [3-S(O)CH₂CH₂SMe] |
| D-4 | [3-S(O)CH₂CH₂S(O)Me] | D-4 | [3-S(O)CH₂CH₂S(O)₂Me] |
| D-4 | [3-S(O)₂Et] | D-4 | [3-S(O)₂Pr-n] |
| D-4 | [3-S(O)₂Pr-i] | D-4 | [3-S(O)₂Bu-tert] |
| D-4 | [3-S(O)₂Et] | D-4 | [3-S(O)₂Pr-n] |
| D-4 | [3-S(O)₂Pr-i] | D-4 | [3-S(O)₂Bu-i] |
| D-4 | [3-S(O)₂Pr-c] | D-4 | [3-S(O)₂Bu-n] |
| D-4 | [3-S(O)₂Bu-i] | D-4 | [3-S(O)₂Bu-sec] |
| D-4 | [3-S(O)₂Bu-tert] | D-4 | [3-S(O)₂Bu-c] |
| D-4 | [3-S(O)₂Pen-c] | D-4 | [3-S(O)₂Hex-c] |
| D-4 | [3-S(O)₂CH₂CH=CH₂] | D-4 | [3-S(O)₂CH₂C≡CH] |
| D-4 | [3-S(O)₂CH₂CH=C(Me)₂] | D-4 | [3-S(O)₂CH₂C≡CMe] |
| D-4 | [3-S(O)₂CHF₂] | D-4 | [3-S(O)₂CF₃] |
| D-4 | [3-S(O)₂CH₂CH₂F] | D-4 | [3-S(O)₂CH₂CH₂Cl] |
| D-4 | [3-S(O)₂CH₂CHF₂] | D-4 | [3-S(O)₂CH₂CH₂CF₃] |
| D-4 | [3-S(O)₂CH₂CF₂CHF₂] | D-4 | [3-S(O)₂CH₂CF₂CF₃] |
| D-4 | [3-S(O)₂CH₂CH=CF₂] | D-4 | [3-S(O)₂CH₂CH=CCl₂] |
| D-4 | [3-S(O)₂CH₂CH₂CF=CF₂] | D-4 | [3-S(O)₂CH₂CH₂CH=CF₂] |
| D-4 | [3-S(O)₂CH₂C≡CCl] | D-4 | [3-S(O)₂CH₂C≡CBr] |
| D-4 | [3-S(O)₂CH₂CH₂OMe] | D-4 | [3-S(O)₂CH₂CH₂SMe] |
| D-4 | [3-S(O)₂CH₂CH₂S(O)Me] | D-4 | [3-S(O)₂CH₂CH₂S(O)₂Me] |
| D-5 | [-] | D-5 | [3-Cl] |
| D-5 | [4-Cl] | D-5 | [5-Cl] |
| D-5 | [3-Br] | D-5 | [4-Br] |
| D-5 | [5-Br] | D-5 | [3-OMe] |
| D-5 | [4-OMe] | D-5 | [5-OMe] |
| D-5 | [3-Me] | D-5 | [4-Me] |
| D-5 | [5-Me] | D-5 | [5-Pr-c] |
| D-5 | [5-CF₃] | D-5 | [5-Pr-c] |
| D-6 | [-] | D-6 | [2-Cl] |
| D-6 | [4-Cl] | D-6 | [5-Cl] |
| D-6 | [2-Br] | D-6 | [4-Br] |
| D-6 | [5-Br] | D-6 | [2-OMe] |
| D-6 | [4-OMe] | D-6 | [5-OMe] |
| D-7 | [-] | D-7 | [3-Cl] |
| D-7 | [4-Cl] | D-7 | [5-Cl] |
| D-7 | [3-Br] | D-7 | [4-Br] |
| D-7 | [5-Br] | D-7 | [3-OMe] |
| D-7 | [4-OMe] | D-7 | [5-OMe] |
| D-7 | [3-CF₃] | D-7 | [5-CF₃] |
| D-7 | [5-SMe] | D-7 | [5-SEt] |
| D-7 | [5-S(O)Me] | D-7 | [5-S(O)Et] |
| D-7 | [5-S(O)₂Me] | D-7 | [5-S(O)₂Et] |
| D-7 | [5-SPr-n] | D-7 | [5-SEt] |
| D-7 | [5-S(O)Pr-n] | D-7 | [5-S(O)Et] |
| D-7 | [5-S(O)₂Pr-n] | D-7 | [5-S(O)₂Et] |
| D-7 | [5-SPr-n] | D-7 | [5-SPr-i] |
| D-7 | [5-S(O)Pr-n] | D-7 | [5-S(O)Pr-i] |
| D-7 | [5-S(O)₂Pr-n] | D-7 | [5-S(O)₂Pr-i] |
| D-8 | [-] | D-8 | [2-Cl] |
| D-8 | [4-Cl] | D-8 | [5-Cl] |
| D-8 | [2-Br] | D-8 | [4-Br] |
| D-8 | [5-Br] | D-8 | [2-OMe] |
| D-8 | [4-OMe] | D-8 | [5-OMe] |
| D-9 | [-] | D-9 | [3-Cl] |
| D-9 | [3-Br] | D-9 | [3-I] |
| D-9 | [3-CN] | D-9 | [3-CF₃] |
| D-10 | [-] | D-10 | [3-Cl] |
| D-10 | [3-Br] | D-10 | [3-I] |
| D-10 | [3-CN] | D-10 | [3-CF₃] |
| D-11 | [-] | D-12 | [-] |
| D-13 | [-] | D-13 | [2-Cl] |
| D-13 | [3-Cl] | D-13 | [2-Br] |
| D-13 | [3-Br] | D-13 | [2-I] |
| D-13 | [3-I] | D-13 | [2-Me] |
| D-13 | [3-Me] | D-13 | [2-OMe] |
| D-13 | [3-OMe] | D-13 | [2-SMe] |
| D-13 | [3-SMe] | D-13 | [2-S(O)Me] |
| D-13 | [3-S(O)Me] | D-13 | [2-S(O)₂Me] |
| D-13 | [3-S(O)₂Me] | D-13 | [2-CN] |
| D-13 | [3-CN] | D-13 | [2-CF₃] |
| D-13 | [3-CF₃] | D-13 | [2,3-Cl₂] |
| D-14 | [-] | D-14 | [2-Cl] |
| D-14 | [3-Cl] | D-14 | [2-Br] |
| D-14 | [3-Br] | D-14 | [2-I] |
| D-14 | [3-I] | D-14 | [2-Me] |
| D-14 | [3-Me] | D-14 | [2-OMe] |
| D-14 | [3-OMe] | D-14 | [2-SMe] |
| D-14 | [3-SMe] | D-14 | [2-S(O)Me] |
| D-14 | [3-S(O)Me] | D-14 | [2-S(O)₂Me] |
| D-14 | [3-S(O)₂Me] | D-14 | [2-CN] |
| D-14 | [3-CN] | D-14 | [2-CF₃] |
| D-14 | [3-CF₃] | D-14 | [2,3-Cl₂] |
| D-15 | [-] | D-15 | [2-Cl] |
| D-15 | [3-Cl] | D-15 | [2-Br] |
| D-15 | [3-Br] | D-15 | [2-I] |
| D-15 | [3-I] | D-15 | [2-Me] |
| D-15 | [3-Me] | D-15 | [2-OMe] |
| D-15 | [3-OMe] | D-15 | [2-SMe] |
| D-15 | [3-SMe] | D-15 | [2-S(O)Me] |
| D-15 | [3-S(O)Me] | D-15 | [2-S(O)₂Me] |
| D-15 | [3-S(O)₂Me] | D-15 | [2-CN] |
| D-15 | [3-CN] | D-15 | [2-CF₃] |
| D-15 | [3-CF₃] | D-15 | [2,3-Cl₂] |
| D-16 | [-] | D-16 | [2-Cl] |
| D-16 | [3-Cl] | D-16 | [2-Br] |
| D-16 | [3-Br] | D-16 | [2-I] |
| D-16 | [3-I] | D-16 | [2-Me] |
| D-16 | [3-Me] | D-16 | [2-OMe] |
| D-16 | [3-OMe] | D-16 | [2-SMe] |
| D-16 | [3-SMe] | D-16 | [2-S(O)Me] |
| D-16 | [3-S(O)Me] | D-16 | [2-S(O)₂Me] |
| D-16 | [3-S(O)₂Me] | D-16 | [2-CN] |
| D-16 | [3-CN] | D-16 | [2-CF₃] |
| D-16 | [3-CF₃] | D-16 | [2,3-Cl₂] |
| D-17 | [-] | | |
| D-18 | [-] | D-18 | [6-F] |
| D-18 | [6-Cl] | D-18 | [6-Me] |
| D-18 | [6-CF₃] | D-18 | [6-OMe] |
| D-18 | [6-OCHF₂] | D-18 | [6-OCF₃] |
| D-18 | [6-SMe] | D-18 | [6-S(O)Me] |
| D-18 | [6-S(O)₂Me] | D-18 | [6-SCHF₂] |
| D-18 | [6-S(O)CHF₂] | D-18 | [6-S(O)₂CHF₂] |
| D-18 | [6-SCF₃] | D-18 | [6-S(O)CF₃] |
| D-18 | [6-S(O)₂CF₃] | D-18 | [6-CN] |
| D-18 | [6,8-F2] | D-18 | [6,8-Cl₂] |
| D-18 | [6-Cl-8-Me] | D-18 | [8-CF₃] |
| D-19 | [-] | D-20 | [-] |
| D-21 | [-] | D-22 | [-] |
| D-23 | [-] | D-24 | [-] |
| D-25 | [-] | D-25 | [4-F] |
| D-25 | [4-Cl] | D-25 | [4-Br] |
| D-25 | [4-I] | D-25 | [4-Me] |
| D-26 | [1-Me-4-OMe] | D-26 | [1-Me-4-Cl] |
| D-26 | [1-Me-4-Br] | D-26 | [1-Me-5-Cl] |
| D-26 | [1-Me-4-Br] | D-26 | [1-Me-5-CF₃] |
| D-26 | [1-Me-5-SMe] | D-26 | [1-Me-5-S(O)Me] |
| D-26 | [1-Me-5-S(O)₂Me] | D-26 | [1-Me] |
| D-27 | [1-Me] | D-27 | [1-Me-3-Cl] |
| D-27 | [1-Me-3-CF₃] | D-27 | [1-Me-5-CF₃] |
| D-27 | [1-CH₂CH₂OMe] | D-27 | [1-CH₂CH₂SMe] |
| D-27 | [1-CH₂CH₂S(O)Me] | D-27 | [1-CH₂CH₂S(O)₂Me] |
| D-28 | [-] | D-28 | [2-Cl] |
| D-28 | [2-OMe] | D-28 | [2-OEt] |
| D-28 | [2-OPr-n] | D-28 | [2-OPr-i] |
| D-28 | [2-SMe] | D-28 | [2-SEt] |
| D-28 | [2-SPr-n] | D-28 | [2-SPr-i] |
| D-28 | [2-S(O)Me] | D-28 | [2-S(O)Et] |
| D-28 | [2-S(O)Pr-n] | D-28 | [2-S(O)Pr-i] |
| D-28 | [2-S(O)₂Me] | D-28 | [2-S(O)₂Et] |
| D-28 | [2-S(O)₂Pr-n] | D-28 | [2-S(O)₂Pr-i] |
| D-29 | [-] | D-30 | [-] |
| D-31 | [-] | D-32 | [-] |
| D-33 | [1-Me] | D-34 | [1-Me] |
| D-35 | [1-Me] | D-36 | [1-Me] |
| D-37 | [1-Me] | D-38 | [1-Me] |
| D-39 | [2-Me] | D-40 | [2-Me] |
| D-41 | [-] | D-42 | [-] |
| D-43 | [-] | D-44 | [-] |
| D-45 | [-] | D-46 | [-] |
| D-47 | [-] | D-48 | [-] |
| D-49 | [-] | D-49 | [-] |
| D-51 | [-] | D-52 | [-] |
| | | | |

Table 1-2 has the same structure as Table 1, except that the condition of Table 1 (i.e., R¹ is Me, R² is Me, W¹ is O, G is H, X is S, and (Z¹) is "-" (no substitution)) is replaced with the following condition (i.e., R¹ is Me, R² is Me, W¹ is O, G is Me, X is O, and (Z¹)ₙ is "-" (no substitution)).

Specifically, the "condition" described in Table 1-2 of Table 2 corresponds to a compound of Formula (1) wherein R¹ is methyl, R² is methyl, W¹ is an oxygen atom, G is methyl, X is O, (Z¹) is "-" (i.e., n is 0, and the substituent Z¹ is not present), R³ is D-1, and (Y¹) is "-" (no substitution). The same shall apply to Tables 1-3 to 1-1342.

**Table 2**

| Table | Condition |
|---|---|
| Table 1-2 | R¹ is Me, R² is Me, W¹ is O, G is H, X is O, and (Z¹)ₙ is "-". |
| Table 1-3 | R¹ is Me, R² is Me, W¹ is O, G is H, X is S, and (Z¹)ₙ is 4-F. |
| Table 1-4 | R¹ is Me, R² is Me, W¹ is O, G is H, X is O, and (Z¹)ₙ is 4-F. |
| Table 1-5 | R¹ is Me, R² is Me, W¹ is O, G is H, X is S, and (Z¹)ₙ is 5-F. |
| Table 1-6 | R¹ is Me, R² is Me, W¹ is O, G is H, X is O, and (Z¹)ₙ is 5-F. |
| Table 1-7 | R¹ is Me, R² is Me, W¹ is O, G is H, X is S, and (Z¹)ₙ is 6-F. |
| Table 1-8 | R¹ is Me, R² is Me, W¹ is O, G is H, X is O, and (Z¹)ₙ is 6-F. |
| Table 1-9 | R¹ is Me, R² is Me, W¹ is O, G is H, X is S, and (Z¹)ₙ is 7-F. |
| Table 1-10 | R¹ is Me, R² is Me, W¹ is O, G is H, X is O, and (Z¹)ₙ is 7-F. |
| Table 1-11 | R¹ is Me, R² is Me, W¹ is O, G is H, X is S, and (Z¹)ₙ is 4-Cl. |
| Table 1-12 | R¹ is Me, R² is Me, W¹ is O, G is H, X is O, and (Z¹)ₙ is 4-Cl. |
| Table 1-13 | R¹ is Me, R² is Me, W¹ is O, G is H, X is S, and (Z¹)ₙ is 5-Cl. |
| Table 1-14 | R¹ is Me, R² is Me, W¹ is O, G is H, X is O, and (Z¹)ₙ is 5-Cl. |
| Table 1-15 | R¹ is Me, R² is Me, W¹ is O, G is H, X is S, and (Z¹)ₙ is 6-Cl. |
| Table 1-16 | R¹ is Me, R² is Me, W¹ is O, G is H, X is O, and (Z¹)ₙ is 6-Cl. |
| Table 1-17 | R¹ is Me, R² is Me, W¹ is O, G is H, X is S, and (Z¹)ₙ is 7-Cl. |
| Table 1-18 | R¹ is Me, R² is Me, W¹ is O, G is H, X is O, and (Z¹)ₙ is 7-Cl. |
| Table 1-19 | R¹ is Me, R² is Me, W¹ is O, G is H, X is S, and (Z¹)ₙ is 4-Me. |
| Table 1-20 | R¹ is Me, R² is Me, W¹ is O, G is H, X is O, and (Z¹)ₙ is 4-Me. |
| Table 1-21 | R¹ is Me, R² is Me, W¹ is O, G is H, X is S, and (Z¹)ₙ is 5-Me. |
| Table 1-22 | R¹ is Me, R² is Me, W¹ is O, G is H, X is O, and (Z¹)ₙ is 5-Me. |
| Table 1-23 | R¹ is Me, R² is Me, W¹ is O, G is H, X is S, and (Z¹)ₙ is 6-Me. |
| Table 1-24 | R¹ is Me, R² is Me, W¹ is O, G is H, X is O, and (Z¹)ₙ is 6-Me. |
| Table 1-25 | R¹ is Me, R² is Me, W¹ is O, G is H, X is S, and (Z¹)ₙ is 7-Me. |
| Table 1-26 | R¹ is Me, R² is Me, W¹ is O, G is H, X is O, and (Z¹)ₙ is 7-Me. |
| Table 1-27 | R¹ is Me, R² is Me, W¹ is O, G is H, X is S, and (Z¹)ₙ is 4-OMe. |
| Table 1-28 | R¹ is Me, R² is Me, W¹ is O, G is H, X is O, and (Z¹)ₙ is 4-OMe. |
| Table 1-29 | R¹ is Me, R² is Me, W¹ is O, G is H, X is S, and (Z¹)ₙ is 5-OMe. |
| Table 1-30 | R¹ is Me, R² is Me, W¹ is O, G is H, X is O, and (Z¹)ₙ is 5-OMe. |
| Table 1-31 | R¹ is Me, R² is Me, W¹ is O, G is H, X is S, and (Z¹)ₙ is 6-OMe. |
| Table 1-32 | R¹ is Me, R² is Me, W¹ is O, G is H, X is O, and (Z¹)ₙ is 6-OMe. |
| Table 1-33 | R¹ is Me, R² is Me, W¹ is O, G is H, X is S, and (Z¹)ₙ is 7-OMe. |
| Table 1-34 | R¹ is Me, R² is Me, W¹ is O, G is H, X is O, and (Z¹)ₙ is 7-OMe. |
| Table 1-35 | R¹ is Me, R² is H, W¹ is O, G is H, X is S, and (Z¹)ₙ is "-". |
| Table 1-36 | R¹ is Me, R² is H, W¹ is O, G is H, X is O, and (Z¹)ₙ is "-". |
| Table 1-37 | R¹ is Me, R² is H, W¹ is O, G is H, X is S, and (Z¹)ₙ is 4-F. |
| Table 1-38 | R¹ is Me, R² is H, W¹ is O, G is H, X is O, and (Z¹)ₙ is 4-F. |
| Table 1-39 | R¹ is Me, R² is H, W¹ is O, G is H, X is S, and (Z¹)ₙ is 5-F. |
| Table 1-40 | R¹ is Me, R² is H, W¹ is O, G is H, X is O, and (Z¹)ₙ is 5-F. |
| Table 1-41 | R¹ is Me, R² is H, W¹ is O, G is H, X is S, and (Z¹)ₙ is 6-F. |
| Table 1-42 | R¹ is Me, R² is H, W¹ is O, G is H, X is O, and (Z¹)ₙ is 6-F. |
| Table 1-43 | R¹ is Me, R² is H, W¹ is O, G is H, X is S, and (Z¹)ₙ is 7-F. |
| Table 1-44 | R¹ is Me, R² is H, W¹ is O, G is H, X is O, and (Z¹)ₙ is 7-F. |
| Table 1-45 | R¹ is Me, R² is H, W¹ is O, G is H, X is S, and (Z¹)ₙ is 4-Cl. |
| Table 1-46 | R¹ is Me, R² is H, W¹ is O, G is H, X is O, and (Z¹)ₙ is 4-Cl. |
| Table 1-47 | R¹ is Me, R² is H, W¹ is O, G is H, X is S, and (Z¹)ₙ is 5-Cl. |
| Table 1-48 | R¹ is Me, R² is H, W¹ is O, G is H, X is O, and (Z¹)ₙ is 5-Cl. |
| Table 1-49 | R¹ is Me, R² is H, W¹ is O, G is H, X is S, and (Z¹)ₙ is 6-Cl. |
| Table 1-50 | R¹ is Me, R² is H, W¹ is O, G is H, X is O, and (Z¹)ₙ is 6-Cl. |
| Table 1-51 | R¹ is Me, R² is H, W¹ is O, G is H, X is S, and (Z¹)ₙ is 7-Cl. |
| Table 1-52 | R¹ is Me, R² is H, W¹ is O, G is H, X is O, and (Z¹)ₙ is 7-Cl. |
| Table 1-53 | R¹ is Me, R² is H, W¹ is O, G is H, X is S, and (Z¹)ₙ is 4-Me. |
| Table 1-54 | R¹ is Me, R² is H, W¹ is O, G is H, X is O, and (Z¹)ₙ is 4-Me. |
| Table 1-55 | R¹ is Me, R² is H, W¹ is O, G is H, X is S, and (Z¹)ₙ is 5-Me. |
| Table 1-56 | R¹ is Me, R² is H, W¹ is O, G is H, X is O, and (Z¹)ₙ is 5-Me. |
| Table 1-57 | R¹ is Me, R² is H, W¹ is O, G is H, X is S, and (Z¹)ₙ is 6-Me. |
| Table 1-58 | R¹ is Me, R² is H, W¹ is O, G is H, X is O, and (Z¹)ₙ is 6-Me. |
| Table 1-59 | R¹ is Me, R² is H, W¹ is O, G is H, X is S, and (Z¹)ₙ is 7-Me. |
| Table 1-60 | R¹ is Me, R² is H, W¹ is O, G is H, X is O, and (Z¹)ₙ is 7-Me. |
| Table 1-61 | R¹ is Me, R² is H, W¹ is O, G is H, X is S, and (Z¹)ₙ is 4-OMe. |
| Table 1-62 | R¹ is Me, R² is H, W¹ is O, G is H, X is O, and (Z¹)ₙ is 4-OMe |
| Table 1-63 | R¹ is Me, R² is H, W¹ is O, G is H, X is S, and (Z¹)ₙ is 5-OMe. |
| Table 1-64 | R¹ is Me, R² is H, W¹ is O, G is H, X is O, and (Z¹)ₙ is 5-OMe |
| Table 1-65 | R¹ is Me, R² is H, W¹ is O, G is H, X is S, and (Z¹)ₙ is 6-OMe. |
| Table 1-66 | R¹ is Me, R² is H, W¹ is O, G is H, X is O, and (Z¹)ₙ is 6-OMe |
| Table 1-67 | R¹ is Me, R² is H, W¹ is O, G is H, X is S, and (Z¹)ₙ is 7-OMe. |
| Table 1-68 | R¹ is Me, R² is H, W¹ is O, G is H, X is O, and (Z¹)ₙ is 7-OMe |
| Table 1-69 | R¹ is Me, R² is Cl, W¹ is O, G is H, X is S, and (Z¹)ₙ is "-". |
| Table 1-70 | R¹ is Me, R² is Cl, W¹ is O, G is H, X is O, and (Z¹)ₙ is "-". |
| Table 1-71 | R¹ is Me, R² is Cl, W¹ is O, G is H, X is O, and (Z¹)ₙ is 4-F. |
| Table 1-72 | R¹ is Me, R² is Cl, W¹ is O, G is H, X is O, and (Z¹)ₙ is 4-F. |
| Table 1-73 | R¹ is Me, R² is Cl, W¹ is O, G is H, X is S, and (Z¹)ₙ is 5-F. |
| Table 1-74 | R¹ is Me, R² is Cl, W¹ is O, G is H, X is O, and (Z¹)ₙ is 5-F. |
| Table 1-75 | R¹ is Me, R² is Cl, W¹ is O, G is H, X is S, and (Z¹)ₙ is 6-F. |
| Table 1-76 | R¹ is Me, R² is Cl, W¹ is O, G is H, X is O, and (Z¹)ₙ is 6-F. |
| Table 1-77 | R¹ is Me, R² is Cl, W¹ is O, G is H, X is S, and (Z¹)ₙ is 7-F. |
| Table 1-78 | R¹ is Me, R² is Cl, W¹ is O, G is H, X is O, and (Z¹)ₙ is 7-F. |
| Table 1-79 | R¹ is Me, R² is Cl, W¹ is O, G is H, X is S, and (Z¹)ₙ is 4-Cl. |
| Table 1-80 | R¹ is Me, R² is Cl, W¹ is O, G is H, X is O, and (Z¹)ₙ is 4-Cl. |
| Table 1-81 | R¹ is Me, R² is Cl, W¹ is O, G is H, X is S, and (Z¹)ₙ is 5-Cl. |
| Table 1-82 | R¹ is Me, R² is Cl, W¹ is O, G is H, X is O, and (Z¹)ₙ is 5-Cl. |
| Table 1-83 | R¹ is Me, R² is Cl, W¹ is O, G is H, X is S, and (Z¹)ₙ is 6-Cl. |
| Table 1-84 | R¹ is Me, R² is Cl, W¹ is O, G is H, X is O, and (Z¹)ₙ is 6-Cl. |
| Table 1-85 | R¹ is Me, R² is Cl, W¹ is O, G is H, X is S, and (Z¹)ₙ is 7-Cl. |
| Table 1-86 | R¹ is Me, R² is Cl, W¹ is O, G is H, X is O, and (Z¹)ₙ is 7-Cl. |
| Table 1-87 | R¹ is Me, R² is Cl, W¹ is O, G is H, X is S, and (Z¹)ₙ is 4-Me. |
| Table 1-88 | R¹ is Me, R² is Cl, W¹ is O, G is H, X is O, and (Z¹)ₙ is 4-Me. |
| Table 1-89 | R¹ is Me, R² is Cl, W¹ is O, G is H, X is S, and (Z¹)ₙ is 5-Me. |
| Table 1-90 | R¹ is Me, R² is Cl, W¹ is O, G is H, X is O, and (Z¹)ₙ is 5-Me. |
| Table 1-91 | R¹ is Me, R² is Cl, W¹ is O, Gis H, X is S, and (Z¹)ₙ is 6-Me. |
| Table 1-92 | R¹ is Me, R² is Cl, W¹ is O, G is H, X is O, and (Z¹)ₙ is 6-Me. |
| Table 1-93 | R¹ is Me, R² is Cl, W¹ is O, G is H, X is S, and (Z¹)ₙ is 7-Me. |
| Table 1-94 | R¹ is Me, R² is Cl, W¹ is O, G is H, X is O, and (Z¹)ₙ is 7-Me. |
| Table 1-95 | R¹ is Me, R² is Cl, W¹ is O, G is H, X is S, and (Z¹)ₙ is 4-OMe. |
| Table 1-96 | R¹ is Me, R² is Cl, W¹ is O, G is H, X is O, and (Z¹)ₙ is 4-OMe. |
| Table 1-97 | R¹ is Me, R² is Cl, W¹ is O, G is H, X is S, and (Z¹)ₙ is 5-OMe. |
| Table 1-98 | R¹ is Me, R² is Cl, W¹ is O, G is H, X is O, and (Z¹)ₙ is 5-OMe. |
| Table 1-99 | R¹ is Me, R² is Cl, W¹ is O, G is H, X is S, and (Z¹)ₙ is 6-OMe. |
| Table 1-100 | R¹ is Me, R² is Cl, W¹ is O, G is H, X is O, and (Z¹)ₙ is 6-OMe. |
| Table 1-101 | R¹ is Me, R² is Cl, W¹ is O, G is H, X is S, and (Z¹)ₙ is 7-OMe. |
| Table 1-102 | R¹ is Me, R² is Cl, W¹ is O, G is H, X is O, and (Z¹)ₙ is 7-OMe. |
| Table 1-103 | R¹ is Me, R² is Me, W¹ is O, G is Me, X is S, and (Z¹)ₙ is "-". |
| Table 1-104 | R¹ is Me, R² is Me, W¹ is O, G is Me, X is O, and (Z¹)ₙ is "-". |
| Table 1-105 | R¹ is Me, R² is Me, W¹ is O, G is Me, X is S, and (Z¹)ₙ is 5-F. |
| Table 1-106 | R¹ is Me, R² is Me, W¹ is O, G is Me, X is O, and (Z¹)ₙ is 5-F. |
| Table 1-107 | R¹ is Me, R² is Me, W¹ is O, G is Me, X is S, and (Z¹)ₙ is 6-F. |
| Table 1-108 | R¹ is Me, R² is Me, W¹ is O, G is Me, X is O, and (Z¹)ₙ is 6-F. |
| Table 1-109 | R¹ is Me, R² is Me, W¹ is O, G is Me, X is S, and (Z¹)ₙ is 7-F. |
| Table 1-110 | R¹ is Me, R² is Me, W¹ is O, G is Me, X is O, and (Z¹)ₙ is 7-F. |
| Table 1-111 | R¹ is Me, R² is Me, W¹ is O, G is Me, X is S, and (Z¹)ₙ is 5-Cl. |
| Table 1-112 | R¹ is Me, R² is Me, W¹ is O, G is Me, X is O, and (Z¹)ₙ is 5-Cl. |
| Table 1-113 | R¹ is Me, R² is Me, W¹ is O, G is Me, X is S, and (Z¹)ₙ is 6-Cl. |
| Table 1-114 | R¹ is Me, R² is Me, W¹ is O, G is Me, X is O, and (Z¹)ₙ is 6-Cl. |
| Table 1-115 | R¹ is Me, R² is Me, W¹ is O, G is Me, X is S, and (Z¹)ₙ is 7-Cl. |
| Table 1-116 | R¹ is Me, R² is Me, W¹ is O, G is Me, X is O, and (Z¹)ₙ is 7-Cl. |
| Table 1-117 | R¹ is Me, R² is Me, W¹ is O, G is Me, X is S, and (Z¹)ₙ is 5-Me. |
| Table 1-118 | R¹ is Me, R² is Me, W¹ is O, G is Me, X is O, and (Z¹)ₙ is 5-Me. |
| Table 1-119 | R¹ is Me, R² is Me, W¹ is O, G is Me, X is S, and (Z¹)ₙ is 6-Me. |
| Table 1-120 | R¹ is Me, R² is Me, W¹ is O, G is Me, X is O, and (Z¹)ₙ is 6-Me. |
| Table 1-121 | R¹ is Me, R² is Me, W¹ is O, G is Me, X is S, and (Z¹)ₙ is 7-Me. |
| Table 1-122 | R¹ is Me, R² is Me, W¹ is O, G is Me, X is O, and (Z¹)ₙ is 7-Me. |
| Table 1-123 | R¹ is Me, R² is Me, W¹ is O, G is Me, X is S, and (Z¹)ₙ is 5-OMe. |
| Table 1-124 | R¹ is Me, R² is Me, W¹ is O, G is Me, X is O, and (Z¹)ₙ is 5-OMe. |
| Table 1-125 | R¹ is Me, R² is Me, W¹ is O, G is Me, X is S, and (Z¹)ₙ is 6-OMe. |
| Table 1-126 | R¹ is Me, R² is Me, W¹ is O, G is Me, X is O, and (Z¹)ₙ is 6-OMe. |
| Table 1-127 | R¹ is Me, R² is Me, W¹ is O, G is Me, X is S, and (Z¹)ₙ is 7-OMe. |
| Table 1-128 | R¹ is Me, R² is Me, W¹ is O, G is Me, X is O, and (Z¹)ₙ is 7-OMe. |
| Table 1-129 | R¹ is Me, R² is Me, W¹ is O, G is C(O)Pr-n, X is S, and (Z¹)ₙ is "-". |
| Table 1-130 | R¹ is Me, R² is Me, W¹ is O, G is C(O)Pr-n, X is O, and (Z¹)ₙ is "-". |
| Table 1-131 | R¹ is Me, R² is Me, W¹ is O, G is C(O)Pr-n, X is S, and (Z¹)ₙ is 5-F. |
| Table 1-132 | R¹ is Me, R² is Me, W¹ is O, G is C(O)Pr-n, X is O, and (Z¹)ₙ is 5-F. |
| Table 1-133 | R¹ is Me, R² is Me, W¹ is O, G is C(O)Pr-n, X is S, and (Z¹)ₙ is 6-F. |
| Table 1-134 | R¹ is Me, R² is Me, W¹ is O, G is C(O)Pr-n, X is O, and (Z¹)ₙ is 6-F. |
| Table 1-135 | R¹ is Me, R² is Me, W¹ is O, G is C(O)Pr-n, X is S, and (Z¹)ₙ is 7-F. |
| Table 1-136 | R¹ is Me, R² is Me, W¹ is O, G is C(O)Pr-n, X is O, and (Z¹)ₙ is 7-F. |
| Table 1-137 | R¹ is Me, R² is Me, W¹ is O, G is C(O)Pr-n, X is S, and (Z¹)ₙ is 5-Cl. |
| Table 1-138 | R¹ is Me, R² is Me, W¹ is O, G is C(O)Pr-n, X is O, and (Z¹)ₙ is 5-Cl |
| Table 1-139 | R¹ is Me, R² is Me, W¹ is O, G is C(O)Pr-n, X is S, and (Z¹)ₙ is 6-Cl. |
| Table 1-140 | R¹ is Me, R² is Me, W¹ is O, G is C(O)Pr-n, X is O, and (Z¹)ₙ is 6-Cl |
| Table 1-141 | R¹ is Me, R ² is Me, W¹ is O, G is C(O)Pr-n, X is S, and (Z¹)ₙ is 7-Cl. |
| Table 1-142 | R¹ is Me, R² is Me, W¹ is O, G is C(O)Pr-n, X is O, and (Z¹)ₙ is 7-Cl. |
| Table 1-143 | R¹ is Me, R² is Me, W¹ is O, G is C(O)Pr-n, X is S, and (Z¹)ₙ is 5-Me. |
| Table 1-144 | R¹ is Me, R² is Me, W¹ is O, G is C(O)Pr-n, X is O, and (Z¹)ₙ is 5-Me. |
| Table 1-145 | R¹ is Me, R² is Me, W¹ is O, G is C(O)Pr-n, X is S, and (Z¹)ₙ is 6-Me. |
| Table 1-146 | R¹ is Me, R² is Me, W¹ is O, G is C(O)Pr-n, X is O, and (Z¹)ₙ is 6-Me. |
| Table 1-147 | R¹ is Me, R² is Me, W¹ is O, G is C(O)Pr-n, X is S, and (Z¹)ₙ is 7-Me. |
| Table 1-148 | R¹ is Me, R² is Me, W¹ is O, G is C(O)Pr-n, X is O, and (Z¹)ₙ is 7-Me. |
| Table 1-149 | R¹ is Me, R² is Me, W¹ is O, G is C(O)Pr-n, X is S, and (Z¹)ₙ is 5-OMe. |
| Table 1-150 | R¹ is Me, R² is Me, W¹ is O, G is C(O)Pr-n, X is O, and (Z¹)ₙ is 5-OMe. |
| Table 1-151 | R¹ is Me, R² is Me, W¹ is O, G is C(O)Pr-n, X is S, and (Z¹)ₙ is 6-OMe. |
| Table 1-152 | R¹ is Me, R² is Me, W¹ is O, G is C(O)Pr-n, X is O, and (Z¹)ₙ is 6-OMe. |
| Table 1-153 | R¹ is Me, R² is Me, W¹ is O, G is C(O)Pr-n, X is S, and (Z¹)ₙ is 7-OMe. |
| Table 1-154 | R¹ is Me, R² is Me, W¹ is O, G is C(O)Pr-n, X is O, and (Z¹)ₙ is 7-OMe. |
| Table 1-155 | R¹ is Me, R² is H, W¹ is O, G is C(O)Pr-n, X is S, and (Z¹)ₙ is "-". |
| Table 1-156 | R¹ is Me, R² is H, W¹ is O, G is C(O)Pr-n, X is O, and (Z¹)ₙ is "-". |
| Table 1-157 | R¹ is Me, R² is H, W¹ is O, G is C(O)Pr-n, X is S, and (Z¹)ₙ is 5-F. |
| Table 1-158 | R¹ is Me, R² is H, W¹ is O, G is C(O)Pr-n, X is O, and (Z¹)ₙ is 5-F. |
| Table 1-159 | R¹ is Me, R² is H, W¹ is O, G is C(O)Pr-n, X is S, and (Z¹)ₙ is 6-F. |
| Table 1-160 | R¹ is Me, R² is H, W¹ is O, G is C(O)Pr-n, X is O, and (Z¹)ₙ is 6-F. |
| Table 1-161 | R¹ is Me, R² is H, W¹ is O, G is C(O)Pr-n, X is S, and (Z¹)ₙ is 7-F. |
| Table 1-162 | R¹ is Me, R² is H, W¹ is O, G is C(O)Pr-n, X is O, and (Z¹)ₙ is 7-F. |
| Table 1-163 | R¹ is Me, R² is H, W¹ is O, G is C(O)Pr-n, X is S, and (Z¹)ₙ is 5-Cl. |
| Table 1-164 | R¹ is Me, R² is H, W¹ is O, G is C(O)Pr-n, X is O, and (Z¹)ₙ is 5-Cl. |
| Table 1-165 | R¹ is Me, R² is H, W¹ is O, G is C(O)Pr-n, X is S, and (Z¹)ₙ is 6-Cl. |
| Table 1-166 | R¹ is Me, R² is H, W¹ is O, G is C(O)Pr-n, X is O, and (Z¹)ₙ is 6-Cl. |
| Table 1-167 | R¹ is Me, R² is H, W¹ is O, G is C(O)Pr-n, X is S, and (Z¹)ₙ is 7-Cl. |
| Table 1-168 | R¹ is Me, R² is H, W¹ is O, G is C(O)Pr-n, X is O, and (Z¹)ₙ is 7-Cl. |
| Table 1-169 | R¹ is Me, R² is H, W¹ is O, G is C(O)Pr-n, X is S, and (Z¹)ₙ is 5-Me. |
| Table 1-170 | R¹ is Me, R² is H, W¹ is O, G is C(O)Pr-n, X is O, and (Z¹)ₙ is 5-Me. |
| Table 1-171 | R¹ is Me, R² is H, W¹ is O, G is C(O)Pr-n, X is S, and (Z¹)ₙ is 6-Me. |
| Table 1-172 | R¹ is Me, R² is H, W¹ is O, G is C(O)Pr-n, X is O, and (Z¹)ₙ is 6-Me. |
| Table 1-173 | R¹ is Me, R² is H, W¹ is O, G is C(O)Pr-n, X is S, and (Z¹)ₙ is 7-Me. |
| Table 1-174 | R¹ is Me, R² is H, W¹ is O, G is C(O)Pr-n, X is O, and (Z¹)ₙ is 7-Me. |
| Table 1-175 | R¹ is Me, R² is H, W¹ is O, G is C(O)Pr-n, X is S, and (Z¹)ₙ is 5-OMe. |
| Table 1-176 | R¹ is Me, R² is H, W¹ is O, G is C(O)Pr-n, X is O, and (Z¹)ₙ is 5-OMe. |
| Table 1-177 | R¹ is Me, R² is H, W¹ is O, G is C(O)Pr-n, X is S, and (Z¹)ₙ is 6-OMe. |
| Table 1-178 | R¹ is Me, R² is H, W¹ is O, G is C(O)Pr-n, X is O, and (Z¹)ₙ is 6-OMe. |
| Table 1-179 | R¹ is Me, R² is H, W¹ is O, G is C(O)Pr-n, X is S, and (Z¹)ₙ is 7-OMe. |
| Table 1-180 | R¹ is Me, R² is H, W¹ is O, G is C(O)Pr-n, X is O, and (Z¹)ₙ is 7-OMe. |
| Table 1-181 | R¹ is Me, R² is Cl, W¹ is O, G is C(O)Pr-n, X is S, and (Z¹)ₙ is "-". |
| Table 1-182 | R¹ is Me, R² is Cl, W¹ is O, G is C(O)Pr-n, X is O, and (Z¹)ₙ is "-". |
| Table 1-183 | R¹ is Me, R² is Cl, W¹ is O, G is C(O)Pr-n, X is S, and (Z¹)ₙ is 5-F. |
| Table 1-184 | R¹ is Me, R² is Cl, W¹ is O, G is C(O)Pr-n, X is O, and (Z¹)ₙ is 5-F. |
| Table 1-185 | R¹ is Me, R² is Cl, W¹ is O, G is C(O)Pr-n, X is S, and (Z¹)ₙ is 6-F. |
| Table 1-186 | R¹ is Me, R² is Cl, W¹ is O, G is C(O)Pr-n, X is O, and (Z¹)ₙ is 6-F. |
| Table 1-187 | R¹ is Me, R² is Cl, W¹ is O, G is C(O)Pr-n, X is S, and (Z¹)ₙ is 7-F. |
| Table 1-188 | R¹ is Me, R² is Cl, W¹ is O, G is C(O)Pr-n, X is O, and (Z¹)ₙ is 7-F. |
| Table 1-189 | R¹ is Me, R² is Cl, W¹ is O, G is C(O)Pr-n, X is S, and (Z¹)ₙ is 5-Cl. |
| Table 1-190 | R¹ is Me, R² is Cl, W¹ is O, G is C(O)Pr-n, X is O, and (Z¹)ₙ is 5-Cl. |
| Table 1-191 | R¹ is Me, R² is Cl, W¹ is O, G is C(O)Pr-n, X is S, and (Z¹)ₙ is 6-Cl. |
| Table 1-192 | R¹ is Me, R² is Cl, W¹ is O, G is C(O)Pr-n, X is O, and (Z¹)n is 6-Cl. |
| Table 1-193 | R¹ is Me, R² is Cl, W¹ is O, G is C(O)Pr-n, X is S, and (Z¹)n is 7-Cl. |
| Table 1-194 | R¹ is Me, R² is Cl, W¹ is O, G is C(O)Pr-n, X is O, and (Z¹)n is 7-Cl. |
| Table 1-195 | R¹ is Me, R² is Cl, W¹ is O, G is C(O)Pr-n, X is S, and (Z¹)n is 5-Me. |
| Table 1-196 | R¹ is Me, R² is Cl, W¹ is O, G is C(O)Pr-n, X is O, and (Z¹)n is 5-Me. |
| Table 1-197 | R¹ is Me, R² is Cl, W¹ is O, G is C(O)Pr-n, X is S, and (Z¹)n is 6-Me. |
| Table 1-198 | R¹ is Me, R² is Cl, W¹ is O, G is C(O)Pr-n, X is O, and (Z¹)n is 6-Me. |
| Table 1-199 | R¹ is Me, R² is Cl, W¹ is O, G is C(O)Pr-n, X is S, and (Z¹)n is 7-Me. |
| Table 1-200 | R¹ is Me, R² is Cl, W¹ is O, G is C(O)Pr-n, X is O, and (Z¹)n is 7-Me. |
| Table 1-201 | R¹ is Me, R² is Cl, W¹ is O, G is C(O)Pr-n, X is S, and (Z¹)n is 5-OMe. |
| Table 1-202 | R¹ is Me, R² is Cl, W¹ is O, G is C(O)Pr-n, X is O, and (Z¹)n is 5-OMe. |
| Table 1-203 | R¹ is Me, R² is Cl, W¹ is O, G is C(O)Pr-n, X is S, and (Z¹)ₙ is 6-OMe. |
| Table 1-204 | R¹ is Me, R² is Cl, W¹ is O, G is C(O)Pr-n, X is O, and (Z¹)n is 6-OMe. |
| Table 1-205 | R¹ is Me, R² is Cl, W¹ is O, G is C(O)Pr-n, X is S, and (Z¹)n is 7-OMe. |
| Table 1-206 | R¹ is Me, R² is Cl, W¹ is O, G is C(O)Pr-n, X is O, and (Z¹)n is 7-OMe. |
| Table 1-207 | R¹ is Me, R² is Me, W¹ is O, G is C(O)Pr-i, X is S, and (Z¹)n is "-". |
| Table 1-208 | R¹ is Me, R² is Me, W¹ is O, G is C(O)Pr-i, X is O, and (Z¹)n is "-". |
| Table 1-209 | R¹ is Me, R² is Me, W¹ is O, G is C(O)Pr-i, X is S, and (Z¹)n is 5-F. |
| Table 1-210 | R¹ is Me, R² is Me, W¹ is O, G is C(O)Pr-i, X is O, and (Z¹)n is 5-F. |
| Table 1-211 | R¹ is Me, R² is Me, W¹ is O, G is C(O)Pr-i, X is S, and (Z¹)n is 6-F. |
| Table 1-212 | R¹ is Me, R² is Me, W¹ is O, G is C(O)Pr-i, X is O, and (Z¹)n is 6-F. |
| Table 1-213 | R¹ is Me, R² is Me, W¹ is O, G is C(O)Pr-i, X is S, and (Z¹)n is 7-F. |
| Table 1-214 | R¹ is Me, R² is Me, W¹ is O, G is C(O)Pr-i, X is O, and (Z¹)n is 7-F. |
| Table 1-215 | R¹ is Me, R² is Me, W¹ is O, G is C(O)Pr-i, X is S, and (Z¹)n is 5-Cl. |
| Table 1-216 | R¹ is Me, R² is Me, W¹ is O, G is C(O)Pr-i, X is O, and (Z¹)n is 5-Cl. |
| Table 1-217 | R¹ is Me, R² is Me, W¹ is O, G is C(O)Pr-i, X is S, and (Z¹)ₙ is 6-Cl. |
| Table 1-218 | R¹ is Me, R² is Me, W¹ is O, G is C(O)Pr-i, X is O, and (Z¹)n is 6-Cl. |
| Table 1-219 | R¹ is Me, R² is Me, W¹ is O, G is C(O)Pr-i, X is S, and (Z¹)n is 7-Cl. |
| Table 1-220 | R¹ is Me, R² is Me, W¹ is O, G is C(O)Pr-i, X is O, and (Z¹)n is 7-Cl. |
| Table 1-221 | R¹ is Me, R² is Me, W¹ is O, G is C(O)Pr-i, X is S, and (Z¹)n is 5-Me. |
| Table 1-222 | R¹ is Me, R² is Me, W¹ is O, G is C(O)Pr-i, X is O, and (Z¹)n is 5-Me. |
| Table 1-223 | R¹ is Me, R² is Me, W¹ is O, G is C(O)Pr-i, X is S, and (Z¹)n is 6-Me. |
| Table 1-224 | R¹ is Me, R² is Me, W¹ is O, G is C(O)Pr-i, X is O, and (Z¹)n is 6-Me. |
| Table 1-225 | R¹ is Me, R² is Me, W¹ is O, G is C(O)Pr-i, X is S, and (Z¹)n is 7-Me. |
| Table 1-226 | R¹ is Me, R² is Me, W¹ is O, G is C(O)Pr-i, X is O, and (Z¹)n is 7-Me. |
| Table 1-227 | R¹ is Me, R² is Me, W¹ is O, G is C(O)Pr-i, X is S, and (Z¹)n is 5-OMe. |
| Table 1-228 | R¹ is Me, R² is Me, W¹ is O, G is C(O)Pr-i, X is O, and (Z¹)n is 5-OMe. |
| Table 1-229 | R¹ is Me, R² is Me, W¹ is O, G is C(O)Pr-i, X is S, and (Z¹)n is 6-OMe. |
| Table 1-230 | R¹ is Me, R² is Me, W¹ is O, G is C(O)Pr-i, X is O, and (Z¹)n is 6-OMe. |
| Table 1-231 | R¹ is Me, R² is Me, W¹ is O, G is C(O)Pr-i, X is S, and (Z¹)n is 7-OMe. |
| Table 1-232 | R¹ is Me, R² is Me, W¹ is O, G is C(O)Pr-i, X is O, and (Z¹)n is 7-OMe. |
| Table 1-233 | R¹ is Me, R² is Me, W¹ is O, G is C(O)OMe, X is S, and (Z¹)n is "-". |
| Table 1-234 | R¹ is Me, R² is Me, W¹ is O, G is C(O)OMe, X is O, and (Z¹)n is "-". |
| Table 1-235 | R¹ is Me, R² is Me, W¹ is O, G is C(O)OMe, X is S, and (Z¹)n is 5-F. |
| Table 1-236 | R¹ is Me, R² is Me, W¹ is O, G is C(O)OMe, X is O, and (Z¹)ₙ is 5-F. |
| Table 1-237 | R¹ is Me, R² is Me, W¹ is O, G is C(O)OMe, X is S, and (Z¹)n is 6-F. |
| Table 1-238 | R¹ is Me, R² is Me, W¹ is O, G is C(O)OMe, X is O, and (Z¹)n is 6-F. |
| Table 1-239 | R¹ is Me, R² is Me, W¹ is O, G is C(O)OMe, X is S, and (Z¹)n is 7-F. |
| Table 1-240 | R¹ is Me, R² is Me, W¹ is O, G is C(O)OMe, X is O, and (Z¹)ₙ is 7-F. |
| Table 1-241 | R¹ is Me, R² is Me, W¹ is O, G is C(O)OMe, X is S, and (Z¹)n is 5-Cl. |
| Table 1-242 | R¹ is Me, R² is Me, W¹ is O, G is C(O)OMe, X is O, and (Z¹)n is 5-Cl. |
| Table 1-243 | R¹ is Me, R² is Me, W¹ is O, G is C(O)OMe, X is S, and (Z¹)n is 6-Cl. |
| Table 1-244 | R¹ is Me, R² is Me, W¹ is O, G is C(O)OMe, X is O, and (Z¹)n is 6-Cl. |
| Table 1-245 | R¹ is Me, R² is Me, W¹ is O, G is C(O)OMe, X is S, and (Z¹)n is 7-Cl. |
| Table 1-246 | R¹ is Me, R² is Me, W¹ is O, G is C(O)OMe, X is O, and (Z¹)ₙ is 7-Cl. |
| Table 1-247 | R¹ is Me, R² is Me, W¹ is O, G is C(O)OMe, X is S, and (Z¹)n is 5-Me. |
| Table 1-248 | R¹ is Me, R² is Me, W¹ is O, G is C(O)OMe, X is O, and (Z¹)n is 5-Me. |
| Table 1-249 | R¹ is Me, R² is Me, W¹ is O, G is C(O)OMe, X is S, and (Z¹)n is 6-Me. |
| Table 1-250 | R¹ is Me, R² is Me, W¹ is O, G is C(O)OMe, X is O, and (Z¹)n is 6-Me. |
| Table 1-251 | R¹ is Me, R² is Me, W¹ is O, G is C(O)OMe, X is S, and (Z¹)n is 7-Me. |
| Table 1-252 | R¹ is Me, R² is Me, W¹ is O, G is C(O)OMe, X is O, and (Z¹)n is 7-Me. |
| Table 1-253 | R¹ is Me, R² is Me, W¹ is O, G is C(O)OMe, X is S, and (Z¹)n is 5-OMe. |
| Table 1-254 | R¹ is Me, R² is Me, W¹ is O, G is C(O)OMe, X is O, and (Z¹)n is 5-OMe. |
| Table 1-255 | R¹ is Me, R² is Me, W¹ is O, G is C(O)OMe, X is S, and (Z¹)n is 6-OMe. |
| Table 1-256 | R¹ is Me, R² is Me, W¹ is O, G is C(O)OMe, X is O, and (Z¹)n is 6-OMe. |
| Table 1-257 | R¹ is Me, R² is Me, W¹ is O, G is C(O)OMe, X is S, and (Z¹)n is 7-OMe. |
| Table 1-258 | R¹ is Me, R² is Me, W¹ is O, G is C(O)OMe, X is O, and (Z¹)n is 7-OMe. |
| Table 1-259 | R¹ is Me, R² is Me, W¹ is O, G is C(O)OEt, X is S, and (Z¹)n is "-". |
| Table 1-260 | R¹ is Me, R² is Me, W¹ is O, G is C(O)OEt, X is O, and (Z¹)n is "-". |
| Table 1-261 | R¹ is Me, R² is Me, W¹ is O, G is C(O)OEt, X is S, and (Z¹)n is 5-F. |
| Table 1-262 | R¹ is Me, R² is Me, W¹ is O, G is C(O)OEt, X is O, and (Z¹)n is 5-F. |
| Table 1-263 | R¹ is Me, R² is Me, W¹ is O, G is C(O)OEt, X is S, and (Z¹)ₙ is 6-F. |
| Table 1-264 | R¹ is Me, R² is Me, W¹ is O, G is C(O)OEt, X is O, and (Z¹)n is 6-F. |
| Table 1-265 | R¹ is Me, R² is Me, W¹ is O, G is C(O)OEt, X is S, and (Z¹)n is 7-F. |
| Table 1-266 | R¹ is Me, R² is Me, W¹ is O, G is C(O)OEt, X is O, and (Z¹)n is 7-F. |
| Table 1-267 | R¹ is Me, R² is Me, W¹ is O, G is C(O)OEt, X is S, and (Z¹)ₙ is 5-Cl. |
| Table 1-268 | R¹ is Me, R² is Me, W¹ is O, G is C(O)OEt, X is O, and (Z¹)n is 5-Cl. |
| Table 1-269 | R¹ is Me, R² is Me, W¹ is O, G is C(O)OEt, X is S, and (Z¹)n is 6-Cl. |
| Table 1-270 | R¹ is Me, R² is Me, W¹ is O, G is C(O)OEt, X is O, and (Z¹)n is 6-Cl. |
| Table 1-271 | R¹ is Me, R² is Me, W¹ is O, G is C(O)OEt, X is S, and (Z¹)n is 7-Cl. |
| Table 1-272 | R¹ is Me, R² is Me, W¹ is O, G is C(O)OEt, X is O, and (Z¹)n is 7-Cl. |
| Table 1-273 | R¹ is Me, R² is Me, W¹ is O, G is C(O)OEt, X is S, and (Z¹)n is 5-Me. |
| Table 1-274 | R¹ is Me, R² is Me, W¹ is O, G is C(O)OEt, X is O, and (Z¹)n is 5-Me. |
| Table 1-275 | R¹ is Me, R² is Me, W¹ is O, G is C(O)OEt, X is S, and (Z¹)n is 6-Me. |
| Table 1-276 | R¹ is Me, R² is Me, W¹ is O, G is C(O)OEt, X is O, and (Z¹)n is 6-Me. |
| Table 1-277 | R¹ is Me, R² is Me, W¹ is O, G is C(O)OEt, X is S, and (Z¹)n is 7-Me. |
| Table 1-278 | R¹ is Me, R² is Me, W¹ is O, G is C(O)OEt, X is O, and (Z¹)n is 7-Me. |
| Table 1-279 | R¹ is Me, R² is Me, W¹ is O, G is C(O)OEt, X is S, and (Z¹)n is 5-OMe. |
| Table 1-280 | R¹ is Me, R² is Me, W¹ is O, G is C(O)OEt, X is O, and (Z¹)n is 5-OMe. |
| Table 1-281 | R¹ is Me, R² is Me, W¹ is O, G is C(O)OEt, X is S, and (Z¹)n is 6-OMe. |
| Table 1-282 | R¹ is Me, R² is Me, W¹ is O, G is C(O)OEt, X is O, and (Z¹)n is 6-OMe. |
| Table 1-283 | R¹ is Me, R² is Me, W¹ is O, G is C(O)OEt, X is S, and (Z¹)n is 7-OMe. |
| Table 1-284 | R¹ is Me, R² is Me, W¹ is O, G is C(O)OEt, X is O, and (Z¹)n is 7-OMe. |
| Table 1-285 | R¹ is Me, R² is Me, W¹ is O, G is C(O)OCH₂CH₂OMe, X is S, and (Z¹)n is "-". |
| Table 1-286 | R¹ is Me, R² is Me, W¹ is O, G is C(O)OCH₂CH₂OMe, X is O, and (Z¹)ₙ is "-". |
| Table 1-287 | R¹ is Me, R² is Me, W¹ is O, G is C(O)OCH₂CH₂OMe, X is S, and (Z¹)n is 5-F. |
| Table 1-288 | R¹ is Me, R² is Me, W¹ is O, G is C(O)OCH₂CH₂OMe, X is O, and (Z¹)n is 5-F. |
| Table 1-289 | R¹ is Me, R² is Me, W¹ is O, G is C(O)OCH₂CH₂OMe, X is S, and (Z¹)n is 6-F. |
| Table 1-290 | R¹ is Me, R² is Me, W¹ is O, G is C(O)OCH₂CH₂OMe, X is O, and (Z¹)n is 6-F. |
| Table 1-291 | R¹ is Me, R² is Me, W¹ is O, G is C(O)OCH₂CH₂OMe, X is S, and (Z¹)ₙ is 7-F. |
| Table 1-292 | R¹ is Me, R² is Me, W¹ is O, G is C(O)OCH₂CH₂OMe, X is O, and (Z¹)ₙ is 7-F. |
| Table 1-293 | R¹ is Me, R² is Me, W¹ is O, G is C(O)OCH₂CH₂OMe, X is S, and (Z¹)n is 5-Cl. |
| Table 1-294 | R¹ is Me, R² is Me, W¹ is O, G is C(O)OCH₂CH₂OMe, X is O, and (Z¹)n is 5-Cl. |
| Table 1-295 | R¹ is Me, R² is Me, W¹ is O, G is C(O)OCH₂CH₂OMe, X is S, and (Z¹)n is 6-Cl. |
| Table 1-296 | R¹ is Me, R² is Me, W¹ is O, G is C(O)OCH₂CH₂OMe, X is O, and (Z¹)n is 6-Cl. |
| Table 1-297 | R¹ is Me, R² is Me, W¹ is O, G is C(O)OCH₂CH₂OMe, X is S, and (Z¹)n is 7-Cl. |
| Table 1-298 | R¹ is Me, R² is Me, W¹ is O, G is C(O)OCH₂CH₂OMe, X is O, and (Z¹)ₙ is 7-Cl. |
| Table 1-299 | R¹ is Me, R² is Me, W¹ is O, G is C(O)OCH₂CH₂OMe, X is S, and (Z¹)n is 5-Me. |
| Table 1-300 | R¹ is Me, R² is Me, W¹ is O, G is C(O)OCH₂CH₂OMe, X is O, and (Z¹)n is 5-Me. |
| Table 1-301 | R¹ is Me, R² is Me, W¹ is O, G is C(O)OCH₂CH₂OMe, X is S, and (Z¹)n is 6-Me. |
| Table 1-302 | R¹ is Me, R² is Me, W¹ is O, G is C(O)OCH₂CH₂OMe, X is O, and (Z¹)n is 6-Me. |
| Table 1-303 | R¹ is Me, R² is Me, W¹ is O, G is C(O)OCH₂CH₂OMe, X is S, and (Z¹)n is 7-Me. |
| Table 1-304 | R¹ is Me, R² is Me, W¹ is O, G is C(O)OCH₂CH₂OMe, X is O, and (Z¹)n is 7-Me. |
| Table 1-305 | R¹ is Me, R² is Me, W¹ is O, G is C(O)OCH₂CH₂OMe, X is S, and (Z¹)n is 5-OMe. |
| Table 1-306 | R¹ is Me, R² is Me, W¹ is O, G is C(O)OCH₂CH₂OMe, X is O, and (Z¹)n is 5-OMe |
| Table 1-307 | R¹ is Me, R² is Me, W¹ is O, G is C(O)OCH₂CH₂OMe, X is S, and (Z¹)n is 6-OMe. |
| Table 1-308 | R¹ is Me, R² is Me, W¹ is O, G is C(O)OCH₂CH₂OMe, X is O, and (Z¹)n is 6-OMe |
| Table 1-309 | R¹ is Me, R² is Me, W¹ is O, G is C(O)OCH₂CH₂OMe, X is S, and (Z¹)n is 7-OMe. |
| Table 1-310 | R¹ is Me, R² is Me, W¹ is O, G is C(O)OCH₂CH₂OMe, X is O, and (Z¹)n is 7-OMe |
| Table 1-311 | R¹ is Me, R² is Me, W¹ is O, G is C(O)[Q-2], X is S, and (Z¹)n is "-". |
| Table 1-312 | R¹ is Me, R² is Me, W¹ is O, G is C(O)[Q-2], X is O, and (Z¹)n is "-". |
| Table 1-313 | R¹ is Me, R² is Me, W¹ is O, G is C(O)[Q-2], X is S, and (Z¹)n is 5-F. |
| Table 1-314 | R¹ is Me, R² is Me, W¹ is O, G is C(O)[Q-2], X is O, and (Z¹)n is 5-F. |
| Table 1-315 | R¹ is Me, R² is Me, W¹ is O, G is C(O)[Q-2], X is S, and (Z¹)ₙ is 6-F. |
| Table 1-316 | R¹ is Me, R² is Me, W¹ is O, G is C(O)[Q-2], X is O, and (Z¹)n is 6-F. |
| Table 1-317 | R¹ is Me, R² is Me, W¹ is O, G is C(O)[Q-2], X is S, and (Z¹)n is 7-F. |
| Table 1-318 | R¹ is Me, R² is Me, W¹ is O, G is C(O)[Q-2], X is O, and (Z¹)n is 7-F. |
| Table 1-319 | R¹ is Me, R² is Me, W¹ is O, G is C(O)[Q-2], X is S, and (Z¹)ₙ is 5-Cl. |
| Table 1-320 | R¹ is Me, R² is Me, W¹ is O, G is C(O)[Q-2], X is O, and (Z¹)n is 5-Cl. |
| Table 1-321 | R¹ is Me, R² is Me, W¹ is O, G is C(O)[Q-2], X is S, and (Z¹)n is 6-Cl. |
| Table 1-322 | R¹ is Me, R² is Me, W¹ is O, G is C(O)[Q-2], X is O, and (Z¹)n is 6-Cl. |
| Table 1-323 | R¹ is Me, R² is Me, W¹ is O, G is C(O)[Q-2], X is S, and (Z¹)n is 7-Cl. |
| Table 1-324 | R¹ is Me, R² is Me, W¹ is O, G is C(O)[Q-2], X is O, and (Z¹)n is 7-Cl. |
| Table 1-325 | R¹ is Me, R² is Me, W¹ is O, G is C(O)[Q-2], X is S, and (Z¹)n is 5-Me. |
| Table 1-326 | R¹ is Me, R² is Me, W¹ is O, G is C(O)[Q-2], X is O, and (Z¹)n is 5-Me. |
| Table 1-327 | R¹ is Me, R² is Me, W¹ is O, G is C(O)[Q-2], X is S, and (Z¹)n is 6-Me. |
| Table 1-328 | R¹ is Me, R² is Me, W¹ is O, G is C(O)[Q-2], X is O, and (Z¹)n is 6-Me. |
| Table 1-329 | R¹ is Me, R² is Me, W¹ is O, G is C(O)[Q-2], X is S, and (Z¹)n is 7-Me. |
| Table 1-330 | R¹ is Me, R² is Me, W¹ is O, G is C(O)[Q-2], X is O, and (Z¹)n is 7-Me. |
| Table 1-331 | R¹ is Me, R² is Me, W¹ is O, G is C(O)[Q-2], X is S, and (Z¹)n is 5-OMe. |
| Table 1-332 | R¹ is Me, R² is Me, W¹ is O, G is C(O)[Q-2], X is O, and (Z¹)n is 5-OMe. |
| Table 1-333 | R¹ is Me, R² is Me, W¹ is O, G is C(O)[Q-2], X is S, and (Z¹)n is 6-OMe. |
| Table 1-334 | R¹ is Me, R² is Me, W¹ is O, G is C(O)[Q-2], X is O, and (Z¹)n is 6-OMe. |
| Table 1-335 | R¹ is Me, R² is Me, W¹ is O, G is C(O)[Q-2], X is S, and (Z¹)n is 7-OMe. |
| Table 1-336 | R¹ is Me, R² is Me, W¹ is O, G is C(O)[Q-2], X is O, and (Z¹)n is 7-OMe. |
| Table 1-337 | R¹ is Me, R² is Me, W¹ is O, G is C(O)[U-1a], X is S, and (Z¹)n is "-". |
| Table 1-338 | R¹ is Me, R² is Me, W¹ is O, G is C(O)[U-1a], X is O, and (Z¹)ₙ is "-". |
| Table 1-339 | R¹ is Me, R² is Me, W¹ is O, G is C(O)[U-1a], X is S, and (Z¹)n is 5-F. |
| Table 1-340 | R¹ is Me, R² is Me, W¹ is O, G is C(O)[U-1a], X is O, and (Z¹)n is 5-F. |
| Table 1-341 | R¹ is Me, R² is Me, W¹ is O, G is C(O)[U-1a], X is S, and (Z¹)ₙ is 6-F. |
| Table 1-342 | R¹ is Me, R² is Me, W¹ is O, G is C(O)[U-1a], X is O, and (Z¹)n is 6-F. |
| Table 1-343 | R¹ is Me, R² is Me, W¹ is O, G is C(O)[U-1a], X is S, and (Z¹)n is 7-F. |
| Table 1-344 | R¹ is Me, R² is Me, W¹ is O, G is C(O)[U-1a], X is O, and (Z¹)n is 7-F. |
| Table 1-345 | R¹ is Me, R² is Me, W¹ is O, G is C(O)[U-1a], X is S, and (Z¹)n is 5-Cl. |
| Table 1-346 | R¹ is Me, R² is Me, W¹ is O, G is C(O)[U-1a], X is O, and (Z¹)n is 5-Cl. |
| Table 1-347 | R¹ is Me, R² is Me, W¹ is O, G is C(O)[U-1a], X is S, and (Z¹)n is 6-Cl. |
| Table 1-348 | R¹ is Me, R² is Me, W¹ is O, G is C(O)[U-1a], X is O, and (Z¹)n is 6-Cl. |
| Table 1-349 | R¹ is Me, R² is Me, W¹ is O, G is C(O)[U-1a], X is S, and (Z¹)n is 7-Cl. |
| Table 1-350 | R¹ is Me, R² is Me, W¹ is O, G is C(O)[U-1a], X is O, and (Z¹)n is 7-Cl. |
| Table 1-351 | R¹ is Me, R² is Me, W¹ is O, G is C(O)[U-1a], X is S, and (Z¹)n is 5-Me. |
| Table 1-352 | R¹ is Me, R² is Me, W¹ is O, G is C(O)[U-1a], X is O, and (Z¹)n is 5-Me. |
| Table 1-353 | R¹ is Me, R² is Me, W¹ is O, G is C(O)[U-1a], X is S, and (Z¹)n is 6-Me. |
| Table 1-354 | R¹ is Me, R² is Me, W¹ is O, G is C(O)[U-1a], X is O, and (Z¹)n is 6-Me. |
| Table 1-355 | R¹ is Me, R² is Me, W¹ is O, G is C(O)[U-1a], X is S, and (Z¹)n is 7-Me. |
| Table 1-356 | R¹ is Me, R² is Me, W¹ is O, G is C(O)[U-1a], X is O, and (Z¹)n is 7-Me. |
| Table 1-357 | R¹ is Me, R² is Me, W¹ is O, G is C(O)[U-1a], X is S, and (Z¹)n is 5-OMe. |
| Table 1-358 | R¹ is Me, R² is Me, W¹ is O, G is C(O)[U-1a], X is O, and (Z¹)n is 5-OMe. |
| Table 1-359 | R¹ is Me, R² is Me, W¹ is O, G is C(O)[U-1a], X is S, and (Z¹)n is 6-OMe. |
| Table 1-360 | R¹ is Me, R² is Me, W¹ is O, G is C(O)[U-1a], X is O, and (Z¹)n is 6-OMe. |
| Table 1-361 | R¹ is Me, R² is Me, W¹ is O, G is C(O)[U-1a], X is S, and (Z¹)n is 7-OMe. |
| Table 1-362 | R¹ is Me, R² is Me, W¹ is O, G is C(O)[U-1a], X is O, and (Z¹)n is 7-OMe. |
| Table 1-363 | R¹ is Me, R² is Me, W¹ is O, G is C(O)[Ph-2-Me], X is S, and (Z¹)n is "-". |
| Table 1-364 | R¹ is Me, R² is Me, W¹ is O, G is C(O)[Ph-2-Me], X is O, and (Z¹)n is "-". |
| Table 1-365 | R¹ is Me, R² is Me, W¹ is O, G is C(O)[Ph-2-Me], X is S, and (Z¹)n is 5-F. |
| Table 1-366 | R¹ is Me, R² is Me, W¹ is O, G is C(O)[Ph-2-Me], X is O, and (Z¹)n is 5-F. |
| Table 1-367 | R¹ is Me, R² is Me, W¹ is O, G is C(O)[Ph-2-Me], X is S, and (Z¹)n is 6-F. |
| Table 1-368 | R¹ is Me, R² is Me, W¹ is O, G is C(O)[Ph-2-Me], X is O, and (Z¹)n is 6-F. |
| Table 1-369 | R¹ is Me, R² is Me, W¹ is O, G is C(O)[Ph-2-Me], X is S, and (Z¹)n is 7-F. |
| Table 1-370 | R¹ is Me, R² is Me, W¹ is O, G is C(O)[Ph-2-Me], X is O, and (Z¹)n is 7-F. |
| Table 1-371 | R¹ is Me, R² is Me, W¹ is O, G is C(O)[Ph-2-Me], X is S, and (Z¹)n is 5-Cl. |
| Table 1-372 | R¹ is Me, R² is Me, W¹ is O, G is C(O)[Ph-2-Me], X is O, and (Z¹)n is 5-Cl. |
| Table 1-373 | R¹ is Me, R² is Me, W¹ is O, G is C(O)[Ph-2-Me], X is S, and (Z¹)n is 6-Cl. |
| Table 1-374 | R¹ is Me, R² is Me, W¹ is O, G is C(O)[Ph-2-Me], X is O, and (Z¹)n is 6-Cl. |
| Table 1-375 | R¹ is Me, R² is Me, W¹ is O, G is C(O)[Ph-2-Me], X is S, and (Z¹)n is 7-Cl. |
| Table 1-376 | R¹ is Me, R² is Me, W¹ is O, G is C(O)[Ph-2-Me], X is O, and (Z¹)n is 7-Cl. |
| Table 1-377 | R¹ is Me, R² is Me, W¹ is O, G is C(O)[Ph-2-Me], X is S, and (Z¹)n is 5-Me. |
| Table 1-378 | R¹ is Me, R² is Me, W¹ is O, G is C(O)[Ph-2-Me], X is O, and (Z¹)n is 5-Me. |
| Table 1-379 | R¹ is Me, R² is Me, W¹ is O, G is C(O)[Ph-2-Me], X is S, and (Z¹)n is 6-Me. |
| Table 1-380 | R¹ is Me, R² is Me, W¹ is O, G is C(O)[Ph-2-Me], X is O, and (Z¹)n is 6-Me. |
| Table 1-381 | R¹ is Me, R² is Me, W¹ is O, G is C(O)[Ph-2-Me], X is S, and (Z¹)n is 7-Me. |
| Table 1-382 | R¹ is Me, R² is Me, W¹ is O, G is C(O)[Ph-2-Me], X is O, and (Z¹)n is 7-Me. |
| Table 1-383 | R¹ is Me, R² is Me, W¹ is O, G is C(O)[Ph-2-Me], X is S, and (Z¹)n is 5-OMe. |
| Table 1-384 | R¹ is Me, R² is Me, W¹ is O, G is C(O)[Ph-2-Me], X is O, and (Z¹)n is 5-OMe |
| Table 1-385 | R¹ is Me, R² is Me, W¹ is O, G is C(O)[Ph-2-Me], X is S, and (Z¹)n is 6-OMe. |
| Table 1-386 | R¹ is Me, R² is Me, W¹ is O, G is C(O)[Ph-2-Me], X is O, and (Z¹)n is 6-OMe |
| Table 1-387 | R¹ is Me, R² is Me, W¹ is O, G is C(O)[Ph-2-Me], X is S, and (Z¹)n is 7-OMe. |
| Table 1-388 | R¹ is Me, R² is Me, W¹ is O, G is C(O)[Ph-2-Me], X is O, and (Z¹)n is 7-OMe |
| Table 1-389 | R¹ is Me, R² is Me, W¹ is O, G is S(O)₂Me, X is S, and (Z¹)n is "-". |
| Table 1-390 | R¹ is Me, R² is Me, W¹ is O, G is S(O)₂Me, X is O, and (Z¹)n is "-". |
| Table 1-391 | R¹ is Me, R² is Me, W¹ is O, G is S(O)₂Me, X is S, and (Z¹)n is 5-F. |
| Table 1-392 | R¹ is Me, R² is Me, W¹ is O, G is S(O)₂Me, X is O, and (Z¹)n is 5-F. |
| Table 1-393 | R¹ is Me, R² is Me, W¹ is O, G is S(O)₂Me, X is S, and (Z¹)n is 6-F. |
| Table 1-394 | R¹ is Me, R² is Me, W¹ is O, G is S(O)₂Me, X is O, and (Z¹)n is 6-F. |
| Table 1-395 | R¹ is Me, R² is Me, W¹ is O, G is S(O)₂Me, X is S, and (Z¹)n is 7-F. |
| Table 1-396 | R¹ is Me, R² is Me, W¹ is O, G is S(O)₂Me, X is O, and (Z¹)n is 7-F. |
| Table 1-397 | R¹ is Me, R² is Me, W¹ is O, G is S(O)₂Me, X is S, and (Z¹)n is 5-Cl. |
| Table 1-398 | R¹ is Me, R² is Me, W¹ is O, G is S(O)₂Me, X is O, and (Z¹)n is 5-Cl. |
| Table 1-399 | R¹ is Me, R² is Me, W¹ is O, G is S(O)₂Me, X is S, and (Z¹)n is 6-Cl. |
| Table 1-400 | R¹ is Me, R² is Me, W¹ is O, G is S(O)₂Me, X is O, and (Z¹)n is 6-Cl. |
| Table 1-401 | R¹ is Me, R² is Me, W¹ is O, G is S(O)₂Me, X is S, and (Z¹)n is 7-Cl. |
| Table 1-402 | R¹ is Me, R² is Me, W¹ is O, G is S(O)₂Me, X is O, and (Z¹)n is 7-Cl. |
| Table 1-403 | R¹ is Me, R² is Me, W¹ is O, G is S(O)₂Me, X is S, and (Z¹)n is 5-Me. |
| Table 1-404 | R¹ is Me, R² is Me, W¹ is O, G is S(O)₂Me, X is O, and (Z¹)n is 5-Me. |
| Table 1-405 | R¹ is Me, R² is Me, W¹ is O, G is S(O)₂Me, X is S, and (Z¹)n is 6-Me. |
| Table 1-406 | R¹ is Me, R² is Me, W¹ is O, G is S(O)₂Me, X is O, and (Z¹)n is 6-Me. |
| Table 1-407 | R¹ is Me, R² is Me, W¹ is O, G is S(O)₂Me, X is S, and (Z¹)n is 7-Me. |
| Table 1-408 | R¹ is Me, R² is Me, W¹ is O, G is S(O)₂Me, X is O, and (Z¹)n is 7-Me. |
| Table 1-409 | R¹ is Me, R² is Me, W¹ is O, G is S(O)₂Me, X is S, and (Z¹)n is 5-OMe. |
| Table 1-410 | R¹ is Me, R² is Me, W¹ is O, G is S(O)₂Me, X is O, and (Z¹)n is 5-OMe. |
| Table 1-411 | R¹ is Me, R² is Me, W¹ is O, G is S(O)₂Me, X is S, and (Z¹)n is 6-OMe. |
| Table 1-412 | R¹ is Me, R² is Me, W¹ is O, G is S(O)₂Me, X is O, and (Z¹)n is 6-OMe. |
| Table 1-413 | R¹ is Me, R² is Me, W¹ is O, G is S(O)₂Me, X is S, and (Z¹)n is 7-OMe. |
| Table 1-414 | R¹ is Me, R² is Me, W¹ is O, G is S(O)₂Me, X is O, and (Z¹)n is 7-OMe. |
| Table 1-415 | R¹ is Me, R² is Me, W¹ is O, G is S(O)₂[Ph-4-Me], X is S, and (Z¹)n is "-". |
| Table 1-416 | R¹ is Me, R² is Me, W¹ is O, G is S(O)₂[Ph-4-Me], X is O, and (Z¹)n is "-". |
| Table 1-417 | R¹ is Me, R² is Me, W¹ is O, G is S(O)₂[Ph-4-Me], X is S, and (Z¹)n is 5-F. |
| Table 1-418 | R¹ is Me, R² is Me, W¹ is O, G is S(O)₂[Ph-4-Me], X is O, and (Z¹)n is 5-F. |
| Table 1-419 | R¹ is Me, R² is Me, W¹ is O, G is S(O)₂[Ph-4-Me], X is S, and (Z¹)n is 6-F. |
| Table 1-420 | R¹ is Me, R² is Me, W¹ is O, G is S(O)₂[Ph-4-Me], X is O, and (Z¹)n is 6-F. |
| Table 1-421 | R¹ is Me, R² is Me, W¹ is O, G is S(O)₂[Ph-4-Me], X is S, and (Z¹)n is 7-F. |
| Table 1-422 | R¹ is Me, R² is Me, W¹ is O, G is S(O)₂[Ph-4-Me], X is O, and (Z¹)n is 7-F. |
| Table 1-423 | R¹ is Me, R² is Me, W¹ is O, G is S(O)₂[Ph-4-Me], X is S, and (Z¹)n is 5-Cl. |
| Table 1-424 | R¹ is Me, R² is Me, W¹ is O, G is S(O)₂[Ph-4-Me], X is O, and (Z¹)n is 5-Cl. |
| Table 1-425 | R¹ is Me, R² is Me, W¹ is O, G is S(O)₂[Ph-4-Me], X is S, and (Z¹)n is 6-Cl. |
| Table 1-426 | R¹ is Me, R² is Me, W¹ is O, G is S(O)₂[Ph-4-Me], X is O, and (Z¹)n is 6-Cl. |
| Table 1-427 | R¹ is Me, R² is Me, W¹ is O, G is S(O)₂[Ph-4-Me], X is S, and (Z¹)n is 7-Cl. |
| Table 1-428 | R¹ is Me, R² is Me, W¹ is O, G is S(O)₂[Ph-4-Me], X is O, and (Z¹)n is 7-Cl. |
| Table 1-429 | R¹ is Me, R² is Me, W¹ is O, G is S(O)₂[Ph-4-Me], X is S, and (Z¹)n is 5-Me. |
| Table 1-430 | R¹ is Me, R² is Me, W¹ is O, G is S(O)₂[Ph-4-Me], X is O, and (Z¹)n is 5-Me. |
| Table 1-431 | R¹ is Me, R² is Me, W¹ is O, G is S(O)₂[Ph-4-Me], X is S, and (Z¹)n is 6-Me. |
| Table 1-432 | R¹ is Me, R² is Me, W¹ is O, G is S(O)₂[Ph-4-Me], X is O, and (Z¹)n is 6-Me. |
| Table 1-433 | R¹ is Me, R² is Me, W¹ is O, G is S(O)₂[Ph-4-Me], X is S, and (Z¹)n is 7-Me. |
| Table 1-434 | R¹ is Me, R² is Me, W¹ is O, G is S(O)₂[Ph-4-Me], X is O, and (Z¹)n is 7-Me. |
| Table 1-435 | R¹ is Me, R² is Me, W¹ is O, G is S(O)₂[Ph-4-Me], X is S, and (Z¹)n is 5-OMe. |
| Table 1-436 | R¹ is Me, R² is Me, W¹ is O, G is S(O)₂[Ph-4-Me], X is O, and (Z¹)n is 5-OMe |
| Table 1-437 | R¹ is Me, R² is Me, W¹ is O, G is S(O)₂[Ph-4-Me], X is S, and (Z¹)n is 6-OMe. |
| Table 1-438 | R¹ is Me, R² is Me, W¹ is O, G is S(O)₂[Ph-4-Me], X is O, and (Z¹)n is 6-OMe |
| Table 1-439 | R¹ is Me, R² is Me, W¹ is O, G is S(O)₂[Ph-4-Me], X is S, and (Z¹)n is 7-OMe. |
| Table 1-440 | R¹ is Me, R² is Me, W¹ is O, G is S(O)₂[Ph-4-Me], X is O, and (Z¹)n is 7-OMe |
| Table 1-441 | R¹ is Me, R² is Me, W¹ is O, G is C(O)CH₂OPh, X is S, and (Z¹)n is "-". |
| Table 1-442 | R¹ is Me, R² is Me, W¹ is O, G is C(O)CH₂OPh, X is O, and (Z¹)n is "-". |
| Table 1-443 | R¹ is Me, R² is Me, W¹ is O, G is C(O)CH₂OPh, X is S, and (Z¹)n is 5-F. |
| Table 1-444 | R¹ is Me, R² is Me, W¹ is O, G is C(O)CH₂OPh, X is O, and (Z¹)n is 5-F. |
| Table 1-445 | R¹ is Me, R² is Me, W¹ is O, G is C(O)CH₂OPh, X is S, and (Z¹)n is 6-F. |
| Table 1-446 | R¹ is Me, R² is Me, W¹ is O, G is C(O)CH₂OPh, X is O, and (Z¹)n is 6-F. |
| Table 1-447 | R¹ is Me, R² is Me, W¹ is O, G is C(O)CH₂OPh, X is S, and (Z¹)n is 7-F. |
| Table 1-448 | R¹ is Me, R² is Me, W¹ is O, G is C(O)CH₂OPh, X is O, and (Z¹)n is 7-F. |
| Table 1-449 | R¹ is Me, R² is Me, W¹ is O, G is C(O)CH₂OPh, X is S, and (Z¹)n is 5-Cl. |
| Table 1-450 | R¹ is Me, R² is Me, W¹ is O, G is C(O)CH₂OPh, X is O, and (Z¹)n is 5-Cl. |
| Table 1-451 | R¹ is Me, R² is Me, W¹ is O, G is C(O)CH₂OPh, X is S, and (Z¹)n is 6-Cl. |
| Table 1-452 | R¹ is Me, R² is Me, W¹ is O, G is C(O)CH₂OPh, X is O, and (Z¹)n is 6-Cl. |
| Table 1-453 | R¹ is Me, R² is Me, W¹ is O, G is C(O)CH₂OPh, X is S, and (Z¹)n is 7-Cl. |
| Table 1-454 | R¹ is Me, R² is Me, W¹ is O, G is C(O)CH₂OPh, X is O, and (Z¹)n is 7-Cl. |
| Table 1-455 | R¹ is Me, R² is Me, W¹ is O, G is C(O)CH₂OPh, X is S, and (Z¹)n is 5-Me. |
| Table 1-456 | R¹ is Me, R² is Me, W¹ is O, G is C(O)CH₂OPh, X is O, and (Z¹)n is 5-Me. |
| Table 1-457 | R¹ is Me, R² is Me, W¹ is O, G is C(O)CH₂OPh, X is S, and (Z¹)n is 6-Me. |
| Table 1-458 | R¹ is Me, R² is Me, W¹ is O, G is C(O)CH₂OPh, X is O, and (Z¹)n is 6-Me. |
| Table 1-459 | R¹ is Me, R² is Me, W¹ is O, G is C(O)CH₂OPh, X is S, and (Z¹)n is 7-Me. |
| Table 1-460 | R¹ is Me, R² is Me, W¹ is O, G is C(O)CH₂OPh, X is O, and (Z¹)n is 7-Me. |
| Table 1-461 | R¹ is Me, R² is Me, W¹ is O, G is C(O)CH₂OPh, X is S, and (Z¹)n is 5-OMe. |
| Table 1-462 | R¹ is Me, R² is Me, W¹ is O, G is C(O)CH₂OPh, X is O, and (Z¹)n is 5-OMe. |
| Table 1-463 | R¹ is Me, R² is Me, W¹ is O, G is C(O)CH₂OPh, X is S, and (Z¹)n is 6-OMe. |
| Table 1-464 | R¹ is Me, R² is Me, W¹ is O, G is C(O)CH₂OPh, X is O, and (Z¹)n is 6-OMe. |
| Table 1-465 | R¹ is Me, R² is Me, W¹ is O, G is C(O)CH₂OPh, X is S, and (Z¹)n is 7-OMe. |
| Table 1-466 | R¹ is Me, R² is Me, W¹ is O, G is C(O)CH₂OPh, X is O, and (Z¹)n is 7-OMe. |
| Table 1-467 | R¹ is Et, R² is Me, W¹ is O, G is H, X is S, and (Z¹)n is "-". |
| Table 1-468 | R¹ is Et, R² is Me, W¹ is O, G is H, X is O, and (Z¹)n is "-". |
| Table 1-469 | R¹ is Et, R² is Me, W¹ is O, G is H, X is S, and (Z¹)n is 4-F. |
| Table 1-470 | R¹ is Et, R² is Me, W¹ is O, G is H, X is O, and (Z¹)n is 4-F. |
| Table 1-471 | R¹ is Et, R² is Me, W¹ is O, G is H, X is S, and (Z¹)n is 5-F. |
| Table 1-472 | R¹ is Et, R² is Me, W¹ is O, G is H, X is O, and (Z¹)n is 5-F. |
| Table 1-473 | R¹ is Et, R² is Me, W¹ is O, G is H, X is S, and (Z¹)n is 6-F. |
| Table 1-474 | R¹ is Et, R² is Me, W¹ is O, G is H, X is O, and (Z¹)n is 6-F. |
| Table 1-475 | R¹ is Et, R² is Me, W¹ is O, G is H, X is S, and (Z¹)n is 7-F. |
| Table 1-476 | R¹ is Et, R² is Me, W¹ is O, G is H, X is O, and (Z¹)n is 7-F. |
| Table 1-477 | R¹ is Et, R² is Me, W¹ is O, G is H, X is S, and (Z¹)n is 4-Cl. |
| Table 1-478 | R¹ is Et, R² is Me, W¹ is O, G is H, X is O, and (Z¹)n is 4-Cl. |
| Table 1-479 | R¹ is Et, R² is Me, W¹ is O, G is H, X is S, and (Z¹)n is 5-Cl. |
| Table 1-480 | R¹ is Et, R² is Me, W¹ is O, G is H, X is O, and (Z¹)n is 5-Cl. |
| Table 1-481 | R¹ is Et, R² is Me, W¹ is O, G is H, X is S, and (Z¹)n is 6-Cl. |
| Table 1-482 | R¹ is Et, R² is Me, W¹ is O, G is H, X is O, and (Z¹)n is 6-Cl. |
| Table 1-483 | R¹ is Et, R² is Me, W¹ is O, G is H, X is S, and (Z¹)n is 7-Cl. |
| Table 1-484 | R¹ is Et, R² is Me, W¹ is O, G is H, X is O, and (Z¹)n is 7-Cl. |
| Table 1-485 | R¹ is Et, R² is Me, W¹ is O, G is H, X is S, and (Z¹)n is 4-Me. |
| Table 1-486 | R¹ is Et, R² is Me, W¹ is O, G is H, X is O, and (Z¹)n is 4-Me. |
| Table 1-487 | R¹ is Et, R² is Me, W¹ is O, G is H, X is S, and (Z¹)n is 5-Me. |
| Table 1-488 | R¹ is Et, R² is Me, W¹ is O, G is H, X is O, and (Z¹)n is 5-Me. |
| Table 1-489 | R¹ is Et, R² is Me, W¹ is O, G is H, X is S, and (Z¹)n is 6-Me. |
| Table 1-490 | R¹ is Et, R² is Me, W¹ is O, G is H, X is O, and (Z¹)n is 6-Me. |
| Table 1-491 | R¹ is Et, R² is Me, W¹ is O, G is H, X is S, and (Z¹)n is 7-Me. |
| Table 1-492 | R¹ is Et, R² is Me, W¹ is O, G is H, X is O, and (Z¹)n is 7-Me. |
| Table 1-493 | R¹ is Et, R² is Me, W¹ is O, G is H, X is S, and (Z¹)n is 4-OMe. |
| Table 1-494 | R¹ is Et, R² is Me, W¹ is O, G is H, X is O, and (Z¹)n is 4-OMe. |
| Table 1-495 | R¹ is Et, R² is Me, W¹ is O, G is H, X is S, and (Z¹)n is 5-OMe. |
| Table 1-496 | R¹ is Et, R² is Me, W¹ is O, G is H, X is O, and (Z¹)n is 5-OMe. |
| Table 1-497 | R¹ is Et, R² is Me, W¹ is O, G is H, X is S, and (Z¹)n is 6-OMe. |
| Table 1-498 | R¹ is Et, R² is Me, W¹ is O, G is H, X is O, and (Z¹)n is 6-OMe. |
| Table 1-499 | R¹ is Et, R² is Me, W¹ is O, G is H, X is S, and (Z¹)n is 7-OMe. |
| Table 1-500 | R¹ is Et, R² is Me, W¹ is O, G is H, X is O, and (Z¹)n is 7-OMe. |
| Table 1-501 | R¹ is CH₂CH=CH₂, R² is Me, W¹ is O, G is H, X is S, and (Z¹)n is "-". |
| Table 1-502 | R¹ is CH₂CH=CH₂, R² is Me, W¹ is O, G is H, X is O, and (Z¹)n is "-". |
| Table 1-503 | R¹ is CH₂CH=CH₂, R² is Me, W¹ is O, G is H, X is S, and (Z¹)n is 4-F. |
| Table 1-504 | R¹ is CH₂CH=CH₂, R² is Me, W¹ is O, G is H, X is O, and (Z¹)n is 4-F. |
| Table 1-505 | R¹ is CH₂CH=CH₂, R² is Me, W¹ is O, G is H, X is S, and (Z¹)n is 5-F. |
| Table 1-506 | R¹ is CH₂CH=CH₂, R² is Me, W¹ is O, G is H, X is O, and (Z¹)n is 5-F. |
| Table 1-507 | R¹ is CH₂CH=CH₂, R² is Me, W¹ is O, G is H, X is S, and (Z¹)n is 6-F. |
| Table 1-508 | R¹ is CH₂CH=CH₂, R² is Me, W¹ is O, G is H, X is O, and (Z¹)n is 6-F. |
| Table 1-509 | R¹ is CH₂CH=CH₂, R² is Me, W¹ is O, G is H, X is S, and (Z¹)n is 7-F. |
| Table 1-510 | R¹ is CH₂CH=CH₂, R² is Me, W¹ is O, G is H, X is O, and (Z¹)n is 7-F. |
| Table 1-511 | R¹ is CH₂CH=CH₂, R² is Me, W¹ is O, G is H, X is S, and (Z¹)n is 4-Cl. |
| Table 1-512 | R¹ is CH₂CH=CH₂, R² is Me, W¹ is O, G is H, X is O, and (Z¹)n is 4-Cl. |
| Table 1-513 | R¹ is CH₂CH=CH₂, R² is Me, W¹ is O, G is H, X is S, and (Z¹)n is 5-Cl. |
| Table 1-514 | R¹ is CH₂CH=CH₂, R² is Me, W¹ is O, G is H, X is O, and (Z¹)n is 5-Cl. |
| Table 1-515 | R¹ is CH₂CH=CH₂, R² is Me, W¹ is O, G is H, X is S, and (Z¹)n is 6-Cl. |
| Table 1-516 | R¹ is CH₂CH=CH₂, R² is Me, W¹ is O, G is H, X is O, and (Z¹)n is 6-Cl. |
| Table 1-517 | R¹ is CH₂CH=CH₂, R² is Me, W¹ is O, G is H, X is S, and (Z¹)n is 7-Cl. |
| Table 1-518 | R¹ is CH₂CH=CH₂, R² is Me, W¹ is O, G is H, X is O, and (Z¹)n is 7-Cl. |
| Table 1-519 | R¹ is CH₂CH=CH₂, R² is Me, W¹ is O, G is H, X is S, and (Z¹)n is 4-Me. |
| Table 1-520 | R¹ is CH₂CH=CH₂, R² is Me, W¹ is O, G is H, X is O, and (Z¹)n is 4-Me. |
| Table 1-521 | R¹ is CH₂CH=CH₂, R² is Me, W¹ is O, G is H, X is S, and (Z¹)n is 5-Me. |
| Table 1-522 | R¹ is CH₂CH=CH₂, R² is Me, W¹ is O, G is H, X is O, and (Z¹)n is 5-Me. |
| Table 1-523 | R¹ is CH₂CH=CH₂, R² is Me, W¹ is O, G is H, X is S, and (Z¹)n is 6-Me. |
| Table 1-524 | R¹ is CH₂CH=CH₂, R² is Me, W¹ is O, G is H, X is O, and (Z¹)n is 6-Me. |
| Table 1-525 | R¹ is CH₂CH=CH₂, R² is Me, W¹ is O, G is H, X is S, and (Z¹)n is 7-Me. |
| Table 1-526 | R¹ is CH₂CH=CH₂, R² is Me, W¹ is O, G is H, X is O, and (Z¹)n is 7-Me. |
| Table 1-527 | R¹ is CH₂CH=CH₂, R² is Me, W¹ is O, G is H, X is S, and (Z¹)n is 4-OMe. |
| Table 1-528 | R¹ is CH₂CH=CH₂, R² is Me, W¹ is O, G is H, X is O, and (Z¹)n is 4-OMe. |
| Table 1-529 | R¹ is CH₂CH=CH₂, R² is Me, W¹ is O, G is H, X is S, and (Z¹)n is 5-OMe. |
| Table 1-530 | R¹ is CH₂CH=CH₂, R² is Me, W¹ is O, G is H, X is O, and (Z¹)n is 5-OMe. |
| Table 1-531 | R¹ is CH₂CH=CH₂, R² is Me, W¹ is O, G is H, X is S, and (Z¹)n is 6-OMe. |
| Table 1-532 | R¹ is CH₂CH=CH₂, R² is Me, W¹ is O, G is H, X is O, and (Z¹)n is 6-OMe. |
| Table 1-533 | R¹ is CH₂CH=CH₂, R² is Me, W¹ is O, G is H, X is S, and (Z¹)n is 7-OMe. |
| Table 1-534 | R¹ is CH₂CH=CH₂, R² is Me, W¹ is O, G is H, X is O, and (Z¹)n is 7-OMe. |
| Table 1-535 | R¹ is CH₂C≡CH, R² is Me, W¹ is O, G is H, X is S, and (Z¹)n is "-". |
| Table 1-536 | R¹ is CH₂C≡CH, R² is Me, W¹ is O, G is H, X is O, and (Z¹)n is "-". |
| Table 1-537 | R¹ is CH₂C≡CH, R² is Me, W¹ is O, G is H, X is S, and (Z¹)n is 4-F. |
| Table 1-538 | R¹ is CH₂C≡CH, R² is Me, W¹ is O, G is H, X is O, and (Z¹)n is 4-F. |
| Table 1-539 | R¹ is CH₂C≡CH, R² is Me, W¹ is O, G is H, X is S, and (Z¹)n is 5-F. |
| Table 1-540 | R¹ is CH₂C≡CH, R² is Me, W¹ is O, G is H, X is O, and (Z¹)n is 5-F. |
| Table 1-541 | R¹ is CH₂C≡CH, R² is Me, W¹ is O, G is H, X is S, and (Z¹)n is 6-F. |
| Table 1-542 | R¹ is CH₂C≡CH, R² is Me, W¹ is O, G is H, X is O, and (Z¹)n is 6-F. |
| Table 1-543 | R¹ is CH₂C≡CH, R² is Me, W¹ is O, G is H, X is S, and (Z¹)n is 7-F. |
| Table 1-544 | R¹ is CH₂C≡CH, R² is Me, W¹ is O, G is H, X is O, and (Z¹)n is 7-F. |
| Table 1-545 | R¹ is CH₂C≡CH, R² is Me, W¹ is O, G is H, X is S, and (Z¹)n is 4-Cl. |
| Table 1-546 | R¹ is CH₂C≡CH, R² is Me, W¹ is O, G is H, X is O, and (Z¹)n is 4-Cl. |
| Table 1-547 | R¹ is CH₂C≡CH, R² is Me, W¹ is O, G is H, X is S, and (Z¹)n is 5-Cl. |
| Table 1-548 | R¹ is CH₂C≡CH, R² is Me, W¹ is O, G is H, X is O, and (Z¹)n is 5-Cl. |
| Table 1-549 | R¹ is CH₂C≡CH, R² is Me, W¹ is O, G is H, X is S, and (Z¹)n is 6-Cl. |
| Table 1-550 | R¹ is CH₂C≡CH, R² is Me, W¹ is O, G is H, X is O, and (Z¹)n is 6-Cl. |
| Table 1-551 | R¹ is CH₂C≡CH, R² is Me, W¹ is O, G is H, X is S, and (Z¹)n is 7-Cl. |
| Table 1-552 | R¹ is CH₂C≡CH, R² is Me, W¹ is O, G is H, X is O, and (Z¹)n is 7-Cl. |
| Table 1-553 | R¹ is CH₂C≡CH, R² is Me, W¹ is O, G is H, X is S, and (Z¹)n is 4-Me. |
| Table 1-554 | R¹ is CH₂C≡CH, R² is Me, W¹ is O, G is H, X is O, and (Z¹)n is 4-Me. |
| Table 1-555 | R¹ is CH₂C≡CH, R² is Me, W¹ is O, G is H, X is S, and (Z¹)n is 5-Me. |
| Table 1-556 | R¹ is CH₂C≡CH, R² is Me, W¹ is O, G is H, X is O, and (Z¹)n is 5-Me. |
| Table 1-557 | R¹ is CH₂C≡CH, R² is Me, W¹ is O, G is H, X is S, and (Z¹)n is 6-Me. |
| Table 1-558 | R¹ is CH₂C≡CH, R² is Me, W¹ is O, G is H, X is O, and (Z¹)n is 6-Me. |
| Table 1-559 | R¹ is CH₂C≡CH, R² is Me, W¹ is O, G is H, X is S, and (Z¹)n is 7-Me. |
| Table 1-560 | R¹ is CH₂C≡CH, R² is Me, W¹ is O, G is H, X is O, and (Z¹)n is 7-Me. |
| Table 1-561 | R¹ is CH₂C≡CH, R² is Me, W¹ is O, G is H, X is S, and (Z¹)n is 4-OMe. |
| Table 1-562 | R¹ is CH₂C≡CH, R² is Me, W¹ is O, G is H, X is O, and (Z¹)n is 4-OMe |
| Table 1-563 | R¹ is CH₂C≡CH, R² is Me, W¹ is O, G is H, X is S, and (Z¹)n is 5-OMe. |
| Table 1-564 | R¹ is CH₂C≡CH, R² is Me, W¹ is O, G is H, X is O, and (Z¹)n is 5-OMe |
| Table 1-565 | R¹ is CH₂C≡CH, R² is Me, W¹ is O, G is H, X is S, and (Z¹)n is 6-OMe. |
| Table 1-566 | R¹ is CH₂C≡CH, R² is Me, W¹ is O, G is H, X is O, and (Z¹)n is 6-OMe |
| Table 1-567 | R¹ is CH₂C≡CH, R² is Me, W¹ is O, G is H, X is S, and (Z¹)n is 7-OMe. |
| Table 1-568 | R¹ is CH₂C≡CH, R² is Me, W¹ is O, G is H, X is O, and (Z¹)n is 7-OMe |
| Table 1-569 | R¹ is Me, R² is Me, W¹ is O, G is H, X is S, and (Z¹)n is 5-OEt. |
| Table 1-570 | R¹ is Me, R² is Me, W¹ is O, G is H, X is O, and (Z¹)n is 5-OEt. |
| Table 1-571 | R¹ is Me, R² is Me, W¹ is O, G is H, X is S, and (Z¹)n is 6-OEt. |
| Table 1-572 | R¹ is Me, R² is Me, W¹ is O, G is H, X is O, and (Z¹)n is 6-OEt. |
| Table 1-573 | R¹ is Me, R² is Me, W¹ is O, G is H, X is S, and (Z¹)n is 7-OEt. |
| Table 1-574 | R¹ is Me, R² is Me, W¹ is O, G is H, X is O, and (Z¹)n is 7-OEt. |
| Table 1-575 | R¹ is Me, R² is Me, W¹ is O, G is H, X is S, and (Z¹)n is 5-OPr-n. |
| Table 1-576 | R¹ is Me, R² is Me, W¹ is O, G is H, X is O, and (Z¹)n is 5-OPr-n. |
| Table 1-577 | R¹ is Me, R² is Me, W¹ is O, G is H, X is S, and (Z¹)n is 6-OPr-n. |
| Table 1-578 | R¹ is Me, R² is Me, W¹ is O, G is H, X is O, and (Z¹)n is 6-OPr-n. |
| Table 1-579 | R¹ is Me, R² is Me, W¹ is O, G is H, X is S, and (Z¹)n is 7-OPr-n. |
| Table 1-580 | R¹ is Me, R² is Me, W¹ is O, G is H, X is O, and (Z¹)n is 7-OPr-n. |
| Table 1-581 | R¹ is Me, R² is Me, W¹ is O, G is H, X is S, and (Z¹)n is 5-OPr-i. |
| Table 1-582 | R¹ is Me, R² is Me, W¹ is O, G is H, X is O, and (Z¹)n is 5-OPr-i. |
| Table 1-583 | R¹ is Me, R² is Me, W¹ is O, G is H, X is S, and (Z¹)n is 6-OPr-i. |
| Table 1-584 | R¹ is Me, R² is Me, W¹ is O, G is H, X is O, and (Z¹)n is 6-OPr-i. |
| Table 1-585 | R¹ is Me, R² is Me, W¹ is O, G is H, X is S, and (Z¹)n is 7-OPr-i. |
| Table 1-586 | R¹ is Me, R² is Me, W¹ is O, G is H, X is O, and (Z¹)n is 7-OPr-i. |
| Table 1-587 | R¹ is Me, R² is Me, W¹ is O, G is H, X is S, and (Z¹)n is 5-OBu-n. |
| Table 1-588 | R¹ is Me, R² is Me, W¹ is O, G is H, X is O, and (Z¹)n is 5-OBu-n. |
| Table 1-589 | R¹ is Me, R² is Me, W¹ is O, G is H, X is S, and (Z¹)n is 6-OBu-n. |
| Table 1-590 | R¹ is Me, R² is Me, W¹ is O, G is H, X is O, and (Z¹)n is 6-OBu-n. |
| Table 1-591 | R¹ is Me, R ² is Me, W¹ is O, G is H, X is S, and (Z¹)n is 7-OBu-n. |
| Table 1-592 | R¹ is Me, R² is Me, W¹ is O, G is H, X is O, and (Z¹)n is 7-OBu-n. |
| Table 1-593 | R¹ is Me, R² is Me, W¹ is O, G is H, X is S, and (Z¹)n is 5-OBu-i. |
| Table 1-594 | R¹ is Me, R² is Me, W¹ is O, G is H, X is O, and (Z¹)n is 5-OBu-i. |
| Table 1-595 | R¹ is Me, R² is Me, W¹ is O, G is H, X is S, and (Z¹)n is 6-OBu-i. |
| Table 1-596 | R¹ is Me, R² is Me, W¹ is O, G is H, X is O, and (Z¹)n is 6-OBu-i. |
| Table 1-597 | R¹ is Me, R² is Me, W¹ is O, G is H, X is S, and (Z¹)n is 7-OBu-i. |
| Table 1-598 | R¹ is Me, R² is Me, W¹ is O, G is H, X is O, and (Z¹)n is 7-OBu-i. |
| Table 1-599 | R¹ is Me, R² is Me, W¹ is O, G is H, X is S, and (Z¹)n is 5-OBu-sec. |
| Table 1-600 | R¹ is Me, R² is Me, W¹ is O, G is H, X is O, and (Z¹)n is 5-OBu-sec. |
| Table 1-601 | R¹ is Me, R² is Me, W¹ is O, G is H, X is S, and (Z¹)n is 6-OBu-sec. |
| Table 1-602 | R¹ is Me, R² is Me, W¹ is O, G is H, X is O, and (Z¹)n is 6-OBu-sec. |
| Table 1-603 | R¹ is Me, R² is Me, W¹ is O, G is H, X is S, and (Z¹)n is 7-OBu-sec. |
| Table 1-604 | R¹ is Me, R² is Me, W¹ is O, G is H, X is O, and (Z¹)n is 7-OBu-sec. |
| Table 1-605 | R¹ is Me, R² is Me, W¹ is O, G is H, X is S, and (Z¹)n is 5-OBu-tert. |
| Table 1-606 | R¹ is Me, R² is Me, W¹ is O, G is H, X is O, and (Z¹)n is 5-OBu-tert. |
| Table 1-607 | R¹ is Me, R² is Me, W¹ is O, G is H, X is S, and (Z¹)n is 6-OBu-tert. |
| Table 1-608 | R¹ is Me, R² is Me, W¹ is O, G is H, X is O, and (Z¹)n is 6-OBu-tert. |
| Table 1-609 | R¹ is Me, R² is Me, W¹ is O, G is H, X is S, and (Z¹)n is 7-OBu-tert. |
| Table 1-610 | R¹ is Me, R² is Me, W¹ is O, G is H, X is O, and (Z¹)n is 7-OBu-tert. |
| Table 1-611 | R¹ is Me, R² is Me, W¹ is O, G is H, X is S, and (Z¹)n is 5-OCH₂CH=CH₂. |
| Table 1-612 | R¹ is Me, R² is Me, W¹ is O, G is H, X is O, and (Z¹)n is 5-OCH₂CH=CH₂. |
| Table 1-613 | R¹ is Me, R² is Me, W¹ is O, G is H, X is S, and (Z¹)n is 6-OCH₂CH=CH₂. |
| Table 1-614 | R¹ is Me, R² is Me, W¹ is O, G is H, X is O, and (Z¹)n is 6-OCH₂CH=CH₂. |
| Table 1-615 | R¹ is Me, R² is Me, W¹ is O, G is H, X is S, and (Z¹)n is 7-OCH₂CH=CH₂. |
| Table 1-616 | R¹ is Me, R² is Me, W¹ is O, G is H, X is O, and (Z¹)n is 7-OCH₂CH=CH₂. |
| Table 1-617 | R¹ is Me, R² is Me, W¹ is O, G is H, X is S, and (Z¹)n is 5-OCH₂C≡CH. |
| Table 1-618 | R¹ is Me, R² is Me, W¹ is O, G is H, X is O, and (Z¹)n is 5-OCH₂C≡CH. |
| Table 1-619 | R¹ is Me, R² is Me, W¹ is O, G is H, X is S, and (Z¹)n is 6-OCH₂C≡CH. |
| Table 1-620 | R¹ is Me, R² is Me, W¹ is O, G is H, X is O, and (Z¹)n is 6-OCH₂C≡CH. |
| Table 1-621 | R¹ is Me, R² is Me, W¹ is O, G is H, X is S, and (Z¹)n is 7-OCH₂C≡CH. |
| Table 1-622 | R¹ is Me, R² is Me, W¹ is O, G is H, X is O, and (Z¹)n is 7-OCH₂C≡CH. |
| Table 1-623 | R¹ is Me, R² is Me, W¹ is O, G is H, X is S, and (Z¹)n is 5-OCHF₂. |
| Table 1-624 | R¹ is Me, R² is Me, W¹ is O, G is H, X is O, and (Z¹)n is 5-OCHF₂. |
| Table 1-625 | R¹ is Me, R² is Me, W¹ is O, G is H, X is S, and (Z¹)n is 6-OCHF₂. |
| Table 1-626 | R¹ is Me, R² is Me, W¹ is O, G is H, X is O, and (Z¹)n is 6-OCHF₂. |
| Table 1-627 | R¹ is Me, R² is Me, W¹ is O, G is H, X is S, and (Z¹)n is 7-OCHF₂. |
| Table 1-628 | R¹ is Me, R² is Me, W¹ is O, G is H, X is O, and (Z¹)n is 7-OCHF₂. |
| Table 1-629 | R¹ is Me, R² is Me, W¹ is O, G is H, X is S, and (Z¹)n is 5-OCF₃. |
| Table 1-630 | R¹ is Me, R² is Me, W¹ is O, G is H, X is O, and (Z¹)n is 5-OCF₃. |
| Table 1-631 | R¹ is Me, R² is Me, W¹ is O, G is H, X is S, and (Z¹)n is 6-OCF₃. |
| Table 1-632 | R¹ is Me, R² is Me, W¹ is O, G is H, X is O, and (Z¹)n is 6-OCF₃. |
| Table 1-633 | R¹ is Me, R² is Me, W¹ is O, G is H, X is S, and (Z¹)n is 7-OCF₃. |
| Table 1-634 | R¹ is Me, R² is Me, W¹ is O, G is H, X is O, and (Z¹)n is 7-OCF₃. |
| Table 1-635 | R¹ is Me, R² is Me, W¹ is O, G is H, X is S, and (Z¹)n is 5-OCH₂CH₂F. |
| Table 1-636 | R¹ is Me, R² is Me, W¹ is O, G is H, X is O, and (Z¹)n is 5-OCH₂CH₂F. |
| Table 1-637 | R¹ is Me, R² is Me, W¹ is O, G is H, X is S, and (Z¹)n is 6-OCH₂CH₂F. |
| Table 1-638 | R¹ is Me, R² is Me, W¹ is O, G is H, X is O, and (Z¹)n is 6-OCH₂CH₂F. |
| Table 1-639 | R¹ is Me, R² is Me, W¹ is O, G is H, X is S, and (Z¹)n is 7-OCH₂CH₂F. |
| Table 1-640 | R¹ is Me, R² is Me, W¹ is O, G is H, X is O, and (Z¹)n is 7-OCH₂CH₂F. |
| Table 1-641 | R¹ is Me, R² is Me, W¹ is O, G is H, X is S, and (Z¹)n is 5-OCH₂CHF₂. |
| Table 1-642 | R¹ is Me, R² is Me, W¹ is O, G is H, X is O, and (Z¹)n is 5-OCH₂CHF₂. |
| Table 1-643 | R¹ is Me, R² is Me, W¹ is O, G is H, X is S, and (Z¹)n is 6-OCH₂CHF₂. |
| Table 1-644 | R¹ is Me, R² is Me, W¹ is O, G is H, X is O, and (Z¹)n is 6-OCH₂CHF₂. |
| Table 1-645 | R¹ is Me, R² is Me, W¹ is O, G is H, X is S, and (Z¹)n is 7-OCH₂CHF₂. |
| Table 1-646 | R¹ is Me, R² is Me, W¹ is O, G is H, X is O, and (Z¹)n is 7-OCH₂CHF₂. |
| Table 1-647 | R¹ is Me, R² is Me, W¹ is O, G is H, X is S, and (Z¹)n is 5-OCH₂CF₃. |
| Table 1-648 | R¹ is Me, R² is Me, W¹ is O, G is H, X is O, and (Z¹)n is 5-OCH₂CF₃. |
| Table 1-649 | R¹ is Me, R² is Me, W¹ is O, G is H, X is S, and (Z¹)n is 6-OCH₂CF₃. |
| Table 1-650 | R¹ is Me, R² is Me, W¹ is O, G is H, X is O, and (Z¹)n is 6-OCH₂CF₃. |
| Table 1-651 | R¹ is Me, R² is Me, W¹ is O, G is H, X is S, and (Z¹)n is 7-OCH₂CF₃. |
| Table 1-652 | R¹ is Me, R² is Me, W¹ is O, G is H, X is O, and (Z¹)n is 7-OCH₂CF₃. |
| Table 1-653 | R¹ is Me, R² is Me, W¹ is O, G is H, X is S, and (Z¹)n is 5-OCH₂CH=C(F)₂. |
| Table 1-654 | R¹ is Me, R² is Me, W¹ is O, G is H, X is O, and (Z¹)n is 5-OCH₂CH=C(F)₂. |
| Table 1-655 | R¹ is Me, R² is Me, W¹ is O, G is H, X is S, and (Z¹)n is 6-OCH₂CH=C(F)₂. |
| Table 1-656 | R¹ is Me, R² is Me, W¹ is O, G is H, X is O, and (Z¹)n is 6-OCH₂CH=C(F)₂. |
| Table 1-657 | R¹ is Me, R² is Me, W¹ is O, G is H, X is S, and (Z¹)n is 7-OCH₂CH=C(F)₂. |
| Table 1-658 | R¹ is Me, R² is Me, W¹ is O, G is H, X is O, and (Z¹)n is 7-OCH₂CH=C(F)₂. |
| Table 1-659 | R¹ is Me, R² is Me, W¹ is O, G is H, X is S, and (Z¹)n is 5-OCH₂CH=C(Cl)₂. |
| Table 1-660 | R¹ is Me, R² is Me, W¹ is O, G is H, X is O, and (Z¹)n is 5-OCH₂CH=C(Cl)₂ |
| Table 1-661 | R¹ is Me, R² is Me, W¹ is O, G is H, X is S, and (Z¹)n is 6-OCH₂CH=C(Cl)₂. |
| Table 1-662 | R¹ is Me, R² is Me, W¹ is O, G is H, X is O, and (Z¹)n is 6-OCH₂CH=C(Cl)₂ |
| Table 1-663 | R¹ is Me, R² is Me, W¹ is O, G is H, X is S, and (Z¹)n is 7-OCH₂CH=C(Cl)₂. |
| Table 1-664 | R¹ is Me, R² is Me, W¹ is O, G is H, X is O, and (Z¹)n is 7-OCH₂CH=C(Cl)₂ |
| Table 1-665 | R¹ is Me, R² is Me, W¹ is O, G is H, X is S, and (Z¹)n is 5-OCH₂C≡CCl. |
| Table 1-666 | R¹ is Me, R² is Me, W¹ is O, G is H, X is O, and (Z¹)n is 5-OCH₂C≡CCl. |
| Table 1-667 | R¹ is Me, R² is Me, W¹ is O, G is H, X is S, and (Z¹)n is 6-OCH₂C≡CCl. |
| Table 1-668 | R¹ is Me, R² is Me, W¹ is O, G is H, X is O, and (Z¹)n is 6-OCH₂C≡CCl. |
| Table 1-669 | R¹ is Me, R² is Me, W¹ is O, G is H, X is S, and (Z¹)n is 7-OCH₂C≡CCl. |
| Table 1-670 | R¹ is Me, R² is Me, W¹ is O, G is H, X is O, and (Z¹)n is 7-OCH₂C≡CCl. |
| Table 1-671 | R¹ is Me, R² is Me, W¹ is O, G is H, X is S, and (Z¹)n is 5-OCH₂C≡CBr. |
| Table 1-672 | R¹ is Me, R² is Me, W¹ is O, G is H, X is O, and (Z¹)n is 5-OCH₂C≡CBr. |
| Table 1-673 | R¹ is Me, R² is Me, W¹ is O, G is H, X is S, and (Z¹)n is 6-OCH₂C≡CBr. |
| Table 1-674 | R¹ is Me, R² is Me, W¹ is O, G is H, X is O, and (Z¹)n is 6-OCH₂C≡CBr. |
| Table 1-675 | R¹ is Me, R² is Me, W¹ is O, G is H, X is S, and (Z¹)n is 7-OCH₂C≡CBr. |
| Table 1-676 | R¹ is Me, R² is Me, W¹ is O, G is H, X is O, and (Z¹)n is 7-OCH₂C≡CBr. |
| Table 1-677 | R¹ is Me, R² is Me, W¹ is O, G is H, X is S, and (Z¹)n is 5-OCH₂(T-1-1). |
| Table 1-678 | R¹ is Me, R² is Me, W¹ is O, G is H, X is O, and (Z¹)n is 5-OCH₂(T-1-1). |
| Table 1-679 | R¹ is Me, R² is Me, W¹ is O, G is H, X is S, and (Z¹)n is 6-OCH₂(T-1-1). |
| Table 1-680 | R¹ is Me, R² is Me, W¹ is O, G is H, X is O, and (Z¹)n is 6-OCH₂(T-1-1). |
| Table 1-681 | R¹ is Me, R² is Me, W¹ is O, G is H, X is S, and (Z¹)n is 7-OCH₂(T-1-1). |
| Table 1-682 | R¹ is Me, R² is Me, W¹ is O, G is H, X is O, and (Z¹)n is 7-OCH₂(T-1-1). |
| Table 1-683 | R¹ is Me, R² is Me, W¹ is O, G is H, X is S, and (Z¹)n is 5-OCH₂(U-la). |
| Table 1-684 | R¹ is Me, R² is Me, W¹ is O, G is H, X is O, and (Z¹)n is 5-OCH₂(U-1a). |
| Table 1-685 | R¹ is Me, R² is Me, W¹ is O, G is H, X is S, and (Z¹)n is 6-OCH₂(U-1a). |
| Table 1-686 | R¹ is Me, R² is Me, W¹ is O, G is H, X is O, and (Z¹)n is 6-OCH₂(U-1a). |
| Table 1-687 | R¹ is Me, R² is Me, W¹ is O, G is H, X is S, and (Z¹)n is 7-OCH₂(U-1a). |
| Table 1-688 | R¹ is Me, R² is Me, W¹ is O, G is H, X is O, and (Z¹)n is 7-OCH₂(U-1a). |
| Table 1-689 | R¹ is Me, R² is Me, W¹ is O, G is H, X is S, and (Z¹)n is 5-OCH₂CH₂OMe. |
| Table 1-690 | R¹ is Me, R² is Me, W¹ is O, G is H, X is O, and (Z¹)n is 5-OCH₂CH₂OMe. |
| Table 1-691 | R¹ is Me, R ² is Me, W¹ is O, G is H, X is S, and (Z¹)n is 6-OCH₂CH₂OMe. |
| Table 1-692 | R¹ is Me, R² is Me, W¹ is O, G is H, X is O, and (Z¹)n is 6-OCH₂CH₂OMe. |
| Table 1-693 | R¹ is Me, R² is Me, W¹ is O, G is H, X is S, and (Z¹)n is 7-OCH₂CH₂OMe. |
| Table 1-694 | R¹ is Me, R² is Me, W¹ is O, G is H, X is O, and (Z¹)n is 7-OCH₂CH₂OMe. |
| Table 1-695 | R¹ is Me, R² is Me, W¹ is O, G is H, X is S, and (Z¹)n is 5-OCH₂CH₂SMe. |
| Table 1-696 | R¹ is Me, R² is Me, W¹ is O, G is H, X is O, and (Z¹)n is 5-OCH₂CH₂SMe. |
| Table 1-697 | R¹ is Me, R² is Me, W¹ is O, G is H, X is S, and (Z¹)n is 6-OCH₂CH₂SMe. |
| Table 1-698 | R¹ is Me, R² is Me, W¹ is O, G is H, X is O, and (Z¹)n is 6-OCH₂CH₂SMe. |
| Table 1-699 | R¹ is Me, R² is Me, W¹ is O, G is H, X is S, and (Z¹)n is 7-OCH₂CH₂SMe. |
| Table 1-700 | R¹ is Me, R² is Me, W¹ is O, G is H, X is O, and (Z¹)n is 7-OCH₂CH₂SMe. |
| Table 1-701 | R¹ is Me, R² is Me, W¹ is O, G is H, X is S, and (Z¹)n is 5-OCH₂CH₂S(O)Me. |
| Table 1-702 | R¹ is Me, R² is Me, W¹ is O, G is H, X is O, and (Z¹)n is 5-OCH₂CH₂S(O)Me. |
| Table 1-703 | R¹ is Me, R² is Me, W¹ is O, G is H, X is S, and (Z¹)n is 6-OCH₂CH₂S(O)Me. |
| Table 1-704 | R¹ is Me, R² is Me, W¹ is O, G is H, X is O, and (Z¹)n is 6-OCH₂CH₂S(O)Me. |
| Table 1-705 | R¹ is Me, R² is Me, W¹ is O, G is H, X is S, and (Z¹)n is 7-OCH₂CH₂S(O)Me. |
| Table 1-706 | R¹ is Me, R² is Me, W¹ is O, G is H, X is O, and (Z¹)n is 7-OCH₂CH₂S(O)Me. |
| Table 1-707 | R¹ is Me, R² is Me, W¹ is O, G is H, X is S, and (Z¹)n is 5-OCH₂CH₂S(O)₂Me. |
| Table 1-708 | R¹ is Me, R² is Me, W¹ is O, G is H, X is O, and (Z¹)n is 5-OCH₂CH₂S(O)₂Me |
| Table 1-709 | R¹ is Me, R² is Me, W¹ is O, G is H, X is S, and (Z¹)n is 6-OCH₂CH₂S(O)₂Me. |
| Table 1-710 | R¹ is Me, R² is Me, W¹ is O, G is H, X is O, and (Z¹)n is 6-OCH₂CH₂S(O)₂Me |
| Table 1-711 | R¹ is Me, R² is Me, W¹ is O, G is H, X is S, and (Z¹)n is 7-OCH₂CH₂S(O)₂Me. |
| Table 1-712 | R¹ is Me, R² is Me, W¹ is O, G is H, X is O, and (Z¹)n is 7-OCH₂CH₂S(O)₂Me |
| Table 1-713 | R¹ is Me, R² is Me, W¹ is O, G is H, X is S, and (Z¹)n is 5-NO₂. |
| Table 1-714 | R¹ is Me, R² is Me, W¹ is O, G is H, X is O, and (Z¹)n is 5-NO₂. |
| Table 1-715 | R¹ is Me, R² is Me, W¹ is O, G is H, X is S, and (Z¹)n is 6-NO₂. |
| Table 1-716 | R¹ is Me, R² is Me, W¹ is O, G is H, X is O, and (Z¹)n is 6-NO₂. |
| Table 1-717 | R¹ is Me, R² is Me, W¹ is O, G is H, X is S, and (Z¹)n is 7-NO₂. |
| Table 1-718 | R¹ is Me, R² is Me, W¹ is O, G is H, X is O, and (Z¹)n is 7-NO₂. |
| Table 1-719 | R¹ is Me, R² is Me, W¹ is O, G is H, X is S, and (Z¹)n is 5-NH₂. |
| Table 1-720 | R¹ is Me, R² is Me, W¹ is O, G is H, X is O, and (Z¹)n is 5-NH₂. |
| Table 1-721 | R¹ is Me, R² is Me, W¹ is O, G is H, X is S, and (Z¹)n is 6-NH₂. |
| Table 1-722 | R¹ is Me, R² is Me, W¹ is O, G is H, X is O, and (Z¹)n is 6-NH₂. |
| Table 1-723 | R¹ is Me, R² is Me, W¹ is O, G is H, X is S, and (Z¹)n is 7-NH₂. |
| Table 1-724 | R¹ is Me, R² is Me, W¹ is O, G is H, X is O, and (Z¹)n is 7-NH₂. |
| Table 1-725 | R¹ is Me, R² is Me, W¹ is O, G is H, X is S, and (Z¹)n is 5-C(O)OH. |
| Table 1-726 | R¹ is Me, R² is Me, W¹ is O, G is H, X is O, and (Z¹)n is 5-C(O)OH. |
| Table 1-727 | R¹ is Me, R² is Me, W¹ is O, G is H, X is S, and (Z¹)n is 6-C(O)OH. |
| Table 1-728 | R¹ is Me, R² is Me, W¹ is O, G is H, X is O, and (Z¹)n is 6-C(O)OH. |
| Table 1-729 | R¹ is Me, R² is Me, W¹ is O, G is H, X is S, and (Z¹)n is 7-C(O)OH. |
| Table 1-730 | R¹ is Me, R² is Me, W¹ is O, G is H, X is O, and (Z¹)n is 7-C(O)OH. |
| Table 1-731 | R¹ is Me, R² is Me, W¹ is O, G is H, X is S, and (Z¹)n is 5-C(O)OMe. |
| Table 1-732 | R¹ is Me, R² is Me, W¹ is O, G is H, X is O, and (Z¹)n is 5-C(O)OMe. |
| Table 1-733 | R¹ is Me, R² is Me, W¹ is O, G is H, X is S, and (Z¹)n is 6-C(O)OMe. |
| Table 1-734 | R¹ is Me, R² is Me, W¹ is O, G is H, X is O, and (Z¹)n is 6-C(O)OMe. |
| Table 1-735 | R¹ is Me, R² is Me, W¹ is O, G is H, X is S, and (Z¹)n is 7-C(O)OMe. |
| Table 1-736 | R¹ is Me, R² is Me, W¹ is O, G is H, X is O, and (Z¹)n is 7-C(O)OMe. |
| Table 1-737 | R¹ is Me, R² is Me, W¹ is O, G is H, X is S, and (Z¹)n is 5-C(O)OEt. |
| Table 1-738 | R¹ is Me, R² is Me, W¹ is O, G is H, X is O, and (Z¹)n is 5-C(O)OEt. |
| Table 1-739 | R¹ is Me, R² is Me, W¹ is O, G is H, X is S, and (Z¹)n is 6-C(O)OEt. |
| Table 1-740 | R¹ is Me, R² is Me, W¹ is O, G is H, X is O, and (Z¹)n is 6-C(O)OEt. |
| Table 1-741 | R¹ is Me, R² is Me, W¹ is O, G is H, X is S, and (Z¹)n is 7-C(O)OEt. |
| Table 1-742 | R¹ is Me, R² is Me, W¹ is O, G is H, X is O, and (Z¹)n is 7-C(O)OEt. |
| Table 1-743 | R¹ is Me, R² is Me, W¹ is O, G is H, X is S, and (Z¹)n is 5-CF₃. |
| Table 1-744 | R¹ is Me, R² is Me, W¹ is O, G is H, X is O, and (Z¹)n is 5-CF₃. |
| Table 1-745 | R¹ is Me, R² is Me, W¹ is O, G is H, X is S, and (Z¹)n is 6-CF₃. |
| Table 1-746 | R¹ is Me, R² is Me, W¹ is O, G is H, X is O, and (Z¹)n is 6-CF₃. |
| Table 1-747 | R¹ is Me, R² is Me, W¹ is O, G is H, X is S, and (Z¹)n is 7-CF₃. |
| Table 1-748 | R¹ is Me, R² is Me, W¹ is O, G is H, X is O, and (Z¹)n is 7-CF₃. |
| Table 1-749 | R¹ is Me, R² is Me, W¹ is O, G is H, X is S, and (Z¹)n is 5-(T-1-5). |
| Table 1-750 | R¹ is Me, R² is Me, W¹ is O, G is H, X is O, and (Z¹)n is 5-(T-1-5). |
| Table 1-751 | R¹ is Me, R² is Me, W¹ is O, Gis H, X is S, and (Z¹)n is 6-(T-1-5). |
| Table 1-752 | R¹ is Me, R² is Me, W¹ is O, G is H, X is O, and (Z¹)n is 6-(T-1-5). |
| Table 1-753 | R¹ is Me, R² is Me, W¹ is O, G is H, X is S, and (Z¹)n is 7-(T-1-5). |
| Table 1-754 | R¹ is Me, R² is Me, W¹ is O, G is H, X is O, and (Z¹)n is 7-(T-1-5). |
| Table 1-755 | R¹ is Me, R² is Me, W¹ is O, G is H, X is S, and (Z¹)n is 5-Pr-c. |
| Table 1-756 | R¹ is Me, R² is Me, W¹ is O, G is H, X is O, and (Z¹)n is 5-Pr-c. |
| Table 1-757 | R¹ is Me, R² is Me, W¹ is O, G is H, X is S, and (Z¹)n is 6-Pr-c. |
| Table 1-758 | R¹ is Me, R² is Me, W¹ is O, G is H, X is O, and (Z¹)n is 6-Pr-c. |
| Table 1-759 | R¹ is Me, R² is Me, W¹ is O, G is H, X is S, and (Z¹)n is 7-Pr-c. |
| Table 1-760 | R¹ is Me, R² is Me, W¹ is O, G is H, X is O, and (Z¹)n is 7-Pr-c. |
| Table 1-761 | R¹ is Me, R² is Me, W¹ is O, G is H, X is S, and (Z¹)n is 5-CN. |
| Table 1-762 | R¹ is Me, R² is Me, W¹ is O, G is H, X is O, and (Z¹)n is 5-CN. |
| Table 1-763 | R¹ is Me, R² is Me, W¹ is O, G is H, X is S, and (Z¹)n is 6-CN. |
| Table 1-764 | R¹ is Me, R² is Me, W¹ is O, G is H, X is O, and (Z¹)n is 6-CN. |
| Table 1-765 | R¹ is Me, R² is Me, W¹ is O, G is H, X is S, and (Z¹)n is 7-CN. |
| Table 1-766 | R¹ is Me, R² is Me, W¹ is O, G is H, X is O, and (Z¹)n is 7-CN. |
| Table 1-767 | R¹ is Me, R² is Me, W¹ is O, G is H, X is S, and (Z¹)n is 5-CH₂OMe. |
| Table 1-768 | R¹ is Me, R² is Me, W¹ is O, G is H, X is O, and (Z¹)n is 5-CH₂OMe. |
| Table 1-769 | R¹ is Me, R² is Me, W¹ is O, G is H, X is S, and (Z¹)n is 6-CH₂OMe. |
| Table 1-770 | R¹ is Me, R² is Me, W¹ is O, G is H, X is O, and (Z¹)n is 6-CH₂OMe. |
| Table 1-771 | R¹ is Me, R² is Me, W¹ is O, G is H, X is S, and (Z¹)n is 7-CH₂OMe. |
| Table 1-772 | R¹ is Me, R² is Me, W¹ is O, G is H, X is O, and (Z¹)n is 7-CH₂OMe. |
| Table 1-773 | R¹ is Me, R² is Me, W¹ is O, G is H, X is S, and (Z¹)n is 5-CH₂OCH₂CF₃. |
| Table 1-774 | R¹ is Me, R² is Me, W¹ is O, G is H, X is O, and (Z¹)n is 5-CHzOCHzCF₃ |
| Table 1-775 | R¹ is Me, R² is Me, W¹ is O, G is H, X is S, and (Z¹)n is 6-CH₂OCH₂CF₃. |
| Table 1-776 | R¹ is Me, R² is Me, W¹ is O, G is H, X is O, and (Z¹)n is 6-CHzOCHzCF₃ |
| Table 1-777 | R¹ is Me, R² is Me, W¹ is O, G is H, X is S, and (Z¹)n is 7-CH₂OCH₂CF₃. |
| Table 1-778 | R¹ is Me, R² is Me, W¹ is O, G is H, X is O, and (Z¹)n is 7-CHzOCHzCF₃ |
| Table 1-779 | R¹ is Me, R² is Me, W¹ is O, G is H, X is S, and (Z¹)n is 5-CH₂SMe. |
| Table 1-780 | R¹ is Me, R² is Me, W¹ is O, G is H, X is O, and (Z¹)n is 5-CH₂SMe. |
| Table 1-781 | R¹ is Me, R² is Me, W¹ is O, G is H, X is S, and (Z¹)n is 6-CH₂SMe. |
| Table 1-782 | R¹ is Me, R² is Me, W¹ is O, G is H, X is O, and (Z¹)n is 6-CH₂SMe. |
| Table 1-783 | R¹ is Me, R² is Me, W¹ is O, G is H, X is S, and (Z¹)n is 7-CH₂SMe. |
| Table 1-784 | R¹ is Me, R² is Me, W¹ is O, G is H, X is O, and (Z¹)n is 7-CH₂SMe. |
| Table 1-785 | R¹ is Me, R² is Me, W¹ is O, G is H, X is S, and (Z¹)n is 5-CHzS(O)Me. |
| Table 1-786 | R¹ is Me, R² is Me, W¹ is O, G is H, X is O, and (Z¹)n is 5-CHzS(O)Me. |
| Table 1-787 | R¹ is Me, R² is Me, W¹ is O, G is H, X is S, and (Z¹)n is 6-CHzS(O)Me. |
| Table 1-788 | R¹ is Me, R² is Me, W¹ is O, G is H, X is O, and (Z¹)n is 6-CHzS(O)Me. |
| Table 1-789 | R¹ is Me, R² is Me, W¹ is O, G is H, X is S, and (Z¹)n is 7-CHzS(O)Me. |
| Table 1-790 | R¹ is Me, R² is Me, W¹ is O, G is H, X is O, and (Z¹)n is 7-CHzS(O)Me. |
| Table 1-791 | R¹ is Me, R² is Me, W¹ is O, G is H, X is S, and (Z¹)n is 5-CH₂S(O)₂Me. |
| Table 1-792 | R¹ is Me, R² is Me, W¹ is O, G is H, X is O, and (Z¹)n is 5-CH₂S(O)₂Me. |
| Table 1-793 | R¹ is Me, R² is Me, W¹ is O, G is H, X is S, and (Z¹)n is 6-CH₂S(O)₂Me. |
| Table 1-794 | R¹ is Me, R² is Me, W¹ is O, G is H, X is O, and (Z¹)n is 6-CH₂S(O)₂Me. |
| Table 1-795 | R¹ is Me, R² is Me, W¹ is O, G is H, X is S, and (Z¹)n is 7-CH₂S(O)₂Me. |
| Table 1-796 | R¹ is Me, R² is Me, W¹ is O, G is H, X is O, and (Z¹)n is 7-CH₂S(O)₂Me. |
| Table 1-797 | R¹ is Me, R² is Me, W¹ is O, G is H, X is S, and (Z¹)n is 5-SMe. |
| Table 1-798 | R¹ is Me, R² is Me, W¹ is O, G is H, X is O, and (Z¹)n is 5-SMe. |
| Table 1-799 | R¹ is Me, R² is Me, W¹ is O, G is H, X is S, and (Z¹)n is 6-SMe. |
| Table 1-800 | R¹ is Me, R² is Me, W¹ is O, G is H, X is O, and (Z¹)n is 6-SMe. |
| Table 1-801 | R¹ is Me, R² is Me, W¹ is O, G is H, X is S, and (Z¹)n is 7-SMe. |
| Table 1-802 | R¹ is Me, R² is Me, W¹ is O, G is H, X is O, and (Z¹)n is 7-SMe. |
| Table 1-803 | R¹ is Me, R² is Me, W¹ is O, G is H, X is S, and (Z¹)n is 5-SEt. |
| Table 1-804 | R¹ is Me, R² is Me, W¹ is O, G is H, X is O, and (Z¹)n is 5-SEt. |
| Table 1-805 | R¹ is Me, R² is Me, W¹ is O, G is H, X is S, and (Z¹)n is 6-SEt. |
| Table 1-806 | R¹ is Me, R² is Me, W¹ is O, G is H, X is O, and (Z¹)n is 6-SEt. |
| Table 1-807 | R¹ is Me, R² is Me, W¹ is O, G is H, X is S, and (Z¹)n is 7-SEt. |
| Table 1-808 | R¹ is Me, R² is Me, W¹ is O, G is H, X is O, and (Z¹)n is 7-SEt. |
| Table 1-809 | R¹ is Me, R² is Me, W¹ is O, G is H, X is S, and (Z¹)n is 5-SPr-n. |
| Table 1-810 | R¹ is Me, R² is Me, W¹ is O, G is H, X is O, and (Z¹)n is 5-SPr-n. |
| Table 1-811 | R¹ is Me, R² is Me, W¹ is O, G is H, X is S, and (Z¹)n is 6-SPr-n. |
| Table 1-812 | R¹ is Me, R² is Me, W¹ is O, G is H, X is O, and (Z¹)n is 6-SPr-n. |
| Table 1-813 | R¹ is Me, R² is Me, W¹ is O, G is H, X is S, and (Z¹)n is 7-SPr-n. |
| Table 1-814 | R¹ is Me, R² is Me, W¹ is O, G is H, X is O, and (Z¹)n is 7-SPr-n. |
| Table 1-815 | R¹ is Me, R² is Me, W¹ is O, G is H, X is S, and (Z¹)n is 5-SPr-i. |
| Table 1-816 | R¹ is Me, R² is Me, W¹ is O, G is H, X is O, and (Z¹)n is 5-SPr-i. |
| Table 1-817 | R¹ is Me, R² is Me, W¹ is O, G is H, X is S, and (Z¹)n is 6-SPr-i. |
| Table 1-818 | R¹ is Me, R² is Me, W¹ is O, G is H, X is O, and (Z¹)n is 6-SPr-i. |
| Table 1-819 | R¹ is Me, R² is Me, W¹ is O, G is H, X is S, and (Z¹)n is 7-SPr-i. |
| Table 1-820 | R¹ is Me, R² is Me, W¹ is O, G is H, X is O, and (Z¹)n is 7-SPr-i. |
| Table 1-821 | R¹ is Me, R² is Me, W¹ is O, G is H, X is S, and (Z¹)n is 5-SBu-n. |
| Table 1-822 | R¹ is Me, R² is Me, W¹ is O, G is H, X is O, and (Z¹)n is 5-SBu-n. |
| Table 1-823 | R¹ is Me, R² is Me, W¹ is O, G is H, X is S, and (Z¹)n is 6-SBu-n. |
| Table 1-824 | R¹ is Me, R² is Me, W¹ is O, G is H, X is O, and (Z¹)n is 6-SBu-n. |
| Table 1-825 | R¹ is Me, R² is Me, W¹ is O, G is H, X is S, and (Z¹)n is 7-SBu-n. |
| Table 1-826 | R¹ is Me, R² is Me, W¹ is O, G is H, X is O, and (Z¹)n is 7-SBu-n. |
| Table 1-827 | R¹ is Me, R² is Me, W¹ is O, G is H, X is S, and (Z¹)n is 5-SBu-i. |
| Table 1-828 | R¹ is Me, R² is Me, W¹ is O, G is H, X is O, and (Z¹)n is 5-SBu-i. |
| Table 1-829 | R¹ is Me, R² is Me, W¹ is O, G is H, X is S, and (Z¹)n is 6-SBu-i. |
| Table 1-830 | R¹ is Me, R² is Me, W¹ is O, G is H, X is O, and (Z¹)n is 6-SBu-i. |
| Table 1-831 | R¹ is Me, R² is Me, W¹ is O, G is H, X is S, and (Z¹)n is 7-SBu-i. |
| Table 1-832 | R¹ is Me, R² is Me, W¹ is O, G is H, X is O, and (Z¹)n is 7-SBu-i. |
| Table 1-833 | R¹ is Me, R² is Me, W¹ is O, G is H, X is S, and (Z¹)n is 5-SBu-sec. |
| Table 1-834 | R¹ is Me, R² is Me, W¹ is O, G is H, X is O, and (Z¹)n is 5-SBu-sec. |
| Table 1-835 | R¹ is Me, R² is Me, W¹ is O, G is H, X is S, and (Z¹)n is 6-SBu-sec. |
| Table 1-836 | R¹ is Me, R² is Me, W¹ is O, G is H, X is O, and (Z¹)n is 6-SBu-sec. |
| Table 1-837 | R¹ is Me, R² is Me, W¹ is O, G is H, X is S, and (Z¹)n is 7-SBu-sec. |
| Table 1-838 | R¹ is Me, R² is Me, W¹ is O, G is H, X is O, and (Z¹)n is 7-SBu-sec. |
| Table 1-839 | R¹ is Me, R² is Me, W¹ is O, G is H, X is S, and (Z¹)n is 5-SBu-tert. |
| Table 1-840 | R¹ is Me, R² is Me, W¹ is O, G is H, X is O, and (Z¹)n is 5-SBu-tert. |
| Table 1-841 | R¹ is Me, R² is Me, W¹ is O, G is H, X is S, and (Z¹)n is 6-SBu-tert. |
| Table 1-842 | R¹ is Me, R² is Me, W¹ is O, G is H, X is O, and (Z¹)n is 6-SBu-tert. |
| Table 1-843 | R¹ is Me, R² is Me, W¹ is O, G is H, X is S, and (Z¹)n is 7-SBu-tert. |
| Table 1-844 | R¹ is Me, R² is Me, W¹ is O, G is H, X is O, and (Z¹)n is 7-SBu-tert. |
| Table 1-845 | R¹ is Me, R² is Me, W¹ is O, G is H, X is S, and (Z¹)n is 5-SCH₂CH=CH₂. |
| Table 1-846 | R¹ is Me, R² is Me, W¹ is O, G is H, X is O, and (Z¹)n is 5-SCH₂CH=CH₂. |
| Table 1-847 | R¹ is Me, R² is Me, W¹ is O, G is H, X is S, and (Z¹)n is 6-SCH₂CH=CH₂. |
| Table 1-848 | R¹ is Me, R² is Me, W¹ is O, G is H, X is O, and (Z¹)n is 6-SCH₂CH=CH₂. |
| Table 1-849 | R¹ is Me, R² is Me, W¹ is O, G is H, X is S, and (Z¹)n is 7-SCH₂CH=CH₂. |
| Table 1-850 | R¹ is Me, R² is Me, W¹ is O, G is H, X is O, and (Z¹)n is 7-SCH₂CH=CH₂. |
| Table 1-851 | R¹ is Me, R² is Me, W¹ is O, G is H, X is S, and (Z¹)n is 5-SCH₂C=CH. |
| Table 1-852 | R¹ is Me, R² is Me, W¹ is O, G is H, X is O, and (Z¹)n is 5-SCH₂C=CH. |
| Table 1-853 | R¹ is Me, R² is Me, W¹ is O, G is H, X is S, and (Z¹)n is 6-SCH₂C--CH. |
| Table 1-854 | R¹ is Me, R² is Me, W¹ is O, G is H, X is O, and (Z¹)n is 6-SCH₂C--CH. |
| Table 1-855 | R¹ is Me, R² is Me, W¹ is O, G is H, X is S, and (Z¹)n is 7-SCH₂C--CH. |
| Table 1-856 | R¹ is Me, R² is Me, W¹ is O, G is H, X is O, and (Z¹)n is 7-SCH₂C--CH. |
| Table 1-857 | R¹ is Me, R² is Me, W¹ is O, G is H, X is S, and (Z¹)n is 5-SCHF₂. |
| Table 1-858 | R¹ is Me, R² is Me, W¹ is O, G is H, X is O, and (Z¹)n is 5-SCHF₂. |
| Table 1-859 | R¹ is Me, R² is Me, W¹ is O, G is H, X is S, and (Z¹)n is 6-SCHF₂. |
| Table 1-860 | R¹ is Me, R² is Me, W¹ is O, G is H, X is O, and (Z¹)n is 6-SCHF₂. |
| Table 1-861 | R¹ is Me, R² is Me, W¹ is O, G is H, X is S, and (Z¹)n is 7-SCHF₂. |
| Table 1-862 | R¹ is Me, R² is Me, W¹ is O, G is H, X is O, and (Z¹)n is 7-SCHF₂. |
| Table 1-863 | R¹ is Me, R² is Me, W¹ is O, G is H, X is S, and (Z¹)n is 5-SCF₃. |
| Table 1-864 | R¹ is Me, R² is Me, W¹ is O, G is H, X is O, and (Z¹)n is 5-SCF₃. |
| Table 1-865 | R¹ is Me, R² is Me, W¹ is O, G is H, X is S, and (Z¹)n is 6-SCF₃. |
| Table 1-866 | R¹ is Me, R² is Me, W¹ is O, G is H, X is O, and (Z¹)n is 6-SCF₃. |
| Table 1-867 | R¹ is Me, R² is Me, W¹ is O, G is H, X is S, and (Z¹)n is 7-SCF₃. |
| Table 1-868 | R¹ is Me, R² is Me, W¹ is O, G is H, X is O, and (Z¹)n is 7-SCF₃. |
| Table 1-869 | R¹ is Me, R² is Me, W¹ is O, G is H, X is S, and (Z¹)n is 5-SCH₂CH₂F. |
| Table 1-870 | R¹ is Me, R² is Me, W¹ is O, G is H, X is O, and (Z¹)n is 5-SCH₂CH₂F. |
| Table 1-871 | R¹ is Me, R² is Me, W¹ is O, G is H, X is S, and (Z¹)n is 6-SCH₂CH₂F. |
| Table 1-872 | R¹ is Me, R² is Me, W¹ is O, G is H, X is O, and (Z¹)n is 6-SCH₂CH₂F. |
| Table 1-873 | R¹ is Me, R² is Me, W¹ is O, G is H, X is S, and (Z¹)n is 7-SCH₂CH₂F. |
| Table 1-874 | R¹ is Me, R² is Me, W¹ is O, G is H, X is O, and (Z¹)n is 7-SCH₂CH₂F. |
| Table 1-875 | R¹ is Me, R² is Me, W¹ is O, G is H, X is S, and (Z¹)n is 5-SCH₂CHF₂. |
| Table 1-876 | R¹ is Me, R² is Me, W¹ is O, G is H, X is O, and (Z¹)n is 5-SCH₂CHF₂. |
| Table 1-877 | R¹ is Me, R² is Me, W¹ is O, G is H, X is S, and (Z¹)n is 6-SCH₂CHF₂. |
| Table 1-878 | R¹ is Me, R² is Me, W¹ is O, G is H, X is O, and (Z¹)n is 6-SCH₂CHF₂. |
| Table 1-879 | R¹ is Me, R² is Me, W¹ is O, G is H, X is S, and (Z¹)n is 7-SCH₂CHF₂. |
| Table 1-880 | R¹ is Me, R² is Me, W¹ is O, G is H, X is O, and (Z¹)n is 7-SCH₂CHF₂. |
| Table 1-881 | R¹ is Me, R² is Me, W¹ is O, G is H, X is S, and (Z¹)n is 5-SCH₂CF₃. |
| Table 1-882 | R¹ is Me, R² is Me, W¹ is O, G is H, X is O, and (Z¹)n is 5-SCH₂CF₃. |
| Table 1-883 | R¹ is Me, R² is Me, W¹ is O, G is H, X is S, and (Z¹)n is 6-SCH₂CF₃. |
| Table 1-884 | R¹ is Me, R² is Me, W¹ is O, G is H, X is O, and (Z¹)n is 6-SCH₂CF₃. |
| Table 1-885 | R¹ is Me, R² is Me, W¹ is O, G is H, X is S, and (Z¹)n is 7-SCH₂CF₃. |
| Table 1-886 | R¹ is Me, R² is Me, W¹ is O, G is H, X is O, and (Z¹)n is 7-SCH₂CF₃. |
| Table 1-887 | R¹ is Me, R² is Me, W¹ is O, G is H, X is S, and (Z¹)n is 5-SCH₂CH=C(F)₂. |
| Table 1-888 | R¹ is Me, R² is Me, W¹ is O, G is H, X is O, and (Z¹)n is 5-SCH₂CH=C(F)₂ |
| Table 1-889 | R¹ is Me, R² is Me, W¹ is O, G is H, X is S, and (Z¹)n is 6-SCH₂CH=C(F)₂. |
| Table 1-890 | R¹ is Me, R² is Me, W¹ is O, G is H, X is O, and (Z¹)n is 6-SCH₂CH=C(F)₂ |
| Table 1-891 | R¹ is Me, R² is Me, W¹ is O, G is H, X is S, and (Z¹)n is 7-SCH₂CH=C(F)₂. |
| Table 1-892 | R¹ is Me, R² is Me, W¹ is O, G is H, X is O, and (Z¹)n is 7-SCH₂CH=C(F)₂. |
| Table 1-893 | R¹ is Me, R² is Me, W¹ is O, G is H, X is S, and (Z¹)n is 5-SCH₂CH=C(Cl)₂. |
| Table 1-894 | R¹ is Me, R² is Me, W¹ is O, G is H, X is O, and (Z¹)n is 5-SCH₂CH=C(Cl)₂. |
| Table 1-895 | R¹ is Me, R² is Me, W¹ is O, G is H, X is S, and (Z¹)n is 6-SCH₂CH=C(Cl)₂. |
| Table 1-896 | R¹ is Me, R² is Me, W¹ is O, G is H, X is O, and (Z¹)n is 6-SCH₂CH=C(Cl)₂. |
| Table 1-897 | R¹ is Me, R² is Me, W¹ is O, G is H, X is S, and (Z¹)n is 7-SCH₂CH=C(Cl)₂. |
| Table 1-898 | R¹ is Me, R² is Me, W¹ is O, G is H, X is O, and (Z¹)n is 7-SCH₂CH=C(Cl)₂. |
| Table 1-899 | R¹ is Me, R² is Me, W¹ is O, G is H, X is S, and (Z¹)n is 5-SCH₂C≡CCl. |
| Table 1-900 | R¹ is Me, R² is Me, W¹ is O, G is H, X is O, and (Z¹)n is 5-SCH₂C≡CCl. |
| Table 1-901 | R¹ is Me, R² is Me, W¹ is O, G is H, X is S, and (Z¹)n is 6-SCH₂C≡CCl. |
| Table 1-902 | R¹ is Me, R² is Me, W¹ is O, G is H, X is O, and (Z¹)n is 6-SCH₂C≡CCl. |
| Table 1-903 | R¹ is Me, R² is Me, W¹ is O, G is H, X is S, and (Z¹)n is 7-SCH₂C≡CCl. |
| Table 1-904 | R¹ is Me, R² is Me, W¹ is O, G is H, X is O, and (Z¹)n is 7-SCH₂C≡CCl. |
| Table 1-905 | R¹ is Me, R² is Me, W¹ is O, G is H, X is S, and (Z¹)n is 5-SCH₂C≡CBr. |
| Table 1-906 | R¹ is Me, R² is Me, W¹ is O, G is H, X is O, and (Z¹)n is 5-SCH₂C≡CBr. |
| Table 1-907 | R¹ is Me, R² is Me, W¹ is O, G is H, X is S, and (Z¹)n is 6-SCH₂C≡CBr. |
| Table 1-908 | R¹ is Me, R² is Me, W¹ is O, G is H, X is O, and (Z¹)n is 6-SCH₂C≡CBr. |
| Table 1-909 | R¹ is Me, R² is Me, W¹ is O, G is H, X is S, and (Z¹)n is 7-SCH₂C≡CBr. |
| Table 1-910 | R¹ is Me, R² is Me, W¹ is O, G is H, X is O, and (Z¹)n is 7-SCH₂C≡CBr. |
| Table 1-911 | R¹ is Me, R² is Me, W¹ is O, G is H, X is S, and (Z¹)n is 5-SCH₂(T-1-1). |
| Table 1-912 | R¹ is Me, R² is Me, W¹ is O, G is H, X is O, and (Z¹)n is 5-SCH₂(T-1-1). |
| Table 1-913 | R¹ is Me, R² is Me, W¹ is O, G is H, X is S, and (Z¹)n is 6-SCH₂(T-1-1). |
| Table 1-914 | R¹ is Me, R² is Me, W¹ is O, G is H, X is O, and (Z¹)n is 6-SCH₂(T-1-1). |
| Table 1-915 | R¹ is Me, R² is Me, W¹ is O, G is H, X is S, and (Z¹)n is 7-SCH₂(T-1-1). |
| Table 1-916 | R¹ is Me, R² is Me, W¹ is O, G is H, X is O, and (Z¹)n is 7-SCH₂(T-1-1). |
| Table 1-917 | R¹ is Me, R² is Me, W¹ is O, G is H, X is S, and (Z¹)n is 5-SCH₂(U-1a). |
| Table 1-918 | R¹ is Me, R² is Me, W¹ is O, G is H, X is O, and (Z¹)n is 5-SCH₂(U-1a). |
| Table 1-919 | R¹ is Me, R² is Me, W¹ is O, G is H, X is S, and (Z¹)n is 6-SCH₂(U-1a). |
| Table 1-920 | R¹ is Me, R² is Me, W¹ is O, G is H, X is O, and (Z¹)n is 6-SCH₂(U-1a). |
| Table 1-921 | R¹ is Me, R² is Me, W¹ is O, G is H, X is S, and (Z¹)n is 7-SCH₂(U-1a). |
| Table 1-922 | R¹ is Me, R² is Me, W¹ is O, G is H, X is O, and (Z¹)n is 7-SCH₂(U-1a). |
| Table 1-923 | R¹ is Me, R² is Me, W¹ is O, G is H, X is S, and (Z¹)n is 5-SCH₂CH₂OMe. |
| Table 1-924 | R¹ is Me, R² is Me, W¹ is O, G is H, X is O, and (Z¹)n is 5-SCH₂CH₂OMe. |
| Table 1-925 | R¹ is Me, R² is Me, W¹ is O, G is H, X is S, and (Z¹)n is 6-SCH₂CH₂OMe. |
| Table 1-926 | R¹ is Me, R² is Me, W¹ is O, G is H, X is O, and (Z¹)n is 6-SCH₂CH₂OMe. |
| Table 1-927 | R¹ is Me, R² is Me, W¹ is O, G is H, X is S, and (Z¹)n is 7-SCH₂CH₂OMe. |
| Table 1-928 | R¹ is Me, R² is Me, W¹ is O, G is H, X is O, and (Z¹)n is 7-SCH₂CH₂OMe. |
| Table 1-929 | R¹ is Me, R² is Me, W¹ is O, G is H, X is S, and (Z¹)n is 5-SCH₂CH₂SMe. |
| Table 1-930 | R¹ is Me, R² is Me, W¹ is O, G is H, X is O, and (Z¹)n is 5-SCH₂CH₂SMe. |
| Table 1-931 | R¹ is Me, R² is Me, W¹ is O, G is H, X is S, and (Z¹)n is 6-SCH₂CH₂SMe. |
| Table 1-932 | R¹ is Me, R² is Me, W¹ is O, G is H, X is O, and (Z¹)n is 6-SCH₂CH₂SMe. |
| Table 1-933 | R¹ is Me, R² is Me, W¹ is O, G is H, X is S, and (Z¹)n is 7-SCH₂CH₂SMe. |
| Table 1-934 | R¹ is Me, R² is Me, W¹ is O, G is H, X is O, and (Z¹)n is 7-SCH₂CH₂SMe. |
| Table 1-935 | R¹ is Me, R² is Me, W¹ is O, G is H, X is S, and (Z¹)n is 5-SCH₂CH₂S(O)Me. |
| Table 1-936 | R¹ is Me, R² is Me, W¹ is O, G is H, X is O, and (Z¹)n is 5-SCH₂CH₂S(O)Me. |
| Table 1-937 | R¹ is Me, R² is Me, W¹ is O, G is H, X is S, and (Z¹)n is 6-SCH₂CH₂S(O)Me. |
| Table 1-938 | R¹ is Me, R² is Me, W¹ is O, G is H, X is O, and (Z¹)n is 6-SCH₂CH₂S(O)Me. |
| Table 1-939 | R¹ is Me, R² is Me, W¹ is O, G is H, X is S, and (Z¹)n is 7-SCH₂CH₂S(O)Me. |
| Table 1-940 | R¹ is Me, R² is Me, W¹ is O, G is H, X is O, and (Z¹)n is 7-SCH₂CH₂S(O)Me. |
| Table 1-941 | R¹ is Me, R² is Me, W¹ is O, G is H, X is S, and (Z¹)ₙ is 5-SCH₂CH₂S(O)₂Me. |
| Table 1-942 | R¹ is Me, R² is Me, W¹ is O, G is H, X is O, and (Z¹)n is 5-SCH₂CH₂S(O)₂Me. |
| Table 1-943 | R¹ is Me, R² is Me, W¹ is O, G is H, X is S, and (Z¹)n is 6-SCH₂CH₂S(O)₂Me. |
| Table 1-944 | R¹ is Me, R² is Me, W¹ is O, G is H, X is O, and (Z¹)n is 6-SCH₂CH₂S(O)₂Me. |
| Table 1-945 | R¹ is Me, R² is Me, W¹ is O, G is H, X is S, and (Z¹)n is 7-SCH₂CH₂S(O)₂Me. |
| Table 1-946 | R¹ is Me, R² is Me, W¹ is O, G is H, X is O, and (Z¹)n is 7-SCH₂CH₂S(O)₂Me. |
| Table 1-947 | R¹ is Me, R² is Me, W¹ is O, G is H, X is S, and (Z¹)n is 5-S(O)Me. |
| Table 1-948 | R¹ is Me, R² is Me, W¹ is O, G is H, X is O, and (Z¹)n is 5-S(O)Me. |
| Table 1-949 | R¹ is Me, R² is Me, W¹ is O, G is H, X is S, and (Z¹)n is 6-S(O)Me. |
| Table 1-950 | R¹ is Me, R² is Me, W¹ is O, G is H, X is O, and (Z¹)n is 6-S(O)Me. |
| Table 1-951 | R¹ is Me, R² is Me, W¹ is O, G is H, X is S, and (Z¹)n is 7-S(O)Me. |
| Table 1-952 | R¹ is Me, R² is Me, W¹ is O, G is H, X is O, and (Z¹)n is 7-S(O)Me. |
| Table 1-953 | R¹ is Me, R² is Me, W¹ is O, G is H, X is S, and (Z¹)n is 5-S(O)Et. |
| Table 1-954 | R¹ is Me, R² is Me, W¹ is O, G is H, X is O, and (Z¹)n is 5-S(O)Et. |
| Table 1-955 | R¹ is Me, R² is Me, W¹ is O, G is H, X is S, and (Z¹)n is 6-S(O)Et. |
| Table 1-956 | R¹ is Me, R² is Me, W¹ is O, G is H, X is O, and (Z¹)n is 6-S(O)Et. |
| Table 1-957 | R¹ is Me, R² is Me, W¹ is O, G is H, X is S, and (Z¹)n is 7-S(O)Et. |
| Table 1-958 | R¹ is Me, R² is Me, W¹ is O, G is H, X is O, and (Z¹)n is 7-S(O)Et. |
| Table 1-959 | R¹ is Me, R² is Me, W¹ is O, G is H, X is S, and (Z¹)n is 5-S(O)Pr-n. |
| Table 1-960 | R¹ is Me, R² is Me, W¹ is O, G is H, X is O, and (Z¹)n is 5-S(O)Pr-n. |
| Table 1-961 | R¹ is Me, R² is Me, W¹ is O, G is H, X is S, and (Z¹)n is 6-S(O)Pr-n. |
| Table 1-962 | R¹ is Me, R² is Me, W¹ is O, G is H, X is O, and (Z¹)n is 6-S(O)Pr-n. |
| Table 1-963 | R¹ is Me, R² is Me, W¹ is O, G is H, X is S, and (Z¹)n is 7-S(O)Pr-n. |
| Table 1-964 | R¹ is Me, R² is Me, W¹ is O, G is H, X is O, and (Z¹)n is 7-S(O)Pr-n. |
| Table 1-965 | R¹ is Me, R² is Me, W¹ is O, G is H, X is S, and (Z¹)n is 5-S(O)Pr-i. |
| Table 1-966 | R¹ is Me, R² is Me, W¹ is O, G is H, X is O, and (Z¹)n is 5-S(O)Pr-i. |
| Table 1-967 | R¹ is Me, R² is Me, W¹ is O, G is H, X is S, and (Z¹)n is 6-S(O)Pr-i. |
| Table 1-968 | R¹ is Me, R² is Me, W¹ is O, G is H, X is O, and (Z¹)n is 6-S(O)Pr-i. |
| Table 1-969 | R¹ is Me, R² is Me, W¹ is O, G is H, X is S, and (Z¹)n is 7-S(O)Pr-i. |
| Table 1-970 | R¹ is Me, R² is Me, W¹ is O, G is H, X is O, and (Z¹)n is 7-S(O)Pr-i. |
| Table 1-971 | R¹ is Me, R² is Me, W¹ is O, G is H, X is S, and (Z¹)n is 5-S(O)Bu-n. |
| Table 1-972 | R¹ is Me, R² is Me, W¹ is O, G is H, X is O, and (Z¹)n is 5-S(O)Bu-n. |
| Table 1-973 | R¹ is Me, R² is Me, W¹ is O, G is H, X is S, and (Z¹)n is 6-S(O)Bu-n. |
| Table 1-974 | R¹ is Me, R² is Me, W¹ is O, G is H, X is O, and (Z¹)n is 6-S(O)Bu-n. |
| Table 1-975 | R¹ is Me, R² is Me, W¹ is O, G is H, X is S, and (Z¹)n is 7-S(O)Bu-n. |
| Table 1-976 | R¹ is Me, R² is Me, W¹ is O, G is H, X is O, and (Z¹)n is 7-S(O)Bu-n. |
| Table 1-977 | R¹ is Me, R² is Me, W¹ is O, G is H, X is S, and (Z¹)n is 5-S(O)Bu-i. |
| Table 1-978 | R¹ is Me, R² is Me, W¹ is O, G is H, X is O, and (Z¹)n is 5-S(O)Bu-i. |
| Table 1-979 | R¹ is Me, R² is Me, W¹ is O, G is H, X is S, and (Z¹)n is 6-S(O)Bu-i. |
| Table 1-980 | R¹ is Me, R² is Me, W¹ is O, G is H, X is O, and (Z¹)n is 6-S(O)Bu-i. |
| Table 1-981 | R¹ is Me, R² is Me, W¹ is O, G is H, X is S, and (Z¹)n is 7-S(O)Bu-i. |
| Table 1-982 | R¹ is Me, R² is Me, W¹ is O, G is H, X is O, and (Z¹)n is 7-S(O)Bu-i. |
| Table 1-983 | R¹ is Me, R² is Me, W¹ is O, G is H, X is S, and (Z¹)n is 5-S(O)Bu-sec. |
| Table 1-984 | R¹ is Me, R² is Me, W¹ is O, G is H, X is O, and (Z¹)n is 5-S(O)Bu-sec. |
| Table 1-985 | R¹ is Me, R² is Me, W¹ is O, G is H, X is S, and (Z¹)n is 6-S(O)Bu-sec. |
| Table 1-986 | R¹ is Me, R² is Me, W¹ is O, G is H, X is O, and (Z¹)n is 6-S(O)Bu-sec. |
| Table 1-987 | R¹ is Me, R² is Me, W¹ is O, G is H, X is S, and (Z¹)n is 7-S(O)Bu-sec. |
| Table 1-988 | R¹ is Me, R² is Me, W¹ is O, G is H, X is O, and (Z¹)n is 7-S(O)Bu-sec. |
| Table 1-989 | R¹ is Me, R² is Me, W¹ is O, G is H, X is S, and (Z¹)n is 5-S(O)Bu-tert. |
| Table 1-990 | R¹ is Me, R² is Me, W¹ is O, G is H, X is O, and (Z¹)n is 5-S(O)Bu-tert. |
| Table 1-991 | R¹ is Me, R² is Me, W¹ is O, G is H, X is S, and (Z¹)n is 6-S(O)Bu-tert. |
| Table 1-992 | R¹ is Me, R² is Me, W¹ is O, G is H, X is O, and (Z¹)n is 6-S(O)Bu-tert. |
| Table 1-993 | R¹ is Me, R² is Me, W¹ is O, G is H, X is S, and (Z¹)n is 7-S(O)Bu-tert. |
| Table 1-994 | R¹ is Me, R² is Me, W¹ is O, G is H, X is O, and (Z¹)n is 7-S(O)Bu-tert. |
| Table 1-995 | R¹ is Me, R² is Me, W¹ is O, G is H, X is S, and (Z¹)n is 5-S(O)CH₂CH=CH₂. |
| Table 1-996 | R¹ is Me, R² is Me, W¹ is O, G is H, X is O, and (Z¹)n is 5-S(O)CH₂CH=CH₂. |
| Table 1-997 | R¹ is Me, R² is Me, W¹ is O, G is H, X is S, and (Z¹)n is 6-S(O)CH₂CH=CH₂. |
| Table 1-998 | R¹ is Me, R² is Me, W¹ is O, G is H, X is O, and (Z¹)n is 6-S(O)CH₂CH=CH₂. |
| Table 1-999 | R¹ is Me, R² is Me, W¹ is O, G is H, X is S, and (Z¹)n is 7-S(O)CH₂CH=CH₂. |
| Table 1-1000 | R¹ is Me, R² is Me, W¹ is O, G is H, X is O, and (Z¹)n is 7-S(O)CH₂CH=CH₂. |
| Table 1-1001 | R¹ is Me, R² is Me, W¹ is O, G is H, X is S, and (Z¹)n is 5-S(O)CH₂C≡CH. |
| Table 1-1002 | R¹ is Me, R² is Me, W¹ is O, G is H, X is O, and (Z¹)n is 5-S(O)CH₂C≡CH. |
| Table 1-1003 | R¹ is Me, R² is Me, W¹ is O, G is H, X is S, and (Z¹)n is 6-S(O)CH₂C≡CH. |
| Table 1-1004 | R¹ is Me, R² is Me, W¹ is O, G is H, X is O, and (Z¹)n is 6-S(O)CH₂C≡CH. |
| Table 1-1005 | R¹ is Me, R² is Me, W¹ is O, G is H, X is S, and (Z¹)n is 7-S(O)CH₂C≡CH. |
| Table 1-1006 | R¹ is Me, R² is Me, W¹ is O, G is H, X is O, and (Z¹)n is 7-S(O)CH₂C≡CH. |
| Table 1-1007 | R¹ is Me, R² is Me, W¹ is O, G is H, X is S, and (Z¹)n is 5-S(O)CHF₂. |
| Table 1-1008 | R¹ is Me, R² is Me, W¹ is O, G is H, X is O, and (Z¹)n is 5-S(O)CHF₂. |
| Table 1-1009 | R¹ is Me, R² is Me, W¹ is O, G is H, X is S, and (Z¹)n is 6-S(O)CHF₂. |
| Table 1-1010 | R¹ is Me, R² is Me, W¹ is O, G is H, X is O, and (Z¹)n is 6-S(O)CHF₂. |
| Table 1-1011 | R¹ is Me, R² is Me, W¹ is O, G is H, X is S, and (Z¹)n is 7-S(O)CHF₂. |
| Table 1-1012 | R¹ is Me, R² is Me, W¹ is O, G is H, X is O, and (Z¹)n is 7-S(O)CHF₂. |
| Table 1-1013 | R¹ is Me, R² is Me, W¹ is O, G is H, X is S, and (Z¹)n is 5-S(O)CF₃. |
| Table 1-1014 | R¹ is Me, R² is Me, W¹ is O, G is H, X is O, and (Z¹)n is 5-S(O)CF₃. |
| Table 1-1015 | R¹ is Me, R² is Me, W¹ is O, G is H, X is S, and (Z¹)n is 6-S(O)CF₃. |
| Table 1-1016 | R¹ is Me, R² is Me, W¹ is O, G is H, X is O, and (Z¹)n is 6-S(O)CF₃. |
| Table 1-1017 | R¹ is Me, R² is Me, W¹ is O, G is H, X is S, and (Z¹)n is 7-S(O)CF₃. |
| Table 1-1018 | R¹ is Me, R² is Me, W¹ is O, G is H, X is O, and (Z¹)n is 7-S(O)CF₃. |
| Table 1-1019 | R¹ is Me, R² is Me, W¹ is O, G is H, X is S, and (Z¹)n is 5-S(O)CH₂CH₂F. |
| Table 1-1020 | R¹ is Me, R² is Me, W¹ is O, G is H, X is O, and (Z¹)n is 5-S(O)CH₂CH₂F. |
| Table 1-1021 | R¹ is Me, R² is Me, W¹ is O, G is H, X is S, and (Z¹)n is 6-S(O)CH₂CH₂F. |
| Table 1-1022 | R¹ is Me, R² is Me, W¹ is O, G is H, X is O, and (Z¹)n is 6-S(O)CH₂CH₂F. |
| Table 1-1023 | R¹ is Me, R² is Me, W¹ is O, G is H, X is S, and (Z¹)n is 7-S(O)CH₂CH₂F. |
| Table 1-1024 | R¹ is Me, R² is Me, W¹ is O, G is H, X is O, and (Z¹)n is 7-S(O)CH₂CH₂F. |
| Table 1-1025 | R¹ is Me, R² is Me, W¹ is O, G is H, X is S, and (Z¹)n is 5-S(O)CH₂CHF₂. |
| Table 1-1026 | R¹ is Me, R² is Me, W¹ is O, G is H, X is O, and (Z¹)n is 5-S(O)CH₂CHF₂. |
| Table 1-1027 | R¹ is Me, R² is Me, W¹ is O, G is H, X is S, and (Z¹)n is 6-S(O)CH₂CHF₂. |
| Table 1-1028 | R¹ is Me, R² is Me, W¹ is O, G is H, X is O, and (Z¹)n is 6-S(O)CH₂CHF₂. |
| Table 1-1029 | R¹ is Me, R² is Me, W¹ is O, G is H, X is S, and (Z¹)n is 7-S(O)CH₂CHF₂. |
| Table 1-1030 | R¹ is Me, R² is Me, W¹ is O, G is H, X is O, and (Z¹)n is 7-S(O)CH₂CHF₂. |
| Table 1-1031 | R¹ is Me, R² is Me, W¹ is O, G is H, X is S, and (Z¹)n is 5-S(O)CH₂CF₃. |
| Table 1-1032 | R¹ is Me, R² is Me, W¹ is O, G is H, X is O, and (Z¹)n is 5-S(O)CH₂CF₃. |
| Table 1-1033 | R¹ is Me, R² is Me, W¹ is O, G is H, X is S, and (Z¹)n is 6-S(O)CH₂CF₃. |
| Table 1-1034 | R¹ is Me, R² is Me, W¹ is O, G is H, X is O, and (Z¹)n is 6-S(O)CH₂CF₃. |
| Table 1-1035 | R¹ is Me, R² is Me, W¹ is O, G is H, X is S, and (Z¹)n is 7-S(O)CH₂CF₃. |
| Table 1-1036 | R¹ is Me, R² is Me, W¹ is O, G is H, X is O, and (Z¹)n is 7-S(O)CH₂CF₃. |
| Table 1-1037 | R¹ is Me, R² is Me, W¹ is O, G is H, X is S, and (Z¹)n is 5-S(O)CH₂CH=C(F)₂. |
| Table 1-1038 | R¹ is Me, R² is Me, W¹ is O, G is H, X is O, and (Z¹)n is 5-S(O)CH₂CH=C(F)₂. |
| Table 1-1039 | R¹ is Me, R² is Me, W¹ is O, G is H, X is S, and (Z¹)n is 6-S(O)CH₂CH=C(F)₂. |
| Table 1-1040 | R¹ is Me, R² is Me, W¹ is O, G is H, X is O, and (Z¹)n is 6-S(O)CH₂CH=C(F)₂. |
| Table 1-1041 | R¹ is Me, R² is Me, W¹ is O, G is H, X is S, and (Z¹)ₙ is 7-S(O)CH₂CH=C(F)₂. |
| Table 1-1042 | R¹ is Me, R² is Me, W¹ is O, G is H, X is O, and (Z¹)n is 7-S(O)CH₂CH=C(F)₂. |
| Table 1-1043 | R¹ is Me, R² is Me, W¹ is O, G is H, X is S, and (Z¹)n is 5-S(O)CH₂CH=C(Cl)₂. |
| Table 1-1044 | R¹ is Me, R² is Me, W¹ is O, G is H, X is O, and (Z¹)n is 5-S(O)CH₂CH=C(Cl)₂. |
| Table 1-1045 | R¹ is Me, R² is Me, W¹ is O, G is H, X is S, and (Z¹)n is 6-S(O)CH₂CH=C(Cl)₂. |
| Table 1-1046 | R¹ is Me, R² is Me, W¹ is O, G is H, X is O, and (Z¹)n is 6-S(O)CH₂CH=C(Cl)₂. |
| Table 1-1047 | R¹ is Me, R² is Me, W¹ is O, G is H, X is S, and (Z¹)n is 7-S(O)CH₂CH=C(Cl)₂. |
| Table 1-1048 | R¹ is Me, R² is Me, W¹ is O, G is H, X is O, and (Z¹)n is 7-S(O)CH₂CH=C(Cl)₂. |
| Table 1-1049 | R¹ is Me, R² is Me, W¹ is O, G is H, X is S, and (Z¹)n is 5-S(O)CH₂C≡CCl. |
| Table 1-1050 | R¹ is Me, R² is Me, W¹ is O, G is H, X is O, and (Z¹)n is 5-S(O)CH₂C≡CCl. |
| Table 1-1051 | R¹ is Me, R² is Me, W¹ is O, G is H, X is S, and (Z¹)n is 6-S(O)CH₂C≡CCl. |
| Table 1-1052 | R¹ is Me, R² is Me, W¹ is O, G is H, X is O, and (Z¹)n is 6-S(O)CH₂C≡CCl. |
| Table 1-1053 | R¹ is Me, R² is Me, W¹ is O, G is H, X is S, and (Z¹)n is 7-S(O)CH₂C≡CCl. |
| Table 1-1054 | R¹ is Me, R² is Me, W¹ is O, G is H, X is O, and (Z¹)n is 7-S(O)CH₂C≡CCl. |
| Table 1-1055 | R¹ is Me, R² is Me, W¹ is O, G is H, X is S, and (Z¹)n is 5-S(O)CH₂C≡CBr. |
| Table 1-1056 | R¹ is Me, R² is Me, W¹ is O, G is H, X is O, and (Z¹)n is 5-S(O)CH₂C≡CBr. |
| Table 1-1057 | R¹ is Me, R² is Me, W¹ is O, G is H, X is S, and (Z¹)n is 6-S(O)CH₂C≡CBr. |
| Table 1-1058 | R¹ is Me, R² is Me, W¹ is O, G is H, X is O, and (Z¹)n is 6-S(O)CH₂C≡CBr. |
| Table 1-1059 | R¹ is Me, R² is Me, W¹ is O, G is H, X is S, and (Z¹)n is 7-S(O)CH₂C≡CBr. |
| Table 1-1060 | R¹ is Me, R² is Me, W¹ is O, G is H, X is O, and (Z¹)n is 7-S(O)CH₂C≡CBr. |
| Table 1-1061 | R¹ is Me, R² is Me, W¹ is O, G is H, X is S, and (Z¹)n is 5-S(O)CH₂(T-1-1). |
| Table 1-1062 | R¹ is Me, R² is Me, W¹ is O, G is H, X is O, and (Z¹)n is 5-S(O)CH₂(T-1-1). |
| Table 1-1063 | R¹ is Me, R² is Me, W¹ is O, G is H, X is S, and (Z¹)n is 6-S(O)CH₂(T-1-1). |
| Table 1-1064 | R¹ is Me, R² is Me, W¹ is O, G is H, X is O, and (Z¹)n is 6-S(O)CH₂(T-1-1). |
| Table 1-1065 | R¹ is Me, R² is Me, W¹ is O, G is H, X is S, and (Z¹)n is 7-S(O)CH₂(T-1-1). |
| Table 1-1066 | R¹ is Me, R² is Me, W¹ is O, G is H, X is O, and (Z¹)n is 7-S(O)CH₂(T-1-1). |
| Table 1-1067 | R¹ is Me, R² is Me, W¹ is O, G is H, X is S, and (Z¹)n is 5-S(O)CH₂(U-1a). |
| Table 1-1068 | R¹ is Me, R² is Me, W¹ is O, G is H, X is O, and (Z¹)n is 5-S(O)CH₂(U-1a). |
| Table 1-1069 | R¹ is Me, R² is Me, W¹ is O, G is H, X is S, and (Z¹)n is 6-S(O)CH₂(U-1a). |
| Table 1-1070 | R¹ is Me, R² is Me, W¹ is O, G is H, X is O, and (Z¹)n is 6-S(O)CH₂(U-1a). |
| Table 1-1071 | R¹ is Me, R² is Me, W¹ is O, G is H, X is S, and (Z¹)n is 7-S(O)CH₂(U-1a). |
| Table 1-1072 | R¹ is Me, R² is Me, W¹ is O, G is H, X is O, and (Z¹)n is 7-S(O)CH₂(U-1a). |
| Table 1-1073 | R¹ is Me, R² is Me, W¹ is O, G is H, X is S, and (Z¹)n is 5-S(O)CH₂CH₂OMe. |
| Table 1-1074 | R¹ is Me, R² is Me, W¹ is O, G is H, X is O, and (Z¹)n is 5-S(O)CH₂CH₂OMe. |
| Table 1-1075 | R¹ is Me, R² is Me, W¹ is O, G is H, X is S, and (Z¹)n is 6-S(O)CH₂CH₂OMe. |
| Table 1-1076 | R¹ is Me, R² is Me, W¹ is O, G is H, X is O, and (Z¹)n is 6-S(O)CH₂CH₂OMe. |
| Table 1-1077 | R¹ is Me, R² is Me, W¹ is O, G is H, X is S, and (Z¹)n is 7-S(O)CH₂CH₂OMe. |
| Table 1-1078 | R¹ is Me, R² is Me, W¹ is O, G is H, X is O, and (Z¹)n is 7-S(O)CH₂CH₂OMe. |
| Table 1-1079 | R¹ is Me, R² is Me, W¹ is O, G is H, X is S, and (Z¹)n is 5-S(O)CH₂CH₂SMe. |
| Table 1-1080 | R¹ is Me, R² is Me, W¹ is O, G is H, X is O, and (Z¹)n is 5-S(O)CH₂CH₂SMe. |
| Table 1-1081 | R¹ is Me, R² is Me, W¹ is O, G is H, X is S, and (Z¹)n is 6-S(O)CH₂CH₂SMe. |
| Table 1-1082 | R¹ is Me, R² is Me, W¹ is O, G is H, X is O, and (Z¹)n is 6-S(O)CH₂CH₂SMe. |
| Table 1-1083 | R¹ is Me, R² is Me, W¹ is O, G is H, X is S, and (Z¹)n is 7-S(O)CH₂CH₂SMe. |
| Table 1-1084 | R¹ is Me, R² is Me, W¹ is O, G is H, X is O, and (Z¹)n is 7-S(O)CH₂CH₂SMe. |
| Table 1-1085 | R¹ is Me, R² is Me, W¹ is O, G is H, X is S, and (Z¹)n is 5-S(O)CH₂CH₂S(O)Me. |
| Table 1-1086 | R¹ is Me, R² is Me, W¹ is O, G is H, X is O, and (Z¹)n is 5-S(O)CH₂CH₂S(O)Me. |
| Table 1-1087 | R¹ is Me, R² is Me, W¹ is O, G is H, X is S, and (Z¹)n is 6-S(O)CH₂CH₂S(O)Me. |
| Table 1-1088 | R¹ is Me, R² is Me, W¹ is O, G is H, X is O, and (Z¹)n is 6-S(O)CH₂CH₂S(O)Me. |
| Table 1-1089 | R¹ is Me, R² is Me, W¹ is O, G is H, X is S, and (Z¹)n is 7-S(O)CH₂CH₂S(O)Me. |
| Table 1-1090 | R¹ is Me, R² is Me, W¹ is O, G is H, X is O, and (Z¹)n is 7-S(O)CH₂CH₂S(O)Me. |
| Table 1-1091 | R¹ is Me, R² is Me, W¹ is O, G is H, X is S, and (Z¹)ₙ is 5-S(O)CH₂CH₂S(O)₂Me. |
| Table 1-1092 | R¹ is Me, R² is Me, W¹ is O, G is H, X is O, and (Z¹)ₙ is 5-S(O)CH₂CH₂S(O)₂Me. |
| Table 1-1093 | R¹ is Me, R² is Me, W¹ is O, G is H, X is S, and (Z¹)ₙ is 6-S(O)CH₂CH₂S(O)₂Me. |
| Table 1-1094 | R¹ is Me, R² is Me, W¹ is O, G is H, X is O, and (Z¹)ₙ is 6-S(O)CH₂CH₂S(O)₂Me. |
| Table 1-1095 | R¹ is Me, R² is Me, W¹ is O, G is H, X is S, and (Z¹)ₙ is 7-S(O)CH₂CH₂S(O)₂Me. |
| Table 1-1096 | R¹ is Me, R² is Me, W¹ is O, G is H, X is O, and (Z¹)ₙ is 7-S(O)CH₂CH₂S(O)₂Me. |
| Table 1-1097 | R¹ is Me, R² is Me, W¹ is O, G is H, X is S, and (Z¹)ₙ is 5-S(O)₂Me. |
| Table 1-1098 | R¹ is Me, R² is Me, W¹ is O, G is H, X is O, and (Z¹)ₙ is 5-S(O)₂Me. |
| Table 1-1099 | R¹ is Me, R² is Me, W¹ is O, G is H, X is S, and (Z¹)ₙ is 6-S(O)₂Me. |
| Table 1-1100 | R¹ is Me, R² is Me, W¹ is O, G is H, X is O, and (Z¹)ₙ is 6-S(O)₂Me. |
| Table 1-1101 | R¹ is Me, R² is Me, W¹ is O, G is H, X is S, and (Z¹)ₙ is 7-S(O)₂Me. |
| Table 1-1102 | R¹ is Me, R² is Me, W¹ is O, G is H, X is O, and (Z¹)ₙ is 7-S(O)₂Me. |
| Table 1-1103 | R¹ is Me, R² is Me, W¹ is O, G is H, X is S, and (Z¹)ₙ is 5-S(O)₂Et. |
| Table 1-1104 | R¹ is Me, R² is Me, W¹ is O, G is H, X is O, and (Z¹)ₙ is 5-S(O)₂Et. |
| Table 1-1105 | R¹ is Me, R² is Me, W¹ is O, G is H, X is S, and (Z¹)ₙ is 6-S(O)₂Et. |
| Table 1-1106 | R¹ is Me, R² is Me, W¹ is O, G is H, X is O, and (Z¹)ₙ is 6-S(O)₂Et. |
| Table 1-1107 | R¹ is Me, R² is Me, W¹ is O, G is H, X is S, and (Z¹)ₙ is 7-S(O)₂Et. |
| Table 1-1108 | R¹ is Me, R² is Me, W¹ is O, G is H, X is O, and (Z¹)ₙ is 7-S(O)₂Et. |
| Table 1-1109 | R¹ is Me, R² is Me, W¹ is O, G is H, X is S, and (Z¹)ₙ is 5-S(O)₂Pr-n. |
| Table 1-1110 | R¹ is Me, R² is Me, W¹ is O, G is H, X is O, and (Z¹)ₙ is 5-S(O)₂Pr-n. |
| Table 1-1111 | R¹ is Me, R² is Me, W¹ is O, G is H, X is S, and (Z¹)ₙ is 6-S(O)₂Pr-n. |
| Table 1-1112 | R¹ is Me, R² is Me, W¹ is O, G is H, X is O, and (Z¹)ₙ is 6-S(O)₂Pr-n. |
| Table 1-1113 | R¹ is Me, R² is Me, W¹ is O, G is H, X is S, and (Z¹)ₙ is 7-S(O)₂Pr-n. |
| Table 1-1114 | R¹ is Me, R² is Me, W¹ is O, G is H, X is O, and (Z¹)ₙ is 7-S(O)₂Pr-n. |
| Table 1-1115 | R¹ is Me, R² is Me, W¹ is O, G is H, X is S, and (Z¹)ₙ is 5-S(O)₂Pr-i. |
| Table 1-1116 | R¹ is Me, R² is Me, W¹ is O, G is H, X is O, and (Z¹)ₙ is 5-S(O)₂Pr-i. |
| Table 1-1117 | R¹ is Me, R² is Me, W¹ is O, G is H, X is S, and (Z¹)ₙ is 6-S(O)₂Pr-i. |
| Table 1-1118 | R¹ is Me, R² is Me, W¹ is O, G is H, X is O, and (Z¹)ₙ is 6-S(O)₂Pr-i. |
| Table 1-1119 | R¹ is Me, R² is Me, W¹ is O, G is H, X is S, and (Z¹)ₙ is 7-S(O)₂Pr-i. |
| Table 1-1120 | R¹ is Me, R² is Me, W¹ is O, G is H, X is O, and (Z¹)ₙ is 7-S(O)₂Pr-i. |
| Table 1-1121 | R¹ is Me, R² is Me, W¹ is O, G is H, X is S, and (Z¹)ₙ is 5-S(O)₂Bu-n. |
| Table 1-1122 | R¹ is Me, R² is Me, W¹ is O, G is H, X is O, and (Z¹)ₙ is 5-S(O)₂Bu-n. |
| Table 1-1123 | R¹ is Me, R² is Me, W¹ is O, G is H, X is S, and (Z¹)ₙ is 6-S(O)₂Bu-n. |
| Table 1-1124 | R¹ is Me, R² is Me, W¹ is O, G is H, X is O, and (Z¹)ₙ is 6-S(O)₂Bu-n. |
| Table 1-1125 | R¹ is Me, R² is Me, W¹ is O, G is H, X is S, and (Z¹)ₙ is 7-S(O)₂Bu-n. |
| Table 1-1126 | R¹ is Me, R² is Me, W¹ is O, G is H, X is O, and (Z¹)ₙ is 7-S(O)₂Bu-n. |
| Table 1-1127 | R¹ is Me, R² is Me, W¹ is O, G is H, X is S, and (Z¹)ₙ is 5-S(O)₂Bu-i. |
| Table 1-1128 | R¹ is Me, R² is Me, W¹ is O, G is H, X is O, and (Z¹)ₙ is 5-S(O)₂Bu-i. |
| Table 1-1129 | R¹ is Me, R² is Me, W¹ is O, G is H, X is S, and (Z¹)ₙ is 6-S(O)₂Bu-i. |
| Table 1-1130 | R¹ is Me, R² is Me, W¹ is O, G is H, X is O, and (Z¹)ₙ is 6-S(O)₂Bu-i. |
| Table 1-1131 | R¹ is Me, R² is Me, W¹ is O, G is H, X is S, and (Z¹)ₙ is 7-S(O)₂Bu-i. |
| Table 1-1132 | R¹ is Me, R² is Me, W¹ is O, G is H, X is O, and (Z¹)ₙ is 7-S(O)₂Bu-i. |
| Table 1-1133 | R¹ is Me, R² is Me, W¹ is O, G is H, X is S, and (Z¹)ₙ is 5-S(O)₂Bu-sec. |
| Table 1-1134 | R¹ is Me, R² is Me, W¹ is O, G is H, X is O, and (Z¹)ₙ is 5-S(O)₂Bu-sec. |
| Table 1-1135 | R¹ is Me, R² is Me, W¹ is O, G is H, X is S, and (Z¹)ₙ is 6-S(O)₂Bu-sec. |
| Table 1-1136 | R¹ is Me, R² is Me, W¹ is O, G is H, X is O, and (Z¹)ₙ is 6-S(O)₂Bu-sec. |
| Table 1-1137 | R¹ is Me, R² is Me, W¹ is O, G is H, X is S, and (Z¹)ₙ is 7-S(O)₂Bu-sec. |
| Table 1-1138 | R¹ is Me, R² is Me, W¹ is O, G is H, X is O, and (Z¹)ₙ is 7-S(O)₂Bu-sec. |
| Table 1-1139 | R¹ is Me, R² is Me, W¹ is O, G is H, X is S, and (Z¹)n is 5-S(O)₂Bu-tert. |
| Table 1-1140 | R¹ is Me, R² is Me, W¹ is O, G is H, X is O, and (Z¹)ₙ is 5-S(O)₂Bu-tert. |
| Table 1-1141 | R¹ is Me, R² is Me, W¹ is O, G is H, X is S, and (Z¹)ₙ is 6-S(O)₂Bu-tert. |
| Table 1-1142 | R¹ is Me, R² is Me, W¹ is O, G is H, X is O, and (Z¹)ₙ is 6-S(O)₂Bu-tert. |
| Table 1-1143 | R¹ is Me, R² is Me, W¹ is O, G is H, X is S, and (Z¹)ₙ is 7-S(O)₂Bu-tert. |
| Table 1-1144 | R¹ is Me, R² is Me, W¹ is O, G is H, X is O, and (Z¹)ₙ is 7-S(O)₂Bu-tert. |
| Table 1-1145 | R¹ is Me, R² is Me, W¹ is O, G is H, X is S, and (Z¹)ₙ is 5-S(O)₂CH₂CH=CH₂. |
| Table 1-1146 | R¹ is Me, R² is Me, W¹ is O, G is H, X is O, and (Z¹)ₙ is 5-S(O)₂CH₂CH=CH₂. |
| Table 1-1147 | R¹ is Me, R² is Me, W¹ is O, G is H, X is S, and (Z¹)ₙ is 6-S(O)₂CH₂CH=CH₂. |
| Table 1-1148 | R¹ is Me, R² is Me, W¹ is O, G is H, X is O, and (Z¹)ₙ is 6-S(O)₂CH₂CH=CH₂. |
| Table 1-1149 | R¹ is Me, R² is Me, W¹ is O, G is H, X is S, and (Z¹)ₙ is 7-S(O)₂CH₂CH=CH₂. |
| Table 1-1150 | R¹ is Me, R² is Me, W¹ is O, G is H, X is O, and (Z¹)ₙ is 7-S(O)₂CH₂CH=CH₂. |
| Table 1-1151 | R¹ is Me, R² is Me, W¹ is O, G is H, X is S, and (Z¹)ₙ is 5-S(O)₂CH₂C≡CH. |
| Table 1-1152 | R¹ is Me, R² is Me, W¹ is O, G is H, X is O, and (Z¹)ₙ is 5-S(O)₂CH₂C≡CH. |
| Table 1-1153 | R¹ is Me, R² is Me, W¹ is O, G is H, X is S, and (Z¹)ₙ is 6-S(O)₂CH₂C≡CH. |
| Table 1-1154 | R¹ is Me, R² is Me, W¹ is O, G is H, X is O, and (Z¹)ₙ is 6-S(O)₂CH₂C≡CH. |
| Table 1-1155 | R¹ is Me, R² is Me, W¹ is O, G is H, X is S, and (Z¹)ₙ is 7-S(O)₂CH₂C≡CH. |
| Table 1-1156 | R¹ is Me, R² is Me, W¹ is O, G is H, X is O, and (Z¹)ₙ is 7-S(O)₂CH₂C≡CH. |
| Table 1-1157 | R¹ is Me, R² is Me, W¹ is O, G is H, X is S, and (Z¹)ₙ is 5-S(O)₂CHF₂. |
| Table 1-1158 | R¹ is Me, R² is Me, W¹ is O, G is H, X is O, and (Z¹)ₙ is 5-S(O)₂CHF₂. |
| Table 1-1159 | R¹ is Me, R² is Me, W¹ is O, G is H, X is S, and (Z¹)ₙ is 6-S(O)₂CHF₂. |
| Table 1-1160 | R¹ is Me, R² is Me, W¹ is O, G is H, X is O, and (Z¹)ₙ is 6-S(O)₂CHF₂. |
| Table 1-1161 | R¹ is Me, R² is Me, W¹ is O, G is H, X is S, and (Z¹)ₙ is 7-S(O)₂CHF₂. |
| Table 1-1162 | R¹ is Me, R² is Me, W¹ is O, G is H, X is O, and (Z¹)ₙ is 7-S(O)₂CHF₂. |
| Table 1-1163 | R¹ is Me, R² is Me, W¹ is O, G is H, X is S, and (Z¹)ₙ is 5-S(O)₂CF₃. |
| Table 1-1164 | R¹ is Me, R² is Me, W¹ is O, G is H, X is O, and (Z¹)ₙ is 5-S(O)₂CF₃. |
| Table 1-1165 | R¹ is Me, R² is Me, W¹ is O, G is H, X is S, and (Z¹)ₙ is 6-S(O)₂CF₃. |
| Table 1-1166 | R¹ is Me, R² is Me, W¹ is O, G is H, X is O, and (Z¹)ₙ is 6-S(O)₂CF₃. |
| Table 1-1167 | R¹ is Me, R² is Me, W¹ is O, G is H, X is S, and (Z¹)ₙ is 7-S(O)₂CF₃. |
| Table 1-1168 | R¹ is Me, R² is Me, W¹ is O, G is H, X is O, and (Z¹)ₙ is 7-S(O)₂CF₃. |
| Table 1-1169 | R¹ is Me, R² is Me, W¹ is O, G is H, X is S, and (Z¹)ₙ is 5-S(O)₂CH₂CH₂F. |
| Table 1-1170 | R¹ is Me, R² is Me, W¹ is O, G is H, X is O, and (Z¹)ₙ is 5-S(O)₂CH₂CH₂F. |
| Table 1-1171 | R¹ is Me, R² is Me, W¹ is O, G is H, X is S, and (Z¹)ₙ is 6-S(O)₂CH₂CH₂F. |
| Table 1-1172 | R¹ is Me, R² is Me, W¹ is O, G is H, X is O, and (Z¹)ₙ is 6-S(O)₂CH₂CH₂F. |
| Table 1-1173 | R¹ is Me, R² is Me, W¹ is O, G is H, X is S, and (Z¹)ₙ is 7-S(O)₂CH₂CH₂F. |
| Table 1-1174 | R¹ is Me, R² is Me, W¹ is O, G is H, X is O, and (Z¹)ₙ is 7-S(O)₂CH₂CH₂F. |
| Table 1-1175 | R¹ is Me, R² is Me, W¹ is O, G is H, X is S, and (Z¹)ₙ is 5-S(O)₂CH₂CHF₂. |
| Table 1-1176 | R¹ is Me, R² is Me, W¹ is O, G is H, X is O, and (Z¹)ₙ is 5-S(O)₂CH₂CHF₂. |
| Table 1-1177 | R¹ is Me, R² is Me, W¹ is O, G is H, X is S, and (Z¹)ₙ is 6-S(O)₂CH₂CHF₂. |
| Table 1-1178 | R¹ is Me, R² is Me, W¹ is O, G is H, X is O, and (Z¹)ₙ is 6-S(O)₂CH₂CHF₂. |
| Table 1-1179 | R¹ is Me, R² is Me, W¹ is O, G is H, X is S, and (Z¹)ₙ is 7-S(O)₂CH₂CHF₂. |
| Table 1-1180 | R¹ is Me, R² is Me, W¹ is O, G is H, X is O, and (Z¹)ₙ is 7-S(O)₂CH₂CHF₂. |
| Table 1-1181 | R¹ is Me, R² is Me, W¹ is O, G is H, X is S, and (Z¹)ₙ is 5-S(O)₂CH₂CF₃. |
| Table 1-1182 | R¹ is Me, R² is Me, W¹ is O, G is H, X is O, and (Z¹)ₙ is 5-S(O)₂CH₂CF₃. |
| Table 1-1183 | R¹ is Me, R² is Me, W¹ is O, G is H, X is S, and (Z¹)ₙ is 6-S(O)₂CH₂CF₃. |
| Table 1-1184 | R¹ is Me, R² is Me, W¹ is O, G is H, X is O, and (Z¹)ₙ is 6-S(O)₂CH₂CF₃. |
| Table 1-1185 | R¹ is Me, R² is Me, W¹ is O, G is H, X is S, and (Z¹)ₙ is 7-S(O)₂CH₂CF₃. |
| Table 1-1186 | R¹ is Me, R² is Me, W¹ is O, G is H, X is O, and (Z¹)ₙ is 7-S(O)₂CH₂CF₃. |
| Table 1-1187 | R¹ is Me, R² is Me, W¹ is O, G is H, X is S, and (Z¹)ₙ is 5-S(O)₂CH₂CH=C(F)₂. |
| Table 1-1188 | R¹ is Me, R² is Me, W¹ is O, G is H, X is O, and (Z¹)ₙ is 5-S(O)₂CH₂CH=C(F)₂ |
| Table 1-1189 | R¹ is Me, R² is Me, W¹ is O, G is H, X is S, and (Z¹)ₙ is 6-S(O)₂CH₂CH=C(F)₂. |
| Table 1-1190 | R¹ is Me, R² is Me, W¹ is O, G is H, X is O, and (Z¹)ₙ is 6-S(O)₂CH₂CH=C(F)₂ |
| Table 1-1191 | R¹ is Me, R² is Me, W¹ is O, G is H, X is S, and (Z¹)ₙ is 7-S(O)₂CH₂CH=C(F)₂. |
| Table 1-1192 | R¹ is Me, R² is Me, W¹ is O, G is H, X is O, and (Z¹)ₙ is 7-S(O)₂CH₂CH=C(F)₂. |
| Table 1-1193 | R¹ is Me, R² is Me, W¹ is O, G is H, X is S, and (Z¹)ₙ is 5-S(O)₂CH₂CH=C(Cl)₂. |
| Table 1-1194 | R¹ is Me, R² is Me, W¹ is O, G is H, X is O, and (Z¹)ₙ is 5-S(O)₂CH₂CH=C(Cl)₂. |
| Table 1-1195 | R¹ is Me, R² is Me, W¹ is O, G is H, X is S, and (Z¹)ₙ is 6-S(O)₂CH₂CH=C(Cl)₂. |
| Table 1-1196 | R¹ is Me, R² is Me, W¹ is O, G is H, X is O, and (Z¹)ₙ is 6-S(O)₂CH₂CH=C(Cl)₂. |
| Table 1-1197 | R¹ is Me, R² is Me, W¹ is O, G is H, X is S, and (Z¹)ₙ is 7-S(O)₂CH₂CH=C(Cl)₂. |
| Table 1-1198 | R¹ is Me, R² is Me, W¹ is O, G is H, X is O, and (Z¹)ₙ is 7-S(O)₂CH₂CH=C(Cl)₂. |
| Table 1-1199 | R¹ is Me, R² is Me, W¹ is O, G is H, X is S, and (Z¹)ₙ is 5-S(O)₂CH₂C≡CCl. |
| Table 1-1200 | R¹ is Me, R² is Me, W¹ is O, G is H, X is O, and (Z¹)ₙ is 5-S(O)₂CH₂C≡CCl. |
| Table 1-1201 | R¹ is Me, R² is Me, W¹ is O, G is H, X is S, and (Z¹)ₙ is 6-S(O)₂CH₂C≡CCl. |
| Table 1-1202 | R¹ is Me, R² is Me, W¹ is O, G is H, X is O, and (Z¹)ₙ is 6-S(O)₂CH₂C≡CCl. |
| Table 1-1203 | R¹ is Me, R² is Me, W¹ is O, G is H, X is S, and (Z¹)ₙ is 7-S(O)₂CH₂C≡CCl. |
| Table 1-1204 | R¹ is Me, R² is Me, W¹ is O, G is H, X is O, and (Z¹)ₙ is 7-S(O)₂CH₂C≡CCl. |
| Table 1-1205 | R¹ is Me, R² is Me, W¹ is O, G is H, X is S, and (Z¹)ₙ is 5-S(O)₂CH₂C≡CBr. |
| Table 1-1206 | R¹ is Me, R² is Me, W¹ is O, G is H, X is O, and (Z¹)ₙ is 5-S(O)₂CH₂C≡CBr. |
| Table 1-1207 | R¹ is Me, R² is Me, W¹ is O, G is H, X is S, and (Z¹)ₙ is 6-S(O)₂CH₂C≡CBr. |
| Table 1-1208 | R¹ is Me, R² is Me, W¹ is O, G is H, X is O, and (Z¹)ₙ is 6-S(O)₂CH₂C≡CBr. |
| Table 1-1209 | R¹ is Me, R² is Me, W¹ is O, G is H, X is S, and (Z¹)ₙ is 7-S(O)₂CH₂C≡CBr. |
| Table 1-1210 | R¹ is Me, R² is Me, W¹ is O, G is H, X is O, and (Z¹)ₙ is 7-S(O)₂CH₂C≡CBr. |
| Table 1-1211 | R¹ is Me, R² is Me, W¹ is O, G is H, X is S, and (Z¹)ₙ is 5-S(O)₂CH₂(T-1-1). |
| Table 1-1212 | R¹ is Me, R² is Me, W¹ is O, G is H, X is O, and (Z¹)ₙ is 5-S(O)₂CH₂(T-1-1). |
| Table 1-1213 | R¹ is Me, R² is Me, W¹ is O, G is H, X is S, and (Z¹)ₙ is 6-S(O)₂CH₂(T-1-1). |
| Table 1-1214 | R¹ is Me, R² is Me, W¹ is O, G is H, X is O, and (Z¹)ₙ is 6-S(O)₂CH₂(T-1-1). |
| Table 1-1215 | R¹ is Me, R² is Me, W¹ is O, G is H, X is S, and (Z¹)ₙ is 7-S(O)₂CH₂(T-1-1). |
| Table 1-1216 | R¹ is Me, R² is Me, W¹ is O, G is H, X is O, and (Z¹)ₙ is 7-S(O)₂CH₂(T-1-1). |
| Table 1-1217 | R¹ is Me, R² is Me, W¹ is O, G is H, X is S, and (Z¹)ₙ is 5-S(O)₂CH₂(U-1a). |
| Table 1-1218 | R¹ is Me, R² is Me, W¹ is O, G is H, X is O, and (Z¹)ₙ is 5-S(O)₂CH₂(U-1a). |
| Table 1-1219 | R¹ is Me, R² is Me, W¹ is O, G is H, X is S, and (Z¹)ₙ is 6-S(O)₂CH₂(U-1a). |
| Table 1-1220 | R¹ is Me, R² is Me, W¹ is O, G is H, X is O, and (Z¹)ₙ is 6-S(O)₂CH₂(U-1a). |
| Table 1-1221 | R¹ is Me, R² is Me, W¹ is O, G is H, X is S, and (Z¹)ₙ is 7-S(O)₂CH₂(U-1a). |
| Table 1-1222 | R¹ is Me, R² is Me, W¹ is O, G is H, X is O, and (Z¹)ₙ is 7-S(O)₂CH₂(U-1a). |
| Table 1-1223 | R¹ is Me, R² is Me, W¹ is O, G is H, X is S, and (Z¹)ₙ is 5-S(O)₂CH₂CH₂OMe. |
| Table 1-1224 | R¹ is Me, R² is Me, W¹ is O, G is H, X is O, and (Z¹)ₙ is 5-S(O)₂CH₂CH₂OMe. |
| Table 1-1225 | R¹ is Me, R² is Me, W¹ is O, G is H, X is S, and (Z¹)ₙ is 6-S(O)₂CH₂CH₂OMe. |
| Table 1-1226 | R¹ is Me, R² is Me, W¹ is O, G is H, X is O, and (Z¹)ₙ is 6-S(O)₂CH₂CH₂OMe. |
| Table 1-1227 | R¹ is Me, R² is Me, W¹ is O, G is H, X is S, and (Z¹)ₙ is 7-S(O)₂CH₂CH₂OMe. |
| Table 1-1228 | R¹ is Me, R² is Me, W¹ is O, G is H, X is O, and (Z¹)ₙ is 7-S(O)₂CH₂CH₂OMe. |
| Table 1-1229 | R¹ is Me, R² is Me, W¹ is O, G is H, X is S, and (Z¹)ₙ is 5-S(O)₂CH₂CH₂SMe. |
| Table 1-1230 | R¹ is Me, R² is Me, W¹ is O, G is H, X is O, and (Z¹)ₙ is 5-S(O)₂CH₂CH₂SMe. |
| Table 1-1231 | R¹ is Me, R² is Me, W¹ is O, G is H, X is S, and (Z¹)ₙ is 6-S(O)₂CH₂CH₂SMe. |
| Table 1-1232 | R¹ is Me, R² is Me, W¹ is O, G is H, X is O, and (Z¹)ₙ is 6-S(O)₂CH₂CH₂SMe. |
| Table 1-1233 | R¹ is Me, R² is Me, W¹ is O, G is H, X is S, and (Z¹)ₙ is 7-S(O)₂CH₂CH₂SMe. |
| Table 1-1234 | R¹ is Me, R² is Me, W¹ is O, G is H, X is O, and (Z¹)ₙ is 7-S(O)₂CH₂CH₂SMe. |
| Table 1-1235 | R¹ is Me, R² is Me, W¹ is O, G is H, X is S, and (Z¹)ₙ is 5-S(O)₂CH₂CH₂S(O)Me. |
| Table 1-1236 | R¹ is Me, R² is Me, W¹ is O, G is H, X is O, and (Z¹)ₙ is 5-S(O)₂CH₂CH₂S(O)Me. |
| Table 1-1237 | R¹ is Me, R² is Me, W¹ is O, G is H, X is S, and (Z¹)ₙ is 6-S(O)₂CH₂CH₂S(O)Me. |
| Table 1-1238 | R¹ is Me, R² is Me, W¹ is O, G is H, X is O, and (Z¹)ₙ is 6-S(O)₂CH₂CH₂S(O)Me. |
| Table 1-1239 | R¹ is Me, R² is Me, W¹ is O, G is H, X is S, and (Z¹)ₙ is 7-S(O)₂CH₂CH₂S(O)Me. |
| Table 1-1240 | R¹ is Me, R² is Me, W¹ is O, G is H, X is O, and (Z¹)ₙ is 7-S(O)₂CH₂CH₂S(O)Me. |
| Table 1-1241 | R¹ is Me, R² is Me, W¹ is O, G is H, X is S, and (Z¹)ₙ is 5-S(O)₂CH₂CH₂S(O)₂Me. |
| Table 1-1242 | R¹ is Me, R² is Me, W¹ is O, G is H, X is O, and (Z¹)ₙ is 5-S(O)₂CH₂CH₂S(O)₂Me |
| Table 1-1243 | R¹ is Me, R² is Me, W¹ is O, G is H, X is S, and (Z¹)ₙ is 6-S(O)₂CH₂CH₂S(O)₂Me. |
| Table 1-1244 | R¹ is Me, R² is Me, W¹ is O, G is H, X is O, and (Z¹)ₙ is 6-S(O)₂CH₂CH₂S(O)₂Me |
| Table 1-1245 | R¹ is Me, R² is Me, W¹ is O, G is H, X is S, and (Z¹)ₙ is 7-S(O)₂CH₂CH₂S(O)₂Me. |
| Table 1-1246 | R¹ is Me, R² is Me, W¹ is O, G is H, X is O, and (Z¹)ₙ is 7-S(O)₂CH₂CH₂S(O)₂Me |
| Table 1-1247 | R¹ is Me, R² is Me, W¹ is O, G is H, X is S, and (Z¹)ₙ is 5,6-F₂. |
| Table 1-1248 | R¹ is Me, R² is Me, W¹ is O, G is H, X is O, and (Z¹)ₙ is 5,6-F₂. |
| Table 1-1249 | R¹ is Me, R² is Me, W¹ is O, G is H, X is S, and (Z¹)ₙ is 5,7-F₂. |
| Table 1-1250 | R¹ is Me, R² is Me, W¹ is O, G is H, X is O, and (Z¹)ₙ is 5,7-F₂. |
| Table 1-1251 | R¹ is Me, R² is Me, W¹ is O, G is H, X is S, and (Z¹)ₙ is 6,7-F₂. |
| Table 1-1252 | R¹ is Me, R² is Me, W¹ is O, G is H, X is O, and (Z¹)ₙ is 6,7-F₂. |
| Table 1-1253 | R¹ is Me, R² is Me, W¹ is O, G is H, X is S, and (Z¹)ₙ is 5,6-Cl₂. |
| Table 1-1254 | R¹ is Me, R² is Me, W¹ is O, G is H, X is O, and (Z¹)ₙ is 5,6-Cl₂. |
| Table 1-1255 | R¹ is Me, R² is Me, W¹ is O, G is H, X is S, and (Z¹)ₙ is 5,7-Cl₂. |
| Table 1-1256 | R¹ is Me, R² is Me, W¹ is O, G is H, X is O, and (Z¹)ₙ is 5,7-Cl₂. |
| Table 1-1257 | R¹ is Me, R² is Me, W¹ is O, G is H, X is S, and (Z¹)ₙ is 6,7-Cl₂. |
| Table 1-1258 | R¹ is Me, R² is Me, W¹ is O, G is H, X is O, and (Z¹)ₙ is 6,7-Cl₂. |
| Table 1-1259 | R¹ is Me, R² is Me, W¹ is O, G is H, X is S, and (Z¹)ₙ is 5,6-(Me)₂. |
| Table 1-1260 | R¹ is Me, R² is Me, W¹ is O, G is H, X is O, and (Z¹)ₙ is 5,6-(Me)₂. |
| Table 1-1261 | R¹ is Me, R² is Me, W¹ is O, G is H, X is S, and (Z¹)ₙ is 5,7-(Me)₂. |
| Table 1-1262 | R¹ is Me, R² is Me, W¹ is O, G is H, X is O, and (Z¹)ₙ is 5,7-(Me)₂. |
| Table 1-1263 | R¹ is Me, R² is Me, W¹ is O, G is H, X is S, and (Z¹)ₙ is 6,7-(Me)₂. |
| Table 1-1264 | R¹ is Me, R² is Me, W¹ is O, G is H, X is O, and (Z¹)ₙ is 6,7-(Me)₂. |
| Table 1-1265 | R¹ is Me, R² is Me, W¹ is O, G is H, X is S, and (Z¹)ₙ is 5,6-(OMe)₂. |
| Table 1-1266 | R¹ is Me, R² is Me, W¹ is O, G is H, X is O, and (Z¹)ₙ is 5,6-(OMe)₂. |
| Table 1-1267 | R¹ is Me, R² is Me, W¹ is O, G is H, X is S, and (Z¹)ₙ is 5,7-(OMe)₂. |
| Table 1-1268 | R¹ is Me, R² is Me, W¹ is O, G is H, X is O, and (Z¹)ₙ is 5,7-(OMe)₂. |
| Table 1-1269 | R¹ is Me, R² is Me, W¹ is O, G is H, X is S, and (Z¹)ₙ is 6,7-(Me)₂. |
| Table 1-1270 | R¹ is Me, R² is Me, W¹ is O, G is H, X is O, and (Z¹)ₙ is 6,7-(Me)₂. |
| Table 1-1271 | R¹ is Me, R² is Me, W¹ is O, G is H, X is S, and (Z¹)ₙ is 5-F-6-OMe. |
| Table 1-1272 | R¹ is Me, R² is Me, W¹ is O, G is H, X is O, and (Z¹)ₙ is 5-F-6-OMe. |
| Table 1-1273 | R¹ is Me, R² is Me, W¹ is O, G is H, X is S, and (Z¹)ₙ is 5-F-6-OEt. |
| Table 1-1274 | R¹ is Me, R² is Me, W¹ is O, G is H, X is O, and (Z¹)ₙ is 5-F-6-OEt. |
| Table 1-1275 | R¹ is Me, R² is Me, W¹ is O, G is H, X is S, and (Z¹)ₙ is 5-F-6-OPr-n. |
| Table 1-1276 | R¹ is Me, R² is Me, W¹ is O, G is H, X is O, and (Z¹)ₙ is 5-F-6-OPr-n. |
| Table 1-1277 | R¹ is Me, R² is Me, W¹ is O, G is H, X is S, and (Z¹)ₙ is 5-F-6-OPr-i. |
| Table 1-1278 | R¹ is Me, R² is Me, W¹ is O, G is H, X is O, and (Z¹)ₙ is 5-F-6-OPr-i. |
| Table 1-1279 | R¹ is Me, R² is Me, W¹ is O, G is H, X is S, and (Z¹)ₙ is 5-F-6-OCHF₂. |
| Table 1-1280 | R¹ is Me, R² is Me, W¹ is O, G is H, X is O, and (Z¹)ₙ is 5-F-6-OCHF₂. |
| Table 1-1281 | R¹ is Me, R² is Me, W¹ is O, G is H, X is S, and (Z¹)ₙ is 5-F-6-OCF₃. |
| Table 1-1282 | R¹ is Me, R² is Me, W¹ is O, G is H, X is O, and (Z¹)ₙ is 5-F-6-OCF₃. |
| Table 1-1283 | R¹ is Me, R² is Me, W¹ is O, G is H, X is S, and (Z¹)ₙ is 5-F-6-OCH₂CH₂F. |
| Table 1-1284 | R¹ is Me, R² is Me, W¹ is O, G is H, X is O, and (Z¹)ₙ is 5-F-6-OCH₂CH₂F. |
| Table 1-1285 | R¹ is Me, R² is Me, W¹ is O, G is H, X is S, and (Z¹)ₙ is 5-F-6-OCH₂CHF₂. |
| Table 1-1286 | R¹ is Me, R² is Me, W¹ is O, G is H, X is O, and (Z¹)ₙ is 5-F-6-OCH₂CHF₂. |
| Table 1-1287 | R¹ is Me, R² is Me, W¹ is O, G is H, X is S, and (Z¹)ₙ is 5-F-6-OCH₂CF₃. |
| Table 1-1288 | R¹ is Me, R² is Me, W¹ is O, G is H, X is O, and (Z¹)ₙ is 5-F-6-OCH₂CF₃. |
| Table 1-1289 | R¹ is Me, R² is Me, W¹ is O, G is H, X is S, and (Z¹)ₙ is 5-Cl-6-OMe. |
| Table 1-1290 | R¹ is Me, R² is Me, W¹ is O, G is H, X is O, and (Z¹)ₙ is 5-Cl-6-OMe. |
| Table 1-1291 | R¹ is Me, R² is Me, W¹ is O, G is H, X is S, and (Z¹)ₙ is 5-Cl-6-OEt. |
| Table 1-1292 | R¹ is Me, R² is Me, W¹ is O, G is H, X is O, and (Z¹)ₙ is 5-Cl-6-OEt. |
| Table 1-1293 | R¹ is Me, R² is Me, W¹ is O, G is H, X is S, and (Z¹)ₙ is 5-Cl-6-OPr-n. |
| Table 1-1294 | R¹ is Me, R² is Me, W¹ is O, G is H, X is O, and (Z¹)ₙ is 5-Cl-6-OPr-n. |
| Table 1-1295 | R¹ is Me, R² is Me, W¹ is O, G is H, X is S, and (Z¹)ₙ is 5-Cl-6-OPr-i. |
| Table 1-1296 | R¹ is Me, R² is Me, W¹ is O, G is H, X is O, and (Z¹)ₙ is 5-Cl-6-OPr-i. |
| Table 1-1297 | R¹ is Me, R² is Me, W¹ is O, G is H, X is S, and (Z¹)ₙ is 5-Cl-6-OCHF₂. |
| Table 1-1298 | R¹ is Me, R² is Me, W¹ is O, G is H, X is O, and (Z¹)ₙ is 5-Cl-6-OCHF₂. |
| Table 1-1299 | R¹ is Me, R² is Me, W¹ is O, G is H, X is S, and (Z¹)ₙ is 5-Cl-6-OCF₃. |
| Table 1-1300 | R¹ is Me, R² is Me, W¹ is O, G is H, X is O, and (Z¹)ₙ is 5-Cl-6-OCF₃. |
| Table 1-1301 | R¹ is Me, R² is Me, W¹ is O, G is H, X is S, and (Z¹)ₙ is 5-Cl-6-OCH₂CH₂F. |
| Table 1-1302 | R¹ is Me, R² is Me, W¹ is O, G is H, X is O, and (Z¹)ₙ is 5-Cl-6-OCH₂CH₂F. |
| Table 1-1303 | R¹ is Me, R² is Me, W¹ is O, G is H, X is S, and (Z¹)ₙ is 5-Cl-6-OCH₂CHF₂. |
| Table 1-1304 | R¹ is Me, R² is Me, W¹ is O, G is H, X is O, and (Z¹)ₙ is 5-Cl-6-OCH₂CHF₂. |
| Table 1-1305 | R¹ is Me, R² is Me, W¹ is O, G is H, X is S, and (Z¹)ₙ is 5-Cl-6-OCH₂CF₃. |
| Table 1-1306 | R¹ is Me, R² is Me, W¹ is O, G is H, X is O, and (Z¹)ₙ is 5-Cl-6-OCH₂CF₃. |
| Table 1-1307 | R¹ is Me, R² is Me, W¹ is O, G is H, X is S, and (Z¹)ₙ is 6-OMe-7-Cl. |
| Table 1-1308 | R¹ is Me, R² is Me, W¹ is O, G is H, X is O, and (Z¹)ₙ is 6-OMe-7-Cl. |
| Table 1-1309 | R¹ is Me, R² is Me, W¹ is O, G is H, X is S, and (Z¹)ₙ is 6-OEt-7-Cl. |
| Table 1-1310 | R¹ is Me, R² is Me, W¹ is O, G is H, X is O, and (Z¹)ₙ is 6-OEt-7-Cl. |
| Table 1-1311 | R¹ is Me, R² is Me, W¹ is O, G is H, X is S, and (Z¹)ₙ is 6-OPr-n-7-Cl. |
| Table 1-1312 | R¹ is Me, R² is Me, W¹ is O, G is H, X is O, and (Z¹)ₙ is 6-OPr-n-7-Cl. |
| Table 1-1313 | R¹ is Me, R² is Me, W¹ is O, G is H, X is S, and (Z¹)ₙ is 6-OPr-i-7-Cl. |
| Table 1-1314 | R¹ is Me, R² is Me, W¹ is O, G is H, X is O, and (Z¹)ₙ is 6-OPr-i-7-Cl. |
| Table 1-1315 | R¹ is Me, R² is Me, W¹ is O, G is H, X is S, and (Z¹)ₙ is 6-OCHF₂-7-Cl. |
| Table 1-1316 | R¹ is Me, R² is Me, W¹ is O, G is H, X is O, and (Z¹)ₙ is 6-OCHF₂-7-Cl. |
| Table 1-1317 | R¹ is Me, R² is Me, W¹ is O, G is H, X is S, and (Z¹)ₙ is 6-OCF₃-7-Cl. |
| Table 1-1318 | R¹ is Me, R² is Me, W¹ is O, G is H, X is O, and (Z¹)ₙ is 6-OCF₃-7-Cl. |
| Table 1-1319 | R¹ is Me, R² is Me, W¹ is O, G is H, X is S, and (Z¹)ₙ is 6-OCH₂CH₂F-7-Cl. |
| Table 1-1320 | R¹ is Me, R² is Me, W¹ is O, G is H, X is O, and (Z¹)ₙ is 6-OCH₂CH₂F-7-Cl. |
| Table 1-1321 | R¹ is Me, R² is Me, W¹ is O, G is H, X is S, and (Z¹)ₙ is 6-OCH₂CHF₂-7-Cl. |
| Table 1-1322 | R¹ is Me, R² is Me, W¹ is O, G is H, X is O, and (Z¹)ₙ is 6-OCH₂CHF₂-7-Cl. |
| Table 1-1323 | R¹ is Me, R² is Me, W¹ is O, G is H, X is S, and (Z¹)ₙ is 6-OCH₂CF₃-7-Cl. |
| Table 1-1324 | R¹ is Me, R² is Me, W¹ is O, G is H, X is O, and (Z¹)ₙ is 6-OCH₂CF₃-7-Cl. |
| Table 1-1325 | R¹ is Me, R² is Me, W¹ is O, G is H, X is S, and (Z¹)ₙ is 6-OMe-7-Br. |
| Table 1-1326 | R¹ is Me, R² is Me, W¹ is O, G is H, X is O, and (Z¹)ₙ is 6-OMe-7-Br. |
| Table 1-1327 | R¹ is Me, R² is Me, W¹ is O, G is H, X is S, and (Z¹)ₙ is 6-OEt-7-Br. |
| Table 1-1328 | R¹ is Me, R² is Me, W¹ is O, G is H, X is O, and (Z¹)ₙ is 6-OEt-7-Br. |
| Table 1-1329 | R¹ is Me, R² is Me, W¹ is O, G is H, X is S, and (Z¹)ₙ is 6-OPr-n-7-Br. |
| Table 1-1330 | R¹ is Me, R² is Me, W¹ is O, G is H, X is O, and (Z¹)ₙ is 6-OPr-n-7-Br. |
| Table 1-1331 | R¹ is Me, R² is Me, W¹ is O, G is H, X is S, and (Z¹)ₙ is 6-OPr-i-7-Br. |
| Table 1-1332 | R¹ is Me, R² is Me, W¹ is O, G is H, X is O, and (Z¹)ₙ is 6-OPr-i-7-Br. |
| Table 1-1333 | R¹ is Me, R² is Me, W¹ is O, G is H, X is S, and (Z¹)ₙ is 6-OCHF₂-7-Br. |
| Table 1-1334 | R¹ is Me, R² is Me, W¹ is O, G is H, X is O, and (Z¹)ₙ is 6-OCHF₂-7-Br. |
| Table 1-1335 | R¹ is Me, R² is Me, W¹ is O, G is H, X is S, and (Z¹)ₙ is 6-OCF₃-7-Br. |
| Table 1-1336 | R¹ is Me, R² is Me, W¹ is O, G is H, X is O, and (Z¹)ₙ is 6-OCF₃-7-Br. |
| Table 1-1337 | R¹ is Me, R² is Me, W¹ is O, G is H, X is S, and (Z¹)ₙ is 6-OCH₂CH₂F-7-Br. |
| Table 1-1338 | R¹ is Me, R² is Me, W¹ is O, G is H, X is O, and (Z¹)ₙ is 6-OCH₂CH₂F-7-Br. |
| Table 1-1339 | R¹ is Me, R² is Me, W¹ is O, G is H, X is S, and (Z¹)ₙ is 6-OCH₂CHF₂-7-Br. |
| Table 1-1340 | R¹ is Me, R² is Me, W¹ is O, G is H, X is O, and (Z¹)ₙ is 6-OCH₂CHF₂-7-Br. |
| Table 1-1341 | R¹ is Me, R ² is Me, W¹ is O, G is H, X is S, and (Z¹)ₙ is 6-OCH₂CF₃-7-Br. |
| Table 1-1342 | R¹ is Me, R² is Me, W¹ is O, G is H, X is O, and (Z¹)ₙ is 6-OCH₂CF₃-7-Br. |

The compound of the present invention can be used as a herbicide for paddy fields in both soil treatment and foliage treatment under submerged conditions. Examples of paddy field weeds include Gramineae weeds, such as chinese sprangletop (*Leptochloa chinensis),* bearded sprangletop (*Leptochloafascicularis*)*,* barnyard grass (*Echinochloa crus-galli*)*,* junglerice (*Echinochloa colonum),* late watergrass (*Echinochloa oryzicola),* southern cutgrass (*Leersia hexandra*)*,* knotgrass (*Paspalum distichum)* saramollagrass *(Ischaemum rugosum),* itchgrass (*Rottboellia cochinchinensis),* broadleaf signalgrass (*Brachiaria platyphylla*)*,* Alexandergrass (*Brachiaria plantaginea*)*,* large crabgrass *(Digitaria sanguinalis*)*,* crowfoot grass *(Dactyloctenium aegyptium*)*,* goosegrass (*Eleusine indica),* red rice *(Oryza sativa*)*,* bermuda grass *(Cynodon dactylon),* and fall panicum *(Panicum dichotomiflorum);* Cyperaceae weeds, such as *Eleocharis kuroguwai,* globe fringerush (*Fimbristylis miliacea*)*,* Japanese bulrush *(Schoenoplectusjuncoides), Schoenoplectus nipponicus,* ricefield bulrush *(Schoenoplectus mucronatus), Cyperus serotinus,* smallflower umbrella sedge *(Cyperus difformis*), rice flat sedge *(Cyperus iria*)*,* purple nutsedge *(Cyperus rotundus*)*,* yellow nutsedge *(Cyperus esculentus),* and cosmopolitan bulrush *(Bolboschoenus martimus);* Alismataceae weeds, such as water plantain (*Alisma canaliculatum),* pygmy arrowhead (*Sagittaria pygmaea*)*,* and threeleaf arrowhead (*Sagittaria trifolia*)*;* Commelinaceae weeds, such as asian spiderwort (*Murdannia keisak)* and benghal dayflower (*Commelina benghalensis);* Pontederiaceae weeds, such as heartleaf false pickerelweed (*Monochoria korsakowii*)*,* oval-leafed pondweed (*Monochoria vaginalis),* ducksalad (*Heteranthera limosa*)*,* and water hyacinth (*Eichhornia crassipes*)*;* Elatinaceae weeds, such as threestamen waterwort (*Elatine triandra*)*;* Lythraceae weeds, such as redstem (*Ammannia coccinea*) and indian toothcup *(Rotala indica);* Oenotheraceae weeds, such as *Ludwigia epilobioides* and mexican primrose-willow (*Ludwigia octovalvis);* Scrophulariaceae weeds, such as rushlike dopatrium (*Dopatrium junceum), Gratiola japonica,* dwarf ambulia (*Limnophila sessiliflora*)*,* prostrate false pimpernel (*Lindernia pyxidaria*)*,* and yellowseed false pimpernel (*Lindernia dubia*)*;* Amaranthaceae weeds, such as alligator weed *(Alternanthera philoxeroides)* and spiny amaranth *(Amaranthus spinosus);* Polygonaceae weeds, such as water pepper *(Polygonum hydropiper);* Sphenocleaceae weeds, such as gooseweed *(Sphenoclea zeylanica*)*;* Fabaceae weeds, Indian jointvetch *(Aeschynomene indica)* and hemp sesbania (*Sesbania exaltata*)*;* Asteraceae weeds, such as devil's beggarticks (*Bidens frondosa*)*,* three-lobe beggarticks (*Bidens tripartita*)*,* false daisy (*Eclipta prostrata*)*,* and goatweed *(Ageratum conyzoides);* Convolvulaceae weeds, such as swamp morningglory (*Ipomoea aquatica*)*;* Marsileaceae weeds, such as water clover (*Marsilea minuta*)*;* Lemnaceae weeds, such as common duckmeat (*Spirodela polyrhiza*) and duckweed (*Lemna paucicostata*)*;* and Potamogetonaceae weeds, such as roundleaf pondweed *(Potamogeton distinctus).*

When the composition of the present invention is used in paddy fields, the treatment can be performed before and after rice planting in a usual manner, and can also be performed in parallel with rice planting.

The compound of the present invention can also be used as a herbicide for farmlands and orchards in any of soil treatment, soil incorporation treatment, and foliage treatment. Examples of farmland weeds include Poaceae weeds, such as fall panicum *(Panicum dichotomiflorum),* shattercane *(Sorgham bicolor),* Johnson grass *(Sorghum halepense),* barnyard grass (*Echinochloa crus-galli var. crus-galli*)*,* cockspur grass (*Echinochloa crus-galli var. praticola),* Japanese barnyard millet (*Echinochloa utilis),* southern crabgrass *(Digitaria ciliaris),* sourgrass *(Digitaria insularis),* Jamaican crabgrass *(Digitaria horizontalis),* wild oat (*Avena fatua*)*,* blackgrass *(Alopecurus myosuroides),* shortawn foxtail *(Alopecurus aequalis*)*,* windgrass (*Apera spica-venti),* downy brome *(Bromus tectorum),* Italian ryegrass *(Lolium multiflorum),* rigid ryegrass *(Lolium rigidum),* littleseed canarygrass (*Phalaris minor),* annual bluegrass (*Poa annua*)*,* goosegrass (*Eleusine indica),* green foxtail *(Setaria viridis),* giant foxtail *(Setariafaberi),* signalgrass *(Brachiaria decumbens),* and southern sandbur *(Cenchrus echinatus);* Cyperaceae weeds, such as purple nutsedge *(Cyperus rotundus*)*;* Solanaceae weeds, such as black nightshade (*Solanum nigrum)* and jimsonweed *(Datura stramonium*)*;* Malvaceae weeds, velvetleaf *(Abutilon theophrasti*) and prickly sida (*Sida spinosa*)*;* Convolvulaceae weeds, such as tall morning-glory (*Ipomoeapurpurea*)*,* ivyleaf morning-glory (*Ipomoea hederacea*)*,* and Japanese bindweed *(Calystegia hederacea);* Amaranthaceae weeds, such as purple amaranth *(Amaranthus lividus),* redroot pigweed *(Amaranthus retroflexus),* palmer amaranth (*Amaranthus palmeri*)*,* and tall waterhemp *(Amaranthus tuberculatus);* Asteraceae weeds, such as common cocklebur *(Xanthium strumarium),* common ragweed (*Ambrosia artemisiifolia*)*,* giant ragweed (*Ambrosia trifida),* horseweed (*Conyza canadensis),* common sunflower *(Helianthus annuus),* pineappleweed *(Matricaria matricarioides),* hairy galinsoga (*Galinsoga ciliata*)*,* Canada thistle *(Cirsium arvense),* common groundsel *(Senecio vulgaris),* and annual fleabane *(Erigeron annuus*)*;* Brassicaceae weeds, such as variableleaf yellowcress (*Rorippa indica),* wild mustard *(Sinapis arvensis*)*,* and shepherd's purse *(Capsella bursa-pastoris);* Polygonaceae weeds, such as oriental lady's thumb *(Persicaria longiseta*) and wild buckwheat *(Polygonum convolvulus);* Portulacaceae weeds, such as common purslane *(Portulaca oleracea);* Chenopodiaceae weeds, such as lambsquarters *(Chenopodium album*)*,* figleaved goosefoot (*Chenopodium ficifolium*)*,* kochia *(Kochia scoparia),* and Russian thistle (*Salsola tragus*); Caryophyllaceae weeds, such as common chickweed (*Stellaria media*)*;* Plantaginaceae weeds, such as Persian speedwell (*Veronica persica*); Commelinaceae weeds, such as Asiatic dayflower *(Commelina communis)* and Benghal dayflowe *(Commelina benghalensis);* Lamiaceae weeds, such as henbit *(Lamium amplexicaule*) and purple deadnettle (*Lamiumpurpureum*); Euphorbiaceae weeds, such as wild poinsettia *(Euphorbia heterophylla*) and spotted spurge *(Euphorbia maculata*); Rubiaceae weeds, such as false cleavers *(Galium spurium)* and Asian madder (*Rubia akane*); Violaceae weeds, such as pansy *(Viola tricolor);* Papaveraceae weeds, such as filed poppy *(Papaver rhoeas);* Fabaceae weeds, such as hemp sesbania (*Sesbania exaltata*) and sicklepod (*Cassia obtusifolia*); and Oxalidaceae weeds, such as creeping woodsorrel (*Oxalis corniculata*).

The compound of the present invention can be used for any of soil treatment, soil incorporation treatment, and foliage treatment in non-agricultural lands such as turfs, play grounds, open grounds, road sides, and line ends, other than the agricultural and horticultural fields, such as paddy fields, farmlands, and orchards. Examples of the weeds in these non-agricultural lands include, besides the aforementioned weeds in farmlands and orchards, annual bluegrass (*Poa annua),* dandelion (*Taraxacum officinale),* hairy fleabane (*Conyza bonariensis),* horseweed (*Conyza canadensis),* guernsey fleabane (*Conyza sumatrensis),* wavy bittercress *(Cardamine flexuosa*), white clover *(Trifolium repens),* lawn pennywort *(Hydrocotyle sibthorpioides*), Chinese plantain *(Plantago asiatica),* green kyllinga (*Kyllinga brevifolia*)*,* and field horsetail *(Equisetum arvense).*

The composition of the present invention can be applied to a useful plant, a place where the useful plant is to be grown or where it is growing, or a non-agricultural land in a simultaneous or separate manner, to thereby control the growth of an unwanted plants relative to the useful plant. The aforementioned useful plant encompasses, for example, a field crop or a paddy crop, a horticultural crop, turf, and a fruit tree.

Specific examples of the term "useful plant" as used herein include, but are not limited to, crops, such as corn, rice, wheat, barley, rye, oat, sorghum, cotton, soybean, peanut, buckwheat, beet, rapeseed, sunflower, sugar cane, and tobacco; vegetables, such as solanaceous vegetables (e.g., eggplant, tomato, pepper, capsicum, and potato), cucurbitaceous vegetables (e.g., cucumber, pumpkin, zucchini, watermelon, and melon), brassicaceous vegetables (e.g., radish, turnip, horseradish, kohlrabi, Chinese cabbage, cabbage, mustard, broccoli, and cauliflower), Asteraceous vegetables (e.g., burdock, garland chrysanthemum, artichoke, and lettuce), liliaceae vegetables (e.g., leek, onion, garlic, and asparagus), ammiaceous vegetables (e.g., carrot, parsley, celery, and parsnip), chenopodiaceous vegetables (e.g., spinach and Swiss chard), labiatae vegetables (e.g., beefsteak plant, mint, and basil), strawberry, sweet potato, Japanese yam, and aroid; fruits, such as pomaceous fruits (e.g., apple, pear, Japanese pear, quince, and marmelo), stone fleshy fruits (e.g., peach, plum, nectarine, Japanese apricot, cherry, apricot, and prune), citrus fruits (e.g., Citrus unshiu, orange, lemon, lime, and grapefruit), nuts (e.g., chestnut, walnut, hazelnut, almond, pistachio, cashew nut, and macadamia nut), berry fruits (e.g., blueberry, cranberry, blackberry, and raspberry), grape, persimmon, olive, loquat, banana, coffee, date palm, coconut, and oil palm; trees other than fruit trees, such as tea, mulberry, street trees (e.g., ash, birch, dogwood, eucalyptus, ginkgo, lilac, maple, oak, poplar, cercis, liquidambar, plane, zelkova, Japanese arborvitae, Japanese fir, hemlock, juniper, pine, spruce, yew, elm, and buckeye), sweet viburnum, shrubby Japanese yew, cedar, cypress, croton, Japanese spindle, and Japanese photinia; turfs, such as Korean lawn grass (e.g., Zoysia japonica, and Zoysia tenuifolia), Bermuda grass (e.g., Cynodon dactylon), bent grass (e.g., creeping bent, Agrostis stolonifera, and Agrostis capillaris), bluegrass (e.g., Kentucky bluegrass and rough bluegrass), fescue (e.g., Festuca arundinacea, Festuca rubra var. commutate, and creeping red fescue), ryegrass (e.g., darnel, rye grass, and Italian grass), cocksfoot, and timothy grass; oil crops, such as oil palm and Jatropha curcas; flowers and ornamental plants (e.g., rose, carnation, chrysanthemum, prairie gentian, gypsophila, gerbera, marigold, salvia, petunia, verbena, tulip, aster, gentian, lily, pansy, cyclamen, orchid, lily of the valley, lavender, stock, ornamental cabbage, primrose, poinsettia, gladiolus, cattleya, daisy, verbena, cymbidium, and begonia); and foliage plants.

Examples of the term "useful plant" as used herein also include plants that are provided with resistance to HPPD inhibitors (e.g., isoxaflutole), ALS inhibitors (e.g., imazethapyr and thifensulfuron-methyl), EPSP synthase inhibitors (e.g., glyphosate), glutamine synthase inhibitors (e.g., glufosinate), acetyl-CoA carboxylase inhibitors (e.g., sethoxydim), PPO inhibitors (e.g., flumioxazin), and herbicides (e.g., bromoxynil, dicamba, and 2,4-D) by a classical breeding method and a genetic transformation technique.

Examples of the "agricultural and horticultural plant" provided with resistance by a classical breeding method include rapeseed, wheat, sunflower, rice, and corn exhibiting resistance to an imidazolinone-based ALS inhibitory herbicide (e.g., imazethapyr), which are already commercially available under a trade name of Clearfield <registered trademark>.

Similarly, there is soybean provided with resistance to a sulfonylurea-based ALS inhibitory herbicide (e.g., thifensulfuron-methyl) by a classical breeding method, which is already commercially available under a trade name of STS soybean. Similarly, examples of the agricultural and horticultural plant provided with resistance to an acetyl-CoA carboxylase inhibitor (e.g., trione oxime or aryloxy phenoxypropionic acid herbicide) by a classical breeding method include SR corn. The agricultural and horticultural plant provided with resistance to an acetyl-CoA carboxylase inhibitor is described in, for example, Proceedings of the National Academy of Sciences of the United States of America (Proc. Natl. Acad. Sci. USA), Vol. 87, pp. 7175-7179 (1990). A variant acetyl-CoA carboxylase resistant to an acetyl-CoA carboxylase inhibitor is reported in, for example, Weed Science, Vol. 53, pp. 728-746 (2005) and a plant resistant to an acetyl-CoA carboxylase inhibitor can be generated by introducing the gene of such a variant acetyl-CoA carboxylase into a plant by genetically engineering technology, or by introducing a resistant-imparting variation into a crop acetyl-CoA carboxylase. Furthermore, a plant resistant to a acetyl-CoA carboxylase inhibitor/herbicide can be generated by inducing a site-specific amino acid substitution variation in a crop (acetyl-CoA carboxylase/herbicide target) gene through introduction, into a plant cell, of a nucleic acid into which a base substitution variation, such as a chimeraplasty technique (Gura T. 1999. Repairing the Genome's Spelling Mistakes. Science 285: 316-318) has been introduced.

Examples of the agricultural and horticultural plant provided with resistance by genetic engineering technology include corn, soybean, cotton, rapeseed, and sugar beet resistant to glyphosate, which are already commercially available under trade names of, for example, RoundupReady <registered trademark> and Agrisure GT <registered trademark>. Similarly, there are corn, soybean, cotton, and rapeseed provided with resistance to glufosinate by genetic engineering technology, which are already commercially available under a trade name of, for example, LibertyLink <registered trademark>. Also, cotton provided with resistance to bromoxynil by genetic engineering technology is already commercially available under a trade name of BXN.

The aforementioned "agricultural and horticultural plant" includes plants that are produced by genetic engineering technology, and can synthesize, for example, selective toxins known in the genus Bacillus.

Examples of insecticidal toxins expressed in such genetically engineered plants include insecticidal proteins derived from *Bacillus cereus* or *Bacillus popilliae;* δ-endotoxins derived from *Bacillus thuringiensis,* such as Cry1Ab, Cry1Ac, Cry IF, Cry1Fa2, Cry2Ab, Cry3A, Cry3Bb1, or Cry9C; insecticidal proteins, such as VIP1, VIP2, VIP3, or VIP3A; insecticidal proteins derived from nematodes; toxins generated by animals, such as scorpion toxin, spider toxin, bee toxin, or insect-specific neurotoxins; mold fungi toxins; plant lectin; agglutinin; protease inhibitors, such as a trypsin inhibitor, a serine protease inhibitor, patatin, cystatin, and a papain inhibitor; ribosome-inactivating proteins (RIP), such as lysine, corn-RIP, abrin, saporin, and briodin; steroid-metabolizing enzymes, such as 3-hydroxysteroid oxidase, ecdysteroid-UDP-glucosyl transferase, and cholesterol oxidase; an ecdysone inhibitor; HMG-COA reductase; ion channel inhibitors, such as sodium channel inhibitor and calcium channel inhibitor; juvenile hormone esterase; diuretic hormone receptor; stilbene synthase; bibenzyl synthase; chitinase; and glucanase.

Examples of toxins expressed in such genetically engineered plants include hybrid toxins of δ-endotoxin proteins, such as Cry1Ab, Cry1Ac, Cry1F, Cry1Fa2, Cry2Ab, Cry3A, Cry3Bb1, or Cry9C and insecticidal proteins, such as VIP1, VIP2, VIP3, or VIP3A; partially deleted toxins; and modified toxins. Such hybrid toxins are produced from a new combination of the different domains of such proteins by using a genetic engineering technology. A known example of a partially deleted toxin is Cry1Ab wherein a portion of the amino acid sequence is deleted. A modified toxin is produced by substitution of one or more amino acids of a natural toxin.

Examples of such toxins and genetically engineered plants capable of synthesizing the toxins are described in patent documents, such as EP-A-0374753, WO 93/07278, WO 95/34656, EP-A-0427529, EP-A-451878, and WO 03/052073. Toxins contained in such genetically engineered plants provide the plants with resistance particularly to insect pests belonging to Coleoptera, Diptera, and Lepidoptera.

Genetically engineered plants containing one or more insecticidal pestresistant genes and expressing one or more toxins have already been known, and some of the genetically engineered plants are commercially available. Examples of the genetically engineered plants include YieldGard <registered trademark> (a corn variety expressing Cry1Ab toxin), YieldGard Rootworm <registered trademark> (a corn variety for expressing Cry3Bbl toxin), YieldGard Plus <registered trademark> (a corn variety expressing Cry1Ab and Cry3Bb1 toxins), Herculex I <registered trademark> (a corn variety expressing phosphinotricine N-acetyl transferase (PAT) for imparting resistance to Cry1Fa2 toxin and glufosinate), NuCOTN33B <registered trademark> (a cotton variety expressing Cry1Ac toxin), Bollgard I <registered trademark> (a cotton variety expressing Cry1Actoxin), Bollgard II <registered trademark> (a cotton variety expressing Cry1Acand Cry2Ab toxins), VIPCOT <registered trademark> (a cotton variety expressing VIP toxin), NewLeaf <registered trademark> (a potato variety expressing Cry3A toxin), NatureGard <registered trademark> Agrisure <registered trademark> GT Advantage (GA21 glyphosate-resistant trait), Agrisure <registered trademark> CB Advantage (Bt11 corn borer (CB) trait), and Protecta <registered trademark>.

The aforementioned useful plant also encompasses plants produced by a genetic engineering technology and exhibiting the ability to generate anti-pathogenic substances having selective action.

Examples of such anti-pathogenic substances include PR proteins (PRPs, described in EP-A-0392225); ion channel inhibitors, such as a sodium channel inhibitor and a calcium channel inhibitor (e.g., KP1, KP4, and KP6 toxins produced by viruses are known); stilbene synthase; bibenzyl synthase; chitinase; glucanase; and anti-pathogenic substances generated by microorganisms, such as a peptide antibiotic, an antibiotic having a hetero ring, and a protein factor associated with resistance to plant diseases (which is called a plant disease-resistant gene and is described in WO 03/000906). These anti-pathogenic substances and genetically engineered plants producing the substances are described in, for example, EP-A-0392225, WO 95/33818, and EP-A-0353191.

The aforementioned useful plant also encompasses plants provided with useful properties (e.g., improved oil ingredient and increased amino acid content) by genetically engineering technology. Examples of such plants include VISTIVE <registered trademark> (low linolenic soybean having reduced linolenic content) or highlysine (high-oil) corn (corn having increased lysine or oil content).

The aforementioned useful plant also encompasses stack varieties including combinations of a plurality of useful properties, such as the aforementioned classic herbicidal properties or herbicide-resistant genes, harmful insect-resistant genes, anti-pathogenic substance-producing genes, and useful properties (e.g., improved oil ingredient, and increased amino acid content).

During formulation or spraying, the compound of the present invention may optionally be applied in the form of a mixture with, for example, another herbicide, any insecticide, a bactericide, a plant growth regulator, or a synergist.

In particular, the application of the compound with another herbicide is expected to cause cost reduction resulting from reduced amount of the applied herbicide, an increase in herbicidal spectrum attributed to synergistic effects of the mixed herbicide, and a higher herbicidal effect. In this case, the compound may be used in combination of a plurality of known herbicides.

Examples of preferred herbicides used in combination with the compound of the present invention include 4-CPA, 4-CPA-salts, 4-CPB, 4-CPP, 2,4-D (2,4-PA), 2,4-D-salts, 2,4-D-esters, 3,4-DA, 2,4-DB, 2,4-DB-salts, 2,4-DB-esters, 3,4-DB, 2,4-DEB, 2,4-DEP, 3,4-DP, 2,4,5-T, 2,4,5-T-salts, 2,4,5-T-esters, 2,4,5-TB, 2,3,6-TBA(TCBA), 2,3,6-TBA-salts, acetochlor, acifluorfen, acifluorfen-methyl, acifluorfen-sodium, aclonifen, acrolein, alachlor, allidochlor (CDAA), alloxydim, alloxydim-sodium, allyl alcohol, alorac, ametridione, ametryn, amibuzin, amicarbazone, amidochlor, amidosulfuron, aminocyclopyrachlor, aminocyclopyrachlor-methyl, aminocyclopyrachlor-potassium, aminopyralid, aminopyralid-salts, amiprophos, amiprophos-methyl, amitrole (aminotriazole, ATA), ammonium sulfamate (AMS), anilofos, anisuron, asulam, asulamsalts, atraton, atrazine, azafenidin, azimsulfuron, aziprotryne (azyprotryn), barban (CBN), BCPC, beflubutamid, beflubutamid-M, benazolin, benazolin-ethyl, benazolin-salts, bencarbazone, benfluralin (benefin), benfuresate, bensulfuron, bensulfuron-methyl, bensulide (SAP), bentazon (bentazone), bentazone-sodium, bentranil, benzadox, benzadox-ammonium, benzfendizone, benzipram, benzobicyclon, benzofenap, benzofluor, benzoylprop, benzoylprop-ethyl, benzthiazuron, bicyclopyrone, bifenox, bilanafos (bialaphos), bilanafos-sodium, binapacryl, bispyribac, bispyribac-sodium, bixlozone, borax, bromacil, bromacil-salts, bromobonil, bromobutide, bromofenoxim, bromopyrazon, bromoxynil, bromoxynil-potassium, bromoxynil-esters, butachlor, butafenacil, butamifos, butenachlor, butralin (butraline), buthidazole, buthiuron, butroxydim, buturon, butylate, cafenstrole, calcium cyanamide, cambendichlor, calcium chlorate, carbasulam, carbetamide, carboxazole, carfentrazone, carfentrazone-ethyl, CDEA, CEPC, chlomethoxyfen (chlomethoxynil), chloramben, chloramben-salts, chloramben-methyl, chloramben-methylammonium, chloranocryl (dicryl), chlorazifop, chlorazifop-propargyl, chlorazine, chlorbromuron, chlorbufam (BIPC), chloreturon, chlorfenac (fenac), chlorfenac-salts, chlorfenprop, chlorfenprop-methyl, chlorflurazole, chlorflurenol, chlorflurenol-methyl, chloridazon (PAC, pyrazon), chlorimuron, chlorimuron-ethyl, chlornidine, chlornitrofen (CNP), chloroacetic acid (monochloroacetic acid), sodium chloroacetate (SMA), chlorotoluron, chloroxuron, chloroxynil, chlorprocarb, chlorphtalim, chlorpropham (IPC), chlorsulfuron, chlorthal (TCTP), chlorthal-esters, chlorthiamid (DCBN), cinidon-ethyl, cinmethylin, cinosulfuron, cisanilide, clacyfos, clethodim, cliodinate, clodinafop, clodinafop-propargyl, clofop, clofop-isobutyl, clomazone, clomeprop, cloprop, cloproxydim, clopyralid, clopyralid-methyl, clopyralid-salts, cloransulam, cloransulam-methyl, copper sulfate, CPMF, CPPC, credazine, cresol, cumyluron, cyanatryn, cyanamide, cyanazine, cycloate, cyclopyranil, cyclopyrimorate, cyclosulfamuron, cycloxydim, cycluron (COMU) , cyhalofop, cyhalofop-butyl, cyperquat, cyperquat-chloride, cyprazine, cyprazole, cypromid, daimuron (dymron), dalapon, dalapon-salts, dazomet, dazomet-sodium, delachlor, desmedipham, desmetryn, di-allate, dicamba (MDBA), dicamba-salts, dicamba-esters, dichlobenil (DBN), dichloraurea (DCU), dichlormate, o-dichlorobenzene, (DCB), dichlorprop, dichlorprop-salts, dichlorprop-esters, dichlorprop-P, dichlorprop-P-salts, dichlorprop-P-esters, diclofop, diclofop-methyl, diclofop-P, diclofop-P-methyl, diclosulam, diethamquat, diethamquat dichloride, diethatyl, diethatyl-ethyl, difenopenten, difenopenten-ethyl, difenoxuron, difenzoquat, difenzoquat methylsulfate, diflufenican, diflufenzopyr, diflufenzopyr-sodium, dimefuron, dimepiperate, dimesulfazet, dimethachlor, dimethametryn, dimethenamid, dimethenamid-p, dimexano, dimidazon, dimethyl disulfide, dinitramine, dinofenate, dinoprop, dinosam, dinoseb (DNBP), dinoseb-salts, dinoseb-esters, dinoterb, dinoterb-salts, dinoterb-esters, diphenamid, dipropalin, dipropetryn, diquqt, diquqt dibromide, disul (2,4-PS), disulsodium, dithiopyr, diuron (DCMU), DMPA, DNOC, DNOC-salts, EBEP, eglinazine, eglinazine-ethyl, endothal, endothal-salts, epronaz, EPTC, epyrifenacil, erbon, esprocarb, ethachlor, ethalfluralin, ethametsulfuron, ethametsulfuron-methyl, ethaprochlor, ethidimuron, ethiolate, ethiozin, ethofumesate, ethoxyfen, ethoxyfen-ethyl, ethoxysulfuron, etinofen, etnipromid, etobenzanid, EXD, fenasulam, fenoprop (2,4,5-TP, silvex), fenopropsalts, fenoprop-esters, fenoxaprop, fenoxaprop-ethyl, fenoxaprop-p, fenoxaprop-p-ethyl, fenoxasulfone, fenquinotrione, fenteracol, fenthiaprop, fenthiaprop-ethyl, fentrazamide, ferrous sulfate, fenuron, fenuron-TCA, flamprop, flamprop-esters, flamprop-M, flamprop-M-esters, flazasulfuron, florasulam, florpyrauxifen, florpyrauxifen-benzyl, fluazifop, fluazifop-esters, fluazifop-P, fluazifop-p-esters, fluazolate, flucarbazone, flucarbazone-sodium, flucetosulfuron, fluchloralin, flufenacet, flufenican, flufenpyr, flufenpyr-ethyl, flumetsulam, flumezin, flumiclorac, flumiclorac-pentyl, flumioxazin, flumipropyn, fluometuron, fluorodifen, fluoroglycofen, fluoroglycofen-ethyl, fluoromidine, fluoronitrofen (CFNP), fluothiuron, flupoxam, flupropacil, flupropanate (tetrapion), flupropanate-sodium, flupyrsulfuron, flupyrsulfuron-methyl, flupyrsulfuron-sodium, flupyrsulfuron-methyl-sodium, fluridone, flurochloridone, fluroxypyr, fluroxypyr-esters, flurtamone, fluthiacet, fluthiacet-methyl, fomesafen, fomesafen-sodium, foramsulfuron, fosamine, fosamine-ammonium, furyloxyfen, glufosinate, glufosinate-salts, glufosinate-P, glufosinate-P-salts, glyphosate, glyphosate-salts, halauxifen, halauxifen-methyl, halosafen, halosulfuron, halosulfuron-methyl, haloxydine, haloxyfop, haloxyfop-sodium, haloxyfop-esters, haloxyfop-P, haloxyfop-P-esters, herbimycin, hexachloroacetone (HCA), hexazinone, imazamethabenz, imazamethabenz-methyl, imazamox, imazamox-ammonium, imazapic, imazapic-ammonium, imazapyr, imazapyr-isopropylammonium, imazaquin, imazaquin-methyl, imazaquin-salts, imazethapyr, imazethapyr-ammonium, imazosulfuron, indanofan, indaziflam, iodobonil, iodosulfuron, iodosulfuron-sodium, iodosulfuron-methyl, iodosulfuron-methyl-sodium, iofensulfuron, iofensulfuron-sodium, ioxynil, ioxynil-salts, ioxynil-esters, ipazine, ipfencarbazone, iprymidam, isocarbamid, isocil (isoprocil), isomethiozin, isonoruron, isopolinate, isopropalin, isoproturon, isouron, isoxaben, isoxachlortole, isoxaflutole, isoxapyrifop, karbutilate, ketospiradox, ketospiradox-potassium, lactofen, lancotrione, lancotrione-sodium, lenacil, linuron, MCPA, MCPA-salts, MCPA-esters, MCPB, MCPB-salts, MCPB-esters, mecoprop (MCPP), mecoprop-salts, mecoprop-esters, mecoprop-P, mecoprop-P-salts, mecoprop-P-esters, medinoterb, medinoterb acetate, mefenacet, mefluidide, mefluidide-salts, mesoprazine, mesosulfuron, mesosulfuron-methyl, mesotrione, metam (carbam), metam-salts, metamifop, metamitron, metazachlor, metazosulfuron, metflurazon, methabenzthiazuron (methibenzuron), methalpropalin, methazole, methiobencarb, methiuron, methometon, methoprotryne (methoprotryn), methoxyphenone, methiopyrisulfuron, methiozolin, methyl azide, methyl bromide, methyl dymron, methyl iodide, methyl isothiocyanate, metobenzuron, metobromuron, metolachlor, S-metolachlor, metosulam, metoxuron, metribuzin, metsulfuron, metsulfuron-methyl, molinate, monalide, monisouron, monolinuron, monosulfuron, monosulfuron-methyl, monuron (CMU), monuron-TCA, morfamquat, morfamquat dichloride, naproanilide, napropamide, napropamide-M, naptalam (NPA), naptalam-sodium, neburon, nicosulfuron, nipyraclofen, nitralin, nitrofen (NIP, niclofen), nitrofluorfen, norflurazon, noruron (norea), OCH, oleic acid, orbencarb, orthosulfamuron, oryzalin, oxadiargyl, oxadiazon, oxapyrazon, oxapyrazon-salts, oxasulfuron, oxaziclomefone, oxyfluorfen, parafluron, paraquat, paraquat dichloride, paraquat dimethylsulfate, pebulate, pelargonic acid (nonanoic acid), pendimethalin, penoxsulam, pentachlorophenol, sodium pentachlorophenoxide, pentachlorophenyl laurate, pentanochlor (solan, CMMP), pentoxazone, perfluidone, pethoxamid, phenisopham, phenmedipham, phenmedipham-ethyl, phenobenzuron, picloram, picloram-salts, picloram-esters, picolinafen, pinoxaden, piperophos, potassium azide, potassium cyanate, pretilachlor, primisulfuron, primisulfuron-methyl, procyazine, prodiamine, profluazol, profluralin, profoxydim, proglinazine, proglinazine-ethyl, prometon, prometryn (prometryne), propachlor, propanil (DCPA), propaquizafop, propazine, propham, propisochlor, propoxycarbazone, propoxycarbazone-sodium, propyrisulfuron, propyzamide (pronamide), prosulfalin, prosulfocarb, prosulfuron, proxan (IPX), proxan-sodium, prynachlor, pydanon, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz-isopropyl, pyribambenz-propyl, pyribenzoxim, pyributicarb, pyriclor, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinclorac-dimethylammonium, quinclorac-methyl, quinmerac, quinoclamine (ACN), quinonamid, quizalofop, quizalofop-esters, quizalofop-P, quizalofop-P-esters, rhodethanil, rimsulfuron, saflufenacil, sebuthylazine, secbumeton, sethoxydim, siduron, simazine (CAT), simeton, simetryn (simetryne), sodium azide, sodium chlorate, sulcotrione, sulfallate (CDEC), sulfentrazone, sulfometuron, sulfometuron-methyl, sulfosulfuron, sulfuric acid, sulglycapin, swep (MCC), tavron, TCA (trichloroacetic acid), TCA-salts, TCA-ethadyl, tebutam (butam), tebuthiuron, tefuryltrione, tembotrione, tepraloxydim, terbacil, terbucarb (terbutol, MPMC), terbuchlor, terbumeton, terbuthylazine, terbutryn, tetflupyrolimet, tetrafluron, thenylchlor, thiazafluron, thiazopyr, thidiazimin, thidiazuron, thiencarbazone, thiencarbazone-methyl, thifensulfuron, thifensulfuron-methyl, thiobencarb (benthiocarb), tiafenacil, tiocarbazil, tioclorim, tolpyralate, topramezone, tralkoxydim, triafamone, triallate (tri-allate), triasulfuron, triaziflam, tribenuron, tribenuron-methyl, tricamba, triclopyr, triclopyr-salts, triclopyr-esters, tridiphane, trietazine, trifloxysulfuron, trifloxysulfuron-sodium, trifludimoxadin, trifluralin, triflusulfuron, triflusulfuron-methyl, trifop, trifop-methyl, trifopsime, trihydroxytriazine (cyanuric acid), trimeturon, tripropindan, tritac, tritosulfuron, vernolate, xylachlor, 6-{(difluoromethyl)thio}-N2,N4-diisopropyl-1,3,5-triazine-2,4-diamine (CAS 103427-73-2), methyl (R)-2-[{7-(2-chloro-4-(trifluoromethyl)phenoxy)naphthalen-2-yl}oxy]propanoate (CAS 103055-25-0), propan-2-one O-(12H-dibenzo[d,g][1,3]dioxocin-6-carbonyl)oxime (CAS 503819-68-9), [{(2-(N-methylmethylsulfonamido)-2-oxoethyl)amino}methyl]phosphonic acid (CAS 98565-18-5), ethyl 2-[{2-(4-((6-chloroquinoxalin-2-yl)oxy)phenoxy)propanoyl}oxy]-3-methyl-3-butenoate (CAS 1191932-79-2), O-(2,4-dimethyl-6-nitrophenyl) O-methylisopropylphosphoramide thioate (CAS 189517-75-7), methyl 5-[N-{(4,6-dimethylpyrimidin-2-yl)carbamoyl}sulfamoyl]-1-(pyridin-2-yl)-lH-pyrazole-4-carboxylate (CAS 104770-29-8), 4-[2-chloro-3-{(3,5-dimethyl-1H-pyrazol-1-yl)methyl}-4-(methylsulfonyl)benzoyl]-1,3-dimethyl-1H-pyrazol-5-yl 1,3-dimethyl-1H-pyrazole-4-carboxylate (CAS 1911613-97-2), 4-[2-chloro-4-(methylsulfonyl)-3-{ (2,2,2-trifluoroethoxy)methyl}benzoyl]-1-ethyl-1H-pyrazol-5-yl 1,3-dimethyl-1H-pyrazole-4-carboxylate (CAS 1992017-55-6), 1-{2-chloro-3-(3-cyclopropyl-5-hydroxy-1-methyl-1H-pyrazole-4-carbonyl)-6-(trifluoromethyl)phenyl}piperidin-2-one (CAS 1855929-45-1), 1,3-dimethyl-4-{2-(methylsulfonyl)-4-(trifluoromethyl)benzoyl}-1H-pyrazol-5-yl 1,3-dimethyl-1H-pyrazole-4-carboxylate (CAS 1622908-18-2), ethyl 2-[{3-(2-chloro-4-fluoro-5-(3-methyl-2,6-dioxo-4-(trifluoromethyl)-3,6-dihydropyrimidin-1(2H)-yl)phenoxy)pyridin-2-yl}oxy]acetate (CAS 353292-31-6), 2-methyl-N-(5-methyl-1,3,4-oxadiazol-2-yl)-3-(methylsulfonyl)-4-(trifluoromethyl)benzamide (CAS 1400904-50-8), 2-chloro-N-(1-methyl-1H-tetrazol-5-yl)-3-(methylthio)-4-(trifluoromethyl)benzamide (CAS 1361139-71-0), 2-chloro-N-(1-methyl-1H-tetrazol-5-yl)-3-(methylsulfonyl)-4-(trifluoromethyl)benzamide (CAS 1361139-73-2), F4050 (test name), F9600 (test name), F9960 (test name), OK-701 (test name), and SL-1201 (test name). These ingredients may be used alone or in combination of two or more species. When two or more species are used in combination, the proportions thereof may be arbitrarily determined.

Examples of the safener include benoxacor, BPCMS (CSB), cloquintocet, cloquintocet-mexyl, cumyluron, cyometrinil, cyprosulfamide, daimuron (dymron), dichlormid, dicyclonon (diclonon), dietholate, dimepiperate, disulphoton, fenchlorazole, fenchlorazole-ethyl, fenclorim, flurazole, fluxofenim, furilazole, hexim, isoxadifen, isoxadifen-ethyl, MCPA, mecoprop, mefenpyr, mefenpyr-diethyl, mephenate, metcamifen, methoxyphenone, 1,8-naphthalic anhydride (NA), octamethylene-diamine, oxabetrinil, 4-(dichloroacetyl)-1-oxa-4-azaspiro[4.5]decane (AD67, MON4660), 4-carboxy-3,4-dihydro-2H-1-benzopyran-4-acetic acid (CL304415), 2,2-dichloro-N-[2-oxo-2-(propenylamino)ethyl]-N-2-propenylacetamide (DKA-24), 2-(dichloromethyl)-2-methyl-1,3-dioxolane (MG191), 2-propenyl 1-oxa-4-azaspiro[4,5]decane-4-carbodithioate (MG838), (3-dichloroacetyl-5-(2-furyl)-2,2-dimethyloxazolidine) (MON13900), (N-allyl-N-[(1,3-dioxolan-2-yl)methyl]dichloroacetamide (PPG-1292), 3-(dichloroacetyl)-2,2-dimethyl-1,3-oxazolidine (R28725), 3-(dichloroacetyl)-2,2,5-trimethyl-1,3-oxazolidine (R29148), and 1-dichloroacetylazepane (TI-35). These ingredients may be used alone or in combination of two or more species. When two or more species are used in combination, the proportions thereof may be arbitrarily determined.

When the compound of the present invention is applied as a herbicide, the compound is usually mixed with an appropriate solid carrier or liquid carrier. If desired, the mixture may be further mixed with a surfactant, a penetrant, a spreading agent, a thickener, an antifreezing agent, a binder, an anticaking agent, a disintegrant, and a stabilizing agent, and the resultant mixture may be practically used in any herbicidal formulation, such as a water-dispersible powder, an emulsion, a flowable agent, a dry flowable agent, a liquid, a powder, a granule, or a gel. From the viewpoint of power saving and an improvement in safety, the aforementioned herbicidal formulation may be encapsulated in a water-soluble package for supply.

Examples of the solid carrier include natural minerals, such as quartz, kaolinite, pyrophyllite, sericite, talc, bentonite, acid clay, attapulgite, zeolite, and diatomaceous earth; inorganic salts, such as calcium carbonate, ammonium sulfate, sodium sulfate, and potassium chloride; synthetic silicic acid; and synthetic silicates.

Examples of the liquid carrier include alcohols, such as ethylene glycol, propylene glycol, and isopropanol; aromatic hydrocarbons, such as xylene, alkylbenzene, and alkylnaphthalene; ethers, such as butyl cellosolve; ketones, such as cyclohexanone; esters, such as γ-butyrolactone; acid amides, such as N-methyl-2-pyrrolidone and N-octyl-2-pyrrolidone; vegetable oils, such as soybean oil, rapeseed oil, cotton seed oil, and castor oil; and water.

These solid and liquid carriers may be used alone or in combination of two or more species.

Examples of the surfactant include nonionic surfactants, such as polyoxyethylene alkyl ethers, polyoxyethylene alkylaryl ethers, polyoxyethylene styrylphenyl ethers, polyoxyethylene-polyoxypropylene block copolymers, polyoxyethylene fatty acid esters, sorbitan fatty acid esters, and polyoxyethylene sorbitan fatty acid esters; anionic surfactants, such as alkyl sulfates, alkylbenzene sulfonates, lignin sulfonates, alkyl sulfosuccinates, naphthalene sulfonate, alkylnaphthalene sulfonates, salts of naphthalene sulfonic acid-formalin condensate, salts of alkylnaphthalene sulfonic acid-formalin condensate, polyoxyethylene alkylaryl ether sulfates and phosphates, polyoxyethylene styrylphenyl ether sulfates and phosphates, polycarboxylates, and polystyrene sulfonates; cationic surfactants, such as alkylamine salts and alkyl quaternary ammonium salts; and amphoteric surfactants, such as amino acid-type surfactants and betaine-type surfactants.

No particular limitation is imposed on the amount of such a surfactant contained in the formulation of the present invention, but the amount of the surfactant is preferably 0.05 to 20 parts by mass relative to 100 parts by mass of the formulation. The aforementioned surfactants may be used alone or in combination of two or more species.

During formulation or spraying, the compound of the present invention may optionally be applied in the form of a mixture with, for example, another herbicide, any insecticide, a bactericide, a plant growth regulator, or a synergist.

In particular, the application of the compound with another herbicide is expected to cause cost reduction resulting from reduced amount of the applied herbicide, an increase in herbicidal spectrum attributed to synergistic effects of the mixed herbicide, and a higher herbicidal effect. In this case, the compound may be used in combination of a plurality of known herbicides.

The amount of application of the compound of the present invention varies depending on, for example, the situation of application, the timing of application, the method of application, or the type of cultivated crop. In general, the appropriate amount of the compound is about 0.005 to 50 kg per hectare (ha) in terms of the amount of an active ingredient.

### Formulation Examples

Next will be described specific agricultural chemical formation examples containing the compound of the present invention as an active ingredient. However, the present invention is not limited to these examples. In the following formulation examples, the term "part(s)" refers to part(s) by mass.

### [Formulation Example 1] Water-Dispersible Powder

| | |
|---|---|
| Compound of the present invention No. 1-024 | 20 parts |
| Pyrophyllite | 76 parts |
| Sorpol 5039 | 2 parts |
| (trade name, anionic surfactant: available from TOHO Chemical Industry Co., Ltd.) | |
| Carplex #80 | 2 parts |
| (trade name, synthetic hydrous silicic acid: available from SHIONOGI & CO., LTD.) | |

These ingredients are homogeneously mixed and pulverized to prepare a water-dispersible powder.

### [Formulation Example 2] Emulsion

| | |
|---|---|
| Compound of the present invention No. 3-009 | 5 parts |
| Xylene | 75 parts |
| N-methyl-2-pyrrolidone | 15 parts |
| Sorpol 2680 | 5 parts |
| (trade name, anionic surfactant: available from TOHO Chemical Industry Co., Ltd.) | |

These ingredients are homogeneously mixed to prepare an emulsion.

### [Formulation Example 3] Flowable Agent

| | |
|---|---|
| Compound of the present invention No. 3-065 | 25 parts |
| Agrisol S-710 | 10 parts |
| (trade name, nonionic surfactant: available from Kao Corporation) | |
| Runox 1000C | 0.5 parts |
| (trade name, anionic surfactant: available from TOHO Chemical Industry Co., Ltd.) | |
| Xanthan gum | 0.02 parts |
| Water | 64.48 parts |

These ingredients are homogeneously mixed and then wet-pulverized to prepare a flowable agent.

### [Formulation Example 4] Dry Flowable Agent

| | |
|---|---|
| Compound of the present invention No. 1-045 | 75 parts |
| Hitenol NE-15 | 5 parts |
| (trade name, anionic surfactant: available from DKS Co. Ltd.) Vanillex N | 10 parts |
| (trade name, anionic surfactant: available from Nippon Paper Industries Co., Ltd.) | |
| Carplex #80 | 10 parts |
| (trade name, synthetic hydrous silicic acid: available from | |

SHIONOGI & CO., LTD.)

These ingredients are homogeneously mixed and pulverized, and a small amount of water is added to the pulverized mixture, followed by mixing and kneading with stirring. The resultant product is granulated with an extrusion granulator and then dried to prepare a dry flowable agent.

### [Formulation Example 5] Granule

| | |
|---|---|
| Compound of the present invention No. 3-089 | 1 part |
| Bentonite | 55 parts |
| Talc | 44 parts |

These ingredients are homogeneously mixed and pulverized, and a small amount of water is added to the pulverized mixture, followed by mixing and kneading with stirring. The resultant product is granulated with an extrusion granulator and then dried to prepare a granule.

### Examples

The present invention will next be described in more detail with reference to Synthesis Examples and Test Examples of the compound of the present invention. However, the present invention should not be construed as being limited to these Examples.

The medium-pressure preparative liquid chromatography described in Synthesis Examples was performed with a medium-pressure preparative apparatus YFLC-Wprep (flow rate: 18 mL/min, column of silica gel 40 µm) available from Yamazen Corporation.

The chemical shift values of proton nuclear magnetic resonance spectroscopy (hereinafter referred to as "¹H-NMR") described bellow were measured at 300 MHz (model: JNM-ECX300 or JNM-ECP300, available from JEOL Ltd.) in deuterated chloroform solvent by use of Me₄Si (tetramethylsilane) as a reference substance. In the case of measurement in deuterated dimethyl sulfoxide solvent, "(DMSO-d6)" is described in chemical shift value data. The symbols of the chemical shift values of ¹H-NMR have the following meanings:
s: singlet, d: doublet, dd: double doublet, dt: doublet triplet, td: triplet doublet, ddd: double double doublet, t: triplet, q: quartet, sep: septet, m: multiplet, and brs: broad singlet. For the signals that can be analyzed when two or more stereoisomers are present, the chemical shift values of each of the signals are marked with "and."

### Synthesis Examples

### [Synthesis Example 1]

### Synthesis of 5-hydroxy-2,6-dimethyl-4-(2-(4-cyanophenyl)benzo[b]thiophen-3-yl)pyridazin-3(2H)-one (compound No. 1-045)

### Step 1: synthesis of 5-methoxy-2,6-dimethyl-4-(2-(4-cyanophenyl)benzo[b]thiophen-3-yl)pyridazin-3 (2H)-one

Firstly, 322 mg of p-cyanophenylboronic acid, 349 mg of potassium phosphate, 51 mg of 2-dicyclohexylphosphino-2',6'-diisopropoxybiphenyl, and 12 mg of palladium acetate were added to a mixture of 200 mg of 4-(2-bromobenzo[b]thiophen-3-yl)-5-methoxy-2,6-dimethylpyridazin-3(2H)-one and 3 mL of toluene at room temperature. After completion of the addition, the reaction container was purged with nitrogen gas. After completion of the nitrogen gas purging, the reaction mixture was stirred at 110°C for three hours. After completion of the stirring, insoluble matter was filtered with celite, and the residue was washed with chloroform (10 mL × 2). The resultant filtrate was concentrated under reduced pressure, and the residue was purified by silica gel chromatography with a gradient of [ethyl acetate : n-hexane = 5 : 95 to 20 : 80 (by volume, the same shall apply hereinafter)], to thereby yield 90 mg of the target product as a yellow solid.
Melting point: 219-221°C
¹H NMR: δ 7.85-7.95 (m, 1H), 7.55-7.70 (m, 4H), 7.35-7.50 (m, 3H), 3.77 (s, 3H), 3.33 (s, 3H), 2.24 (s, 3H).

### Step 2: synthesis of 5-hydroxy-2,6-dimethyl-4-(2-(4-cyanophenyl)benzo[b]thiophen-3-yl)pyridazin-3(2H)-one (compound No. 1-045)

Firstly, 2 mL of morpholine was added to 90 mg of 5-methoxy-2,6-dimethyl-4-(2-(4-cyanophenyl)benzo[b]thiophen-3-yl)pyridazin-3(2H)-one at room temperature. After completion of the addition, the reaction container was purged with nitrogen gas. After completion of the nitrogen gas purging, the reaction mixture was stirred at 95°C for five hours. After completion of the stirring, the solvent contained in the reaction mixture was distilled off under reduced pressure. The resultant residue was dissolved in 10 mL of chloroform, and 1 mol/L hydrochloric acid was added to the solution under ice cooling so as to achieve a pH of 1, followed by stirring of the resultant mixture at room temperature for one hour. After completion of the stirring, the organic phase was separated from the mixture. The resultant organic phase was washed with 5 mL of 1 mol/L hydrochloric acid, and then dehydrated and dried sequentially with saturated salt water and anhydrous sodium sulfate. Thereafter, the solvent was distilled off under reduced pressure. The resultant residue was purified by silica gel chromatography (with a gradient of ethyl acetate : n-hexane = 20 : 80 to 75 : 25), to thereby yield 83 mg of the target product as a white solid.
Melting point: 240-242°C
¹H NMR: δ 7.75-7.85 (m, 1H), 7.25-7.50 (m, 7H), 3.47 (s, 3H), 2.17 (s, 3H).

### [Synthesis Example 2]

### Synthesis of 5-hydroxy-2,6-dimethyl-4-(2-(4-chloro-1H-pyrazol-1-yl)benzo[b]thiophen-3-yl)pyridazin-3(2H)-one (compound No. 1-097)

### Step 1: synthesis of 1,3-dimethyl-6-oxo-5-(2-(4-chloro-1H-pyrazol-1-yl)benzo[b]thiophen-3-yl)-1,6-dihydropyridazin-4-yl=(n-butyrate) (compound No. 1-098)

Firstly, 68 mg of 4-chloropyrazole, 206 mg of potassium carbonate, 11 mg of copper iodide, and 21 mg of (1S,2S)-(+)-N,N'-dimethylcyclohexane-1,2-diamine were added to a mixture of 200 mg of 4-(2-bromobenzo[b]thiophen-3-yl)-5-methoxy-2,6-dimethylpyridazin-3(2H)-one and 5 mL of N,N-dimethylacetamide at room temperature. After completion of the addition, the reaction container was purged with nitrogen gas. After completion of the nitrogen gas purging, the reaction mixture was stirred at 130°C for eight hours. After completion of the stirring, 20 mL of ethyl acetate and 20 mL of 28% by mass aqueous ammonia were added to the reaction mixture, and the precipitated insoluble matter was filtered with celite. Subsequently, 35% by mass hydrochloric acid was added to the resultant filtrate under ice cooling so as to achieve a pH of 1, and the resultant mixture was subjected to extraction with 20 mL of ethyl acetate. A solution of 58 mg of lithium hydroxide in 20 mL of water was added to the resultant organic phase at room temperature, to thereby separate the aqueous phase from the mixture. Thereafter, 35% by mass hydrochloric acid was added to the resultant aqueous phase under ice cooling so as to achieve a pH of 1, and then the precipitated solid was filtered. The resultant solid was dissolved in 2 mL of dichloromethane, and 61 mg of triethylamine and 70 mg of n-butyryl chloride were added to the solution under ice cooling. After completion of the addition, the resultant reaction mixture was stirred at room temperature for one hour. After completion of the stirring, the solvent contained in the reaction mixture was distilled off under reduced pressure, and the resultant residue was purified by silica gel chromatography (with a gradient of ethyl acetate : n-hexane = 2 : 98 to 30 : 70), to thereby yield 57 mg of the target product as a yellow oily product.

¹H NMR: δ 7.75-7.85 (m, 1H), 7.72 (s, 1H), 7.63 (s, 1H), 7.30-7.40 (m, 3H), 3.83 (s, 3H), 2.30 (s, 3H), 2.10-2.20 (m, 2H), 1.20-1.35 (m, 2H), 0.58 (t, J=7.5Hz, 3H).

### Step 2: synthesis of 5-hydroxy-2,6-dimethyl-4-(2-(4-chloro-1H-pyrazol-1-yl)benzo[b]thiophen-3-yl)pyridazin-3(2H)-one (compound No. 1-097)

Firstly, a solution of 10 mg of lithium hydroxide in 2 mL of water was added to a mixture of 36 mg of 1,3-dimethyl-6-oxo-5-(2-(4-chloro-1H-pyrazol-1-yl)benzo[b]thiophen-3-yl)-1,6-dihydropyridazin-4-yl=(n-butyrate) and 1 mL of tetrahydrofuran at room temperature. After completion of the addition, the reaction mixture was stirred at room temperature for one hour. After completion of the stirring, the solvent contained in the reaction mixture was distilled off under reduced pressure. Subsequently, 35% by mass hydrochloric acid was added to the resultant residue so as to achieve a pH of 1, and the precipitated solid was filtered, to thereby yield 30 mg of the target product as a white solid.

¹H NMR (DMSO-d6): δ 8.30 (s, 1H), 8.09 (s, 1H), 7.95-8.05 (m, 1H), 7.90 (s, 1H), 7.30-7.45 (m, 2H), 7.20-7.30 (m, 1H), 3.58 (s, 3H), 2.26 (s, 3H).

### [Synthesis Example 3]

### Synthesis of 5-(6-methoxy-2-(2-methoxyphenyl)benzo[b]thiophen-3-yl)-1,3-dimethyl-6-oxo-1,6-dihydropyridazin-4-yl=(n-butyrate) (compound No. 3-066)

### Step 1: synthesis of methyl 2-(6-methoxy-2-(2-methoxyphenyl)benzo[b]thiophen-3-yl)acetate

Firstly, 27.0 g of 2-bromoanisole, 649 mg of palladium acetate, 1.5g of triphenylphosphine, and 12.0 g of potassium carbonate were added to a mixture of 12.9 g of 2-(6-methoxybenzo[b]thiophen-3-yl)acetic acid and 115 mL of N,N-dimethylformamide at room temperature. After completion of the addition, air in the reaction container was replaced with nitrogen gas, and the reaction mixture was stirred at 100°C for 11 hours. After completion of the stirring, the reaction mixture was cooled to room temperature, and 24.0 g of potassium carbonate and 49.2 g of methyl iodide were added to the reaction mixture, followed by stirring at room temperature for 13 hours. After completion of the stirring, 200 mL of toluene was added to the reaction mixture, and insoluble matter was filtered with celite. The residue was washed with 20 mL of toluene, and the resultant filtrate was washed with 100 mL of water. Thereafter, the resultant filtrate was dehydrated and dried sequentially with saturated salt water and anhydrous sodium sulfate, and then the solvent was distilled off under reduced pressure. The resultant residue was purified by silica gel column chromatography (with a gradient of ethyl acetate : n-hexane = 1 : 99 to 20 : 80), to thereby yield 7.93 g of the target product as a colorless oily product.

¹H NMR: δ 7.61 (d, J=8.9Hz, 1H), 7.35-7.45 (m, 2H), 7.32 (d, J=2.4Hz, 1H), 6.95-7.10 (m, 3H), 3.89 (s, 3H), 3.79 (s, 3H), 3.69 (s, 2H), 3.67 (s, 3H).

### Step 2: synthesis of 2-(6-methoxy-2-(2-methoxyphenyl)benzo[b]thiophen-3-yl) acetic acid

Firstly, a solution of 3.4 g of sodium hydroxide in 100 mL of water was added to a mixture of 9.0 g of methyl 2-(6-methoxy-2-(2-methoxyphenyl)benzo[b]thiophen-3-yl)acetate and 60 mL of tetrahydrofuran at room temperature. After completion of the addition, the resultant mixture was stirred at room temperature for 12 hours. After completion of the stirring, the solvent contained in the reaction mixture was distilled off under reduced pressure. Subsequently, 20 mL of water and 50 mL of hexane were added to the resultant residue, and the aqueous phase was separated from the reaction mixture. Thereafter, 35% by mass hydrochloric acid was added to the resultant aqueous phase under ice cooling so as to achieve a pH of 1, and the precipitated solid was recovered through filtration. The resultant solid was washed sequentially with 20 mL of water and 20 mL of hexane, to thereby yield 7.0 g of the target product as a white solid.
Melting point: 232-234°C
¹H NMR: δ 7.65-7.80 (m, 2H), 7.43 (d, J=2.4Hz, 1H), 7.30-7.45 (m, 1H), 7.10 (d, J=8.4Hz, 1H), 6.95-7.05 (m, 1H), 6.94 (dd, J=8.4, 2.4Hz, 1H), 3.81 (s, 3H), 3.76 (s, 3H), 3.34 (s, 2H).

### Step 3: synthesis of ethyl 2-(2-(2-(6-methoxy-2-(2-methoxyphenyl)benzo[b]thiophen-3-yl)acetyl-2-methylhydrazinylidene)propionate

Firstly, 4.5 g of 1-(3-dimethylaminopropyl)-3-ethylcarbodiimide hydrochloride and 4.3 g of pentafluorophenol were added to a mixture of 7.0 g of 2-(6-methoxy-2-(2-methoxyphenyl)benzo[b]thiophen-3-yl)acetic acid and 22 mL of dichloromethane at room temperature. After completion of the addition, the resultant mixture was stirred at room temperature for five hours. Subsequently, 8.3 g of diisopropylethylamine was added at room temperature to a mixture of 6.2 g of methylhydrazine sulfate and 30 mL of dichloromethane prepared in another reaction container. After completion of the addition, the resultant mixture was stirred at room temperature for five hours. After completion of the stirring, the previously prepared mixture of 2-(6-methoxy-2-(2-methoxyphenyl)benzo[b]thiophen-3-yl)acetic acid and 1-(3-dimethylaminopropyl)-3-ethylcarbodiimide hydrochloride was added to this reaction mixture at room temperature. After completion of the addition, the resultant mixture was stirred at room temperature for three hours. After completion of the stirring, 40 mL of water was added to the reaction mixture, and the resultant mixture was subjected to extraction with 30 mL of dichloromethane. The resultant organic phase was washed with water (30 mL × 2), and then dehydrated and dried sequentially with saturated salt water and anhydrous sodium sulfate. Thereafter, the solvent was distilled off under reduced pressure. The resultant residue was dissolved in 62 mL of ethanol, and 5.0 g of ethyl pyruvate was added to the solution at room temperature. After completion of the addition, the resultant mixture was stirred at room temperature for 12 hours. After completion of the stirring, the solvent contained in the reaction mixture was distilled off under reduced pressure. The resultant residue was purified by silica gel column chromatography (with a gradient of ethyl acetate : n-hexane = 1 : 99 to 15 : 85), to thereby yield 7.5 g of the target product as a brown oily product.

¹H NMR: δ 7.55-7.70 (m, 1H), 7.25-7.45 (m, 3H), 6.90-7.05 (m, 3H), 4.24 (q, J=6.9Hz, 2H), 4.06 (s, 2H), 3.88 (s, 3H), 3.79 (s, 3H), 3.32 (s, 3H), 2.19 (s, 3H), 1.28 (t, J=6.9Hz, 3H).

### Step 4: synthesis of 5-hydroxy-4-(6-methoxy-2-(2-methoxyphenyl)benzo[b]thiophen-3-yl)-2,6-dimethylpyridazin-3(2H)-one (compound No. 3-065)

Firstly, 2.1 g of 1,8-diazabicyclo[5,4,0]-7-undecene was added to a mixture of 3.1 g of ethyl 2-(2-(2-(6-methoxy-2-(2-methoxyphenyl)benzo[b]thiophen-3-yl)acetyl)-2-methylhydrazinylidene)propionate and 70 mL of acetonitrile at room temperature. After completion of the addition, the reaction container was purged with nitrogen gas. After completion of the nitrogen gas purging, the mixture was stirred at 80°C for five hours. After completion of the stirring, the solvent contained in the reaction mixture was distilled off under reduced pressure. The resultant residue was dissolved in 120 mL of ethyl acetate, and 10 mL of 1 mol/L hydrochloric acid was added to the solution at room temperature. After completion of the addition, the resultant mixture was stirred at room temperature for one hour. After completion of the stirring, the organic phase was separated from the mixture. The resultant organic phase was dehydrated and dried sequentially with saturated salt water and anhydrous sodium sulfate, and the solvent was distilled off under reduced pressure. Subsequently, 20 mL of diisopropyl ether was added to the resultant residue, and the precipitated solid was recovered through filtration. The resultant solid was washed with 5 mL of a mixture of diisopropyl ether and acetonitrile (20 : 1), to thereby yield 952 mg of the target product as a white solid.
Melting point: 243-244°C
¹H NMR: δ 7.25-7.40 (m, 4H), 6.90-7.05 (m, 3H), 6.32 (s, 1H), 3.89 (s, 3H), 3.82 (s, 3H), 3.75 (s, 3H), 2.18 (s, 3H).

### Step 5: synthesis of 5-(6-methoxy-2-(2-methoxyphenyl)benzo[b]thiophen-3-yl)-1,3-dimethyl-6-oxo-1,6-dihydropyridazin-4-yl=(n-butyrate) (compound No. 3-066)

Firstly, 30 mg of triethylamine was added to a mixture of 100 mg of 5-hydroxy-4-(6-methoxy-2-(2-methoxyphenyl)benzo[b]thiophen-3-yl)-2,6-dimethylpyridazin-3(2H)-one and 2 mL of dichloromethane under ice cooling. After completion of the addition, the resultant mixture was stirred at room temperature for 30 minutes. After completion of the stirring, 29 mg of n-butyryl chloride was added to the mixture at room temperature. After completion of the addition, the resultant mixture was stirred at room temperature for one hour. After completion of the stirring, 5 mL of water was added to the reaction mixture, and the resultant mixture was subjected to extraction with 5 mL of ethyl acetate. The resultant organic phase was washed with 2 mL of water, and then dehydrated and dried sequentially with saturated salt water and anhydrous sodium sulfate. The solvent was distilled off under reduced pressure. The resultant residue was purified by silica gel column chromatography (with a gradient of ethyl acetate : n-hexane = 1 : 99 to 25 : 75), to thereby yield 116 mg of the target product as a yellow oily product.

¹H NMR: δ 7.25-7.35 (m, 4H), 6.85-7.00 (m, 3H), 3.86 (s, 3H), 3.83 (s, 3H), 3.72 (s, 3H), 2.12 (t, J=7.5Hz, 2H), 2.10 (s, 3H), 1.25-1.45 (m, 2H), 0.68 (t, J=7.3Hz, 3H).

### [Synthesis Example 4]

### Synthesis of 4-(2-(4-(difluoromethoxy)phenyl)-5-fluorobenzo[b]thiophen-3-yl)-5-hydroxy-2,6-dimethylpyridazin-3(2H)-one (compound No. 2-042)

### Step 1: synthesis of methyl 2-(2-(4-(difluoromethoxy)phenyl)-5-fluorobenzo[b]thiophen-3-yl)acetate

Firstly, 1.0 g of 2-(5-fluorobenzo[b]thiophen-3-yl)acetic acid, 250 mg of triphenylphosphine, 987 mg of potassium carbonate, and 107 mg of palladium acetate were added to a mixture of 1.3 g of 1-bromo-4-(difluoromethoxy)benzene and 5 mL of N,N-dimethylformamide at room temperature. After completion of the addition, the reaction container was purged with nitrogen gas. After completion of the nitrogen gas purging, the reaction mixture was stirred at 100°C for five hours. After completion of the stirring, 789 mg of potassium carbonate and 1.0 g of methyl iodide were added to the reaction mixture under ice cooling, and the resultant mixture was stirred at room temperature for one hour. After completion of the stirring, insoluble matter was filtered with celite, and the residue was washed with 150 mL of toluene. The resultant filtrate was washed with water (15 mL × 2), and then dehydrated and dried sequentially with saturated salt water and anhydrous sodium sulfate. The solvent was distilled off under reduced pressure. The resultant residue was purified by silica gel chromatography (with a gradient of chloroform : n-hexane = 1 : 99 to 15 : 85), to thereby yield 1.0 g of the target product as a white solid.
Melting point: 111-113°C
¹H NMR: δ 7.74 (dd, J=8.7, 4.8Hz, 1H), 7.55-7.65 (m, 2H), 7.43 (dd, J=9.6, 2.4Hz, 1H), 7.25-7.15 (m, 2H), 7.05 (ddd, J=8.7, 8.7, 2.4Hz, 1H), 6.57 (t, J=73.8Hz, 1H), 3.78 (s, 2H), 3.74 (s, 3H).

### Step 2: synthesis of 2-(2-(4-(difluoromethoxy)phenyl)-5-fluorobenzo[b]thiophen-3-yl)acetic acid

Firstly, 30 mL of 10% by mass aqueous lithium hydroxide solution was added to a mixture of 2.1 g of methyl 2-(2-(4-(difluoromethoxy)phenyl)-5-fluorobenzo[b]thiophen-3-yl)acetate, 15 mL of tetrahydrofuran, and 15 mL of ethanol at room temperature. After completion of the addition, the reaction mixture was stirred at room temperature for three hours. After completion of the stirring, the solvent contained in the reaction mixture was distilled off under reduced pressure. Subsequently, 35% by mass hydrochloric acid was added to the resultant residue under ice cooling so as to achieve a pH of 1, and the precipitated solid was recovered through filtration. The resultant solid was washed with water (15 mL × 2), to thereby yield 1.8 g of the target product as a white solid.
Melting point: 203-204°C
¹H NMR: δ 7.75 (dd, J=9.0, 4.8Hz, 1H), 7.55-7.65 (m, 2H), 7.45 (dd, J=9.6, 2.4Hz, 1H), 7.20-7.30 (m, 2H), 7.12 (ddd, J=9.0, 9.0, 2.4Hz, 1H), 6.57 (t, J=73.8Hz, 1H), 3.82 (s, 2H).

### Step 3: synthesis of ethyl 2-(2-(2-(2-(4-(difluoromethoxy)phenyl)-5-fluorobenzo[b]thiophen-3-yl)acetyl)-2-methylhydrazinylidene)propionate

Firstly, 1.1 g of 1-(3-dimethylaminopropyl)-3-ethylcarbodiimide hydrochloride and 1.0 g of pentafluorophenol were added to a mixture of 1.8 g of 2-(2-(4-(difluoromethoxy)phenyl)-5-fluorobenzo[b]thiophen-3-yl)acetic acid and 9 mL of dichloromethane at room temperature. After completion of the addition, the resultant mixture was stirred at room temperature for two hours. Subsequently, 2.2 g of triethylamine was added dropwise under ice cooling to a mixture of 1.6 g of methylhydrazine sulfate and 5 mL of dichloromethane prepared in another reaction container. After completion of the dropwise addition, the resultant mixture was stirred at room temperature for three hours. After completion of the stirring, the previously prepared mixture of 2-(2-(4-(difluoromethoxy)phenyl)-5-fluorobenzo[b]thiophen-3-yl)acetic acid, 1-(3-dimethylaminopropyl)-3-ethylcarbodiimide hydrochloride, and pentafluorophenol was added dropwise to the reaction mixture under ice cooling. After completion of the dropwise addition, the resultant mixture was stirred at room temperature for 14 hours. After completion of the stirring, 15 mL of water was added to the reaction mixture, and the organic phase was separated. The resultant organic phase was washed with 15 mL of aqueous saturated sodium hydrogen carbonate solution, and then dehydrated and dried sequentially with saturated salt water and anhydrous sodium sulfate. The solvent was distilled off under reduced pressure. The resultant residue was dissolved in 8 mL of ethanol, and 1.2 g of ethyl pyruvate was added to the solution at room temperature. After completion of the addition, the reaction container was purged with nitrogen gas. After completion of the nitrogen gas purging, the reaction mixture was stirred at 80°C for four hours. After completion of the stirring, the solvent was distilled off under reduced pressure. The resultant residue was purified by silica gel column chromatography (with a gradient of ethyl acetate : n-hexane = 3 : 97 to 25 : 75), to thereby yield 1.5 g of the target product as a pale yellow solid.
Melting point: 100-101°C
¹H NMR: δ 7.73 (dd, J=8.7, 4.8Hz, 1H), 7.55-7.65 (m, 2H), 7.35-7.50 (m, 1H), 7.15-7.25 (m, 2H), 7.12 (ddd, J=8.7, 8.7, 2.4Hz, 1H), 6.56 (t, J=73.8Hz, 1H), 4.29 (q, J=7.5Hz, 2H), 4.16 (s, 2H), 3.40 (s, 3H), 2.10 (s, 3H), 1.31 (t, J=7.5Hz, 3H).

### Step 4: synthesis of 4-(2-(4-(difluoromethoxy)phenyl)-5-fluorobenzo[b]thiophen-3-yl)-5-hydroxy-2,6-dimethylpyridazin-3(2H)-one (compound No. 2-042)

Firstly, 904 mg of 1,8-diazabicyclo[5,4,0]-7-undecene was added to a mixture of 1.4 g of ethyl 2-(2-(2-(2-(4-(difluoromethoxy)phenyl)-5-fluorobenzo[b]thiophen-3-yl)acetyl)-2-methylhydrazinylidene)propionate and 30 mL of acetonitrile at room temperature. After completion of the addition, the reaction container was purged with nitrogen gas. After completion of the nitrogen gas purging, the resultant mixture was heated to 90°C and stirred for one hour. After completion of the stirring, the solvent contained in the reaction mixture was distilled off under reduced pressure. The resultant residue was dissolved in 14 mL of ethyl acetate, and 1 mol/L hydrochloric acid was added to the solution so as to achieve a pH of 1 under ice cooling. The resultant mixture was stirred at room temperature for one hour. After completion of the stirring, the organic phase was separated from the mixture. The resultant organic phase was washed with 5 mL of 1 mol/L hydrochloric acid, and then dehydrated and dried sequentially with saturated salt water and anhydrous sodium sulfate. The solvent was distilled off under reduced pressure. Subsequently, 10 mL of diisopropyl ether and 0.5 mL of acetonitrile were added to the resultant residue, and the precipitated solid was recovered through filtration, to thereby yield 469 mg of the target product as a white solid.
Melting point: 164-165°C
¹H NMR (DMSO-d6): δ 10.54 (brs, 1H), 8.06 (dd, J=8.7, 4.8Hz, 1H), 7.40-7.50 (m, 2H), 7.27 (t, J=74.1Hz, 1H), 7.20-7.25 (m, 1H), 7.15-7.20 (m, 2H), 7.10 (dd, J=9.9, 2.4Hz, 1H), 3.57 (s, 3H), 2.19 (s, 3H).

### [Synthesis Example 5]

### Synthesis of 4-(2-(4-chlorophenyl)-5-fluorobenzo[b]thiophen-3-yl)-5-hydroxy-2,6-dimethylpyridazin-3(2H)-one (compound No. 2-010)

### Step 1: synthesis of 3-bromomethyl-2-(4-chlorophenyl)-5-fluorobenzo[b]thiophene

Firstly, 30% by mass hydrogen bromide-acetic acid solution was added dropwise under ice cooling to a mixture of 10.5 g of 2-(4-chlorophenyl)-5-fluorobenzo[b]thiophene, 3.2 g of paraformaldehyde, and 60 mL of chloroform, and the resultant mixture was stirred at 40°C for three hours. After completion of the stirring, 200 mL of water was added to the reaction mixture, and the resultant mixture was subjected to extraction with 40 mL of chloroform. The resultant organic phase was washed with 100 mL of water and 100 mL of aqueous saturated sodium hydrogen carbonate solution, and then dehydrated and dried sequentially with saturated salt water and anhydrous sodium sulfate. The solvent was distilled off under reduced pressure. Subsequently, 50 mL of n-hexane was added to the resultant residue, and then the precipitated solid was recovered through filtration, to thereby yield 12.4 g of the target product as a gray solid.
Melting point: 124-126°C
¹H NMR: δ 7.76 (dd, J=8.7, 4.8Hz, 1H), 7.45-7.65 (m, 5H), 7.10-7.20 (m, 1H), 4.66 (s, 2H).

### Step 2: synthesis of 2-(2-(4-chlorophenyl)-5-fluorobenzo[b]thiophen-3-yl)acetonitrile

Firstly, 1.7 g of potassium carbonate and 1.2 g of trimethylsilyl cyanide were added to a mixture of 3.6 g of 3-bromomethyl-2-(4-chlorophenyl)-5-fluorobenzo[b]thiophene and 15 mL of acetonitrile at room temperature, and the resultant mixture was stirred at 80°C for 12 hours. After completion of the stirring, 20 mL of 10% by mass aqueous sodium hydroxide solution was added to the reaction mixture under ice cooling, and the resultant mixture was subjected to extraction with 40 mL of dichloromethane. The resultant organic phase was washed with 10 mL of water, and then dehydrated and dried sequentially with saturated salt water and anhydrous sodium sulfate. The solvent was distilled off under reduced pressure. Subsequently, 50 mL of n-hexane and 5 mL of diisopropyl ether were added to the resultant residue, and then the precipitated solid was recovered through filtration, to thereby yield 2.8 g of the target product as a pale yellow solid.
Melting point: 161-163°C
¹H NMR: δ 7.81 (dd, J=8.1, 4.8Hz, 1H), 7.40-7.55 (m, 5H), 7.15-7.25 (m, 1H), 3.81 (s, 2H).

### Step 3: synthesis of 2-(2-(4-chlorophenyl)-5-fluorobenzo[b]thiophen-3-yl)acetic acid (compound No. 12-002)

Firstly, 10 mL of concentrated sulfuric acid was added to a mixture of 10.0 g of 2-(2-(4-chlorophenyl)-5-fluorobenzo[b]thiophen-3-yl)acetonitrile, 60 mL of acetic acid, and 20 mL of water under ice cooling. After completion of the addition, the reaction mixture was stirred under reflux with heating for 12 hours. After completion of the stirring, the reaction mixture was added dropwise to 200 mL of water under ice cooling, and the precipitated solid was recovered through filtration. To the resultant solid were added 150 mL of 5% by mass aqueous potassium hydroxide solution and 150 mL of dichloromethane at room temperature, and the organic phase was separated through a phase separation process. The resultant organic phase was subjected to extraction with 150 mL of 5% by mass aqueous potassium hydroxide solution. The resultant aqueous phases were combined and then washed with 50 mL of dichloromethane. Subsequently, 20 mL of 35% by mass hydrochloric acid was added to the resultant aqueous phase under ice cooling, and the resultant mixture was subjected to extraction with 200 mL of ethyl acetate. The resultant organic phase was dehydrated and dried sequentially with saturated salt water and anhydrous sodium sulfate, and the solvent was distilled off under reduced pressure. Subsequently, 300 mL of n-hexane and 30 mL of diisopropyl ether were added to the resultant residue, and then the precipitated solid was recovered through filtration, to thereby yield 7.7 g of the target product as a white solid.
Melting point: 202-204°C
¹H NMR: δ 7.76 (dd, J=9.0, 4.8Hz, 1H), 7.50-7.60 (m, 2H), 7.40-7.50 (m, 3H), 7.10-7.20 (m, 1H), 3.82 (s, 2H).

### Step 4: synthesis of ethyl 2-(2-(2-(2-(4-chlorophenyl)-5-fluorobenzo[b]thiophen-3-yl)acetyl)-2-methylhydrazinylidene)propionate

Firstly, 10.2 g of 1-(3-dimethylaminopropyl)-3-ethylcarbodiimide hydrochloride and 9.8 g of pentafluorophenol were added to a mixture of 17.1 g of 2-(2-(4-chlorophenyl)-5-fluorobenzo[b]thiophen-3-yl)acetic acid and 150 mL of dichloromethane at room temperature. After completion of the addition, the resultant mixture was stirred at room temperature for 18 hours. In another reaction container, 21.6 g of triethylamine was added dropwise to a mixture of 15.4 g of methylhydrazine sulfate and 90 mL of dichloromethane under ice cooling. After completion of the dropwise addition, the resultant mixture was stirred under ice cooling for one hour. After completion of the stirring, the previously prepared mixture of 2-(2-(4-chlorophenyl)-5-fluorobenzo[b]thiophen-3-yl)acetic acid, 1-(3-dimethylaminopropyl)-3-ethylcarbodiimide hydrochloride, and pentafluorophenol was added dropwise to the reaction mixture under ice cooling. After completion of the dropwise addition, the resultant mixture was stirred at room temperature for 18 hours. After completion of the stirring, 200 mL of water was added to the reaction mixture at room temperature, and the resultant mixture was subjected to extraction with 150 mL of dichloromethane. The resultant organic phase was dehydrated and dried sequentially with saturated salt water and anhydrous sodium sulfate, and the solvent was distilled off under reduced pressure. The resultant residue was dissolved in 100 mL of ethanol, and 12.4 g of ethyl pyruvate was added to the solution. After completion of the addition, the resultant mixture was stirred at 70°C for two hours. After completion of the stirring, the solvent was distilled off under reduced pressure. Subsequently, 100 mL of water was added to the resultant residue, and the resultant mixture was subjected to extraction with 150 mL of diethyl ether. The resultant organic phase was washed with 5% by mass aqueous potassium carbonate solution (100 mL × 2), and then dehydrated and dried sequentially with saturated salt water and anhydrous sodium sulfate. The solvent was distilled off under reduced pressure, to thereby yield 11.1 g of the target product as a white solid.
Melting point: 125-127°C
¹H NMR: δ 7.74 (dd, J=8.9, 4.8Hz, 1H), 7.56 (d, J=8.5Hz, 2H), 7.37-7.48 (m, 3H), 7.05-7.14 (m, 1H), 4.30 (q, J=7.2Hz, 2H), 4.16 (s, 2H), 3.41 (s, 3H), 2.29 (s, 3H), 1.31 (t, J=7.2Hz, 3H).

### Step 5: synthesis of 4-(2-(4-chlorophenyl)-5-fluorobenzo[b]thiophen-3-yl)-5-hydroxy-2,6-dimethylpyridazin-3(2H)-one (compound No. 2-010)

Firstly, 11.3 g of 1,8-diazabicyclo[5.4.0]-7-undecene was added to a mixture of 11.1 g of ethyl 2-(2-(2-(2-(4-chlorophenyl)-5-fluorobenzo[b]thiophen-3-yl)acetyl)-2-methylhydrazinylidene)propionate and 50 mL of acetonitrile at room temperature. After completion of the addition, the reaction mixture was stirred at 80°C for six hours. After completion of the stirring, the solvent was distilled off under reduced pressure. The resultant residue was dissolved in 500 mL of ethyl acetate, and 100 mL of 1 mol/L hydrochloric acid and 200 mL of water were added to the solution under ice cooling. Thereafter, the organic phase was separated through a phase separation process. The resultant aqueous phase was subjected to extraction with ethyl acetate (300 mL × 2). The resultant organic phases were combined, and then dehydrated and dried sequentially with saturated salt water and anhydrous sodium sulfate. The solvent was distilled off under reduced pressure. Subsequently, 50 mL of diisopropyl ether and 5 mL of acetonitrile were added to the resultant residue, and then the precipitated solid was recovered through filtration, to thereby yield 9.9 g of the target product as a white solid.
Melting point: 258-259°C
¹H NMR: δ 7.75 (dd, J=8.9, 4.8Hz, 1H), 7.24-7.26 (m, 4H), 7.07-7.16 (m, 1H), 6.94-7.01 (m, 1H), 6.31 (brs, 1H), 3.63 (s, 3H), 2.19 (s, 3H).
¹H NMR (DMSO-d6): δ 10.57 (brs, 1H), 8.08 (dd, J=8.9, 4.8Hz, 1H), 7.40-7.50 (m, 4H), 7.25-7.35 (m, 1H), 7.11 (dd, J=9.9, 2.4Hz, 1H), 3.57 (s, 3H), 2.19 (s, 3H).

### [Synthesis Example 6]

### Synthesis of 4-(5-chloro-2-(4-fluoro-2-methoxyphenyl)benzo[b]thiophen-3-yl)-5-hydroxy-2,6-dimethylpyridazin-3(2H)-one (compound No. 2-095)

### Step 1: synthesis of ethyl 2-(2-bromo-5-chlorobenzo[b]thiophen-3-yl)acetate

Firstly, 24.5 g of N-bromosuccinimide was added to a mixture of 33.4 g of ethyl 2-(5-chlorobenzo[b]thiophen-3-yl)acetate, 140 mL of N,N-dimethylformamide, and 14 mL of acetic acid at room temperature. After completion of the addition, the reaction container was purged with nitrogen gas. After completion of the nitrogen gas purging, the resultant mixture was heated to 60°C and stirred at 60°C for four hours. After completion of the stirring, 14.0 g of N-bromosuccinimide was added to the mixture at room temperature. After completion of the addition, the reaction container was purged with nitrogen gas. After completion of the nitrogen gas purging, the resultant mixture was heated to 60°C and stirred at 60°C for one hour. After completion of the stirring, the reaction mixture was added dropwise to 140 mL of water under ice cooling. After completion of the dropwise addition, the reaction mixture was subj ected to extraction with 150 mL of diethyl ether. The resultant organic phase was washed sequentially with 50 mL of water, 20 mL of aqueous saturated ammonium chloride solution, and 20 mL of 5% by mass aqueous sodium hydrogen sulfite solution. The resultant organic phase was dehydrated and dried sequentially with saturated salt water and anhydrous sodium sulfate, and the solvent was distilled off under reduced pressure. Subsequently, 100 mL of hexane was added to the resultant residue, and the resultant mixture was stirred at room temperature for 12 hours. Thereafter, the precipitated solid was recovered through filtration, and the resultant solid was washed with 20 mL of hexane, to thereby yield 26.3 g of the target product as a pale yellow solid.
Melting point: 70-71°C
¹H NMR: δ 7.60-7.70 (m, 2H), 7.31 (dd, J=8.7, 1.9Hz, 1H), 4.18 (q, J=7.2Hz, 2H), 3.84 (s, 2H), 1.26 (t, J=7.2Hz, 3H).

### Step 2: synthesis of ethyl 2-(5-chloro-2-(4-fluoro-2-methoxyphenyl)benzo[b]thiophen-3-yl)acetate

Firstly, 3.8 g of 4-fluoro-2-methoxyphenylboronic acid, 8.3 g of potassium carbonate, and 1.7 g of tetrakis(triphenylphosphine)palladium were added to a mixture of 5.0 g of ethyl 2-(2-bromo-5-chlorobenzo[b]thiophen-3-yl)acetate, 50 mL of 1,4-dioxane, and 10 mL of water at room temperature. After completion of the addition, the reaction container was purged with nitrogen gas. After completion of the nitrogen gas purging, the reaction mixture was stirred in a nitrogen atmosphere at 90°C for three hours. After completion of the stirring, 50 mL of water and 100 mL of ethyl acetate were added to the reaction mixture at room temperature. After completion of the addition, insoluble matter was filtered with celite. The organic phase was separated from the resultant filtrate. The resultant organic phase was washed with 50 mL of water, and then dehydrated and dried sequentially with saturated salt water and anhydrous sodium sulfate. The solvent was distilled off under reduced pressure. The resultant residue was purified by silica gel chromatography (with a gradient of ethyl acetate : n-hexane = 2 : 98 to 10 : 90), to thereby yield 3.7 g of the target product as a pale yellow solid.
Melting point: 103-105°C
¹H NMR: δ 7.65-7.75 (m, 2H), 7.25-7.45 (m, 2H), 6.70-6.80 (m, 2H), 4.14 (q, J=7.2Hz, 2H), 3.77 (s, 3H), 3.64 (s, 2H), 1.23 (t, J=7.2Hz, 3H).

### Step 3: synthesis of 2-(5-chloro-2-(4-fluoro-2-methoxyphenyl)benzo[b]thiophen-3-yl)acetic acid

Firstly, a mixture of 6.8 g of sodium hydroxide and 190 mL of water was added dropwise to a mixture of 21.6 g of ethyl 2-(5-chloro-2-(4-fluoro-2-methoxyphenyl)benzo[b]thiophen-3-yl)acetate, 266 mL of tetrahydrofuran, and 150 mL of water under ice cooling. After completion of the dropwise addition, the reaction mixture was stirred at room temperature for 13 hours. After completion of the stirring, the organic solvent was distilled off under reduced pressure. Subsequently, 35% by mass hydrochloric acid was added to the resultant residue under ice cooling so as to achieve a pH of 1, and the precipitated solid was recovered through filtration. The resultant solid was washed sequentially with water (20 mL × 2) and 20 mL of hexane, to thereby yield 19.9 g of the target product as a white solid.
Melting point: 186-188°C
¹H NMR: δ 7.65-7.75 (m, 2H), 7.25-7.40 (m, 2H), 6.70-6.80 (m, 2H), 3.77 (s, 3H), 3.68 (s, 2H).

### Step 4: synthesis of ethyl 2-(2-(2-(5-chloro-2-(4-fluoro-2-methoxyphenyl)benzo[b]thiophen-3-yl)acetyl)-2-methylhydrazinylidene)propionate

Firstly, 12.0 g of 1-(3-dimethylaminopropyl)-3-ethylcarbodiimide hydrochloride and 11.5 g of pentafluorophenol were added to a mixture of 20.0 g of 2-(5-chloro-2-(4-fluoro-2-methoxyphenyl)benzo[b]thiophen-3-yl)acetic acid and 300 mL of dichloromethane at room temperature. After completion of the addition, the resultant mixture was stirred at room temperature for 13 hours. In another reaction container, 29.5 g of diisopropylethylamine was added dropwise to a mixture of 24.6 g of methylhydrazine sulfate and 300 mL of dichloromethane at room temperature. After completion of the dropwise addition, the resultant mixture was stirred at room temperature for 13 hours. After completion of the stirring, the previously prepared mixture of 2-(5-chloro-2-(4-fluoro-2-methoxyphenyl)benzo[b]thiophen-3-yl)acetic acid, 1-(3-dimethylaminopropyl)-3-ethylcarbodiimide hydrochloride, and pentafluorophenol was added dropwise to the reaction mixture under ice cooling. After completion of the dropwise addition, the resultant mixture was stirred at room temperature for three hours. After completion of the stirring, 500 mL of water was added to the reaction mixture, and the organic phase was separated through a phase separation process. The resultant organic phase was washed with 500 mL of water, and then dehydrated and dried sequentially with saturated salt water and anhydrous sodium sulfate. The solvent was distilled off under reduced pressure. The resultant residue was dissolved in 180 mL of ethanol, and 13.2 g of ethyl pyruvate was added to the solution at room temperature. After completion of the addition, the reaction container was purged with nitrogen gas. After completion of the nitrogen gas purging, the reaction mixture was stirred in a nitrogen atmosphere at 45°C for 10 hours. After completion of the stirring, the solvent was distilled off under reduced pressure. The resultant residue was purified by silica gel chromatography (with a gradient of ethyl acetate : n-hexane = 2 : 98 to 20 : 80). Subsequently, 50 mL of diisopropyl ether was added to the resultant crude product, and the precipitated solid was recovered through filtration. The resultant solid was washed with 10 mL of diisopropyl ether, to thereby yield 15.9 g of the target product as a white solid.
Melting point: 126-128°C
¹H NMR: δ 7.65-7.75 (m, 2H), 7.35-7.45 (m, 1H), 7.25-7.35 (m, 1H), 6.65-6.80 (m, 2H), 4.26 (q, J=7.2Hz, 2H), 4.03 (s, 2H), 3.77 (s, 3H), 3.34 (s, 3H), 2.24 (s, 3H), 1.30 (t, J=7.2Hz, 3H).

### Step 5: synthesis of 4-(5-chloro-2-(4-fluoro-2-methoxyphenyl)benzo[b]thiophen-3-yl)-5-hydroxy-2,6-dimethylpyridazin-3(2H)-one (compound No. 2-095)

Firstly, 8.6 g of 1,8-diazabicyclo[5,4,0]-7-undecene was added to a mixture of 13.4 g of ethyl 2-(2-(2-(5-chloro-2-(4-fluoro-2-methoxyphenyl)benzo[b]thiophen-3-yl)acetyl)-2-methylhydrazinylidene)propionate and 57 mL of acetonitrile at room temperature. After completion of the addition, the reaction container was purged with nitrogen gas. After completion of the nitrogen gas purging, the resultant mixture was heated to 90°C and stirred at 90°C for five hours. After completion of the stirring, the solvent contained in the reaction mixture was distilled off under reduced pressure. Subsequently, 50 mL of water and 350 mL of ethyl acetate were added to the resultant residue, and 35% by mass hydrochloric acid was added to the mixture under ice cooling so as to achieve a pH of 1. The organic phase was separated from the resultant mixture. The resultant organic phase was washed sequentially with 1 mol/L hydrochloric acid (50 mL × 2) and 100 mL of water, and then dehydrated and dried sequentially with saturated salt water and anhydrous sodium sulfate. The solvent was distilled off under reduced pressure. Subsequently, 100 mL of diisopropyl ether and 2 mL of acetonitrile were added to the resultant residue. After completion of the addition, the precipitated solid was recovered through filtration, to thereby yield 11.3 g of the target product as a white solid.
Melting point: 221-223°C
¹H NMR: δ 7.70-7.80 (m, 1H), 7.20-7.40 (m, 3H), 6.60-6.75 (m, 2H), 6.02 (s, 1H), 3.79 (s, 3H), 3.73 (s, 3H), 2.21 (s, 3H).

### [Synthesis Example 7]

### Synthesis of 4-(5-cyclopropyl-2-(2-methoxyphenyl)benzo[b]thiophen-3-yl)-5-hydroxy-2,6-dimethylpyridazin-3(2H)-one (compound No. 2-160)

### Step 1: synthesis of 4-(5-chloro-2-(2-methoxyphenyl)benzo[b]thiophen-3-yl)-5-methoxy-2,6-dimethylpyridazin-3(2H)-one (compound No. 2-102)

Firstly, 3.7 g of 2-methoxyphenylboronic acid, 10.6 g of potassium phosphate, and 1.2 g of tetrakis(triphenylphosphine)palladium were added to a mixture of 8.0 g of 4-(2-bromo-5-chlorobenzo[b]thiophen-3-yl)-5-methoxy-2,6-dimethylpyridazin-3(2H)-one and 60 mL of toluene at room temperature. After completion of the addition, the reaction container was purged with nitrogen gas. After completion of the nitrogen gas purging, the reaction mixture was stirred in a nitrogen atmosphere at 110°C for four hours. After completion of the stirring, insoluble matter was filtered with celite, and the residue was washed with 100 mL of ethyl acetate. The resultant filtrate was washed with 100 mL of water, and then dehydrated and dried sequentially with saturated salt water and anhydrous sodium sulfate. The solvent was distilled off under reduced pressure. The resultant residue was purified by silica gel chromatography (with a gradient of ethyl acetate : n-hexane = 20 : 80 to 40 : 60), to thereby yield 7.0 g of the target product as an ocher solid.
Melting point: 174-175°C
¹H NMR: δ 7.72 (d, J=8.7Hz 1H), 7.43 (dd, J=2.1, 0.6Hz, 1H), 7.28-7.36 (m, 3H), 6.86-6.96 (m, 2H), 3.74 (s, 3H), 3.70 (s, 3H), 3.36 (s, 3H), 2.11 (s, 3H).

### Step 2: synthesis of tert-butyl (3-(5-methoxy-2,6-dimethyl-3-oxo-2,3-dihydropyridazin-4-yl)-2-(2-methoxyphenyl)benzo[b]thiophen-5-yl)carbamate (compound No. 2-182)

Firstly, 1.5 g of tert-butyl carbamate, 127 mg of 2-dicyclohexylphosphino-2',6'-dimethoxybiphenyl, 3.0 g of cesium carbonate, and 42 mg of palladium acetate were added to a mixture of 1.2 g of 4-(5-chloro-2-(2-methoxyphenyl)benzo[b]thiophen-3-yl)-5-methoxy-2,6-dimethylpyridazin-3(2H)-one and 15 mL of 1,4-dioxane at room temperature. After completion of the addition, the reaction container was purged with nitrogen gas. After completion of the nitrogen gas purging, the reaction mixture was stirred in a nitrogen atmosphere at 110°C for six hours. After completion of the stirring, insoluble matter was filtered with celite, and the residue was washed with 30 mL of ethyl acetate. The resultant filtrate was washed with 20 mL of water, and then dehydrated and dried sequentially with saturated salt water and anhydrous sodium sulfate. The solvent was distilled off under reduced pressure, to thereby yield 1.4 g of the target product as a pale yellow oily product. This product was used in the next step without further purification.

¹H NMR: δ 7.72 (d, J=9.0Hz, 1H), 7.40-7.50 (m, 1H), 7.28-7.38 (m, 3H), 6.88-6.95 (m, 1H), 6.87 (d, J=9.0Hz, 1H), 6.60 (brs, 1H), 3.74 (s, 3H), 3.69 (s, 3H), 3.36 (s, 3H), 2.08 (s, 3H), 1.50 (s, 9H).

### Step 3: synthesis of 4-(5-amino-2-(2-methoxyphenyl)benzo[b]thiophen-3-yl)-5-methoxy-2,6-dimethylpyridazin-3(2H)-one (compound No. 2-174)

Firstly, 5 mL of trifluoroacetic acid was added to a mixture of 1.4 g of tert-butyl (3-(5-methoxy-2,6-dimethyl-3-oxo-2,3-dihydropyridazin-4-yl)-2-(2-methoxyphenyl)benzo[b]thiophen-5-yl)carbamate and 10 mL of dichloromethane under ice cooling. After completion of the addition, the resultant mixture was stirred at room temperature for 20 hours. After completion of the stirring, the solvent was distilled off under reduced pressure. Subsequently, 80 mL of ethyl acetate was added to the resultant residue, and the mixture was washed with 30 mL of aqueous saturated sodium hydrogen carbonate solution and 20 mL of water. The resultant organic phase was dehydrated and dried sequentially with saturated salt water and anhydrous sodium sulfate, and the solvent was distilled off under reduced pressure. Subsequently, 20 mL of diisopropyl ether was added to the resultant residue, and then the precipitated solid was recovered through filtration, to thereby yield 390 mg of the target product as a pale yellow solid.
Melting point: 219-221°C
¹H NMR: δ 7.57 (d, J=9.3Hz, 1H), 7.35 (dd, J=7.5, 1.5Hz, 1H), 7.20-7.28 (m, 2H), 6.75-6.95 (m, 3H), 3.74 (s, 3H), 3.68 (s, 3H), 3.37 (s, 3H), 2.09 (s, 3H), 1.61 (brs, 2H).

### Step 4: synthesis of 4-(5-iodo-2-(2-methoxyphenyl)benzo[b]thiophen-3-yl)-5-methoxy-2,6-dimethylpyridazin-3(2H)-one (compound No. 2-131)

Firstly, 4.4 g of 35% by mass hydrochloric acid was added to a mixed suspension of 1.7 g of 4-(5-amino-2-(2-methoxyphenyl)benzo[b]thiophen-3-yl)-5-methoxy-2,6-dimethylpyridazin-3(2H)-one and 15 mL of water under ice cooling. After completion of the addition, a mixture of 345 mg of sodium nitrite and 2.5 mL of water was added dropwise to the mixed suspension under ice cooling, and the resultant mixture was stirred under ice cooling for 30 minutes. After completion of the stirring, a mixture of 1.9 g of sodium iodide and 5.5 mL of water was added dropwise to the resultant mixture under ice cooling. After completion of the dropwise addition, the reaction mixture was stirred at room temperature for 20 hours. After completion of the stirring, 30 mL of water was added to the reaction mixture, and the resultant mixture was subjected to extraction with 50 mL of ethyl acetate. The resultant organic phase was washed with 5% by mass aqueous sodium thiosulfate solution (30 mL × 2), and then dehydrated and dried sequentially with saturated salt water and anhydrous sodium sulfate. The solvent was distilled off under reduced pressure. The resultant residue was purified by silica gel chromatography (with a gradient of ethyl acetate : n-hexane = 20 : 80 to 40 : 60), to thereby yield 1.5 g of the target product as an orange solid.
Melting point: 161-163°C
¹H NMR: δ 7.76-7.78 (m, 1H), 7.53-7.62 (m, 2H), 7.26-7.36 (m, 2H), 6.92 (dd, J=7.5, 1.2Hz, 1H), 6.87 (d, J=7.5Hz, 1H), 3.74 (s, 3H), 3.70 (s, 3H), 3.35 (s, 3H), 2.11 (s, 3H).

### Step 5: synthesis of 4-(5-cyclopropyl-2-(2-methoxyphenyl)benzo[b]thiophen-3-yl)-5-methoxy-2,6-dimethylpyridazin-3(2H)-one (compound No. 2-161)

Firstly, 62 mg of cyclopropylboronic acid, 256 mg of potassium phosphate, and 28 mg of tetrakis(triphenylphosphine)palladium were added to a mixture of 250 mg of 4-(5-iodo-2-(2-methoxyphenyl)benzo[b]thiophen-3-yl)-5-methoxy-2,6-dimethylpyridazin-3(2H)-one and 3 mL of toluene at room temperature. After completion of the addition, the reaction container was purged with nitrogen gas. After completion of the nitrogen gas purging, the reaction mixture was stirred in a nitrogen atmosphere at 110°C for two hours. After completion of the stirring, insoluble matter was filtered with celite, and the residue was washed with 10 mL of ethyl acetate. The resultant filtrate was washed with water (5 mL × 2), and then dehydrated and dried sequentially with saturated salt water and anhydrous sodium sulfate. The solvent was distilled off under reduced pressure. The resultant residue was purified by silica gel chromatography (with a gradient of ethyl acetate : n-hexane = 20 : 80 to 40 : 60), to thereby yield 157 mg of the target product as an orange oily product.

¹H NMR: δ 7.69 (d, J=7.8Hz, 1H), 7.26-7.36 (m, 2H), 7.20 (d, J=1.8Hz, 1H), 6.85-7.00 (m, 3H), 3.74 (s, 3H), 3.68 (s, 3H), 3.37 (s, 3H), 2.12 (s, 3H), 1.95-2.05 (m, 1H), 0.92-1.00 (m, 2H), 0.62-0.70 (m, 2H).

### Step 6: synthesis of 4-(5-cyclopropyl-2-(2-methoxyphenyl)benzo[b]thiophen-3-yl)-5-hydroxy-2,6-dimethylpyridazin-3(2H)-one (compound No. 2-160)

Firstly, a mixture of 140 mg of 4-(5-cyclopropyl-2-(2-methoxyphenyl)benzo[b]thiophen-3 -yl)-5-methoxy-2,6-dimethylpyridazin-3 (2H)-one and 1.5 mL of morpholine was stirred at 100°C for eight hours. After completion of the stirring, 5 mL of water was added to the reaction mixture, and 35% by mass hydrochloric acid was added to the mixture under ice cooling so as to achieve a pH of 1. After completion of the addition, the resultant mixture was subjected to extraction with 15 mL of ethyl acetate. The resultant organic phase was washed with 5 mL of water, and then dehydrated and dried sequentially with saturated salt water and anhydrous sodium sulfate. The solvent was distilled off under reduced pressure. Subsequently, 5 mL of diisopropyl ether was added to the resultant residue, and then the precipitated solid was recovered through filtration, to thereby yield 42 mg of the target product as a white solid.
Melting point: 182-184°C
¹H NMR: δ 7.72 (d, J=8.4Hz, 1H), 7.30-7.40 (m, 2H), 7.15-7.18 (m, 1H), 7.02-7.08 (m, 1H), 6.90-7.00 (m, 2H), 6.39 (brs, 1H), 3.78 (s, 3H), 3.74 (s, 3H), 2.17 (s, 3H), 1.92-2.04 (m, 1H), 0.90-1.00 (m, 2H), 0.65-0.75 (m, 2H).

[Synthesis Example 8]

### Synthesis of 5-hydroxy-4-(2-(2-methoxyphenyl)-5-((trifluoromethyl)thio)benzo[b]thiophen-3-yl)-2,6-dimethylpyridazin-3(2H)-one (compound No. 2-378)

### Step 1: synthesis of 5-methoxy-4-(2-(2-methoxyphenyl)-5-((trifluoromethyl)thio)benzo[b]thiophen-3-yl)-2,6-dimethylpyridazin-3(2H)-one (compound No. 2-379)

Firstly, trifluoromethanethiol copper (I) was added to a mixture of 150 mg of 4-(5-iodo-2-(2-methoxyphenyl)benzo[b]thiophen-3-yl)-5-methoxy-2,6-dimethylpyridazin-3(2H)-one and 1.5 mL of 1,3-dimethyl-2-imidazolidine at room temperature. After completion of the addition, the reaction mixture was stirred at 130°C for three hours. After completion of the stirring, insoluble matter was filtered with celite, and the residue was washed with 10 mL of ethyl acetate. The resultant filtrate was washed with 10 mL of aqueous saturated ammonium chloride solution and 5 mL of water, and then dehydrated and dried sequentially with saturated salt water and anhydrous sodium sulfate. The solvent was distilled off under reduced pressure. The resultant residue was purified by silica gel chromatography (with a gradient of ethyl acetate : n-hexane = 10 : 90 to 30 : 70), to thereby yield 126 mg of the target product as a pale yellow solid.
Melting point: 152-155°C
¹H NMR: δ 7.87 (d, J=8.4Hz, 1H), 7.75 (d, J=1.8Hz, 1H), 7.55-7.62 (m, 1H), 7.30-7.38 (m, 2H), 6.86-6.98 (m, 2H), 3.74 (s, 3H), 3.72 (s, 3H), 3.36 (s, 3H), 2.13 (s, 3H).

### Step 2: synthesis of 5-hydroxy-4-(2-(2-methoxyphenyl)-5-((trifluoromethyl)thio)benzo[b]thiophen-3-yl)-2,6-dimethylpyridazin-3(2H)-one (compound No. 2-378)

Firstly, a mixture of 110 mg of 5-methoxy-4-(2-(2-methoxyphenyl)-5-((trifluoromethyl)thio)benzo[b]thiophen-3-yl)-2,6-dimethylpyridazin-3(2H)-one and 1.5 mL of morpholine was stirred at 100°C for seven hours. After completion of the stirring, 5 mL of water was added to the reaction mixture, and 35% by mass hydrochloric acid was added to the mixture under ice cooling so as to achieve a pH of 1. After completion of the addition, the resultant mixture was subj ected to extraction with 15 mL of ethyl acetate. The resultant organic phase was washed with 5 mL of water, and then dehydrated and dried sequentially with saturated salt water and anhydrous sodium sulfate. The solvent was distilled off under reduced pressure. Subsequently, 5 mL of diisopropyl ether was added to the resultant residue, and then the precipitated solid was recovered through filtration, to thereby yield 65 mg of the target product as a white solid.
Melting point: 183-186°C
¹H NMR: δ 7.89 (d, J=8.1Hz, 1H), 7.60-7.70 (m, 2H), 7.28-7.42 (m, 2H), 6.94-7.00 (m, 2H), 6.33 (brs, 1H), 3.81 (s, 3H), 3.74 (s, 3H), 2.20 (s, 3H).

[Synthesis Example 9]

### Synthesis of 6-chloro-5-hydroxy-4-(6-methoxy-2-(2-methoxyphenyl)benzo[b]thiophen-3-yl)-2-methylpyridazin-3(2H)-one (compound No. 3-088)

### Step 1: synthesis of ethyl 2-(dibenzylamino)-2-(2-methylhydrazinylidene)acetate

Firstly, a mixture of 1.3 g of ethyl 2-(2-methylhydrazinylidene)acetate and 10 mL of N,N-dimethylformamide was heated to 50°C, and 1.5 g of N-chlorosuccinimide was added to the mixture at 50°C. After completion of the addition, the reaction mixture was stirred at 50 to 60°C for three hours. After completion of the stirring, 30 mL of water was added to the reaction mixture, and the resultant mixture was subjected to extraction with tert-butyl methyl ether (30 mL × 2). The resultant organic phase was washed with 30 mL of water, and then dehydrated and dried sequentially with saturated salt water and anhydrous sodium sulfate. The solvent was distilled off under reduced pressure. Subsequently, 10 mL of tert-butyl methyl ether and 1.0 g of dibenzylamine were added to the resultant residue at room temperature. After completion of the addition, the reaction mixture was cooled to 0°C, and 1.1 g of 1,8-diazabicyclo[5,4,0]-7-undecene was added to the mixture at 0°C. After completion of the addition, the reaction mixture was stirred at room temperature for 18 hours. After completion of the stirring, 30 mL of water was added to the reaction mixture, and the resultant mixture was subjected to extraction with ethyl acetate (30 mL × 2). The resultant organic phase was washed with 30 mL of water, and then dehydrated and dried sequentially with saturated salt water and anhydrous sodium sulfate. The solvent was distilled off under reduced pressure. The resultant residue was purified by silica gel column chromatography (with a gradient of ethyl acetate : n-hexane = 5 : 95 to 10 : 90), to thereby yield 714 mg of the target product as a colorless oily product.

¹H NMR: δ 7.02-7.34 (m, 10H), 4.26 (q, 7.2Hz, 2H), 4.02 (s, 4H), 2.86 (d, J=4.2Hz, 3H), 1.31 (t, J=7.2Hz, 3H).

### Step 2: synthesis of ethyl 2-(dibenzylamino)-2-(2-(2-(6-methoxy-2-(2-methoxyphenyl)benzo[b]thiophen-3-yl)acetyl)-2-methylhydrazinylidene)acetate

Firstly, 458 mg of oxalyl chloride and 10 mg of N,N-dimethylformamide were added to a mixture of 790 mg of 2-(6-methoxy-2-(2-methoxyphenyl)benzo[b]thiophen-3-yl)acetic acid produced in step 2 of Synthesis Example 3 and 8 mL of dichloromethane at room temperature. After completion of the addition, the reaction mixture was stirred at room temperature for two hours. After completion of the stirring, the solvent was distilled off under reduced pressure. The resultant residue was dissolved in 10 mL of toluene, and 712 mg of ethyl 2-(dibenzylamino)-2-(2-methylhydrazinylidene)acetate and 243 mg of pyridine were added to the solution. After completion of the addition, the resultant mixture was stirred at 100°C for one hour. After completion of the stirring, 30 mL of water was added to the mixture, and the resultant mixture was subjected to extraction with ethyl acetate (50 mL × 2). The resultant organic phase was washed with 20 mL of water, and then dehydrated and dried sequentially with saturated salt water and anhydrous sodium sulfate. The solvent was distilled off under reduced pressure. The resultant residue was purified by silica gel column chromatography (with a gradient of ethyl acetate : n-hexane = 5 : 95 to 50 : 50), to thereby yield 743 mg of the target product as a yellow oily product.

¹H NMR: δ 7.57 (d, J=8.7Hz, 1H), 7.49 (dd, J=7.5, 1.5Hz, 1H), 7.25-7.28 (m, 9H), 7.18-7.20 (m, 3H), 6.91-6.99 (m, 3H), 4.32 (brs, 4H), 4.28 (q, J=7.2Hz, 2H), 3.85 (s, 3H), 3.78 (s, 3H), 3.73 (s, 2H), 3.14 (s, 3H), 1.22 (t, J=7.2Hz, 3H).

### Step 3: synthesis of 6-(dibenzylamino)-5-hydroxy-4-(6-methoxy-2-(2-methoxyphenyl)benzo[b]thiophen-3-yl)-2-methylpyridazin-3(2H)-one (compound No. 3-086)

Firstly, a mixture of 743 mg of ethyl 2-(dibenzylamino)-2-(2-(2-(6-methoxy-2-(2-methoxyphenyl)benzo[b]thiophen-3-yl)acetyl)-2-methylhydrazinylidene)acetate and 5 mL of tetrahydrofuran was cooled to 0°C, and 387 mg of potassium tert-butoxide was added to the mixture at 0°C. After completion of the addition, the reaction mixture was stirred at 0°C for one hour. After completion of the stirring, 5 mL of 1 mol/L hydrochloric acid and 20 mL of water were added to the mixture, and then the precipitated solid was recovered through filtration. The resultant solid was washed sequentially with 5 mL of water and 5 mL of diisopropyl ether, to thereby yield 576 mg of the target product as a yellow solid.
Melting point: 163-166°C
¹H NMR: δ 7.24-7.35 (m, 10H), 7.08-7.11 (m, 4H), 6.95-7.00 (m 2H), 6.88 (d, J=7.8Hz, 1H), 6.57 (brs, 1H), 4.00-4.30 (m, 4H), 3.88 (s, 3H), 3.69 (s, 3H), 3.58 (s, 3H).

### Step 4: synthesis of 6-amino-5-hydroxy-4-(6-methoxy-2-(2-methoxyphenyl)benzo[b]thiophen-3-yl)-2-methylpyridazin-3(2H)-one (compound No. 3-087)

Firstly, 550 mg of 5% palladium carbon (available from N.E.CHEMCAT CORPORATION, STD type, 50% hydrous product) was added to a mixture of 549 mg of 6-(dibenzylamino)-5-hydroxy-4-(6-methoxy-2-(2-methoxyphenyl)benzo[b]thiophen-3-yl)-2-methylpyridazin-3(2H)-one, 5 mL of methanol, and 0.5 mL of 35% by mass hydrochloric acid, and the reaction container was purged with hydrogen gas. After completion of the hydrogen gas purging, the reaction mixture was stirred at room temperature for 20 hours. After completion of the stirring, insoluble matter was filtered with celite, and the residue was washed with 10 mL of methanol. The resultant filtrate was concentrated under reduced pressure. The resultant residue was purified by silica gel column chromatography (with a gradient of ethyl acetate : n-hexane = 50 : 50 to 100 : 0), to thereby yield 260 mg of the target product as a white solid.
Melting point: 286-288°C
¹H NMR: δ 7.30-7.38 (m, 4H), 6.97-7.01 (m, 3H), 6.56 (s, 1H), 4.15 (brs, 2H), 3.88 (s, 3H), 3.82 (s, 3H), 3.63 (s, 3H).

### Step 5: synthesis of 6-chloro-5-hydroxy-4-(6-methoxy-2-(2-methoxyphenyl)benzo[b]thiophen-3-yl)-2-methylpyridazin-3(2H)-one (compound No. 3-088)

Firstly, a mixture of 242 mg of 6-amino-5-hydroxy-4-(6-methoxy-2-(2-methoxyphenyl)benzo[b]thiophen-3-yl)-2-methylpyridazin-3(2H)-one, 3 mL of acetonitrile, and 2 mL of 35% by mass hydrochloric acid was cooled to 0°C, and 110 mg of sodium nitrite was added to the mixture. After completion of the addition, the resultant mixture was stirred at room temperature for five hours. After completion of the stirring, 30 mL of water was added to the reaction mixture, and the resultant mixture was subjected to extraction with ethyl acetate (30 mL × 2). The resultant organic phase was dehydrated and dried sequentially with saturated salt water and anhydrous sodium sulfate, and the solvent was distilled off under reduced pressure. The resultant residue was purified by silica gel column chromatography (with a gradient of ethyl acetate : n-hexane = 30 : 70 to 70 : 30), to thereby yield 51 mg of the target product as a white solid.
Melting point: 209-211°C
¹H NMR: δ 7.28-7.40 (m, 4H), 6.95-7.02 (m, 3H), 6.73 (brs, 1H), 3.88 (s, 3H), 3.82 (s, 3H), 3.75 (s, 3H).

### [Synthesis Example 10]

### Synthesis of 5-hydroxy-4-(2-(2-methoxyphenyl)-6-(2,2,2-trifluoroethoxy)benzo[b]thiophen-3-yl)-2,6-dimethylpyridazin-3(2H)-one (compound No. 3-116)

### Step 1: synthesis of ethyl 2-(2-bromo-6-methoxybenzo[b]thiophen-3-yl)acetate

Firstly, 8.5 g of N-bromosuccinimide was added to a mixture of 12.9 g of ethyl 2-(6-methoxybenzo[b]thiophen-3-yl)acetate and 70 mL of acetonitrile under ice cooling, and the resultant mixture was stirred under ice cooling for one hour. After completion of the stirring, the solvent was distilled off under reduced pressure. Subsequently, 200 mL of aqueous saturated ammonium chloride solution was added to the resultant residue, and the resultant mixture was subjected to extraction with ethyl acetate (200 mL × 2). The resultant organic phase was dehydrated and dried sequentially with saturated salt water and anhydrous sodium sulfate. The solvent was distilled off under reduced pressure, to thereby yield 12.1 g of the target product as a brown solid. This product was used in the next step without further purification.
Melting point: 143-148°C
¹H NMR: δ 7.53-7.58 (m, 1H), 7.18-7.22 (m, 1H), 6.95-7.05 (m, 1H), 4.16 (q, J=7.2Hz, 2H), 3.82 (s, 2H), 3.86 (s, 3H), 1.24 (t, J=7.2Hz, 3H).

### Step 2: synthesis of ethyl 2-(6-(benzyloxy)-2-bromobenzo[b]thiophen-3-yl)acetate

Firstly, 60 mL of 1 mol/L dichloromethane solution of boron tribromide was added dropwise under ice cooling to a mixture of 12.1 g of ethyl 2-(2-bromo-6-methoxybenzo[b]thiophen-3-yl)acetate and 100 mL of dichloromethane, and the resultant mixture was stirred under ice cooling for one hour. After completion of the stirring, the reaction mixture was poured into 200 mL of water cooled at 0°C, and then the precipitated solid was recovered through filtration. The resultant solid was washed with 50 mL of water and 50 mL of diisopropyl ether. Subsequently, 8.4 g of potassium carbonate and 8.5 g of benzyl bromide were added to a mixture of the resultant solid and 30 mL of N,N-dimethylformamide at room temperature, and the resultant mixture was stirred at room temperature overnight. After completion of the stirring, 100 mL of water was added to the resultant mixture, and the mixture was subjected to extraction with a mixed solvent of 100 mL of diethyl ether and 100 mL of ethyl acetate. The resultant organic phase was washed with 100 mL of water, and then dehydrated and dried sequentially with saturated salt water and anhydrous sodium sulfate. The solvent was distilled off under reduced pressure. Diisopropyl ether was added to the resultant residue, and then the precipitated solid was recovered through filtration, to thereby yield 9.2 g of the target product as a pale orange solid.
Melting point: 103-105°C
¹H NMR: δ 7.53-7.58 (m, 1H), 7.30-7.45 (m, 5H), 7.25-7.28 (m, 1H), 7.03-7.08 (m, 1H), 5.11 (s, 2H), 4.15 (q, J=7.2Hz, 2H), 3.79 (s, 2H), 1.23 (t, J=7.2Hz, 3H).

### Step 3: synthesis of 2-(6-(benzyloxy)-2-bromobenzo[b]thiophen-3-yl)acetic acid

Firstly, 18 mL of 10% by mass aqueous sodium hydroxide solution was added to a mixture of 9.2 g of ethyl 2-(6-(benzyloxy)-2-bromobenzo[b]thiophen-3-yl)acetate, 30 mL of methanol, and 30 mL of tetrahydrofuran under ice cooling. After completion of the addition, the reaction mixture was stirred at room temperature for 18 hours. After completion of the stirring, the organic solvent was distilled off under reduced pressure, and 20 mL of 35% by mass hydrochloric acid was added to the resultant residue under ice cooling. After completion of the addition, the precipitated solid was recovered through filtration, and then washed with 50 mL of water, to thereby yield 8.5 g of the target product as a white solid.
Melting point: 170-172°C
¹H NMR (DMSO-d6): δ 12.56 (brs, 1H), 7.60-7.70 (m, 2H), 7.25-7.50 (m, 5H), 7.11 (dd, J=8.8, 2.3Hz, 1H), 5.16 (s, 2H), 3.79 (s, 2H).

### Step 4: synthesis of 4-(6-(benzyloxy)-2-bromobenzo[b]thiophen-3-yl)-5-hydroxy-2,6-dimethylpyridazin-3 (2H)-one

Firstly, 4.3 g of 1-(3-dimethylaminopropyl)-3-ethylcarbodiimide hydrochloride and 4.2 g of pentafluorophenol were added to a mixture of 8.5 g of 2-(6-(benzyloxy)-2-bromobenzo[b]thiophen-3-yl)acetic acid and 50 mL of dichloromethane at room temperature. After completion of the addition, the resultant mixture was stirred at room temperature for 18 hours. In another reaction container, 6.9 g of triethylamine was added dropwise to a mixture of 6.5 g of methylhydrazine sulfate and 50 mL of dichloromethane under ice cooling. After completion of the dropwise addition, the resultant mixture was stirred under ice cooling for one hour. After completion of the stirring, the previously prepared mixture of 2-(6-(benzyloxy)-2-bromobenzo[b]thiophen-3-yl)acetic acid, 1-(3-dimethylaminopropyl)-3-ethylcarbodiimide hydrochloride, and pentafluorophenol was added dropwise to the reaction mixture under ice cooling. After completion of the dropwise addition, the resultant mixture was stirred at room temperature for 18 hours. After completion of the stirring, 100 mL of water was added to the reaction mixture at room temperature, and the resultant mixture was subj ected to extraction with 100 mL of dichloromethane. The resultant organic phase was dehydrated and dried sequentially with saturated salt water and anhydrous sodium sulfate, and the solvent was distilled off under reduced pressure.

The resultant residue was dissolved in 50 mL of ethanol, and 5.2 g of ethyl pyruvate was added to the solution at room temperature. After completion of the addition, the resultant mixture was stirred at 70°C for two hours. After completion of the stirring, the solvent was distilled off from the reaction mixture under reduced pressure. Subsequently, 100 mL of water was added to the resultant residue, and the resultant mixture was subjected to extraction with 100 mL of diethyl ether. The resultant organic phase was dehydrated and dried sequentially with saturated salt water and anhydrous sodium sulfate, and the solvent was distilled off under reduced pressure.

The resultant residue was dissolved in 50 mL of acetonitrile, and 2.6 g of 1,8-diazabicyclo[5.4.0]-7-undecene was added to the solution at room temperature, followed by stirring at 80°C for six hours. After completion of the stirring, the solvent was distilled off under reduced pressure. The resultant residue was dissolved in 50 mL of ethyl acetate, and 35 mL of 1 mol/L hydrochloric acid and 15 mL of water were added to the solution under ice cooling, followed by separation of the organic phase and the aqueous phase. The resultant aqueous phase was subjected to extraction with ethyl acetate (50 mL × 2). The resultant organic phases were combined, and then dehydrated and dried sequentially with saturated salt water and anhydrous sodium sulfate. The solvent was distilled off under reduced pressure, to thereby yield 2.6 g of the crude target product as a brown amorphous product. This product was used in the next step without further purification.

¹H NMR: δ 7.10-7.50 (m, 7H), 7.02 (dd, J=9.0, 2.5Hz, 1H), 5.97 (brs, 1H), 5.12 (s, 2H), 3.75 (s, 3H), 2.35 (s, 3H).

### Step 5: synthesis of 4-(6-(benzyloxy)-2-bromobenzo[b]thiophen-3-yl)-5-methoxy-2,6-dimethylpyridazin-3 (2H)-one

Firstly, 2.4 g of potassium carbonate and 1.6 g of iodomethane were added to a mixture of 2.6 g of 4-(6-(benzyloxy)-2-bromobenzo[b]thiophen-3-yl)-5-hydroxy-2,6-dimethylpyridazin-3(2H)-one and 10 mL of N,N-dimethylformamide. After completion of the addition, the resultant mixture was stirred at room temperature for 18 hours. After completion of the stirring, 50 mL of water was added to the reaction mixture, and the resultant mixture was subjected to extraction with 50 mL of ethyl acetate. The resultant organic phase was dehydrated and dried sequentially with saturated salt water and anhydrous sodium sulfate, and the solvent was distilled off under reduced pressure. The resultant residue was purified by silica gel chromatography (with a gradient of ethyl acetate : n-hexane = 5 : 95 to 25 : 75), to thereby yield 1.8 g of the target product as a pale yellow solid.
Melting point: 167-169°C
¹H NMR: δ 7.25-7.50 (m, 7H), 7.04 (dd, J=8.9, 2.4Hz, 1 H), 5.11 (s, 2H), 3.75 (s, 3H), 3.43 (s, 3H), 2.32 (s, 3H).

### Step 6: synthesis of 4-(6-(benzyloxy)-2-(2-methoxyphenyl)benzo[b]thiophen-3-yl)-5-methoxy-2,6-dimethylpyridazin-3(2H)-one

Firstly, 2.5 g of potassium phosphate, 1.2 g of (2-methoxyphenyl)boronic acid, and 450 mg of tetrakis(triphenylphosphine)palladium were added to a mixture of 1.2 g of 4-(6-(benzyloxy)-2-bromobenzo[b]thiophen-3-yl)-5-methoxy-2,6-dimethylpyridazin-3(2H)-one and 15 mL of toluene. After completion of the addition, the reaction container was purged with nitrogen gas. After completion of the nitrogen gas purging, the reaction mixture was stirred in a nitrogen atmosphere at 100°C for two hours. After completion of the stirring, insoluble matter was filtered with celite. The resultant filtrate was concentrated under reduced pressure. The resultant residue was purified by silica gel chromatography (with a gradient of ethyl acetate : n-hexane = 10 : 90 to 50 : 50), to thereby yield 1.9 g of the target product as a dark red amorphous product.

¹H NMR: δ 7.28-7.48 (m, 9H), 7.04-7.10 (m, 1H), 6.68-7.02 (m, 2H), 5.14 (s, 2H), 3.74 (s, 3H), 3.70 (s, 3H), 3.37 (s, 3H), 2.10 (s, 3H).

### Step 7: synthesis of 4-(6-hydroxy-2-(2-methoxyphenyl)benzo[b]thiophen-3-yl)-5-methoxy-2,6-dimethylpyridazin-3(2H)-one (compound No. 3-172)

Firstly, 1.9 g of 5% palladium carbon (available from N.E.CHEMCAT CORPORATION, STD type, 50% hydrous product) was added to a mixture of 1.9 g of 4-(6-(benzyloxy)-2-(2-methoxyphenyl)benzo[b]thiophen-3-yl)-5-methoxy-2,6-dimethylpyridazin-3(2H)-one, 40 mL of methanol, and 20 mL of tetrahydrofuran. After completion of the addition, the reaction container was purged with nitrogen gas. After completion of the nitrogen gas purging, the reaction container was purged with hydrogen gas. After completion of the hydrogen gas purging, the reaction mixture was stirred at room temperature for 48 hours. After completion of the stirring, the palladium carbon was separated through filtration with celite, and the residue was washed with 100 mL of chloroform. The solvent was distilled off from the resultant filtrate under reduced pressure, to thereby yield 1.5 g of the target product as a pale yellow solid.

¹H NMR: δ 7.74 (s, 1H), 7.27-7.40 (m, 2H), 7.03-7.11 (m, 1H), 6.82-6.99 (m, 2H), 6.68-6.73 (m, 1H), 6.56-6.61 (m, 1H), 3.81 (s, 3H), 3.71 (s, 3H), 3.45 (s, 3H), 2.13 (s, 3H).

### Step 8: synthesis of 5-methoxy-4-(2-(2-methoxyphenyl)-6-(2,2,2-trifluoroethoxy)benzo[b]thiophen-3-yl)-2,6-dimethylpyridazin-3(2H)-one (compound No. 3-117)

Firstly, 200 mg of potassium carbonate and 340 mg of 2,2,2-trifluoroethyl trifluoromethanesulfonate were added to a mixture of 200 mg of 4-(6-hydroxy-2-(2-methoxyphenyl)benzo[b]thiophen-3-yl)-5-methoxy-2,6-dimethylpyridazin-3(2H)-one and 3 mL of acetonitrile at room temperature. After completion of the addition, the reaction mixture was stirred at room temperature for 18 hours. After completion of the stirring, insoluble matter was filtered with celite, and the resultant filtrate was concentrated under reduced pressure. The resultant residue was purified by silica gel chromatography (with a gradient of ethyl acetate : n-hexane = 5 : 95 to 25 : 75), to thereby yield 162 mg of the target product as a colorless oily product.

¹H NMR: δ 7.24-7.44 (m, 4H), 7.03 (dd, J=8.9, 2.4Hz, 1H), 6.83-6.95 (m, 2H), 4.40 (q, J=8.2Hz, 2H), 3.73 (s, 3H), 3.69 (s, 3H), 3.35 (s, 3H), 2.09 (s, 3H).

### Step 9: synthesis of 5-hydroxy-4-(2-(2-methoxyphenyl)-6-(2,2,2-trifluoroethoxy)benzo[b]thiophen-3-yl)-2,6-dimethylpyridazin-3(2H)-one (compound No. 3-116)

Firstly, 1 mL of morpholine was added to a mixture of 162 mg of 5-methoxy4-(2-(2-methoxyphenyl)-6-(2,2,2-trifluoroethoxy)benzo[b]thiophen-3-yl)-2,6-dimethylpyridazin-3(2H)-one and 3 mL of N-methyl-2-pyrrolidone at room temperature, and the resultant mixture was stirred at 100°C for three hours. After completion of the stirring, 20 mL of 1 mol/L hydrochloric acid was added to the reaction mixture under ice cooling, and the resultant mixture was subjected to extraction with ethyl acetate (30 mL × 2). The resultant organic phase was dehydrated and dried sequentially with saturated salt water and anhydrous sodium sulfate, and the solvent was distilled off under reduced pressure. Subsequently, 2 mL of diisopropyl ether was added to the resultant residue, and then the precipitated solid was recovered through filtration, to thereby yield 19 mg of the target product as a white solid.
Melting point: 216-218°C
¹H NMR: δ 7.35-7.40 (m, 3H), 7.27-7.34 (m, 1H), 7.01-7.07 (m, 1H), 6.91-6.99 (m, 2H), 4.42 (q, J=8.2Hz, 2H), 3.81 (s, 3H), 3.74 (s, 3H), 2.18 (s, 3H).

### [Synthesis Example 11]

### Synthesis of 4-(7-chloro-6-methoxy-2-(2-methoxyphenyl)benzo[b]thiophen-3-yl)-5-hydroxy-2,6-dimethylpyridazin-3-(2H)-one (compound No. 7-001)

Firstly, 33 mg of N-chlorosuccinimide was added to a mixture of 100 mg of 4-(6-methoxy-2-(2-methoxyphenyl)benzo[b]thiophen-3-yl)-5-hydroxy-2,6-dimethylpyridazin-3-(2H)-one and 3 mL of N,N-dimethylformamide at room temperature. After completion of the addition, the reaction mixture was stirred at 80°C for one hour. After completion of the stirring, 10 mL of 1 mol/L hydrochloric acid was added to the reaction mixture under ice cooling, and the resultant mixture was subjected to extraction with 20 mL of ethyl acetate. The resultant organic phase was washed with 5 mL of 1 mol/L hydrochloric acid, and then dehydrated and dried sequentially with saturated salt water and anhydrous sodium sulfate. The solvent was distilled off under reduced pressure. The resultant residue was purified by silica gel chromatography (with a gradient of ethyl acetate : n-hexane = 5 : 95 to 30 : 70), to thereby yield 75 mg of the target product as a white solid.
Melting point: 229-231°C
¹H NMR: δ 7.25-7.40 (m, 3H), 7.06 (d, J=8.5Hz, 1H), 6.90-7.00 (m, 2H), 6.45 (brs, 1H), 3.97 (s, 3H), 3.82 (s, 3H), 3.73 (s, 3H), 2.17 (s, 3H).

### [Synthesis Example 12]

### Synthesis of 5-hydroxy-4-(2-(4-(2-methoxyethoxy)phenyl)benzofuran-3-yl)-2,6-dimethylpyridazin-3-(2H)-one(compound No. 8-007)

### Step 1: synthesis of 4-(2-(4-(benzyloxy)phenyl)benzofuran-3-yl)-5-methoxy-2,6-dimethylpyridazin-3-(2H)-one

Firstly, 872 mg of p-benzyloxyphenylboronic acid, 1.6 g of potassium phosphate, and 294 mg of tetrakis(triphenylphosphine)palladium were added to a mixture of 890 mg of 4-(2-bromobenzofuran-3-yl)-5-methoxy-2,6-dimethylpyridazin-3-(2H)-one and 20 mL of toluene at room temperature. After completion of the addition, the reaction container was purged with nitrogen gas. After completion of the nitrogen gas purging, the reaction mixture was stirred in a nitrogen atmosphere at 110°C for three hours. After completion of the stirring, insoluble matter was filtered with celite, and the residue was washed with chloroform (10 mL × 2). The resultant filtrate was concentrated under reduced pressure. The resultant residue was purified by silica gel chromatography (with a gradient of ethyl acetate : n-hexane = 5 : 95 to 25 : 75), to thereby yield 1.3 g of the target product as a pale yellow oily product.

¹H NMR: δ 7.60-7.70 (m, 2H), 7.20-7.55 (m, 9H), 6.95-7.05 (m, 2H), 5.08 (s, 2H), 3.76 (s, 3H), 3.48 (s, 3H), 2.32 (s, 3H).

### Step 2: synthesis of 4-(2-(4-hydroxyphenyl)benzofuran-3-yl)-5-methoxy-2,6-dimethylpyridazin-3-(2H)-one(compound No. 8-006)

Firstly, 650 mg of 10% palladium carbon (available from N.E.CHEMCAT CORPORATION, PE type, 50% hydrous product) was suspended in 20 mL of tetrahydrofuran, and 1.3 g of 4-(2-(4-(benzyloxy)phenyl)benzofuran-3-yl)-5-methoxy-2,6-dimethylpyridazin-3-(2H)-one was added to the suspension at room temperature. After completion of the addition, the reaction container was purged with nitrogen gas. After completion of the nitrogen gas purging, the reaction container was purged with hydrogen gas. After completion of the hydrogen gas purging, the reaction mixture was stirred at room temperature for three hours. After completion of the stirring, insoluble matter was filtered with celite, and the residue was washed with chloroform (20 mL × 2). The resultant filtrate was concentrated under reduced pressure. Subsequently, 40 mL of diisopropyl ether was added to the resultant residue, and then the precipitated solid was recovered through filtration, to thereby yield 770 mg of the target product as a white solid.
Melting point: 251-253°C
¹H NMR: δ 7.10-7.55 (m, 6H), 6.55-6.60 (m, 2H), 3.82 (brs, 3H), 3.54 (s, 3H), 2.37 (brs, 3H).

### Step 3: synthesis of 5-methoxy-4-(2-(4-(2-methoxyethoxy)phenyl)benzofuran-3-yl)-2,6-dimethylpyridazin-3-(2H)-one

Firstly, 86 mg of potassium carbonate and 69 mg of 1-bromo-2-methoxyethane were added to a mixture of 150 mg of 4-(2-(4-hydroxyphenyl)benzofuran-3-yl)-5-methoxy-2,6-dimethylpyridazin-3-(2H)-one and 3 mL of acetonitrile at room temperature. After completion of the addition, the reaction mixture was stirred at 90°C for three hours. After completion of the stirring, 5 mL of water was added to the reaction mixture, and the resultant mixture was subj ected to extraction with 10 mL of ethyl acetate. The resultant organic phase was dehydrated and dried sequentially with saturated salt water and anhydrous sodium sulfate, and then the solvent was distilled off under reduced pressure. The resultant residue was purified by silica gel column chromatography (with a gradient of ethyl acetate : n-hexane = 5 : 95 to 30 : 70), to thereby yield 148 mg of the target product as a colorless oily product.

¹H NMR: δ 7.60-7.70 (m 2H), 7.50-7.55 (m, 1H), 7.15-7.35 (m, 3H), 6.90-7.00 (m, 2H), 4.10-4.20 (m, 2H), 3.70-3.80 (m, 5H), 3.48 (s, 3H), 3.46 (s, 3H), 2.32 (s, 3H).

### Step 4: synthesis of 5-hydroxy-4-(2-(4-(2-methoxyethoxy)phenyl)benzofuran-3-yl)-2,6-dimethylpyridazin-3-(2H)-one (compound No. 8-007)

Firstly, 1 mL of morpholine was added to a mixture of 148 mg of 5-methoxy-4-(2-(4-(2-methoxyethoxy)phenyl)benzofuran-3-yl)-2,6-dimethylpyridazin-3-(2H)-one and 1 mL of N-methyl-2-pyrrolidone at room temperature. After completion of the addition, the reaction container was purged with nitrogen gas. After completion of the nitrogen gas purging, the reaction mixture was stirred in a nitrogen atmosphere at 100°C for six hours. After completion of the stirring, 1 mol/L hydrochloric acid was added to the mixture under ice cooling so as to achieve a pH of 1, and the resultant mixture was subjected to extraction with 20 mL of ethyl acetate. The resultant organic phase was washed with 1 mol/L hydrochloric acid (5 mL × 2), and then dehydrated and dried sequentially with saturated salt water and anhydrous sodium sulfate. The solvent was distilled off under reduced pressure, and then the resultant residue was purified by silica gel chromatography (with a gradient of ethyl acetate : n-hexane = 20 : 80 to 75 : 25), to thereby yield 78 mg of the target product as a white solid.
Melting point: 214-216°C
¹H NMR: δ 7.50-7.65 (m, 3H), 7.15-7.35 (m, 3H), 6.85-6.95 (m, 2H), 5.96 (brs, 1H), 4.10-4.15 (m, 2H), 3.70-3.80 (m, 5H), 3.43 (s, 3H), 2.30 (s, 3H).

[Synthesis Example 13]

### Synthesis of 4-(5-fluoro-2-(4-methoxyphenyl)benzofuran-3-yl)-5-hydroxy-2,6-dimethylpyridazin-3(2H)-one (compound No. 9-020)

### Step 1: synthesis of 5-(5-fluoro-2-(4-methoxyphenyl)benzofuran-3-yl)-1,3-dimethyl-6-oxo-1,6-dihydropyridazin-4-yl=(n-butyrate)

Firstly, 86 mg of p-methoxyphenylboronic acid, 240 mg of potassium phosphate, and 44 mg of tetrakis(triphenylphosphine)palladium were added to a mixture of 160 mg of 5-(2-bromo-5-fluorobenzofuran-3-yl)-1,3-dimethyl-6-oxo-1,6-dihydropyridazin-4-yl=(n-butyrate) and 6 mL of toluene at room temperature. After completion of the addition, the reaction container was purged with nitrogen gas. After completion of the nitrogen gas purging, the reaction mixture was stirred in a nitrogen atmosphere at 110°C for three hours. After completion of the stirring, insoluble matter was filtered with celite, and the residue was washed with chloroform (10 mL × 2). The resultant filtrate was concentrated under reduced pressure, and then the residue was purified by silica gel chromatography (with a gradient of ethyl acetate : n-hexane = 5 : 95 to 20 : 80), to thereby yield 53 mg of the target product as a pale yellow oily product.

¹H NMR: δ 7.65-7.75 (m, 2H), 7.35-7.45 (m, 1H), 6.85-7.05 (m, 4H), 3.83 (s, 3H), 3.80 (s, 3H), 2.29 (s, 3H), 2.00-2.25 (m, 2H), 1.20-1.40 (m, 2H), 0.59 (t, J=7.5Hz, 3H).

### Step 2: synthesis of 4-(5-fluoro-2-(4-methoxyphenyl)benzofuran-3-yl)-5-hydroxy-2,6-dimethylpyridazin-3(2H)-one (compound No. 9-020)

Firstly, a solution of 50 mg of lithium hydroxide in 1 mL of water was added to a mixture of 53 mg of 5-(5-fluoro-2-(4-methoxyphenyl)benzofuran-3-yl)-1,3-dimethyl-6-oxo-1,6-dihydropyridazin-4-yl=(n-butyrate) and 2 mL of tetrahydrofuran at room temperature. After completion of the addition, the reaction mixture was stirred at room temperature for one hour. After completion of the stirring, the solvent contained in the reaction mixture was distilled off under reduced pressure. Subsequently, 35% by mass hydrochloric acid was added to the resultant residue so as to achieve a pH of 1, and then the precipitated solid was recovered through filtration, to thereby yield 39 mg of the target product as a white solid.
Melting point: 248-250°C
¹H NMR: δ 7.60-7.70 (m, 2H), 7.40-7.50 (m, 1H), 6.85-7.05 (m, 4H), 3.82 (s, 3H), 3.77 (s, 3H), 2.33 (s, 3H).

The compound of the present invention can be produced according to the aforementioned production methods and Examples. Pyridazinone compounds produced in the same manner as in Synthesis Examples 1 to 13, which are included in the compound of the present invention, will be shown in Tables 3 to 13, and exemplary production intermediates of the pyridazinone compounds will be shown in Table 14. The pyridazinone compounds included in the present invention and the intermediates of the compounds are not limited to those shown below.

In the following Tables, Me denotes methyl; n-Pr and Pr-n, normal propyl; i-Pr and Pr-i, isopropyl; c-Pr and Pr-c, cyclopropyl; n-Bu and Bu-n, normal butyl; i-Bu and Bu-i, isobutyl; t-Bu and Bu-t, tertiary butyl; n-Pen and Pen-n, normal pentyl; c-Pen and Pen-c, cyclopentyl; n-Hex and Hex-n, normal hexyl; c-Hex and Hex-c, cyclohexyl; Ph, phenyl; and Bn, benzyl. In the following Tables, the symbol "=" denotes a double bond, and the symbol "=" denotes a triple bond.

In the following Tables, the expression "m.p." denotes a melting point. The expression "^{∗}1" in the column of melting point refers to the case where the corresponding compound is in an oily form or a resin form.

D-1, D-2, D-3, D-4, D-5, D-6, D-7, D-8, D-9, D-10, D-11, D-12, D-13, D-14, D-15, D-16, D-17, D-18, D-19a, D-20a, D-21a, D-22a, D-23, D-24a, D-25, D-26, D-27, and D-28 in the Tables correspond to the following structures.

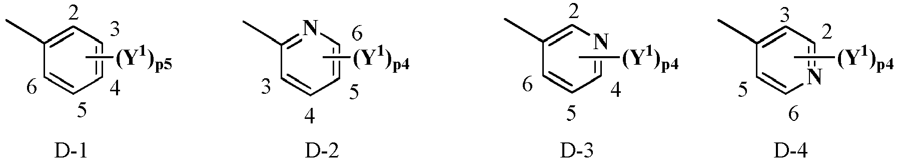

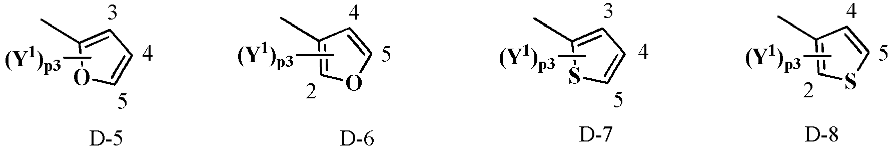

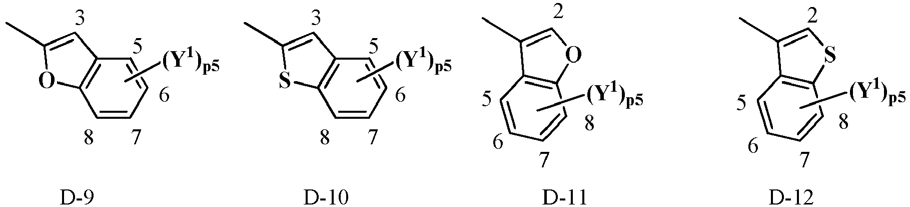

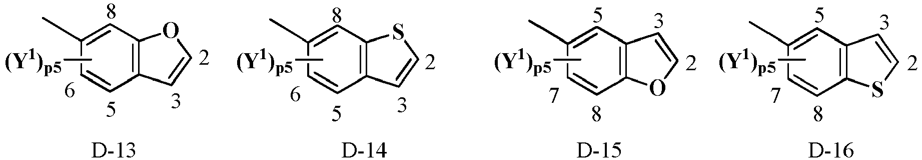

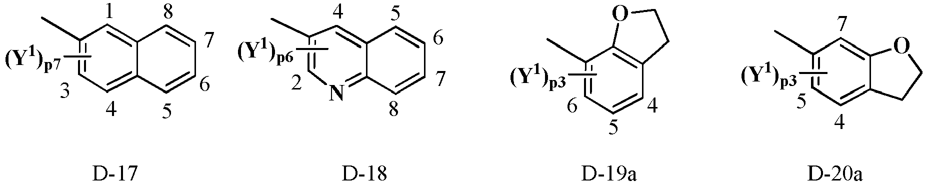

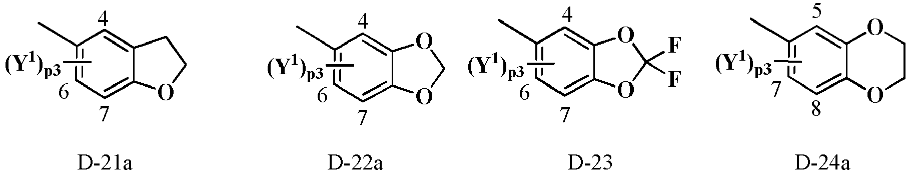

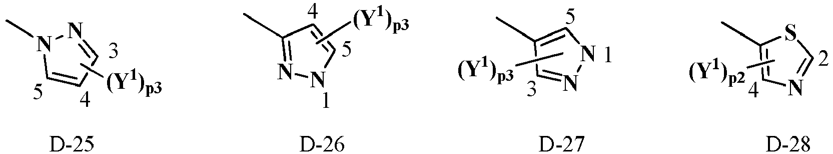

The expression "(Y¹)" in the Tables refers to (Y¹)ₚ₇, (Y¹)ₚ₆, (Y¹)ₚ₅, (Y¹)_{P4}, (Y¹)_{P3}, or (Y¹)ₚ₇ corresponding to each of the aforementioned structures of D-1 to D-28 specified in the column of R³. The substitution position number corresponds to the numbered position in each of the aforementioned structural formulae. The expression "-" in the column of "(Y¹)" refers to no substitution.

U-1a to U-6a, Q-1, Q-2a to Q-4a, Q-17a, Q-17b, and T-1-1 to T-3 in the Tables correspond to the following structures.

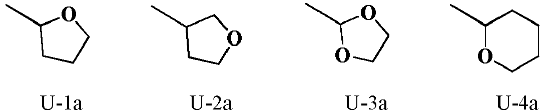

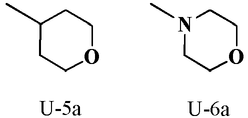

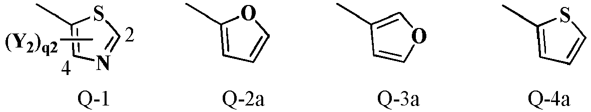

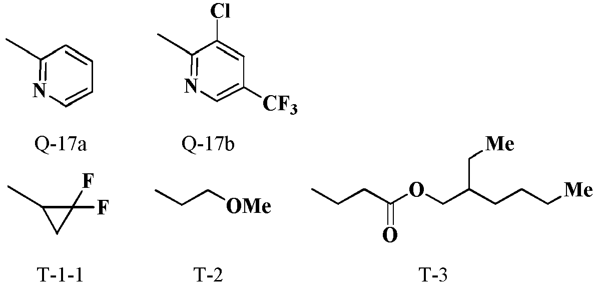

**Table 3**

| 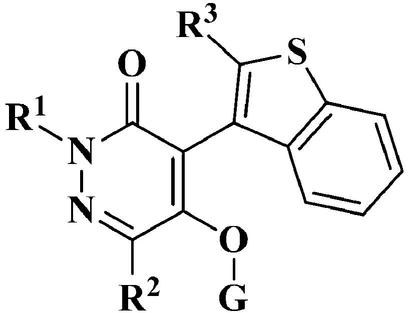 | | | | | | |
|---|---|---|---|---|---|---|
| No. | R¹ | R² | R³ | (Y¹) | G | mp (°C) |
| 1-001 | Me | H | D-1 | 4-F | H | 122-129 |
| 1-002 | Me | H | D-1 | 4-F | Me | 147-149 |
| 1-003 | Me | Me | D-1 | 2-Et | H | 237-239 |
| 1-004 | Me | Me | D-1 | 2-Et | Me | 123-125 |
| 1-005 | Me | Me | D-1 | 4-Bu-n | H | 146-148 |
| 1-006 | Me | Me | D-1 | 4-Bu-n | C(O)Pr-n | ∗1 |
| 1-007 | Me | Me | D-1 | 2-CF₃ | H | 235-236 |
| 1-008 | Me | Me | D-1 | 2-CF₃ | Me | 124-126 |
| 1-009 | Me | Me | D-1 | 2-OH | H | 269-271 |
| 1-010 | Me | Me | D-1 | 2-OH | Me | 187-189 |
| 1-011 | Me | Me | D-1 | 2-OEt | H | 196-198 |
| 1-012 | Me | Me | D-1 | 2-OEt | C(O)Pr-n | 143-145 |
| 1-013 | Me | Me | D-1 | 4-OEt | H | 154-156 |
| 1-014 | Me | Me | D-1 | 4-OEt | C(O)Pr-n | 129-131 |
| 1-015 | Me | Me | D-1 | 2-OPr-n | H | 188-190 |
| 1-016 | Me | Me | D-1 | 2-OPr-n | Me | ∗1 |
| 1-017 | Me | Me | D-1 | 2-OPr-i | H | 197-198 |
| 1-018 | Me | Me | D-1 | 2-OPr-i | Me | 155-156 |
| 1-019 | Me | Me | D-1 | 4-OPr-n | H | 80-82 |
| 1-020 | Me | Me | D-1 | 4-OPr-i | H | 137-139 |
| 1-021 | Me | Me | D-1 | 4-OPr-i | C(O)Pr-n | ∗1 |
| 1-022 | Me | Me | D-1 | 2-OBu-n | H | 179-181 |
| 1-023 | Me | Me | D-1 | 2-OBu-n | C(O)Pr-n | ∗1 |
| 1-024 | Me | Me | D-1 | 4-OBu-n | H | 114-116 |
| 1-025 | Me | Me | D-1 | 4-OBu-n | C(O)Pr-n | ∗1 |
| 1-026 | Me | Me | D-1 | 4-OPen-n | H | 100-102 |
| 1-027 | Me | Me | D-1 | 4-OPen-n | C(O)Pr-n | ∗1 |
| 1-028 | Me | Me | D-1 | 4-OHex-n | H | 81-83 |
| 1-029 | Me | Me | D-1 | 4-OHex-n | C(O)Pr-n | ∗1 |
| 1-030 | Me | Me | D-1 | 2-OCF₃ | H | 220-221 |
| 1-031 | Me | Me | D-1 | 4-OCF₃ | H | 112-114 |
| 1-032 | Me | Me | D-1 | 4-OCF₃ | C(O)Pr-n | ∗1 |
| 1-033 | Me | Me | D-1 | 2-OPh | H | 242-244 |
| 1-034 | Me | Me | D-1 | 2-SMe | H | 216-218 |
| 1-035 | Me | Me | D-1 | 2-SMe | C(O)Pr-n | ∗1 |
| 1-036 | Me | Me | D-1 | 4-SMe | H | 110-112 |
| 1-037 | Me | Me | D-1 | 4-SMe | C(O)Pr-n | ∗1 |
| 1-038 | Me | Me | D-1 | 2-S(O)₂Et | H | 268-270 |
| 1-039 | Me | Me | D-1 | 4-NMe₂ | H | 145-146 |
| 1-040 | Me | Me | D-1 | 4-NMe₂ | C(O)Pr-n | 162-164 |
| 1-041 | Me | Me | D-1 | 4-C(O)Me | H | 239-241 |
| 1-042 | Me | Me | D-1 | 4-C(O)Me | C(O)Pr-n | ∗1 |
| 1-043 | Me | Me | D-1 | 4-C(=N-OMe)Me | H | 161-163 |
| 1-044 | Me | Me | D-1 | 4-C(=N-OMe)Me | C(0)Pr-n | ∗1 |
| 1-045 | Me | Me | D-1 | 4-CN | H | 240-242 |
| 1-046 | Me | Me | D-1 | 3,4-F₂ | H | 239-241 |
| 1-047 | Me | Me | D-1 | 3,4-F₂ | C(O)Pr-n | ∗1 |
| 1-048 | Me | Me | D-1 | 2-F-3-OMe | H | 162-163 |
| 1-049 | Me | Me | D-1 | 3-F-4-OMe | H | 233-234 |
| 1-050 | Me | Me | D-1 | 3-F-4-OMe | Me | 127-129 |
| 1-051 | Me | Me | D-1 | 3-Me-4-F | H | 211-213 |
| 1-052 | Me | Me | D-1 | 3-Me-4-F | C(O)Pr-n | ∗1 |
| 1-053 | Me | Me | D-1 | 2-F-5-OMe | H | 226-228 |
| 1-054 | Me | Me | D-1 | 2-OMe-3-F | H | 200-201 |
| 1-055 | Me | Me | D-1 | 2-OMe-4-F | H | 229-231 |
| 1-056 | Me | Me | D-1 | 2-OMe-4-F | C(O)Pr-n | 163-165 |
| 1-057 | Me | Me | D-1 | 2-OMe-4-F | Me | 193-194 |
| 1-058 | Me | Me | D-1 | 2-OMe-5-F | H | 228-230 |
| 1-059 | Me | Me | D-1 | 2-OMe-5-F | C(O)Pr-n | ∗1 |
| 1-060 | Me | Me | D-1 | 2-OMe-4-Cl | H | 221-223 |
| 1-061 | Me | Me | D-1 | 2-OEt-4-F | H | 202-203 |
| 1-062 | Me | Me | D-1 | 2-OEt-4-F | C(O)Pr-n | 144-146 |
| 1-063 | Me | Me | D-1 | 2-SMe-4-F | H | 226-228 |
| 1-064 | Me | Me | D-1 | 2-SMe-4-F | C(0)Pr-n | ∗1 |
| 1-065 | Me | Me | D-1 | 3, 4-Me₂ | H | 225-227 |
| 1-066 | Me | Me | D-1 | 3, 4-Me₂ | C(O)Pr-n | 124-126 |
| 1-067 | Me | Me | D-1 | 2-OMe-3-Me | H | 197-199 |
| 1-068 | Me | Me | D-1 | 3-Me-4-OMe | H | 149-151 |
| 1-069 | Me | Me | D-1 | 3-Me-4-OMe | C(O)Pr-n | 100-102 |
| 1-070 | Me | Me | D-1 | 2,4-(OMe)₂ | H | 131-133 |
| 1-071 | Me | Me | D-1 | 2,4-(OMe)₂ | C(O)Pr-n | ∗1 |
| 1-072 | Me | Me | D-1 | 3,4-(OMe)₂ | H | 205-207 |
| 1-073 | Me | Me | D-1 | 3,4-(OMe)₂ | C(O)Pr-n | 127-130 |
| 1-074 | Me | Me | D-1 | 3, 5-Me₂-4-OMe | H | 126-128 |
| 1-075 | Me | Me | D-1 | 3, 5-Me₂-4-OMe | C(O)Pr-n | ∗1 |
| 1-076 | Me | Me | D-3 | 4-OEt | H | 186-188 |
| 1-077 | Me | Me | D-3 | 4-OEt | C(O)Pr-n | ∗1 |
| 1-078 | Me | Me | D-3 | 4-CF₃ | H | 129-132 |
| 1-079 | Me | Me | D-3 | 2,4-(OMe)₂ | H | 206-208 |
| 1-080 | Me | Me | D-3 | 2,4-(OMe)₂ | C(O)Pr-n | ∗1 |
| 1-081 | Me | Me | D-6 | - | H | 233-235 |
| 1-082 | Me | Me | D-6 | - | C(O)Pr-n | 140-142 |
| 1-083 | Me | Me | D-8 | - | H | 240-242 |
| 1-084 | Me | Me | D-8 | - | C(O)Pr-n | 133-135 |
| 1-085 | Me | Me | D-20a | - | H | 231-233 |
| 1-086 | Me | Me | D-20a | - | Me | 173-175 |
| 1-087 | Me | Me | D-22a | - | H | 252-254 |
| 1-088 | Me | Me | D-22a | - | C(O)Pr-n | 134-136 |
| 1-089 | Me | Me | D-24a | - | H | 210-212 |
| 1-090 | Me | Me | D-24a | - | C(O)Pr-n | 61-63 |
| 1-091 | Me | Me | D-24a | - | C(O)0Me | ∗1 |
| 1-092 | Me | Me | D-24a | - | C(O)SBu-t | ∗1 |
| 1-093 | Me | Me | D-24a | - | C(O)(Ph-2-Me) | ∗1 |
| 1-094 | Me | Me | D-24a | - | S(O)₂Me | ∗1 |
| 1-095 | Me | Me | D-24a | 7-C1 | H | 230-233 |
| 1-096 | Me | Me | D-24a | 7-C1 | C(O)Pr-n | 96-98 |
| 1-097 | Me | Me | D-25 | 4-Cl | H | 232-234 |
| 1-098 | Me | Me | D-25 | 4-Cl | C(O)Pr-n | ∗1 |
| 1-099 | Me | Me | D-23 | - | H | 129-131 |
| 1-100 | Me | Me | D-23 | - | C(O)Pr-n | ∗1 |
| 1-101 | Me | Me | D-21a | - | H | 159-161 |
| 1-102 | Me | Me | D-21a | - | C(O)Pr-n | ∗1 |
| 1-103 | Me | Me | D-19a | - | H | 224-225 |
| 1-104 | Me | Me | D-19a | - | Me | ∗1 |
| 1-105 | Me | Me | D-26 | 1-Me-4-OMe | H | 186-188 |
| 1-106 | Me | Me | D-26 | 1-Me-4-OMe | Me | 214-216 |
| 1-107 | Me | Me | D-26 | 1-H-4-OMe | Me | 181-183 |
| 1-108 | Me | Me | D-18 | - | H | 279-281 |
| 1-109 | Me | Me | D-18 | - | Me | ∗1 |
| 1-110 | Me | Me | D-1 | 2-OCH₂CH=CH₂ | H | 193-195 |
| 1-111 | Me | Me | D-1 | 2-OCH₂CH=CH₂ | Me | ∗1 |
| 1-112 | Me | Me | D-1 | 2-OCH₂C≡CH | H | 189-190 |
| 1-113 | Me | Me | D-1 | 2-OCH₂Pr-c | H | 194-195 |
| 1-114 | Me | Me | D-1 | 2-OCH₂Pr-c | Me | ∗1 |
| 1-115 | Me | Me | D-1 | 2-OCH₂OMe | C(O)Pr-n | 70-72 |
| 1-116 | Me | Me | D-1 | 2-OCH₂CHF₂ | H | 203-205 |
| 1-117 | Me | Me | D-1 | 2-OCH₂CHF₂ | Me | 178-179 |
| 1-118 | Me | Me | D-1 | 2-OCH₂CF₃ | H | 205-206 |
| 1-119 | Me | Me | D-1 | 2-OCH₂CF₃ | Me | 146-148 |
| 1-120 | Me | Me | D-1 | 2-OCH₂[T-1-1] | H | 198-199 |
| 1-121 | Me | Me | D-1 | 2-OCH₂[T-1-1] | Me | ∗1 |
| 1-122 | Me | Me | D-1 | 2-OCH₂[(Q-1)-2-Cl] | Me | ∗1 |
| 1-123 | Me | Me | D-1 | 3-CN | H | 233-235 |
| 1-124 | Me | Me | D-1 | 3-CN | C(O)Pr-n | 147-149 |
| 1-125 | Me | Me | D-1 | 4-Pr-i | H | 135-137 |
| 1-126 | Me | Me | D-1 | 4-Pr-i | C(O)Pr-n | ∗1 |
| 1-127 | Me | Me | D-1 | 4-CH=CH₂ | H | 214-216 |
| 1-128 | Me | Me | D-1 | 4-CH=CH₂ | C(O)Pr-n | ∗1 |
| 1-129 | Me | Me | D-1 | 4-CF₃ | H | 219-221 |
| 1-130 | Me | Me | D-1 | 4-CF₃ | C(O)Pr-n | 109-111 |
| 1-131 | Me | Me | D-1 | 4-CH₂OMe | H | ∗1 |
| 1-132 | Me | Me | D-1 | 4-CH₂OMe | Me | 140-142 |
| 1-133 | Me | Me | D-1 | 4-OCH₂Ph | H | ∗1 |
| 1-134 | Me | Me | D-1 | 4-OCH₂Ph | Me | 125-127 |
| 1-135 | Me | Me | D-1 | 4-OCH₂Ph | CH₂OMe | ∗1 |
| 1-136 | Me | Me | D-1 | 4-OCH₂C=CH | H | ∗1 |
| 1-137 | Me | Me | D-1 | 4-OCH₂CN | H | 125-127 |
| 1-138 | Me | Me | D-1 | 4-OCH₂[T-1-1] | H | ∗1 |
| 1-139 | Me | Me | D-1 | 4-OCH₂CH=CH₂ | H | ∗1 |
| 1-140 | Me | Me | D-1 | 4-OCH₂CF₃ | H | 183-185 |
| 1-141 | Me | Me | D-1 | 4-OCH₂CH₂SMe | H | 114-115 |
| 1-142 | Me | Me | D-1 | 4-OCH₂Pr-c | H | ∗1 |
| 1-143 | Me | Me | D-1 | 4-OCH₂C(O)Me | H | ∗1 |
| 1-144 | Me | Me | D-1 | 4-OCH₂[(Q-1)-2-Cl] | H | ∗1 |
| 1-145 | Me | Me | D-1 | 4-OCH₂C(O)NHCH₂CF₃ | H | ∗1 |
| 1-146 | Me | Me | D-1 | 4-OS(O)₂Me | H | ∗1 |
| 1-147 | Me | Me | D-1 | 4-OS(O)₂CF₃ | H | ∗1 |
| 1-148 | Me | Me | D-1 | 4-OCH₂CH₂OMe | H | ∗1 |
| 1-149 | Me | Me | D-1 | 4-OPh | H | 112-114 |
| 1-150 | Me | Me | D-1 | 4-OPh | C(O)Pr-n | ∗1 |
| 1-151 | Me | Me | D-1 | 4-NO₂ | H | 143-145 |
| 1-152 | Me | Me | D-1 | 4-NO₂ | C(O)Pr-n | 122-125 |
| 1-153 | Me | Me | D-1 | 4-SiMe₃ | H | 126-127 |
| 1-154 | Me | Me | D-1 | 4-SiMe₃ | C(O)Pr-n | ∗1 |
| 1-155 | Me | Me | D-1 | 2-OMe-4-CF₃ | H | 227-229 |
| 1-156 | Me | Me | D-1 | 2-OMe-4-CF₃ | C(O)Pr-n | ∗1 |
| 1-157 | Me | Me | D-1 | 2-OMe-5-Br | Me | ∗1 |
| 1-158 | Me | Me | D-1 | 2,6-(OMe)₂ | H | 145-147 |
| 1-159 | Me | Me | D-1 | 2-Cl-6-OMe | H | 182-184 |
| 1-160 | Me | OMe | D-1 | 3-OCF₃ | H | 217-219 |
| 1-161 | Me | OMe | D-1 | 3-OCF₃ | C(O)Pr-n | ∗1 |
| 1-162 | Me | OMe | D-1 | 4-OCF₃ | H | 209-211 |
| 1-163 | Me | OMe | D-1 | 4-OCF₃ | C(O)Pr-n | ∗1 |
| 1-164 | Me | OMe | D-1 | 4-OPh | H | 272-274 |
| 1-165 | Me | OMe | D-1 | 4-OPh | C(O)Pr-n | ∗1 |
| 1-166 | Me | OMe | D-1 | 4-Ph | H | 276-277 |
| 1-167 | Me | OMe | D-1 | 4-Ph | C(O)Pr-n | 144-146 |
| 1-168 | Me | OMe | D-1 | 4-NO₂ | H | 190-192 |
| 1-169 | Me | OMe | D-1 | 4-NO₂ | C(O)Pr-n | 69-71 |
| 1-170 | Me | OMe | D-1 | 4-CN | H | 248-250 |
| 1-171 | Me | OMe | D-1 | 4-CN | C(O)Pr-n | 167-169 |
| 1-172 | Me | OMe | D-1 | 3,4-F₂ | H | 216-218 |
| 1-173 | Me | OMe | D-1 | 3,4-F₂ | C(O)Pr-n | 121-123 |
| 1-174 | Me | OMe | D-1 | 3-Me-4-F | H | 222-224 |
| 1-175 | Me | OMe | D-1 | 3-Me-4-F | C(O)Pr-n | ∗1 |
| 1-176 | Me | OMe | D-1 | 2-OMe-4-F | H | 222-224 |
| 1-177 | Me | OMe | D-1 | 2-OMe-4-F | C(O)Pr-n | 75-77 |
| 1-178 | Me | OMe | D-1 | 2-OMe-5-Cl | H | 140-143 |
| 1-179 | Me | OMe | D-1 | 2-OMe-5-Cl | C(O)Pr-n | 122-124 |
| 1-180 | Me | OMe | D-1 | 2,4-(OMe)₂ | H | 135-137 |
| 1-181 | Me | OMe | D-1 | 2,4-(OMe)₂ | C(O)Pr-n | ∗1 |
| 1-182 | Me | OMe | D-1 | 3,4-(OMe)₂ | H | 137-139 |
| 1-183 | Me | OMe | D-1 | 3,4-(OMe)₂ | C(O)Pr-n | 146-148 |
| 1-184 | Me | OMe | D-1 | 3,5-(OMe)₂ | H | 219-221 |
| 1-185 | Me | OMe | D-1 | 3,5-(OMe)₂ | C(O)Pr-n | ∗1 |
| 1-186 | Me | OMe | D-1 | 3, 4, 5-F₃ | H | 197-199 |
| 1-187 | Me | OMe | D-1 | 3, 4, 5-F₃ | C(O)Pr-n | 122-124 |
| 1-188 | Me | OMe | D-1 | 2, 4, 6-Me₃ | H | ∗1 |
| 1-189 | Me | OMe | D-1 | 2, 4, 6-Me₃ | C(O)Pr-n | ∗1 |
| 1-190 | Me | OMe | D-6 | - | H | 145-147 |
| 1-191 | Me | OMe | D-6 | - | C(O)Pr-n | ∗1 |
| 1-192 | Me | OMe | D-8 | - | H | 181-183 |
| 1-193 | Me | OMe | D-8 | - | C(O)Pr-n | 146-148 |
| 1-194 | Me | OMe | D-9 | - | H | 160-162 |
| 1-195 | Me | OMe | D-9 | - | C(O)Pr-n | ∗1 |
| 1-196 | Me | OMe | D-10 | - | H | 255-257 |
| 1-197 | Me | OMe | D-10 | - | C(O)Pr-n | ∗1 |
| 1-198 | Me | OMe | D-24a | - | H | 230-232 |
| 1-199 | Me | OMe | D-24a | - | C(O)Pr-n | 89-91 |
| 1-200 | Me | Me | D-1 | 4-OH | H | ∗1 |
| 1-201 | Me | Me | D-1 | 4-OCHF₂ | H | 169-171 |
| 1-202 | Me | Me | D-1 | 4-OCHF₂ | CH₂OCH₂CH₂OMe | ∗1 |
| 1-203 | Me | Me | D-1 | 4-OCH₂CHF₂ | H | ∗1 |
| 1-204 | Me | Me | D-1 | 4-OCH₂CH₂Br | H | ∗1 |
| 1-205 | Me | Me | D-1 | 4-OCH₂CH₂Br | CH₂OCH₂CH₂OMe | ∗1 |
| 1-206 | Me | Me | D-1 | 4-OCH₂CH₂CF₃ | H | ∗1 |
| 1-207 | Me | Me | D-1 | 4-OCH₂CH₂CH=CH₂ | H | 118-120 |
| 1-208 | Me | Me | D-1 | 4-OCH₂C(Me)=CH₂ | H | ∗1 |
| 1-209 | Me | Me | D-1 | 4-OCH₂C(Me)=CH₂ | CH₂OCH₂CH₂OMe | ∗1 |
| 1-210 | Me | Me | D-1 | 4-OCH₂CH₂C(Me) =CH₂ | H | 84-86 |
| 1-211 | Me | Me | D-1 | 4-OCH₂CH=CHCH₃ | H | ∗1 |
| 1-212 | Me | Me | D-1 | 4-OCH₂CH=CHCH₃ | CH₂OCH₂CH₂OMe | ∗1 |
| 1-213 | Me | Me | D-1 | 4-OCH (Me)CH₂CH=CH₂ | H | 177-179 |
| 1-214 | Me | Me | D-1 | 4-OCH (Me)CH₂CH=CH₂ | CH₂OCH₂CH₂OMe | ∗1 |
| 1-215 | Me | Me | D-1 | 4-OCH₂C (Br) =CH₂ | H | 146-149 |
| 1-216 | Me | Me | D-1 | 4-OCH₂C(Br)=CH₂ | CH₂OCH₂CH₂OMe | ∗1 |
| 1-217 | Me | Me | D-1 | 4-OCH(Me)C≡CH | H | ∗1 |
| 1-218 | Me | Me | D-1 | 4-OCH(Me)C≡CH | CH₂OCH₂CH₂OMe | ∗1 |
| 1-219 | Me | Me | D-1 | 4-OCH₂C≡CMe | H | 167-168 |
| 1-220 | Me | Me | D-1 | 4-OCH₂C≡CMe | CH₂OCH₂CH₂OMe | *1 |
| 1-221 | Me | Me | D-1 | 4-OCH₂CH₂SMe | CH₂OCH₂CH₂OMe | *1 |
| 1-222 | Me | Me | D-1 | 4-OCH₂CH₂CH₂SMe | H | 124-126 |
| 1-223 | Me | Me | D-1 | 4-OCH₂CH₂[U-3a] | H | ∗1 |
| 1-224 | Me | Me | D-1 | 4-OCH₂CH₂[U-3a] | CH₂OCH₂CH₂OMe | ∗1 |
| 1-225 | Me | Me | D-1 | 4-OCH₂CH₂ON=C (Me)₂ | CH₂OCH₂CH₂OMe | ∗1 |
| 1-226 | Me | Me | D-1 | 4-OCH₂CH₂OPr-i | H | 129-131 |
| 1-227 | Me | Me | D-1 | 4-OCH₂CH₂OCH₂CH₂Cl | H | 125-127 |
| 1-228 | Me | Me | D-1 | 4-OCH (Me) CH₂OMe | H | 158-163 |
| 1-229 | Me | Me | D-1 | 4-OCH(Me)CH₂OMe | CH₂OCH₂CH₂OMe | ∗1 |
| 1-230 | Me | Me | D-1 | 4-OCH₂CH(Et)OMe | H | 167-170 |
| 1-231 | Me | Me | D-1 | 4-OCH₂CH(Et)OMe | CH₂OCH₂CH₂OMe | ∗1 |
| 1-232 | Me | Me | D-1 | 4-OCH₂C(O)OMe | H | 139-141 |
| 1-233 | Me | Me | D-1 | 4-OCH₂C(O)OMe | CH₂OCH₂CH₂OMe | ∗1 |
| 1-234 | Me | Me | D-1 | 4-OCH(Me)C(O)OMe | H | 229-231 |
| 1-235 | Me | Me | D-1 | 4-OCH(Me)C(O)OMe | CH₂OCH₂CH₂OMe | ∗1 |
| 1-236 | Me | Me | D-1 | 4-OCH(Me)CN | H | ∗1 |
| 1-237 | Me | Me | D-1 | 4-OCH(Me)CN | CH₂OCH₂CH₂OMe | ∗1 |
| 1-238 | Me | Me | D-1 | 4-CH₂OH | H | 260-261 |
| 1-239 | Me | Me | D-1 | 4-CH₂OH | Me | 194-195 |
| 1-240 | Me | Me | D-1 | 4-CH₂Cl | Me | 167-168 |
| 1-241 | Me | Me | D-1 | 4-CH₂OCH₂CH=CH₂ | H | ∗1 |
| 1-242 | Me | Me | D-1 | 4-CH₂SH | Me | ∗1 |
| 1-243 | Me | Me | D-1 | 4-CH₂SPr-i | H | 184-185 |
| 1-244 | Me | Me | D-1 | 4-CH₂SPr-i | Me | ∗1 |
| 1-245 | Me | Me | D-1 | 4-CH₂SCH₂CH=CH₂ | H | ∗1 |
| 1-246 | Me | Me | D-1 | 4-CH₂SCH₂CH=CH₂ | Me | ∗1 |
| 1-247 | Me | Me | D-1 | 4-CH₂SCH₂C≡CH | H | ∗1 |
| 1-248 | Me | Me | D-1 | 4-CH₂SCH₂C≡CH | Me | ∗1 |
| 1-249 | Me | Me | D-1 | 4-CH₂SCH₂CF₃ | H | 129-131 |
| 1-250 | Me | Me | D-1 | 4-CH₂SCH₂CF₃ | Me | ∗1 |
| 1-251 | Me | Me | D-1 | 4-CH₂SCH₂CH₂OMe | H | ∗1 |
| 1-252 | Me | Me | D-1 | 4-CH₂SCH₂CH₂OMe | Me | ∗1 |
| 1-253 | Me | Me | D-1 | 4-CH₂SCH₂CH₂SMe | H | ∗1 |
| 1-254 | Me | Me | D-1 | 4-CH₂SCH₂CH₂SMe | Me | ∗1 |
| 1-255 | Me | Me | D-1 | 4-CH₂OCH₂CH₂OMe | H | ∗1 |
| 1-256 | Me | Me | D-1 | 4-CH₂OCH₂CH₂OMe | Me | ∗1 |

**Table 4**

| 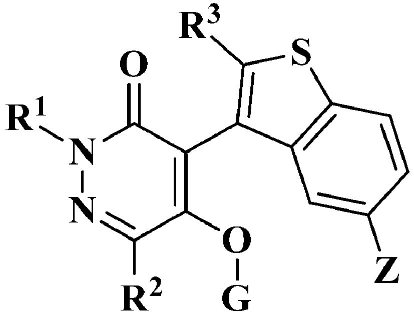 | | | | | | | |
|---|---|---|---|---|---|---|---|
| No. | R¹ | R² | R³ | (Y¹) | G | Z | mp(°C) |
| 2-001 | Me | Me | D-1 | 4-F | H | F | 248-250 |
| 2-002 | Me | Me | D-1 | 4-F | C(O)Pr-n | F | 147-149 |
| 2-003 | Me | Me | D-1 | 4-F | C(O) CH₂CH₂OMe | F | ∗1 |
| 2-004 | Me | Me | D-1 | 4-F | C(O)OEt | F | ∗1 |
| 2-005 | Me | Me | D-1 | 4-F | C(O)[Ph-2-Cl] | F | ∗1 |
| 2-006 | Me | Me | D-1 | 4-F | CH₂C≡CH | F | 173-175 |
| 2-007 | Me | Me | D-1 | 4-F | C(O)[U-6a] | F | 265-267 |
| 2-008 | Me | Me | D-1 | 4-F | CH₂C(O)Ph | F | 197-198 |
| 2-009 | Me | Me | D-1 | 4-F | S(O)₂NMe₂ | F | 177-179 |
| 2-010 | Me | Me | D-1 | 4-Cl | H | F | 258-259 |
| 2-011 | Me | Me | D-1 | 4-Cl | C(O)Pr-n | F | 163-165 |
| 2-012 | Me | Me | D-1 | 4-Me | H | F | 229-231 |
| 2-013 | Me | Me | D-1 | 4-Me | C(O)Pr-n | F | 64-66 |
| 2-014 | Me | Me | D-1 | 4-Pr-i | H | F | 233-235 |
| 2-015 | Me | Me | D-1 | 4-Pr-i | CH₂OCH₂CH₂OMe | F | 112-115 |
| 2-016 | Me | Me | D-1 | 4-Et | C(O)Pr-n | F | ∗1 |
| 2-017 | Me | Me | D-1 | 4-Bu-tert | C(O)Pr-n | F | ∗1 |
| 2-018 | Me | Me | D-1 | 4-CH=CH₂ | H | F | 216-218 |
| 2-019 | Me | Me | D-1 | 4-CH=CH₂ | C(O)Pr-n | F | ∗1 |
| 2-020 | Me | Me | D-1 | 4-CF₃ | H | F | 249-251 |
| 2-021 | Me | Me | D-1 | 4-CF₃ | C(O)Pr-n | F | 154-156 |
| 2-022 | Me | Me | D-1 | 4-CH₂OMe | H | F | 99-101 |
| 2-023 | Me | Me | D-1 | 4-CH₂OMe | C(O)Pr-n | F | ∗1 |
| 2-024 | Me | Me | D-1 | 2-OMe | H | F | 226-228 |
| 2-025 | Me | Me | D-1 | 2-OMe | C(O)Pr-n | F | ∗1 |
| 2-026 | Me | Me | D-1 | 4-OMe | H | F | 197-199 |
| 2-027 | Me | Me | D-1 | 4-OMe | C(O)Pr-n | F | ∗1 |
| 2-028 | Me | Me | D-1 | 2-OEt | H | F | 195-197 |
| 2-029 | Me | Me | D-1 | 2-OEt | C(O)Pr-n | F | ∗1 |
| 2-030 | Me | Me | D-1 | 4-OEt | H | F | 119-121 |
| 2-031 | Me | Me | D-1 | 4-OEt | C(O)Pr-n | F | 153-155 |
| 2-032 | Me | Me | D-1 | 4-OPr-n | H | F | 197-199 |
| 2-033 | Me | Me | D-1 | 4-OPr-n | C(O)Pr-n | F | ∗1 |
| 2-034 | Me | Me | D-1 | 4-OPr-i | H | F | 131-133 |
| 2-035 | Me | Me | D-1 | 4-OPr-i | C(O)Pr-n | F | ∗1 |
| 2-036 | Me | Me | D-1 | 4-OBu-n | H | F | 157-159 |
| 2-037 | Me | Me | D-1 | 4-OBu-n | C(O)Pr-n | F | ∗1 |
| 2-038 | Me | Me | D-1 | 4-OPen-n | C(O)Pr-n | F | ∗1 |
| 2-039 | Me | Me | D-1 | 4-OPen-c | H | F | ∗1 |
| 2-040 | Me | Me | D-1 | 4-OPen-c | Me | F | 200-201 |
| 2-041 | Me | Me | D-1 | 4-OHex-n | C(O)Pr-n | F | ∗1 |
| 2-042 | Me | Me | D-1 | 4-OCHF₂ | H | F | 164-165 |
| 2-043 | Me | Me | D-1 | 2-OCF₃ | H | F | 232-234 |
| 2-044 | Me | Me | D-1 | 2-OCF₃ | C(O)Pr-n | F | ∗1 |
| 2-045 | Me | Me | D-1 | 4-OCF₃ | H | F | 179-181 |
| 2-046 | Me | Me | D-1 | 4-OCF₃ | C(O)Pr-n | F | 133-135 |
| 2-047 | Me | Me | D-1 | 4-OCH₂CH₂F | H | F | ∗1 |
| 2-048 | Me | Me | D-1 | 4-OCH₂CH₂F | Me | F | 205-206 |
| 2-049 | Me | Me | D-1 | 4-OCH₂CF₃ | H | F | 200-202 |
| 2-050 | Me | Me | D-1 | 4-OCH₂CF₃ | Me | F | 177-178 |
| 2-051 | Me | Me | D-1 | 4-OCH₂CH=CH₂ | H | F | ∗1 |
| 2-052 | Me | Me | D-1 | 4-OCH₂CH=CH₂ | Me | F | 134-136 |
| 2-053 | Me | Me | D-1 | 4-OCH₂C≡CH | H | F | ∗1 |
| 2-054 | Me | Me | D-1 | 4-OCH₂C≡CH | CH₂C≡CH | F | ∗1 |
| 2-055 | Me | Me | D-1 | 4-OCH₂C≡CH | CH₂OCH₂CH₂OMe | F | ∗1 |
| 2-056 | Me | Me | D-1 | 4-OCH₂CH₂OMe | H | F | ∗1 |
| 2-057 | Me | Me | D-1 | 4-OCH₂CH₂OMe | Me | F | ∗1 |
| 2-058 | Me | Me | D-1 | 4-OCH₂CH₂SMe | H | F | ∗1 |
| 2-059 | Me | Me | D-1 | 4-OCH₂CH₂SMe | CH₂OCH₂CH₂OMe | F | ∗1 |
| 2-060 | Me | Me | D-1 | 4-OCH₂Pr-c | H | F | ∗1 |
| 2-061 | Me | Me | D-1 | 4-OCH₂Pr-c | Me | F | 169-170 |
| 2-062 | Me | Me | D-1 | 4-OCH₂Ph | H | F | 123-125 |
| 2-063 | Me | Me | D-1 | 4-OCH₂Ph | Me | F | 143-145 |
| 2-064 | Me | Me | D-1 | 4-OCH₂Ph | CH₂OCH₂CH₂OMe | F | 112-113 |
| 2-065 | Me | Me | D-1 | 4-OCH₂Ph | CH₂Ph | F | 168-170 |
| 2-066 | Me | Me | D-1 | 4-OCH₂CN | H | F | ∗1 |
| 2-067 | Me | Me | D-1 | 4-OCH₂CN | CH₂OCH₂CH₂OMe | F | ∗1 |
| 2-068 | Me | Me | D-1 | 4-OCH₂C(O)Me | H | F | ∗1 |
| 2-069 | Me | Me | D-1 | 4-OCH₂C (=NOMe) | Me H | F | ∗1 |
| 2-070 | Me | Me | D-1 | 4-OCH₂C (O) OMe | H | F | 226-228 |
| 2-071 | Me | Me | D-1 | 4-OCH₂C (O) OMe | CH₂OCH₂CH₂OMe | F | ∗1 |
| 2-072 | Me | Me | D-1 | 4-OPh | H | F | 233-235 |
| 2-073 | Me | Me | D-1 | 4-OPh | C(O)Pr-n | F | ∗1 |
| 2-074 | Me | Me | D-1 | 4-O[U-2a] | H | F | 208-210 |
| 2-075 | Me | Me | D-1 | 4-OS(O)₂CF₃ | H | F | 210-212 |
| 2-076 | Me | Me | D-1 | 4-SMe | H | F | 178-180 |
| 2-077 | Me | Me | D-1 | 4-SMe | C(O)Pr-n | F | 146-148 |
| 2-078 | Me | Me | D-1 | 4-C(O)Me | C(O)Pr-n | F | 152-154 |
| 2-079 | Me | Me | D-1 | 4-C(=NOMe)Me | H | F | 164-166 |
| 2-080 | Me | Me | D-1 | 4-C(=NOMe)Me | C(O)Pr-n | F | ∗1 |
| 2-081 | Me | Me | D-1 | 3,4-Cl₂ | H | F | 236-238 |
| 2-082 | Me | Me | D-1 | 3,4-Cl₂ | C(O)Pr-n | F | 186-187 |
| 2-083 | Me | Me | D-1 | 2-OMe-4-F | H | F | 242-244 |
| 2-084 | Me | Me | D-1 | 2-OEt-4-F | H | F | 214-216 |
| 2-085 | Me | Me | D-1 | 2-OEt-4-F | C(O)Pr-n | F | 159-161 |
| 2-086 | Me | Me | D-1 | 3-F-4-OMe | H | F | 229-231 |
| 2-087 | Me | Me | D-1 | 3-F-4-OMe | C(O)Pr-n | F | 168-170 |
| 2-088 | Me | Me | D-1 | 3,4-(OMe)₂ | H | F | 129-131 |
| 2-089 | Me | Me | D-1 | 3,4-(OMe)₂ | C(O)Pr-n | F | 145-147 |
| 2-090 | Me | Me | D-22a | - | H | F | 224-226 |
| 2-091 | Me | Me | D-22a | - | C(O)Pr-n | F | 146-148 |
| 2-092 | Me | Me | D-24a | - | H | F | 155-158 |
| 2-093 | Me | Me | D-24a | - | C(O)Pr-n | F | 160-162 |
| 2-094 | Me | Me | D-1 | - | H | Cl | 280-282 |
| 2-095 | Me | Me | D-1 | 2-OMe-4-F | H | Cl | 221-223 |
| 2-096 | Me | Me | D-1 | 2-OMe-4-F | C(O)Pr-n | Cl | ∗1 |
| 2-097 | Me | Me | D-1 | 4-F | H | Cl | 292-294 |
| 2-098 | Me | Me | D-1 | 4-F | C(O)Pr-n | Cl | 158-160 |
| 2-099 | Me | Me | D-1 | 4-Cl | H | Cl | 292-293 |
| 2-100 | Me | Me | D-1 | 4-Cl | C(O)Pr-n | Cl | 161-162 |
| 2-101 | Me | Me | D-1 | 2-OMe | H | Cl | 232-233 |
| 2-102 | Me | Me | D-1 | 2-OMe | Me | Cl | 174-175 |
| 2-103 | Me | Me | D-1 | 4-OMe | H | Cl | 336-338 |
| 2-104 | Me | Me | D-1 | 4-OMe | C(O)Pr-n | Cl | 157-159 |
| 2-105 | Me | Me | D-1 | 2-OEt | H | Cl | 205-207 |
| 2-106 | Me | Me | D-1 | 2-OEt | Me | Cl | ∗1 |
| 2-107 | Me | Me | D-1 | 4-OPr-i | H | Cl | 216-218 |
| 2-108 | Me | Me | D-1 | 4-OPr-i | C(O)Pr-n | Cl | ∗1 |
| 2-109 | Me | Me | D-1 | 4-OBu-n | H | Cl | 182-185 |
| 2-110 | Me | Me | D-1 | 4-OBu-n | C(O)Pr-n | Cl | ∗1 |
| 2-111 | Me | Me | D-1 | 4-CF₃ | H | Cl | 282-284 |
| 2-112 | Me | Me | D-1 | 4-CN | H | Cl | 251-253 |
| 2-113 | Me | Me | D-1 | 4-C(O)OH | H | Cl | 322-324 |
| 2-114 | Me | Me | D-1 | 2-OMe-4-F | C(O)OCH₂CH₂OMe | Cl | ∗1 |
| 2-115 | Me | Me | D-1 | 2-OEt-4-F | H | Cl | 227-228 |
| 2-116 | Me | Me | D-1 | 2-OEt-4-F | Me | Cl | 244-246 |
| 2-117 | Me | Me | D-1 | 3-F-4-OMe | H | Cl | ∗1 |
| 2-118 | Me | Me | D-1 | 3-F-4-OMe | C(O)Pr-n | Cl | 126-128 |
| 2-119 | Me | Me | D-1 | 3-Me-4-OMe | H | Cl | 163-165 |
| 2-120 | Me | Me | D-1 | 3-Me-4-OMe | C(O)Pr-n | Cl | ∗1 |
| 2-121 | Me | Me | D-1 | 2,6-(OMe)₂ | H | Cl | 254-256 |
| 2-122 | Me | Me | D-1 | 2,6-(OMe)₂ | C(O)Pr-n | Cl | 179-181 |
| 2-123 | Me | Me | D-1 | 3,4-(OMe)₂ | H | Cl | ∗1 |
| 2-124 | Me | Me | D-1 | 3,4-(OMe)₂ | C(O)Pr-n | Cl | ∗1 |
| 2-125 | Me | Me | D-22a | - | H | Cl | 329-331 |
| 2-126 | Me | Me | D-22a | - | C(O)Pr-n | Cl | 159-161 |
| 2-127 | Me | Me | D-24a | - | H | Cl | 180-182 |
| 2-128 | Me | Me | D-24a | - | C(O)Pr-n | Cl | 142-144 |
| 2-129 | Me | Me | D-1 | 2-OMe-5-Br | H | Br | 236-238 |
| 2-130 | Me | Me | D-1 | 2-OMe | H | I | 278-281 |
| 2-131 | Me | Me | D-1 | 2-OMe | Me | I | 161-163 |
| 2-132 | Me | Me | D-1 | - | H | Me | 291-293 |
| 2-133 | Me | Me | D-1 | - | C(O)Pr-n | Me | ∗1 |
| 2-134 | Me | Me | D-1 | 4-F | H | Me | 286-288 |
| 2-135 | Me | Me | D-1 | 4-F | C(O)Pr-n | Me | ∗1 |
| 2-136 | Me | Me | D-1 | 4-Me | H | Me | 269-271 |
| 2-137 | Me | Me | D-1 | 4-Me | C(O)Pr-n | Me | ∗1 |
| 2-138 | Me | Me | D-1 | 2-OMe | H | Me | 109-111 |
| 2-139 | Me | Me | D-1 | 2-OMe | C(O)Pr-n | Me | 176-178 |
| 2-140 | Me | Me | D-1 | 4-OMe | H | Me | ∗1 |
| 2-141 | Me | Me | D-1 | 4-OMe | C(O)Pr-n | Me | ∗1 |
| 2-142 | Me | Me | D-1 | 2-SMe | H | Me | 196-198 |
| 2-143 | Me | Me | D-1 | 2-SMe | C(O)Pr-n | Me | ∗1 |
| 2-144 | Me | Me | D-1 | 4-NO₂ | H | Me | 158-160 |
| 2-145 | Me | Me | D-1 | 4-NO₂ | C(O)Pr-n | Me | ∗1 |
| 2-146 | Me | Me | D-1 | 4-CN | H | Me | 193-195 |
| 2-147 | Me | Me | D-1 | 4-CN | C(O)Pr-n | Me | ∗1 |
| 2-148 | Me | Me | D-1 | 2-OMe-4-F | H | Me | 224-226 |
| 2-149 | Me | Me | D-1 | 2-OMe-4-F | C(O)Pr-n | Me | 196-198 |
| 2-150 | Me | Me | D-1 | 2-OMe-5-F | H | Me | 201-203 |
| 2-151 | Me | Me | D-1 | 2-OMe-5-F | C(O)Pr-n | Me | 166-168 |
| 2-152 | Me | Me | D-1 | 3,4-(OMe)₂ | H | Me | 188-190 |
| 2-153 | Me | Me | D-1 | 3,4-(OMe)₂ | C(O)Pr-n | Me | 151-153 |
| 2-154 | Me | Me | D-17 | - | H | Me | 233-236 |
| 2-155 | Me | Me | D-17 | - | C(O)Pr-n | Me | ∗1 |
| 2-156 | Me | Me | D-22a | - | H | Me | 262-264 |
| 2-157 | Me | Me | D-22a | - | C(O)Pr-n | Me | 137-139 |
| 2-158 | Me | Me | D-24a | - | H | Me | 254-256 |
| 2-159 | Me | Me | D-24a | - | C(O)Pr-n | Me | ∗1 |
| 2-160 | Me | Me | D-1 | 2-OMe | H | Pr-c | 182-184 |
| 2-161 | Me | Me | D-1 | 2-OMe | Me | Pr-c | ∗1 |
| 2-162 | Me | Me | D-1 | 2-OMe | H | CF₃ | 119-121 |
| 2-163 | Me | Me | D-1 | 2-OMe | Me | CF₃ | 144-146 |
| 2-164 | Me | Me | D-1 | - | H | OMe | 211-213 |
| 2-165 | Me | Me | D-1 | 2-OMe | H | OMe | 86-88 |
| 2-166 | Me | Me | D-1 | - | H | Ph | >300 |
| 2-167 | Me | Me | D-1 | 2-OMe-4-F | H | [Ph-4-OPr-i] | 273-274 |
| 2-168 | Me | Me | D-1 | 2-OMe-4-F | Me | [Ph-4-OPr-i] | 174-176 |
| 2-169 | Me | Me | D-1 | 2-OMe-4-F | C(O)Pr-n | [Ph-4-OPr-i] | 223-225 |
| 2-170 | Me | Me | D-1 | 2-OMe-4-F | H | Q-3 | 252-254 |
| 2-171 | Me | Me | D-1 | 2-OMe-4-F | Me | Q-3 | 200-202 |
| 2-172 | Me | Me | D-1 | 2-OMe | Me | OH | 199-201 |
| 2-173 | Me | Me | D-1 | 2-OMe-4-F | H | OPr-n | 252-254 |
| 2-174 | Me | Me | D-1 | 2-OMe | Me | NH₂ | 219-221 |
| 2-175 | Me | Me | D-1 | 2-OMe-4-F | H | NHC(O)Me | 294-295 |
| 2-176 | Me | Me | D-1 | 2-OMe-4-F | Me | NHC(O)Me | 254-255 |
| 2-177 | Me | Me | D-1 | 2-OMe-4-F | H | N(Me)C(O)Me | 265-267 |
| 2-178 | Me | Me | D-1 | 2-OMe-4-F | Me | N(Me)C(O)Me | 223-225 |
| 2-179 | Me | Me | D-1 | 2-OMe-4-F | H | NHC(O)CH₂OMe | 302-304 |
| 2-180 | Me | Me | D-1 | 2-OMe-4-F | Me | NHC(O)CH₂OMe | 201-203 |
| 2-181 | Me | Me | D-1 | 2-OMe | H | NHC(O)OBu-t | 140-142 |
| 2-182 | Me | Me | D-1 | 2-OMe | Me | NHC(O)OBu-t | ∗1 |
| 2-183 | Me | Me | D-1 | 2-OMe-4-F | H | NHS(O)₂Me | 160-162 |
| 2-184 | Me | Me | D-1 | 2-OMe-4-F | Me | N(S(O)₂Me)₂ | 259-261 |
| 2-185 | Me | Me | D-1 | 2-OMe | H | CH=CH₂ | 227-229 |
| 2-186 | Me | Me | D-1 | 2-OMe | Me | CH=CH₂ | ∗1 |
| 2-187 | Me | Me | D-1 | 2-OMe | H | C≡CH | 252-254 |
| 2-188 | Me | Me | D-1 | 2-OMe | H | C≡CSiMe₃ | 247-249 |
| 2-189 | Me | Me | D-1 | 2-OMe | Me | C≡CSiMe₃ | ∗1 |
| 2-190 | Me | Me | D-1 | 2-OMe | H | CN | 243-244 |
| 2-191 | Me | Me | D-1 | 2-OMe | Me | CN | 196-198 |
| 2-192 | Me | Me | D-1 | 2-OMe-4-F | H | C(O)Me | 267-269 |
| 2-193 | Me | Me | D-1 | 2-OMe-4-F | Me | C(O)Me | ∗1 |
| 2-194 | Me | Me | D-1 | 2-OMe-4-F | Me | C(=NOH)Me | 254-256 |
| 2-195 | Me | Me | D-1 | 2-OMe-4-F | H | C(=NOPr-i)Me | 224-225 |
| 2-196 | Me | Me | D-1 | 2-OMe-4-F | Me | C(=NOPr-i)Me | 181-182 |
| 2-197 | Me | Me | D-1 | - | H | F | 209-211 |
| 2-198 | Me | Me | D-1 | - | C(O)Pr-n | F | 119-121 |
| 2-199 | Me | Me | D-1 | 2-F | H | F | 223-225 |
| 2-200 | Me | Me | D-1 | 2-F | C(O)Pr-n | F | 142-144 |
| 2-201 | Me | Me | D-1 | 3-F | H | F | 249-250 |
| 2-202 | Me | Me | D-1 | 3-F | C(O)Pr-n | F | 128-130 |
| 2-203 | Me | Me | D-1 | 2-Cl | H | F | 246-248 |
| 2-204 | Me | Me | D-1 | 2-Cl | C(O)Pr-n | F | ∗1 |
| 2-205 | Me | Me | D-1 | 4-Br | H | F | 167-169 |
| 2-206 | Me | Me | D-1 | 4-Br | C(O)Pr-n | F | 159-160 |
| 2-207 | Me | Me | D-1 | 4-Et | H | F | 223-225 |
| 2-208 | Me | Me | D-1 | 4-Bu-t | H | F | 194-197 |
| 2-209 | Me | Me | D-1 | 3-OMe | H | F | 222-224 |
| 2-210 | Me | Me | D-1 | 3-OMe | C(O)Pr-n | F | ∗1 |
| 2-211 | Me | Me | D-1 | 3-OEt | H | F | 210-212 |
| 2-212 | Me | Me | D-1 | 3-OEt | C(O)Pr-n | F | ∗1 |
| 2-213 | Me | Me | D-1 | 3-OPr-n | H | F | 182-184 |
| 2-214 | Me | Me | D-1 | 3-OPr-n | C(O)Pr-n | F | 112-113 |
| 2-215 | Me | Me | D-1 | 3-OPr-i | H | F | 107-109 |
| 2-216 | Me | Me | D-1 | 3-OPr-i | C(O)Pr-n | F | ∗1 |
| 2-217 | Me | Me | D-1 | 4-OPr-c | H | F | 190-192 |
| 2-218 | Me | Me | D-1 | 4-OPr-c | C(O)Pr-n | F | ∗1 |
| 2-219 | Me | Me | D-1 | 3-OBu-n | H | F | 95-97 |
| 2-220 | Me | Me | D-1 | 3-OBu-n | C(O)Pr-n | F | 128-129 |
| 2-221 | Me | Me | D-1 | 3-OBu-i | H | F | 102-104 |
| 2-222 | Me | Me | D-1 | 3-OBu-i | C(O)Pr-n | F | ∗1 |
| 2-223 | Me | Me | D-1 | 3-OBu-t | H | F | 196-198 |
| 2-224 | Me | Me | D-1 | 3-OBu-t | C(O)Pr-n | F | ∗1 |
| 2-225 | Me | Me | D-1 | 3-OPen-n | H | F | 99-101 |
| 2-226 | Me | Me | D-1 | 3-OHex-n | H | F | 87-89 |
| 2-227 | Me | Me | D-1 | 4-CHF₂ | H | F | 246-248 |
| 2-228 | Me | Me | D-1 | 4-CHF₂ | C(O)Pr-n | F | 155-157 |
| 2-229 | Me | Me | D-1 | 2-CF₃ | H | F | 220-223 |
| 2-230 | Me | Me | D-1 | 2-CF₃ | C(O)Pr-n | F | ∗1 |
| 2-231 | Me | Me | D-1 | 3-CF₃ | H | F | 122-124 |
| 2-232 | Me | Me | D-1 | 3-CF₃ | C(O)Pr-n | F | 101-103 |
| 2-233 | Me | Me | D-1 | 4-CH₂Cl | Me | F | 107-109 |
| 2-234 | Me | Me | D-1 | 3-OCHF₂ | H | F | 171-173 |
| 2-235 | Me | Me | D-1 | 3-OCHF₂ | C(O)Pr-n | F | ∗1 |
| 2-236 | Me | Me | D-1 | 3-OCF₃ | H | F | 181-183 |
| 2-237 | Me | Me | D-1 | 3-OCF₃ | C(O)Pr-n | F | ∗1 |
| 2-238 | Me | Me | D-1 | 3-OCH₂CF₃ | H | F | 228-231 |
| 2-239 | Me | Me | D-1 | 3-OCH₂CF₃ | C(O)Pr-n | F | ∗1 |
| 2-240 | Me | Me | D-1 | 3-SMe | H | F | 161-163 |
| 2-241 | Me | Me | D-1 | 3-SMe | C(O)Pr-n | F | 125-127 |
| 2-242 | Me | Me | D-1 | 3-SEt | H | F | 171-173 |
| 2-243 | Me | Me | D-1 | 3-SEt | C(O)Pr-n | F | 101-103 |
| 2-244 | Me | Me | D-1 | 4-SEt | H | F | 135-137 |
| 2-245 | Me | Me | D-1 | 4-SEt | C(O)Pr-n | F | ∗1 |
| 2-246 | Me | Me | D-1 | 3-SPr-n | H | F | 170-172 |
| 2-247 | Me | Me | D-1 | 3-SPr-n | C(O)Pr-n | F | 121-122 |
| 2-248 | Me | Me | D-1 | 4-SPr-n | H | F | 96-98 |
| 2-249 | Me | Me | D-1 | 4-SPr-n | C(O)Pr-n | F | ∗1 |
| 2-250 | Me | Me | D-1 | 3-SPr-i | H | F | 182-184 |
| 2-251 | Me | Me | D-1 | 3-SPr-i | C(O)Pr-n | F | ∗1 |
| 2-252 | Me | Me | D-1 | 4-SPr-i | H | F | 189-191 |
| 2-253 | Me | Me | D-1 | 4-SPr-i | C(O)Pr-n | F | ∗1 |
| 2-254 | Me | Me | D-1 | 4-SPr-c | H | F | 222-224 |
| 2-255 | Me | Me | D-1 | 4-SPr-c | C(O)Pr-n | F | ∗1 |
| 2-256 | Me | Me | D-1 | 4-S(O)Me | H | F | 141-143 |
| 2-257 | Me | Me | D-1 | 4-S(O)Pr-i | H | F | 236-238 |
| 2-258 | Me | Me | D-1 | 4-S(O)₂Me | H | F | 234-236 |
| 2-259 | Me | Me | D-1 | 4-S(O)₂Pr-i | H | F | 300-302 |
| 2-260 | Me | Me | D-1 | 4-SCH₂CHF₂ | H | F | ∗1 |
| 2-261 | Me | Me | D-1 | 4-SCH₂CHF₂ | Me | F | 160-161 |
| 2-262 | Me | Me | D-1 | 3-SCH₂CF₃ | H | F | 121-123 |
| 2-263 | Me | Me | D-1 | 3-SCH₂CF₃ | C(O)Pr-n | F | 105-107 |
| 2-264 | Me | Me | D-1 | 4-S(O)CH₂CHF₂ | H | F | 165-167 |
| 2-265 | Me | Me | D-1 | 4-SCH₂CH₂OMe | H | F | 130-133 |
| 2-266 | Me | Me | D-1 | 4-SCH₂CH₂OMe | Me | F | ∗1 |
| 2-267 | Me | Me | D-1 | 3-OBn | H | F | 83-85 |
| 2-268 | Me | Me | D-1 | 3-OBn | C(O)Pr-n | F | ∗1 |
| 2-269 | Me | Me | D-1 | 3-OCH₂CH=CH₂ | H | F | 219-221 |
| 2-270 | Me | Me | D-1 | 3-OCH₂CH=CH₂ | C(O)Pr-n | F | ∗1 |
| 2-271 | Me | Me | D-1 | 3-OCH₂CH₂OMe | H | F | 198-200 |
| 2-272 | Me | Me | D-1 | 3-OCH₂CH₂OMe | C(O)Pr-n | F | ∗1 |
| 2-273 | Me | Me | D-1 | 3-OCH₂CH₂SMe | H | F | 214-216 |
| 2-274 | Me | Me | D-1 | 3-OCH₂CH₂SMe | C(O)Pr-n | F | ∗1 |
| 2-275 | Me | Me | D-1 | 3-OCH₂C(O)Me | H | F | 151-154 |
| 2-276 | Me | Me | D-1 | 3-OCH₂C(O)Me | C(O)Pr-n | F | 142-144 |
| 2-277 | Me | Me | D-1 | 3-OCH(Me)CN | H | F | 189-191 |
| 2-278 | Me | Me | D-1 | 3-OCH(Me)CN | C(O)Pr-n | F | ∗1 |
| 2-279 | Me | Me | D-1 | 4-OS(O)₂CF₃ | Me | F | 198-200 |
| 2-280 | Me | Me | D-1 | 4-OCHF₂ | C(O)Pen-c | F | 124-126 |
| 2-281 | Me | Me | D-1 | 4-OCHF₂ | C(O)C(Me)=CH₂ | F | 99-101 |
| 2-282 | Me | Me | D-1 | 4-OCHF₂ | C(O)C(Me)₂Ph | F | ∗1 |
| 2-283 | Me | Me | D-1 | 4-OCHF₂ | C(O)CH₂OMe | F | ∗1 |
| 2-284 | Me | Me | D-1 | 4-OCHF₂ | C(O)CH₂OPh | F | ∗1 |
| 2-285 | Me | Me | D-1 | 4-OCHF₂ | C(O)[Ph-2-Me] | F | 85-87 |
| 2-286 | Me | Me | D-1 | 4-OCHF₂ | C(O) [Ph-4-Cl] | F | ∗1 |
| 2-287 | Me | Me | D-1 | 4-OCHF₂ | C(O)[Q-2a] | F | 195-197 |
| 2-288 | Me | Me | D-1 | 4-OCHF₂ | C(O)[Q-4a] | F | 178-179 |
| 2-289 | Me | Me | D-1 | 4-OCHF₂ | C(O)[U-1a] | F | ∗1 |
| 2-290 | Me | Me | D-1 | 4-OCHF₂ | C(O)[U-6a] | F | 171-173 |
| 2-291 | Me | Me | D-1 | 4-OCHF₂ | C(O)NMe₂ | F | 92-94 |
| 2-292 | Me | Me | D-1 | 4-OCHF₂ | C(O)OEt | F | 123-125 |
| 2-293 | Me | Me | D-1 | 4-OCHF₂ | C(O)OCH₂CH₂C1 | F | ∗1 |
| 2-294 | Me | Me | D-1 | 4-OCHF₂ | C(O)OCH₂CH=CH₂ | F | ∗1 |
| 2-295 | Me | Me | D-1 | 4-OCHF₂ | C(O)OCH₂CH₂OMe | F | ∗1 |
| 2-296 | Me | Me | D-1 | 4-OCHF₂ | C(O)SBu-t | F | 155-158 |
| 2-297 | Me | Me | D-1 | 4-OCHF₂ | C(S)OPh | F | ∗1 |
| 2-298 | Me | Me | D-1 | 4-OCHF₂ | C(S)SPh | F | 169-171 |
| 2-299 | Me | Me | D-1 | 4-OCHF₂ | C(O)C(O)Ph | F | ∗1 |
| 2-300 | Me | Me | D-1 | 4-OCHF₂ | CH₂CH=CH₂ | F | ∗1 |
| 2-301 | Me | Me | D-1 | 4-OCHF₂ | CH₂C≡CH | F | ∗1 |
| 2-302 | Me | Me | D-1 | 4-OCHF₂ | CH₂CF₃ | F | ∗1 |
| 2-303 | Me | Me | D-1 | 4-OCHF₂ | CH₂CN | F | 134-136 |
| 2-304 | Me | Me | D-1 | 4-OCHF₂ | CH₂C(O)Me | F | 194-196 |
| 2-305 | Me | Me | D-1 | 4-OCHF₂ | CH₂C(O)Ph | F | 156-158 |
| 2-306 | Me | Me | D-1 | 4-OCHF₂ | CH₂C(O)OMe | F | 152-154 |
| 2-307 | Me | Me | D-1 | 4-OCHF₂ | CH₂OCH₂CH₂OMe | F | ∗1 |
| 2-308 | Me | Me | D-1 | 4-OCHF₂ | CH(Me)OC(O)OEt | F | ∗1 |
| 2-309 | Me | Me | D-1 | 4-OCHF₂ | S(O)₂Pr-i | F | ∗1 |
| 2-310 | Me | Me | D-1 | 4-OCHF₂ | S(O)₂NMe | F | 168-170 |
| 2-311 | Me | Me | D-1 | 4-OCHF₂ | S(O)₂[Ph-4-Cl] | F | 193-195 |
| 2-312 | Me | Me | D-1 | 4-OCHF₂ | S(O)₂[Ph-4-Me] | F | 181-183 |
| 2-313 | Me | Me | D-1 | 4-OCHF₂ | S(O)₂[Ph-3-OMe] | F | ∗1 |
| 2-314 | Me | Me | D-1 | 4-CH₂OH | Me | F | 170-172 |
| 2-315 | Me | Me | D-1 | 4-CH₂OCH₂CF₃ | H | F | 77-79 |
| 2-316 | Me | Me | D-1 | 4-CH₂OCH₂CF₃ | Me | F | ∗1 |
| 2-317 | Me | Me | D-1 | 4-CH₂SMe | H | F | ∗1 |
| 2-318 | Me | Me | D-1 | 4-CH₂SMe | Me | F | 150-151 |
| 2-319 | Me | Me | D-1 | 4-CH₂SEt | H | F | ∗1 |
| 2-320 | Me | Me | D-1 | 4-CH₂SEt | Me | F | 123-124 |
| 2-321 | Me | Me | D-1 | 4-CH₂SPr-i | H | F | ∗1 |
| 2-322 | Me | Me | D-1 | 4-CH₂SPr-i | Me | F | ∗1 |
| 2-323 | Me | Me | D-1 | 4-CH₂SCH₂CH=CH₂ | H | F | ∗1 |
| 2-324 | Me | Me | D-1 | 4-CH₂SCH₂CH=CH₂ | Me | F | ∗1 |
| 2-325 | Me | Me | D-1 | 4-CH₂SCH₂C≡CH | H | F | ∗1 |
| 2-326 | Me | Me | D-1 | 4-CH₂SCH₂C≡CH | Me | F | 107-109 |
| 2-327 | Me | Me | D-1 | 4-CH₂SCH₂CH₂OMe | H | F | ∗1 |
| 2-328 | Me | Me | D-1 | 4-CH₂SCH₂CH₂OMe | Me | F | ∗1 |
| 2-329 | Me | Me | D-1 | 4-CH₂SCH₂CH₂SMe | H | F | 172-175 |
| 2-330 | Me | Me | D-1 | 4-CH₂SCH₂CHF₂ | H | F | ∗1 |
| 2-331 | Me | Me | D-1 | 4-CH₂SCH₂CF₃ | H | F | ∗1 |
| 2-332 | Me | Me | D-1 | 4-CH₂S(O)Me | H | F | 154-156 |
| 2-333 | Me | Me | D-1 | 4-CH₂S(O)Et | H | F | 200-201 |
| 2-334 | Me | Me | D-1 | 4-CH₂S(O)₂Me | H | F | 157-159 |
| 2-335 | Me | Me | D-1 | 4-CH₂S(O)₂Et | H | F | ∗1 |
| 2-336 | Me | Me | D-1 | 3-OH | H | F | 171-173 |
| 2-337 | Me | Me | D-1 | 4-OH | H | F | 256-258 |
| 2-338 | Me | Me | D-1 | 4-OH | Me | F | 264-266 |
| 2-339 | Me | Me | D-1 | 4-C(O)Me | H | F | 154-156 |
| 2-340 | Me | Me | D-1 | 4-CN | H | F | 262-264 |
| 2-341 | Me | Me | D-1 | 4-NO₂ | H | F | 276-278 |
| 2-342 | Me | Me | D-1 | 2, 4-F₂ | H | F | 262-264 |
| 2-343 | Me | Me | D-1 | 2, 4-F₂ | C(O)Pr-n | F | 113-115 |
| 2-344 | Me | Me | D-1 | 3, 4-F₂ | H | F | 284-286 |
| 2-345 | Me | Me | D-1 | 3, 4-F₂ | C(O)Pr-n | F | 152-154 |
| 2-346 | Me | Me | D-1 | 2, 4-Cl₂ | H | F | 280-281 |
| 2-347 | Me | Me | D-1 | 2, 4-Cl₂ | C(O)Pr-n | F | 153-154 |
| 2-348 | Me | Me | D-1 | 2-F-4-Cl | H | F | 250-252 |
| 2-349 | Me | Me | D-1 | 2-F-4-Cl | C(O)Pr-n | F | 150-151 |
| 2-350 | Me | Me | D-1 | 3-F-4-Cl | H | F | 278-280 |
| 2-351 | Me | Me | D-1 | 3-F-4-Cl | C(O)Pr-n | F | 182-184 |
| 2-352 | Me | Me | D-1 | 3-Cl-4-F | H | F | 236-238 |
| 2-353 | Me | Me | D-1 | 3-Cl-4-F | C(O)Pr-n | F | 149-150 |
| 2-354 | Me | Me | D-1 | 2-F-4-CF₃ | H | F | 251-253 |
| 2-355 | Me | Me | D-1 | 2-F-4-CF₃ | C(O)Pr-n | F | 145-147 |
| 2-356 | Me | Me | D-1 | 2-Cl-4-CF₃ | H | F | 264-266 |
| 2-357 | Me | Me | D-1 | 2-Cl-4-CF₃ | C(O)Pr-n | F | 147-149 |
| 2-358 | Me | Me | D-1 | 3, 5-(CF₃)₂ | H | F | 130-132 |
| 2-359 | Me | Me | D-1 | 3, 5-(CF₃)₂ | C(O)Pr-n | F | 158-160 |
| 2-360 | Me | Me | D-1 | 3, 5-(OMe)₂ | H | F | 226-227 |
| 2-361 | Me | Me | D-1 | 3, 5-(OMe)₂ | C(O)Pr-n | F | ∗1 |
| 2-362 | Me | Me | D-1 | 2-F-3-OMe | H | F | 216-217 |
| 2-363 | Me | Me | D-1 | 2-F-3-OMe | C(O)Pr-n | F | 79-81 |
| 2-364 | Me | Me | D-1 | 2-F-4-OMe | H | F | 226-228 |
| 2-365 | Me | Me | D-1 | 2-F-4-OMe | C(O)Pr-n | F | 157-158 |
| 2-366 | Me | Me | D-1 | 2-F-5-OMe | H | F | 202-204 |
| 2-367 | Me | Me | D-1 | 2-F-5-OMe | C(O)Pr-n | F | ∗1 |
| 2-368 | Me | Me | D-1 | 2-OMe-4-Cl | H | F | 236-238 |
| 2-369 | Me | Me | D-1 | 2-OMe-4-Cl | C(O)Pr-n | F | ∗1 |
| 2-370 | Me | Me | D-1 | 3-Cl-4-OMe | H | F | 239-241 |
| 2-371 | Me | Me | D-1 | 3-Cl-4-OMe | C(O)Pr-n | F | 154-156 |
| 2-372 | Me | Me | D-1 | 2, 4, 6-F₃ | H | F | ∗1 |
| 2-373 | Me | Me | D-1 | 4-Cl | Me | Cl | 187-189 |
| 2-374 | Me | Me | D-1 | 4-OCHF₂ | H | Cl | 180-183 |
| 2-375 | Me | Me | D-1 | 4-OCHF₂ | C(O)Pr-n | Cl | 146-148 |
| 2-376 | Me | Me | D-1 | 2-OMe-4-F | H | SMe | 219-221 |
| 2-377 | Me | Me | D-1 | 2-OMe-4-F | H | S(O)₂Me | 230-232 |
| 2-378 | Me | Me | D-1 | 2-OMe | H | SCF₃ | 183-186 |
| 2-379 | Me | Me | D-1 | 2-OMe | Me | SCF₃ | 152-155 |
| 2-380 | Me | Me | D-1 | 2-OMe-4-F | Me | S(T-3) | ∗1 |
| 2-381 | Me | Me | D-1 | 4-Cl | Me | NH₂ | 223-227 |
| 2-382 | Me | Me | D-2 | 6-OMe | H | F | 264-266 |
| 2-383 | Me | Me | D-3 | 4-F | H | F | 282-284 |
| 2-384 | Me | Me | D-3 | 4-F | C(O)Pr-n | F | 154-156 |
| 2-385 | Me | Me | D-3 | 4-Cl | H | F | 260-262 |
| 2-386 | Me | Me | D-3 | 4-Cl | C(O)Pr-n | F | 148-150 |
| 2-387 | Me | Me | D-3 | 4-CF₃ | H | F | 244-246 |
| 2-388 | Me | Me | D-3 | 4-CF₃ | C(O)Pr-n | F | 183-185 |
| 2-389 | Me | Me | D-3 | 4-SMe | H | F | 152-154 |
| 2-390 | Me | Me | D-7 | 5-Cl | H | F | 266-268 |
| 2-391 | Me | Me | D-7 | 5-Cl | C(O)Pr-n | F | 113-114 |
| 2-392 | Me | Me | D-23 | - | H | F | 138-140 |
| 2-393 | Me | Me | D-23 - | | C(O)Pr-n | F | 154-156 |
| 2-394 | Me | Me | D-28 2-Cl | | H | F | 174-176 |
| 2-395 | Me | Me | D-1 | 2-OMe | H | CHO | ∗1 |
| 2-396 | Me | Me | D-1 | 2-OMe | Me | CHO | ∗1 |
| 2-397 | Me | Me | D-1 | 4-Cl | Me | N=C(Ph)₂ | 235-239 |
| 2-398 | Et | Me | D-1 | 4-F | H | F | 242-244 |
| 2-399 | Et | Me | D-1 | 4-F | C(O)Pr-n | F | 115-117 |
| 2-400 | Et | Me | D-1 | 4-Cl | H | F | 242-244 |
| 2-401 | Et | Me | D-1 | 4-Cl | C(O)Pr-n | F | 128-131 |
| 2-402 | Et | Me | D-1 | 4-CF₃ | H | F | 195-197 |
| 2-403 | Et | Me | D-1 | 4-CF₃ | C(O)Pr-n | F | 136-138 |
| 2-404 | Et | Me | D-1 | 2-OMe | H | F | 216-218 |
| 2-405 | Et | Me | D-1 | 2-OMe | C(O)Pr-n | F | 183-185 |
| 2-406 | Me | Et | D-1 | 4-F | H | F | 182-184 |
| 2-407 | Me | Et | D-1 | 4-F | C(O)Pr-n | F | 125-127 |
| 2-408 | Me | Et | D-1 | 4-Cl | H | F | 224-226 |
| 2-409 | Me | Et | D-1 | 4-Cl | C(O)Pr-n | F | 127-129 |
| 2-410 | Me | Et | D-1 | 4-CF₃ | H | F | 205-207 |
| 2-411 | Me | Et | D-1 | 4-CF₃ | C(O)Pr-n | F | 156-158 |
| 2-412 | Me | Et | D-1 | 4-OCF₃ | H | F | 199-201 |
| 2-413 | Me | Et | D-1 | 4-OCF₃ | C(O)Pr-n | F | 95-97 |
| 2-414 | Me | Et | D-1 | 2-OMe | H | F | 217-219 |
| 2-415 | Me | Et | D-1 | 2-OMe | C(O)Pr-n | F | 166-167 |
| 2-416 | Me | Et | D-1 | 2-OEt | H | F | 170-172 |
| 2-417 | Me | Et | D-1 | 2-OEt | C(O)Pr-n | F | 102-104 |
| 2-418 | Me | Et | D-1 | 4-OPr-n | H | F | 121-124 |
| 2-419 | Me | Et | D-1 | 4-OPr-n | C(O)Pr-n | F | 91-94 |
| 2-420 | Me | Me | D-1 | 4-OCH₂CH₂SMe | CH₂CH₂SMe | F | ∗1 |
| 2-421 | Me | Me | D-1 | 4-SCH₂CH₂SMe | H | F | ∗1 |
| 2-422 | Me | Me | D-1 | 4-SCH₂CH=CH₂ | H | F | 67-70 |
| 2-423 | Me | Me | D-1 | 4-SCH₂CN | H | F | ∗1 |
| 2-424 | Me | Me | D-1 | 4-SCH₂C(O)Me | H | F | ∗1 |
| 2-425 | Me | Me | D-1 | 4-NMe₂ | H | F | 156-158 |
| 2-426 | Me | Me | D-1 | 4-NMe₂ | C(O)Pr-n | F | 148-151 |
| 2-427 | Me | Me | D-4 | 2-F | Me | F | 61-63 |
| 2-428 | H | Me | D-1 | 4-F | H | F | 151-154 |
| 2-429 | T-2 Me | | D-1 | 4-F | H | F | ∗1 |
| 2-430 | CH₂CN | Me | D-1 | 4-F | H | F | ∗1 |
| 2-431 | CH₂CN | Me | D-1 | 2-OMe | H | F | 189-191 |
| 2-432 | Bn | Me | D-1 | 4-F | H | F | 126-128 |
| 2-433 | Bn | Me | D-1 | 4-F | C(O)Pr-n | F | 99-101 |
| 2-434 | Bn | Me | D-1 | 4-Cl | H | F | 196-198 |
| 2-435 | Bn | Me | D-1 | 4-Cl | C(O)Pr-n | F | 74-76 |
| 2-436 | Bn | Me | D-1 | 4-CF₃ | H | F | 232-234 |
| 2-437 | Bn | Me | D-1 | 4-CF₃ | C(O)Pr-n | F | 135-137 |
| 2-438 | Bn | Me | D-1 | 2-OMe | H | F | 220-222 |
| 2-439 | Bn | Me | D-1 | 2-OMe | C(O)Pr-n | F | ∗1 |
| 2-440 | Me | Cl | D-1 | 4-OCH₂CH₂OMe | H | F | ∗1 |
| 2-441 | Me | H | D-1 | 4-OCH₂CH₂OMe | H | F | 170-173 |
| 2-442 | Me | Me | D-1 | 4-OCH₂CH=CCl₂ | H | F | ∗1 |
| 2-443 | Me | Me | D-1 | 4-OCH₂CH₂S(O)Me | H | F | 162-164 |
| 2-444 | Me | Me | D-1 | 4-OCH₂CH₂S(O)₂Me | H | F | ∗1 |
| 2-445 | Me | Me | D-1 | 4-CH₂OCH₂CH₂OMe | H | F | ∗1 |
| 2-446 | Me | Me | D-1 | 4-CH₂OCH₂CH₂SMe | H | F | ∗1 |
| 2-447 | Me | Me | D-1 | 4-OCH₂CH=CCl₂ | Me | F | 155-157 |
| 2-448 | Me | Me | D-1 | 4-Cl | Me | OH | ∗1 |
| 2-449 | Me | Me | D-1 | 4-Cl | Me | OMe | ∗1 |
| 2-450 | Me | Me | D-1 | 4-Cl | H | OMe | 164-166 |
| 2-451 | Me | Me | D-1 | 4-SCH₂C(=NOMe)Me | H | F | 87-89 |
| 2-452 | Me | Me | D-1 | 4-SCH₂C(=NOH)Me | H | F | 124-127 |
| 2-453 | Me | Me | D-1 | 4-OCH₂C(=NOH)Me | H | F | 173-175 |
| 2-454 | Me | Me | D-1 | 4-S(O)CH₂CH₂OMe | H | F | 106-109 |
| 2-455 | Me | Me | D-1 | 4-S(O)₂CH₂CH₂OMe | H | F | 223-225 |
| 2-456 | Me | NH₂ | D-1 | 4-OCH₂CH₂OMe | H | F | 135-138 |
| 2-457 | Me | Me | D-1 | 4-OCF₃ | H | Cl | 163-165 |
| 2-458 | Me | Me | D-1 | 4-OCF₃ | C(O)Pr-n | Cl | 172-174 |
| 2-459 | Me | Me | D-1 | 4-OCHF₂ | H | Me | ∗1 |
| 2-460 | Me | Me | D-1 | 3-S(O)Et | H | F | ∗1 |
| 2-461 | Me | Me | D-6 | - | H | F | 225-227 |
| 2-462 | Me | Me | D-6 | - | C(O)Pr-n | F | 133-135 |
| 2-463 | Me | Me | D-8 | - | H | F | 224-226 |
| 2-464 | Me | Me | D-8 | - | C(O)Pr-n | F | 150-152 |
| 2-465 | Me | Me | D-1 | 4-SCH₂C≡CH | H | F | ∗1 |
| 2-466 | Me | Me | D-1 | 4-SCH₂C≡CH | Me | F | 154-156 |
| 2-467 | Me | Me | D-1 | 4-O[Q-17b] | H | F | ∗1 |
| 2-468 | Me | Me | D-1 | 4-O[Q-17b] | CH₂OCH₂CH₂OMe | F | ∗1 |

**Table 5**

| | | | | | | | |
|---|---|---|---|---|---|---|---|
| 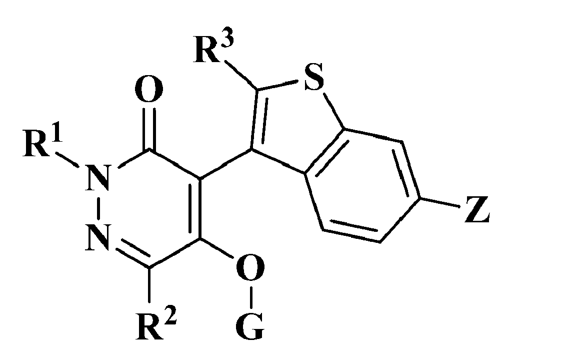 | | | | | | | |

| No. | R¹ | R² | R³ | (Y¹) | G | Z | mp (°C) |
|---|---|---|---|---|---|---|---|
| 3-001 | Me | Me | D-1 | 4-F | H | F | 230-232 |
| 3-002 | Me | Me | D-1 | 4-F | C(O)Pr-n | F | 160-162 |
| 3-003 | Me | Me | D-1 | 2-OMe | H | F | 205-206 |
| 3-004 | Me | Me | D-1 | 2-OMe | C(O)Pr-n | F | ∗1 |
| 3-005 | Me | Me | D-1 | 4-OMe | H | F | 119-121 |
| 3-006 | Me | Me | D-1 | 2-OEt | H | F | 189-191 |
| 3-007 | Me | Me | D-1 | 2-OEt | C(O)Pr-n | F | ∗1 |
| 3-008 | Me | Me | D-1 | 4-OMe | C(O)Pr-n | F | ∗1 |
| 3-009 | Me | Me | D-1 | 2-OMe-4-F | H | F | 219-221 |
| 3-010 | Me | Me | D-1 | 2-OMe-4-F | C(O)Pr-n | F | ∗1 |
| 3-011 | Me | Me | D-1 | 2-OEt-4-F | H | F | 182-184 |
| 3-012 | Me | Me | D-1 | 2-OEt-4-F | C(O)Pr-n | F | ∗1 |
| 3-013 | Me | Me | D-24a | - | H | F | 152-154 |
| 3-014 | Me | Me | D-24a | - | C(O)Pr-n | F | 142-144 |
| 3-015 | Me | Me | D-1 | 4-Cl | H | F | 252-254 |
| 3-016 | Me | Me | D-1 | 4-Cl | C(O)Pr-n | F | ∗1 |
| 3-017 | Me | Me | D-1 | 4-C(=NOMe)Me | H | F | 127-129 |
| 3-018 | Me | Me | D-1 | 4-C(=NOMe)Me | C(O)Pr-n | F | 137-139 |
| 3-019 | Me | Me | D-1 | 4-OPr-i | H | F | 122-124 |
| 3-020 | Me | Me | D-1 | 4-OPr-i | C(O)Pr-n | F | ∗1 |
| 3-021 | Me | Me | D-1 | 2-OMe | C(O)CH₂OPh | F | 160-162 |
| 3-022 | Me | Me | D-1 | 2-OMe | C(O)[Ph-2-Me] | F | 165-167 |
| 3-023 | Me | Me | D-1 | 2-OMe | C(O)[Q-2a] | F | 200-202 |
| 3-024 | Me | Me | D-1 | 2-OMe | C(O)OEt | F | 171-173 |
| 3-025 | Me | Me | D-1 | 2-OMe | C(O)[U-6a] | F | 87-89 |
| 3-026 | Me | Me | D-1 | 2-OMe | C(O)SBu-t | F | 178-180 |
| 3-027 | Me | Me | D-1 | 2-OMe | S(O)₂[Ph-4-Me] | F | 169-171 |
| 3-028 | Me | Me | D-1 | 2-OMe | S(O)₂NMe₂ | F | 126-128 |
| 3-029 | Me | Me | D-1 | 2-OMe | CH(Me)OC(O)OEt | F | 61-64 |
| 3-030 | Me | Me | D-1 | 2-OMe | CH₂C(O)Ph | F | 198-200 |
| 3-031 | Me | Me | D-1 | 4-F | H | Cl | 114-116 |
| 3-032 | Me | Me | D-1 | 4-F | C(O)Pr-n | Cl | ∗1 |
| 3-033 | Me | Me | D-1 | 4-Cl | H | Cl | 248-250 |
| 3-034 | Me | Me | D-1 | 4-Cl | C(O)Pr-n | Cl | ∗1 |
| 3-035 | Me | Me | D-1 | 2-OMe | H | Cl | 256-258 |
| 3-036 | Me | Me | D-1 | 2-OMe | C(O)Pr-n | Cl | ∗1 |
| 3-037 | Me | Me | D-1 | 4-OMe | H | Cl | 151-153 |
| 3-038 | Me | Me | D-1 | 4-OMe | C(O)Pr-n | Cl | ∗1 |
| 3-039 | Me | Me | D-1 | 2-OEt | H | Cl | 206-208 |
| 3-040 | Me | Me | D-1 | 2-OEt | C(O)Pr-n | Cl | ∗1 |
| 3-041 | Me | Me | D-1 | 4-OPr-i | H | Cl | 133-135 |
| 3-042 | Me | Me | D-1 | 4-OPr-i | C(O)Pr-n | Cl | ∗1 |
| 3-043 | Me | Me | D-1 | 2-OMe-4-F | H | Cl | 224-226 |
| 3-044 | Me | Me | D-1 | 2-OMe-4-F | C(O)Pr-n | Cl | ∗1 |
| 3-045 | Me | Me | D-1 | 2-OEt-4-F | H | Cl | 210-212 |
| 3-046 | Me | Me | D-1 | 2-OEt-4-F | C(O)Pr-n | Cl | ∗1 |
| 3-047 | Me | Me | D-24a | - | H | Cl | 176-178 |
| 3-048 | Me | Me | D-24a | - | C(O)Pr-n | Cl | ∗1 |
| 3-049 | Me | Me | D-1 | 2-OMe | H | Me | 233-235 |
| 3-050 | Me | Me | D-1 | 2-OMe | C(O)Pr-n | Me | ∗1 |
| 3-051 | Me | Me | D-1 | 4-OMe | H | Me | 130-132 |
| 3-052 | Me | Me | D-1 | 4-OMe | C(O)Pr-n | Me | 159-161 |
| 3-053 | Me | Me | D-1 | 2-OEt | H | Me | 104-106 |
| 3-054 | Me | Me | D-1 | 2-OEt | C(O)Pr-n | Me | ∗1 |
| 3-055 | Me | Me | D-1 | 2-OMe-4-F | H | Me | 206-208 |
| 3-056 | Me | Me | D-1 | 2-OMe-4-F | C(O)Pr-n | Me | 164-166 |
| 3-057 | Me | Me | D-1 | 2-OEt-4-F | H | Me | 182-184 |
| 3-058 | Me | Me | D-1 | 2-OEt-4-F | C(O)Pr-n | Me | ∗1 |
| 3-059 | Me | Me | D-24a | - | H | Me | 224-226 |
| 3-060 | Me | Me | D-24a | - | C(O)Pr-n | Me | 107-109 |
| 3-061 | Me | Me | D-1 | - | H | OMe | 189-191 |
| 3-062 | Me | Me | D-1 | - | C(O)Pr-n | OMe | ∗1 |
| 3-063 | Me | Me | D-1 | 4-F | H | OMe | 239-241 |
| 3-064 | Me | Me | D-1 | 4-F | C(O)Pr-n | OMe | 178-180 |
| 3-065 | Me | Me | D-1 | 2-OMe | H | OMe | 243-244 |
| 3-066 | Me | Me | D-1 | 2-OMe | C(O)Pr-n | OMe | ∗1 |
| 3-067 | Me | Me | D-1 | 2-OMe | Me | OMe | 148-149 |
| 3-068 | Me | Me | D-1 | 4-OMe | H | OMe | 118-120 |
| 3-069 | Me | Me | D-1 | 4-OMe | C(O)Pr-n | OMe | 109-111 |
| 3-070 | Me | Me | D-1 | 2-OEt | H | OMe | 103-105 |
| 3-071 | Me | Me | D-1 | 2-OEt | C(O)Pr-n | OMe | ∗1 |
| 3-072 | Me | Me | D-1 | 4-OEt | H | OMe | 207-209 |
| 3-073 | Me | Me | D-1 | 4-OEt | C(O)Pr-n | OMe | 152-154 |
| 3-074 | Me | Me | D-1 | 4-SMe | H | OMe | 205-207 |
| 3-075 | Me | Me | D-1 | 4-SMe | C(O)Pr-n | OMe | ∗1 |
| 3-076 | Me | Me | D-1 | 2-OMe-4-F | H | OMe | 213-215 |
| 3-077 | Me | Me | D-1 | 2-OMe-4-F | C(O)Pr-n | OMe | ∗1 |
| 3-078 | Me | Me | D-1 | 2-OEt-4-F | H | OMe | 165-167 |
| 3-079 | Me | Me | D-1 | 2-OEt-4-F | C(O)Pr-n | OMe | ∗1 |
| 3-080 | Me | Me | D-1 | 3,4-(OMe)₂ | H | OMe | 138-140 |
| 3-081 | Me | Me | D-1 | 3,4-(OMe)₂ | C (O)Pr-n | OMe | 148-150 |
| 3-082 | Me | Me | D-24a | - | H | OMe | 200-202 |
| 3-083 | Me | Me | D-24a | - | C(O)Pr-n | OMe | 70-72 |
| 3-084 | Et | Me | D-1 | 2-OMe | H | OMe | 213-216 |
| 3-085 | Et | Me | D-1 | 2-OEt | H | OMe | 91-94 |
| 3-086 | Me | N(Bn | )₂ D-1 | 2-OMe | H | OMe | 163-166 |
| 3-087 | Me | NH₂ | D-1 | 2-OMe | H | OMe | 286-288 |
| 3-088 | Me | Cl | D-1 | 2-OMe | H | OMe | 209-211 |
| 3-089 | Me | Me | D-1 | 2-OMe | H | OEt | 225-227 |
| 3-090 | Me | Me | D-1 | 2-OMe | C(O)Pr-n | OEt | ∗1 |
| 3-091 | Me | Me | D-1 | 4-OMe | H | OEt | 187-189 |
| 3-092 | Me | Me | D-1 | 4-OMe | C(O)Pr-n | OEt | ∗1 |
| 3-093 | Me | Me | D-1 | 2-OEt | H | OEt | 122-124 |
| 3-094 | Me | Me | D-1 | 2-OEt | C(O)Pr-n | OEt | ∗1 |
| 3-095 | Me | Me | D-1 | 2-OEt-4-F | H | OEt | 115-117 |
| 3-096 | Me | Me | D-1 | 2-OEt-4-F | C(O)Pr-n | OEt | ∗1 |
| 3-097 | Me | Me | D-1 | 2-OEt | H | OH | 268-270 |
| 3-098 | Me | Me | D-1 | 2-OEt | Me | OH | 268-269 |
| 3-099 | Me | Me | D-1 | 2-OMe | H | OPr-n | 220-221 |
| 3-100 | Me | Me | D-1 | 2-OMe | Me | OPr-n | 148-150 |
| 3-101 | Me | Me | D-1 | 2-OMe | H | OPr-i | 242-244 |
| 3-102 | Me | Me | D-1 | 2-OMe | Me | OPr-i | 133-135 |
| 3-103 | Me | Me | D-1 | 2-OEt | H | OPr-n | ∗1 |
| 3-104 | Me | Me | D-1 | 2-OEt | Me | OPr-n | ∗1 |
| 3-105 | Me | Me | D-1 | 2-OMe | H | OBu-n | 203-205 |
| 3-106 | Me | Me | D-1 | 2-OMe | Me | OBu-n | ∗1 |
| 3-107 | Me | Me | D-1 | 2-OMe | H | OBu-i | 227-229 |
| 3-108 | Me | Me | D-1 | 2-OMe | H | OPen-n | 199-201 |
| 3-109 | Me | Me | D-1 | 2-OMe | H | OPen-c | ∗1 |
| 3-110 | Me | Me | D-1 | 2-OMe | Me | OPen-c | ∗1 |
| 3-111 | Me | Me | D-1 | 2-OMe | H | OCH₂CH=CH₂ | 194-196 |
| 3-112 | Me | Me | D-1 | 2-OMe | Me | OCH₂CH=CH₂ | 141-143 |
| 3-113 | Me | Me | D-1 | 2-OMe | H | OCHF₂ | 191-199 |
| 3-114 | Me | Me | D-1 | 2-OMe | H | OCH₂CHF₂ | ∗1 |
| 3-115 | Me | Me | D-1 | 2-OMe | Me | OCH₂CHF₂ | 168-169 |
| 3-116 | Me | Me | D-1 | 2-OMe | H | OCH₂CF₃ | 216-218 |
| 3-117 | Me | Me | D-1 | 2-OMe | Me | OCH₂CF₃ | ∗1 |
| 3-118 | Me | Me | D-1 | 2-OMe | H | OCH₂Ph | 156-158 |
| 3-119 | Me | Me | D-1 | 2-OEt | H | OCH₂Ph | 165-166 |
| 3-120 | Me | Me | D-1 | 2-OEt | Me | OCH₂Ph | 167-168 |
| 3-121 | Me | Me | D-1 | 2-OMe | H | OCH₂CH₂OMe | 196-198 |
| 3-122 | Me | Me | D-1 | 2-OMe | Me | OCH₂CH₂OMe | ∗1 |
| 3-123 | Me | Me | D-1 | 2-OEt | H | OCH₂CH₂OMe | 127-129 |
| 3-124 | Me | Me | D-1 | 2-OEt | Me | OCH₂CH₂OMe | 144-145 |
| 3-125 | Me | Me | D-1 | 2-OMe | H | OCH₂CH₂SMe | ∗1 |
| 3-126 | Me | Me | D-1 | 2-OMe | Me | OCH₂CH₂SMe | 113-114 |
| 3-127 | Me | Me | D-1 | 2-OMe | H | OCH₂CH₂S(O)₂Me | 257-259 |
| 3-128 | Me | Me | D-1 | 2-OMe | Me | OCH₂CH₂S(O)₂Me | 128-130 |
| 3-129 | Me | Me | D-1 | 2-OMe | H | OCH₂Pr-c | ∗1 |
| 3-130 | Me | Me | D-1 | 2-OMe | Me | OCH₂Pr-c | 133-135 |
| 3-131 | Me | Me | D-1 | 2-OMe | H | OCH₂[T-1-1] | 202-204 |
| 3-132 | Me | Me | D-1 | 2-OMe | Me | OCH₂[T-1-1] | 125-127 |
| 3-133 | Me | Me | D-1 | 2-OMe | H | OCH₂[U-1a] | 221-224 |
| 3-134 | Me | Me | D-1 | 2-OMe | H | OS(O)₂NMe₂ | 261-263 |
| 3-135 | Me | Me | D-1 | 2-OMe | H | CH₂OH | 198-200 |
| 3-136 | Me | Me | D-1 | 2-OMe | Me | CH₂OH | 121-124 |
| 3-137 | Me | Me | D-1 | 2-OMe | H | CH₂OMe | 218-220 |
| 3-138 | Me | Me | D-1 | 2-OMe | Me | CH₂OMe | 171-173 |
| 3-139 | Me | Me | D-1 | 4-CF₃ | H | F | 222-224 |
| 3-140 | Me | Me | D-1 | 4-CF₃ | C(O)Pr-n | F | 144-146 |
| 3-141 | Me | Me | D-1 | 4-OCHF₂ | H | F | 176-178 |
| 3-142 | Me | Me | D-1 | 4-OCHF₂ | C(O)Pr-n | F | ∗1 |
| 3-143 | Me | Me | D-1 | 4-OCF₃ | H | F | 139-141 |
| 3-144 | Me | Me | D-1 | 4-OCF₃ | C(O)Pr-n | F | 120-122 |
| 3-145 | Me | Me | D-1 | 4-CF₃ | H | Cl | 247-249 |
| 3-146 | Me | Me | D-1 | 4-CF₃ | C(O)Pr-n | Cl | 157-159 |
| 3-147 | Me | Me | D-1 | 4-OCHF₂ | H | Cl | 198-200 |
| 3-148 | Me | Me | D-1 | 4-OCHF₂ | C(O)Pr-n | Cl | ∗1 |
| 3-149 | Me | Me | D-1 | 4-OCF₃ | H | Cl | 187-189 |
| 3-150 | Me | Me | D-1 | 4-OCF₃ | C(O)Pr-n | Cl | 123-126 |
| 3-151 | Me | Me | D-1 | 4-OCHF₂ | H | Me | 120-122 |
| 3-152 | Me | Me | D-1 | 4-OCHF₂ | C(O)Pr-n | Me | ∗1 |
| 3-153 | Me | Me | D-1 | 2-OMe | CH₂CN | OMe | 152-154 |
| 3-154 | Me | Me | D-1 | 2-OMe | CH₂C≡CH | OMe | 171-173 |
| 3-155 | Me | Me | D-1 | 2-OMe | C(O)C(Me)=CH₂ | OMe | ∗1 |
| 3-156 | Me | Me | D-1 | 2-OMe | C(O)Hex-c | OMe | 155-157 |
| 3-157 | Me | Me | D-1 | 2-OMe | C(O)CH(Me)Ph | OMe | ∗1 |
| 3-158 | Me | Me | D-1 | 2-OMe | C(O)[Ph-2-OMe] | OMe | ∗1 |
| 3-159 | Me | Me | D-1 | 4-OCHF₂ | H | OMe | 234-236 |
| 3-160 | Me | Me | D-1 | 4-OCHF₂ | C(O)Pr-n | OMe | 180-182 |
| 3-161 | Me | Me | D-1 | 2-OMe | H | OS(O)₂CF₃ | 237-238 |
| 3-162 | Me | Me | D-1 | 2-OMe | Me | OS(O)₂CF₃ | 188-189 |
| 3-163 | Me | Me | D-1 | 2-OMe | H | SMe | 233-234 |
| 3-164 | Me | Me | D-1 | 2-OMe | Me | SMe | 112-114 |
| 3-165 | Me | Me | D-1 | 2-OMe | H | S(O)₂Me | 268-270 |
| 3-166 | Me | Me | D-1 | 2-OMe | Me | S(O)₂Me | 197-198 |
| 3-167 | Me | Me | D-1 | 2-OMe | Me | SCH₂CH₂C(O)OEt | ∗1 |
| 3-168 | Me | Me | D-1 | 4-OCF₃ | H | Me | 136-138 |
| 3-169 | Me | Me | D-1 | 4-OCF₃ | C(O)Pr-n | Me | 116-118 |
| 3-170 | Me | Me | D-1 | 4-OCF₃ | H | OMe | 200-202 |
| 3-171 | Me | Me | D-1 | 2-OMe | Me | S (T-3) | ∗1 |
| 3-172 | Me | Me | D-1 | 2-OMe | Me | OH | ∗1 |

**Table 6**

| 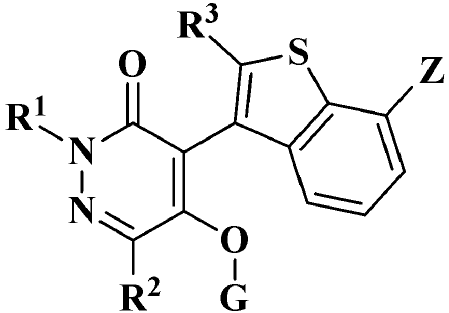 | | | | | | | |
|---|---|---|---|---|---|---|---|
| No. | R¹ | R² | R³ | (Y¹) | G | Z | mp (°C) |
| 4-001 | Me | Me | D-1 | 4-F | H | F | 245-247 |
| 4-002 | Me | Me | D-1 | 4-F | C(O)Pr-n | F | ∗1 |
| 4-003 | Me | Me | D-1 | 2-OMe | H | F | 220-221 |
| 4-004 | Me | Me | D-1 | 2-OMe | C(O)Pr-n | F | ∗1 |
| 4-005 | Me | Me | D-1 | 4-OMe | H | F | 237-239 |
| 4-006 | Me | Me | D-1 | 4-OMe | C(O)Pr-n | F | ∗1 |
| 4-007 | Me | Me | D-1 | 2-OEt | H | F | 171-173 |
| 4-008 | Me | Me | D-1 | 2-OEt | C(O)Pr-n | F | ∗1 |
| 4-009 | Me | Me | D-1 | 4-OPr-n | H | F | 131-133 |
| 4-010 | Me | Me | D-1 | 4-OPr-n | C(O)Pr-n | F | ∗1 |
| 4-011 | Me | Me | D-1 | 2-OMe-4-F | H | F | 232-234 |
| 4-012 | Me | Me | D-1 | 2-OMe-4-F | C(O)Pr-n | F | ∗1 |
| 4-013 | Me | Me | D-1 | 3,4-(OMe)₂ | H | F | 156-158 |
| 4-014 | Me | Me | D-1 | 3,4-(OMe)₂ | C(O)Pr-n | F | 166-168 |
| 4-015 | Me | Me | D-1 | 4-F | H | Cl | 230-232 |
| 4-016 | Me | Me | D-1 | 4-F | C(O)Pr-n | Cl | ∗1 |
| 4-017 | Me | Me | D-1 | 4-C1 | H | Cl | 118-120 |
| 4-018 | Me | Me | D-1 | 4-C1 | C(O)Pr-n | Cl | ∗1 |
| 4-019 | Me | Me | D-1 | 4-Me | H | Cl | 153-155 |
| 4-020 | Me | Me | D-1 | 4-Me | C(O)Pr-n | Cl | ∗1 |
| 4-021 | Me | Me | D-1 | 2-OMe | H | Cl | 231-233 |
| 4-022 | Me | Me | D-1 | 2-OMe | C(O)Pr-n | Cl | ∗1 |
| 4-023 | Me | Me | D-1 | 4-OMe | H | Cl | 151-153 |
| 4-024 | Me | Me | D-1 | 4-OMe | C(O)Pr-n | Cl | ∗1 |
| 4-025 | Me | Me | D-1 | 2-OEt | H | Cl | 216-218 |
| 4-026 | Me | Me | D-1 | 2-OEt | C(O)Pr-n | Cl | ∗1 |
| 4-027 | Me | Me | D-1 | 4-OPr-n | H | Cl | 140-142 |
| 4-028 | Me | Me | D-1 | 4-OPr-n | C(O)Pr-n | Cl | ∗1 |
| 4-029 | Me | Me | D-1 | 4-CF₃ | H | Cl | 213-217 |
| 4-030 | Me | Me | D-1 | 4-CF₃ | C(O)Pr-n | Cl | ∗1 |
| 4-031 | Me | Me | D-1 | 4-CN | H | Cl | 169-171 |
| 4-032 | Me | Me | D-1 | 4-CN | C(O)Pr-n | Cl | ∗1 |
| 4-033 | Me | Me | D-1 | 2-OMe-4-F | H | Cl | 260-262 |
| 4-034 | Me | Me | D-1 | 2-OMe-4-F | C(O)Pr-n | Cl | ∗1 |
| 4-035 | Me | Me | D-1 | 2-OEt-4-F | H | Cl | 210-212 |
| 4-036 | Me | Me | D-1 | 2-OEt-4-F | C(O)Pr-n | Cl | ∗1 |
| 4-037 | Me | Me | D-1 | 2-F-4-CF₃ | H | Cl | 249-251 |
| 4-038 | Me | Me | D-1 | 2-F-4-CF₃ | C(O)Pr-n | Cl | ∗1 |
| 4-039 | Me | Me | D-1 | 2-OMe | H | OMe | ∗1 |
| 4-040 | Me | Me | D-1 | 4-C1 | H | F | 270-272 |
| 4-041 | Me | Me | D-1 | 4-C1 | C(O)Pr-n | F | 115-117 |
| 4-042 | Me | Me | D-1 | 4-Pr-i | H | F | 183-185 |
| 4-043 | Me | Me | D-1 | 4-Pr-i | C(O)Pr-n | F | ∗1 |
| 4-044 | Me | Me | D-1 | 4-CF₃ | H | F | 132-134 |
| 4-045 | Me | Me | D-1 | 4-CF₃ | C(O)Pr-n | F | ∗1 |
| 4-046 | Me | Me | D-1 | 4-OCHF₂ | H | F | 91-93 |
| 4-047 | Me | Me | D-1 | 4-OCHF₂ | C(O)Pr-n | F | ∗1 |
| 4-048 | Me | Me | D-1 | 4-OCF₃ | H | F | 167-169 |
| 4-049 | Me | Me | D-1 | 4-OCF₃ | C(O)Pr-n | F | ∗1 |
| 4-050 | Me | Me | D-1 | 4-SMe | H | F | 125-127 |
| 4-051 | Me | Me | D-1 | 4-SMe | C(O)Pr-n | F | 148-150 |
| 4-052 | Me | Me | D-1 | 4-CN | H | F | 155-157 |
| 4-053 | Me | Me | D-1 | 4-CN | C(O)Pr-n | F | ∗1 |
| 4-054 | Me | Me | D-1 | 3-F-4-Cl | H | F | 276-278 |
| 4-055 | Me | Me | D-1 | 3-F-4-Cl | C(O)Pr-n | F | ∗1 |
| 4-056 | Me | Me | D-1 | - | H | Cl | 166-170 |
| 4-057 | Me | Me | D-1 | - | C(O)Pr-n | Cl | 120-122 |
| 4-058 | Me | Me | D-1 | 2-F | H | Cl | 219-221 |
| 4-059 | Me | Me | D-1 | 2-F | C(O)Pr-n | Cl | ∗1 |
| 4-060 | Me | Me | D-1 | 2-Me | H | Cl | 223-225 |
| 4-061 | Me | Me | D-1 | 2-Me | C(O)Pr-n | Cl | 126-128 |
| 4-062 | Me | Me | D-1 | 2-Et | H | Cl | 259-261 |
| 4-063 | Me | Me | D-1 | 2-Et | C(O)Pr-n | Cl | 87-89 |
| 4-064 | Me | Me | D-1 | 3-OMe | H | Cl | 196-198 |
| 4-065 | Me | Me | D-1 | 3-OMe | C(O)Pr-n | Cl | ∗1 |
| 4-066 | Me | Me | D-1 | 3-OEt | H | Cl | 201-203 |
| 4-067 | Me | Me | D-1 | 3-OEt | C(O)Pr-n | Cl | ∗1 |
| 4-068 | Me | Me | D-1 | 3-OBu-n | H | Cl | 156-158 |
| 4-069 | Me | Me | D-1 | 3-OBu-n | C(O)Pr-n | Cl | ∗1 |
| 4-070 | Me | Me | D-1 | 2-CF₃ | H | Cl | 230-232 |
| 4-071 | Me | Me | D-1 | 2-CF₃ | C(O)Pr-n | Cl | 87-89 |
| 4-072 | Me | Me | D-1 | 3-CF₃ | H | Cl | 222-224 |
| 4-073 | Me | Me | D-1 | 3-CF₃ | C(O)Pr-n | Cl | ∗1 |
| 4-074 | Me | Me | D-1 | 4-OCHF₂ | H | Cl | 113-115 |
| 4-075 | Me | Me | D-1 | 4-OCHF₂ | C(O)Pr-n | Cl | ∗1 |
| 4-076 | Me | Me | D-1 | 2-OCF₃ | H | Cl | 280-282 |
| 4-077 | Me | Me | D-1 | 2-OCF₃ | C(O)Pr-n | Cl | ∗1 |
| 4-078 | Me | Me | D-1 | 4-OCF₃ | H | Cl | 228-230 |
| 4-079 | Me | Me | D-1 | 4-OCF₃ | C(O)Pr-n | Cl | ∗1 |
| 4-080 | Me | Me | D-1 | 2-OH | C(O)Pr-n | Cl | ∗1 |
| 4-081 | Me | Me | D-1 | 2-CH₂OMe | H | Cl | 202-204 |
| 4-082 | Me | Me | D-1 | 2-CH₂OMe | C(O)Pr-n | Cl | 93-95 |
| 4-083 | Me | Me | D-1 | 2-OCH₂OMe | H | Cl | 190-192 |
| 4-084 | Me | Me | D-1 | 2-OCH₂OMe | C(O)Pr-n | Cl | 143-145 |
| 4-085 | Me | Me | D-1 | 2-OCH₂Ph | C(O)Pr-n | Cl | ∗1 |
| 4-086 | Me | Me | D-1 | 2-SMe | H | Cl | 244-246 |
| 4-087 | Me | Me | D-1 | 2-SMe | C(O)Pr-n | Cl | ∗1 |
| 4-088 | Me | Me | D-1 | 4-SMe | H | Cl | 135-137 |
| 4-089 | Me | Me | D-1 | 4-SMe | C(O)Pr-n | Cl | 160-161 |
| 4-090 | Me | Me | D-1 | 2-SEt | H | Cl | 208-210 |
| 4-091 | Me | Me | D-1 | 2-SEt | C(O)Pr-n | Cl | 98-100 |
| 4-092 | Me | Me | D-1 | 3-SEt | H | Cl | 184-186 |
| 4-093 | Me | Me | D-1 | 3-SEt | C(O)Pr-n | Cl | ∗1 |
| 4-094 | Me | Me | D-1 | 4-SEt | H | Cl | 186-188 |
| 4-095 | Me | Me | D-1 | 4-SEt | C(O)Pr-n | Cl | ∗1 |
| 4-096 | Me | Me | D-1 | 4-SPr-i | H | Cl | 184-186 |
| 4-097 | Me | Me | D-1 | 4-SPr-i | C(O)Pr-n | Cl | ∗1 |
| 4-098 | Me | Me | D-19a | - | H | Cl | 256-258 |
| 4-099 | Me | Me | D-19a | - | C(O)Pr-n | Cl | 106-108 |
| 4-100 | Me | Me | D-1 | 4-F | H | Me | 214-216 |
| 4-101 | Me | Me | D-1 | 4-F | C(O)Pr-n | Me | ∗1 |
| 4-102 | Me | Me | D-1 | 4-Cl | H | Me | 209-215 |
| 4-103 | Me | Me | D-1 | 4-C1 | C(O)Pr-n | Me | 85-87 |
| 4-104 | Me | Me | D-1 | 4-Br | H | Me | 254-256 |
| 4-105 | Me | Me | D-1 | 4-Br | C(O)Pr-n | Me | ∗1 |
| 4-106 | Me | Me | D-1 | 2-OMe | H | Me | 224-226 |
| 4-107 | Me | Me | D-1 | 2-OMe | C(O)Pr-n | Me | 131-133 |
| 4-108 | Me | Me | D-1 | 3-OMe | H | Me | 191-193 |
| 4-109 | Me | Me | D-1 | 3-OMe | C(O)Pr-n | Me | ∗1 |
| 4-110 | Me | Me | D-1 | 4-OMe | H | Me | 170-172 |
| 4-111 | Me | Me | D-1 | 4-OMe | C(O)Pr-n | Me | 104-106 |
| 4-112 | Me | Me | D-1 | 2-OEt | H | Me | 193-195 |
| 4-113 | Me | Me | D-1 | 2-OEt | C(O)Pr-n | Me | ∗1 |
| 4-114 | Me | Me | D-1 | 3-OEt | H | Me | 180-182 |
| 4-115 | Me | Me | D-1 | 3-OEt | C(O)Pr-n | Me | ∗1 |
| 4-116 | Me | Me | D-1 | 2-SMe | H | Me | 219-221 |
| 4-117 | Me | Me | D-1 | 2-SMe | C(O)Pr-n | Me | ∗1 |
| 4-118 | Me | Me | D-1 | 4-SMe | H | Me | 143-145 |
| 4-119 | Me | Me | D-1 | 4-SMe | C(O)Pr-n | Me | 163-165 |
| 4-120 | Me | Me | D-1 | 2-SEt | H | Me | 199-201 |
| 4-121 | Me | Me | D-1 | 2-SEt | C(O)Pr-n | Me | 90-92 |
| 4-122 | Me | Me | D-1 | 3-SEt | H | Me | 172-174 |
| 4-123 | Me | Me | D-1 | 3-SEt | C(O)Pr-n | Me | ∗1 |
| 4-124 | Me | Me | D-1 | 4-SEt | H | Me | 115-117 |
| 4-125 | Me | Me | D-1 | 4-SEt | C(O)Pr-n | Me | 114-116 |
| 4-126 | Me | Me | D-1 | 4-CF₃ | H | Me | 234-236 |
| 4-127 | Me | Me | D-1 | 4-CF₃ | C(O)Pr-n | Me | 138-140 |
| 4-128 | Me | Me | D-1 | 4-OCHF₂ | H | Me | 102-104 |
| 4-129 | Me | Me | D-1 | 4-OCHF₂ | C(O)Pr-n | Me | 123-125 |
| 4-130 | Me | Me | D-1 | 4-OCF₃ | H | Me | 210-212 |
| 4-131 | Me | Me | D-1 | 4-OCF₃ | C(O)Pr-n | Me | ∗1 |

**Table 7**

| 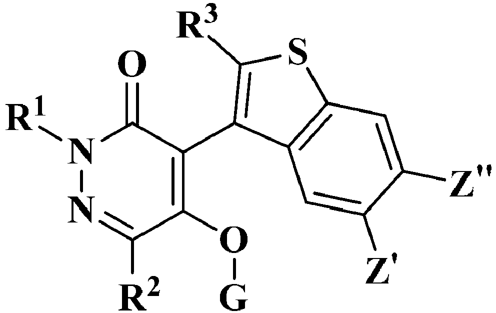 | | | | | | | | |
|---|---|---|---|---|---|---|---|---|
| No. | R¹ | R² | R³ | (Y¹) | G | Z′ | Zʺ | mp(°C) |
| 5-001 | Me | Me | D-1 | 4-F | H | F | F | 248-250 |
| 5-002 | Me | Me | D-1 | 4-F | C(O)Pr-n | F | F | 157-159 |
| 5-003 | Me | Me | D-1 | 4-Me | H | F | F | 226-228 |
| 5-004 | Me | Me | D-1 | 4-Me | C(O)Pr-n | F | F | 153-155 |
| 5-005 | Me | Me | D-1 | 4-Pr-i | H | F | F | 153-156 |
| 5-006 | Me | Me | D-1 | 4-Pr-i | C(O)Pr-n | F | F | 76-78 |
| 5-007 | Me | Me | D-1 | 4-CF₃ | H | F | F | 259-261 |
| 5-008 | Me | Me | D-1 | 4-CF₃ | C(O)Pr-n | F | F | 182-184 |
| 5-009 | Me | Me | D-1 | 4-CH₂OMe | H | F | F | 201-203 |
| 5-010 | Me | Me | D-1 | 4-CH₂OMe | C(O)Pr-n | F | F | ∗1 |
| 5-011 | Me | Me | D-1 | 2-OMe | H | F | F | 237-238 |
| 5-012 | Me | Me | D-1 | 2-OMe | C(O)Pr-n | F | F | 167-169 |
| 5-013 | Me | Me | D-1 | 2-OMe | Me | F | F | 171-172 |
| 5-014 | Me | Me | D-1 | 4-OMe | H | F | F | 124-127 |
| 5-015 | Me | Me | D-1 | 4-OMe | C(O)Pr-n | F | F | 172-174 |
| 5-016 | Me | Me | D-1 | 2-OEt | H | F | F | 220-222 |
| 5-017 | Me | Me | D-1 | 2-OEt | C(O)Pr-n | F | F | 146-148 |
| 5-018 | Me | Me | D-1 | 2-0Pr-i | H | F | F | 178-180 |
| 5-019 | Me | Me | D-1 | 4-0Pr-i | H | F | F | 127-130 |
| 5-020 | Me | Me | D-1 | 4-0Pr-i | C(O)Pr-n | F | F | 146-148 |
| 5-021 | Me | Me | D-1 | 2-OCF₃ | H | F | F | 246-248 |
| 5-022 | Me | Me | D-1 | 2-OCF₃ | C(O)Pr-n | F | F | 147-149 |
| 5-023 | Me | Me | D-1 | 4-OCH₂C(O)Me | H | F | F | 86-90 |
| 5-024 | Me | Me | D-1 | 4-O[U-4a] | H | F | F | 134-138 |
| 5-025 | Me | Me | D-1 | 4-CN | H | F | F | 295-297 |
| 5-026 | Me | Me | D-1 | 4-CN | C(O)Pr-n | F | F | 167-169 |
| 5-027 | Me | Me | D-1 | 4-C(O)OH | H | F | F | 251-254 |
| 5-028 | Me | Me | D-1 | 4-C(O)OMe | C(O)Pr-n | F | F | 78-80 |
| 5-029 | Me | Me | D-1 | 2-OMe-4-F | H | F | F | 250-252 |
| 5-030 | Me | Me | D-1 | 2-OMe-4-F | C(O)Pr-n | F | F | 176-178 |
| 5-031 | Me | Me | D-1 | 2-OEt-4-F | H | F | F | 220-222 |
| 5-032 | Me | Me | D-1 | 2-OEt-4-F | C(O)Pr-n | F | F | 122-124 |
| 5-033 | Me | Me | D-1 | 3,4-(OMe)₂ | H | F | F | 131-133 |
| 5-034 | Me | Me | D-1 | 3,4-(OMe)₂ | C(O)Pr-n | F | F | 165-167 |
| 5-035 | Me | Me | D-22a | - | H | F | F | 209-211 |
| 5-036 | Me | Me | D-22a | - | C(O)Pr-n | F | F | 174-176 |
| 5-037 | Me | Me | D-24a | - | H | F | F | 192-194 |
| 5-038 | Me | Me | D-24a | - | C(O)Pr-n | F | F | 198-200 |
| 5-039 | Me | Me | D-1 | 4-F | H | C1 | C1 | 160-163 |
| 5-040 | Me | Me | D-1 | 4-F | C(O)Pr-n | C1 | C1 | 197-198 |
| 5-041 | Me | Me | D-1 | 2-OMe | H | C1 | C1 | 125-127 |
| 5-042 | Me | Me | D-1 | 2-OMe | C(O)Pr-n | C1 | C1 | ∗1 |
| 5-043 | Me | Me | D-1 | 4-OMe | H | C1 | C1 | 235-237 |
| 5-044 | Me | Me | D-1 | 4-OMe | C(O)Pr-n | C1 | C1 | 89-91 |
| 5-045 | Me | Me | D-1 | 2-OEt | H | C1 | C1 | 244-246 |
| 5-046 | Me | Me | D-1 | 2-OEt | C(O)Pr-n | C1 | C1 | 131-133 |
| 5-047 | Me | Me | D-1 | 4-CF₃ | H | C1 | C1 | 290-291 |
| 5-048 | Me | Me | D-1 | 4-CF₃ | C(O)Pr-n | C1 | C1 | 198-200 |
| 5-049 | Me | Me | D-1 | 4-CN | H | C1 | C1 | 171-173 |
| 5-050 | Me | Me | D-1 | 4-CN | C(O)Pr-n | C1 | C1 | 186-188 |
| 5-051 | Me | Me | D-1 | 2-OMe-4-F | H | C1 | C1 | 224-226 |
| 5-052 | Me | Me | D-1 | 2-OMe-4-F | C(O)Pr-n | C1 | C1 | ∗1 |
| 5-053 | Me | Me | D-1 | 2-OEt-4-F | H | C1 | C1 | 263-265 |
| 5-054 | Me | Me | D-1 | 2-OEt-4-F | C(O)Pr-n | C1 | C1 | ∗1 |
| 5-055 | Me | Me | D-1 | - | H | OMe | OMe | 138-140 |
| 5-056 | Me | Me | D-1 | - | C(O)Pr-n | OMe | OMe | 176-178 |
| 5-057 | Me | Me | D-1 | 4-F | H | OMe | OMe | 161-163 |
| 5-058 | Me | Me | D-1 | 4-F | C(O)Pr-n | OMe | OMe | 201-203 |
| 5-059 | Me | Me | D-1 | 2-OMe | H | OMe | OMe | 116-118 |
| 5-060 | Me | Me | D-1 | 2-OMe-4-F | H | OMe | OMe | 131-133 |
| 5-061 | Me | Me | D-1 | 2-OMe-4-F | C(O)Pr-n | OMe | OMe | ∗1 |
| 5-062 | Me | Me | D-1 | 3, 4- (OMe) | ₂ H | OMe | OMe | 149-151 |
| 5-063 | Me | Me | D-1 | 3,4-(OMe)₂ | C(O)Pr-n | OMe | OMe | 151-153 |
| 5-064 | Me | Me | D-1 | 2-OMe | H | F | OMe | 250-252 |
| 5-065 | Me | Me | D-1 | 2-OMe | H | F | OEt | 245-246 |
| 5-066 | Me | Me | D-1 | 2-OMe | Et | F | OEt | 171-173 |
| 5-067 | Me | Me | D-1 | 4-F | H | C1 | OMe | 189-191 |
| 5-068 | Me | Me | D-1 | 2-OMe | H | C1 | OMe | 139-141 |
| 5-069 | Me | Me | D-1 | 2-OMe | C(O)Pr-n | C1 | OMe | 201-203 |
| 5-070 | Me | Me | D-1 | 4-OMe | H | C1 | OMe | 154-156 |
| 5-071 | Me | Me | D-1 | 4-OMe | C(O)Pr-n | C1 | OMe | 190-192 |
| 5-072 | Me | Me | D-1 | 2-OEt | H | C1 | OMe | 126-128 |
| 5-073 | Me | Me | D-1 | 2-OEt | C(O)Pr-n | C1 | OMe | 181-183 |
| 5-074 | Me | Me | D-1 | 4-CN | H | C1 | OMe | 115-117 |
| 5-075 | Me | Me | D-1 | 2-OMe-4-F | H | C1 | OMe | 241-243 |
| 5-076 | Me | Me | D-1 | 2-OMe-4-F | C(O)Pr-n | C1 | OMe | ∗1 |
| 5-077 | Me | Me | D-1 | 2-OEt-4-F | H | C1 | OMe | 218-220 |
| 5-078 | Me | Me | D-1 | 4-F | H | [Ph-4-F] | OMe | 187-189 |
| 5-079 | Me | Me | D-1 | 2-OEt | H | [Ph-2-0Et] | OMe | 138-140 |
| 5-080 | Me | Me | D-1 | 2-OEt | C(O)Pr-n | [Ph-2-OEt] | OMe | ∗1 |
| 5-081 | Me | Me | D-1 | 2-OEt-4-F | H | [Ph-2-OEt-4-F] | OMe | 156-159 |
| 5-082 | Me | Me | D-1 | 4-C1 | H | F | F | 123-125 |
| 5-083 | Me | Me | D-1 | 4-C1 | Bn | F | F | 156-157 |
| 5-084 | Me | Me | D-1 | 4-OCHF₂ | H | F | F | 222-224 |
| 5-085 | Me | Me | D-1 | 4-OCHF₂ | C(O)Pr-n | F | F | 142-144 |
| 5-086 | Me | Me | D-1 | 4-OCHF₂ | H | C1 | C1 | 127-129 |
| 5-087 | Me | Me | D-1 | 4-OCHF₂ | C(O)Pr-n | C1 | C1 | 152-154 |
| 5-088 | Me | Me | D-1 | 4-F | H | Me | Me | 262-264 |
| 5-089 | Me | Me | D-1 | 4-F | C(O)Pr-n | Me | Me | 203-205 |
| 5-090 | Me | Me | D-1 | 2-0Me | H | Me | Me | 220-222 |
| 5-091 | Me | Me | D-1 | 2-OMe | C(O)Pr-n | Me | Me | 160-162 |
| 5-092 | Me | Me | D-1 | 4-OMe | H | Me | Me | 142-144 |
| 5-093 | Me | Me | D-1 | 4-OMe | C(O)Pr-n | Me | Me | 160-162 |
| 5-094 | Me | Me | D-1 | 2-OEt | H | Me | Me | 235-237 |
| 5-095 | Me | Me | D-1 | 2-OEt | C(O)Pr-n | Me | Me | 74-76 |
| 5-096 | Me | Me | D-1 | 4-CF₃ | H | Me | Me | 168-170 |
| 5-097 | Me | Me | D-1 | 4-CF₃ | C(O)Pr-n | Me | Me | 151-153 |
| 5-098 | Me | Me | D-1 | 4-OCHF₂ | H | Me | Me | 169-171 |
| 5-099 | Me | Me | D-1 | 4-OCHF₂ | C(O)Pr-n | Me | Me | 144-145 |
| 5-100 | Me | Me | D-1 | 4-CN | H | Me | Me | 182-184 |
| 5-101 | Me | Me | D-1 | 2-OMe-4-F | H | Me | Me | 234-236 |
| 5-102 | Me | Me | D-1 | 2-OMe-4-F | C(O)Pr-n | Me | Me | 181-182 |
| 5-103 | Me | Me | D-1 | 2-OEt-4-F | H | Me | Me | 202-204 |
| 5-104 | Me | Me | D-1 | 2-OEt-4-F | C(O)Pr-n | Me | Me | 87-90 |
| 5-105 | Me | Me | D-1 | 4-OCF₃ | H | F | F | 149-151 |
| 5-106 | Me | Me | D-1 | 4-OCF₃ | C(O)Pr-n | F | F | 88-90 |
| 5-107 | Me | Me | D-1 | 4-OCF₃ | H | C1 | C1 | 207-209 |
| 5-108 | Me | Me | D-1 | 4-OCF₃ | C(O)Pr-n | C1 | C1 | 182-184 |
| 5-109 | Me | Me | D-1 | 4-OCF₃ | H | Me | Me | 147-149 |
| 5-110 | Me | Me | D-1 | 4-OCF₃ | C(O)Pr-n | Me | Me | 115-117 |

**Table 8**

| 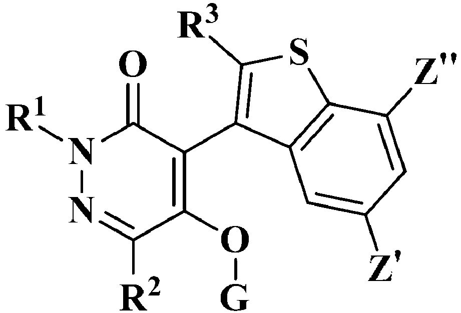 | | | | | | | | |
|---|---|---|---|---|---|---|---|---|
| No. | R¹ | R² | R³ | (Y¹) | G | Z' | Zʺ | mp(°C) |
| 6-001 | Me | Me | D-1 | 2-OMe | H | F | F | 122-124 |
| 6-002 | Me | Me | D-1 | 2-OMe | H | Me | Me | 221-223 |

**Table 9**

| | | | | | | | | |
|---|---|---|---|---|---|---|---|---|
| 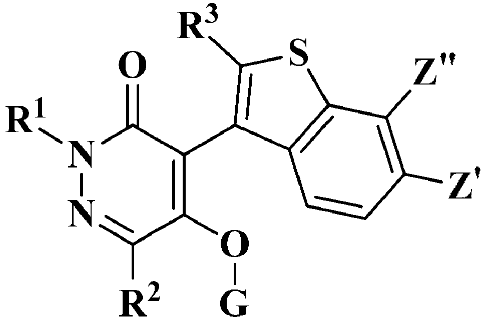 | | | | | | | | |

| No. | R¹ | R² | R³ | (Y¹) | G | Z′ | Zʺ | mp (°C) |
|---|---|---|---|---|---|---|---|---|
| 7-001 | Me | Me | D-1 | 2-OMe | H | OMe | C1 | 229-231 |
| 7-002 | Me | Me | D-1 | 2-OMe | H | OMe | Br | 236-238 |
| 7-003 | Me | Me | D-1 | 2-OMe | H | OEt | C1 | 223-225 |
| 7-004 | Me | Me | D-1 | 2-OEt | H | OEt | C1 | 203-205 |

**Table 10**

| | | | | | | |
|---|---|---|---|---|---|---|
| 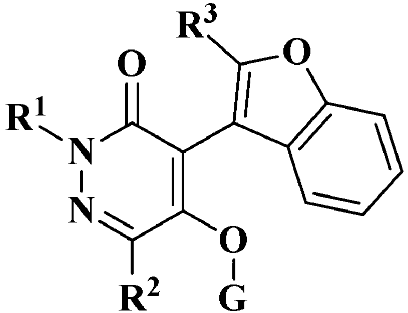 | | | | | | |

| No. | R¹ | R² | R³ | (Y¹) | G | mp (°C) |
|---|---|---|---|---|---|---|
| 8-001 | Me | Me | D-1 | 2-OMe-4-F | H | 235-237 |
| 8-002 | Me | Me | D-24a | - | H | 257-258 |
| 8-003 | Me | Me | D-1 | 4-OPr-n | H | 229-231 |
| 8-004 | Me | Me | D-1 | 4-OCH₂CH=CH₂ | H | 221-223 |
| 8-005 | Me | Me | D-1 | 4-OCH₂CF₃ | H | 234-236 |
| 8-006 | Me | Me | D-1 | 4-OH | Me | 251-253 |
| 8-007 | Me | Me | D-1 | 4-OCH₂CH₂OMe | H | 214-216 |
| 8-008 | Me | Me | D-1 | 4-OCH₂CH₂SMe | H | 94-96 |
| 8-009 | Me | Me | D-1 | 4-OBn | Me | ∗1 |

**Table 11**

| | | | | | | | |
|---|---|---|---|---|---|---|---|
| 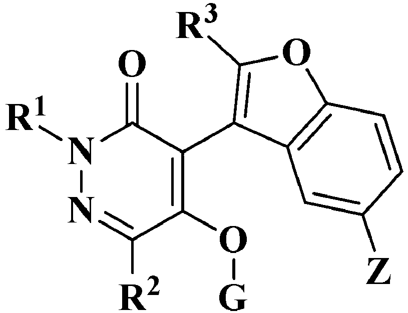 | | | | | | | |

| No. | R¹ | R² | R³ | (Y¹) | G | Z | mp (°C) |
|---|---|---|---|---|---|---|---|
| 9-001 | Me | Me | D-1 | 2-OMe | H | C1 | 246-247 |
| 9-002 | Me | Me | D-1 | 4-OMe | H | C1 | 269-270 |
| 9-003 | Me | Me | D-1 | 2-OEt | H | C1 | 243-245 |
| 9-004 | Me | Me | D-1 | 4-F | H | Me | 284-286 |
| 9-005 | Me | Me | D-1 | 4-F | C(O)Pr-n | Me | ∗1 |
| 9-006 | Me | Me | D-1 | 2-OMe | H | Me | 177-179 |
| 9-007 | Me | Me | D-1 | 4-OMe | H | Me | 232-234 |
| 9-008 | Me | Me | D-1 | 4-OMe | C(O)Pr-n | Me | ∗1 |
| 9-009 | Me | Me | D-1 | 2-OMe-4-F | H | Me | 237-239 |
| 9-010 | Me | Me | D-1 | 2-OEt-4-F | C(O)Pr-n | Me | ∗1 |
| 9-011 | Me | Me | D-1 | 2-OEt-4-F | H | Me | 211-213 |
| 9-012 | Me | Me | D-24a | - | H | Me | 198-200 |
| 9-013 | Me | Me | D-24a | - | C(O)Pr-n | Me | 171-173 |
| 9-014 | Me | Me | D-1 | 4-F | H | F | 293-295 |
| 9-015 | Me | Me | D-1 | 4-F | C(O)Pr-n | F | 135-136 |
| 9-016 | Me | Me | D-1 | 4-C1 | H | F | 262-263 |
| 9-017 | Me | Me | D-1 | 4-Me | H | F | 214-216 |
| 9-018 | Me | Me | D-1 | 4-Me | C(O)Pr-n | F | 144-146 |
| 9-019 | Me | Me | D-1 | 2-OMe | H | F | 226-228 |
| 9-020 | Me | Me | D-1 | 4-OMe | H | F | 248-250 |
| 9-021 | Me | Me | D-1 | 4-OEt | H | F | 230-232 |
| 9-022 | Me | Me | D-1 | 4-OEt | C(O)Pr-n | F | 133-134 |
| 9-023 | Me | Me | D-1 | 4-OPr-n | H | F | 182-184 |
| 9-024 | Me | Me | D-1 | 4-OPr-n | C(O)Pr-n | F | 103-105 |
| 9-025 | Me | Me | D-1 | 4-OPr-i | H | F | 162-164 |
| 9-026 | Me | Me | D-1 | 4-OPr-i | C(O)Pr-n | F | ∗1 |
| 9-027 | Me | Me | D-1 | 4-OBu-i | H | F | 155-157 |
| 9-028 | Me | Me | D-1 | 4-OBu-i | C(O)Pr-n | F | 132-134 |
| 9-029 | Me | Me | D-1 | 4-CF₃ | H | F | 201-203 |
| 9-030 | Me | Me | D-1 | 4-CF₃ | C(O)Pr-n | F | 187-189 |
| 9-031 | Me | Me | D-1 | 4-OCHF₂ | H | F | 186-188 |
| 9-032 | Me | Me | D-1 | 4-OCHF₂ | C(O)Pr-n | F | 160-162 |
| 9-033 | Me | Me | D-1 | 4-OCF₃ | H | F | 174-176 |
| 9-034 | Me | Me | D-1 | 4-OCF₃ | C(O)Pr-n | F | 140-142 |
| 9-035 | Me | Me | D-1 | 4-OCH₂CF₃ H | | F | 168-170 |
| 9-036 | Me | Me | D-1 | 4-OCH₂CF₃ | C(O)Pr-n | F | 193-195 |
| 9-037 | Me | Me | D-1 | 4-SMe | H | F | 151-153 |
| 9-038 | Me | Me | D-1 | 4-SMe | C(O)Pr-n | F | 178-180 |
| 9-039 | Me | Me | D-1 | 4-SEt | H | F | 186-188 |
| 9-040 | Me | Me | D-1 | 4-SEt | C(O)Pr-n | F | ∗1 |
| 9-041 | Me | Me | D-1 | 4-SPr-i | H | F | 155-157 |
| 9-042 | Me | Me | D-1 | 4-SPr-i | C(O)Pr-n | F | ∗1 |
| 9-043 | Me | Me | D-1 | 4-CH_{z}OMe | H | F | 192-194 |
| 9-044 | Me | Me | D-1 | 4-CH_{z}OMe | C(O)Pr-n | F | 129-131 |
| 9-045 | Me | Me | D-1 | 4-OCH₂CH₂OMe | H | F | 242-244 |
| 9-046 | Me | Me | D-1 | 4-OCH₂CH₂OMe | C(O)Pr-n | F | 115-117 |
| 9-047 | Me | Me | D-7 | 5-C1 | H | F | 115-117 |
| 9-048 | Me | Me | D-7 | 5-C1 | C(O)Pr-n | F | ∗1 |
| 9-049 | Me | Me | D-1 | 4-OPr-n | H | Me | 224-225 |
| 9-050 | Me | Me | D-1 | 4-OPr-n | C(O)Pr-n | Me | 114-116 |
| 9-051 | Me | Me | D-1 | 4-OBu-i | H | Me | 199-201 |
| 9-052 | Me | Me | D-1 | 4-OBu-i | C(O)Pr-n | Me | 99-101 |
| 9-053 | Me | Me | D-1 | 4-OCHF₂ | H | Me | 206-208 |
| 9-054 | Me | Me | D-1 | 4-OCHF₂ | C(O)Pr-n | Me | 117-119 |
| 9-055 | Me | Me | D-1 | 4-SMe | H | Me | 229-231 |
| 9-056 | Me | Me | D-1 | 4-SMe | C(O)Pr-n | Me | 149-151 |
| 9-057 | Me | Me | D-1 | 4-SEt | H | Me | 203-205 |
| 9-058 | Me | Me | D-1 | 4-SEt | C(O)Pr-n | Me | ∗1 |
| 9-059 | Me | Me | D-1 | 4-SPr-i | H | Me | 180-182 |
| 9-060 | Me | Me | D-1 | 4-SPr-i | C(O)Pr-n | Me | ∗1 |
| 9-061 | Me | Me | D-1 | 4-CH_{z}OMe | H | Me | 225-227 |
| 9-062 | Me | Me | D-1 | 4-CH_{z}OMe | C(O)Pr-n | Me | 91-93 |
| 9-063 | Me | Me | D-1 | 4-OCH₂CH₂OMe | H | Me | 203-205 |
| 9-064 | Me | Me | D-1 | 4-OCH₂CH₂OMe | C(O)Pr-n | Me | 78-80 |

**Table 12**

| | | | | | | | |
|---|---|---|---|---|---|---|---|
| 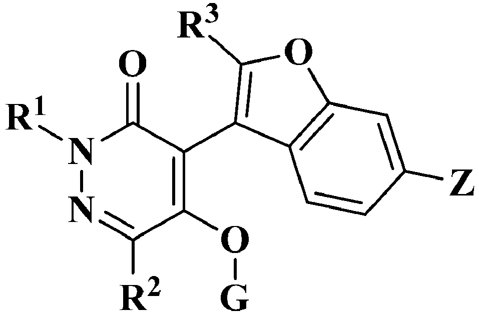 | | | | | | | |

| No. | R¹ | R² | R³ | (Y¹) | G | Z | mp(°C) |
|---|---|---|---|---|---|---|---|
| 10-001 | Me | Me | D-1 | 2-OMe | H | OMe | 204-206 |

**Table 13**

| | | | | | | | |
|---|---|---|---|---|---|---|---|
| 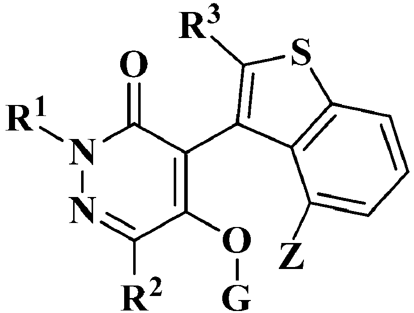 | | | | | | | |

| No. | R¹ | R² | R³ | (Y¹) | G | Z | mp (°C) |
|---|---|---|---|---|---|---|---|
| 11-001 | Me | Me | D-1 | 4-C1 | H | F | 238-239 |

**Table 14**

| 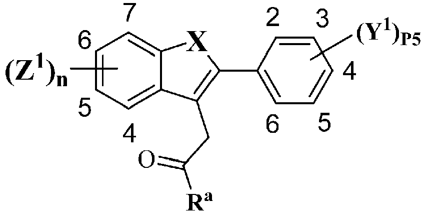 | | | | | |
|---|---|---|---|---|---|
| No. | X | R^{a} | (Z¹)ₙ | (Y¹)_{P5} | mp(°C) |
| 12-001 | S | OH | 4-F | 4-C1 | 238-239 |
| 12-002 | S | OH | 5-F | 4-C1 | 202-204 |
| 12-003 | S | OH | 5-F | 4-OCHF₂ | 203-204 |
| 12-004 | S | OH | 6-F | 2-OMe | 156-157 |
| 12-005 | S | OH | 5-C1 | 2-OMe-4-F | 186-188 |
| 12-006 | S | OH | 5-Me | - | 192-194 |
| 12-007 | S | OH | 4-OMe | 2-OMe | 176-178 |
| 12-008 | S | OH | 5-OMe | - | 179-181 |
| 12-009 | S | OH | 5-OMe | 2-OMe | 157-158 |
| 12-010 | S | OH | 6-OMe | 2-OMe | 232-234 |
| 12-011 | S | OH | 6-OMe | 2-OMe-4-F | 196-198 |
| 12-012 | 0 | OH | 6-OMe | 2-OMe | 131-133 |
| 12-013 | S | OH | 6-OBn | 2-OMe | 179-181 |
| 12-014 | S | OH | 5,7-F₂ | 2-OMe | 184-186 |
| 12-015 | S | OH | 5,7-Me₂ | 2-OMe | 171-173 |
| 12-016 | S | OMe | 5-F | 4-OCHF₂ | 111-113 |
| 12-017 | S | OMe | 5-C1 | 2-OMe-4-F | 95-96 |
| 12-018 | S | OMe | 5-Me | - | ∗1 |
| 12-019 | S | OMe | 5-OMe | - | 84-86 |
| 12-020 | S | OMe | 6-OMe | 2-OMe | ∗1 |
| 12-021 | S | OMe | 5,7-F₂ | 2-OMe | 144-146 |
| 12-022 | S | OMe | 5,7-Me₂ | 2-OMe | 103-105 |
| 12-023 | S | OEt | 5-F | 4-C1 | 49-51 |
| 12-024 | S | OEt | 6-F | 2-OMe | ∗1 |
| 12-025 | S | OEt | 6-OMe | 2-OMe | ∗1 |
| 12-026 | S | OEt | 6-OMe | 2-OMe-4-F | 104-106 |
| 12-027 | S | OEt | 6-OBn | 2-OMe | 88-90 |
| 12-028 | S | N(Me)NH₂ | 4-F | 4-C1 | 158-160 |
| 12-029 | S | N(Me)NH₂ | 5-F | 4-C1 | 131-133 |
| 12-030 | S | N(Me)NH₂ | 5-F | 4-OCHF₂ | 103-104 |
| 12-031 | S | N(Me)N=C(Me)C₂Et | 4-F | 4-C1 | 124-126 |
| 12-032 | S | N(Me)N=C(Me)CO₂Et | 5-F | 4-C1 | 125-127 |
| 12-033 | S | N(Me)N=C(Me)CO₂Et | 5-F | 4-OCHF₂ | 100-101 |
| 12-034 | S | N(Me)N=C(Me)CO₂Et | 6-F | 2-OMe | 120-122 |
| 12-035 | S | N(Me)N=C(Me)CO₂Et | 5-C1 | 2-OMe-4-F | 126-128 |
| 12-036 | S | N (Me) N=C (Me) CO₂Et | 5-Me | - | ∗1 |
| 12-037 | S | N(Me)N=C(Me)CO₂Et | 6-OMe | 2-OMe | 109-111 |
| 12-038 | S | N(Me)N=C(Me)CO₂Et | 6-OMe | 2-OMe-4-F | 123-124 |
| 12-039 | S | N(Me)N=C(Me)CO₂Et | 7-OMe | 2-OMe | ∗1 |
| 12-040 | S | N(Me)N=C(Me)CO₂Et | 5,7-Me₂ | 2-OMe | ∗1 |

Table 15 shows the spectrum data of compounds (among the compounds shown in Tables 3 to 14) whose melting points are not described in the Tables.

**Table 15**

| No. | ¹H NMR |
|---|---|
| 1-006 | δ 7. 75-7. 85 (m, 1H), 7.25-7.40 (m, 5H), 7.10-7.20 (m, 2H) |
| | 3.78 (s, 3H), 2.62 (t, J=7.5Hz, 2H), 2.22 (s, 3H), |
| | 2.00-2.15 (m, 2H), 1. 55-1. 70 (m, 2H), 1. 30-1. 45 (m, 2H) |
| | 1. 15-1. 30 (m, 2H), 0.94 (t, J=7.2Hz, 3H), |
| | 0.56 (t, J=7.5Hz, 3H). |
| 1-016 | δ 7. 80-7. 90 (m, 1H), 7.40-7.50 (m, 1H), 7.20-7.40 (m, 4H), |
| | 6.80-7.00 (m, 2H), 3.95 (t, J=6. 7Hz, 2H), 3.77 (s, 3H), |
| | 3.37 (s, 3H), 2.12 (s, 3H), 1. 65-1. 85 (m, 2H), |
| | 0.99 (t, J=7.5Hz, 3H). |
| 1-021 | δ 7. 75-7.85 (m, 1H), 7.25-7.45 (m, 5H), 6.80-6.90 (m, 2H), |
| | 4.50-4.65 (m, 1H), 3.79 (s, 3H), 2.23 (s, 3H), |
| | 2.00-2.15 (m, 2H), 1.35 (dd, J=6. 1, 2.1Hz, 6H), |
| | 1.20-1.30 (m, 2H), 0.56 (t, J=7. 4Hz, 3H). |
| 1-023 | δ 7. 75-7.85 (m, 1H), 7.35-7.45 (m, 1H), 7.20-7.35 (m, 4H), |
| | 6.80-7.00 (m, 2H), 4.00 (t, J=6. 8Hz, 2H), 3.83 (s, 3H), |
| | 2.05-2.15 (m, 2H), 2.12 (s, 3H), 1. 70-1. 85 (m, 2H), |
| | 1.40-1.55 (m, 2H), 1. 25-1. 40 (m, 2H), |
| | 0.97 (t, J=7.7Hz, 3H), 0.64 (t, J=7.5Hz, 3H). |
| 1-025 | δ 7. 75-7.85 (m, 1H), 7.25-7.45 (m, 5H), 6.80-6.90 (m, 2H), |
| | 3.97 (t, J=6.6Hz, 2H), 3.79 (s, 3H), 2.23 (s, 3H), |
| | 1. 95-2. 20 (m, 2H), 1. 70-1. 85 (m, 2H), 1. 40-1. 60 (m, 2H), |
| | 1. 15-1. 35 (m, 2H), 0.98 (t, J=7. 4Hz, 3H), |
| | 0.56 (t, J=7. 4Hz, 3H). |
| 1-027 | δ 7. 75-7.85 (m, 1H), 7.25-7.45 (m, 5H), 6.80-6.90 (m, 2H), |
| | 3.96 (t, J=6.6Hz, 3H), 3.79 (s, 3H), 2.23 (s, 3H), |
| | 2.00-2.15 (m, 2H), 1. 70-1. 85 (m, 2H), 1. 35-1. 50 (m, 4H), |
| | 1.20-1.35 (m, 2H), 0.90-1.00 (m, 3H), |
| | 0.56 (t, J=7. 4Hz, 3H). |
| 1-029 | δ 7. 75-7. 85 (m, 1H), 7.25-7.45 (m, 5H), 6.80-6.90 (m, 2H), |
| | 3.96 (t, J=6.6Hz, 2H), 3.79 (s, 3H), 2.23 (s, 3H), |
| | 2.07 (td, J=7.3, 3. 5Hz, 2H), 1.70-1.85 (m, 2H), |
| | 1. 40-1. 55 (m, 2H), 1. 30-1. 40 (m, 4H), 1. 15-1. 30 (m, 2H), |
| | 0.85-0.95 (m, 3H), 0.56 (t, J=7. 4Hz, 3H). |
| 1-032 | δ 7.80-7.90 (m, 1H), 7.50-7.60 (m, 2H), 7.30-7.45 (m, 3H), |
| | 7. 15-7.25 (m, 2H), 3.76 (s, 3H), 2.26 (s, 3H), |
| | 1.95-2.20 (m, 2H), 1.15-1.35 (m, 2H), 0.54 (t, J=7.5Hz, 3H). |
| 1-035 | δ 7. 75-7.85 (m, 1H), 7.25-7.45 (m, 6H), |
| | 7.10 (td, J=7.2, 1.9Hz, 1H), 3.77 (s, 3H), 2.44 (s, 3H), |
| | 2.21 (t, J=7. 3Hz, 2H), 1. 25-1. 45 (m, 2H), |
| | 0.65 (t, J=7.5Hz, 3H). |
| 1-037 | δ 7. 75-7.85 (m, 1H), 7. 30-7. 45 (m, 5H), 7.15-7.25 (m, 2H), |
| | 3.78 (s, 3H), 2.49 (s, 3H), 2.24 (s, 3H), 2.00-2.15 (m, 2H), |
| | 1.20-1.35 (m, 2H), 0.55 (t, J=7. 3Hz, 3H). |
| 1-042 | δ 7. 90-8. 00 (m, 2H), 7.80-7.90 (m, 1H), 7. 55-7. 65 (m, 2H), |
| | 7.35-7.45 (m, 3H), 3.76 (s, 3H), 2.61 (s, 3H), |
| | 2.25 (s, 3H), 1. 95-2. 20 (m, 2H), 1. 15-1. 30 (m, 2H), |
| | 0.54 (t, J=7. 4Hz, 3H). |
| 1-044 | δ 7. 75-7.85 (m, 1H), 7.60-7.70 (m, 2H), 7.45-7.55 (m, 2H), |
| | 7.30-7.45 (m, 3H), 4.00 (s, 3H), 3.79 (s, 3H), 2.22 (s, 6H), |
| | 2.00-2.10 (m, 2H), 1. 20-1. 30 (m, 2H), 0.54 (t, J=7. 4Hz, 3H). |
| 1-047 | δ 7. 75-7.95 (m, 1H), 7.25-7.65 (m, 5H), 7.05-7.20 (m, 1H), |
| | 3.77 (s, 3H), 2.27 (s, 3H), 2.00-2.20 (m, 2H), |
| | 1.15-1.40 (m, 2H), 0.50-0.60 (m, 3H). |
| 1-052 | δ 7. 75-7.85 (m, 1H), 7.20-7.45 (m, 5H), 6.95 (t, J=8. 9Hz, 1H), |
| | 3.76 (s, 3H), 2.20-2.35 (m, 6H), 1. 95-2. 15 (m, 2H), |
| | 1.20-1.35 (m, 2H), 0.55 (t, J=7.5Hz, 3H). |
| 1-059 | δ 7. 75-7.85 (m, 1H), 7.40-7.50 (m, 1H), 7. 30-7. 40 (m, 2H), |
| | 7.13 (dd, J=8. 9, 3.1Hz, 1H), 6.95-7.05 (m, 1H), |
| | 6.84 (dd, J=9. 2, 4.4Hz, 1H), 3.83 (s, 3H), 3.70 (s, 3H), |
| | 2.14 (s, 3H), 2.11 (t, J=7. 4Hz, 2H), 1. 25-1. 40 (m, 2H), |
| | 0.64 (t, J=7. 3Hz, 3H). |
| 1-064 | δ 7. 75-7. 85 (m, 1H), 7. 30-7. 45 (m , 4H), |
| | 6.94 (dd, J=9. 9, 2. 4Hz, 1H), 6.78 (td, J=8. 3, 2.4Hz, 1H), |
| | 3.76 (s, 3H), 2.44 (s, 3H), 2.19 (t, J=7.2Hz, 2H), |
| | 2.16 (s, 3H), 1.25-1.40 (m, 2H), 0.64 (t, J=7. 3Hz, 3H). |
| 1-071 | δ 7. 75-7.85 (m, 1H), 7.40-7.50 (m, 1H), 7. 30-7. 40 (m, 2H), |
| | 6.95-7.00 (m, 1H), 6.80-6.90 (m, 2H), 3.81 (s, 3H), |
| | 3.71 (s, 3H), 3.68 (s, 3H), 2.05-2.15 (m, 5H), |
| | 1.20-1.40 (m, 2H), 0.63 (t, J=7. 3Hz, 3H). |
| 1-075 | δ 7. 75-7.85 (m, 1H), 7.25-7.40 (m, 3H), 7.14 (s, 2H), |
| | 3.79 (s, 3H), 3.73 (s, 3H), 2.25 (s, 6H), 2.34 (s, 3H), |
| | 2.08 (dt, J=7. 2, 2.1Hz, 2H), 1.15-1.30 (m, 2H), |
| | 0.56 (t, J=7.5Hz, 3H). |
| 1-077 | δ 8.30 (d, J=2. 7Hz, 1H), 7. 75-7. 85 (m, 1H), |
| | 7.69 (dd, J= 8.5, 2. 7Hz, 1H), 7.30-7.40 (m, 3H), |
| | 6.71 (d, J=8. 5Hz, 1H), 4.38 (q, J=7. 2Hz, 2H), 3.78 (s, 3H), |
| | 2.24 (s, 3H), 1. 95-2. 15 (m, 2H), 1.41 (t, J=7.2Hz, 3H), |
| | 1. 15-1. 30 (m, 2H), 0.55 (t, J=7.5, 3H). |
| 1-080 | δ 7. 75-7.85 (m, 1H), 7.54 (d, J=8. 0Hz, 1H), 7.35-7.45 (m, 1H), |
| | 7.25-7.35 (m, 2H), 6.29 (d, J=8. 0Hz, 1H), 3.95 (s, 3H), |
| | 3.89 (s, 3H), 3.83 (s, 3H), 2.16 (s, 3H), 2.05-2.15 (m, 2H), |
| | 1.20-1.35 (m, 2H), 0.63 (t, J=7. 4Hz, 3H). |
| 1-091 | δ 7. 75-7.85 (m, 1H), 7.25-7.40 (m, 3H), 7.03 (d, J=2.1Hz, 1H), |
| | 6.95 (dd, J=8.4, 2.1Hz, 1H), 6.82 (d, J=8.4Hz, 1H), |
| | 4.25 (s, 4H), 3.82 (s, 3H), 3.53 (s, 3H), 2.28 (s, 3H). |
| 1-092 | δ 7. 75-7.85 (m, 1H), 7.25-7.40 (m, 3H), 7.04 (d, J=2.1Hz, 1H), |
| | 6.95 (dd, J=8.4, 2.1Hz, 1H), 6.81 (d, J=8.4Hz, 1H), |
| | 4.26 (s, 4H), 3.83 (s, 3H), 2.25 (s, 3H), 1.15 (s, 9H). |
| 1-093 | δ 7. 70-7. 80 (m, 1H), 7.25-7.50 (m, 5H), 7.00-7.20 (m, 2H), |
| | 6.85-6.95 (m, 2H), 6.80 (d, J=8. 7Hz, 1H), 4.27 (s, 4H), |
| | 3.86 (s, 3H), 2.29 (s, 3H), 2.25 (s, 3H). |
| 1-094 | δ 7. 75-7. 85 (m, 1H), 7. 30-7. 45 (m, 3H), 7.05 (d, J=2.1Hz, 1H), |
| | 6.95 (dd, J=8.4, 2.1Hz, 1H), 6.84 (d, J=8.4Hz, 1H), |
| | 4.28 (s, 4H), 3.80 (s, 3H), 2.47 (s, 3H), 2.28 (s, 3H). |
| 1-098 | δ 7. 75-7. 85 (m, 1H), 7.72 (s, 1H), 7.63 (s, 1H), |
| | 7.30-7.40 (m, 3H), 3.83 (s, 3H), 2.30 (s, 3H), |
| | 2.10-2.20 (m, 2H), 1. 20-1. 35 (m, 2H), 0.58 (t, J=7. 5Hz, 3H). |
| 1-100 | δ 7. 80-7. 90 (m, 1H), 7.25-7.40 (m, 5H), 7.00-7.10 (m, 1H), |
| | 3.76 (s, 3H), 2.28 (s, 3H), 2.00-2.20 (m, 2H), |
| | 1.15-1.30 (m, 2H), 0.53 (t, J=7. 4Hz, 3H). |
| 1-102 | δ 7. 75-7.85 (m, 1H), 7.25-7.40 (m, 4H), |
| | 7.24 (dd, J=8. 3, 2.1Hz, 1H), 6.74 (d, J=8. 3Hz, 1H), |
| | 4.60 (t, J=8. 7Hz, 2H), 3.79 (s, 3H), 3.22 (t, J=8. 7Hz, 2H), |
| | 2.24 (s, 3H), 2.00-2.15 (m, 2H), 1. 15-1. 30 (m, 2H), |
| | 0.56 (t, J=7. 4Hz, 3H). |
| 1-104 | δ 7. 80-7. 90 (m, 1H), 7.45-7.50 (m, 1H), 7. 30-7. 40 (m, 2H), |
| | 7.10-7.25 (m, 2H), 6.82 (t, J=7.7Hz, 1H), 4.40-4.60 (m, 2H), |
| | 3.77 (s, 3H), 3.35 (s, 3H), 3.22 (t, J=8. 7Hz, 2H), |
| | 2.17 (s, 3H). |
| 1-109 | δ 9.01 (d, J=2. 4Hz, 1H), 8.30 (d, J=2.1Hz, 1H), |
| | 8.07 (d, J=8. 5Hz, 1H), 7.83-7.89 (m, 1H), |
| | 7.80 (dd, J=8. 2Hz, 1. 0Hz, 1H), |
| | 7.70 (td, J=7.7Hz, 1.4Hz, 1H), 7.44-7.57 (m, 2H), |
| | 7.35-7.41 (m, 2H), 3.75 (s, 3H), 3.37 (s, 3H), |
| | 2. 19 (s, 3H). |
| 1-111 | δ 7. 75-7.90 (m, 1H), 7.20-7.60 (m, 5H), 6.80-7.05 (m, 2H), |
| | 5.85-6.00 (m, 1H), 5.05-5.45 (m, 2H), 4.40-4.70 (m, 2H), |
| | 3.73 (s, 3H), 3.35 (s, 3H), 2.11 (s, 3H). |
| 1-114 | δ 7.80-7.90 (m, 1H), 7.40-7.55 (m, 1H), 7.20-7.40 (m, 4H), |
| | 6.80-6.95 (m, 2H), 3.76-3.90 (m, 2H), 3.74 (s, 3H), |
| | 3.40 (s, 3H), 2.12 (s, 3H), 1. 10-1. 25 (m, 1H), |
| | 0.45-0.65 (m, 2H), 0.20-0.45 (m, 2H). |
| 1-121 | δ7.80 7.90 (m, 1H), 7.40-7.50 (m, 1H), 7.20-7.40 (m, 4H), |
| | 6.85-6.95 (m, 2H), 3.95-4.10 (m, 2H), 3.75 and 3.74 (s, 3H), |
| | 3.38 and 3.35 (s, 3H), 2.12 and 2.11 (s, 3H), |
| | 1. 90-2. 10 (m, 1H), 1. 40-1. 60 (m, 1H), 1. 10-1. 35 (m, 1H). |
| 1-122 | δ7. 80-7. 90 (m, 1H), 7.25-7.50 (m, 6H), 6.90-7.05 (m, 2H), |
| | 5.05- 5.25 (m, 2H), 3.71 (s, 3H), 3.30 (s, 3H), |
| | 2.12 (s, 3H). |
| 1-126 | δ7.75-7.85 (m, 1H), 7.25-7.45 (m, 5H), 7.15-7.25 (m, 2H), |
| | 3.77 (s, 3H), 2. 85-2. 95 (m, 1H), 2.22 (s, 3H), |
| | 2.00-2.10 (m, 2H), 1. 15-1. 30 (m, 8H), 0.54 (t, J=7.3Hz, 3H). |
| 1-128 | δ7. 75-7.85 (m, 1H), 7.30-7.50 (m, 7H), |
| | 6.70 (dd, J=17. 6, 10.9 Hz, 1H), 5.77 (dd, J=17. 6, 0.8Hz, 1H), |
| | 5.27 (dd, J=10. 9, 0.8Hz, 1H), 3.78 (s, 3H), 2.22 (s, 3H), |
| | 2.00-2.10 (m, 2H), 1. 15-1. 30 (m, 2H), 0.54 (t, J=7.4Hz, 3H). |
| 1-131 | δ7. 75-7. 90 (m, 1H), 7. 30-7. 40 (m, 5H), 7.15-7.25 (m, 2H), |
| | 6.39 (brs, 1H), 4.38 (s, 2H), 3.63 (s, 3H), 3.33 (s, 3H), |
| | 2. 16 (s, 3H). |
| 1-133 | δ7. 75-7. 85 (m, 1H), 7.25-7.45 (m, 10H), 6.80-6.95 (m, 2H), |
| | 6.00 (brs, 1H), 5.04 (s, 2H), 3.68 (s, 3H), 2.18 (s, 3H). |
| 1-135 | δ7. 75-7.85 (m, 1H), 7.25-7.45 (m, 10H), 6.90-7.00 (m, 2H), |
| | 5.06 (s, 2H), 4.64 (d, J=5.5 Hz, 1H), 4.42 (d, J=5.5Hz, 1H), |
| | 3.76 (s, 3H), 3.16 (s, 3H), 2.30 (s, 3H). |
| 1-136 | δ7. 75-7. 85 (m, 1H), 7. 30-7. 40 (m, 5H), 6.85-6.95 (m, 2H), |
| | 6.24 (brs, 1H), 4.67 (d, J=2.4Hz, 2H), 3.67 (s, 3H), |
| | 2.54 (t, J=2.4Hz, 1H), 2.18 (s, 3H). |
| 1-138 | δ7. 80-7. 85 (m, 1H), 7. 30-7. 40 (m, 5H), 6. 75-6. 85 (m, 2H), |
| | 6.13 (s, 1H), 3.90-4.15 (m, 2H), 3.69 (s, 3H), 2.19 (s, 3H), |
| | 1. 95-2. 15 (m, 1H), 1. 50-1. 65 (m, 1H), 1. 15-1. 35 (m, 1H). |
| 1-139 | δ7. 75-7. 90 (m, 1H), 7. 30-7. 40 (m, 5H), 6.80-6.90 (m, 2H), |
| | 5.95-6.15 (m, 2H), 5.35-5.45 (m, 1H), 5.25-5. 35 (m, 1H), |
| | 4.50-4.55 (m, 2H), 3.71 (s, 3 H), 2.19 (s, 3 H). |
| 1-142 | δ7.80-7.85 (m, 1H), 7.30-7.40 (m, 5H), 6.75-6.90 (m, 2H), |
| | 5.95 (brs, 1H), 3.79 (d, J=6.8 Hz, 2H), 3.73 (s, 3H), |
| | 2.20 (s, 3H), 1. 20-1. 35 (m, 1H), |
| | 0. 60-0. 70 (m, 2H), 0. 30-0. 40 (m, 2H). |
| 1-143 | δ7.80-7.85 (m, 1H), 7.30-7.40 (m, 5H), 6.75-6.85 (m, 2H), |
| | 6.14 (s, 1H), 4.50 (s, 2H), 3.71 (s, 3H), 2.25 (s, 3H), |
| | 2.21 (s, 3H). |
| 1-144 | δ7.75-7.85 (m, 1H), 7.44 (s, 1H), 7.30-7.40 (m, 5H), |
| | 6.80-6.90 (m, 2H), 6.33 (s, 1H), 5.13 (d, J=0.7 Hz, 2H), |
| | 3.72 (s, 3H), 2.22 (s, 3H). |
| 1-145 | δ10.43 (brs, 1H), 7.90- 8.05 (m, 1H), 7.20-7.45 (m, 5H), |
| | 6.90-7.00 (m, 2H), 5.75 (s, 1H), 4.62 (s, 2H), |
| | 3.85-4.05 (m, 2H), 3.57 (s, 3H), 2.19 (s, 3H). |
| 1-146 | δ7.75-7.85 (m, 1H), 7.20-7.40 (m, 6H), 7.05-7.15 (m, 2H), |
| | 3.44 (s, 3H), 3.10 (s, 3H), 2.10 (s, 3H). |
| 1-147 | δ7.80-7.90 (m, 1H), 7.30-7.45 (m, 5H), 7.10-7.20 (m, 2H), |
| | 6.40 (s, 1H), 3.59 (s, 3H), 2.19 (s, 3H). |
| 1-148 | δ7.80-7.90 (m, 1H), 7.30-7.45 (m, 5H), 6.85-6.95 (m, 2H), |
| | 5.57 (s, 1H), 4. 05-4. 20 (m, 2H), 3.81 (s, 3H), |
| | 3.70-3.80 (m, 2H), 3.45 (s, 3H), 2.24 (s, 3H). |
| 1-150 | δ7.77-7.83 (m, 1H), 7.45 (d, J=8.9Hz, 2H), 7.28-7.39 (m, 5H), |
| | 7.14 (t, J=7.5Hz, 1H), 7.02-7.08 (m, 2H), |
| | 6.95 (d, J=8.9Hz, 2H), 3.77 (s, 3H), 2.23 (s, 3H), |
| | 1. 97-2. 16 (m, 2H), 1. 17-1. 30 (m, 2H), 0.55 (t, J=7. 3Hz, 3H). |
| 1-154 | δ7.73-7.84 (m, 1H), 7.45-7.52 (m, 4H), 7. 36-7. 42 (m, 1H), |
| | 7.28-7.34 (m, 2H), 3.76 (s, 3H), 2.23 (s, 3H), |
| | 1. 98-2. 26 (m, 2H), 1. 13-1. 28 (m, 2H), 0.52 (t, J=7.5Hz, 3H), |
| | 0.26 (s, 9H). |
| 1-156 | δ7.75-7.85 (m, 1H), 7.40-7.50 (m, 2H), 7.30-7.40 (m, 2H), |
| | 7. 15-7.25 (m, 1H), 7. 10-7. 15 (m, 1H), 3.80 (s, 3H), |
| | 3.79 (s, 3H), 2.14 (s, 3H), 2.05-2.15 (m, 2H), |
| | 1.25-1.40 (m, 2H), 0.60 (t, J=7.5Hz, 3H). |
| 1-157 | δ7.80-7.86 (m, 1H), 7.46-7.53 (m, 2H), 7.30-7.42 (m, 3H), |
| | 6.74 (d, J=9.0Hz, 1H), 3.75 (s, 3H), 3.66 (s, 3H), |
| | 3.33 (s, 3H), 2.14 (s, 3H). |
| 1-161 | δ7.80-7.90 (m, 1H), 7. 30-7. 50 (m, 6H), 7.10-7.20 (m, 1H), |
| | 3.89 (s, 3H), 3.68 (s, 3H), 2.15 (td, J=7.2, 1. 8Hz, 2H), |
| | 1.25-1.40 (m, 2H), 0.61 (t, J=7.5Hz, 3H). |
| 1-163 | δ7.80-7.90 (m, 1H), 7.50-7.60 (m, 2H), 7.40-7.50 (m, 1H), |
| | 7.30-7.40 (m, 2H), 7.15-7.25 (m, 2H), 3.91 (s, 3H), |
| | 3.68 (s, 3H), 2.16 (td, J=7. 2, 2.1Hz, 2H), 1. 25-1. 40 (m, 2H), |
| | 0.61 (t, J=7.5Hz, 3H). |
| 1-165 | δ7. 80-7. 85 (m, 1H), 7. 30-7. 50 (m, 7H), 7.10-7.20 (m, 1H), |
| | 7.00-7.10 (m, 2H), 6.95-7.00 (m, 2H), 3.89 (s, 3H), |
| | 2.16 (td, J=7.2, 1. 5Hz, 2H), 1. 25-1. 40 (m, 2H), |
| | 0.63 (t, J=7.4Hz, 3H). |
| 1-175 | δ7.75-7.85 (m, 1H), 7.20-7.45 (m, 5H), |
| | 6.90-7.05 (m, 1H), 3.88 (s, 3H), 3.68 (s, 3H), |
| | 2.26 (d, J=2.1Hz, 3H), 2.15 (td, J=7.2, 1. 2Hz, 2H), |
| | 1.25-1.45 (m, 2H), 0.62 (t, J=7.3Hz, 3H). |
| 1-181 | δ7.75-7.80 (m, 1H), 7.35-7.45 (m, 1H), 7.20-7.35 (m, 3H), |
| | 6.40-6.50 (m, 2H), 3.82 (s, 6H), 3.74 (s, 3H), 3.69 (s, 3H), |
| | 2.14 (t, J=7.4Hz, 2H), 1. 30-1. 45 (m, 2H), |
| | 0.68 (t, J=7.5Hz, 3H). |
| 1-185 | δ7. 80-7. 85 (m, 1H), 7. 30-7. 50 (m, 3H), 6.69 (d, J=2.1Hz, 2H), |
| | 6.43 (t, J=2.1Hz, 1H), 3.89 (s, 3H), 3.75 (s, 6H), |
| | 3.69 (s, 3H), 2.17 (t, J=7.3Hz, 2H), 1. 25-1. 40 (m, 2H), |
| | 0.60 (t, J=7.5Hz, 3H). |
| 1-188 | δ7.80-7.90 (m, 1H), 7. 30-7. 45 (m, 3H), 6.80-6.90 (m, 2H), |
| | 5.81 (brs, 1H), 3.90 (s, 3H), 3.60 (s, 3H), 2.28 (s, 3H), |
| | 2.80 (s, 6H). |
| 1-189 | δ7.80-7.85 (m, 1H), 7.25-7.45 (m, 3H), 6.80-6.90 (m, 2H), |
| | 3.82 (s, 3H), 3.62 (s, 3H), 2.36 (s, 3H), 2. 25-2. 30 (m, 8H), |
| | 1.35-1.50 (m, 2H), 0.73 (t, J=7.5Hz, 3H). |
| 1-191 | δ7.75-7.85 (m, 1H), 7.60-7.65 (m, 1H), 7.40-7.45 (m, 1H), |
| | 7.25-7.40 (m, 3H), 6.50-6.55 (m, 1H), 3.93 (s, 3H), |
| | 3.73 (s, 3H), 2.10-2.20 (m, 2H), 1. 25-1. 40 (m, 2H), |
| | 0.60 (t, J=7.3Hz, 3H). |
| 1-195 | δ7.75-7.90 (m, 1H), 7.50-7.60 (m, 1H), 7.40-7.50 (m, 2H), |
| | 7.20-7.40 (m, 4H), 6.81 (d, J=1.0Hz, 1H), 3.95 (s, 3H), |
| | 3.77 (s, 3H), 2.14 (t, J=7.2Hz, 2H), 1. 20-1. 35 (m, 2H), |
| | 0.58 (t, J=7.5Hz, 3H). |
| 1-197 | δ7. 70-7.85 (m, 3H), 7.49 (s, 1H), 7.25-7.45 (m, 5H), |
| | 3.94 (s, 3H), 3.75 (s, 3H), 2.18 (t, J=7.2Hz, 2H), |
| | 1.25-1.40 (m, 2H), 0.60 (t, J=7.4Hz, 3H). |
| 1-200 | δ7.82-7.88 (m, 1H), 7. 34-7. 38 (m, 3H), 7.28 (d, J=8.9Hz, 2H), |
| | 6.70 (d, J=8.9Hz, 2H), 5.90 (brs, 1H), 5.55 (brs, 1H), |
| | 3.81 (s, 3H), 2.27 (s, 3H). |
| 1-202 | δ7.80-7.87 (m, 1H), 7.48 (d, J=8.5Hz, 2H), 7.31-7.42 (m, 3H), |
| | 7.09 (d, J=8.5Hz, 2H), 6.53 (t, J=73.6Hz, 1H), |
| | 4.74 (d, J=5.8Hz, 1H), 4.54 (d, J=5.8Hz, 1H), 3.75 (s, 3H), |
| | 3.42-3.48 (m, 2H), 3.24-3.30 (m, 2H), 3.23 (s, 3H), |
| | 2.29 (s, 3H). |
| 1-203 | δ7.75-7.85 (m, 1H), 7.20-7.40 (m, 5H), 6. 70-6. 80 (m, 3H), |
| | 6.06 (tt, J=55.2, 4.2Hz, 1H), 4.13 (td, J=13.1, 4.2Hz, 2H), |
| | 3.54 (s, 3H), 2.12 (s, 3H). |
| 1-204 | δ7.80-7.90 (m, 1H), 7.30-7.45 (m, 5H), 6.80-6.95 (m, 2H), |
| | 5.59 (s, 1H), 4.30 (t, J=6.3Hz, 2H), 3.78 (s, 3H), |
| | 3.64 (t, J=6.3 Hz, 2H), 2.24 (s, 3H). |
| 1-205 | δ7.75-7.90 (m, 1H), 7.25-7.45 (m, 5H), 6.80-6.90 (m, 2H), |
| | 4.45-4.75 (m, 2H), 4.29 (t, J=6.3Hz, 2H), 3.77 (s, 3H), |
| | 3.64 (t, J=6.3Hz, 2H), 3.40-3.50 (m, 2H), 3.25-3.35 (m, 2H), |
| | 3.24 (s, 3H), 2.28 (s, 3H). |
| 1-206 | δ7.80-7.90 (m, 1H), 7.30-7.45 (m, 5H), 6.80-6.90 (m, 2H), |
| | 5.54 (s, 1H), 4.20 (t, J=6.6 Hz, 2H), 3.79 (s, 3H), |
| | 2.55-2.75 (m, 2H), 2.24 (s, 3H). |
| 1-208 | δ7.80-7.90 (m, 1H), 7.30-7.45 (m, 5H), 6.80-6.95 (m, 2H), |
| | 5.60-5.70 (m, 1H), 5.05-5.15 (m, 1H), 4.95-5.05 (m, 1H), |
| | 4.43 (s, 2H), 3.78 (s, 3H), 2.23 (s, 3H), 1.83 (s, 3H). |
| 1-209 | δ7.75-7.85 (m, 1H), 7.25-7.45 (m, 5H), 6.85-6.90 (m, 2H), |
| | 4.95-5.15 (m, 2H), 4.45-4.75 (m, 2H), 4.43 (s, 2H), |
| | 3.77 (s, 3H), 3.40-3.50 (m, 2H), 3.25-3.30 (m, 2H), |
| | 3.23 (s, 3H), 2.28 (s, 3H), 1.83 (s, 3H). |
| 1-211 | δ7.80-7.90 (m, 1H), 7.30-7.40 (m, 5H), 6.80-6.90 (m, 2H), |
| | 5.80-5.95 (m, 1H), 5.60-5.80 (m, 2H), 4.40-4.50 (m, 2H), |
| | 3.75 (s, 3H), 2.21 (s, 3H), 1. 70-1. 80 (m, 3H). |
| 1-212 | δ7. 75-7.85 (m, 1H), 7.25-7.45 (m, 5H), 6.80-6.90 (m, 2H), |
| | 5.80-5.95 (m, 1H), 5.65-5.80 (m, 1H), 4.40-4.75 (m, 4H), |
| | 3.76 (s, 3H), 3.40-3.50 (m, 2H), 3.25-3.30 (m, 2H), |
| | 3.23 (s, 3H), 2.27 (s, 3H), 1. 70-1. 80 (m, 3H). |
| 1-214 | δ7.75-7.85 (m, 1H), 7.25-7.40 (m, 5H), 6.80-6.90 (m, 2H), |
| | 5.75-5.95 (m, 1H), 5.05-5.20 (m, 2H), 4.50-4.75 (m, 2H), |
| | 4.35-4.50 (m, 1H), 3.77 (s, 3H), 3.40-3.50 (m, 2H), |
| | 3.25-3.35 (m, 2H), 3.23 (s, 3H), 2.30-2.60 (m, 2H), |
| | 2.28 (s, 3H), 1.32 (d, J=6.1Hz, 3H). |
| 1-216 | δ7.75-7.85 (m, 1H), 7.25-7.45 (m, 5H), 6.80-6.95 (m, 2H), |
| | 5.95-6.05 (m, 1H), 5.65-5.70 (m, 1H), 4.45-4.80 (m, 4H), |
| | 3.77 (s, 3H), 3.40-3.50 (m, 2H), 3.25-3.35 (m, 2H), |
| | 3.24 (s, 3H), 2.28 (s, 3H). |
| 1-217 | δ7.79-7.87 (m, 1H), 7. 30-7. 41 (m, 5H), 6.92-6.99 (m, 2H), |
| | 6.82 (brs, 1H), 4.80-4.91 (m, 1H), 3.72-3.76 (m, 3H), |
| | 2.49-2.51 (m, 1H), 2.20-2.23 (m, 3H), 1.67 (d, J=6.5Hz, 3H). |
| 1-218 | δ7.77-7.85 (m, 1H), 7.27-7.44 (m, 5H), 6.93-7.00 (m, 2H), |
| | 4.81-4.91 (m, 1H), 4.72 (d, J=5.8Hz, 1H), |
| | 4.51 (d, J=5.8Hz, 1H), 3.75-3.78 (s, 3H), |
| | 3.41-3.47 (m, 2H), 3.24-3.30 (m, 2H), |
| | 3.23 (s, 3H), 2. 48-2. 50 (m, 1H), 2. 26-2. 29 (m, 3H), |
| | 1.66 (d, J=6.5Hz, 3H). |
| 1-220 | δ7. 75-7.85 (m, 1H), 7.25-7.45 (m, 5H), 6.90-6.95 (m, 2H), |
| | 4.45-4.75 (m, 4H), 3.77 (s, 3H), 3.40-3.50 (m, 2H), |
| | 3.25-3.35 (m, 2H), 3.23 (s, 3H), 2.28 (s, 3H), |
| | 1.87 (t, J=2.2Hz, 3H). |
| 1-221 | δ7.76-7.83 (m, 1H), 7.39 (d, J=8.9Hz, 2H), 7.25-7.37 (m, 3H), |
| | 6.86 (d, J=8.9Hz, 2H), 4.72 (d, J=5.8Hz, 1H), |
| | 4.51 (d, J=5.8Hz, 1H), 4.14 (t, J=6.8Hz, 2H), 3.76 (s, 3H), |
| | 3.41-3.47 (m, 2H), 3.24-3.30 (m, 2H), 3.22 (s, 3H), |
| | 2.86 (t, J=6.8Hz, 2H), 2.28 (s, 3H), 2.20 (s, 3H). |
| 1-223 | δ7.79-7.87 (m, 1H), 7. 30-7. 39 (m, 5H), 6.86 (d, J=8.9Hz, 2H), |
| | 5.75 (brs, 1H), 5.07 (t, J=4.8Hz, 1H), 4.06-4.16 (m, 2H), |
| | 3.93-4.02 (m, 2H), 3.83-3.92 (m, 2H), 3.77 (s, 3H), |
| | 2.22 (s, 3H), 2.09-2.19 (m, 2H). |
| 1-224 | δ7.76-7.86 (m, 1H), 7.27-7.41 (m, 5H), 6.85 (d, J=8.9Hz, 2H), |
| | 5.05-5.10 (m, 1H), 4.71 (d, J=5.8Hz, 1H), |
| | 4.58 (d, J=5.8Hz, 1H), 4.50 (d, J=5.8Hz, 1H), |
| | 4.08-4.15 (m, 2H), 3.93-4.02 (m, 2H), 3.83-3.92 (m, 2H), |
| | 3.76 (s, 3H), 3.40-3.48 (m, 2H), 3.24-3.31 (m, 2H), |
| | 3.23 (s, 3H), 2.27 (s, 3H), 2. 11-2. 20 (m, 2H). |
| 1-225 | δ7.75-7.85 (m, 1H), 7.25-7.75 (m, 5H), 6.85-6.95 (m, 2H), |
| | 4.45-4.75 (m, 2H), 4.30-4.40 (m, 2H), 4.15-4.25 (m, 2H), |
| | 3.77 (s, 3H), 3.40-3.50 (m, 2H), 3.25-3.35 (m, 2H), |
| | 3.23 (s, 3H), 1.88 (s, 6H), 1.86 (s, 3H). |
| 1-229 | δ7. 75-7.85 (m, 1H), 7.25-7.40 (m, 5H), 6.80-6.95 (m, 2H), |
| | 4.45-4.75 (m, 3H), 3.77 (d, J=0.9 Hz, 3H), 3.45-3.60 (m, 4H), |
| | 3.41 (s, 3H), 3.25-3.35 (m, 2H), 3.24 (s, 3H), |
| | 2.28 (d, J=1.2Hz, 3H), 1. 25-1. 35 (m, 3H). |
| 1-231 | δ7.75-7.85 (m, 1H), 7.25-7.40 (m, 5H), 6.80-6.90 (m, 2H), |
| | 4.45-4.75 (m, 2H), 3.95-4.00 (m, 2H), 3.77 (s, 3H), |
| | 3.47 (s, 3H), 3.35-3.50 (m, 3H), 3.25-3.35 (m, 2H), |
| | 3.23 (s, 3H), 2.28 (d, J=0.6 Hz, 3H), 1. 60-1. 75 (m, 2H), |
| | 0.99 (t, J=7.4Hz, 3H). |
| 1-233 | δ7.77-7.85 (m, 1H), 7.41 (d, J=8.9Hz, 2H), 7.26-7.38 (m, 3H), |
| | 6.86 (d, J=8.9Hz, 2H), 4.72 (d, J=5.8Hz, 1H), 4.63 (s, 2H), |
| | 4.51 (d, J=5.8Hz, 1H), 3.80 (s, 3H), 3.76 (s, 3H), |
| | 3.41-3.47 (m, 2H), 3.24-3.30 (m, 2H), 3.23 (s, 3H), |
| | 2.28 (s, 3H). |
| 1-235 | δ7.75-7.85 (m, 1H), 7.25-7.42 (m, 5H), 6.82 (d, J=8. 5Hz, 2H), |
| | 4.75 (q, J=6.8Hz, 1H), 4.71 (dd, J=5.6, 0.9Hz, 1H), |
| | 4.50 (dd, J=5.6, 0.9Hz, 1H), 3.76 (s, 3H), 3.73 (s, 3H), |
| | 3.40-3.47 (m, 2H), 3.23-3.30 (m, 2H), 3.22 (s, 3H), |
| | 2.27 (s, 3H), |
| | 1.61 (d, J=6.8Hz, 3H). |
| 1-236 | δ7.76-7.84 (m, 1H), 7.24-7.37 (m, 5H), 6.85 (d, J=8.9Hz, 2H), |
| | 6.80 (brs, 1H), 4. 77-4. 89 (m, 1H), 3. 54-3. 58 (m, 3H), |
| | 2. 14-2. 16 (m, 3H), 1.75 (d, J=6.8Hz, 3H). |
| 1-237 | δ7.78-7.85 (m, 1H), 7.46 (d, J=8. 5Hz, 2H), 7.28-7.41 (m, 3H), |
| | 6.96 (d, J=8.5Hz, 2H), 4. 83-4. 94 (m, 1H), 4. 69-4. 76 (m, 1H), |
| | 4.49-4.57 (m, 1H), 3.74-3.78 (m, 3H), 3.41-3.48 (m, 2H), |
| | 3.23-3.30 (m, 2H), 3.22 (s, 3H), 2.27-2.30 (m, 3H), |
| | 1. 72-1.80 (m, 3H). |
| 1-241 | δ7.75-7.90 (m, 1H), 7.20-7.40 (m, 7H), 6.33 (s, 1H), |
| | 5.85-6.00 (m, 1H), 5.15-5.35 (m, 2H), 4.47 (s, 2H), |
| | 3.95-4.05 (m, 2H), 3.61 (s, 3H), 2.14 (s, 3H). |
| 1-242 | δ7.80-7.90 (m, 1H), 7.30-7.45 (m, 5H), 7.15-7.25 (m, 2H), |
| | 3.72 (d, J=2.4Hz, 3H), 3.56 (s, 2H), 3.25-3.30 (m, 3H), |
| | 2. 14 (s, 3H). |
| 1-244 | δ7.80-7.90 (m, 1H), 7.25-7.45 (m, 7H), 3.76 (s, 3H), |
| | 3.73 (s, 2H), 3.32 (s, 3H), 2. 70-2. 90 (m, 1H), 2.20 (s, 3H), |
| | 1.20-1.30 (m, 6H). |
| 1-245 | δ7.80-7.90 (m, 1H), 7.20-7.45 (m, 7H), 5. 70-5. 90 (m, 1H), |
| | 5.62 (s, 1H), 5.00-5.20 (m, 2H), 3.76 (s, 3H), 3.65 (s, 2H), |
| | 3.03 (dt, J=7.2, 1.0Hz, 2H), 2.22 (s, 3H). |
| 1-246 | δ7.80-7.90 (m, 1H), 7.25-7.45 (m, 7H), 5.70-5.95 (m, 1H), |
| | 5. 00-5. 20 (m, 2H), 3.76 (s, 3H), 3.65 (s, 2H), 3.32 (s, 3H), |
| | 3.00-3.10 (m, 2H), 2.20 (s, 3H). |
| 1-247 | δ7.85-7.90 (m, 1H), 7.25-7.45 (m, 7H), 5.55 (s, 1H), |
| | 3.86 (s, 2H), 3.77 (s, 3H), 3.09 (d, J=2.6Hz, 2H), |
| | 2.29 (t, J=2.6Hz, 1H), 2.23 (s, 3H). |
| 1-248 | δ7. 80-7. 90 (m, 1H), 7.25-7.50 (m, 7H), 3.86 (s, 2H), |
| | 3.76 (s, 3H), 3.33 (s, 3H), 3.09 (d, J=2.7Hz, 2H), |
| | 2.25-2.30 (m, 1H), 2.21 (s, 3H). |
| 1-250 | δ7. 80-7. 90 (m, 1H), 7.25-7.50 (m, 7H), 3.82 (s, 2H), |
| | 3.76 (s, 3H), 3.33 (s, 3H), 2.91 (q, J=10. 1 Hz, 2H), |
| | 2.21 (s, 3H). |
| 1-251 | δ7.80-7.90 (m, 1H), 7.30-7.40 (m, 5H), 7.20-7.30 (m, 2H), |
| | 5.95 (brs, 1H), 3.73 (s, 2H), 3.72 (s, 3H), |
| | 3.48 (t, J=6.6 Hz, 2H), 3.31 (s, 3H), |
| | 2.59 (t, J=6.6Hz, 2H), 2.20 (s, 3H). |
| 1-252 | δ7.80-7.90 (m, 1H), 7.25-7.50 (m, 7H), 3.76 (s, 3H), |
| | 3.75 (s, 2H), 3.51 (t, J=6.5Hz, 2H), 3.33 (s, 3H), |
| | 3.32 (s, 3H), 2.61 (t, J=6.5Hz, 2H), 2.21 (s, 3H). |
| 1-253 | δ7.80-7.90 (m, 1H), 7.25-7.45 (m, 7H), 5.63 (brs, 1H), |
| | 3.76 (s, 3H), 3.74 (s, 2H), 2. 60-2. 70 (m, 4H), 2.23 (s, 3H), |
| | 2.06 (s, 3H). |
| 1-254 | δ7.80-7.90 (m, 1H), 7.25-7.50 (m, 7H), 3.76 (s, 3H), |
| | 3.74 (s, 2H), 3.32 (s, 3H), 2. 55-2. 70 (m, 4H), 2.21 (s, 3H), |
| | 2.07 (s, 3H). |
| 1-255 | δ7.80-7.90 (m, 1H), 7.25-7.45 (m, 7H), 5.99 (brs, 1H), |
| | 4.54 (s, 2H), 3.71 (s, 3H), 3.50-3.65 (m, 4H), 3.37 (s, 3H), |
| | 2. 19 (s, 3H). |
| 1-256 | δ7.80-7.90 (m, 1H), 7. 30-7. 50 (m, 7H), 4.57 (s, 2H), |
| | 3.76 (s, 3H), 3.55-3.70 (m, 4H), 3.40 (s, 3H), 3.31 (s, 3H), |
| | 2.20 (s, 3H). |
| 2-003 | δ7.75 (dd, J=8.9, 4.8Hz, 1H), 7.40-7.50 (m, 2H), |
| | 7.00-7.15 (m, 4H), 3.75 (s, 3H), 3.20-3.40 (m, 2H), |
| | 3.11 (s, 3H), 2.25-2.45 (m, 2H), 2.24 (s, 3H). |
| 2-004 | δ 7. 70-7. 80 (m, 1H), 7.40-7.50 (m, 2H), 6.95-7.15 (m, 4H), |
| | 3.85-4.05 (m, 2H), 3.79 (s, 3H), 2.26 (s, 3H), |
| | 1.02 (t, J=7.2Hz, 3H). |
| 2-005 | δ7.72 (dd, J=8.7, 4.9Hz, 1H), 7.35-7.45 (m, 4H), |
| | 6.95-7.25 (m, 6H), 3.82 (s, 3H), 2.32 (s, 3H). |
| 2-010 | δ7.75 (dd, J=8.9, 4.8Hz, 1H), 7.24-7.26 (m, 4H), |
| | 7.07-7.16 (m, 1H), 6.94-7.01 (m, 1H), 6.31 (brs, 1H), |
| | 3.63 (s, 3H), 2.19 (s, 3H). |
| 2-016 | δ7.70-7.74 (m, 1H), 7. 36-7. 40 (m, 2H), 7. 15-7. 18 (m, 2H), |
| | 7.01-7.10 (m, 2H), 3.77 (s, 3H), 2.65 (q, J=7.5Hz, 2H), |
| | 2.22 (s, 3H), 2.07-2.13 (m, 2H), 1. 21-1. 33 (m, 5H), |
| | 0.57 (t, J=7.5Hz, 3H). |
| 2-017 | δ7.70-7.75 (m, 1H), 7. 32-7. 41 (m, 4H), 7.01-7.10 (m, 2H), |
| | 3.76 (s, 3H), 2.24 (s, 3H), 2.08-2.14 (m, 2H), |
| | 1.31 (s, 9H), 1. 20-1. 29 (m, 2H), 0.56 (t, J=7.5Hz, 3H). |
| 2-019 | δ7.74 (dd, J=8.9, 4.6Hz, 1H), 7.35-7.50 (m, 4H), |
| | 7.00-7.15 (m, 2H), 6.70 (dd, J=17. 6, 10. 9Hz, 1H), |
| | 5.77 (dd, J=17. 6, 0.8Hz, 1H), 5.29 (dd, J=10. 9, 0.8Hz, 1H), |
| | 3.78 (s, 3H), 2.23 (s, 3H), 2. 05-2. 15 (m, 2H), |
| | 1.20-1.35 (m, 2H), 0.57 (t, J=7.5Hz, 3H). |
| 2-023 | δ7. 70-7. 80 (m, 1H), 7.40-7.50 (m, 2H), 7.25-7.35 (m, 2H), |
| | 7.00-7.15 (m, 2H), 4.46 (s, 2H), 3.78 (s, 3H), 3.40 (s, 3H), |
| | 2.21 (s, 3H), 2.10 (td, J=7.3, 2. 3Hz, 2H), 1. 20-1. 35 (m, 2H), |
| | 0.57 (t, J=7.4Hz, 3H). |
| 2-025 | δ7.65-7.75 (m, 1H), 7.30-7.40 (m, 2H), 7.00-7.15 (m, 2H), |
| | 6.85-7.00 (m, 2H), 3.83 (s, 3H), 3.72 (s, 3H), |
| | 2.05-2.15 (m, 5H), 1. 25-1. 40 (m, 2H), 0.65 (t, J=7.5Hz, 3H). |
| 2-027 | δ7. 65-7. 75 (m, 1H), 7.35-7.45 (m, 2H), 6.95-7.10 (m, 2H), |
| | 6.80-6.90 (m, 2H), 3.81 (s, 3H), 3.77 (s, 3H), 2.22 (s, 3H), |
| | 1. 95-2. 15 (m, 2H), 1. 20-1. 35 (m, 2H), 0.57 (t, J=7.5Hz, 3H). |
| 2-029 | δ7.65-7.75 (m, 1H), 7.20-7.35 (m, 2H), 7.00-7.15 (m, 2H), |
| | 6.85-6.95 (m, 2H), 4.05 (q, J=7.2Hz, 2H), 3.83 (s, 3H), |
| | 2.05-2.20 (m, 5H), 1. 25-1. 40 (m, 5H), 0.65 (t, J=7.2Hz, 3H). |
| 2-033 | δ7.72 (dd, J=8.7, 4.9Hz, 1H), 7.35-7.45 (m, 2H), |
| | 7.00-7.15 (m, 2H), 6.80-6.90 (m, 2H), |
| | 3.93 (t, J=6.7Hz, 2H), 3.79 (s, 3H), 2.24 (s, 3H), |
| | 2.05-2.15 (m, 2H), 1. 75-1. 90 (m, 2H), 1. 20-1. 35 (m, 2H), |
| | 1.04 (t, J=7.5Hz, 3H), 0.58 (t, J=7.3Hz, 3H). |
| 2-035 | δ7.72 (dd, J=8.7, 4.9Hz, 1H), 7.35-7.45 (m, 2H), |
| | 7.00-7.15 (m, 2H), 6.80-6.90 (m, 2H), |
| | 4. 50-4. 65 (m, 1H), 3.79 (s, 3H), 2.24 (s, 3H), |
| | 2.05-2.15 (m, 2H), 1. 25-1. 40 (m, 8H), |
| | 0.58 (t, J=7.5Hz, 3H). |
| 2-037 | δ7.72 (dd, J=8.7, 4.9Hz, 1H), 7.35-7.45 (m, 2H), |
| | 7.00-7.15 (m, 2H), 6.80-6.90 (m, 2H), |
| | 3.97 (t, J=6.7Hz, 2H), 3.79 (s, 3H), 2.24 (s, 3H), |
| | 2.05-2.15 (m, 2H), 1. 70-1. 85 (m, 2H), |
| | 1. 40-1. 55 (m, 2H), 1. 20-1. 35 (m, 2H), |
| | 0.98 (t, J=7.3Hz, 3H), 0.58 (t, J=7.5Hz, 3H). |
| 2-038 | δ7. 68-7. 73 (m, 1H), 7. 35-7. 40 (m, 2H), 7.00-7.09 (m, 2H), |
| | 6.82-6.87 (m, 2H), 3.94 (t, J=6.9Hz, 2H), 3.77 (s, 3H), |
| | 2.22 (s, 3H), 2.07-2.13 (m, 2H), 1. 74-1. 83 (m, 2H), |
| | 1. 33-1. 46 (m, 4H), 1.26 (q, J=7.8Hz, 2H), |
| | 0.93 (t, J=7.2Hz, 3H), 0.57 (t, 7. 5Hz, 3H). |
| 2-039 | δ7.67 (dd, J=8.8, 4.8Hz, 1H), 7.10-7.25 (m, 2H), |
| | 7.03 (td, J=8.8, 2.4Hz, 1H), 6.92 (dd, J=9.5, 2.4Hz, 1H), |
| | 6.65-6.80 (m, 2H) 5.54 (brs, 1H), 4.60-4.80 (m, 1H), |
| | 3.58 (s, 3H), 2.12 (s, 3H), 1. 55-2. 00 (m, 8H). |
| 2-041 | δ7.68-7.73 (m, 1H), 7. 36-7. 39 (m, 2H), 7.00-7.09 (m, 2H), |
| | 6.83-6.87 (m, 2H), 3.95 (t, J=6.6Hz, 2H), 3.77 (s, 3H), |
| | 2.22 (s, 3H), 2.07-2.13 (m, 2H), 1. 73-1. 80 (m, 2H), |
| | 1.23-1.51 (m, 8H), 0.88-0.93 (m, 3H), 0.57 (t, J=7.2Hz, 3H). |
| 2-044 | δ7.70-7.80 (m, 1H), 7.30-7.50 (m, 3H), |
| | 7.05-7.30 (m, 3H), 3.77 (s, 3H), 2.10-2.20 (m, 5H), |
| | 1.25-1.40 (m, 2H), 0.63 (t, J=7.4Hz, 3H). |
| 2-047 | δ7.73 (dd, J=8.8, 4.8Hz, 1H), 7.25-7.35 (m, 2H), |
| | 7.09 (td, J=8.8, 2.4Hz, 1H). 6.98 (dd, J=9.5, 2.4Hz, 1H), |
| | 6.75-6.90 (m, 2H), 6.29 (brs, 1H), 4.60-4.95 (m, 2H), |
| | 4.10-4.35 (m, 2H), 3.68 (s, 3H), 2.19 (s, 3H). |
| 2-051 | δ7.73 (dd, J=8.8, 4.8Hz, 1H), 7.28-7.34 (m, 2H), |
| | 7.09 (td, J=8.8, 2.6Hz, 1H), 6.99 (dd, J=9.4, 2.6Hz, 1H), |
| | 6.75-6.90 (m, 2H), 6.22 (brs, 1H), 5.95-6.15 (m, 1H), |
| | 5.25-5.50 (m, 2H), 4.50-4.60 (m, 2H), 3.69 (s, 3H), |
| | 2. 19 (s, 3H). |
| 2-053 | δ7.72 (dd, J=8.8, 4.8Hz, 1H), 7.25-7.35 (m, 2H), |
| | 7.08 (td, J=8.8, 2.4Hz, 1H), 6.97 (dd, J=9.5, 2.4Hz, 1H), |
| | 6.80-6.95 (m, 2H), 4.69 (d, J=2.4Hz, 2H), 3.65 (s, 3H), |
| | 2.56 (t, J=2.4Hz, 1H), 2.18 (s, 3H). |
| 2-054 | δ7.75 (dd, J=8.5, 4.8Hz, 1H), 7.35-7.45 (m, 2H), |
| | 7.00-7.15 (m, 2H), 6.90-7.00 (m, 2H), 4.70 (d, J=2.4Hz, 2H), |
| | 3.95-4.30 (m, 2H), 3.77 (s, 3H), 2.54 (t, J=2.6Hz, 1H), |
| | 2.36 (t, J=2.6Hz, 1H), 2.29 (s, 3H). |
| 2-055 | δ7.75 (dd, J=8.9, 4.8Hz, 1H), 7.35-7.50 (m, 2H), |
| | 7.00-7.20 (m, 2H), 6.85-7.00 (m, 2H), 4.50-4.80 (m, 4H), |
| | 3.77 (s, 3H), 3.40-3.55 (m, 2H), 3.25-3.35 (m, 2H), |
| | 3.25 (s, 3H), 2.55 (t, J=2.4Hz, 1H), 2.29 (s, 3H). |
| 2-056 | δ7.71 (dd, J=8.8, 4.8Hz, 1 H) 7.20-7.30 (m, 2H), |
| | 7.06 (td, J=8.8, 2.4Hz, 1 H) 6.95 (dd, J=9.6, 2.4Hz, 1 H), |
| | 6.75-6.85 (m, 2 H), 4.90 (brs, 1H), 4.00-4.15 (m, 2H), |
| | 3.70-3.75 (m, 2H), 3.65 (s, 3H), 3.41 (s, 3H), 2.16 (s, 3H). |
| 2-057 | δ7.75 (dd, J=8.5, 4.8Hz, 1H), 7.30-7.50 (m, 2H), |
| | 7.00-7.15 (m, 2H), 6.80-7.00 (m, 2H), 4.00-4.20 (m, 2H), |
| | 3.65-3.90 (m, 5H), 3.45 (s, 3H), 3.33 (s, 3H), 2.21 (s, 3H). |
| 2-058 | δ7.69 (dd, J=8.9, 4.8Hz, 1H), 7.15-7.25 (m, 2H), |
| | 6.70-7.10 (m, 5H), 4.00-4.20 (m, 2H), 3.60 (s, 3H), |
| | 2.86 (t, J=6.8Hz, 2H), 2.20 (s, 3H), 2.15 (s, 3H). |
| 2-059 | δ7.73 (dd, J=8.9, 4.8Hz, 1H), 7.35-7.40 (m, 2H), |
| | 7.00-7.15 (m, 2H), 6.80-6.90 (m, 2H), 4.74 (d, J=5.8Hz, 1H), |
| | 4.54 (d, J=5.5Hz, 1H), 4.15 (t, J=6.6Hz, 2H), 3.76 (s, 3H), |
| | 3.40-3.50 (m, 2H), 3.25-3.30 (m, 2H), 3.23 (s, 3H), |
| | 2.87 (t, J=6.6Hz, 2H), 2.28 (s, 3H), 2.20 (s, 3H). |
| 2-060 | δ7.73 (dd, J=8.9, 4.6Hz, 1H), 7.25-7.40 (m, 2H), |
| | 7.08 (td, J=8.9, 2. 5Hz, 1H), 6.99 (dd, J=9.4, 2.5Hz, 1H), |
| | 6.75-6.90 (m, 2H), 6.52 (s, 1H), 3.79 (d, J=7.2Hz, 2H), |
| | 3.73 (s, 3H), 2.20 (s, 3H), 1. 15-1. 35 (m, 1H), |
| | 0. 60-0. 70 (m, 2H), 0.25-0.45 (m, 2H). |
| 2-066 | δ7.71 (dd, J=8.8, 4. 8Hz, 1H), 7.20-7.35 (m, 2H), |
| | 7.08 (td, J=8.8, 2.4Hz, 1H), 6.94 (dd, J=9.2, 2.4Hz, 1H), |
| | 6.80-6.90 (m, 2H), 6.78 (brs, 1H), 4.73 (s, 2H), |
| | 3.58 (s, 3H), 2.15 (s, 3H). |
| 2-067 | δ7.76 (dd, J=8.5, 4.8Hz, 1H), 7.40-7.55 (m, 2H), |
| | 7.00-7.20 (m, 2H), 6.90-7.00 (m, 2H), |
| | 4.50-4.80 (m, 4H), 3.76 (s, 3H), 3.45-3.50 (m, 2H), |
| | 3.25-3.35 (m, 2H), 3.25 (s, 3H), 2.30 (s, 3H). |
| 2-068 | δ7.75 (dd, J=8.9, 4. 6Hz, 1H), 7.30-7.40 (m, 2H), |
| | 7.10 (td, J=8.9, 2. 5Hz, 1H), 7.00 (dd, J=9.4, 2.5 Hz, 1H), |
| | 6.75-6.90 (m, 2H), 6.22 (brs, 1H), 4.52 (s, 2H), |
| | 3.71 (s, 3H), 2.26 (s, 3H), 2.21 (s, 3H). |
| 2-069 | δ7.67 (dd, J=8.9, 4.8Hz, 1H), 7.15-7.25 (m, 2H), |
| | 7.03 (td, J=8.9, 2.4Hz, 1H), 6.90 (dd, J=9.5, 2.4Hz, 1H), |
| | 6.70-6.80 (m, 2H), 4.79 and 4.47 (s, 2H), |
| | 3.88 and 3.86 (s, 3H), 3.52 (s, 3H), |
| | 2.11 and 2.10 (s, 3H), 1.93 and 1.92 (s, 3H). |
| 2-071 | δ 7. 70-7. 77 (m, 1H), 7.39 (d, J=8.9Hz, 2H), 6.99-7.13 (m, 2H), |
| | 6.86 (d, J=8.9Hz, 2H), 4.71-4.75 (m, 1H), 4.63 (s, 2H), |
| | 4.52-4.56 (m, 1H), 3.80 (s, 3H), 3.74 (s, 3H), |
| | 3.43-3.49 (m, 2H), 3.26-3.32 (m, 2H), 3.23 (s, 3H), |
| | 2.28 (s, 3H). |
| 2-073 | δ7.65-7.80 (m, 1H), 7.40-7.50 (m, 2H), 7.30-7.40 (m, 2H), |
| | 7.00-7.20 (m, 5H), 6.90-7.00 (m, 2H), 3.77 (s, 3H), |
| | 2.23 (s, 3H), 2.11 (td, J=7.2, 3.9Hz, 2H), |
| | 1.20-1.35 (m, 2H), 0.50-0.65 (m, 3H). |
| 2-080 | δ7.75 (dd, J=8.5, 4.8Hz, 1H), 7.60-7.70 (m, 2H), |
| | 7.45-7.55 (m, 2H), 7.00-7.15 (m, 2H), 4.01 (s, 3H), |
| | 3.79 (s, 3H), 2.23 (s, 3H), 2.22 (s, 3H), |
| | 2.00-2.15 (m, 2H), 1. 20-1. 35 (m, 2H), |
| | 0.57 (t, J=7.3Hz, 3H). |
| 2-096 | δ7.71 (d, J=8.6Hz, 1H), 7.39 (d, J=2.2Hz, 1H), |
| | 7.25-7.35 (m, 2H), 6.60-6.70 (m, 2H), 3.82 (s, 3H), |
| | 3.74 (s, 3H), 2.15 (s, 3H), 2.10-2.20 (m, 2H), |
| | 1.20-1.45 (m, 2H), 0.65 (t, J=7.5H, 3H). |
| 2-106 | δ7.73 (d, J=8.6Hz, 1H), 7.41 (d, J=2.1Hz, 1H), |
| | 7.15-7.35 (m, 3H), 6.80-6.95 (m, 2H), 4.03 (q, J=6.9Hz, 2H), |
| | 3.75 (s, 3H), 3.36 (s, 3H), 2.11 (s, 3H), |
| | 1.31 (t, J=6.9Hz, 3H). |
| 2-108 | δ7.70 (d, J=8. 5Hz, 1H), 7.25-7.40 (m, 4H), 6.80-6.85 (m, 2H), |
| | 4. 45-4. 60 (m, 1H), 3.78 (s, 3H), 2.23 (s, 3H), |
| | 2.10 (td, J=7.2, 1. 7Hz, 2H), 1. 20-1. 40 (m, 8H), |
| | 0.58 (t, J=7.3 Hz, 3H). |
| 2-110 | δ7.70 (d, J=8. 5Hz, 1H), 7.25-7.45 (m, 4H), 6.80-6.90 (m, 2H), |
| | 3.96 (t, J=6.5Hz, 2H), 3.78 (s, 3H), 2.23 (s, 3H), |
| | 2.10 (td, J=7.2, 2.2Hz, 2H), 1.65-1.85 (m, 2H), |
| | 1. 40-1. 55 (m, 2H), 1. 20-1. 35 (m, 2H), 0.97 (t, J=7. 16Hz, 3H), |
| | 0.58 (t, J=7.5 Hz, 3H). |
| 2-114 | δ7.71 (d, 7. 8Hz, 1H), 7.25-7.35 (m, 3H), 6.55-6.70 (m, 2H), |
| | 3.95-4.10 (m, 2H), 3.83 (s, 3H), 3.67 (s, 3H), |
| | 3.35-3.45 (m, 2H), 3.27 (s, 3H), 2.18 (s, 3H). |
| 2-117 | δ7. 75-7. 80 (m, 1H), 7. 30-7. 40 (m, 2H), 7.15-7.25 (m, 2H), |
| | 6.85-7.00 (m, 1H), 5.38 (s, 1H), 3.91 (s, 3H), 3.82 (s, 3H), |
| | 2.28 (s, 3H). |
| 2-120 | δ7.70 (d, J=8. 5Hz, 1H), 7.20-7.35 (m, 4H), 6. 70-6. 80 (m, 1H), |
| | 3.83 (s, 3H), 3.78 (s, 3H), 2.24 (s, 3H), 2.19 (s, 3H), |
| | 2.11 (td, J=7.2, 2.0Hz, 2H), 1.20-1.36 (m, 2H), |
| | 0.58 (t, J=7.3Hz, 3H). |
| 2-123 | δ7. 70-7. 80 (m, 1H), 7.25-7.35 (m, 2H), 6.95-7.10 (m, 2H), |
| | 6.82 (d, J=8.5Hz, 1H), 5.63 (brs, 1H), 3.89 (s, 3H), |
| | 3.76 (s, 3H), 3.75 (s, 3H), 2.24 (s, 3H). |
| 2-124 | δ7.72 (d, J=8. 5Hz, 1H), 7.25-7.40 (m, 2H), 7.00-7.15 (m, 2H), |
| | 6.86 (d, J=8.5Hz, 1H), 3.89 (s, 3H), 3.76 (s, 3H), |
| | 3.73 (s, 3H), 2.26 (s, 3H), 2.12 (td, J=7. 1, 3.9Hz, 2H), |
| | 1.25-1.35 (m, 2H), 0.56 (t, J=7.5Hz, 3H). |
| 2-133 | δ7.65-7.75 (m, 1H), 7.40- 7.50 (m, 2H), 7.25-7.40 (m, 3H), |
| | 7.10-7.20 (m, 2H), 3.79 (s, 3H), 2.42 (s, 3H), 2.20 (s, 3H), |
| | 2.06 (td, J=7.2, 1. 5Hz, 2H), 1.15-1.35 (m, 2H), |
| | 0.58 (t, J=7.5Hz, 3H). |
| 2-135 | δ7. 65-7. 70 (m, 1H), 7.40-7.50 (m, 2H), 7.10-7.20 (m, 2H), |
| | 7.00-7.10 (m, 2H), 3.78 (s, 3H), 2.42 (s, 3H), 2.23 (s, 3H), |
| | 2.00-2.15 (m, 2H), 1. 15-1. 35 (m, 2H), 0.57 (t, J=7.4Hz, 3H). |
| 2-137 | δ7.67 (d, J=8.6Hz, 1H), 7.30-7.40 (m, 2H), 7.10-7.20 (m, 4H), |
| | 3.80 (s, 3H), 2.41 (s, 3H), 2.34 (s, 3H), 2.21 (s, 3H), |
| | 2.00-2.10 (m, 2H), 1. 15-1. 35 (m, 2H), 0.57 (t, J=7.4Hz, 3H). |
| 2-140 | δ7.70 (d, J=8.2Hz, 1H), 7.25-7.40 (m, 2H), |
| | 7.17 (dd, J=8.2, 1.4Hz, 1H), 7. 10-7. 15 (m, 1H), |
| | 6. 75-6. 85 (m, 2H), 6.39 (s, 1H), 3.79 (s, 3H), |
| | 3.71 (s, 3H), 2.41 (s, 3H), 2.21 (s, 3H). |
| 2-141 | δ7. 60-7. 70 (m, 1H), 7.35-7.45 (m, 2H), 7.10-7.20 (m, 2H), |
| | 6.80-6.95 (m, 2H), 3.81 (s, 3H), 3.80 (s, 3H), |
| | 2.42 (s, 3H), 2.23 (s, 3H), 2. 00-2. 15 (m, 2H), |
| | 1.20-1.35 (m, 2H), 0.58 (t, J=7.3Hz, 3H). |
| 2-143 | δ7.69 (d, J=8.2Hz, 1H), 7.20-7.35 (m, 3H), 7.00-7.20 (m, 3H), |
| | 3.78 (s, 3H), 2.43 (s, 3H), 2.42 (s, 3H), 2.15-2.25 (m, 2H), |
| | 2.13 (s, 3H), 1. 30-1. 45 (m, 2H), 0.68 (t, J=7.3Hz, 3H). |
| 2-145 | δ8.15-8.25 (m, 2H), 7.65-7.80 (m, 3H), 7.15-7.25 (m, 2H), |
| | 3.76 (s, 3H), 2.45 (s, 3H), 2.28 (s, 3H), |
| | 2.00-2.20 (m, 2H), 1. 15-1. 30 (m, 2H), 0.55 (t, J=7.5Hz, 3H). |
| 2-147 | δ7. 50-7. 80 (m, 5H), 7.15-7.25 (m, 2H), 3.76 (s, 3H), |
| | 2.45 (s, 3H), 2.28 (s, 3H), 2.00-2.20 (m, 2H), |
| | 1. 15-1. 35 (m, 2H), 0.55 (t, J=7.3Hz, 3H). |
| 2-155 | δ7. 95-8.00 (m, 1H), 7.75-7.85 (m, 3H), 7.65-7.75 (m, 1H), |
| | 7.45-7.60 (m, 3H), 7.15-7.20 (m, 2H), 3.79 (s, 3H), |
| | 2.44 (s, 3H), 2.19 (s, 3H), 2.00-2.15 (m, 2H), |
| | 1. 15-1. 35 (m, 2H), 0.57 (t, J=7.4Hz, 3H). |
| 2-159 | δ7. 60-7. 70 (m, 1H), 7.10-7.20 (m, 2H), 7.03 (d, J=2.1Hz, 1H), |
| | 6.96 (dd, J=8.6, 2.1Hz, 1H), 6.81 (d, J=8.6Hz, 1H), |
| | 4.26 (s, 4H), 3.81 (s, 3H), 2.41 (s, 3H), 2.24 (s, 3H), |
| | 2.08 (td, J=7.3, 2.0Hz, 2H), 1.15-1.35 (m, 2H), |
| | 0.57 (t, J=7.4Hz, 3H). |
| 2-161 | δ7.69 (d, J=7.8Hz, 1H), 7. 36-7. 26 (m, 2H), |
| | 7.20 (d, J=1.8Hz, 1H), 7.00-6.85 (m, 3H), 3.74 (s, 3H), |
| | 3.68 (s, 3H), 3.37 (s, 3H), 2.12 (s, 3H), 2. 05-1. 95 (m, 1H), |
| | 1.00-0.92 (m, 2H), 0.70-0.62 (m, 2H). |
| 2-182 | δ7.72 (d, J=9.0Hz, 1H), 7.40-7.50 (m, 1H), 7.28-7.38 (m, 3H), |
| | 6. 88-6. 95 (m, 1H), 6.87 (d, J=9.0Hz, 1H), 6.60 (brs, 1H), |
| | 3.74 (s, 3H), 3.69 (s, 3H), 3.36 (s, 3H), 2.08 (s, 3H), |
| | 1.50 (s, 9H). |
| 2-186 | δ7.77 (d, J=8.4Hz, 1H), 7.48 (dd, J=8.4, 1. 8Hz, 1H), |
| | 7.40-7.26 (m, 3H), 6.95-6.70 (m, 3H), |
| | 5.75 (dd, J=18.0, 0.9Hz, 1H), |
| | 5.75 (dd, J=10.8Hz, 0.9Hz, 1H), |
| | 3.74 (s, 3H), 3.71 (s, 3H), 3.37 (s, 3H), 2.12 (s, 3H). |
| 2-189 | δ7.74 (dd, J=8.4, 0.9Hz, 1H), 7.55-7.57 (m, 1H), |
| | 7.31 (dd, J=8.4, 0.9Hz, 1H), 7.45-7.25 (m, 2H), |
| | 6. 60-6. 70 (m, 2H), 3.75 (s, 3H), 3.71 (s, 3H), |
| | 3.24 (s, 3H), 2.11 (s, 3H), 0.25 (s, 9H). |
| 2-193 | δ8.00-8.05 (m, 1H), 7.85-8.00 (m, 2H), 7.25-7.40 (m, 1H), |
| | 6. 60-6. 70 (m, 2H), 3.75 (s, 3H), 3.73 (s, 3H), 3.36 (s, 3H), |
| | 2.65 (s, 3H), 2.16 (s, 3H). |
| 2-204 | δ7.75 (dd, J=9.5, 4.8Hz, 1H), 7.35-7.50 (m, 2H), |
| | 7.05-7.35 (m, 4H), 3.76 (s, 3H), 2.21 (t, J=7.2Hz, 2H), |
| | 2.13 (s, 3H), 1. 30-1. 45 (m, 2H), 0.65 (t, J=7.5Hz, 3H). |
| 2-210 | δ7. 72-7. 76 (m, 1H), 7.23-7.29 (m, 1H), 7.03-7.13 (m, 4H), |
| | 6.86-6.90 (m, 1H), 3.76 (s, 3H), 3.74 (s, 3H), 2.23 (s, 3H), |
| | 2. 09-2. 14(m, 2H), 1. 21-1. 33 (m, 2H), 0.56 (t, J=7.2Hz, 3H). |
| 2-212 | δ7.74 (dd, J=8.5, 4.8Hz, 1H), 7.20-7.30 (m, 1H), |
| | 7.00-7.15 (m, 4H), 6.80- 6.90 (m, 1H), 3.90-4.05 (m, 2H), |
| | 3.76 (s, 3H), 2.22 (s, 3H), 2. 05-2. 15 (m, 2H), |
| | 1.20-1.40 (m, 5H), 0.57 (t, J=7.3Hz, 3H). |
| 2-216 | δ7. 70-7. 80 (m, 1H), 7.20-7.30 (m, 1H), 6.95-7.15 (m, 4H), |
| | 6.85 (ddd, J=8. 2, 2.4, 1.0Hz, 1H), 4. 40-4. 55 (m, 1H), |
| | 3.76 (s, 3H), 2.22 (s, 3H), 2. 05-2. 15 (m, 2H), |
| | 1.20-1.35 (m, 8H), 0.57 (t, J=7.5Hz, 3H). |
| 2-218 | δ7.65-7.75 (m, 1H), 7.35-7.45 (m, 2H), 6.95-7.15 (m, 4H), |
| | 3.77 (s, 3H), 3. 65-3. 75 (m, 1H), 2.24 (s, 3H), |
| | 2.05-2.15 (m, 2H), 1.20-1.35 (m, 2H), 0.75-0.80 (m, 4H), |
| | 0.57 (t, J=7.2Hz, 3H). |
| 2-222 | δ7.65-7.75 (m, 1H), 7.35-7.45 (m, 2H), 7.00-7.10 (m, 2H), |
| | 6.80-6.90 (m, 2H), 3.78 (s, 3H), 3.72 (d, J=6.5 Hz, 2H), |
| | 2.23 (s, 3H), 2.00-2.15 (m, 3H), 1. 20-1. 35 (m, 2H), |
| | 1.02 (d, J=6.8Hz, 6H), 0.57 (t, J=7.3Hz, 3H). |
| 2-224 | δ7. 70-7. 75 (m, 1H), 7.30-7.40 (m, 2H), 7.00-7.15 (m, 2H), |
| | 6.90-7.00 (m, 2H), 3.77 (s, 3H), 2.20 (s, 3H), |
| | 2.05-2.15 (m, 2H), 1.36 (s, 9H), 1. 20-1. 35 (m, 2H), |
| | 0.59 (t, J=7.5Hz, 3H). |
| 2-230 | δ7.19-7.77 (m, 5H), 7.03-7.15 (m, 2H), 3.84 and 3.75 (s, 3H), |
| | 2.13-2.28 (m, 5H), 1.34-1.41 (m, 2H), 0.64-0.70 (m, 3H). |
| 2-235 | δ7. 75-7.83 (m, 1H), 7.26-7.43 (m, 2H), 7.02-7.20 (m, 4H), |
| | 5.80 (t, 71.9Hz, 1H), 3.82 (s, 3H), 2.50 (t, J=7.2Hz, 2H), |
| | 2.29 (s, 3H), 1. 67-1. 83 (m, 2H), 1.02 (t, J=7.5Hz, 3H). |
| 2-237 | δ7. 74-7. 79 (m, 1H), 7.38-7.47 (m, 3H), 7.07-7.19 (m, 3H), |
| | 3.74 (s, 3H), 2.24 (s, 3H), 2.04-2.16 (m, 2H), |
| | 1.24-1.30 (m, 2H), 0.56 (t, J=7.5Hz, 3H). |
| 2-239 | δ7. 77-7. 85 (m, 1H), 7.29-7.42 (m, 2H), 7. 13-7. 18 (m, 2H), |
| | 7.03-7.11 (m, 2H), 4.23-4.27 (m, 2H), 3.79 (s, 3H), |
| | 2.51 (t, J=7. 3Hz, 2H), 2.26 (s, 3H), 1. 68-1. 83 (m, 2H), |
| | 1.03 (t, J=7.3Hz, 3H). |
| 2-245 | δ7.73 (dd, J=8. 5, 5.1Hz, 1H), 7.35-7.45 (m, 2H), |
| | 7.20-7.30 (m, 2H), 7.00-7.15 (m, 2H), 3.77 (s, 3H), |
| | 2.96 (q, J=7.3Hz, 2H), 2.23 (s, 3H), 2.05-2.15 (m, 2H), |
| | 1.20-1.40 (m, 5H), 0.57 (t, J=7.3Hz, 3H). |
| 2-249 | δ7.73 (dd, J=8.7, 4.9Hz, 1H), 7.35-7.40 (m, 2H), |
| | 7.20-7.30 (m, 2H), 7.00-7.15 (m, 2H), 3.77 (s, 3H), |
| | 2.85-3.00 (m, 2H), 2.23 (s, 3H),2.05-2. 15 (m, 2H), |
| | 1.60-1.80 (m, 2H), 1. 20-1. 35 (m, 2H), |
| | 1.04 (t, J=7.3Hz, 3H), 0.57 (t, J=7.3Hz, 3H). |
| 2-251 | δ7. 71-7. 79 (m, 1H), 7.46-7.51 (m, 1H), 7.27-7.37 (m, 3H), |
| | 7.03-7.14 (m, 2H), 3.75 (s, 3H), 3.24-3.60 (m, 1H), |
| | 2.22 (s, 3H), 2.07-2.15 (m, 2H), 1. 25-1. 33 (m, 2H), |
| | 1.24 (d, J=4.1Hz, 3H), 1.22 (d, J=3.8Hz, 3H), |
| | 0.57 (t, J=7.3Hz, 3H). |
| 2-253 | δ7.74 (dd, J=8.9, 4.8Hz, 1H), 7.35-7.40 (m, 2H), |
| | 7.30-7.35 (m, 2H), 7.00-7.15 (m, 2H), 3.76 (s, 3H), |
| | 3.35-3.50 (m, 1H), 2.23 (s, 3H), 2.05-2.15 (m, 2H), |
| | 1.20-1.35 (m, 8H), 0.57 (t, J=7.3Hz, 3H). |
| 2-255 | δ7. 70-7. 75 (m, 1H), 7.35-7.45 (m, 2H), 7.30-7.35 (m, 2H), |
| | 7.00-7.15 (m, 2H), 3.78 (s, 3H), 2.24 (s, 3H), |
| | 2.05-2.20 (m, 3H), 1. 20-1. 35 (m, 2H), 1. 05-1. 15 (m, 2H), |
| | 0.65-0.75 (m, 2H), 0.57 (t, J=7.3Hz, 3H). |
| 2-256 | δ7.60-7.78 (m, 2H), 7.44-7.50 (m, 1H), 7.21-7.41 (m, 2H), |
| | 7.07-7.09 (m, 1H), 7.00-7.06 (m, 1H), 3.58-3.70 (m, 3H), |
| | 2.27-2.43 (m, 3H), 2.17-2.24 (m, 3H). |
| 2-260 | δ7.69 (dd, J=8.9, 4.8Hz, 1H), 7.00-7.25 (m, 5H), |
| | 6.90 (dd, J=9.4, 2.4Hz, 1H), 5.65-6.10 (m, 1H), 3.49 (s, 3H), |
| | 3. 15-3. 30 (m, 2H), 2.10 (s, 3H). |
| 2-266 | δ7.75 (dd, J=8.5, 4.8Hz, 1H), 7.35-7.40 (m, 2H), |
| | 7.25-7.35 (m, 2H), 7.00-7.15 (m, 2H), 3.76 (s, 3H), |
| | 3.60 (t, J=6.7Hz, 2H), 3.37 (s, 3H), 3.33 (s, 3H), |
| | 3.13 (t, J=6.7Hz, 2H), 2.22 (s, 3H). |
| 2-268 | δ7. 70-7. 80 (m, 1H), 7.20-7.45 (m, 6H), 7.00-7.20 (m, 4H), |
| | 6.90-7.00 (m, 1H), 4.90-5.10 (m, 2H), 3.75 (s, 3H), |
| | 2.23 (s, 3H), 2.05-2.15 (m, 2H), 1. 20-1. 35 (m, 2H), |
| | 0.56 (t, J=7.5Hz, 3H). |
| 2-270 | δ7. 74-7.81 (m, 1H), 7.28-7.41 (m, 2H), 7.19-7.22 (m, 1H), |
| | 7.01-7.16 (m, 3H), 5.51-5.66 (m, 1H), 4.90-5.07 (m, 2H), |
| | 3. 98-4. 07 (m, 1H), 3. 77-3. 86 (m, 1H), 3.77 (s, 3H), |
| | 2.51 (t, J=7.2Hz, 2H), 2.25 (s, 3H), 1. 69-1. 83 (m, 2H), |
| | 1.04 (t, J=7.5Hz, 3H). |
| 2-272 | δ7. 73-7. 80 (m, 1H), 7.29-7.35 (m, 2H), 7.18-7.21 (m, 1H), |
| | 7.01-7.15 (m, 3H), 3.75 (s, 3H), 3.61-3.70 (m, 1H), |
| | 3.35-3.43 (m, 1H), 3.23-3.43 (m, 2H), 2.94 (s, 3H), |
| | 2.50 (t, J=7.8Hz, 2H), 2.25 (s, 3H), 1. 68-1. 83 (m, 2H), |
| | 1.02 (t, J=7.5Hz, 3H). |
| 2-274 | δ7. 72-7.80 (m, 1H), 7.18-7.40 (m, 3H), 7.00-7.15 (m, 3H), |
| | 3.76 (s, 3H), 3.38-3.70 (m, 2H), 2.50 (t, J=7.3Hz, 2H), |
| | 2.40 (t, J=6. 5Hz, 2H), 2.24 (s, 3H), 1. 65-1. 83 (m, 5H), |
| | 1.01 (t, J=7.3Hz, 3H). |
| 2-278 | δ7. 76-7.85 (m, 1H), 7.31-7.45 (m, 2H), 6.99-7.25 (m, 4H), |
| | 4.05-4.45 (m, 1H), 3.73-3.87 (m, 3H), 2.47-2.54 (m, 2H), |
| | 2.25-2.39 (m, 3H), 1. 67-1. 82 (m, 2H), |
| | 1.22-1.33 (m, 3H), 0.98-1.06 (m, 3H). |
| 2-282 | δ7. 69-7. 73 (m, 1H), 7.30-7.48 (m, 4H), 7.03-7.15 (m, 5H), |
| | 6.90-6.98 (m, 2H), 6.51 (t, J=74. 4Hz, 1H), 3.71 (s, 3H), |
| | 2.04 (s, 3H), 1.34 (s, 3H), 1.20 (s, 3H). |
| 2-283 | δ7. 73-7. 77 (m, 1H), 7.56-7.51 (m, 2H), 7.03-7.15 (m, 4H), |
| | 6.53 (t, J=74.7Hz, 1H), 3.65-3.90 (m, 5H), 3.05 (s, 3H), |
| | 2.26 (s, 3H). |
| 2-284 | δ7. 76-7.80 (m, 1H), 7.42-7.47 (m, 2H), 7.04-7.25 (m, 6H), |
| | 6.89-6.95 (m, 1H), 6.51 (t, J=74.1Hz, 1H), 6.43-6.47 (m, 2H), |
| | 4.28-4.53 (m, 2H), 3.75 (s, 3H), 2.24 (s, 3H). |
| 2-286 | δ7.65-7.70 (m, 1H), 7.56-7.60 (m, 2H), 7.37-7.42 (m, 2H), |
| | 7.29-7.35 (m, 2H), 7.05-7.17 (m, 4H), 6.55 (t, J=73.8Hz, 1H), |
| | 3.82 (s, 3H), 2.25 (s, 3H). |
| 2-289 | δ7. 72-7. 78 (m, 1H), 7.46-7.54 (m, 2H), 7.00-7.15 (m, 4H), |
| | 6.53 (t, J=74. 1Hz, 1H), 4. 30-4. 39 (m, 1H), 3. 51-3. 79 (m, 5H), |
| | 2.28 and 2.26 (s, 3H), 0.86-2.01 (m, 4H). |
| 2-293 | δ7. 73-7. 78 (m, 1H), 7.44-7.49 (m, 2H), 7.04-7.14 (m, 4H), |
| | 6.53 (t, J=74.1Hz, 1H), 3.80 (s, 3H), 4.08-4.22 (m, 2H), |
| | 3.36-3.45 (m, 2H), 2.28 (s, 3H). |
| 2-294 | δ7. 72-7. 77 (m, 1H), 7.43-7.50 (m, 2H), 7.03-7.14 (m, 4H), |
| | 6.52 (t, J=74. 1Hz, 1H), 5. 52-5. 65 (m, 1H), 5. 03-5. 15 (m, 2H), |
| | 4.31-4.43 (m, 2H), 3.78 (s, 3H), 2.27 (s, 3H). |
| 2-295 | δ7. 72-7. 77 (m, 1H), 7.44-7.49 (m, 2H), 7.04-7.14 (m, 4H), |
| | 6.53 (t, J=74.1Hz, 1H), 3.99-4.13 (m, 2H), 3.78 (s, 3H), |
| | 3.36 (t, J=4.2Hz, 2H), 3.24 (s, 3H), 2.27 (s, 3H). |
| 2-297 | δ7. 76-7. 81 (m, 1H), 7.46-7.51 (m, 2H), 7. 19-7. 32 (m, 3H), |
| | 7.06-7.14 (m, 4H), 6.64-6.67 (m, 2H), 6.52 (t, J=74.1Hz, 1H), |
| | 3.86 (s, 3H), 2.33 (s, 3H). |
| 2-299 | δ7. 74-7. 79 (m, 1H), 7.54-7.60 (m, 1H), 7.38-7.43 (m, 2H), |
| | 7.23-7.31 (m, 4H), 7.04-7.14 (m, 4H), 6.52 (t, J=73.2Hz, 1H), |
| | 3.83 (s, 3H), 2.35 (s, 3H). |
| 2-300 | δ7. 75-7. 80 (m, 1H), 7.43-7.48 (m, 2H), 7.04-7.16 (m, 4H), |
| | 6.53 (t, J=73. 2Hz, 1H), 5. 53-5. 66 (m, 1H), 4. 93-5. 07 (m, 2H), |
| | 3.99-4.06 (m, 1H), 3. 80-3. 87 (m, 1H), 3.77 (s, 3H), |
| | 2.25 (s, 3H). |
| 2-301 | δ7. 75-7. 80 (m, 1H), 7.45-7.50 (m, 2H), 7.05-7.16 (m, 4H), |
| | 6.53 (t, J=73.2Hz, 1H), 4.02-4.25 (m, 2H), 3.77 (s, 3H), |
| | 2.37 (t, J=2.4Hz, 1H), 2.30 (s, 3H). |
| 2-302 | δ7. 78-7. 83 (m, 1H), 7.39-7.43 (m, 2H), 7.04-7.19 (m, 4H), |
| | 6.54 (t, J=74. 1Hz, 1H), 3. 72-3. 84 (m, 4H), 3. 46-3. 52 (m, 1H), |
| | 2.26 (s, 3H). |
| 2-307 | δ7. 73-7. 78 (m, 1H), 7.45-7.50 (m ,2H), 7.03-7.15 (m, 4H), |
| | 6.53 (t, J=74.1Hz, 1H), 4.75 (d, J=6.0Hz, 1H), |
| | 4.57 (d, J=6.0Hz, 1H), 3.75 (s, 3H), 3.45-3.48 (m, 2H), |
| | 3.27-3.34 (m, 2H), 3.25 (s, 3H), 2.30 (s, 3H). |
| 2-308 | δ7. 75-7.80 (m, 1H), 7.66-7.70 (m, 1H), 7.33-7.37 (m, 1H), |
| | 6.92-7.23 (m, 4H), 6.53 (t, J=74.1Hz, 1H), |
| | 5.78 and 5.46 (q, J=5.1Hz, 1H), 4.01-4.11 (m, 2H), |
| | 3.83 and 3.70 (s, 3H), 2.30 and 2.14 (s, 3H), |
| | 1. 20-1. 26 (m, 3H), 1.07 and 0.83 (d, J=5.1Hz, 3H). |
| 2-309 | δ7. 76-7. 81 (m, 1H), 7.50-7.55 (m, 2H), 7.04-7.17 (m, 4H), |
| | 6.54 (t, J=73. 5Hz, 1H), 3.74 (s, 3H), 2.50 (s, 3H), |
| | 2.22-2.36 (m, 1H), 1.12 (d, J=6.9Hz, 3H), |
| | 0.81 (d, J=6.9Hz, 3H). |
| 2-313 | δ7.45-7.52 (m, 1H), 7.33-7.38 (m, 2H), 7.08-7.13 (m, 2H), |
| | 6.89-7.04 (m, 3H), 6.30-6.81 (m, 4H), 3.73 (m, 3H), |
| | 3.63 (m, 3H), 2.53 (s, 3H). |
| 2-316 | δ7.78 (dd, J=8.5, 4.8Hz, 1H), 7.45-7.55 (m, 2H), |
| | 7.30-7.40 (m, 2H), 7.05-7.15 (m, 2H), 4.68 (s, 2H), |
| | 3.87 (q. J=8.5Hz, 2H), 3.77 (s, 3H), 3.35 (s, 3H), |
| | 2.22 (s, 3H). |
| 2-317 | δ7.72 (dd, J=8.8, 4. 7Hz, 1H), 7.15-7.30 (m, 4H), |
| | 7.08 (ddd, J=8.8, 8.8, 2. 5Hz, 1H), |
| | 6.96 (dd, J=9.4, 2. 5Hz, 1H), |
| | 6.54 (brs, 1H), 3.63 (s, 2H), 3.60 (s, 3H), 2.14 (s, 3H), |
| | 1.97 (s, 3H). |
| 2-319 | δ7.72 (dd, J=8.8, 4. 7Hz, 1H), 7.15-7.30 (m, 4H), |
| | 7.08 (ddd, J=8.8, 8.8, 2. 5Hz, 1H), |
| | 6.95 (dd, J=9.6, 2. 3Hz, 1H), |
| | 6.52 (brs, 1H), 3.67 (s, 2H), 3.60 (s, 3H), |
| | 2.42 (q, J=7.4Hz, 2H), 2.13 (s, 3H), 1.21 (t, J=7.4Hz, 3H). |
| 2-321 | δ7. 76-7. 82 (m, 1H), 7.36-7.41 (m, 2H), 7.28-7.34 (m, 2H), |
| | 7.09-7.18 (m, 1H), 7.02-7.08 (m, 1H), 5.55 (brs, 1H), |
| | 3.78 (s, 3H), 3.73 (s, 2H), 2.80 (sep, J=6.8Hz, 1H), |
| | 2.22 (s, 3H), 1. 22-1. 29 (m, 6H). |
| 2-322 | δ7. 73-7. 79 (m, 1H), 7.37-7.43 (m, 2H), 7.28-7.34 (m, 2H), |
| | 7.02-7.15 (m, 2H), 3.76 (s, 3H), 3.73 (s, 2H), 3.33 (s, 3H), |
| | 2.81 (sep, J=6.8Hz, 1H), 2.20 (s, 3H), |
| | 1.26 (d, J=6.8Hz, 6H). |
| 2-323 | δ7. 75-7. 82 (m, 1H), 7.36-7.41 (m, 2H), 7.26-7.31 (m, 2H), |
| | 7.09-7.18 (m, 1H), 7.02-7.08 (m, 1H), 5. 71-5. 86 (m, 1H), |
| | 5.58 (brs, 1H), 5.03-5.16 (m, 2H), 3.77 (s, 3H), |
| | 3.65 (s, 2H), 3.04 (dt, J=7.2, 1.0Hz, 2H), 2.22 (s, 3H). |
| 2-324 | δ7. 73-7.80 (m, 1H), 7.37-7.43 (m, 2H), 7.26-7.31 (m, 2H), |
| | 7.03-7.15 (m, 2H), 5. 70-5. 87 (m, 1H), 5.03-5.17 (m, 2H), |
| | 3.76 (s, 3H), 3.65 (s, 2H), 3.33 (s, 3H), |
| | 3.04 (dt, J=7.2, 1.2Hz, 2H), 2.21 (s, 3H). |
| 2-325 | δ7. 76-7.83 (m, 1H), 7.38-7.43 (m, 2H), 7.29-7.34 (m, 2H), |
| | 7. 10-7. 18 (m, 1H), 7.02-7.08 (m, 1H), 5.59 (brs, 1H), |
| | 3.86 (s, 2H), 3.77 (s, 3H), 3.09 (d, J=2.7Hz, 2H), |
| | 2.30 (t, J=2. 7Hz, 1H), 2.23 (s, 3H). |
| 2-327 | δ7. 76-7. 82 (m, 1H), 7.37-7.42 (m, 2H), 7.28-7.33 (m, 2H), |
| | 7.09-7.18 (m, 1H), 7.01-7.08 (m, 1H), 5.60 (brs, 1H), |
| | 3.78 (s, 3H), 3.75 (s, 2H), 3.51 (t, J=6. 5Hz, 2H), |
| | 3.33 (s, 3H), 2.61 (t, J=6.5Hz, 2H), 2.23 (s, 3H). |
| 2-328 | δ7. 72-7. 79 (m, 1H), 7.37-7.43 (m, 2H), 7.27-7.32 (m, 2H), |
| | 7.02-7.14 (m, 2H), 3.75 (s, 3H), 3.74 (s, 2H), |
| | 3.51 (t, J=6.7Hz, 2H), 3.329 (s, 3H), 3.326 (s, 3H), |
| | 2.61 (t, J=6.7Hz, 2H), 2.20 (s, 3H). |
| 2-330 | δ7. 75-7.85 (m, 1H), 7.35-7.45 (m, 2H), 7.25-7.35 (m, 2H), |
| | 7.10-7.20 (m, 1H), 7.00-7.10 (m, 1H), 5.60-6.05 (m, 1H), |
| | 3.78 (s, 2H), 3.78 (s, 3H), 2. 65-2. 80 (m, 2H), 2.23 (s, 3H). |
| 2-331 | δ7.80 (dd, J=8.8, 4.8Hz, 1H), 7.35-7.45 (m, 2H), |
| | 7.25-7.35 (m, 2H), 7.14 (ddd, J= 8.8, 8.8, 2.4Hz, 1H), |
| | 7.05 (dd, J=9.6, 2.4Hz, 1H), 5.71 (brs, 1H), 3.82 (s, 2H), |
| | 3.76 (s, 3H), 2.90 (q, J=9.9Hz, 2H), 2.23 (s, 3H). |
| 2-335 | δ7.78 (dd, J=8.8, 4.7, 1H), 7.40-7.45 (m, 2H), |
| | 7.30-7.40 (m, 2H), 7.14 (ddd=8. 8, 8.8, 2. 7Hz, 1H), |
| | 7.01 (dd, J=9.4, 2.1Hz, 1H), 6.09 (s, 1H), 4.18 (s, 2H), |
| | 3.69 (s, 3H), 2.87 (q, J=7.4Hz, 2H), |
| | 2.21 (s, 3H), 1.36 (t, J=7.4Hz, 3H). |
| 2-361 | δ7. 70-7. 78 (m, 1H), 7.03-7.14 (m, 2H), 6.68 (d, J=2.4Hz, 2H), |
| | 6.43 (t, J=2.4Hz, 1H), 3.75 (s, 3H), 3.72 (s, 6H), |
| | 2.24 (s, 3H), 2.08-2.16 (m, 2H), 1. 18-1. 35 (m, 2H), |
| | 0.55 (t, J=7.3Hz, 3H). |
| 2-367 | δ7.69-7.80 (m, 1H), 7.08-71.7 (m, 2H), 6.99-7.07 (m, 1H), |
| | 6.92-6.96 (m, 1H), 6. 82-6. 88 (m, 1H), 3.78 (s, 3H), |
| | 3.69 (s, 3H), 2.17 (s, 3H), 2.14 (t, J=7.5Hz, 2H), |
| | 1.25-1.37 (m, 2H), 0.62 (t, J=7.2Hz, 3H). |
| 2-369 | δ7. 65-7. 75 (m, 1H), 7.20-7.30 (m, 1H), 7.05-7.15 (m, 2H), |
| | 6.85-6.95 (m, 2H), 3.81 (s, 3H), 3.74 (s, 3H), |
| | 2.05-2.25 (m, 5H), 1. 25-1. 40 (m, 2H), 0.63 (t, J=7.4Hz, 3H). |
| 2-372 | δ7.86 (dd, J=8.9, 4.4 Hz, 1H), 7. 10-7.25 (m, 2H), |
| | 6.65-6.80 (m, 2H), 5.55 (brs, 1H), 3.68 (s, 3H), |
| | 2.30 (s, 3H). |
| 2-380 | δ7. 72-7. 78 (m, 1H), 7.30-7.50 (m, 3H), 6.56-6.68 (m, 2H), |
| | 3.95-4.05 (m, 1H), 3.66-3.76 (m, 7H), 3.36 (s, 3H), |
| | 3.15 (t, J=7.5Hz, 2H), 2.62 (t, J=7.5Hz, 2H), 2.14 (s, 3H), |
| | 1.20-1.40 (m, 9H), 0.82-0.94 (m, 6H). |
| 2-395 | δ10.00 (s, 1H), 7.96 (d, J=8. 7Hz, 1H), 7.85-7.90 (m, 2H), |
| | 7.35-7.43 (m, 1H), 7.30 (dd, J=7.8, 1.8Hz, 1H), |
| | 6.94-7.02 (m, 2H), 6.46 (brs, 1H), 3.82 (s, 3H), |
| | 3.75 (s, 3H), 2.20 (s, 3H). |
| 2-396 | δ10.05 (s, 1H), 7.94-8.00 (m, 2H), 7.85-7.90 (m, 1H), |
| | 7.30-7.38 (m, 2H), 6.88-7.00 (m, 2H), 3.76 (s, 3H), |
| | 3.73 (s, 3H), 3.36 (s, 3H), 2.13 (s, 3H). |
| 2-420 | δ7.74 (dd, J=8.5, 4.8Hz, 1H), 7.30-7.45 (m, 2H), |
| | 6.95-7.15 (m, 2H), 6.80-6.90 (m, 2H), 4.15 (t, J=6.8 Hz, 2H), |
| | 3.77 (s, 3H), 3. 60-3. 75 (m, 1H), 3. 40-3. 55 (m, 1H), |
| | 2.87 (t, J=6.8 Hz, 2H), 2.40 (t, J=6.5Hz, 2H), 2.25 (s, 3H), |
| | 2.20 (s, 3H), 1.75 (s, 3H). |
| 2-421 | δ7.77 (dd, J=8.8Hz, 1H), 7.20-7.35 (m, 4H), |
| | 7.12 (ddd, J=8.8, 8.8, 2. 5Hz, 1H), |
| | 7.01 (dd, J=9.6, 2.4Hz, 1H), 5.98 (brs, 1H), 3.72 (s, 3H), |
| | 3.10-3.20 (m, 2H), 2.70-2.80 (m, 2H), |
| | 2.22 (s, 3H), 2.15 (s, 3H). |
| 2-422 | δ7.77 (dd, J=8.8, 4.8Hz, 1H), 7.20-7.35 (m, 4H), |
| | 7.12 (td, J=8.8, 2. 5Hz, 1H), 7.03 (dd, J=9.2, 2.5Hz, 1H), |
| | 5. 80-5. 95 (m, 1H), 5. 05-5. 25 (m, 2H), 3.75 (s, 3H), |
| | 3.55-3.60 (m, 2H), 2.23 (s, 3H). |
| 2-423 | δ7.81 (dd, J=8.8, 4.8Hz, 1H), 7.10-7.50 (m, 4H), |
| | 7.16 (td, J=8.8, 2.6Hz, 1H), 7.05 (dd, J=9.4, 2.6Hz, 1H), |
| | 5.67 (s, 1H), 3.76 (s, 3H), 3.61 (s, 2H), 2.26 (s, 3H). |
| 2-424 | δ7.78 (dd, J=8.8, 4.8Hz, 1H), 7.30-7.40 (m, 2H), |
| | 7.20-7.30 (m, 2H), 7.13 (td, J=8.8, 2.4Hz, 1H), |
| | 7.03 (dd, J=9.2, 2.4Hz, 1H), 5.74 (brs, 1H), 3.76 (s, 3H), |
| | 3.70 (s, 2H), 2.29 (s, 3H), 2.24 (s, 3H). |
| 2-429 | δ7. 73-7. 76 (m, 1H), 7.31-7.39 (m, 2H), 7.07-7.14 (m, 1H), |
| | 6.94-7.02 (m, 3H), 6.22 (brs, 1H), 4.21-4.27 (m, 2H), |
| | 3.65-3.72 (m, 2H), 3.33 (s, 3H), 2.21 (s, 3H). |
| 2-430 | δ7. 77-7.82 (m, 1H), 7.33-7.38 (m, 2H), 7. 12-7. 19 (m, 1H), |
| | 6.99-7.08 (m, 3H), 6.11 (brs, 1H), 4.87-5.00 (m, 2H), |
| | 2.25 (s, 3H). |
| 2-433 | δ7. 71-7. 76 (m, 1H), 7.40-7.46 (m, 2H), 7.22-7.32 (m, 5H), |
| | 7.03-7.13 (m, 2H), 6.91-6.99 (m, 2H), 5.45 (d, J=14. 1Hz, 1H), |
| | 5.03 (d, J=14. 1Hz, 1H), 2.25 (s, 3H), 2.00-2.19 (m, 2H), |
| | 1. 19-1. 32 (m, 2H), 0.54 (t, J=7.2Hz, 3H). |
| 2-439 | δ7. 68-7. 73 (m, 1H), 7.43-7.47 (m, 2H), 7.26-7.35 (m, 5H), |
| | 7.00-7.12 (m, 2H), 6.78-6.93 (m, 2H), 5.59 (d, J=13. 5Hz, 1H), |
| | 5.11 (d, J=13. 5Hz, 1H), 3.31 (s, 3H), 2.08-2.30 (m, 5H), |
| | 1.27-1.39 (m, 2H), 0.65 (t, J=6.9Hz, 3H). |
| 2-440 | δ7. 74-7. 78 (m, 1H), 7.29-7.34 (m, 2H), 7.07-7.14 (m, 1H), |
| | 7.00 (dd, J=9.9Hz, 3. 3Hz, 1H), 6.86-6.91 (m, 2H), |
| | 6.05 (brs, 1H), 4. 07-4. 12 (m, 2H), 3.78 (s, 3H), |
| | 3.73-3.76 (m, 2H), 3.44 (s, 3H). |
| 2-442 | δ7.73 (dd, J=8. 5, 4. 8Hz, 1H), 7.25-7.35 (m, 2H), |
| | 7.09 (td, J=8.8, 2.6Hz, 1H), 6.98 (dd, J=9.5, 2.4Hz, 1H), |
| | 6.75-6.85 (m, 2H), 6.10-6.20 (m, 2H), 4.64 (d, J=6.1Hz, 2H), |
| | 3.67 (s, 3H), 2.19 (s, 3H). |
| 2-444 | δ7.77 (dd, J=8.9, 4.8Hz, 1H), 7.35-7.45 (m, 2H), |
| | 7.12 (td, J=8.8, 2.6Hz, 1H), 7.03 (dd, J=9.5, 2.4Hz, 1H), |
| | 6.80-6.90 (m, 2H), 5.58 (s, 1H), 4.44 (t, J=5.4Hz, 2H), |
| | 3.78 (s, 3H), 3.44 (t, J=5.4Hz, 2H), 3.05 (s, 3H), |
| | 2.25 (s, 3H). |
| 2-445 | δ7.74 (dd, J=8.7, 4. 7Hz, 1H), 7.20-7.40 (m, 4H), |
| | 7.09 (td, J=8. 7, 2. 5Hz, 1H), 6.98 (dd, J=9.3, 2.3Hz, 1H), |
| | 6.33 (brs, 1H), 4.53 (s, 2H), 3.69 (s, 3H), |
| | 3.50-3.65 (m, 4H), 3.36 (s, 3H), 2.18 (s, 3H). |
| 2-446 | δ7.73 (dd, J=8.7, 4. 7Hz, 1H), 7.20-7.40 (m, 4H), |
| | 7.09 (td, J=8. 7, 2. 5Hz, 1H), 6.97 (dd, J=9.5, 2.5Hz, 1H), |
| | 6.36 (brs, 1H), 4.51 (s, 2H), 3.60-3.70 (m, 5H), |
| | 2.72 (t, J=6.7Hz, 2H), 2.16 (s, 3H), 2.13 (s, 3H). |
| 2-448 | δ8.79 (brs, 1H), 7.73 (dd, J=8.7, 0.9Hz, 1H), |
| | 7.24-7.32 (m, 4H), 6.76-6.84 (m, 2H), 3.76 (s, 3H), |
| | 3.31 (s, 3H), 2.21 (s, 3H). |
| 2-449 | δ7.73 (dd, J=8.4, 0.6Hz, 1H), 7.35-7.42 (m, 2H), |
| | 7.20-7.33 (m, 3H), 6.84-6.90 (m, 1H), 3.81 (s, 3H), |
| | 3.77 (s, 3H), 3.33 (s, 3H), 2.22 (s, 3H). |
| 2-459 | δ7.74 (d, J=8.1Hz, 1H), 7.43-7.48 (m, 2H), 7.20-7.23 (m, 1H), |
| | 7.14-7.15 (m, 1H), 7.06-7.09 (m, 2H), 6.52 (t, J=74.1Hz, 1H), |
| | 5.63 (brs, 1H), 3.78 (s, 3H), 2.42 (s, 3H), 2.27 (s, 3H). |
| 2-460 | δ7.65-7.75 (m, 1H), 7.53-7.65 (m, 1H), 7.45-7.52 (m, 1H), |
| | 7.25-7.42 (m, 3H), 7.00-7.13 (m, 2H), 3.53-3.65 (s, 3H), |
| | 2.33-2.72 (m, 2H), 2.13-2.24 (m, 3H), 0.80-0.94 (m, 3H). |
| 2-465 | δ7.78 (dd, J=8.9, 4.8Hz, 1H), 7.30-7.45 (m, 4H), |
| | 7.00-7.20 (m, 2H), 5.60-5.75 (m, 1H), 3.75-3.80 (m, 3H), |
| | 3.64 (d, J=2.4Hz, 2H), 2.26 (t, 2. 7Hz, 1H), 2.24 (s, 3H). |
| 2-467 | δ8.24-8.27 (m, 1H), 7.97-8.02 (m, 1H), 7.63-7.70 (m, 1H), |
| | 7.46 (brs, 1H), 7.27 (d, J=8.7Hz, 2H), 6.98-7.10 (m, 3H), |
| | 6. 87-6. 92 (m, 1H), 3.49 (s, 3H), 2.11 (s, 3H). |
| 2-468 | δ8.27-8.35 (m, 1H), 7.98-8.01 (m, 1H), 7. 73-7. 80 (m, 1H), |
| | 7.55 (d, J=8.5Hz, 2H), 7.17 (d, J=8.5Hz, 2H), |
| | 7.05-7.12 (m, 2H), 4.78 (d, J=5.8Hz, 1H), |
| | 4.59 (d, J=5.8 Hz, 1H), 3.76 (s, 3H), 3.45-3.51 (m, 2H), |
| | 3.27-3.33 (m, 2H), 3.24 (s, 3H), 2.30 (s, 3H). |
| 3-004 | δ 7.47 (dd, J=9.0, 2.4Hz, 1H), 7.25-7.40 (m, 3H), |
| | 7.00-7.10 (m, 1H), 6.85-7.00 (m, 2H), 3.83 (s, 3H), |
| | 3.72 (s, 3H), 2.12 (t, J=7.2Hz, 2H), 2.09 (s, 3H), |
| | 1.25-1.40 (m, 2H), 0.65 (t, J=7.5Hz, 3H). |
| 3-007 | δ 7.47 (dd, J=9.0, 2. 4Hz, 1H), 7.20-7.40 (m, 3H), |
| | 7. 00-7. 15(m, 1H), 6.80-6.95 (m, 2H), 4.05 (q, J=6.9Hz, 2H), |
| | 3.83 (s, 3H), 2.14 (t, J=7.2Hz, 2H), 2.10 (s, 3H), |
| | 1.25-1.40 (m, 5H), 0.65 (t, J=7.5Hz, 3H). |
| 3-008 | δ 7.48 (dd, J=9.0, 2. 4Hz, 1H), 7.35-7.40 (m, 2H), |
| | 7.25-7.35 (m, 1H), 7.05-7.15 (m, 1H), 6.85-6.95 (m, 2H), |
| | 3.81 (s, 3H), 3.78 (s, 3H), 2.21 (s. 3H), |
| | 2.05-2.15 (m, 2H), 1. 25-1. 35 (m, 2H), 0.58 (t, J=7. 5Hz, 3H). |
| 3-010 | δ 7.47 (dd, J=9.0, 2. 7Hz, 1H), 7.35- 7.40 (m, 1H), |
| | 7.15-7.30 (m, 1H), 7.00-7.15 (m, 1H), 6.55-6.65 (m, 2H), |
| | 3.81 (s, 3H), 3.73 (s, 3H), 2.13 (s, 3H), |
| | 2.12 (t, J=7.2Hz, 2H), 1. 25-1. 40 (m, 2H), |
| | 0.64 (t, J=7.8Hz, 3H). |
| 3-012 | δ 7.47 (dd, J=8.7, 1. 8Hz, 1H), 7.34 (dd, J=9.0, 5.4Hz, 1H), |
| | 7.15-7.25 (m, 1H), 7.00-7.10 (m, 1H), 6.50-6.70 (m, 2H), |
| | 4.04 (q, J=6.9Hz, 2H), 3.81 (s, 3H), 2.10-2.20 (m, 5H), |
| | 1.20-1.40 (m, 5H), 0.64 (t, J=7.2Hz, 3H). |
| 3-016 | δ 7.50 (dd, J=8.5, 2. 4Hz, 1H), 7.25-7.45 (m, 5H), |
| | 7.05-7.15 (m, 1H), 3.77 (s, 3H), 2.23 (s, 3H), |
| | 1.95-2.20 (m, 2H), 1. 20-1. 35 (m, 2H), 0.57 (t, J=7. 3Hz, 3H). |
| 3-020 | δ 7.48 (dd, J=8.9, 2. 4Hz, 1H), 7.25-7.40 (m, 3H), |
| | 7.07 (td, J=8.9, 2.4Hz, 1H), 6.75-6.95 (m, 2H), |
| | 4.50-4.60 (m, 1H), 3.78 (s, 3H), 2.21 (s, 3H), |
| | 2.05-2.15 (m, 2H), 1. 20-1. 40 (m, 8H), 0.58 (t, J=7. 3Hz, 3H). |
| 3-032 | δ 7. 75-7.85 (m, 1H), 7.40-7.50 (m, 2H), 7.25-7.35 (m, 2H), |
| | 7.00-7.10 (m, 2H), 3.76 (s, 3H), 2.22 (s, 3H), |
| | 2.05-2.20 (m, 2H), 1. 20-1. 35 (m, 2H), 0.59 (t, J=7.5Hz, 3H). |
| 3-034 | δ 7. 75-7.85 (m, 1H), 7.25-7.45 (m, 6H), 3.76 (s, 3H), |
| | 2.22 (s, 3H), 2. 00-2. 20 (m, 2H), 1. 15-1. 35 (m, 2H), |
| | 0.57 (t, J=7.5Hz, 3H). |
| 3-036 | δ 7.78 (d, J=1.7Hz, 1H), 7.25-7.40 (m, 4H), 6.85-7.00 (m, 2H), |
| | 3.83 (s, 3H), 3.72 (s, 3H), 2.05-2.25 (m, 5H), |
| | 1.25-1.45 (m, 2H), 0.66 (t, J=7.5Hz, 3H). |
| 3-038 | δ7.75-7.80 (m, 1H), 7.20-7.45 (m, 4H), 6.85-6.90 (m, 2H), |
| | 3.81 (s, 3H), 3.78 (s, 3H), 2.22 (s, 3H), 2.05-2.15 (m, 2H), |
| | 1.20-1.40 (m, 2H), 0.59 (t, J=7.3Hz, 3H). |
| 3-040 | δ 7.78 (d, J=1.4Hz, 1H), 7.15-7.45 (m, 4H), 6.80-7.00 (m, 2H), |
| | 4.00-4.10 (m, 2H), 3.82 (s, 3H), 2.05-2.20 (m, 5H), |
| | 1.30-1.45 (m, 5H), 0.66 (t, J=7.5Hz, 3H). |
| 3-042 | δ7.75-7.80 (m, 1H), 7.30-7.40 (m, 2H), 7.25-7.30 (m, 2H), |
| | 6.80-6.90 (m, 2H), 4.50-4.60 (m, 1H), 3.78 (s, 3H), |
| | 2.21 (s, 3H), 2.09 (td, J=7.2, 2. 7Hz, 2H), |
| | 1.34 (dd, J=6.0, 1. 9Hz, 6H), 1. 20-1. 30 (m, 2H), |
| | 0.59 (t, J=7.3Hz, 3H). |
| 3-044 | δ 7. 75-7. 80 (m, 1H), 7.20-7.45 (m, 3H), 6.55-6.75 (m, 2H), |
| | 3.81 (s, 3H), 3.73 (s, 3H), 2.05-2.25 (m, 5H), |
| | 1.25-1.45 (m, 2H), 0.65 (t, J=7.5Hz, 3H). |
| 3-046 | δ 7. 75-7.80 (m, 1H), 7.15-7.35 (m, 3H), 6.55-6.70 (m, 2H), |
| | 4.04 (q, J=6.8Hz, 2H), 3.81 (s, 3H), 2.05-2.20 (m, 5H), |
| | 1. 25-1. 45 (m, 5H), 0.65 (t, J=7.3Hz, 3H). |
| 3-048 | δ7.75-7.80 (m, 1H), 7.20-7.35 (m, 2H), 7.01 (d, J=2.4Hz, 1H), |
| | 6.90-7.00 (m, 1H), 6.75-6.90 (m, 1H), 4.20-4.35 (m, 4H), |
| | 3.79 (s, 3H), 2.23 (s, 3H), 2.10 (td, J=7.2, 3.6Hz, 2H), |
| | 1.25-1.35 (m, 2H), 0.58 (t, J=7.3Hz, 3H). |
| 3-050 | δ7.55-7.65 (m, 1H), 7.25-7.35 (m, 3H), |
| | 7.14 (dd, J=8.0, 1.2Hz, 1H), 6.85-7.00 (m, 2H), 3.83 (s, 3H), |
| | 3.71 (s, 3H), 2.46 (s, 3H), 2.11 (t, J=7.2Hz, 2H), |
| | 2.10 (s. 3H), 1. 25-1. 45 (m, 2H), 0.67 (t, J=7. 3Hz, 3H). |
| 3-054 | δ 7. 55-7.65 (m, 1H), 7.20-7.35 (m, 3H), |
| | 7.13 (dd, J=8.3, 0.9Hz, 1H), 6.80-6.95 (m, 2H), |
| | 4.04 (q, J=7.1Hz, 2H), 3.82 (s, 3H), 2.45 (s, 3H), |
| | 2.12 (t, J=7.2Hz, 2H), 2.10 (s, 3H), 1. 25-1. 40 (m, 5H), |
| | 0.66 (t, J=7.5Hz, 3H). |
| 3-058 | δ 7.59 (s, 1H), 7.05-7.35 (m, 3H), 6.50-6.70 (m, 2H), |
| | 4.03 (q, J=6.8z, 2H), 3.81 (s, 3H), 2.45 (s, 3H), |
| | 2.13 (s, 3H), 2.11 (t, J=7.2Hz, 2H), 1. 20-1. 45 (m, 5H), |
| | 0.64 (t, J=7.4Hz, 3H). |
| 3-062 | δ7.40-7.50 (m, 2H), 7.20-7.35 (m, 5H), |
| | 6.95 (dd, J=9.0, 2.4Hz, 1H), 3.87 (s, 3H), 3.77 (s, 3H), |
| | 2.20 (s, 3H), 2.00-2.15 (m, 2H), 1.20-1.35 (m, 2H), |
| | 0.60 (t, J=7.2Hz, 3H). |
| 3-066 | δ7.25-7.35 (m, 4H), 6.85-7.00 (m, 3H), 3.86 (s, 3H), |
| | 3.83 (s, 3H), 3.72 (s, 3H), 2.12 (t, J=7. 5Hz, 2H), |
| | 2.10 (s, 3H), 1. 25-1. 45 (m, 2H), 0.68 (t, J=7. 3Hz, 3H). |
| 3-071 | δ7.20-7.35 (m, 4H), 6.80-7.05 (m, 3H), 4.06 (q, J=7.1Hz, 2H), |
| | 3.87 (s, 3H), 3.83 (s, 3H), 2.14 (t, J=6.7Hz, 2H), |
| | 2.11 (s, 3H), 1.30-1.45 (m, 5H), 0.67 (t, J=7.5Hz, 3H). |
| 3-075 | δ7.35-7.45 (m, 2H), 7.15-7.30 (m, 4H), |
| | 6.96 (dd, J=8.9, 2.4Hz, 1H), 3.88 (s, 3H), 3.78 (s, 3H), |
| | 2.49 (s, 3H), 2.23 (s, 3H), 2. 05-2. 15 (m, 2H), |
| | 1.20-1.35 (m, 2H), 0.59 (t, J=7.3Hz, 3H). |
| 3-077 | δ 7.20-7.30 (m, 3H), 6.94 (dd, J=8.7, 2. 4Hz, 1H), |
| | 6.55-6.65 (m, 2H), 3.86 (s, 3H), 3.80 (s, 3H), 3.71 (s, 3H), |
| | 2.12 (s, 3H) 2.10 (t, J=7.2Hz, 2H), 1.25-1.40 (m, 2H), |
| | 0.66 (t, J=7.5Hz, 3H). |
| 3-079 | δ 7. 15-7. 35 (m, 3H), 6.95 (dd, J=8.9, 2. 5Hz, 1H), |
| | 6.50-6.70 (m, 2H), 4.04 (q, J=7.1Hz, 2H), 3.87 (s, 3H), |
| | 3.81 (s, 3H), 2.10-2.20 (m, 5H), 1.39 (t, J=7.1Hz, 3H), |
| | 1.25-1.40 (m, 2H), 0.66 (t, J=7.4Hz, 3H). |
| 3-090 | δ 7.25-7.35 (m, 4H), 6.85-7.00 (m, 3H), 4.09 (q, J=7.1Hz, 2H), |
| | 3.83 (s, 3H), 3.72 (s, 3H), 2.05-2.15 (m, 5H), |
| | 1.44 (t, J=7.1Hz, 3H), 1. 25-1. 40 (m, 2H), |
| | 0.67 (t, J=7.3Hz, 3H). |
| 3-092 | δ 7. 40-7. 50 (m, 2H), 7.30-7.35 (m, 2H), |
| | 7.00 (dd, J=8.9, 2.4Hz, 1H), 6.85-6.95 (m, 2H), |
| | 4.14 (q, J=6.9Hz, 2H), 3.86 (s, 3H), 3.82 (s, 3H), |
| | 2.27 (s, 3H), 2.05-2.20 (m, 2H), 1.50 (t, J=6.9Hz, 1H), |
| | 1.25-1.40 (m, 2H), 0.64 (t, J=7.5Hz, 3H). |
| 3-094 | δ 7.20-7.35 (m, 4H), 6.80-7.05 (m, 3H), 4.00-4.20 (m, 4H), |
| | 3.83 (s, 3H), 2.10-2.20 (m, 5H), 1. 30-1. 50 (m, 8H), |
| | 0.67 (t, J=7.5Hz, 3H). |
| 3-096 | δ7.20-7.35 (m, 3H), 6.94 (dd, J=8.4, 2. 4Hz, 1H), |
| | 6.64 (dd, J=10. 9, 2.4Hz, 1H), 6.58 (td, J=8.4, 2. 4Hz, 1H), |
| | 3.95-4.20 (m, 4H), 3.81 (s, 3H), 2.05-2.20 (m, 5H), |
| | 1.25-1.50 (m, 8H), 0.65 (t, J=7.5Hz, 3H). |
| 3-103 | δ 7.23-7.34 (m, 4H), 6.97 (dd, J=8.9, 2. 4Hz, 1H), |
| | 6.84-6.93 (m, 2H), 6.74 (brs, 1H), 4.02-4.12 (m, 2H), |
| | 3.97 (t, J=6.7Hz, 2H), 3.71 (s, 3H), 2.14 (s, 3H), |
| | 1. 75-1. 90 (m, 2H), 1.32 (t, J=7.0Hz, 3H), |
| | 1.04 (t, J=7.3Hz, 3H). |
| 3-104 | δ 7.33 (d, J=8.5Hz, 1H), 7.27-7.31 (m, 2H), 7.19-7.26 (m, 1H), |
| | 6.97 (dd, J=8. 5, 2. 4Hz, 1H), 6.81-6.90 (m, 2H), |
| | 3. 94-4. 06 (m, 4H), 3.74 (s, 3H), 3.38 (s, 3H), 2.09 (s, 3H), |
| | 1. 75-1. 90 (m, 2H), 1.31 (t, J=7.0Hz, 3H), |
| | 1.04 (t, J=7.3Hz, 3H). |
| 3-106 | δ 7. 31-7. 38 (m, 2H), 7.22-7.30 (m, 2H), |
| | 6.98 (dd, J=8.9, 2. 4Hz, 1H), |
| | 6.90 (td, J=7. 5, 1.0Hz, 1H), 6. 82-6. 87 (m, 1H), |
| | 4.01 (td, J=6.5, 0.7Hz, 2H), 3.73 (s, 3H), 3.68 (s, 3H), |
| | 3.37 (s, 3H), 2.09 (s, 3H), 1. 73-1. 84 (m, 2H), |
| | 1.43-1.57 (m, 2H), 0.97 (t, J=7.3Hz, 3H). |
| 3-109 | δ 7.27-7.38 (m, 4H), 6.90-6.98 (m, 3H), 6.36 (brs, 1H), |
| | 4.76-4.86 (m, 1H), 3.80 (s, 3H), 3.73 (s, 3H), 2.17 (s, 3H), |
| | 1.75-1.98 (m, 6H), 1. 59-1. 70 (m, 2H). |
| 3-110 | δ7.32-7.37 (m, 2H), 7.22-7.29 (m, 2H), 6.82-6.97 (m, 3H), |
| | 4.77-4.85 (m, 1H), 3.73 (s, 3H), 3.68 (s, 3H), |
| | 3.37 (s, 3H), 2.09 (s, 3H), 1. 72-1. 95 (m, 6H), |
| | 1.54-1.70 (m, 2H). |
| 3-114 | δ 7. 24-7. 36 (m, 4H), 6.96-7.01 (m, 1H), 6.88-6.94 (m, 2H), |
| | 6.72 (brs, 1H), 6.10 (tt, J=54. 9Hz, 4.4Hz, 1H), |
| | 4.21 (tdd, J=13.0Hz, 3.8Hz, 0.7Hz, 2H), 3.76 (s, 3H), |
| | 3.68 (s, 3H), 2.15 (s, 3H). |
| 3-117 | δ 7. 24-7. 44 (m, 4H), 7.03 (dd, J=8.9, 2. 4Hz, 1H), |
| | 6.83-6.95 (m, 2H), 4.40 (q, J=8.2Hz, 2H), 3.73 (s, 3H), |
| | 3.69 (s, 3H), 3.35 (s, 3H), 2.09 (s, 3H). |
| 3-122 | δ 7.29-7.39 (m, 3H), 7.26 (td, J=7.8, 1. 7Hz, 1H), |
| | 7.02 (dd, J=8.9, 2.4Hz, 1H), |
| | 6.90 (td, J=7.5, 1.0Hz, 1H), 6.85 (d, J=8.5Hz, 1H), |
| | 4.16 (dd, J=5.5, 4.1Hz, 2H), 3.74-3.78 (m, 2H), |
| | 3.73 (s, 3H), 3.67 (s, 3H), 3.44 (s, 3H), 3.36 (s, 3H), |
| | 2.09 (s, 3H). |
| 3-125 | δ 7.38 (d, J=8. 5Hz, 1H), 7.34 (dd, J=7. 5, 1. 7Hz, 1H), |
| | 7.31 (d, J=2.1Hz, 1H), 7.20-7.28 (m, 1H), |
| | 6.99 (dd, J=8.9, 2.4Hz, 1H), 6.89 (td, J=7. 5, 1.0Hz, 1H), |
| | 6.84 (d, J=8.2Hz, 1H), 4.19 (t, J=6.8Hz, 2H), 3.73 (s, 3H), |
| | 3.66 (s, 3H), 3.36 (s, 3H), 2.87 (t, J=7.2Hz, 2H), |
| | 2.18 (s, 3H), 2.08 (s, 3H). |
| 3-129 | δ7.28-7.36 (m, 4H), 7.00 (dd, J=8.9Hz, 2.1Hz, 1H), |
| | 6.90-6.97 (m, 2H), 6.36 (brs, 1H), 3.87 (d, J=7.2Hz, 2H), |
| | 3.80 (s, 3H), 3.73 (s, 3H), 2.17 (s, 3H), 1. 24-1. 39 (m, 1H), |
| | 0.62-0.71 (m, 2H), 0.34-0.41 (m, 2H). |
| 3-142 | δ 7.44-7.52 (m, 3H), 7.30-7.34 (m, 1H), 7.06-7.13 (m, 3H), |
| | 6.52 (t, J=74. 1Hz, 1H), 3.76 (s, 3H), 2.23 (s, 3H), |
| | 2.00-2.15 (m, 2H), 1. 20-1. 33 (m, 2H), 0.57 (t, J=7.8Hz, 3H). |
| 3-148 | δ7.79-7.80 (m, 1H), 7.45-7.50 (m, 2H), 7.29-7.30 (m, 2H), |
| | 7.06-7.07 (m, 2H), 6.52 (t, J=74. 1Hz, 1H), 3.76 (s, 3H), |
| | 2.23 (s, 3H), 2. 00-2. 18 (m, 2H), 1. 21-1. 34 (m, 2H), |
| | 0.58 (t, J=7.5Hz, 3H). |
| 3-152 | δ7.59-7.60 (m, 1H), 7.45-7.50 (m, 2H), 7.24-7.26 (m, 1H), |
| | 7.13-7.16 (m, 1H), 7.04-7.09 (m, 2H), 6.52 (t, J=74.7Hz, 1H), |
| | 3.30 (s, 3H), 2.46 (s, 3H), 2.23 (s, 3H), 1. 95-2. 16 (m, 2H), |
| | 1.19-1.31 (m, 2H), 0.56 (t, J=7.5Hz, 3H). |
| 3-155 | δ 7.24-7.34 (m, 4H), 6.84-6.96 (m, 3H), 5.81-5.82 (m, 1H), |
| | 5.52-5.53 (m, 1H), 3.87 (s, 3H), 3.83 (s, 3H), 3.65 (s, 3H), |
| | 2.10 (s, 3H), 1. 67-1. 69 (m, 3H). |
| 3-157 | δ7.09-7.33 (m, 7H), 6.84-7.00 (m, 5H), 3.89 and 3.87 (s, 3H), |
| | 3.77 (s, 3H), 3.70 and 3.65 (s, 3H), 3.53 (q, J=7.5Hz, 1H), |
| | 1.87 and 1.75 (s, 3H), 1.20 (dd, J=15.3Hz, 6.9Hz, 3H). |
| 3-158 | δ 7.24-7.47 (m, 5H), 7. 15-7. 18 (m, 1H), |
| | 6.95 (dd, J=9.0Hz, 2.4Hz, 1H), 6.79-6.88 (m, 4H), |
| | 3.85 (s, 6H), 3.66 (s, 3H), 3.53 (s, 3H), 2.17 (s, 3H). |
| 3-167 | δ 7.87 (dd, J=1.5, 0.9Hz, 1H), 7.10-7.45 (m, 4H), |
| | 6.85-7.00 (m, 2H), 4.15 (q, J=7. 1 Hz, 2H), 3.75 (s, 3H), |
| | 3.70 (s, 3H), 3.36 (s, 3H), 3.10-3.25 (m, 2H), |
| | 2.55-2.65 (m, 2H), 2.10 (s, 3H), 1.26 (t, J=7.1Hz, 3H). |
| 3-171 | δ7.85-7.90 (m, 1H), 7.25-7.45 (m, 4H), 6.85-7.00 (m, 2H), |
| | 3.95-4.10 (m, 2H), 3.74 (s, 3H), 3.70 (s, 3H), 3.36 (s, 3H), |
| | 3.10-3.25 (m, 2H), 2.55-2.70 (m, 2H), 2.10 (s, 3H), |
| | 1.50-1.65 (m, 1H), 1. 20-1. 45 (m, 8H), 0.85-0.95 (m , 6H). |
| 3-172 | δ 7.74 (s, 1H), 7.27-7.40 (m, 2H), 7.03-7.11 (m, 1H), |
| | 6.82-6.99 (m, 2H), 6. 68-6. 73 (m, 1H), 6.56-6.61 (m, 1H), |
| | 3.81 (s, 3H), 3.71 (s, 3H), 3.45 (s, 3H), 2.13 (s, 3H). |
| 4-002 | δ7.43-7.51 (m, 2H), 7.25-7.34 (m, 1H), 7. 16-7. 18 (m, 1H), |
| | 7.00-7.09 (m, 3H), 3.76 (s, 3H), 2.23 (s, 3H), |
| | 2.03-2.12 (m, 2H), 1. 21-1. 33 (m, 2H), |
| | 0.58 (t, J=7.5Hz, 3H). |
| 4-004 | δ7.20-7.36 (m, 4H), 6.89-7.04 (m, 3H), 3.82 (s, 3H), |
| | 3.73 (s, 3H), 2.12 (t, J=7.2Hz, 2H), 2.10 (s, 3H), |
| | 1.28-1.40 (m, 2H), 0.66 (t, J=7.5Hz, 3H). |
| 4-006 | δ7.38-7.43 (m, 2H), 7.24-7.31 (m, 1H), 7. 13-7. 16 (m, 1H), |
| | 6.97-7.03 (m, 1H), 6.85-6.90 (m, 2H), 3.82 (s, 3H), |
| | 3.78 (s, 3H), 2.22 (s, 3H), 2. 05-2. 12 (m, 2H), |
| | 1.23-1.33 (m, 2H), 0.58 (t, J=7.5Hz, 3H). |
| 4-008 | δ 7.19-7.32 (m, 4H), 6.85-7.03 (m, 3H), |
| | 4.06 (q, J=7.2Hz, 2H), 3.82 (s, 3H), |
| | 2.13 (t, J=7.2Hz, 3H), 2.10 (s, 3H), 1. 30-1. 40 (m, 4H), |
| | 0.65 (t, J=7.5Hz, 3H). |
| 4-010 | δ7.35-7.45 (m, 2H), 7.20-7.35 (m, 1H), 7.10-7.20 (m, 1H), |
| | 6.95-7.05 (m, 1H), 6.80-6.90 (m, 2H), 3.92 (t, J=6. 5Hz, 2H), |
| | 3.78 (s, 3H), 2.21 (s, 3H), 2.08 (td, J=7.2, 2.2Hz, 2H), |
| | 1. 70-1. 90 (m, 2H), 1. 20-1. 40 (m, 2H), 1.03 (t, J=7.3 Hz, 3H), |
| | 0.58 (t, J=7.5Hz, 3H). |
| 4-012 | δ 7.19-7.32 (m, 3H), 6.98-7.04 (m, 1H), 6.60-6.66 (m, 2H), |
| | 3.81 (s, 3H), 3.74 (s, 3H), 2.13 (s, 3H), |
| | 2.11 (t, J=7.2Hz, 2H), 1. 27-1. 40 (m, 2H), |
| | 0.65 (t, J=7.5Hz, 3H). |
| 4-016 | δ 7.42-7.50 (m, 2H), 7.27-7.36 (m, 3H), 7.02-7.10 (m, 2H), |
| | 3.76 (s, 3H), 2.22 (s, 3H), 2.15-2.05 (m, 2H), |
| | 1.32-1.22 (m, 2H), 0.58 (t, J=7.2Hz, 3H). |
| 4-018 | δ 7.28-7.44 (m, 7H), 3.77 (s, 3H), 2.22 (s, 3H), |
| | 2.04-2.14 (m, 2H), 1. 22-1. 32 (m, 2H), 0.58 (t, J=7.2Hz, 3H). |
| 4-020 | δ7.26-7.38 (m, 5H), 7. 12-7. 18 (m, 2H), 3.78 (s, 3H), |
| | 2.36 (s, 3H), 2.20 (s, 3H), 2. 12-2. 05 (m, 2H), |
| | 1.34-1.20 (m, 2H), 0.59 (t, J=7.2Hz, 3H). |
| 4-022 | δ7.24-7.36 (m, 5H), 6.88-6.98 (m, 2H), 3.83 (s, 3H), |
| | 3.73 (s, 3H), 2.12 (t, J=7.2Hz, 2H), 2.09 (s, 3H), |
| | 1.28-1.42 (m, 2H), 0.66 (3H, J=7.2Hz, 3H). |
| 4-024 | δ7.38-7.44 (m, 2H), 7.25-7.33 (m, 3H), 6.84-6.90 (m, 2H), |
| | 3.82 (s, 3H), 3.78 (s, 3H), 2.21 (s, 3H), 2.15-2.05 (m, 2H), |
| | 1.22-1.32 (m, 2H), 0.59 (t, J=7.2Hz, 3H). |
| 4-026 | δ7.22-7.36 (m, 5H), 6.84-6.98 (m, 2H), 4.06 (q, J=7.2Hz, 3H), |
| | 3.82 (s, 3H), 2.13 (t, J=7.2Hz, 2H), 2.10 (s, 3H), |
| | 1. 30-1. 40 (m, 2H), 1.37 (t, J=7.2Hz, 3H), |
| | 0.66 (t, J=7.2Hz, 3H). |
| 4-028 | δ 7.35-7.45 (m, 2H), 7.25-7.35 (m, 3H), 6.80-6.90 (m, 2H), |
| | 3.92 (t, J=6.6Hz, 2H), 3.79 (s, 3H), 2.20 (s, 3H), |
| | 2.08 (td, J=7.2, 2.0 Hz, 2H), 1.70-1.90 (m, 2H), |
| | 1. 20-1. 35 (m, 2H), 1.03 (t, J=7. 5Hz, 3H), |
| | 0.58 (t, J=7.3Hz, 3H). |
| 4-030 | δ 7.60-7.65 (m, 4H), 7.30-7.40 (m, 3H), 3.75 (s, 3H), |
| | 2.24 (s, 3H), 2.02-2.15 (m, 2H), 1. 20-1. 34 (m, 2H), |
| | 0.57 (t, J=7.2Hz, 3H). |
| 4-032 | δ 7.62-7.66 (m, 4H), 7.32-7.42 (m, 3H), 3.74 (s, 3H), |
| | 2.26 (s, 3H), 2. 06-2. 12 (m, 2H), 1. 22-1. 32 (m, 2H), |
| | 0.56 (t, J=7.5Hz, 3H). |
| 4-034 | δ 7.24-7.36 (m, 4H), 6.68-6.60 (m, 2H), 3.80 (s, 3H), |
| | 3.74 (s, 3H), 2.08-2.16 (m, 2H), 2.04 (s, 3H), |
| | 1.42-1.28 (m, 2H), 0.65 (t, J=7.2Hz, 3H). |
| 4-036 | δ 7.20-7.34 (m, 4H), 6.55-6.70 (m, 2H), 4.03 (q, J=7.2Hz, 2H), |
| | 3.81 (s, 3H), 2.05-2.16 (m, 2H), 2.04 (s, 3H), |
| | 1. 28-1. 42 (m, 2H), 1.26 (t, J=7.2Hz, 3H), |
| | 0.65 (t, J=7.2Hz, 3H). |
| 4-038 | δ7.50-7.60 (m, 2H), 7. 30-7. 40 (m, 4H), 3.77 (s, 3H), |
| | 2.10-2.20 (m, 2H), 2.18 (s, 3H), 1. 22-1. 42 (m, 2H), |
| | 0.63 (t, J=7.2Hz, 3H). |
| 4-039 | δ 7.25-7.40 (m, 3H), 7.06 (dd, J=7.8, 0.7Hz, 1H), |
| | 6.90-7.00 (m, 2H), 6.75-6.85 (m, 1H), 6.40 (brs 1H), |
| | 4.02 (s, 3H), 3.80 (s, 3H), 3.74 (s, 3H), 2.19 (s, 3H). |
| 4-043 | δ7.38-7.41 (m, 2H), 7.24-7.32 (m, 1H), 7.15-7.21 (m, 3H), |
| | 6.98-7.04 (m, 1H), 3.77 (s, 3H), 2.91 (q, J=6.6Hz, 1H), |
| | 2.21 (s, 3H), 2.06-2.12 (m, 2H), 1. 23-1. 32 (m, 8H), |
| | 0.58 (t, J=7.5Hz, 3H). |
| 4-045 | δ 7.62 (s, 4H), 7.30-7.37 (m, 1H), 7.19-7.22 (m, 1H), |
| | 7.04-7.10 (m, 1H), 3.75 (s, 3H), 2.28 (s, 3H), |
| | 2.01-2.18 (m, 2H), 1. 21-1. 33 (m, 2H), 0.57 (t, J=7.8Hz, 3H). |
| 4-047 | δ 7.47-7.52 (m, 2H), 7.27-7.34 (m, 1H), 7.17 (d, J=8.4Hz, 1H), |
| | 7.01-7.12 (m, 3H), 6.53 (t, J=74. 1Hz, 1H), 3.75 (s, 3H), |
| | 2.23 (s, 3H), 2.00-2.18 (m, 2H), 1.21-133 (m, 2H), |
| | 0.57 (t, J=7.5Hz, 3H). |
| 4-049 | δ 7.51-7.56 (m, 2H), 7.28-7.35 (m, 1H), 7.18-7.22 (m, 3H), |
| | 7.03-7.09 (m, 1H), 3.75 (s, 3H), 2.24 (s, 3H), |
| | 2.00-2.16 (m, 2H), 1. 21-1. 33 (m, 2H), 0.57 (t, J=6.9Hz, 3H). |
| 4-053 | δ 7.69-7.79 (m, 1H), 7.64 (s, 4H), 7. 31-7. 38 (m, 1H), |
| | 7.06-7.12 (m, 1H), 3.73 (s, 3H), 2.26 (s, 3H), |
| | 2.01-2.16 (m, 2H), 1.22-1.30 (m, 2H), 0.56 (t, J=7.8Hz, 3H). |
| 4-055 | δ 7.17-7.41 (m, 5H), 7.03-7.09 (m, 1H), 3.76 (s, 3H), |
| | 2.26 (s, 3H), 2. 02-2. 17 (m, 2H), 1. 23-1. 30 (m, 2H), |
| | 0.57 (t, J=7.5Hz, 3H). |
| 4-059 | δ 7.28-7.40 (m, 5H), 7.08-7.16 (m, 2H), 3.80 (s, 3H), |
| | 2.14 (t, J=7.2Hz, 2H), 2.13 (s, 3H), 1.30-1.40 (m, 2H), |
| | 0.65 (t, J=7.2Hz, 3H). |
| 4-065 | δ 7.22-7.36 (m, 4H), 7.02-7.10 (m, 2H), 6.86-6.92 (m, 1H), |
| | 3.77 (s, 3H), 3.75 (s, 3H), 2.08-2.12 (m, 2H), 2.21 (s, 3H), |
| | 1.20-1.34 (m, 2H), 0.58 (t, J=7.2Hz, 3H). |
| 4-067 | δ 7. 20-7. 35 (m, 4H), 7.00-7.06 (m, 2H), 6.84-6.90 (m, 1H), |
| | 3.97 (q, J=7.2Hz, 2H), 3.77 (s, 3H), 2.20 (s, 3H), |
| | 2.09 (dt, J=7.2, 1.5Hz, 2H), 1.37 (t, J=7.2Hz, 3H), |
| | 1.22-1.32 (m, 2H), 0.58 (t, J=7.2Hz, 3H). |
| 4-069 | δ 7.20-7.36 (m, 5H), 6.82-6.96 (m, 2H), 3.99 (t, J=7.2Hz, 2H), |
| | 3.82 (s, 3H), 2.12 (t, J=7.2Hz, 2H), 2.09 (s, 3H), |
| | 1. 70-1. 82 (m, 2H), 1. 45-1. 60 (m, 2H), 1. 28-1. 42 (m, 2H), |
| | 0.97 (t, J=7.2Hz, 3H), 0.65 (t, J=7.2Hz, 3H). |
| 4-073 | δ 7. 78-7. 82 (m, 1H), 7.64-7.70 (m, 1H), 7.58-7.64 (m, 1H), |
| | 7.44-7.52 (m, 1H), 7. 32-7. 40 (m, 3H), 3.76 (s, 3H), |
| | 2.21 (s, 3H), 2.02-2.14 (m, 2H), 1. 20-1. 35 (m, 2H), |
| | 0.59 (t, J=7.2Hz, 3H). |
| 4-075 | δ7.48-7.53 (m, 2H), 7.28-7.36 (m, 3H), 7.07-7.12 (m, 2H), |
| | 6.53 (t, J=74.1Hz, 1H), 3.76 (s, 3H), 2.23 (s, 3H), |
| | 2.01-2.15 (m, 2H), 1.21-1.31 (m, 2H), 0.58 (t, J=7.5Hz, 3H). |
| 4-077 | δ 7.20-7.50 (m, 7H), 3.77 (s, 3H), 2.08-2.16 (m, 5H), |
| | 1.22-1.42 (m, 2H), 0.65 (t, J=7.2Hz, 3H). |
| 4-079 | δ 7. 50-7. 56 (m, 2H), 7.30-7.38 (m, 3H), 7.16-7.22 (m, 2H), |
| | 3.75 (s, 3H), 2.23 (s, 3H), 2. 00-2. 16 (m, 2H), |
| | 1.22-1.32 (m, 2H), 0.57 (t, J=7.2Hz, 3H). |
| 4-080 | δ 7.25-7.35 (m, 5H), 6.88-6.96 (m, 2H), 5.03 (brs, 1H), |
| | 3.75 (s, 3H), 2.12 (s, 3H), 1. 95-2. 05 (m, 2H), |
| | 1.20-1.30 (m, 2H), 0.66 (t, J=7.2Hz, 3H). |
| 4-085 | δ 7.25-7.35 (m, 10H), 6.88-6.96 (m, 2H), 5.13 (d, J=1.8Hz, 2H), |
| | 3.69 (s, 3H), 2.10 (s, 3H), 1. 96-2. 04 (m, 2H), |
| | 1. 16-1. 26 (m, 2H), 0.50 (t, J=7.2Hz, 3H). |
| 4-087 | δ 7.27-7.36 (m, 6H), 7.06-7.14 (m, 1H), 3.76 (s, 3H), |
| | 2.44 (s, 3H), 2.23 (t, J=7.2Hz, 2H), 2.11 (s, 3H), |
| | 1.32-1.46 (m, 2H), 0.68 (t, J=7.2Hz, 3H). |
| 4-093 | δ 7.40-7.44 (m, 1H), 7.28-7.36 (m, 6H), 3.77 (s, 3H), |
| | 2.80-2.94 (m, 2H), 2.20 (s, 3H), 2.06-2.12 (m, 2H), |
| | 1.20-1.32 (m, 5H), 0.58 (t, J=7.2Hz, 3H). |
| 4-095 | δ7.22-7.42 (m, 7H), 3.77 (s, 3H), 2.97 (q, J=7.2Hz, 2H), |
| | 2.21 (s, 3H), 2.09 (dt, J=7.2Hz, 2.4Hz, 2H), |
| | 1.34 (t, J=7.2Hz, 3H), 1.22-1.32 (m, 2H), |
| | 0.58 (t, J=7.2Hz, 3H). |
| 4-097 | δ7.36-7.42 (m, 2H), 7.28-7.35 (m, 5H), 3.77 (s, 3H), |
| | 3.40-3.50 (m, 1H), 2.21 (s, 3H), 2.09 (dt, J=7.2, 2.4Hz, 2H), |
| | 1.32 (d, J=7.2Hz, 6H), 1.22-1.30 (m, 2H), |
| | 0.58 (t, J=7.2Hz, 3H). |
| 4-101 | δ 7.44-7.52 (m, 2H), 7.20-7.30 (m, 2H), 7. 10-7. 15 (m, 1H), |
| | 6.98-7.08 (m, 2H), 3.75 (s, 3H), 2.56 (s, 3H), 2.23 (s, 3H), |
| | 2.06-2.14 (m, 2H), 1. 24-1. 30 (m, 2H), 0.55 (t, J=7.2Hz, 3H). |
| 4-105 | δ7.35-7.40 (m, 2H), 7.20-7.30 (m, 3H), 7. 10-7. 15 (m, 2H), |
| | 3.76 (s, 3H), 2.56 (s, 3H), 2.23 (s, 3H), 2. 10-2. 18 (m, 2H), |
| | 1.20-1.30 (m, 2H), 0.54 (t, J=7.2Hz, 3H). |
| 4-109 | δ 7.20-7.30 (m, 3H), 7.05-7.15 (m, 3H), 6.82-6.88 (m, 1H), |
| | 3.76 (s, 3H), 3.74 (s, 3H), 2.57 (s, 3H), 2.21 (s, 3H), |
| | 2.04-2.10 (m, 2H), 1. 20-1. 30 (m, 2H), 0.55 (t, J=7.2Hz, 3H). |
| 4-113 | δ 7.22-7.30 (m, 4H), 7.08-7.15 (m, 1H), 6.84-6.94 (m, 2H), |
| | 4.04 (q, J=7.2Hz, 2H), 3.81 (s, 3H), 2.55 (s, 3H), |
| | 2.08-2.16 (m, 5H), 1. 30-1. 40 (m, 5H), 0.64 (t, J=7.2Hz, 3H). |
| 4-115 | δ 7.18-7.28 (m, 3H), 7. 10-7. 14 (m, 1H), 7.04-7.08 (m, 2H), |
| | 6.80-6.88 (m, 1H), 3. 92-4. 02 (m, 2H), 3.76 (s, 3H), |
| | 2.56 (s, 3H), 2.21 (s, 3H), 2.05-2.10 (m, 2H), |
| | 1.36 (t, J=7.2Hz, 3H), 1.20-1.30 (m, 2H), |
| | 0.55 (t, J=7.2Hz, 3H). |
| 4-117 | δ7.20-7.34 (m, 5H), 7.05-7.15 (m, 2H), 3.75 (s, 3H), |
| | 2.56 (s, 3H), 2.43 (s, 3H), 2.20 (t, J=7.5Hz, 2H), |
| | 2.11 (s, 3H), 1. 30-1. 45 (m, 2H), 0.66 (t, J=7. 5Hz, 3H). |
| 4-123 | δ 7.42-7.46 (m, 1H), 7.20-7.32 (m, 5H), 7. 10-7. 14 (m, 1H), |
| | 3.76 (s, 3H), 2.80-2.94 (m, 2H), 2.57 (s, 3H), 2.21 (s, 3H), |
| | 2.05-2.10 (m, 2H), 1. 30-1. 40 (m, 5H), 0.55 (t, J=7.2Hz, 3H). |
| 4-131 | δ7.52-7.58 (m, 2H), 7. 15-7. 30 (m, 5H), 3.74 (s, 3H), |
| | 2.57 (s, 3H), 2.24 (s, 3H), 2. 00-2. 15 (m, 2H), |
| | 1.20-1.30 (m, 2H), 0.54 (t, J=7.5Hz, 3H). |
| 5-010 | δ7.56-7.61 (m, 1H), 7.39-7.42 (m, 2H), 7.30-7.33 (m, 2H), |
| | 7. 12-7. 18 (m, 1H), 4.56 (s, 2H), 3.79 (s, 3H), |
| | 3.41 (s, 3H), 2.20 (s, 3H), 2.09-2.14 (m, 2H), |
| | 1.29 (q, J=6.0Hz, 2H), 0.60 (t, J=6.0Hz, 3H). |
| 5-042 | δ 7.88 (s, 1H), 7.50 (s, 1H), 7.25-7.40 (m, 2H), |
| | 6.85-7.00 (m, 2H), 3.84 (s, 3H), 3.73 (s, 3H), |
| | 2.05-2.20 (m, 5H), 1.30-1.45 (m, 2H), |
| | 0.69 (t, J=7.4Hz, 3H). |
| 5-052 | δ 7.88 (s, 1H), 7.49 (s, 1H), 7.20-7.35 (m, 1H), |
| | 6.60-6.70 (m, 2H), 3.82 (s, 3H), 3.73 (s, 3H), |
| | 2. 10-2.25 (m, 5H), 1. 30-1. 45 (m, 2H), 0.68 (t, J=7.4Hz, 3H). |
| 5-054 | δ 7.88 (s, 1H), 7.47 (s, 1H), 7.21 (dd, J=8.3, 6. 7Hz, 1H), |
| | 6.50-6.70 (m, 2H), 4.04 (q, J=7.0Hz, 2H), 3.82 (s, 3H), |
| | 2.10-2.20 (m, 5H), 1. 30-1. 45 (m, 5H), 0.67 (t, J=7.4Hz, 3H). |
| 5-061 | δ7.25-7.30 (m, 1H), 7.21 (s, 1), 6.79 (s, 1H), |
| | 6.55-6.65 (m, 2H), 3.94 (s, 3H), 3.87 (s, 3H), |
| | 3.79 (s, 3H), 3.70 (s, 3H), 2.14 (s, 3H), |
| | 2.12 (t, J=7.5Hz, 2H), 1. 30-1. 40 (m, 2H), |
| | 0.66 (t, J=7.5Hz, 3H). |
| 5-076 | δ 7.40 (s, 1H), 7.20-7.30 (m, 2H), 6.55-6.70 (m, 2H), |
| | 3.96 (s, 3H), 3.80 (s, 3H), 3.73 (s, 3H), |
| | 2.05-2.20 (m, 5H), 1. 25-1. 45 (m, 2H), 0.67 (t, J=7.5Hz, 3H). |
| 5-080 | δ 7.15-7. 35 (m, 6H), 6.80-7.05 (m, 4H), 3.95-4.15 (m, 4H), |
| | 3.83 (s, 3H), 3.74 (s, 3H), 2.14 (td, J=7.2, 1. 5 Hz, 2H), |
| | 2.09 (s, 3H), 1. 20-1. 45 (m, 8H), 0.66 (t, J=7. 3 Hz, 3H). |
| 8-009 | δ 7. 60-7. 70 (m, 2H), 7. 15-7. 55 (m, 9H), 6.95-7.00 (m, 2H), |
| | 5.08 (s, 2H), 3.76 (s, 3H), 3.48 (s, 3H), 2.32 (s, 3H). |
| 9-005 | δ7. 60-7. 70 (m, 2H), 7.38 (d, J=8. 7Hz, 1H), 7.00-7.15 (m, 4H), |
| | 3.80 (s, 3H), 2.42 (s, 3H), 2.30 (s, 3H), 2.00-2.15 (m, 2H), |
| | 1.20-1.35 (m, 2H), 0.58 (t, J=7.2H, 3H). |
| 9-008 | δ7. 55-7. 65 (m, 2H), 7. 30-7. 40 (m, 1H), 7.00-7.10 (m, 2H), |
| | 6.85-6. 95 (m, 2H), 3.83 (s, 3H), 3.81 (s, 3H), 2.41 (s, 3H), |
| | 2.29 (s, 3H), 2.00-2.15 (m, 2H), 1. 20-1. 35 (m, 2H), |
| | 0.58 (t, J=7.5Hz, 3H). |
| 9-010 | δ7. 60-7. 70 (m, 1H), 7.36 (d, J=8.1Hz, 1H), 7.15-7.25 (m, 1H), |
| | 7.05-7.15 (m, 1H), 6.65-6.75 (m, 1H), 6. 55-6. 65 (m, 1H), |
| | 3.85-4.00 (m, 2H), 3.83 (s, 3H), 2.42 (s, 3H), |
| | 2.18 (s, 3H), 1. 90-2. 05 (m, 2H), 1. 25-1. 40 (m, 2H), |
| | 1.13 (t, J=7.2Hz, 3H), 0.70 (t, J=7.5Hz, 3H). |
| 9-026 | δ7. 55-7. 60 (m, 2H), 7.35-7.45 (m, 1H), 6.90-7.05 (m, 2H), |
| | 6.80-6.90 (m, 2H), 4.50-4.65 (m, 1H), 3.80 (s, 3H), |
| | 2.29 (s, 3H), 2.00-2.20 (m, 2H), 1.20-1.40 (m, 8H), |
| | 0.59 (t, J=7.5Hz, 3H). |
| 9-040 | δ7.55-7.60 (m, 2H), 7.40-7.50 (m, 1H), 7.25-7.30 (m, 2H), |
| | 6.95-7.05 (m, 2H), 3.81 (s, 3H), 2.98 (q, J=7.3Hz, 2H), |
| | 2.29 (s, 3H), 2.05-2.20 (m, 2H), 1. 20-1. 40 (m, 5H), |
| | 0.59 (t, J=7.5Hz, 3H). |
| 9-042 | δ7. 55-7. 60 (m, 2H), 7.40-7.50 (m, 1H), 7. 30-7. 40 (m, 2H), |
| | 6.95-7.05 (m, 2H), 3.81 (s, 3H), 3.40-3.50 (m, 1H), |
| | 2.29 (s, 3H), 2.00-2.20 (m, 2H), 1.20-1.40 (m, 8H), |
| | 0.59 (t, J=7.5Hz, 3H). |
| 9-048 | δ7.69 (dd, J=8.6, 4. 6Hz, 1H), 6.95-7.15 (m, 3H), |
| | 6.83 (d, J=4.0Hz, 1H), 3.83 (s, 3H), 2.29 (s, 3H), |
| | 2.05-2.20 (m, 2H), 1. 15-1. 35 (m, 2H), 0.56 (t, J=7.4Hz, 3H). |
| 9-058 | δ7. 50-7. 60 (m, 2H), 7. 35-7. 40 (m, 1H), 7.20-7.30 (m, 2H), |
| | 7.05-7.15 (m, 2H), 3.81 (s, 3H), 2.97 (q, J=7.2Hz, 2H), |
| | 2.41 (s, 3H), 2.29 (s, 3H), 1. 95-2. 15 (m, 2H), |
| | 1.20-1.40 (m, 5H), 0.58 (t, J=7.3Hz, 3H). |
| 9-060 | δ7. 55-7. 60 (m, 2H), 7.30-7.40 (m, 3H), 7.05-7.15 (m, 2H), |
| | 3.81 (s, 3H), 3.35-3.55 (m, 1H), 2.41 (s, 3H), 2.29 (s, 3H), |
| | 1. 95-2. 15 (m, 2H), 1. 20-1. 35 (m, 8H), 0.58 (t, J=7. 5Hz, 3H). |
| 12-018 | δ7.73 (d, 8.2Hz, 1H), 7.35-7.65 (m, 6H), 7.21 (d, 8.2Hz, 1H), |
| | 3.87 (s, 2H), 3.75 (s, 3H), 2.51 (s, 3H). |
| 12-020 | δ7.61 (d, J=8.9Hz, 1H), 7.35-7.45 (m, 2H), |
| | 7.32 (d, J=2.4Hz, 1H), 6.95-7.10 (m, 3H), 3.89 (s, 3H), |
| | 3.79 (s, 3H), 3.69 (s, 2H), 3.67 (s, 3H). |
| 12-024 | δ7.66 (ddd, J=8.9, 8.9, 5.1Hz, 1H), |
| | 7.50 (dd, J=8.9, 2.4Hz, 1H), 7.35-7.45 (m, 2H), |
| | 6.95-7.20 (m, 3H), 4.12 (q, J=7.2 Hz, 2H), |
| | 3.78 (s, 3H), 3.68 (s, 2H), 1.21 (t, J=7.2Hz, 3H). |
| 12-036 | δ7.70 (d, J=8.3 Hz, 1H), 7.30-7.60 (m, 6H), |
| | 7. 10-7.20 (m, 1H), 4.15-4.40 (m, 4H), 3.39 (s, 3H), |
| | 2.46 (s, 3H), 2.26 (s, 3H), 1.20-1.40 (m, 3H). |
| 12-039 | δ7.25-7.50 (m, 4H), 6.90-7.10 (m, 2H), 6.70-6.85 (m, 1H), |
| | 4.15-4.30 (m, 2H), 4.07 (s, 2H), 3.99 (s, 3H), 3.77 (s, 3H), |
| | 3.31 (s, 3H), 2.18 (s, 3H), 1.20-1.35 (m, 3H). |
| 12-040 | δ7.28-7. 50 (m, 3H), 6.90-7.08 (m, 3H), 4.00-4.32 (m, 4H), |
| | 3.75 (s, 3H), 3.30 (s, 3H), 2.51 (s, 3H), 2.44 (s, 3H), |
| | 2.17 (s, 3H), 1.27 (t, J=7.2Hz, 3H). |

### [Referential Example 1]

### Synthesis of 4-(2-bromobenzo[b]thiophen-3-yl)-5-methoxy-2,6-dimethylpyridazin-3(2H)-one

### Step 1: production of 4-(benzo[b]thiophen-3-yl)-5-methoxy-2,6-dimethylpyridazin-3(2H)-one

Firstly, 1.00 g of benzo[b]thiophene-3-boronic acid, 2.39 g of potassium phosphate, 350 mg of 2-dicyclohexylphosphino-2',6'-diisopropoxybiphenyl, and 84 mg of palladium acetate were added to a mixture of 873 mg of 4-bromo-5-methoxy-2,6-dimethylpyridazin-3(2H)-one and 7.5 mL of toluene at room temperature. After completion of the addition, air in the reaction container was replaced with nitrogen gas. After completion of the gas replacement, the reaction mixture was stirred at 90°C for four hours. After completion of the stirring, 10 mL of water and 10 mL of ethyl acetate were added to the reaction mixture, and insoluble matter was filtered with celite. The residue was washed with ethyl acetate (10 mL × 2). The organic phase was separated from the resultant filtrate, and the resultant organic phase was washed with water (10 mL × 1). Thereafter, the organic phase was dehydrated and dried sequentially with saturated salt water and anhydrous sodium sulfate, and then the solvent was distilled off under reduced pressure. The resultant residue was purified by silica gel chromatography (elution with a gradient of ethyl acetate : n-hexane = 5 : 95 to 20 : 80), to thereby yield 1.05 g of the target product as a yellow oily product.

¹H NMR: δ 7.80-7.90 (m, 1H), 7.61 (s, 1H), 7.50-7.55 (m, 1H), 7.30-7.40 (m, 2H), 3.75 (s, 3H), 3.26 (s, 3H), 2.31 (s, 3H).

### Step 2: production of 4-(2-bromobenzo[b]thiophen-3-yl)-5-methoxy-2,6-dimethylpyridazin-3 (2H)-one

Firstly, 746 mg of N-bromosuccinimide was added to a mixture of 1.0 g of 4-(benzo[b]thiophen-3-yl)-5-methoxy-2,6-dimethylpyridazin-3(2H)-one and 5 mL of N,N-dimethylformamide at room temperature. After completion of the addition, the reaction mixture was stirred at 80°C for two hours. After completion of the stirring, 20 mL of 1 mol/L aqueous sodium hydroxide solution was added to the reaction mixture, and the resultant mixture was subjected to extraction with 60 mL of diethyl ether. The resultant organic phase was washed with water (10 mL × 1), and then dehydrated and dried sequentially with saturated salt water and anhydrous sodium sulfate. The solvent was distilled off under reduced pressure. The resultant residue was purified by silica gel column chromatography (elution with a gradient of ethyl acetate : n-hexane = 2 : 98 to 20 : 80), to thereby yield 962 mg of the target product as a white solid.
Melting point: 250-252°C
¹H NMR: δ 7.70-7.80 (m, 1H), 7.30-7.45 (m, 3H), 3.77 (s, 3H), 3.42 (s, 3H), 2.34 (s, 3H).

### [Referential Example 2]

### Production of 2-(5-fluorobenzo[b]thiophen-3-yl)acetic acid

### Step 1: synthesis of ethyl 2-(5-fluorobenzo[b]thiophen-3-yl)acetate

Firstly, a mixture of 32.3 g of potassium carbonate, 120 mL of N,N-dimethylformamide, and 25.0 g of 4-fluorobenzenethiol was cooled with ice, and ethyl 4-chloroacetoacetate was added dropwise to the mixture under ice cooling over one hour. After completion of the dropwise addition, the reaction mixture was stirred under ice cooling for one hour. After completion of the stirring, 250 mL of water was added to the reaction mixture, and the resultant mixture was subjected to extraction with 200 mL of toluene. The resultant organic phase was washed with 100 mL of water and 50 mL of aqueous saturated ammonium chloride solution, and then dehydrated and dried with anhydrous sodium sulfate. Thereafter, the solvent was distilled off under reduced pressure, to thereby yield a residue. The resultant residue was added dropwise under ice cooling over one hour to a suspension of 78.0 g of aluminum trichloride and 200 mL of 1,2-dichloroethane prepared in another reaction container. After completion of the dropwise addition, the reaction mixture was stirred at room temperature for 16 hours. After completion of the stirring, the reaction mixture was added dropwise over one hour to a mixture of 500 mL of ice and 100 mL of 1,2-dichloroethane. After completion of the dropwise addition, the reaction mixture was stirred at room temperature for two hours. After completion of the stirring, the organic phase was separated from the reaction mixture. The resultant organic phase washed with 200 mL of water, and then dehydrated and dried sequentially with saturated salt water and anhydrous sodium sulfate, followed by addition of 4.0 g of powdery activated carbon. After completion of the addition, the resultant suspension was stirred at room temperature for 30 minutes. After completion of the stirring, insoluble matter was filtered with celite, and the residue was washed with chloroform (50 mL × 2). The solvent was distilled off under reduced pressure, to thereby yield 32.5g of the target product as a brown oily product. This product was used in the next step without further purification.

¹H NMR: δ 7.77 (dd, J=8.9, 4.9Hz, 1H), 7.40-7.50 (m, 2H), 7.12 (ddd, 8.9, 8.9, 2.5Hz, 1H), 4.18 (q, J=7.2Hz, 2H), 3.81 (s, 2H), 1.27 (t, J=7.2Hz, 3H).

### Step 2: synthesis of 2-(5-fluorobenzo[b]thiophen-3-yl)acetic acid

Firstly, a solution of 13.5 g of potassium hydroxide in 100 mL of water was added to a mixture of 32.5 g of ethyl 2-(5-fluorobenzo[b]thiophen-3-yl)acetate, 150 mL of tetrahydrofuran, and 50 mL of ethanol at room temperature. After completion of the addition, the reaction mixture was stirred at room temperature for 18 hours. After completion of the stirring, the solvent contained in the reaction mixture was distilled off under reduced pressure, and 100 mL of water was added to the resultant residue. The resultant mixture was washed sequentially with 100 mL of toluene and 50 mL of hexane. After completion of the washing, 35% by mass hydrochloric acid was added to the resultant mixture under ice cooling so as to achieve a pH of 1, and the precipitated solid was recovered through filtration. The resultant solid was washed with water (50 mL × 2), to thereby yield 27.5 g of the target product as a white solid.

### Melting point: 106-108°C

¹H NMR: δ 7.77 (dd, J=9.00, 4.9Hz, 1H), 7.45 (s, 1H), 7.41 (dd, J=9.4, 2.5Hz, 1H), 7.12 (ddd, J=8.8, 8.8, 2.5Hz, 1H), 3.85 (s, 2H).

### Test Examples

The usefulness of the compound of the present invention as herbicide will next be described in detail with reference to the following Test Examples. However, the present invention should not be construed as being limited to these Examples.

### [Test Example 1] Herbicidal Activity Test by Application before Weed Generation under Submerged Conditions

After alluvial soil was placed into 1/10000 are of Wagner pot, water was poured and mixed to form a submerged condition having a water depth of 4 cm. Seeds of late watergrass, Japanese bulrush, and oval-leafed pondweed were sowed in a mixed manner in the aforementioned pot, and then 2.5 leaf stage rice plant seedlings were transplanted thereto. On the day of sowing seeds, the emulsion agent of the compound of the present invention prepared according to Formulation Example 2 was diluted with water so as to achieve a predetermined herbicide amount, and the diluted emulsion agent was applied to the surface of the water. The pot was placed in a greenhouse at 25°C to 30°C for growth of the plants. Three weeks after the herbicide application, the effect of the herbicide on each plant was evaluated according to the following criteria. The results are shown in Table 16.

### Evaluation Criteria

5: Herbicidal rate of 90% or more (almost completely withered)
4: Herbicidal rate of 70% or more and less than 90%
3: Herbicidal rate of 40% or more and less than 70%
2: Herbicidal rate of 20% or more and less than 40%
1: Herbicidal rate of 5% or more and less than 20%
0: Herbicidal rate of 5% or less (almost no effect)

### [Test Example 2] Herbicidal Activity Test by Application during Weed Generation under Submerged Conditions

After alluvial soil was placed into 1/10000 are of Wagner pot, water was poured and mixed to form a submerged condition having a water depth of 4 cm. Seeds of late watergrass, Japanese bulrush, and oval-leafed pondweed were sowed in a mixed manner in the aforementioned pot, and the pot was placed in a greenhouse at 25°C to 30°C for growth of the plants. When late watergrass, Japanese bulrush, and oval-leafed pondweed were grown to one leaf stage to two leaf stage, the emulsion agent of the compound of the present invention prepared according to Formulation Example 2 was diluted with water so as to achieve a predetermined herbicide amount, and the diluted emulsion agent was applied to the surface of the water. Three weeks after the herbicide application, the effect of the herbicide on each plant was evaluated according to the criteria of Test Example 1. The results are shown in Table 17.

### [Test Example 3] Herbicidal Effect Test by Foliage Application

After alluvial soil was placed into 1/10000 are of Wagner pot, water was poured and mixed to form a submerged condition having a water depth of 0.1 cm to 0.5 cm. Seeds of barnyard grass, Chinese sprangletop, rice flat sedge, and rice were sowed and the pot was placed in a greenhouse at 25°C to 30°C for growth of the plants. After 14-day growth of the plants, the emulsion agent of the compound of the present invention prepared according to Formulation Example 2 was diluted with water so as to achieve a predetermined herbicide amount, and the diluted emulsion agent was uniformly applied to a foliage part with a small-sized spray. Three weeks after the herbicide application, the effect of the herbicide on each plant was evaluated according to the criteria of Test Example 1. The results are shown in Table 18.

### [Test Example 4] Herbicidal Effect Test by Soil Treatment

Sterilized diluvial soil was placed in a plastic box having a length of 21 cm, a width of 13 cm, and a depth of 7 cm. The seeds of each of southern crabgrass, green foxtail, barnyard grass, wild oat, blackgrass, Italian ryegrass, windgrass, velvetleaf, redroot pigweed, lambsquarters, chickweed, false cleavers, persian speedwell, corn, soybean, rice, wheat, beet, and rapeseed were sowed in a spot-like manner and covered with the soil having a thickness of about 1.5 cm. Subsequently, the emulsion agent of the compound of the present invention prepared according to Formulation Example 2 was diluted with water so as to achieve a predetermined herbicide amount, and the diluted emulsion agent was uniformly applied to the surface of the soil with a small-sized spray. The plastic box was placed in a greenhouse at 25°C to 30°C for growth of the plants. Three weeks after the herbicide application, the effect of the herbicide on each plant was evaluated according to the criteria of Test Example 1. The results are shown in Table 19.

### [Test Example 5] Herbicidal Effect Test by Foliage Application

Sterilized diluvial soil was placed in a plastic box having a length of 21 cm, a width of 13 cm, and a depth of 7 cm. The seeds of each of southern crabgrass, green foxtail, barnyard grass, wild oat, blackgrass, Italian ryegrass, windgrass, velvetleaf, redroot pigweed, lambsquarters, chickweed, false cleavers, persian speedwell, corn, soybean, rice, wheat, beet, and rapeseed were sowed in a spot-like manner and covered with the soil having a thickness of about 1.5 cm. Thereafter, the plants were grown in a greenhouse at 25°C to 30°C. After 14-day growth of the plants, the emulsion agent of the compound of the present invention prepared according to Formulation Example 2 was diluted with water so as to achieve a predetermined herbicide amount, and the diluted emulsion agent was uniformly applied to a foliage part with a small-sized spray. Three weeks after the herbicide application, the effect of the herbicide on each plant was evaluated according to the criteria of Test Example 1. The results are shown in Table 20.

### The symbols in Tables 16 to 20 have the following meanings.

A: late watergrass, B: Japanese bulrush, C: oval-leafed pondweed, D: Chinese sprangletop, E: rice flat sedge, F: southern crabgrass, G: green foxtail, H: barnyard grass, I: wild oat, J: blackgrass, K: Italian ryegrass, L: windgrass, M: velvetleaf, N: redroot pigweed, O: lambsquarters, P: chickweed, Q: false cleavers, R: persian speedwell, a: transplanted rice, b: directly sowed rice, c: corn, d: soybean, e: wheat, f: beet, and g: rapeseed.

The term "amount of applied herbicide (g/ha)" refers to the case where the concentration is adjusted so that the herbicide is applied in a described amount (g) per hectare (ha).

**Table 16**

| (AAH hereinbelow indicates "Amount of Applied Herbicide") | | | | | |
|---|---|---|---|---|---|
| No. | **A A H** (g/ha) | A | B | C | a |
| 1-005 | 320 | 2 | 0 | 0 | 0 |
| 1-006 | 320 | 3 | 1 | 0 | 0 |
| 1-011 | 320 | 5 | 0 | 0 | 0 |
| 1-012 | 320 | 3 | 1 | 3 | 0 |
| 1-013 | 320 | 4 | 4 | 3 | 0 |
| 1-014 | 320 | 5 | 5 | 5 | 0 |
| 1-015 | 320 | 3 | 3 | 3 | 0 |
| 1-017 | 320 | 3 | 3 | 3 | 1 |
| 1-019 | 320 | 5 | 5 | 4 | 1 |
| 1-020 | 320 | 4 | 5 | 4 | 2 |
| 1-021 | 320 | 4 | 5 | 4 | 0 |
| 1-022 | 320 | 2 | 3 | | 3 |
| 1-024 | 320 | 5 | 5 | 5 | 3 |
| 1-025 | 320 | 3 | 5 | 4 | 1 |
| 1-026 | 320 | 1 | 2 | 1 | 0 |
| 1-027 | 320 | 0 | 2 | 0 | 0 |
| 1-028 | 320 | 1 | 2 | 0 | 2 |
| 1-029 | 320 | 0 | 1 | 2 | 1 |
| 1-030 | 320 | 2 | 5 | 4 | 0 |
| 1-037 | 320 | 4 | 4 | 3 | 1 |
| 1-040 | 320 | 3 | 3 | 1 | 0 |
| 1-041 | 320 | 3 | 4 | 4 | 0 |
| 1-042 | 320 | 3 | 4 | 3 | 0 |
| 1-044 | 320 | 5 | 5 | 5 | 1 |
| 1-045 | 320 | 1 | 4 | 4 | 0 |
| 1-047 | 320 | 3 | 0 | 0 | 0 |
| 1-049 | 320 | 4 | 1 | 0 | 1 |
| 1-051 | 320 | 4 | 0 | 0 | 0 |
| 1-052 | 320 | 3 | 1 | 3 | 0 |
| 1-053 | 320 | 3 | 2 | 1 | 0 |
| 1-055 | 272 | 4 | 0 | 0 | 0 |
| 1-059 | 320 | 3 | 2 | 1 | 0 |
| 1-061 | 320 | 3 | 3 | 3 | 0 |
| 1-062 | 320 | 2 | 2 | 0 | 0 |
| 1-065 | 320 | 3 | 0 | 0 | 2 |
| 1-069 | 320 | 3 | 3 | 3 | 0 |
| 1-070 | 320 | 3 | 3 | 1 | 1 |
| 1-072 | 320 | 3 | 3 | 0 | 0 |
| 1-073 | 320 | 5 | 4 | 0 | 0 |
| 1-087 | 320 | 3 | 1 | 0 | 0 |
| 1-089 | 320 | 5 | 4 | 4 | 0 |
| 1-090 | 320 | 5 | 4 | 5 | 1 |
| 1-091 | 320 | 5 | 4 | 4 | 2 |
| 1-092 | 320 | 5 | 3 | 2 | 0 |
| 1-093 | 320 | 3 | 1 | 0 | 0 |
| 1-101 | 320 | 4 | 5 | 5 | 0 |
| 1-102 | 320 | 4 | 5 | 4 | 0 |
| 1-105 | 320 | 3 | 3 | 2 | 0 |
| 1-108 | 320 | 3 | 2 | 0 | 0 |
| 1-110 | 320 | 3 | 0 | 4 | 0 |
| 1-113 | 320 | 3 | 0 | 0 | 0 |
| 1-115 | 320 | 2 | 0 | 2 | 0 |
| 1-120 | 320 | 3 | 0 | 0 | 0 |
| 1-125 | 320 | 3 | 0 | 0 | 0 |
| 1-126 | 320 | 3 | 3 | 3 | 0 |
| 1-128 | 246 | 0 | 5 | 5 | 0 |
| 1-130 | 320 | 3 | 3 | 3 | 0 |
| 1-131 | 320 | 2 | 1 | 0 | 0 |
| 1-133 | 320 | 5 | 5 | 3 | 1 |
| 1-135 | 320 | 4 | 3 | 3 | 0 |
| 1-136 | 320 | 3 | 2 | 0 | 0 |
| 1-138 | 320 | 3 | 3 | 3 | 0 |
| 1-140 | 320 | 3 | 2 | 0 | 0 |
| 1-141 | 320 | 3 | 0 | 0 | 0 |
| 1-142 | 320 | 3 | 5 | 5 | 0 |
| 1-143 | 320 | 3 | 0 | 0 | 0 |
| 1-146 | 320 | 3 | 0 | 0 | 0 |
| 1-148 | 368 | 3 | 3 | 3 | 0 |
| 1-151 | 320 | 3 | 3 | 3 | 0 |
| 1-152 | 320 | 4 | 3 | 5 | 0 |
| 1-154 | 320 | 2 | 0 | 0 | 0 |
| 1-158 | 320 | 0 | 2 | 0 | 0 |
| 1-159 | 320 | 2 | 0 | 0 | 0 |
| 1-198 | 320 | 3 | 0 | 0 | 0 |
| 1-201 | 320 | 3 | 4 | 1 | 0 |
| 1-203 | 320 | 5 | 5 | 4 | 3 |
| 1-204 | 320 | 3 | 3 | 3 | 0 |
| 1-206 | 320 | 5 | 5 | 5 | 5 |
| 1-207 | 320 | 5 | 5 | 5 | 3 |
| 1-208 | 320 | 5 | 5 | 5 | 3 |
| 1-210 | 320 | 5 | 5 | 5 | 4 |
| 1-211 | 80 | 5 | 3 | 3 | 0 |
| 1-213 | 320 | 5 | 5 | 5 | 1 |
| 1-215 | 320 | 5 | 4 | 4 | 0 |
| 1-217 | 320 | 4 | 5 | 3 | 2 |
| 1-218 | 100 | 4 | 3 | 0 | 2 |
| 1-219 | 80 | 5 | 3 | 3 | 0 |
| 1-221 | 320 | 5 | 3 | 3 | 3 |
| 1-222 | 320 | 5 | 4 | 4 | 4 |
| 1-223 | 246 | 5 | 4 | 4 | 3 |
| 1-224 | 320 | 5 | 5 | 3 | 3 |
| 1-226 | 320 | 5 | 4 | 4 | 3 |
| 1-227 | 320 | 5 | 5 | 5 | 0 |
| 1-228 | 320 | 5 | 5 | 4 | 0 |
| 1-230 | 320 | 5 | 5 | 4 | 3 |
| 1-236 | 320 | 3 | 0 | 0 | 0 |
| 1-241 | 320 | 0 | 3 | 2 | 0 |
| 1-245 | 320 | 3 | 3 | 0 | 0 |
| 1-247 | 320 | 2 | 2 | 0 | 0 |
| 1-253 | 320 | 3 | 3 | 0 | 0 |
| 2-001 | 320 | 2 | 3 | 1 | 0 |
| 2-004 | 320 | 0 | 2 | 2 | 0 |
| 2-005 | 320 | 0 | 2 | 2 | 0 |
| 2-007 | 320 | 2 | 0 | 0 | 0 |
| 2-010 | 320 | 3 | 3 | 1 | 0 |
| 2-011 | 320 | 3 | 3 | 3 | 0 |
| 2-012 | 320 | 3 | 0 | 0 | 0 |
| 2-013 | 320 | 3 | 2 | 2 | 0 |
| 2-014 | 320 | 3 | 0 | 0 | 2 |
| 2-016 | 320 | 5 | 3 | 5 | 0 |
| 2-017 | 320 | 2 | 0 | 0 | 0 |
| 2-018 | 320 | 0 | 3 | 0 | 0 |
| 2-020 | 320 | 0 | 2 | 0 | 0 |
| 2-022 | 320 | 2 | 5 | 5 | 0 |
| 2-023 | 320 | 2 | 0 | 0 | 0 |
| 2-026 | 320 | 3 | 4 | 4 | 0 |
| 2-027 | 320 | 4 | 5 | 4 | 0 |
| 2-029 | 266 | 2 | 0 | 0 | 0 |
| 2-030 | 320 | 3 | 0 | 0 | 0 |
| 2-031 | 320 | 3 | 4 | 4 | 0 |
| 2-032 | 320 | 4 | 4 | 4 | 0 |
| 2-033 | 320 | 4 | 5 | 4 | 0 |
| 2-034 | 320 | 3 | 4 | 2 | 2 |
| 2-035 | 320 | 5 | 4 | 4 | 0 |
| 2-036 | 320 | 3 | 4 | 4 | 0 |
| 2-037 | 320 | 3 | 2 | 3 | 0 |
| 2-038 | 320 | 3 | 0 | 0 | 0 |
| 2-039 | 320 | 3 | 4 | 5 | 0 |
| 2-041 | 320 | 3 | 0 | 0 | 0 |
| 2-043 | 320 | 0 | 2 | 1 | 0 |
| 2-044 | 320 | 3 | 0 | 0 | 0 |
| 2-046 | 320 | 4 | 4 | 3 | 0 |
| 2-047 | 320 | 3 | 3 | 2 | 0 |
| 2-049 | 320 | 2 | 3 | 3 | 0 |
| 2-051 | 320 | 3 | 4 | 4 | 0 |
| 2-053 | 320 | 2 | 3 | 4 | 3 |
| 2-055 | 320 | 0 | 3 | 1 | 0 |
| 2-056 | 320 | 3 | 5 | 5 | 0 |
| 2-058 | 320 | 0 | 2 | 0 | 0 |
| 2-059 | 269 | 3 | 3 | 4 | 0 |
| 2-060 | 320 | 0 | 5 | 4 | 0 |
| 2-062 | 320 | 4 | 4 | 3 | 0 |
| 2-064 | 320 | 4 | 4 | 3 | 0 |
| 2-071 | 320 | 3 | 0 | 0 | 0 |
| 2-073 | 320 | 3 | 0 | 0 | 0 |
| 2-075 | 320 | 2 | 2 | 0 | 0 |
| 2-076 | 320 | 3 | 0 | 0 | 0 |
| 2-077 | 320 | 3 | 4 | 5 | 0 |
| 2-078 | 320 | 5 | 4 | 4 | 3 |
| 2-079 | 320 | 1 | 3 | 1 | 2 |
| 2-080 | 320 | 5 | 5 | 4 | 1 |
| 2-081 | 320 | 4 | 2 | 0 | 0 |
| 2-082 | 320 | 2 | 3 | 0 | 0 |
| 2-083 | 320 | 2 | 1 | 3 | 0 |
| 2-084 | 320 | 1 | 1 | 2 | 0 |
| 2-085 | 320 | 4 | 3 | 1 | 0 |
| 2-087 | 320 | 5 | 4 | 4 | 0 |
| 2-088 | 320 | 3 | 2 | 0 | 0 |
| 2-089 | 320 | 5 | 4 | 4 | 0 |
| 2-091 | 320 | 3 | 4 | 5 | 0 |
| 2-092 | 320 | 2 | 3 | 2 | 1 |
| 2-095 | 320 | 2 | 0 | 0 | 0 |
| 2-096 | 320 | 1 | 3 | 2 | 0 |
| 2-100 | 320 | 3 | 3 | 2 | 0 |
| 2-101 | 320 | 0 | 3 | 3 | 0 |
| 2-107 | 320 | 2 | 0 | 0 | 0 |
| 2-109 | 320 | 0 | 3 | 0 | 0 |
| 2-111 | 320 | 3 | 3 | 0 | 0 |
| 2-112 | 320 | 0 | 2 | 0 | 0 |
| 2-113 | 320 | 3 | 0 | 0 | 0 |
| 2-118 | 320 | 4 | 3 | 0 | 0 |
| 2-125 | 320 | 0 | 2 | 0 | 0 |
| 2-129 | 320 | 5 | 0 | 0 | 0 |
| 2-130 | 320 | 0 | 3 | 0 | 0 |
| 2-138 | 176 | 5 | 0 | 0 | 0 |
| 2-139 | 320 | 4 | 3 | 1 | 0 |
| 2-149 | 320 | 3 | 1 | 0 | 0 |
| 2-164 | 320 | 3 | 0 | 0 | 0 |
| 2-165 | 320 | 3 | 0 | 1 | 0 |
| 2-166 | 80 | 2 | 0 | 0 | 0 |
| 2-173 | 320 | 2 | 0 | 0 | 0 |
| 2-175 | 320 | 3 | 0 | 0 | 0 |
| 2-179 | 320 | 3 | 0 | 0 | 0 |
| 2-183 | 320 | 2 | 0 | 0 | 0 |
| 2-185 | 320 | 0 | 5 | 5 | 0 |
| 2-188 | 320 | 4 | 0 | 0 | 0 |
| 2-190 | 320 | 3 | 0 | 0 | 0 |
| 2-197 | 320 | 3 | 3 | 3 | 0 |
| 2-198 | 320 | 5 | 3 | 3 | 0 |
| 2-199 | 320 | 1 | 3 | 2 | 0 |
| 2-200 | 320 | 3 | 0 | 0 | 0 |
| 2-201 | 320 | 2 | 2 | 0 | 0 |
| 2-202 | 320 | 3 | 0 | 0 | 0 |
| 2-203 | 320 | 2 | 0 | 0 | 0 |
| 2-205 | 320 | 4 | 3 | 1 | 0 |
| 2-206 | 320 | 5 | 5 | 4 | 0 |
| 2-207 | 320 | 2 | 4 | 3 | 0 |
| 2-208 | 320 | 0 | 2 | 0 | 0 |
| 2-210 | 320 | 3 | 0 | 0 | 0 |
| 2-211 | 320 | 2 | 0 | 0 | 0 |
| 2-213 | 320 | 3 | 0 | 0 | 0 |
| 2-215 | 320 | 3 | 0 | 0 | 0 |
| 2-217 | 320 | 5 | 5 | 4 | 4 |
| 2-218 | 320 | 5 | 5 | 5 | 2 |
| 2-219 | 320 | 3 | 0 | 0 | 1 |
| 2-221 | 320 | 5 | 5 | 5 | 2 |
| 2-222 | 320 | 5 | 4 | 4 | 0 |
| 2-223 | 320 | 4 | 4 | 2 | 1 |
| 2-224 | 320 | 5 | 0 | 0 | 0 |
| 2-225 | 320 | 0 | 3 | 3 | 0 |
| 2-226 | 320 | 2 | 0 | 0 | 0 |
| 2-227 | 320 | 3 | 0 | 0 | 2 |
| 2-228 | 320 | 5 | 4 | 4 | 0 |
| 2-230 | 320 | 3 | 0 | 0 | 0 |
| 2-232 | 320 | 3 | 0 | 0 | 0 |
| 2-236 | 320 | 3 | 3 | 2 | 0 |
| 2-237 | 320 | 3 | 0 | 0 | 0 |
| 2-238 | 224 | 3 | 0 | 0 | 0 |
| 2-244 | 320 | 3 | 0 | 0 | 1 |
| 2-245 | 320 | 4 | 3 | 3 | 0 |
| 2-246 | 320 | 2 | 0 | 0 | 0 |
| 2-249 | 320 | 3 | 2 | 0 | 0 |
| 2-252 | 320 | 3 | 2 | 1 | 1 |
| 2-253 | 320 | 5 | 3 | 0 | 0 |
| 2-254 | 320 | 5 | 3 | 1 | 3 |
| 2-255 | 320 | 5 | 3 | 3 | 3 |
| 2-256 | 320 | 2 | 0 | 0 | 0 |
| 2-257 | 320 | 1 | 0 | 2 | 0 |
| 2-258 | 320 | 5 | 3 | 0 | 5 |
| 2-260 | 320 | 3 | 0 | 0 | 0 |
| 2-264 | 320 | 4 | 4 | 3 | 1 |
| 2-267 | 320 | 2 | 0 | 0 | 0 |
| 2-269 | 320 | 3 | 3 | 3 | 0 |
| 2-273 | 320 | 3 | 0 | 0 | 0 |
| 2-275 | 320 | 3 | 3 | 3 | 0 |
| 2-277 | 320 | 1 | 0 | 2 | 0 |
| 2-280 | 320 | 5 | 3 | 0 | 0 |
| 2-281 | 320 | 5 | 5 | 4 | 0 |
| 2-283 | 320 | 3 | 5 | 3 | 0 |
| 2-284 | 320 | 3 | 3 | 3 | 0 |
| 2-285 | 320 | 5 | 3 | 0 | 0 |
| 2-286 | 320 | 5 | 3 | 1 | 0 |
| 2-287 | 320 | 5 | 4 | 4 | 0 |
| 2-288 | 320 | 5 | 5 | 4 | 0 |
| 2-289 | 320 | 4 | 3 | 3 | 0 |
| 2-292 | 320 | 5 | 5 | 4 | 0 |
| 2-293 | 320 | 5 | 5 | 4 | 0 |
| 2-294 | 320 | 5 | 5 | 4 | 0 |
| 2-295 | 320 | 4 | 3 | 2 | 0 |
| 2-298 | 320 | 3 | 3 | 3 | 0 |
| 2-302 | 320 | 3 | 0 | 0 | 0 |
| 2-305 | 320 | 3 | 5 | 2 | 0 |
| 2-307 | 320 | 0 | 3 | 0 | 0 |
| 2-310 | 320 | 0 | 2 | 0 | 0 |
| 2-311 | 320 | 3 | 3 | 3 | 0 |
| 2-312 | 320 | 2 | 3 | 0 | 0 |
| 2-313 | 320 | 2 | 3 | 0 | 0 |
| 2-315 | 320 | 4 | 0 | 0 | 0 |
| 2-317 | 320 | 3 | 0 | 0 | 0 |
| 2-319 | 320 | 4 | 2 | 3 | 0 |
| 2-321 | 320 | 3 | 3 | 0 | 0 |
| 2-323 | 320 | 4 | 2 | 0 | 0 |
| 2-325 | 320 | 3 | 2 | 0 | 0 |
| 2-327 | 320 | 3 | 3 | 3 | 0 |
| 2-329 | 320 | 2 | 0 | 0 | 0 |
| 2-330 | 320 | 3 | 0 | 0 | 0 |
| 2-331 | 320 | 3 | 3 | 0 | 0 |
| 2-332 | 320 | 2 | 0 | 0 | 0 |
| 2-333 | 320 | 2 | 0 | 0 | 0 |
| 2-334 | 320 | 3 | 0 | 0 | 0 |
| 2-335 | 320 | 3 | 0 | 0 | 1 |
| 2-336 | 320 | 0 | 3 | 0 | 0 |
| 2-337 | 320 | 4 | 3 | 3 | 0 |
| 2-339 | 320 | 4 | 5 | 5 | 5 |
| 2-340 | 320 | 5 | 5 | 4 | 1 |
| 2-341 | 320 | 3 | 4 | 5 | 0 |
| 2-342 | 320 | 2 | 3 | 0 | 0 |
| 2-344 | 320 | 0 | 2 | 0 | 0 |
| 2-345 | 320 | 4 | 4 | 3 | 0 |
| 2-347 | 320 | 3 | 0 | 0 | 0 |
| 2-348 | 320 | 2 | 3 | 0 | 0 |
| 2-349 | 320 | 3 | 0 | 0 | 0 |
| 2-350 | 320 | 3 | 0 | 0 | 0 |
| 2-351 | 320 | 5 | 3 | 3 | 0 |
| 2-352 | 320 | 3 | 2 | 2 | 0 |
| 2-353 | 320 | 2 | 0 | 0 | 0 |
| 2-355 | 320 | 4 | 3 | 0 | 0 |
| 2-357 | 320 | 4 | 2 | 0 | 0 |
| 2-358 | 320 | 3 | 0 | 0 | 0 |
| 2-360 | 320 | 3 | 2 | 0 | 0 |
| 2-369 | 320 | 0 | 0 | 2 | 0 |
| 2-370 | 320 | 3 | 0 | 0 | 0 |
| 2-371 | 320 | 3 | 3 | 4 | 0 |
| 2-374 | 320 | 3 | 0 | 0 | 0 |
| 2-376 | 246 | 3 | 0 | 0 | 0 |
| 2-386 | 320 | 2 | 0 | 0 | 0 |
| 2-387 | 320 | 3 | 3 | 0 | 0 |
| 2-388 | 320 | 5 | 0 | 0 | 0 |
| 2-389 | 320 | 3 | 0 | 0 | 0 |
| 2-390 | 320 | 2 | 0 | 0 | 0 |
| 2-391 | 320 | 5 | 4 | 5 | 0 |
| 2-392 | 320 | 3 | 0 | 0 | 0 |
| 2-393 | 320 | 3 | 0 | 0 | 0 |
| 2-395 | 320 | 0 | 2 | 0 | 0 |
| 2-398 | 320 | 3 | 3 | 0 | 0 |
| 2-407 | 320 | 3 | 0 | 0 | 0 |
| 2-409 | 320 | 3 | 3 | 2 | 0 |
| 2-411 | 320 | 3 | 0 | 0 | 0 |
| 2-413 | 320 | 3 | 0 | 0 | 0 |
| 2-414 | 320 | 2 | 3 | 2 | 0 |
| 2-416 | 320 | 3 | 3 | 2 | 0 |
| 2-418 | 320 | 4 | 4 | 2 | 0 |
| 2-420 | 320 | 2 | 3 | 0 | 0 |
| 2-425 | 320 | 3 | 3 | 0 | 0 |
| 2-429 | 320 | 3 | 2 | 3 | 0 |
| 2-430 | 320 | 3 | 0 | 0 | 0 |
| 2-431 | 320 | 2 | 0 | 0 | 0 |
| 2-432 | 320 | 2 | 0 | 0 | 0 |
| 2-440 | 320 | 3 | 4 | 3 | 0 |
| 2-441 | 320 | 3 | 3 | 3 | 0 |
| 2-442 | 320 | 3 | 2 | 2 | 0 |
| 2-443 | 320 | 3 | 0 | 0 | 1 |
| 2-444 | 320 | 3 | 0 | 0 | 3 |
| 2-445 | 320 | 3 | 0 | 0 | 0 |
| 2-450 | 320 | 2 | 3 | 0 | 0 |
| 2-451 | 320 | 2 | 0 | 0 | 0 |
| 2-453 | 320 | 3 | 3 | 3 | 0 |
| 2-454 | 320 | 3 | 0 | 0 | 0 |
| 2-455 | 320 | 3 | 0 | 0 | 0 |
| 2-460 | 403 | 3 | 0 | 0 | 0 |
| 2-461 | 320 | 3 | 0 | 0 | 0 |
| 2-465 | 320 | 4 | 2 | 3 | 3 |
| 3-002 | 320 | 5 | 3 | 4 | 0 |
| 3-003 | 320 | 3 | 4 | 5 | 0 |
| 3-004 | 320 | 5 | 3 | 3 | 3 |
| 3-005 | 320 | 4 | 4 | 4 | 0 |
| 3-006 | 320 | 5 | 5 | 4 | 2 |
| 3-007 | 320 | 5 | 5 | 4 | 0 |
| 3-008 | 320 | 5 | 5 | 5 | 0 |
| 3-009 | 320 | 1 | 3 | 1 | 0 |
| 3-010 | 320 | 5 | 4 | 3 | 1 |
| 3-011 | 320 | 5 | 4 | 3 | 1 |
| 3-012 | 320 | 5 | 5 | 4 | 0 |
| 3-013 | 320 | 3 | 3 | 2 | 0 |
| 3-014 | 320 | 5 | 3 | 5 | 0 |
| 3-018 | 49 | 3 | 2 | 0 | 0 |
| 3-019 | 320 | 3 | 3 | 3 | 2 |
| 3-020 | 320 | 3 | 3 | 2 | 0 |
| 3-021 | 320 | 3 | 3 | 4 | 0 |
| 3-023 | 320 | 3 | 3 | 4 | 0 |
| 3-024 | 320 | 3 | 0 | 0 | 0 |
| 3-025 | 320 | 0 | 0 | 3 | 0 |
| 3-026 | 320 | 2 | 0 | 3 | 0 |
| 3-027 | 320 | 2 | 3 | 5 | 0 |
| 3-028 | 320 | 2 | 0 | 0 | 0 |
| 3-030 | 320 | 2 | 0 | 3 | 0 |
| 3-034 | 320 | 3 | 3 | 3 | 0 |
| 3-035 | 320 | 3 | 3 | 0 | 0 |
| 3-039 | 320 | 2 | 1 | 0 | 0 |
| 3-040 | 320 | 4 | 3 | 2 | 0 |
| 3-041 | 320 | 2 | 0 | 0 | 0 |
| 3-043 | 320 | 0 | 1 | 2 | 0 |
| 3-044 | 320 | 3 | 0 | 0 | 0 |
| 3-045 | 320 | 2 | 1 | 0 | 0 |
| 3-046 | 320 | 4 | 0 | 0 | 0 |
| 3-047 | 320 | 3 | 2 | 0 | 0 |
| 3-049 | 274 | 5 | 3 | 3 | 1 |
| 3-050 | 224 | 2 | 0 | 1 | 0 |
| 3-053 | 320 | 5 | 5 | 4 | 3 |
| 3-054 | 304 | 5 | 5 | 4 | 1 |
| 3-055 | 320 | 4 | 4 | 3 | 0 |
| 3-056 | 320 | 3 | 0 | 0 | 0 |
| 3-057 | 320 | 5 | 5 | 5 | 4 |
| 3-058 | 320 | 5 | 5 | 3 | 2 |
| 3-059 | 320 | 2 | 1 | 2 | 0 |
| 3-060 | 320 | 1 | 2 | 0 | 0 |
| 3-062 | 320 | 3 | 4 | 4 | 0 |
| 3-064 | 320 | 0 | 3 | 4 | 0 |
| 3-065 | 214 | 1 | 3 | 3 | 0 |
| 3-066 | 296 | 5 | 5 | 5 | 1 |
| 3-069 | 320 | 0 | 3 | 2 | 0 |
| 3-070 | 80 | 5 | 5 | 4 | 3 |
| 3-071 | 320 | 5 | 5 | 5 | 3 |
| 3-072 | 320 | 1 | 5 | 4 | 0 |
| 3-074 | 320 | 0 | 2 | 0 | 1 |
| 3-075 | 320 | 1 | 1 | 2 | 0 |
| 3-076 | 320 | 4 | 5 | 5 | 3 |
| 3-077 | 320 | 5 | 5 | 5 | 0 |
| 3-078 | 80 | 5 | 5 | 4 | 2 |
| 3-079 | 320 | 5 | 5 | 5 | 0 |
| 3-082 | 320 | 3 | 5 | 4 | 1 |
| 3-083 | 320 | 4 | 4 | 4 | 0 |
| 3-084 | 320 | 0 | 3 | 1 | 0 |
| 3-085 | 320 | 5 | 5 | 4 | 0 |
| 3-086 | 320 | 0 | 2 | 0 | 0 |
| 3-088 | 320 | 5 | 5 | 5 | 0 |
| 3-089 | 320 | 5 | 5 | 5 | 3 |
| 3-090 | 310 | 5 | 5 | 4 | 0 |
| 3-091 | 320 | 0 | 4 | 4 | 0 |
| 3-092 | 320 | 0 | 3 | 3 | 0 |
| 3-093 | 320 | 5 | 5 | 5 | 3 |
| 3-094 | 323 | 5 | 5 | 5 | 3 |
| 3-095 | 320 | 5 | 5 | 5 | 3 |
| 3-096 | 320 | 5 | 5 | 5 | 0 |
| 3-099 | 320 | 5 | 5 | 5 | 3 |
| 3-101 | 320 | 5 | 5 | 5 | 1 |
| 3-103 | 320 | 5 | 5 | 5 | 3 |
| 3-105 | 320 | 5 | 5 | 5 | 3 |
| 3-107 | 320 | 4 | 5 | 5 | 4 |
| 3-108 | 320 | 3 | 3 | 5 | 0 |
| 3-109 | 320 | 5 | 5 | 5 | 0 |
| 3-111 | 320 | 5 | 5 | 5 | 1 |
| 3-113 | 320 | 4 | 3 | 2 | 0 |
| 3-114 | 320 | 5 | 5 | 5 | 4 |
| 3-116 | 320 | 5 | 5 | 5 | 4 |
| 3-118 | 320 | 3 | 3 | 5 | 0 |
| 3-119 | 320 | 3 | 3 | 3 | 0 |
| 3-121 | 320 | 5 | 5 | 5 | 5 |
| 3-123 | 320 | 5 | 5 | 5 | 3 |
| 3-125 | 320 | 3 | 3 | 1 | 3 |
| 3-129 | 320 | 5 | 5 | 5 | 3 |
| 3-131 | 320 | 5 | 5 | 5 | 5 |
| 3-133 | 320 | 3 | 3 | 4 | 3 |
| 3-134 | 320 | 3 | 4 | | 0 |
| 3-135 | 320 | 2 | 2 | 0 | 2 |
| 3-137 | 320 | 3 | 0 | 0 | 1 |
| 3-140 | 320 | 5 | 3 | 3 | 0 |
| 3-141 | 320 | 3 | 0 | 0 | 0 |
| 3-144 | 320 | 5 | 3 | 0 | 0 |
| 3-145 | 320 | 3 | 2 | 0 | 0 |
| 3-153 | 320 | 3 | 3 | 3 | 0 |
| 3-155 | 320 | 5 | 5 | 5 | 3 |
| 3-156 | 320 | 5 | 4 | 4 | 2 |
| 3-157 | 320 | 5 | 3 | 3 | 1 |
| 3-158 | 320 | 5 | 5 | 5 | 3 |
| 3-159 | 320 | 0 | 3 | 3 | 0 |
| 3-163 | 320 | 3 | 3 | 3 | 3 |
| 4-003 | 320 | 3 | 0 | 0 | 0 |
| 4-006 | 320 | 3 | 3 | 0 | 0 |
| 4-007 | 320 | 3 | 0 | 0 | 0 |
| 4-008 | 320 | 3 | 3 | 0 | 0 |
| 4-009 | 320 | 2 | 1 | 0 | 0 |
| 4-010 | 320 | 3 | 0 | 3 | 0 |
| 4-013 | 320 | 3 | 0 | 3 | 0 |
| 4-020 | 320 | 4 | 3 | 0 | 0 |
| 4-023 | 320 | 3 | 0 | 0 | 0 |
| 4-024 | 320 | 3 | 2 | 3 | 0 |
| 4-026 | 320 | 5 | 3 | 4 | 0 |
| 4-028 | 320 | 3 | 3 | 0 | 0 |
| 4-031 | 320 | 2 | 0 | 0 | 0 |
| 4-032 | 320 | 0 | 2 | 0 | 0 |
| 4-035 | 320 | 2 | 0 | 0 | 0 |
| 4-036 | 320 | 4 | 3 | 4 | 0 |
| 4-041 | 320 | 3 | 0 | 0 | 0 |
| 4-042 | 320 | 3 | 3 | 0 | 0 |
| 4-045 | 320 | 4 | 2 | 0 | 0 |
| 4-049 | 320 | 3 | 0 | 0 | 0 |
| 4-050 | 320 | 3 | 0 | 0 | 0 |
| 4-051 | 320 | 4 | 3 | 0 | 0 |
| 4-053 | 320 | 5 | 3 | 2 | 0 |
| 4-054 | 320 | 3 | 3 | 0 | 0 |
| 4-061 | 320 | 3 | 0 | 0 | 0 |
| 4-062 | 320 | 0 | 2 | 0 | 0 |
| 4-065 | 320 | 4 | 0 | 1 | 0 |
| 4-070 | 320 | 3 | 2 | 1 | 0 |
| 4-076 | 320 | 0 | 3 | 0 | 0 |
| 4-077 | 320 | 3 | 0 | 0 | 0 |
| 4-079 | 320 | 2 | 0 | 0 | 0 |
| 4-083 | 320 | 3 | 0 | 0 | 0 |
| 4-098 | 320 | 3 | 0 | 0 | 0 |
| 4-100 | 320 | 3 | 3 | 1 | 0 |
| 4-102 | 320 | 4 | 0 | 0 | 0 |
| 4-106 | 320 | 2 | 0 | 0 | 0 |
| 4-110 | 320 | 5 | 5 | 4 | 0 |
| 4-112 | 320 | 3 | 3 | 0 | 0 |
| 4-114 | 320 | 3 | 0 | 0 | 0 |
| 4-118 | 320 | 3 | 2 | 2 | 0 |
| 4-124 | 320 | 4 | 3 | 2 | 0 |
| 4-126 | 320 | 3 | 0 | 0 | 0 |
| 5-001 | 320 | 3 | 1 | 0 | 0 |
| 5-004 | 320 | 2 | 3 | 0 | 0 |
| 5-008 | 320 | 3 | 0 | 0 | 0 |
| 5-009 | 320 | 2 | 0 | 0 | 0 |
| 5-010 | 320 | 0 | 2 | 0 | 0 |
| 5-011 | 320 | 2 | 2 | 0 | 0 |
| 5-012 | 320 | 4 | 3 | 2 | 0 |
| 5-015 | 320 | 3 | 0 | 0 | 0 |
| 5-016 | 320 | 3 | 1 | 0 | 0 |
| 5-017 | 320 | 3 | 2 | 2 | 0 |
| 5-018 | 320 | 0 | 5 | 5 | 0 |
| 5-019 | 320 | 3 | 0 | 0 | 0 |
| 5-020 | 320 | 3 | 0 | 0 | 0 |
| 5-024 | 320 | 3 | 0 | 5 | 0 |
| 5-026 | 320 | 3 | 0 | 0 | 0 |
| 5-028 | 320 | 0 | 2 | 2 | 0 |
| 5-030 | 320 | 3 | 1 | 0 | 0 |
| 5-031 | 320 | 2 | 0 | 0 | 0 |
| 5-032 | 320 | 4 | 1 | 0 | 0 |
| 5-034 | 320 | 3 | 0 | 0 | 0 |
| 5-036 | 320 | 2 | 2 | 2 | 0 |
| 5-038 | 320 | 4 | 3 | 4 | 0 |
| 5-041 | 320 | 0 | 5 | 5 | 0 |
| 5-043 | 320 | 5 | 2 | 0 | 0 |
| 5-045 | 320 | 0 | 3 | 0 | 0 |
| 5-046 | 320 | 0 | 3 | 0 | 0 |
| 5-050 | 320 | 3 | 0 | 0 | 0 |
| 5-052 | 320 | 0 | 3 | 0 | 0 |
| 5-053 | 320 | 0 | 3 | 0 | 0 |
| 5-055 | 320 | 2 | 0 | 0 | 0 |
| 5-061 | 320 | 5 | 3 | 3 | 1 |
| 5-064 | 320 | 2 | 0 | 0 | 0 |
| 5-068 | 320 | 0 | 5 | | 0 |
| 5-069 | 320 | 3 | 1 | 0 | 0 |
| 5-070 | 320 | 0 | 2 | 0 | 0 |
| 5-072 | 320 | 0 | 2 | 0 | 0 |
| 5-073 | 320 | 5 | 4 | 4 | 0 |
| 5-076 | 344 | 2 | 0 | 1 | 0 |
| 5-077 | 320 | 2 | 0 | 4 | 0 |
| 5-080 | 370 | 0 | 3 | 0 | 0 |
| 5-083 | 320 | 0 | 3 | 0 | 0 |
| 5-084 | 320 | 3 | 2 | 0 | 0 |
| 5-089 | 320 | 3 | 0 | 0 | 0 |
| 5-090 | 320 | 3 | 3 | 3 | 0 |
| 5-091 | 320 | 3 | 3 | 3 | 0 |
| 5-093 | 320 | 2 | 0 | 0 | 0 |
| 5-094 | 320 | 4 | 4 | 4 | 0 |
| 5-095 | 320 | 5 | 4 | 4 | 0 |
| 5-097 | 320 | 3 | 0 | 0 | 0 |
| 5-098 | 320 | 3 | 0 | 0 | 0 |
| 5-101 | 320 | 3 | 3 | 3 | 0 |
| 5-102 | 320 | 4 | 3 | 2 | 0 |
| 5-103 | 320 | 5 | 4 | 4 | 0 |
| 5-104 | 320 | 4 | 0 | 0 | 0 |
| 5-105 | 320 | 2 | 0 | 0 | 0 |
| 5-107 | 320 | 3 | 0 | 0 | 0 |
| 7-001 | 320 | 3 | 3 | 2 | 0 |
| 7-002 | 320 | 3 | 3 | 3 | 0 |
| 7-003 | 320 | 2 | 3 | 0 | 0 |
| 7-004 | 320 | 3 | 3 | 0 | 0 |
| 8-002 | 320 | 4 | 5 | 4 | 0 |
| 8-003 | 320 | 5 | 5 | 5 | 2 |
| 8-004 | 320 | 5 | 5 | 4 | 2 |
| 8-005 | 320 | 5 | 5 | 4 | 1 |
| 8-007 | 320 | 5 | 5 | 4 | 3 |
| 8-008 | 320 | 5 | 5 | 4 | 3 |
| 9-002 | 320 | 0 | 3 | 2 | 0 |
| 9-005 | 390 | 5 | 4 | 3 | 0 |
| 9-007 | 320 | 5 | 5 | 5 | 0 |
| 9-008 | 320 | 5 | 5 | 5 | 0 |
| 9-010 | 246 | 5 | 3 | 3 | 1 |
| 9-011 | 320 | 3 | 2 | 1 | 0 |
| 9-012 | 320 | 4 | 5 | 5 | 0 |
| 9-013 | 320 | 3 | 4 | 4 | 0 |
| 9-016 | 320 | 3 | 0 | 0 | 0 |
| 9-019 | 320 | 3 | 0 | 0 | 0 |
| 9-020 | 320 | 5 | 5 | 5 | 0 |
| 9-021 | 320 | 5 | 5 | 5 | 0 |
| 9-023 | 320 | 5 | 5 | 5 | 3 |
| 9-025 | 320 | 5 | 5 | 5 | 0 |
| 9-027 | 320 | 5 | 5 | 5 | 3 |
| 9-031 | 320 | 2 | 0 | 0 | 0 |
| 9-033 | 320 | 2 | 0 | 0 | 0 |
| 9-035 | 320 | 2 | 0 | 0 | 0 |
| 9-037 | 320 | 3 | 0 | 0 | 0 |
| 9-043 | 320 | 3 | 0 | 2 | 0 |
| 9-045 | 320 | 5 | 5 | 5 | 0 |
| 9-049 | 320 | 5 | 5 | 5 | 5 |
| 9-050 | 320 | 5 | 5 | 5 | 1 |
| 9-051 | 320 | 5 | 5 | 5 | 5 |
| 9-052 | 320 | 5 | 5 | 4 | 0 |
| 9-055 | 320 | 5 | 4 | 4 | 0 |
| 9-056 | 438 | 5 | 4 | 4 | 1 |
| 9-057 | 320 | 5 | 4 | 4 | 5 |
| 9-058 | 320 | 5 | 5 | 5 | 5 |
| 9-059 | 320 | 5 | 3 | 3 | 0 |
| 9-060 | 402 | 5 | 4 | 5 | 5 |
| 9-062 | 320 | 5 | 5 | 5 | 3 |
| 9-063 | 320 | 5 | 5 | 5 | 5 |
| 9-064 | 320 | 5 | 5 | 5 | 4 |
| 10-001 | 320 | 0 | 0 | 3 | 0 |
| 11-001 | 320 | 3 | 5 | 5 | 0 |

**Table 17**

| (AAH hereinbelow indicates "Amount of Applied Herbicide") | | | | |
|---|---|---|---|---|
| No. | **A A H** (g/ha) A | B | C | |
| 1-013 | 320 | 3 | 3 | |
| 1-014 | 320 3 | 3 | 3 | |
| 1-015 | 320 3 | 1 | 2 | |
| 1-019 | 320 3 | 4 | | |
| 1-020 | 320 2 | 3 | 3 | |
| 1-021 | 320 2 | 3 | 0 | |
| 1-024 | 320 4 | 4 | 5 | |
| 1-025 | 320 0 | 3 | 3 | |
| 1-026 | 320 2 | 3 | | |
| 1-030 | 320 1 | 3 | 3 | |
| 1-032 | 320 0 | 1 | 3 | |
| 1-037 | 320 0 | 1 | 3 | |
| 1-041 | 320 0 | 3 | 3 | |
| 1-044 | 320 4 | 3 | 1 | |
| 1-045 | 320 1 | 3 | 4 | |
| 1-051 | 320 2 | 0 | 0 | |
| 1-061 | 320 0 | 3 | 4 | |
| 1-068 | 320 0 | 1 | 3 | |
| 1-072 | 320 2 | 3 | 5 | |
| 1-073 | 320 4 | 5 | 5 | |
| 1-085 | 320 1 | 1 | 2 | |
| 1-087 | 320 | 2 | 1 | 3 |
| 1-088 | 320 | 1 | 3 | 4 |
| 1-089 | 320 | 5 | 1 | 1 |
| 1-090 | 320 | 4 | 3 | 4 |
| 1-091 | 320 | 3 | 4 | 4 |
| 1-092 | 320 | 3 | 1 | 0 |
| 1-101 | 320 | 3 | 5 | 5 |
| 1-102 | 320 | 3 | 5 | 5 |
| 1-108 | 320 | 0 | 2 | 0 |
| 1-110 | 320 | 3 | 2 | 0 |
| 1-118 | 320 | 3 | 0 | 0 |
| 1-126 | 320 | 2 | 3 | 1 |
| 1-130 | 320 | 2 | 2 | 0 |
| 1-131 | 320 | 0 | 1 | 3 |
| 1-133 | 320 | 1 | 3 | 3 |
| 1-136 | 320 | 0 | 2 | 0 |
| 1-138 | 320 | 0 | 3 | 0 |
| 1-140 | 320 | 3 | 3 | 0 |
| 1-141 | 320 | 0 | 3 | 0 |
| 1-142 | 320 | 0 | 2 | 0 |
| 1-144 | 320 | 2 | 0 | 0 |
| 1-148 | 368 | 3 | 3 | 1 |
| 1-151 | 320 | 0 | 2 | 0 |
| 1-152 | 320 | 3 | 3 | 0 |
| 1-154 | 320 | 0 | 2 | 0 |
| 1-201 | 320 | 0 | 3 | 3 |
| 1-202 | 178 | 2 | 3 | 0 |
| 1-203 | 320 | 4 | 3 | 4 |
| 1-204 | 320 | 0 | 3 | 2 |
| 1-206 | 320 | 5 | 3 | 5 |
| 1-207 | 320 | 5 | 4 | 5 |
| 1-208 | 320 | 5 | 3 | 3 |
| 1-210 | 320 | 3 | 3 | 4 |
| 1-213 | 320 | 3 | 3 | 4 |
| 1-215 | 320 | 3 | 3 | 3 |
| 1-217 | 320 | 0 | 3 | 3 |
| 1-218 | 100 | 3 | 3 | 4 |
| 1-221 | 320 | 5 | 3 | 3 |
| 1-222 | 320 | 5 | 3 | 4 |
| 1-223 | 246 | 3 | 3 | 3 |
| 1-224 | 320 | 3 | 3 | |
| 1-226 | 320 | 5 | 3 | 3 |
| 1-227 | 320 | 4 | 3 | 3 |
| 1-228 | 320 | 5 | 3 | 3 |
| 1-230 | 320 | 4 | 3 | 4 |
| 1-232 | 320 | 3 | 0 | 0 |
| 1-241 | 320 | 3 | 3 | |
| 1-247 | 320 | 3 | 0 | 0 |
| 1-253 | 320 | 3 | 0 | 0 |
| 2-001 | 320 | 1 | 2 | 3 |
| 2-002 | 320 | 2 | 0 | 3 |
| 2-011 | 320 | 2 | 3 | 3 |
| 2-012 | 320 | 3 | 3 | 0 |
| 2-013 | 320 | 4 | 2 | 3 |
| 2-015 | 320 | 4 | 0 | 0 |
| 2-016 | 320 | 3 | 4 | 4 |
| 2-018 | 320 | 3 | 2 | 3 |
| 2-021 | 320 | 0 | 2 | 0 |
| 2-022 | 320 | 2 | 2 | 4 |
| 2-023 | 320 | 3 | 0 | 0 |
| 2-026 | 320 | 1 | 4 | 5 |
| 2-027 | 320 | 3 | 4 | 5 |
| 2-028 | 320 | 3 | 1 | 3 |
| 2-029 | 266 | 3 | 2 | 3 |
| 2-030 | 320 | 0 | 2 | 0 |
| 2-031 | 320 | 4 | 3 | 3 |
| 2-032 | 320 | 1 | 4 | 5 |
| 2-033 | 320 | 1 | 4 | 4 |
| 2-034 | 320 | 1 | 3 | 3 |
| 2-035 | 320 | 1 | 4 | 4 |
| 2-036 | 320 | 0 | 3 | 3 |
| 2-037 | 320 | 0 | 1 | 3 |
| 2-038 | 320 | 2 | 0 | 0 |
| 2-039 | 320 | 0 | 0 | 2 |
| 2-043 | 320 | 0 | 2 | 0 |
| 2-044 | 320 | 2 | 3 | 3 |
| 2-046 | 320 | 1 | 3 | 0 |
| 2-047 | 320 | 0 | 3 | 2 |
| 2-049 | 320 | 0 | 2 | 2 |
| 2-051 | 320 | 0 | 3 | 1 |
| 2-053 | 320 | 0 | 3 | 3 |
| 2-055 | 320 | 0 | 3 | 3 |
| 2-056 | 320 | 3 | 3 | 3 |
| 2-060 | 320 | 0 | 3 | 1 |
| 2-062 | 320 | 0 | 3 | 2 |
| 2-064 | 320 | 3 | 3 | 0 |
| 2-065 | 320 | 3 | 0 | 0 |
| 2-067 | 320 | 2 | 0 | 0 |
| 2-068 | 320 | 2 | 3 | 3 |
| 2-074 | 320 | 0 | 3 | 0 |
| 2-076 | 320 | 2 | 3 | 0 |
| 2-077 | 320 | 4 | 3 | 3 |
| 2-078 | 320 | 3 | 4 | 4 |
| 2-079 | 320 | 0 | 0 | 2 |
| 2-080 | 320 | 2 | 4 | 4 |
| 2-084 | 320 | 2 | 1 | 3 |
| 2-085 | 320 | 2 | 3 | 3 |
| 2-087 | 320 | 0 | 2 | 0 |
| 2-088 | 320 | 3 | 0 | 2 |
| 2-089 | 320 | 4 | 3 | 4 |
| 2-091 | 320 | 0 | 3 | 4 |
| 2-092 | 320 | 0 | 3 | 4 |
| 2-093 | 320 | 3 | 5 | 5 |
| 2-100 | 320 | 3 | 0 | 0 |
| 2-101 | 320 | 0 | 3 | 5 |
| 2-104 | 320 | 0 | 0 | 2 |
| 2-108 | 320 | 3 | 0 | 0 |
| 2-111 | 320 | 2 | 1 | 0 |
| 2-112 | 320 | 3 | 0 | 0 |
| 2-113 | 320 | 2 | 0 | 0 |
| 2-117 | 320 | 0 | 2 | 3 |
| 2-122 | 320 | 0 | 0 | 2 |
| 2-126 | 320 | 0 | 0 | 2 |
| 2-128 | 320 | 0 | 0 | 3 |
| 2-139 | 320 | 3 | 3 | 0 |
| 2-166 | 80 | 0 | 3 | 0 |
| 2-185 | 320 | 3 | 0 | 0 |
| 2-197 | 320 | 3 | 3 | 4 |
| 2-198 | 320 | 2 | 3 | 4 |
| 2-199 | 320 | 0 | 2 | 0 |
| 2-202 | 320 | 4 | 3 | 4 |
| 2-205 | 320 | 2 | 3 | 2 |
| 2-206 | 320 | 4 | 3 | 4 |
| 2-207 | 320 | 3 | 3 | 1 |
| 2-215 | 320 | 2 | 0 | 0 |
| 2-217 | 320 | 3 | 3 | 0 |
| 2-218 | 320 | 1 | 3 | 3 |
| 2-221 | 320 | 5 | 3 | 3 |
| 2-222 | 320 | 2 | 3 | 3 |
| 2-223 | 320 | 3 | 2 | 0 |
| 2-225 | 320 | 3 | 3 | 0 |
| 2-227 | 320 | 0 | 2 | 0 |
| 2-228 | 320 | 3 | 3 | 3 |
| 2-244 | 320 | 5 | 3 | 4 |
| 2-245 | 320 | 5 | 3 | 4 |
| 2-248 | 320 | 4 | 0 | 0 |
| 2-249 | 320 | 4 | 0 | 2 |
| 2-252 | 320 | 4 | 3 | 2 |
| 2-253 | 320 | 3 | 3 | 3 |
| 2-254 | 320 | 4 | 0 | 0 |
| 2-255 | 320 | 4 | 3 | 2 |
| 2-257 | 320 | 2 | 0 | 0 |
| 2-258 | 320 | 4 | 0 | 3 |
| 2-264 | 320 | 3 | 3 | 3 |
| 2-271 | 320 | 3 | 0 | 0 |
| 2-280 | 320 | 4 | 3 | |
| 2-281 | 320 | 5 | 4 | 5 |
| 2-283 | 320 | 5 | 3 | 4 |
| 2-284 | 320 | 3 | 3 | 3 |
| 2-285 | 320 | 3 | 3 | 0 |
| 2-286 | 320 | 3 | 3 | 3 |
| 2-287 | 320 | 3 | 3 | 4 |
| 2-288 | 320 | 3 | 3 | 3 |
| 2-289 | 320 | 3 | 3 | 4 |
| 2-292 | 320 | 5 | 5 | 5 |
| 2-293 | 320 | 4 | 3 | 4 |
| 2-294 | 320 | 3 | 3 | 3 |
| 2-295 | 320 | 5 | 3 | 3 |
| 2-298 | 320 | 3 | 3 | 0 |
| 2-305 | 320 | 3 | 3 | 0 |
| 2-308 | 320 | 3 | 0 | 0 |
| 2-313 | 320 | 3 | 3 | 3 |
| 2-323 | 320 | 3 | 0 | 0 |
| 2-325 | 320 | 2 | 2 | 0 |
| 2-327 | 320 | 0 | 2 | 0 |
| 2-331 | 320 | 3 | 0 | 0 |
| 2-332 | 320 | 3 | 0 | 0 |
| 2-334 | 320 | 3 | 0 | 0 |
| 2-337 | 320 | 0 | 2 | 0 |
| 2-339 | 320 | 3 | 3 | 3 |
| 2-340 | 320 | 5 | 4 | 3 |
| 2-341 | 320 | 3 | 3 | 3 |
| 2-345 | 320 | 5 | 5 | 4 |
| 2-348 | 320 | 3 | 2 | 0 |
| 2-349 | 320 | 3 | 3 | 3 |
| 2-350 | 320 | 0 | 2 | 0 |
| 2-351 | 320 | 3 | 3 | 4 |
| 2-352 | 320 | 0 | 2 | 0 |
| 2-353 | 320 | 4 | 3 | 3 |
| 2-355 | 320 | 3 | 3 | 3 |
| 2-357 | 320 | 2 | 0 | 0 |
| 2-360 | 320 | 2 | 0 | 0 |
| 2-371 | 320 | 0 | 3 | 4 |
| 2-387 | 320 | 4 | 4 | 4 |
| 2-391 | 320 | 3 | 4 | 5 |
| 2-392 | 320 | 2 | 0 | 0 |
| 2-406 | 320 | 2 | 0 | 0 |
| 2-409 | 320 | 2 | 3 | 3 |
| 2-411 | 320 | 3 | 0 | 0 |
| 2-413 | 320 | 3 | 1 | 0 |
| 2-416 | 320 | 2 | 3 | 2 |
| 2-418 | 320 | 2 | 3 | 0 |
| 2-440 | 320 | 3 | 3 | 3 |
| 2-441 | 320 | 3 | 0 | 0 |
| 2-445 | 320 | 3 | 0 | 0 |
| 2-450 | 320 | 3 | 2 | 0 |
| 2-453 | 320 | 3 | 4 | 4 |
| 2-454 | 320 | 2 | 0 | 0 |
| 2-455 | 320 | 3 | 0 | 0 |
| 2-459 | 320 | 3 | 0 | 0 |
| 3-002 | 320 | 3 | 3 | 4 |
| 3-003 | 320 | 2 | 3 | 4 |
| 3-004 | 320 | 4 | 2 | 4 |
| 3-005 | 320 | 3 | 3 | 4 |
| 3-006 | 320 | 3 | 4 | 5 |
| 3-007 | 320 | 3 | 4 | 4 |
| 3-008 | 320 | 0 | 2 | 4 |
| 3-009 | 320 | 0 | 1 | 3 |
| 3-010 | 320 | 3 | | 4 |
| 3-011 | 320 | 3 | 3 | 4 |
| 3-012 | 320 | 4 | 5 | 4 |
| 3-013 | 320 | 0 | 0 | 3 |
| 3-014 | 320 | 3 | 0 | 5 |
| 3-016 | 320 | 3 | 0 | 0 |
| 3-020 | 320 | 1 | 0 | 2 |
| 3-023 | 320 | 2 | 2 | 4 |
| 3-024 | 320 | 2 | 3 | 3 |
| 3-027 | 320 | 3 | 2 | 3 |
| 3-028 | 320 | 2 | 0 | 0 |
| 3-029 | 285 | 0 | 3 | 1 |
| 3-034 | 320 | 3 | 0 | 0 |
| 3-039 | 320 | 2 | 2 | 0 |
| 3-040 | 320 | 2 | 2 | 3 |
| 3-043 | 320 | 3 | 0 | 0 |
| 3-045 | 320 | 2 | 2 | 0 |
| 3-046 | 320 | 0 | 2 | 1 |
| 3-049 | 274 | 4 | 3 | 2 |
| 3-050 | 224 | 0 | 1 | 3 |
| 3-053 | 320 | 3 | 3 | 3 |
| 3-054 | 304 | 2 | 3 | 3 |
| 3-055 | 320 | 1 | 1 | 3 |
| 3-056 | 320 | 2 | 0 | 0 |
| 3-057 | 320 | 4 | 4 | 3 |
| 3-058 | 320 | 4 | 5 | 4 |
| 3-062 | 320 | 0 | 3 | 3 |
| 3-064 | 320 | 0 | 1 | 3 |
| 3-065 | 214 | 0 | 1 | 3 |
| 3-066 | 296 | 3 | 4 | 5 |
| 3-069 | 320 | 0 | 3 | 4 |
| 3-070 | 80 | 3 | 5 | 4 |
| 3-071 | 320 | 5 | 5 | 5 |
| 3-072 | 320 | 3 | 2 | 3 |
| 3-076 | 320 | 3 | 4 | 4 |
| 3-077 | 320 | 2 | 4 | 4 |
| 3-078 | 80 | 1 | 5 | 4 |
| 3-079 | 320 | 5 | 5 | 5 |
| 3-082 | 320 | 2 | 1 | 3 |
| 3-083 | 320 | 2 | 3 | 4 |
| 3-085 | 320 | 0 | 3 | 3 |
| 3-088 | 320 | 3 | 3 | 3 |
| 3-089 | 320 | 4 | 5 | 5 |
| 3-090 | 310 | 4 | 4 | 5 |
| 3-091 | 320 | 0 | 3 | 3 |
| 3-092 | 320 | 0 | 1 | 3 |
| 3-093 | 320 | 3 | 5 | 5 |
| 3-094 | 323 | 3 | 4 | 4 |
| 3-095 | 320 | 4 | 5 | 5 |
| 3-096 | 320 | 4 | 5 | 4 |
| 3-099 | 320 | 3 | 4 | 3 |
| 3-101 | 320 | 3 | 3 | 2 |
| 3-103 | 320 | 2 | 3 | 3 |
| 3-105 | 320 | 3 | 3 | 4 |
| 3-107 | 320 | 3 | 5 | 5 |
| 3-108 | 320 | 2 | 2 | 0 |
| 3-109 | 320 | 0 | 3 | 2 |
| 3-111 | 320 | 4 | 3 | 3 |
| 3-113 | 320 | 0 | 2 | 0 |
| 3-114 | 320 | 3 | 4 | 5 |
| 3-116 | 320 | 4 | 3 | 4 |
| 3-118 | 320 | 1 | 2 | 0 |
| 3-121 | 320 | 3 | 3 | 3 |
| 3-123 | 320 | 3 | 3 | 2 |
| 3-125 | 320 | 0 | 3 | 3 |
| 3-129 | 320 | 3 | 3 | 3 |
| 3-131 | 320 | 5 | 3 | 5 |
| 3-133 | 320 | 2 | 3 | 1 |
| 3-140 | 320 | 3 | 3 | 3 |
| 3-144 | 320 | 2 | 3 | 2 |
| 3-145 | 320 | 3 | 2 | 0 |
| 3-155 | 320 | 5 | 5 | 5 |
| 3-156 | 320 | 3 | 4 | 5 |
| 3-157 | 320 | 4 | 3 | 4 |
| 3-158 | 320 | 4 | 3 | 5 |
| 3-163 | 320 | 3 | 0 | 0 |
| 3-170 | 320 | 2 | 0 | 0 |
| 4-004 | 320 | 3 | 0 | 2 |
| 4-006 | 320 | 3 | 3 | 2 |
| 4-010 | 320 | 4 | 0 | 3 |
| 4-016 | 320 | 2 | 0 | 0 |
| 4-018 | 320 | 0 | 3 | 3 |
| 4-019 | 320 | 4 | 0 | 0 |
| 4-020 | 320 | 0 | 3 | 0 |
| 4-022 | 320 | 0 | 2 | 0 |
| 4-024 | 320 | 3 | 0 | 0 |
| 4-026 | 320 | 3 | 3 | 2 |
| 4-028 | 320 | 3 | 3 | 0 |
| 4-030 | 320 | 3 | 3 | 3 |
| 4-032 | 320 | 0 | 3 | 0 |
| 4-036 | 320 | 3 | 0 | 0 |
| 4-038 | 320 | 2 | 3 | 0 |
| 4-041 | 320 | 0 | 3 | 0 |
| 4-045 | 320 | 3 | 0 | 0 |
| 4-051 | 320 | 3 | 3 | 0 |
| 4-053 | 320 | 4 | 2 | |
| 4-054 | 320 | 2 | 2 | 0 |
| 4-076 | 320 | 3 | 2 | 0 |
| 4-102 | 320 | 2 | 0 | 0 |
| 4-110 | 320 | 0 | 3 | 5 |
| 4-124 | 320 | 3 | 0 | 0 |
| 5-008 | 320 | 2 | 0 | 0 |
| 5-012 | 320 | 0 | 2 | 2 |
| 5-021 | 320 | 0 | 2 | 0 |
| 5-024 | 320 | 0 | 2 | 0 |
| 5-026 | 320 | 2 | 0 | 0 |
| 5-039 | 320 | 3 | 0 | 0 |
| 5-043 | 320 | 2 | 0 | 0 |
| 5-050 | 320 | 0 | 2 | 0 |
| 5-089 | 320 | 3 | 0 | 0 |
| 5-090 | 320 | 0 | 2 | 0 |
| 5-091 | 320 | 3 | 2 | 3 |
| 5-093 | 320 | 2 | 0 | 0 |
| 5-094 | 320 | 2 | 3 | 3 |
| 5-095 | 320 | 4 | 4 | 4 |
| 5-101 | 320 | 0 | 2 | 2 |
| 5-102 | 320 | 3 | 3 | 2 |
| 5-103 | 320 | 2 | 3 | 3 |
| 5-104 | 320 | 4 | 4 | 4 |
| 5-107 | 320 | 3 | 3 | 3 |
| 5-109 | 320 | 2 | 0 | 0 |
| 7-001 | 320 | 3 | 3 | 3 |
| 7-002 | 320 | 3 | 2 | 2 |
| 7-003 | 320 | 0 | 3 | 0 |
| 7-004 | 320 | 3 | 3 | 2 |
| 8-002 | 320 | 3 | 4 | 5 |
| 8-003 | 320 | 4 | 3 | 3 |
| 8-004 | 320 | 3 | 3 | 3 |
| 8-005 | 320 | 4 | 3 | 3 |
| 8-007 | 320 | 5 | 4 | 3 |
| 8-008 | 320 | 2 | 3 | 2 |
| 9-002 | 320 | 0 | 0 | 3 |
| 9-005 | 390 | 0 | 1 | 3 |
| 9-007 | 320 | 4 | 5 | 5 |
| 9-008 | 320 | 1 | 5 | 5 |
| 9-010 | 246 | 0 | 0 | 2 |
| 9-012 | 320 | 1 | 4 | 5 |
| 9-013 | 320 | 0 | 0 | 5 |
| 9-020 | 320 | 3 | 3 | 3 |
| 9-021 | 320 | 4 | 3 | 4 |
| 9-023 | 320 | 5 | 3 | 5 |
| 9-025 | 320 | 4 | 3 | 4 |
| 9-027 | 320 | 4 | 3 | 4 |
| 9-037 | 320 | 0 | 2 | 2 |
| 9-045 | 320 | 3 | 3 | 5 |
| 9-049 | 320 | 5 | 4 | 4 |
| 9-050 | 320 | 3 | 3 | 5 |
| 9-051 | 320 | 5 | 4 | 5 |
| 9-052 | 320 | 3 | 3 | 4 |
| 9-055 | 320 | 4 | 3 | 3 |
| 9-056 | 438 | 3 | 3 | 4 |
| 9-057 | 320 | 5 | 3 | 4 |
| 9-058 | 320 | 4 | 3 | 4 |
| 9-059 | 320 | 4 | 3 | 4 |
| 9-060 | 402 | 4 | 3 | 4 |
| 9-062 | 320 | 3 | 3 | 3 |
| 9-063 | 320 | 5 | 4 | 4 |
| 9-064 | 320 | 4 | 3 | 4 |
| 11-001 | 320 | 2 | 3 | 3 |

**Table 18**

| (AAH hereinbelow indicates "Amount of Applied Herbicide") | | | | | |
|---|---|---|---|---|---|
| No. | **A A H** (g/ha) | H | D | E | b |
| 1-001 | 320 | 3 | 0 | 5 | 0 |
| 1-003 | 320 | 1 | 3 | 3 | 0 |
| 1-005 | 320 | 4 | 0 | 5 | 3 |
| 1-006 | 320 | 3 | 0 | 5 | 1 |
| 1-007 | 320 | 5 | 5 | 4 | 0 |
| 1-009 | 320 | 3 | 2 | 2 | 0 |
| 1-011 | 320 | 5 | 3 | 5 | 1 |
| 1-012 | 320 | 5 | 0 | 5 | 0 |
| 1-013 | 320 | 5 | 1 | 5 | 0 |
| 1-014 | 320 | 4 | 0 | 5 | 0 |
| 1-015 | 320 | 5 | 3 | 5 | 1 |
| 1-017 | 320 | 5 | 0 | 3 | 0 |
| 1-019 | 320 | 5 | 4 | 5 | 5 |
| 1-020 | 320 | 5 | 1 | 5 | 3 |
| 1-021 | 320 | 5 | 1 | 5 | 2 |
| 1-022 | 320 | 3 | 0 | 4 | 0 |
| 1-023 | 320 | 1 | 0 | 3 | 0 |
| 1-024 | 320 | 5 | 4 | 5 | 5 |
| 1-025 | 320 | 5 | 4 | 5 | 1 |
| 1-026 | 320 | 4 | 1 | 4 | 3 |
| 1-027 | 320 | 3 | 2 | 1 | 1 |
| 1-028 | 320 | 3 | 0 | 4 | 1 |
| 1-030 | 320 | 5 | 3 | 4 | 2 |
| 1-031 | 320 | 4 | 0 | 5 | 0 |
| 1-032 | 320 | 4 | 0 | 5 | 0 |
| 1-033 | 320 | 1 | 0 | 1 | 0 |
| 1-034 | 320 | 1 | 0 | 3 | 0 |
| 1-035 | 320 | 3 | 0 | 4 | 0 |
| 1-036 | 320 | 5 | 3 | 5 | 0 |
| 1-037 | 320 | 5 | 3 | 5 | 3 |
| 1-038 | 123 | 0 | 4 | 3 | 0 |
| 1-039 | 295 | 3 | 1 | 3 | 0 |
| 1-040 | 320 | 4 | 3 | 5 | 0 |
| 1-041 | 320 | 1 | 4 | 5 | 2 |
| 1-042 | 320 | 3 | 0 | 5 | 1 |
| 1-043 | 320 | 5 | 5 | 0 | 3 |
| 1-044 | 320 | 4 | 4 | 5 | 3 |
| 1-045 | 320 | 5 | 4 | 5 | 0 |
| 1-046 | 320 | 3 | 0 | 5 | 0 |
| 1-047 | 320 | 2 | 0 | 5 | 0 |
| 1-048 | 320 | 4 | 5 | 4 | 0 |
| 1-049 | 320 | 5 | 3 | 5 | 1 |
| 1-051 | 320 | 4 | 0 | 5 | 1 |
| 1-052 | 320 | 3 | 2 | 5 | 0 |
| 1-053 | 320 | 3 | 0 | 1 | 0 |
| 1-054 | 320 | 5 | 3 | 4 | 0 |
| 1-055 | 272 | 5 | 2 | 5 | 0 |
| 1-056 | 320 | 4 | 2 | 5 | 0 |
| 1-058 | 320 | 4 | 3 | 5 | 0 |
| 1-059 | 320 | 3 | 1 | 5 | 0 |
| 1-060 | 320 | 4 | 3 | 4 | 0 |
| 1-061 | 320 | 5 | 4 | 5 | 2 |
| 1-062 | 320 | 5 | 3 | 5 | 0 |
| 1-063 | 320 | 2 | 0 | 3 | 0 |
| 1-064 | 320 | 3 | 0 | 3 | 0 |
| 1-065 | 320 | 3 | 3 | 5 | 0 |
| 1-066 | 320 | 2 | 0 | 5 | 0 |
| 1-067 | 99 | 3 | 0 | 0 | 0 |
| 1-068 | 320 | 3 | 1 | 5 | 1 |
| 1-069 | 320 | 3 | 0 | 5 | 0 |
| 1-070 | 320 | 1 | 0 | 3 | 0 |
| 1-071 | 320 | 2 | 0 | 0 | 0 |
| 1-072 | 320 | 5 | 5 | 5 | 4 |
| 1-073 | 320 | 5 | 3 | 5 | 1 |
| 1-074 | 320 | 3 | 0 | 5 | 1 |
| 1-075 | 320 | 3 | 0 | 5 | 0 |
| 1-076 | 320 | 1 | 0 | 5 | 0 |
| 1-077 | 320 | 4 | 1 | 4 | 0 |
| 1-078 | 144 | 1 | 0 | 3 | 0 |
| 1-080 | 320 | 0 | 0 | 4 | 0 |
| 1-081 | 320 | 3 | 0 | 4 | 0 |
| 1-083 | 320 | 3 | 3 | 4 | 0 |
| 1-085 | 320 | 5 | 4 | 5 | 3 |
| 1-087 | 320 | 4 | 3 | 5 | 0 |
| 1-088 | 320 | 5 | 3 | 5 | 0 |
| 1-089 | 320 | 5 | 0 | 5 | 0 |
| 1-090 | 320 | 5 | 1 | 5 | 1 |
| 1-091 | 320 | 5 | 1 | 5 | 1 |
| 1-092 | 320 | 4 | 1 | 5 | 0 |
| 1-093 | 320 | 3 | 0 | 5 | 0 |
| 1-094 | 320 | 3 | 0 | 3 | 1 |
| 1-095 | 197 | 4 | 0 | 1 | 0 |
| 1-096 | 485 | 5 | 2 | 0 | 2 |
| 1-097 | 320 | 0 | 0 | 4 | 0 |
| 1-098 | 320 | 0 | 0 | 4 | 0 |
| 1-099 | 320 | 0 | 0 | 5 | 0 |
| 1-100 | 320 | 0 | 0 | 4 | 0 |
| 1-101 | 320 | 5 | 5 | 5 | 1 |
| 1-102 | 320 | 4 | 3 | 5 | 0 |
| 1-103 | 320 | 3 | 4 | 3 | 0 |
| 1-105 | 320 | 4 | 3 | 4 | 0 |
| 1-108 | 320 | 0 | 3 | 5 | 0 |
| 1-110 | 320 | 4 | 3 | 4 | 0 |
| 1-112 | 320 | 4 | 3 | 3 | 0 |
| 1-113 | 320 | 4 | 0 | 3 | 0 |
| 1-115 | 320 | 4 | 2 | 2 | 0 |
| 1-116 | 320 | 5 | 2 | 4 | 0 |
| 1-118 | 320 | 4 | 2 | 2 | 0 |
| 1-120 | 320 | 3 | 0 | 2 | 1 |
| 1-123 | 296 | 3 | 3 | 3 | 0 |
| 1-124 | 320 | 0 | 2 | 3 | 0 |
| 1-125 | 320 | 5 | 4 | 5 | 4 |
| 1-126 | 320 | 5 | 5 | 4 | 1 |
| 1-127 | 320 | 0 | 3 | 5 | 0 |
| 1-128 | 246 | 4 | 5 | 3 | 0 |
| 1-129 | 320 | 3 | 3 | 5 | 0 |
| 1-130 | 320 | 4 | 3 | 5 | 0 |
| 1-131 | 320 | 5 | 4 | 5 | 0 |
| 1-133 | 320 | 5 | 3 | 5 | 1 |
| 1-135 | 320 | 2 | 3 | 3 | 0 |
| 1-136 | 320 | 5 | 4 | 5 | 1 |
| 1-137 | 320 | 3 | 3 | 5 | 0 |
| 1-138 | 320 | 5 | 3 | 5 | 4 |
| 1-139 | 320 | 5 | 0 | 5 | 0 |
| 1-140 | 320 | 5 | 3 | 5 | 5 |
| 1-141 | 320 | 5 | 3 | 5 | 4 |
| 1-142 | 320 | 3 | 1 | 5 | 0 |
| 1-143 | 320 | 5 | 0 | | 0 |
| 1-144 | 320 | 5 | 3 | 5 | 2 |
| 1-146 | 320 | 5 | 2 | | 0 |
| 1-148 | 368 | 5 | 4 | 5 | 3 |
| 1-149 | 320 | 3 | 3 | 5 | 0 |
| 1-150 | 320 | 2 | 3 | 3 | 0 |
| 1-151 | 320 | 4 | 3 | 5 | 0 |
| 1-152 | 320 | 5 | 5 | 3 | 1 |
| 1-153 | 320 | 4 | 2 | 3 | 0 |
| 1-154 | 320 | 2 | 3 | 2 | 0 |
| 1-155 | 320 | 3 | 0 | 3 | 0 |
| 1-156 | 320 | 3 | 3 | 3 | 0 |
| 1-158 | 320 | 2 | 0 | 0 | 0 |
| 1-159 | 320 | 2 | 0 | 0 | 0 |
| 1-160 | 320 | 0 | 1 | 4 | 0 |
| 1-161 | 320 | 1 | 0 | 3 | 0 |
| 1-162 | 80 | 4 | 3 | 5 | 0 |
| 1-163 | 80 | 1 | 3 | 0 | 0 |
| 1-164 | 80 | 4 | 4 | 4 | 1 |
| 1-165 | 80 | 2 | 0 | 0 | 0 |
| 1-166 | 80 | 4 | 4 | 5 | 1 |
| 1-168 | 320 | 0 | 0 | 3 | 0 |
| 1-169 | 320 | 1 | 0 | 2 | 0 |
| 1-170 | 320 | 2 | 1 | 5 | 0 |
| 1-171 | 320 | 3 | 0 | 4 | 0 |
| 1-172 | 320 | 0 | 0 | 5 | 0 |
| 1-173 | 320 | 1 | 0 | 5 | 0 |
| 1-174 | 320 | 1 | 0 | 3 | 0 |
| 1-175 | 320 | 0 | 0 | 3 | 0 |
| 1-176 | 320 | 4 | 3 | 5 | 1 |
| 1-177 | 320 | 4 | 0 | 4 | 1 |
| 1-178 | 320 | 4 | 0 | 5 | 0 |
| 1-179 | 320 | 4 | 0 | 3 | 0 |
| 1-180 | 320 | 1 | 0 | 4 | 0 |
| 1-182 | 80 | 4 | 3 | 5 | 3 |
| 1-183 | 80 | 3 | 2 | 0 | 0 |
| 1-187 | 320 | 0 | 0 | 3 | 0 |
| 1-191 | 80 | 2 | 5 | 4 | 0 |
| 1-192 | 80 | 1 | 4 | 4 | 0 |
| 1-193 | 80 | 2 | 1 | 0 | 0 |
| 1-194 | 320 | 0 | 0 | 2 | 0 |
| 1-196 | 320 | 3 | 0 | 5 | 1 |
| 1-197 | 320 | 4 | 2 | 4 | 0 |
| 1-198 | 320 | 5 | 3 | 5 | 1 |
| 1-199 | 320 | 0 | 0 | 5 | 0 |
| 1-200 | 320 | 4 | 3 | 3 | 1 |
| 1-201 | 320 | 5 | 4 | 5 | 0 |
| 1-202 | 178 | 5 | 3 | 5 | 2 |
| 1-203 | 320 | 5 | 4 | 5 | 5 |
| 1-204 | 320 | 4 | 2 | 5 | 0 |
| 1-206 | 320 | 5 | 5 | 5 | 5 |
| 1-207 | 320 | 5 | 5 | 4 | 4 |
| 1-208 | 320 | 5 | 5 | 4 | 3 |
| 1-210 | 320 | 5 | 5 | 4 | 5 |
| 1-211 | 80 | 4 | 4 | 4 | 2 |
| 1-213 | 320 | 5 | 4 | 3 | 5 |
| 1-215 | 320 | 5 | 5 | 5 | 3 |
| 1-217 | 320 | 5 | 5 | 3 | 2 |
| 1-218 | 100 | 4 | 4 | 4 | 0 |
| 1-219 | 80 | 5 | 5 | 3 | 4 |
| 1-221 | 320 | 5 | 5 | 5 | 5 |
| 1-222 | 320 | 5 | 5 | 4 | 5 |
| 1-223 | 246 | 5 | 5 | 4 | 3 |
| 1-224 | 320 | 5 | 3 | 5 | 2 |
| 1-226 | 320 | 5 | 5 | 4 | 5 |
| 1-227 | 320 | 5 | 4 | 4 | 5 |
| 1-228 | 320 | 5 | 5 | 4 | 5 |
| 1-230 | 320 | 5 | 5 | 3 | 5 |
| 1-232 | 320 | 3 | 3 | 3 | 1 |
| 1-236 | 320 | 5 | 5 | 5 | 3 |
| 1-237 | 320 | 5 | 4 | 4 | 0 |
| 1-238 | 320 | 4 | 4 | 4 | 0 |
| 1-241 | 320 | 5 | 3 | 4 | 2 |
| 1-243 | 320 | 5 | 4 | 4 | 1 |
| 1-245 | 320 | 5 | 3 | 3 | 2 |
| 1-247 | 320 | 5 | 1 | 4 | 0 |
| 1-249 | 320 | 5 | 3 | 3 | 0 |
| 1-251 | 320 | 5 | 3 | 3 | 2 |
| 1-253 | 320 | 5 | 3 | 3 | 0 |
| 1-255 | 320 | 5 | 3 | 5 | 1 |
| 2-001 | 320 | 5 | 5 | 5 | 1 |
| 2-002 | 320 | 5 | 5 | 5 | 0 |
| 2-003 | 320 | 5 | 3 | 5 | 0 |
| 2-004 | 320 | 5 | 3 | 4 | 0 |
| 2-005 | 320 | 5 | 3 | 5 | 0 |
| 2-006 | 320 | 2 | 0 | 0 | 0 |
| 2-007 | 320 | 1 | 3 | 3 | 0 |
| 2-008 | 320 | 2 | 1 | 4 | 0 |
| 2-009 | 320 | 5 | 3 | 4 | 0 |
| 2-010 | 320 | 5 | 4 | 5 | 0 |
| 2-011 | 320 | 5 | 4 | 5 | 0 |
| 2-012 | 320 | 5 | 5 | 5 | 1 |
| 2-013 | 320 | 5 | 5 | 5 | 3 |
| 2-014 | 320 | 5 | 5 | 5 | 5 |
| 2-015 | 320 | 5 | 5 | 5 | 3 |
| 2-016 | 320 | 5 | 5 | 5 | 5 |
| 2-017 | 320 | 5 | 3 | 2 | 0 |
| 2-018 | 320 | 5 | 4 | 5 | 1 |
| 2-019 | 320 | 5 | 3 | 4 | 4 |
| 2-020 | 320 | 5 | 3 | 5 | 3 |
| 2-021 | 320 | 5 | 3 | 5 | 0 |
| 2-022 | 320 | 5 | 4 | 4 | 3 |
| 2-023 | 320 | 5 | 4 | 5 | 1 |
| 2-024 | 294 | 4 | 3 | 4 | 0 |
| 2-025 | 283 | 4 | 0 | 4 | 0 |
| 2-026 | 320 | 5 | 4 | 5 | 3 |
| 2-027 | 320 | 5 | 4 | 5 | 0 |
| 2-028 | 320 | 4 | 3 | 5 | 1 |
| 2-029 | 266 | 5 | 3 | 3 | 0 |
| 2-030 | 320 | 5 | 3 | 5 | 1 |
| 2-031 | 320 | 5 | 4 | 4 | 0 |
| 2-032 | 320 | 5 | 3 | 5 | 4 |
| 2-033 | 320 | 5 | 1 | 5 | 1 |
| 2-034 | 320 | 5 | 0 | 5 | 4 |
| 2-035 | 320 | 5 | 0 | 5 | 1 |
| 2-036 | 320 | 5 | 3 | 5 | 4 |
| 2-037 | 320 | 4 | 2 | 4 | 0 |
| 2-038 | 320 | 4 | 4 | 4 | 0 |
| 2-039 | 320 | 5 | 5 | 3 | 1 |
| 2-041 | 320 | 3 | 3 | 1 | 0 |
| 2-042 | 320 | 5 | 3 | 5 | 1 |
| 2-043 | 320 | 5 | 3 | 5 | 0 |
| 2-044 | 320 | 3 | 2 | 5 | 0 |
| 2-045 | 320 | 5 | 2 | 5 | 1 |
| 2-046 | 320 | 5 | 3 | 5 | 0 |
| 2-047 | 320 | 5 | 4 | 5 | 4 |
| 2-049 | 320 | 5 | 5 | 5 | 4 |
| 2-051 | 320 | 5 | 5 | 5 | 4 |
| 2-053 | 320 | 5 | 5 | 4 | 4 |
| 2-054 | 320 | 4 | 5 | 3 | 0 |
| 2-055 | 320 | 5 | 5 | 4 | 1 |
| 2-056 | 320 | 5 | 5 | 5 | 4 |
| 2-058 | 320 | 5 | 5 | 5 | 5 |
| 2-059 | 269 | 5 | 5 | 5 | 4 |
| 2-060 | 320 | 3 | 3 | 5 | 3 |
| 2-062 | 320 | 5 | 5 | 5 | 5 |
| 2-064 | 320 | 3 | 3 | 0 | 0 |
| 2-065 | 320 | 3 | 3 | 3 | 0 |
| 2-066 | 320 | 4 | 3 | 1 | 1 |
| 2-067 | 320 | 4 | 5 | 3 | 0 |
| 2-068 | 320 | 5 | 4 | 5 | 3 |
| 2-069 | 320 | 5 | 4 | 5 | 5 |
| 2-070 | 320 | 2 | 0 | 0 | 2 |
| 2-071 | 320 | 2 | 3 | 2 | 0 |
| 2-072 | 320 | 2 | 3 | 5 | 0 |
| 2-073 | 320 | 4 | 3 | 3 | 0 |
| 2-074 | 320 | 5 | 5 | 5 | 5 |
| 2-075 | 320 | 2 | 2 | 4 | 0 |
| 2-076 | 320 | 5 | 5 | 5 | 2 |
| 2-077 | 320 | 5 | 5 | 5 | 2 |
| 2-078 | 320 | 3 | 5 | 5 | 4 |
| 2-079 | 320 | 5 | 4 | 5 | 5 |
| 2-080 | 320 | 5 | 5 | 5 | 5 |
| 2-081 | 320 | 5 | 3 | 5 | 1 |
| 2-082 | 320 | 4 | 2 | 4 | 0 |
| 2-083 | 320 | 5 | 4 | 5 | 1 |
| 2-084 | 320 | 5 | 4 | 5 | 1 |
| 2-085 | 320 | 5 | 0 | 4 | 0 |
| 2-086 | 320 | 3 | 3 | 5 | 0 |
| 2-087 | 320 | 3 | 2 | 5 | 0 |
| 2-088 | 320 | 5 | 3 | 5 | 0 |
| 2-089 | 320 | 4 | 3 | 5 | 0 |
| 2-090 | 320 | 5 | 2 | 5 | 0 |
| 2-091 | 320 | 3 | 3 | 5 | 0 |
| 2-092 | 320 | 5 | 4 | 5 | 3 |
| 2-093 | 320 | 5 | 2 | 5 | 0 |
| 2-094 | 96 | 2 | 0 | | 0 |
| 2-095 | 320 | 5 | 4 | 5 | 1 |
| 2-096 | 320 | 3 | 1 | 3 | 0 |
| 2-097 | 320 | 5 | 3 | 4 | 0 |
| 2-098 | 320 | 5 | 4 | 2 | 0 |
| 2-099 | 320 | 5 | 4 | 5 | 2 |
| 2-100 | 320 | 5 | 4 | 3 | 1 |
| 2-101 | 320 | 4 | 0 | 5 | 0 |
| 2-103 | 320 | 3 | 2 | 5 | 0 |
| 2-104 | 320 | 3 | 0 | 5 | 0 |
| 2-105 | 320 | 4 | 0 | 3 | 0 |
| 2-107 | 320 | 4 | 0 | 2 | 3 |
| 2-108 | 320 | 4 | 0 | 2 | 0 |
| 2-109 | 320 | 4 | 2 | 2 | 1 |
| 2-110 | 320 | 4 | 4 | 4 | 0 |
| 2-111 | 320 | 4 | 2 | 3 | 0 |
| 2-112 | 320 | 5 | 4 | 5 | 0 |
| 2-113 | 320 | 3 | 1 | 0 | 0 |
| 2-114 | 320 | 4 | 3 | 5 | 0 |
| 2-115 | 320 | 5 | 1 | 3 | 1 |
| 2-117 | 320 | 3 | 3 | 5 | 0 |
| 2-118 | 320 | 2 | 0 | 0 | 0 |
| 2-119 | 320 | 3 | 2 | 3 | 0 |
| 2-122 | 320 | 2 | 0 | 0 | 0 |
| 2-123 | 320 | 3 | 0 | 5 | 0 |
| 2-124 | 320 | 3 | 1 | 0 | 0 |
| 2-125 | 320 | 4 | 3 | 5 | 0 |
| 2-127 | 320 | 3 | 0 | 5 | 0 |
| 2-128 | 320 | 3 | 0 | 0 | 0 |
| 2-129 | 320 | 3 | 0 | 0 | 0 |
| 2-130 | 320 | 3 | 0 | 4 | 0 |
| 2-132 | 320 | 0 | 0 | 5 | 0 |
| 2-133 | 320 | 0 | 0 | 5 | 0 |
| 2-134 | 320 | 3 | 0 | 5 | 0 |
| 2-135 | 320 | 1 | 0 | 3 | 0 |
| 2-136 | 320 | 2 | 0 | 5 | 0 |
| 2-137 | 320 | 1 | 1 | 5 | 1 |
| 2-138 | 176 | 5 | 1 | 4 | 0 |
| 2-139 | 320 | 4 | 0 | 4 | 0 |
| 2-142 | 320 | 2 | 0 | 2 | 1 |
| 2-144 | 320 | 2 | 0 | 3 | 0 |
| 2-145 | 320 | 3 | 0 | 4 | 0 |
| 2-146 | 221 | 4 | 0 | 3 | 0 |
| 2-147 | 320 | 1 | 0 | 5 | 0 |
| 2-148 | 320 | 5 | 3 | 5 | 3 |
| 2-149 | 320 | 5 | 0 | 3 | 0 |
| 2-150 | 320 | 4 | 2 | 5 | 0 |
| 2-151 | 320 | 3 | 0 | 3 | 0 |
| 2-152 | 320 | 0 | 0 | 3 | 0 |
| 2-154 | 320 | 3 | 0 | 3 | 0 |
| 2-156 | 320 | 3 | 0 | 4 | 0 |
| 2-157 | 320 | 3 | 0 | 0 | 0 |
| 2-158 | 320 | 0 | 1 | 4 | 0 |
| 2-159 | 320 | 0 | 0 | 3 | 0 |
| 2-160 | 320 | 4 | 3 | 3 | 0 |
| 2-162 | 320 | 3 | 0 | 3 | 0 |
| 2-164 | 320 | 2 | 3 | 4 | 0 |
| 2-165 | 320 | 5 | 3 | 5 | 5 |
| 2-173 | 320 | 3 | 2 | 2 | 0 |
| 2-175 | 320 | 4 | 3 | | 1 |
| 2-177 | 320 | 3 | 4 | 3 | 0 |
| 2-179 | 320 | 2 | 2 | 4 | 0 |
| 2-181 | 320 | 2 | 3 | 0 | 0 |
| 2-183 | 320 | 4 | 3 | 0 | 0 |
| 2-185 | 320 | 3 | 0 | 2 | 0 |
| 2-187 | 320 | 4 | 0 | 0 | 0 |
| 2-188 | 320 | 4 | 3 | 0 | 0 |
| 2-190 | 320 | 2 | 2 | 4 | 0 |
| 2-192 | 320 | 4 | 2 | 2 | 1 |
| 2-195 | 320 | 3 | 0 | 0 | 0 |
| 2-197 | 320 | 5 | 5 | 5 | 0 |
| 2-198 | 320 | 5 | 5 | 5 | 0 |
| 2-199 | 320 | 5 | 5 | 5 | 0 |
| 2-200 | 320 | 4 | 5 | 5 | 0 |
| 2-201 | 320 | 5 | 4 | 5 | 0 |
| 2-202 | 320 | 5 | 5 | 5 | 0 |
| 2-203 | 320 | 4 | 4 | 5 | 0 |
| 2-204 | 320 | 4 | 3 | 3 | 0 |
| 2-205 | 320 | 5 | 4 | 5 | 0 |
| 2-206 | 320 | 5 | 4 | 5 | 0 |
| 2-207 | 320 | 4 | 5 | 5 | 3 |
| 2-208 | 320 | 5 | 5 | 5 | 1 |
| 2-209 | 320 | 5 | 4 | 5 | 0 |
| 2-210 | 320 | 5 | 5 | 5 | 0 |
| 2-211 | 320 | 5 | 5 | 4 | 2 |
| 2-212 | 320 | 5 | 3 | 4 | 0 |
| 2-213 | 320 | 4 | 3 | 3 | 0 |
| 2-214 | 320 | 3 | 3 | 4 | 0 |
| 2-215 | 320 | 5 | 5 | 4 | 2 |
| 2-216 | 320 | 5 | 3 | 4 | 0 |
| 2-217 | 320 | 5 | 5 | 5 | 5 |
| 2-218 | 320 | 5 | 3 | 5 | 0 |
| 2-219 | 320 | 5 | 3 | 3 | 0 |
| 2-220 | 320 | 5 | 3 | 3 | 0 |
| 2-221 | 320 | 5 | 5 | 5 | 4 |
| 2-222 | 320 | 5 | 5 | 4 | 0 |
| 2-223 | 320 | 5 | 3 | 4 | 3 |
| 2-224 | 320 | 4 | 4 | 3 | 0 |
| 2-225 | 320 | 4 | 3 | 4 | 0 |
| 2-226 | 320 | 4 | 3 | 3 | 0 |
| 2-227 | 320 | 5 | 3 | 5 | 3 |
| 2-228 | 320 | 5 | 4 | 5 | 0 |
| 2-229 | 320 | 4 | 3 | 4 | 0 |
| 2-230 | 320 | 4 | 5 | 5 | 0 |
| 2-231 | 320 | 4 | 3 | 5 | 0 |
| 2-232 | 320 | 4 | 5 | 5 | 0 |
| 2-234 | 320 | 3 | 0 | 0 | 0 |
| 2-236 | 320 | 4 | 3 | 5 | 0 |
| 2-237 | 320 | 3 | 4 | 5 | 0 |
| 2-238 | 224 | 3 | 2 | 3 | 0 |
| 2-240 | 320 | 4 | 3 | 4 | 3 |
| 2-242 | 320 | 3 | 0 | 2 | 0 |
| 2-244 | 320 | 5 | 5 | 5 | 5 |
| 2-245 | 320 | 5 | 4 | 4 | 3 |
| 2-246 | 320 | 3 | 0 | 3 | 0 |
| 2-248 | 320 | 5 | 5 | 4 | 3 |
| 2-249 | 320 | 5 | 3 | 5 | 2 |
| 2-250 | 320 | 3 | 2 | 3 | 0 |
| 2-252 | 320 | 5 | 5 | 5 | 2 |
| 2-253 | 320 | 5 | 3 | 4 | 0 |
| 2-254 | 320 | 5 | 5 | 5 | 5 |
| 2-255 | 320 | 5 | 4 | 5 | 2 |
| 2-256 | 320 | 5 | 4 | 4 | 3 |
| 2-257 | 320 | 5 | 3 | 5 | 2 |
| 2-258 | 320 | 5 | 5 | 5 | 5 |
| 2-259 | 320 | 4 | 3 | | 1 |
| 2-260 | 320 | 5 | 3 | 3 | 4 |
| 2-262 | 320 | 3 | 0 | 2 | 0 |
| 2-264 | 320 | 5 | 3 | 5 | 1 |
| 2-265 | 320 | 5 | 4 | 5 | 0 |
| 2-267 | 320 | 5 | 5 | 3 | 0 |
| 2-268 | 320 | 4 | 3 | 3 | 0 |
| 2-269 | 320 | 3 | 0 | 3 | 0 |
| 2-271 | 320 | 3 | 0 | 3 | 0 |
| 2-275 | 320 | 3 | 0 | 0 | 0 |
| 2-280 | 320 | 5 | 3 | 4 | 0 |
| 2-281 | 320 | 5 | 3 | 5 | 0 |
| 2-282 | 320 | 3 | 0 | 0 | 0 |
| 2-283 | 320 | 5 | 4 | 5 | 0 |
| 2-284 | 320 | 5 | 3 | 5 | 0 |
| 2-285 | 320 | 5 | 3 | 5 | 0 |
| 2-286 | 320 | 5 | 4 | 4 | 0 |
| 2-287 | 320 | 5 | 3 | 5 | 0 |
| 2-288 | 320 | 5 | 4 | 5 | 0 |
| 2-289 | 320 | 5 | 3 | 5 | 1 |
| 2-290 | 320 | 4 | 4 | 3 | 0 |
| 2-291 | 320 | 4 | 3 | 2 | 0 |
| 2-292 | 320 | 5 | 4 | 4 | 1 |
| 2-293 | 320 | 5 | 3 | 5 | 0 |
| 2-294 | 320 | 5 | 4 | 5 | 0 |
| 2-295 | 320 | 5 | 4 | 5 | 0 |
| 2-296 | 320 | 5 | 2 | 3 | 0 |
| 2-297 | 310 | 5 | 0 | 5 | 0 |
| 2-298 | 320 | 5 | 3 | 5 | 0 |
| 2-300 | 320 | 3 | | 3 | 0 |
| 2-301 | 320 | 3 | 0 | 2 | 0 |
| 2-302 | 320 | 3 | 0 | 3 | 0 |
| 2-303 | 320 | 5 | 4 | 3 | 0 |
| 2-304 | 320 | 3 | 3 | 3 | 0 |
| 2-305 | 320 | 5 | 5 | 3 | 0 |
| 2-306 | 320 | 3 | 3 | 3 | 0 |
| 2-307 | 320 | 5 | 5 | 4 | 0 |
| 2-308 | 320 | 5 | 3 | 4 | 0 |
| 2-309 | 320 | 5 | 3 | 5 | 0 |
| 2-310 | 320 | 5 | 4 | 3 | 0 |
| 2-311 | 320 | 5 | 2 | 4 | 0 |
| 2-312 | 320 | 5 | 3 | 5 | 0 |
| 2-313 | 320 | 5 | 2 | 5 | 0 |
| 2-315 | 320 | 5 | 3 | 5 | 4 |
| 2-317 | 320 | 5 | 2 | 5 | 3 |
| 2-319 | 320 | 5 | 4 | 5 | 5 |
| 2-321 | 320 | 5 | 3 | 5 | 4 |
| 2-323 | 320 | 5 | 2 | 3 | 4 |
| 2-325 | 320 | 5 | 3 | 5 | 4 |
| 2-327 | 320 | 5 | 3 | 4 | 4 |
| 2-329 | 320 | 5 | 4 | 4 | 4 |
| 2-330 | 320 | 5 | 2 | 4 | 3 |
| 2-331 | 320 | 5 | 0 | 4 | 3 |
| 2-332 | 320 | 5 | 3 | 3 | 2 |
| 2-333 | 320 | 5 | 2 | 5 | 1 |
| 2-334 | 320 | 5 | 4 | 5 | 4 |
| 2-335 | 320 | 5 | 3 | 5 | 3 |
| 2-336 | 320 | 5 | 0 | 3 | 0 |
| 2-337 | 320 | 3 | 0 | 3 | 0 |
| 2-339 | 320 | 5 | 4 | 4 | 4 |
| 2-340 | 320 | 5 | 5 | 5 | 0 |
| 2-341 | 320 | 4 | 3 | 3 | 0 |
| 2-342 | 320 | 5 | 5 | 5 | 0 |
| 2-343 | 320 | 5 | 5 | 4 | 0 |
| 2-344 | 320 | 5 | 4 | 5 | 0 |
| 2-345 | 320 | 5 | 5 | 5 | 0 |
| 2-346 | 320 | 4 | 3 | 1 | 0 |
| 2-347 | 320 | 4 | 4 | 4 | 0 |
| 2-348 | 320 | 5 | 5 | 5 | 0 |
| 2-349 | 320 | 5 | 5 | 4 | 0 |
| 2-350 | 320 | 5 | 5 | 5 | 0 |
| 2-351 | 320 | 5 | 5 | 4 | 0 |
| 2-352 | 320 | 5 | 5 | 5 | 1 |
| 2-353 | 320 | 5 | 5 | 5 | 0 |
| 2-354 | 320 | 5 | 5 | 5 | 3 |
| 2-355 | 320 | 5 | 5 | 5 | 0 |
| 2-356 | 320 | 4 | 4 | 4 | 0 |
| 2-357 | 320 | 5 | 3 | 2 | 0 |
| 2-358 | 320 | 3 | 1 | 3 | 0 |
| 2-359 | 320 | 3 | 5 | 3 | 0 |
| 2-360 | 320 | 5 | 4 | 3 | 1 |
| 2-362 | 320 | 3 | 0 | 3 | 0 |
| 2-364 | 320 | 4 | 4 | 5 | 0 |
| 2-365 | 320 | 4 | 5 | 5 | 0 |
| 2-366 | 320 | 3 | 0 | 3 | 0 |
| 2-368 | 320 | 4 | 3 | 3 | 0 |
| 2-369 | 320 | 4 | 5 | 4 | 0 |
| 2-370 | 320 | 3 | 2 | 5 | 0 |
| 2-371 | 320 | 4 | 4 | 4 | 0 |
| 2-372 | 84 | 4 | 4 | 3 | 0 |
| 2-374 | 320 | 5 | 4 | 5 | 5 |
| 2-376 | 246 | 5 | 3 | 3 | 1 |
| 2-377 | 101 | 4 | 3 | 3 | 0 |
| 2-378 | 320 | 5 | 5 | 3 | 0 |
| 2-382 | 280 | 0 | 0 | 3 | 0 |
| 2-383 | 320 | 5 | 5 | 5 | 0 |
| 2-384 | 320 | 3 | 3 | 3 | 0 |
| 2-385 | 320 | 5 | 5 | 5 | 2 |
| 2-386 | 320 | 5 | 4 | 3 | 0 |
| 2-387 | 320 | 5 | 5 | 5 | |
| 2-388 | 320 | 5 | 3 | 3 | 0 |
| 2-389 | 320 | 5 | 3 | 5 | 2 |
| 2-390 | 320 | 5 | 3 | 5 | 1 |
| 2-391 | 320 | 5 | 4 | 5 | 1 |
| 2-392 | 320 | 5 | 4 | 5 | 1 |
| 2-393 | 320 | 5 | 1 | 3 | 0 |
| 2-394 | 320 | 5 | 3 | 5 | 0 |
| 2-395 | 320 | 3 | 3 | 3 | 0 |
| 2-398 | 320 | | 0 | 3 | 0 |
| 2-400 | 320 | | 5 | 5 | 0 |
| 2-402 | 320 | 5 | 3 | 3 | 1 |
| 2-404 | 320 | 3 | 0 | 3 | 0 |
| 2-406 | 320 | 4 | 4 | 5 | 0 |
| 2-407 | 320 | 5 | 4 | 4 | 0 |
| 2-408 | 320 | 4 | 4 | 5 | 0 |
| 2-409 | 320 | 5 | 4 | 5 | 0 |
| 2-410 | 80 | 4 | 0 | 1 | 0 |
| 2-411 | 320 | 5 | 3 | 3 | 0 |
| 2-412 | 320 | 4 | 0 | 4 | 0 |
| 2-413 | 320 | 5 | 4 | 4 | 0 |
| 2-414 | 320 | 4 | 3 | 3 | 0 |
| 2-416 | 320 | 5 | 3 | 4 | 0 |
| 2-418 | 320 | 5 | 3 | 5 | 4 |
| 2-420 | 320 | 5 | 3 | 4 | 0 |
| 2-421 | 320 | 5 | 2 | 5 | 0 |
| 2-422 | 320 | 5 | 3 | 5 | 0 |
| 2-424 | 320 | 5 | 3 | 4 | 0 |
| 2-425 | 320 | 4 | 2 | 5 | 1 |
| 2-427 | 320 | 3 | 0 | 2 | 0 |
| 2-428 | 125 | 4 | 0 | 0 | 0 |
| 2-429 | 320 | 3 | 0 | 0 | 0 |
| 2-430 | 320 | 2 | 0 | 3 | 0 |
| 2-431 | 320 | 5 | 3 | 5 | 0 |
| 2-432 | 320 | 3 | 0 | 2 | 0 |
| 2-434 | 320 | 2 | | 2 | 0 |
| 2-436 | 320 | 3 | 0 | 2 | 0 |
| 2-438 | 320 | 2 | 0 | 2 | 0 |
| 2-440 | 320 | 5 | 0 | 0 | 3 |
| 2-441 | 320 | 5 | 0 | | 1 |
| 2-442 | 320 | 5 | 2 | 3 | 1 |
| 2-443 | 320 | 5 | 4 | 5 | 5 |
| 2-444 | 320 | 5 | 4 | 5 | 5 |
| 2-450 | 320 | 3 | 0 | 5 | 0 |
| 2-451 | 320 | 5 | 3 | 5 | 2 |
| 2-452 | 320 | 5 | 0 | 5 | 0 |
| 2-453 | 320 | 5 | 3 | 2 | 4 |
| 2-454 | 320 | 5 | 2 | 5 | 3 |
| 2-455 | 320 | 5 | 2 | 0 | 1 |
| 2-456 | 320 | 3 | 0 | 0 | 0 |
| 2-457 | 320 | 5 | 3 | 5 | 3 |
| 2-459 | 320 | 3 | 0 | 0 | 0 |
| 2-460 | 403 | 4 | 0 | 0 | 2 |
| 2-461 | 320 | 4 | 0 | 4 | 0 |
| 2-463 | 320 | 3 | 0 | 0 | 0 |
| 2-465 | 320 | 5 | 3 | 5 | 1 |
| 2-467 | 320 | 3 | 4 | 4 | 0 |
| 2-468 | 320 | 3 | 2 | 2 | 0 |
| 3-001 | 320 | 5 | 5 | 5 | 0 |
| 3-002 | 320 | 5 | 0 | 3 | 0 |
| 3-003 | 320 | 5 | 3 | 5 | 3 |
| 3-004 | 320 | 5 | 4 | 5 | 1 |
| 3-005 | 320 | 5 | 3 | 5 | 3 |
| 3-006 | 320 | 5 | 4 | 5 | 1 |
| 3-007 | 320 | 5 | 5 | 5 | 1 |
| 3-008 | 320 | 4 | 4 | 5 | 0 |
| 3-009 | 320 | 5 | 5 | 5 | 0 |
| 3-010 | 320 | 5 | 0 | 4 | 3 |
| 3-011 | 320 | 5 | 4 | 5 | 3 |
| 3-012 | 320 | 5 | 2 | 5 | 3 |
| 3-013 | 320 | 4 | 4 | 5 | 0 |
| 3-014 | 320 | 5 | 0 | 4 | 0 |
| 3-015 | 320 | 3 | 3 | 3 | 0 |
| 3-016 | 320 | 3 | 4 | 4 | 0 |
| 3-017 | 320 | 3 | 0 | 0 | 0 |
| 3-018 | 45 | 3 | 0 | 2 | 0 |
| 3-019 | 320 | 3 | 0 | 3 | 3 |
| 3-020 | 320 | 4 | 3 | 5 | 0 |
| 3-021 | 320 | 5 | 3 | 5 | 0 |
| 3-022 | 320 | 4 | 2 | 0 | 0 |
| 3-023 | 320 | 5 | 3 | 5 | 0 |
| 3-024 | 320 | 5 | 3 | 5 | 0 |
| 3-025 | 320 | 3 | 0 | 2 | 0 |
| 3-026 | 320 | 4 | 0 | 2 | 0 |
| 3-027 | 320 | 5 | 3 | 5 | 0 |
| 3-028 | 320 | 4 | 3 | 5 | 0 |
| 3-029 | 285 | 5 | 3 | 5 | 1 |
| 3-030 | 320 | 4 | 2 | 0 | 0 |
| 3-031 | 320 | 5 | 2 | 3 | 1 |
| 3-032 | 320 | 5 | 3 | 3 | 0 |
| 3-033 | 320 | 4 | 3 | 5 | 0 |
| 3-034 | 320 | 5 | 4 | 5 | 0 |
| 3-035 | 320 | 4 | 3 | 5 | 0 |
| 3-036 | 320 | 5 | 0 | 4 | 0 |
| 3-037 | 320 | 4 | 3 | 4 | 0 |
| 3-038 | 320 | 3 | 1 | 3 | 0 |
| 3-039 | 320 | 4 | 3 | 5 | 0 |
| 3-040 | 320 | 5 | 0 | 5 | 0 |
| 3-041 | 320 | 2 | 0 | 1 | 0 |
| 3-042 | 320 | 4 | 0 | 3 | 0 |
| 3-043 | 320 | 5 | 3 | 5 | 2 |
| 3-044 | 320 | 5 | 0 | 3 | 0 |
| 3-045 | 320 | 5 | 4 | 5 | 1 |
| 3-046 | 320 | 5 | 2 | 5 | 0 |
| 3-047 | 320 | 3 | 3 | 2 | 0 |
| 3-048 | 320 | 4 | 0 | 2 | 0 |
| 3-049 | 274 | 5 | 5 | 3 | 5 |
| 3-050 | 224 | 4 | 3 | 3 | 2 |
| 3-051 | 320 | 1 | 0 | 3 | 1 |
| 3-053 | 320 | 5 | 4 | 5 | 5 |
| 3-054 | 304 | 5 | 3 | 5 | 3 |
| 3-055 | 320 | 5 | 4 | 5 | 5 |
| 3-056 | 320 | 3 | 1 | 3 | 0 |
| 3-057 | 320 | 5 | 3 | 4 | 5 |
| 3-058 | 320 | 5 | 4 | 5 | 5 |
| 3-059 | 320 | 4 | 0 | 4 | 0 |
| 3-060 | 320 | 3 | 4 | 3 | 1 |
| 3-061 | 320 | 2 | 3 | 5 | 0 |
| 3-062 | 320 | 2 | 1 | 5 | 1 |
| 3-063 | 320 | 0 | 0 | 5 | 0 |
| 3-065 | 214 | 5 | 2 | 5 | 1 |
| 3-066 | 296 | 5 | 1 | 5 | 2 |
| 3-068 | 320 | 3 | 2 | 3 | 1 |
| 3-069 | 320 | 0 | 0 | 3 | 0 |
| 3-070 | 80 | 5 | 5 | 5 | 4 |
| 3-071 | 320 | 5 | 5 | 5 | 5 |
| 3-072 | 320 | 4 | 3 | 4 | 0 |
| 3-074 | 320 | 4 | 3 | 5 | 0 |
| 3-075 | 320 | 0 | 3 | 3 | 0 |
| 3-076 | 320 | 5 | 3 | 5 | 3 |
| 3-077 | 320 | 5 | 1 | 5 | 2 |
| 3-078 | 80 | 5 | 3 | 5 | 5 |
| 3-079 | 320 | 5 | 4 | 5 | 5 |
| 3-080 | 320 | 1 | 0 | 4 | 0 |
| 3-082 | 320 | 5 | 3 | 5 | 1 |
| 3-083 | 320 | 4 | 4 | 4 | 0 |
| 3-084 | 320 | 5 | 0 | 3 | 0 |
| 3-085 | 320 | 5 | 0 | 4 | 4 |
| 3-086 | 320 | 3 | 0 | 0 | 0 |
| 3-087 | 320 | 4 | 0 | 3 | 1 |
| 3-088 | 320 | 5 | 4 | 5 | 0 |
| 3-089 | 320 | 5 | 4 | 5 | 5 |
| 3-090 | 310 | 5 | 5 | 5 | 4 |
| 3-091 | 320 | 2 | 0 | 4 | 0 |
| 3-092 | 320 | 0 | | 4 | 0 |
| 3-093 | 320 | 5 | 5 | 5 | 4 |
| 3-094 | 323 | 5 | 5 | 5 | 3 |
| 3-095 | 320 | 5 | 5 | 5 | 5 |
| 3-096 | 320 | 5 | 5 | 5 | 4 |
| 3-097 | 320 | 4 | 4 | 3 | 1 |
| 3-099 | 320 | 5 | 5 | 5 | 5 |
| 3-101 | 320 | 5 | 3 | 5 | 5 |
| 3-103 | 320 | 4 | 5 | 5 | 5 |
| 3-105 | 320 | 5 | 5 | 4 | 4 |
| 3-107 | 320 | 4 | 3 | 4 | 5 |
| 3-108 | 320 | 3 | 2 | 1 | 0 |
| 3-109 | 320 | 3 | 4 | 4 | 1 |
| 3-111 | 320 | 5 | 3 | 5 | 5 |
| 3-113 | 320 | 5 | 3 | 5 | 5 |
| 3-114 | 320 | 5 | 4 | 5 | 5 |
| 3-116 | 320 | 5 | 5 | 5 | 5 |
| 3-118 | 320 | 4 | 0 | 5 | 1 |
| 3-119 | 320 | 4 | 4 | 5 | 0 |
| 3-121 | 320 | 5 | 4 | 5 | 5 |
| 3-123 | 320 | 5 | 5 | 5 | 5 |
| 3-125 | 320 | 5 | 3 | 5 | 4 |
| 3-127 | 320 | 4 | 3 | 5 | 3 |
| 3-129 | 320 | 5 | 4 | 5 | 4 |
| 3-131 | 320 | 5 | 3 | 5 | 5 |
| 3-133 | 320 | 3 | 3 | 5 | 5 |
| 3-134 | 320 | 3 | 4 | 3 | 0 |
| 3-135 | 320 | 4 | 3 | 2 | 3 |
| 3-137 | 320 | 3 | 5 | 5 | 3 |
| 3-139 | 320 | 4 | 4 | 4 | 0 |
| 3-140 | 320 | 5 | 4 | 5 | 0 |
| 3-141 | 320 | 5 | 2 | 5 | 0 |
| 3-143 | 320 | 5 | 4 | 5 | 0 |
| 3-144 | 320 | 5 | 4 | 5 | 1 |
| 3-145 | 320 | 3 | 5 | 4 | 0 |
| 3-146 | 320 | 3 | 3 | 3 | 1 |
| 3-147 | 320 | 4 | 2 | 3 | 0 |
| 3-149 | 320 | 4 | 5 | 4 | 0 |
| 3-150 | 320 | 5 | 3 | 2 | 0 |
| 3-151 | 320 | 3 | 0 | 3 | 0 |
| 3-153 | 320 | 5 | 2 | 0 | 0 |
| 3-154 | 320 | 3 | 2 | 3 | 0 |
| 3-155 | 320 | 5 | 4 | 4 | 2 |
| 3-156 | 320 | 5 | 4 | 3 | 1 |
| 3-157 | 320 | 5 | 3 | 3 | 2 |
| 3-158 | 320 | 5 | 3 | 4 | 1 |
| 3-159 | 320 | 2 | 0 | 3 | 0 |
| 3-161 | 320 | 5 | 3 | 3 | 0 |
| 3-163 | 320 | 5 | 5 | 3 | 5 |
| 3-165 | 320 | 5 | 4 | 3 | 2 |
| 3-170 | 320 | 3 | 0 | 0 | 0 |
| 4-001 | 320 | 4 | 3 | 5 | 0 |
| 4-002 | 320 | 5 | 2 | 5 | 0 |
| 4-003 | 320 | 3 | 3 | 3 | 0 |
| 4-004 | 320 | 4 | 3 | 4 | 0 |
| 4-005 | 320 | 4 | 3 | 5 | 1 |
| 4-006 | 320 | 5 | 5 | 5 | 0 |
| 4-007 | 320 | 5 | 3 | 5 | 1 |
| 4-008 | 320 | 5 | 3 | 5 | 0 |
| 4-009 | 320 | 5 | 3 | 5 | 0 |
| 4-010 | 320 | 5 | 4 | 5 | 1 |
| 4-011 | 320 | 4 | 3 | 5 | 0 |
| 4-012 | 320 | 5 | 3 | 5 | 0 |
| 4-013 | 320 | 3 | 3 | 4 | 0 |
| 4-014 | 320 | 3 | 0 | 0 | 0 |
| 4-015 | 320 | 3 | 3 | 5 | 0 |
| 4-016 | 320 | 3 | 5 | 4 | 0 |
| 4-017 | 320 | 4 | 0 | 3 | 0 |
| 4-018 | 320 | 4 | 4 | 3 | 0 |
| 4-019 | 320 | 3 | 4 | 5 | 0 |
| 4-020 | 320 | 4 | 5 | 5 | 0 |
| 4-021 | 320 | 4 | 4 | 5 | 0 |
| 4-022 | 320 | 5 | 5 | 4 | 0 |
| 4-023 | 320 | 5 | 3 | 5 | 0 |
| 4-024 | 320 | 4 | 3 | 5 | 0 |
| 4-025 | 320 | 5 | 3 | 5 | 0 |
| 4-026 | 320 | 5 | 5 | 5 | 0 |
| 4-027 | 320 | 5 | 0 | 0 | 0 |
| 4-028 | 320 | 5 | 3 | 5 | 0 |
| 4-029 | 320 | 3 | 0 | 0 | 0 |
| 4-030 | 320 | 3 | 5 | 3 | 0 |
| 4-031 | 320 | 3 | 3 | 5 | 0 |
| 4-032 | 320 | 4 | 3 | 5 | 0 |
| 4-033 | 320 | 4 | 2 | 3 | 0 |
| 4-034 | 320 | 4 | 5 | 4 | 0 |
| 4-035 | 320 | 5 | 3 | 5 | 1 |
| 4-036 | 320 | 5 | 5 | 5 | 1 |
| 4-037 | 320 | 3 | 1 | 0 | 0 |
| 4-038 | 320 | 4 | 5 | 4 | 0 |
| 4-039 | 320 | 4 | 3 | 3 | 1 |
| 4-040 | 320 | 5 | 4 | 5 | 0 |
| 4-041 | 320 | 5 | 4 | 5 | 0 |
| 4-044 | 320 | 5 | 5 | 5 | 0 |
| 4-045 | 320 | 5 | 3 | 5 | 0 |
| 4-046 | 320 | 5 | 1 | 5 | 1 |
| 4-048 | 320 | 5 | 5 | 5 | 0 |
| 4-049 | 320 | 5 | 3 | 5 | 0 |
| 4-050 | 320 | 5 | 4 | 4 | 0 |
| 4-051 | 320 | 5 | 3 | 3 | 0 |
| 4-052 | 320 | 5 | 3 | 4 | 0 |
| 4-053 | 320 | 4 | 3 | 4 | 0 |
| 4-054 | 320 | 2 | 0 | 0 | 0 |
| 4-056 | 320 | 4 | 4 | 4 | 0 |
| 4-058 | 320 | 4 | 3 | 3 | 0 |
| 4-059 | 320 | 4 | 4 | 4 | 0 |
| 4-060 | 320 | 3 | 1 | 3 | 0 |
| 4-061 | 320 | 3 | 3 | 4 | 0 |
| 4-062 | 320 | 4 | 4 | 3 | 0 |
| 4-063 | 320 | 5 | 3 | 4 | 0 |
| 4-064 | 320 | 4 | 3 | 4 | 0 |
| 4-065 | 320 | 4 | 3 | 3 | 0 |
| 4-066 | 320 | | 0 | 3 | 0 |
| 4-068 | 320 | 4 | 3 | 3 | 0 |
| 4-069 | 320 | 4 | 3 | 3 | 1 |
| 4-070 | 320 | 5 | 4 | 4 | 0 |
| 4-072 | 320 | 3 | 3 | 4 | 0 |
| 4-074 | 320 | 4 | 0 | 3 | 0 |
| 4-076 | 320 | 4 | 3 | 3 | 0 |
| 4-077 | 320 | 5 | 4 | 5 | 0 |
| 4-078 | 320 | 3 | 2 | 3 | 0 |
| 4-079 | 320 | 2 | 2 | 2 | 0 |
| 4-080 | 320 | 2 | 3 | 4 | 0 |
| 4-081 | 320 | 3 | 0 | 0 | 0 |
| 4-083 | 320 | 5 | 4 | 5 | 0 |
| 4-084 | 320 | 5 | 4 | 4 | 0 |
| 4-085 | 320 | 2 | 0 | 2 | 0 |
| 4-086 | 320 | 4 | 3 | 3 | 0 |
| 4-088 | 320 | 5 | 3 | 5 | 1 |
| 4-090 | 320 | 5 | 3 | 2 | 0 |
| 4-091 | 320 | 5 | 5 | 3 | 0 |
| 4-092 | 320 | 5 | 3 | 4 | 0 |
| 4-093 | 320 | 5 | 4 | 4 | 1 |
| 4-094 | 320 | 5 | 4 | 4 | 1 |
| 4-096 | 320 | 5 | 3 | 3 | 1 |
| 4-098 | 320 | 4 | 3 | 3 | 0 |
| 4-099 | 320 | 5 | 5 | 4 | 0 |
| 4-100 | 320 | 2 | 0 | 0 | 0 |
| 4-102 | 320 | 1 | 3 | 5 | 0 |
| 4-104 | 320 | 2 | 0 | 0 | 0 |
| 4-106 | 320 | 4 | 0 | 5 | 0 |
| 4-108 | 320 | 4 | 0 | 5 | 0 |
| 4-110 | 320 | 5 | 0 | 5 | 0 |
| 4-112 | 320 | 5 | 4 | 5 | 0 |
| 4-114 | 320 | 4 | 3 | 4 | 0 |
| 4-116 | 320 | 3 | 4 | 3 | 0 |
| 4-118 | 320 | 4 | 3 | 4 | 0 |
| 4-120 | 320 | 2 | 3 | 2 | 0 |
| 4-122 | 320 | 5 | 0 | 4 | 0 |
| 4-124 | 320 | 5 | 3 | 5 | 1 |
| 4-126 | 320 | 3 | 0 | 3 | 0 |
| 4-128 | 320 | 3 | 0 | 5 | 0 |
| 4-130 | 320 | 3 | 0 | 4 | 0 |
| 5-001 | 320 | 4 | 3 | 5 | 0 |
| 5-002 | 320 | 4 | 3 | 0 | 0 |
| 5-003 | 320 | 3 | 3 | 5 | 0 |
| 5-004 | 320 | 3 | 0 | 0 | 0 |
| 5-005 | 320 | 3 | 5 | 4 | 0 |
| 5-006 | 320 | 4 | 1 | 3 | 0 |
| 5-007 | 320 | 4 | 1 | 2 | 0 |
| 5-008 | 320 | 4 | 0 | 0 | 0 |
| 5-009 | 320 | 4 | 3 | 3 | 1 |
| 5-010 | 320 | 3 | 3 | 4 | 0 |
| 5-011 | 320 | 5 | 3 | 5 | 0 |
| 5-012 | 320 | 4 | 2 | 3 | 0 |
| 5-014 | 320 | 4 | 0 | 5 | 0 |
| 5-015 | 320 | 2 | 2 | 3 | 0 |
| 5-016 | 320 | 5 | 2 | 5 | 0 |
| 5-017 | 320 | 5 | 0 | 5 | 0 |
| 5-018 | 320 | 4 | 0 | 3 | 0 |
| 5-019 | 320 | 4 | 0 | 1 | 0 |
| 5-020 | 320 | 3 | 0 | 0 | 0 |
| 5-021 | 320 | 4 | 3 | 5 | 0 |
| 5-022 | 272 | 4 | 3 | 3 | 0 |
| 5-023 | 320 | 4 | 3 | 0 | 0 |
| 5-024 | 320 | 4 | 3 | 0 | 4 |
| 5-025 | 320 | 3 | 4 | 4 | 0 |
| 5-026 | 320 | 3 | 4 | 5 | 0 |
| 5-027 | 320 | 3 | 0 | 0 | 0 |
| 5-028 | 344 | 5 | 0 | 0 | 0 |
| 5-029 | 320 | 4 | 4 | 5 | 0 |
| 5-030 | 320 | 4 | 0 | 0 | 0 |
| 5-031 | 320 | 5 | 4 | 5 | 3 |
| 5-032 | 320 | 4 | 3 | 0 | 0 |
| 5-033 | 320 | 4 | 3 | 4 | 0 |
| 5-034 | 320 | 4 | 1 | 0 | 0 |
| 5-035 | 320 | 4 | 3 | 3 | 0 |
| 5-036 | 320 | 2 | 0 | 0 | 0 |
| 5-037 | 320 | 4 | 4 | 5 | 0 |
| 5-038 | 320 | 2 | 2 | 4 | 0 |
| 5-039 | 320 | 4 | 3 | 5 | 0 |
| 5-040 | 320 | 4 | 1 | 0 | 0 |
| 5-041 | 320 | 4 | 3 | 3 | 0 |
| 5-042 | 320 | 5 | 2 | 4 | 0 |
| 5-043 | 320 | 3 | 3 | 4 | 0 |
| 5-044 | 320 | 3 | 3 | 3 | 0 |
| 5-045 | 320 | 4 | 3 | 3 | 1 |
| 5-046 | 320 | 5 | 3 | 5 | 0 |
| 5-047 | 320 | 3 | 0 | 0 | 0 |
| 5-048 | 320 | 3 | 4 | 0 | 0 |
| 5-049 | 320 | 3 | 0 | 0 | 0 |
| 5-050 | 320 | 2 | 4 | 0 | 0 |
| 5-051 | 320 | 4 | 3 | 3 | 0 |
| 5-052 | 320 | 5 | 3 | 5 | 1 |
| 5-053 | 320 | 3 | 3 | 5 | 0 |
| 5-054 | 320 | 4 | 2 | 5 | 0 |
| 5-055 | 320 | 1 | 0 | 3 | 0 |
| 5-058 | 320 | 0 | 0 | 3 | 0 |
| 5-059 | 320 | 5 | 3 | 4 | 4 |
| 5-060 | 320 | 5 | 2 | 3 | 3 |
| 5-061 | 320 | 5 | 3 | 4 | 3 |
| 5-063 | 320 | 3 | 0 | 2 | 0 |
| 5-064 | 320 | 5 | 2 | 5 | 3 |
| 5-065 | 320 | 4 | 3 | 4 | 2 |
| 5-067 | 320 | 3 | 5 | 4 | 0 |
| 5-068 | 320 | 4 | 0 | 1 | 0 |
| 5-069 | 320 | 4 | 0 | 0 | 0 |
| 5-070 | 320 | 3 | 0 | 0 | 0 |
| 5-072 | 320 | 5 | 3 | 5 | 4 |
| 5-073 | 320 | 5 | 0 | 0 | 2 |
| 5-074 | 320 | 3 | 5 | 3 | 0 |
| 5-075 | 320 | 5 | 3 | 3 | 1 |
| 5-076 | 344 | 5 | 0 | 0 | 0 |
| 5-077 | 320 | 5 | 2 | 4 | 2 |
| 5-078 | 270 | 4 | 4 | 3 | 3 |
| 5-079 | 320 | 3 | 0 | 0 | 0 |
| 5-080 | 370 | 3 | 3 | 2 | 0 |
| 5-081 | 320 | 2 | 2 | 0 | 0 |
| 5-082 | 320 | 5 | 4 | 4 | 3 |
| 5-083 | 320 | 1 | 0 | 2 | 0 |
| 5-084 | 320 | 4 | 0 | 3 | 0 |
| 5-086 | 320 | 3 | 0 | 0 | 0 |
| 5-088 | 320 | 3 | 2 | 2 | 0 |
| 5-089 | 320 | 1 | 3 | 3 | 0 |
| 5-090 | 320 | 5 | 2 | 5 | 4 |
| 5-091 | 320 | 4 | 4 | 3 | 3 |
| 5-092 | 320 | 2 | 0 | 0 | 0 |
| 5-093 | 320 | 1 | 2 | 2 | 0 |
| 5-094 | 320 | 5 | 3 | 4 | 3 |
| 5-095 | 320 | 5 | 5 | 4 | 5 |
| 5-096 | 320 | 3 | 2 | 2 | 0 |
| 5-097 | 320 | 4 | 2 | 1 | 2 |
| 5-101 | 320 | 5 | 2 | 3 | 3 |
| 5-102 | 320 | 4 | 4 | 3 | 4 |
| 5-103 | 320 | 5 | 4 | 5 | 4 |
| 5-104 | 320 | 5 | 5 | 4 | 5 |
| 5-105 | 320 | 5 | 0 | 0 | 1 |
| 5-107 | 320 | 3 | 0 | 0 | 0 |
| 6-001 | 320 | 3 | 2 | 4 | 0 |
| 6-002 | 320 | 4 | 5 | 3 | 3 |
| 7-001 | 320 | 5 | 1 | 4 | 0 |
| 7-002 | 320 | 5 | 2 | 4 | 1 |
| 7-003 | 320 | 4 | 3 | 5 | 3 |
| 7-004 | 320 | 5 | 5 | 5 | 4 |
| 8-001 | 320 | 4 | 3 | 1 | 0 |
| 8-002 | 320 | 4 | 3 | 4 | 0 |
| 8-003 | 320 | 5 | 3 | 4 | 0 |
| 8-004 | 320 | 5 | 3 | 4 | 0 |
| 8-005 | 320 | 5 | 4 | 4 | 1 |
| 8-007 | 320 | 5 | 3 | 5 | 2 |
| 8-008 | 320 | 5 | 4 | 4 | 3 |
| 9-002 | 320 | 3 | 0 | 2 | 1 |
| 9-003 | 320 | 3 | 0 | 1 | 0 |
| 9-005 | 390 | 3 | 0 | 2 | 0 |
| 9-006 | 147 | 1 | 0 | 2 | 0 |
| 9-007 | 320 | 4 | 1 | 5 | 0 |
| 9-008 | 320 | 2 | 3 | 5 | 0 |
| 9-009 | 320 | 3 | 0 | 1 | 0 |
| 9-010 | 246 | 4 | 5 | 4 | 1 |
| 9-011 | 320 | 5 | 0 | 4 | 1 |
| 9-012 | 320 | 4 | 0 | 5 | 0 |
| 9-013 | 320 | 1 | 0 | 3 | 0 |
| 9-014 | 320 | 3 | 3 | 5 | 0 |
| 9-016 | 320 | 5 | 5 | 4 | 0 |
| 9-017 | 320 | 5 | 3 | 5 | 2 |
| 9-019 | 320 | 4 | 4 | 4 | 0 |
| 9-020 | 320 | 5 | 4 | 5 | 0 |
| 9-021 | 320 | 3 | 3 | 5 | 0 |
| 9-023 | 320 | 5 | 3 | 5 | 1 |
| 9-025 | 320 | 5 | 2 | 5 | 0 |
| 9-027 | 320 | 5 | 2 | 5 | 5 |
| 9-029 | 320 | 4 | 3 | 5 | 0 |
| 9-031 | 320 | 5 | 1 | 5 | 1 |
| 9-033 | 320 | 4 | 3 | 5 | 0 |
| 9-035 | 320 | 5 | 3 | 5 | 5 |
| 9-037 | 320 | 5 | 2 | 5 | 3 |
| 9-039 | 320 | 5 | 1 | 5 | 3 |
| 9-041 | 320 | 5 | 2 | 5 | 1 |
| 9-043 | 320 | 4 | 3 | 5 | 2 |
| 9-045 | 320 | 5 | 3 | 5 | 4 |
| 9-047 | 320 | 2 | 0 | 3 | 0 |
| 9-049 | 320 | 5 | 2 | 3 | 1 |
| 9-050 | 320 | 4 | 3 | 5 | 1 |
| 9-051 | 320 | 5 | 3 | 4 | 4 |
| 9-052 | 320 | 5 | 0 | 4 | 1 |
| 9-053 | 320 | 4 | 0 | 4 | 0 |
| 9-055 | 320 | 5 | 5 | 5 | 1 |
| 9-056 | 438 | 5 | 3 | 4 | 2 |
| 9-057 | 320 | 5 | 3 | 5 | 5 |
| 9-058 | 320 | 5 | 3 | 5 | 5 |
| 9-059 | 320 | 5 | 3 | 5 | 5 |
| 9-060 | 402 | 4 | 2 | 5 | 3 |
| 9-061 | 320 | 5 | 4 | 5 | 5 |
| 9-062 | 320 | 5 | 3 | 5 | 2 |
| 9-063 | 320 | 5 | 3 | 4 | 5 |
| 9-064 | 320 | 5 | 1 | 5 | 3 |
| 10-001 | 320 | 3 | 0 | 2 | 0 |
| 11-001 | 320 | 3 | 3 | 5 | 0 |

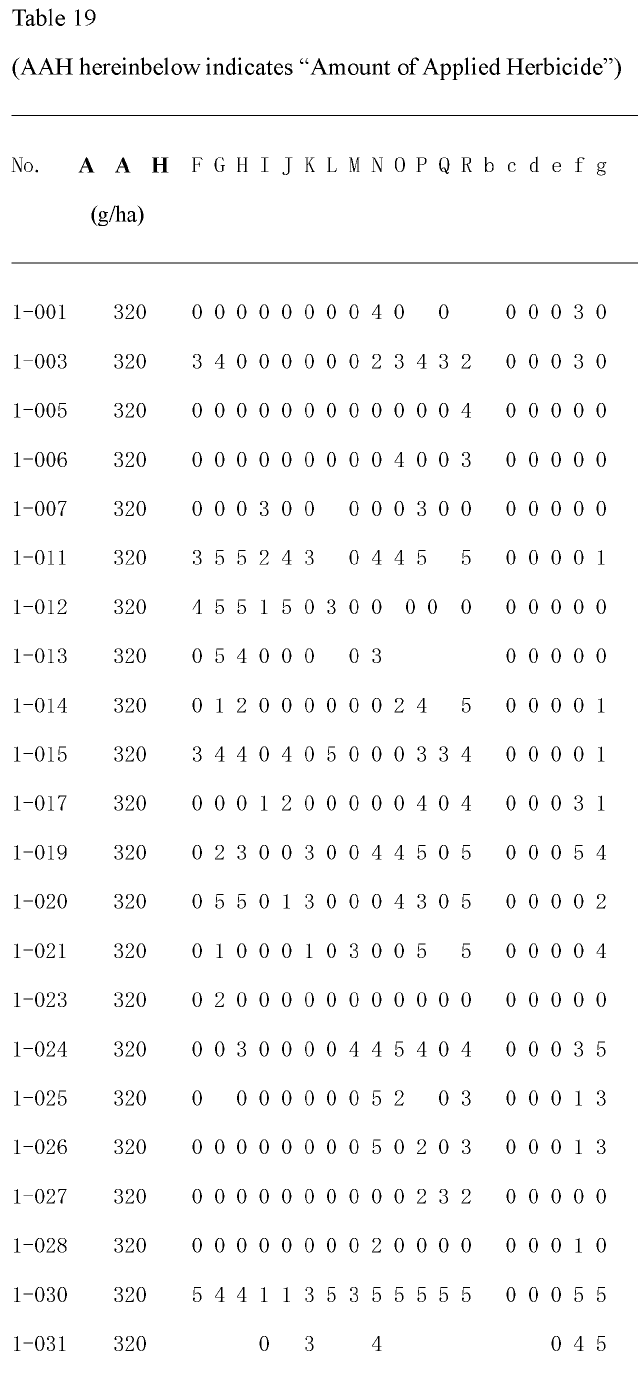

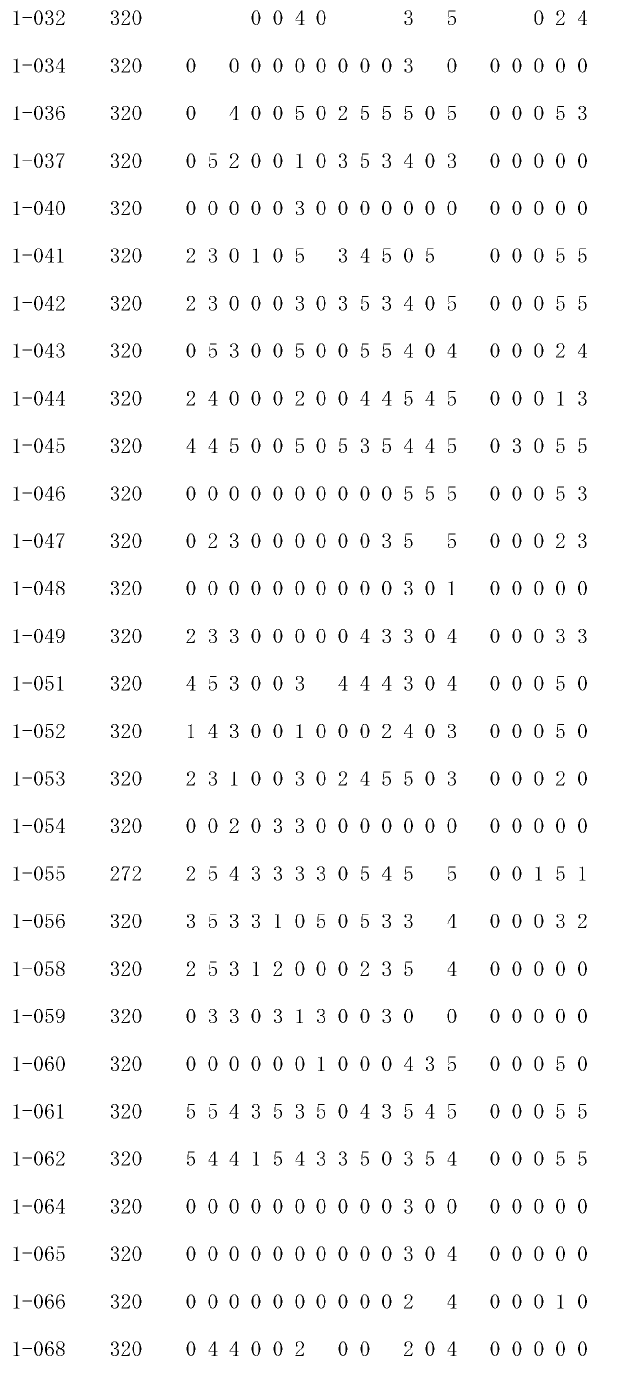

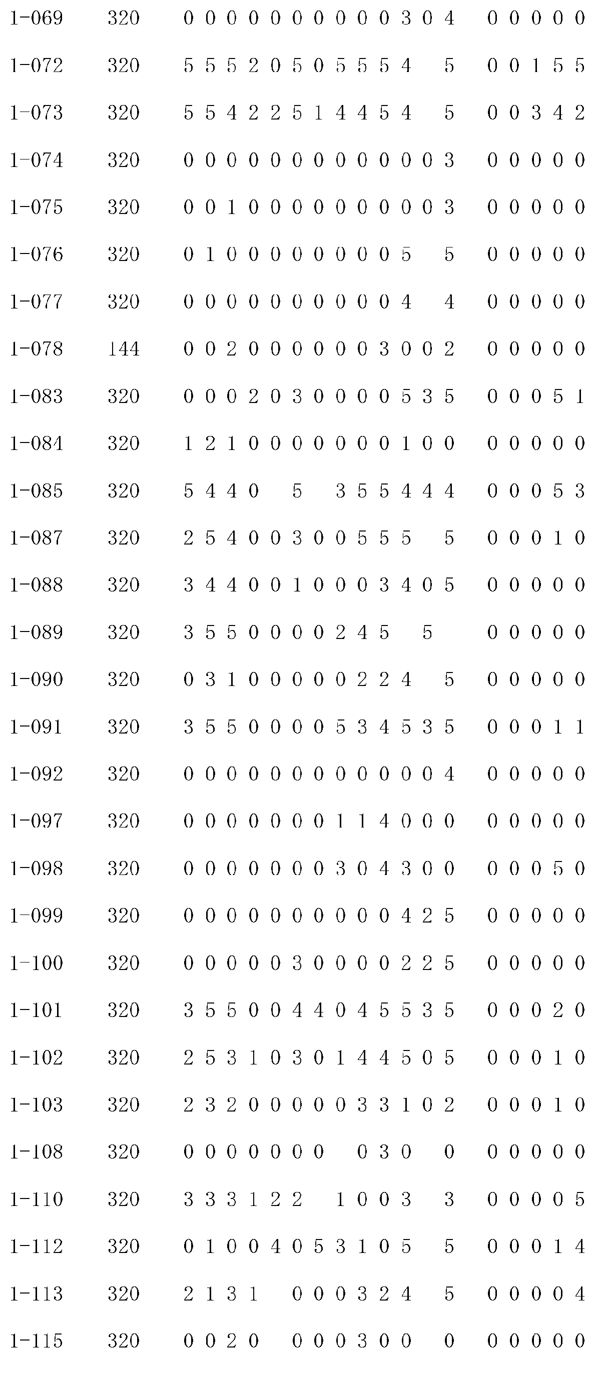

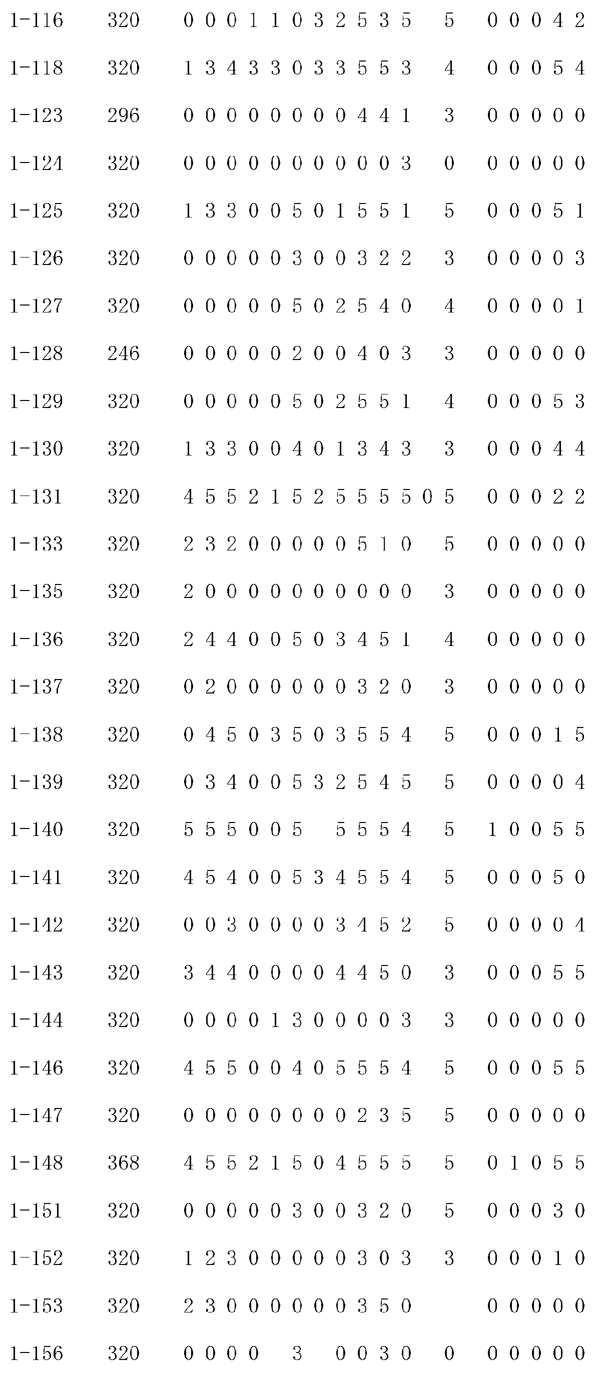

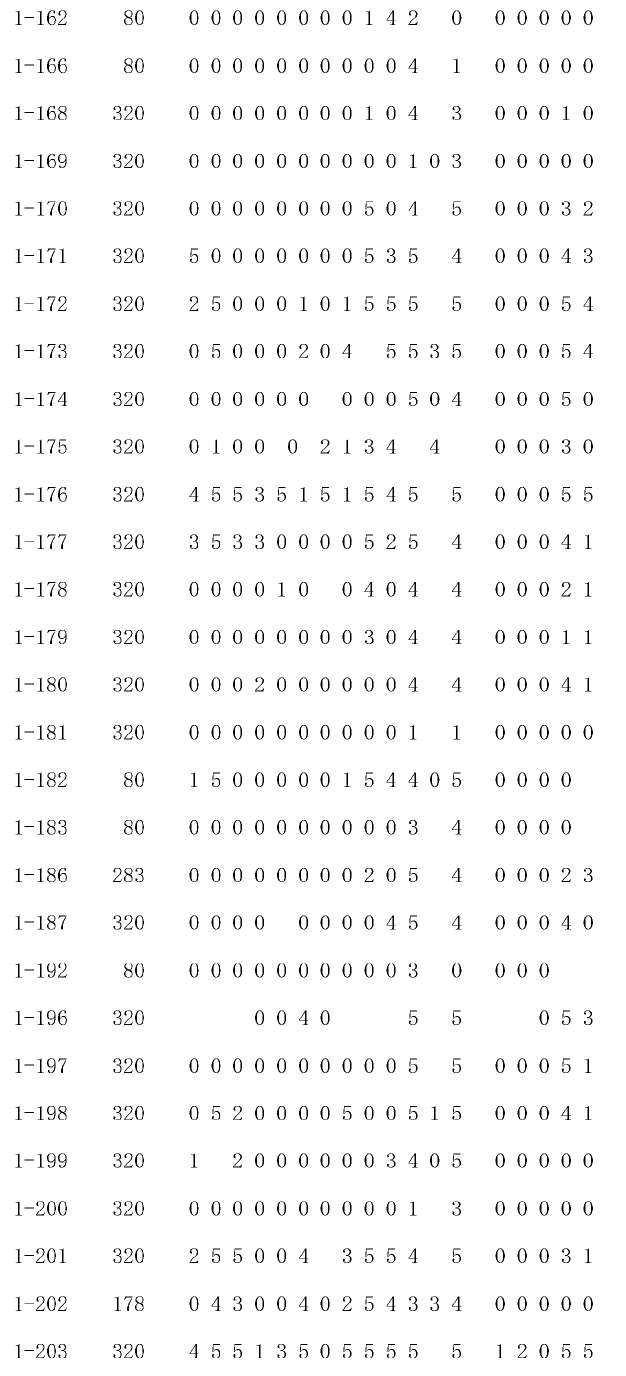

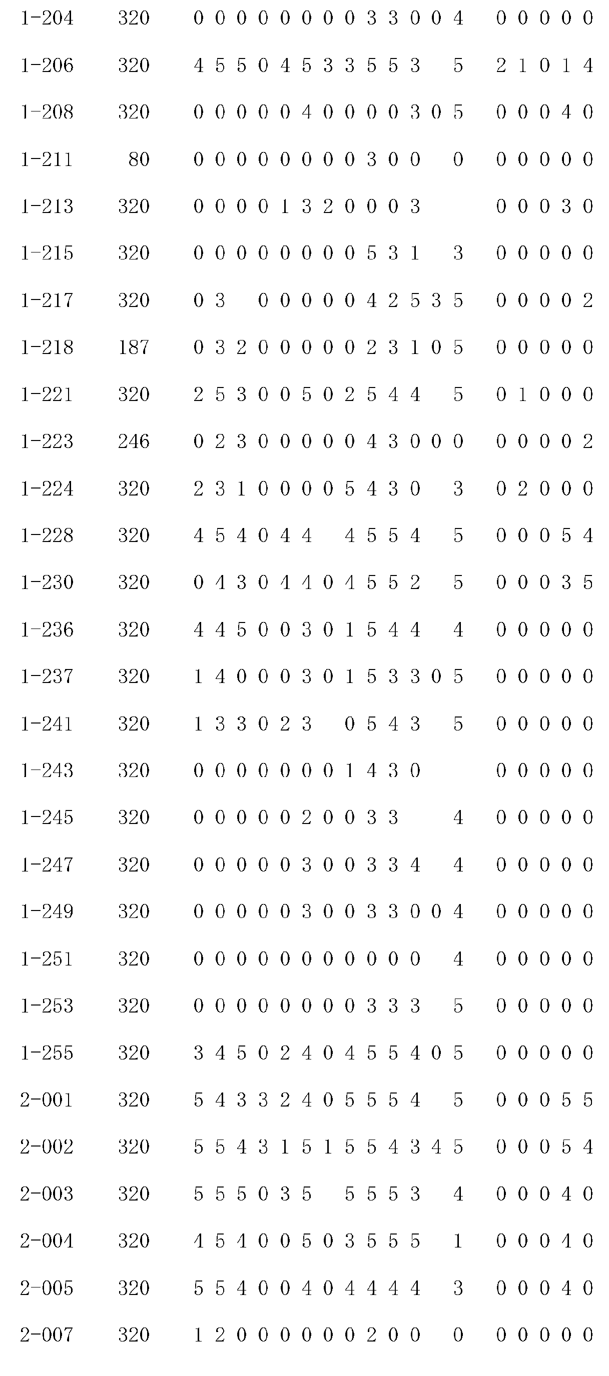

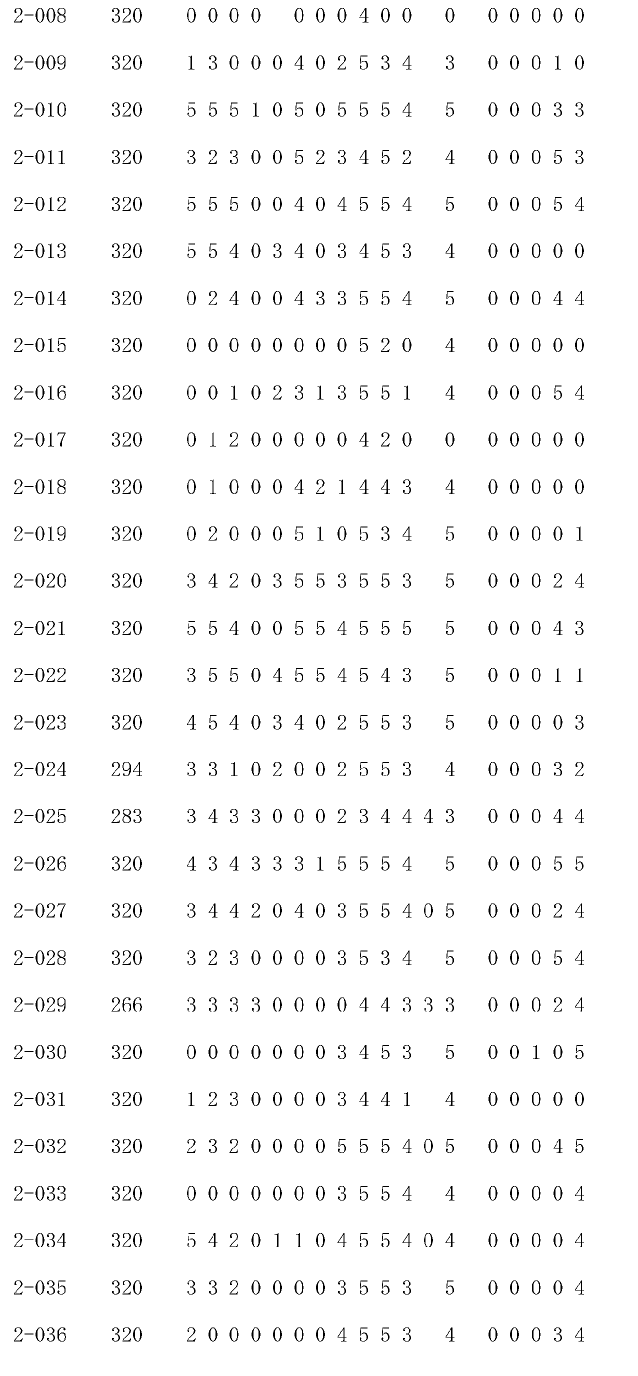

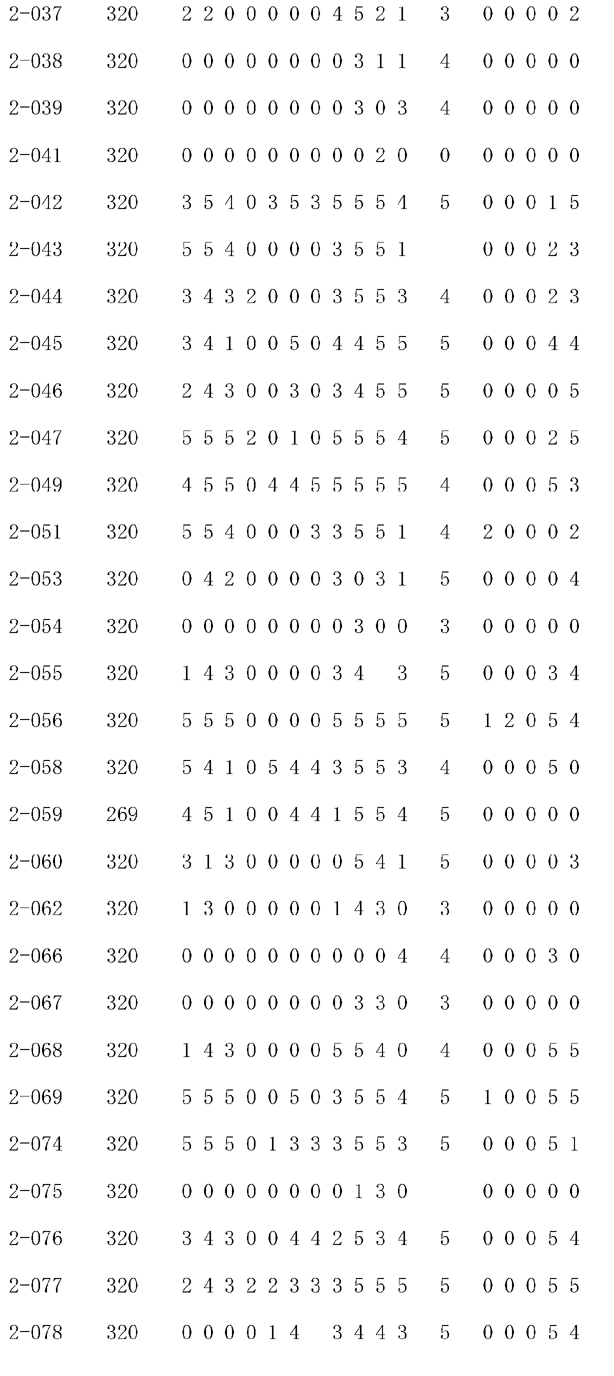

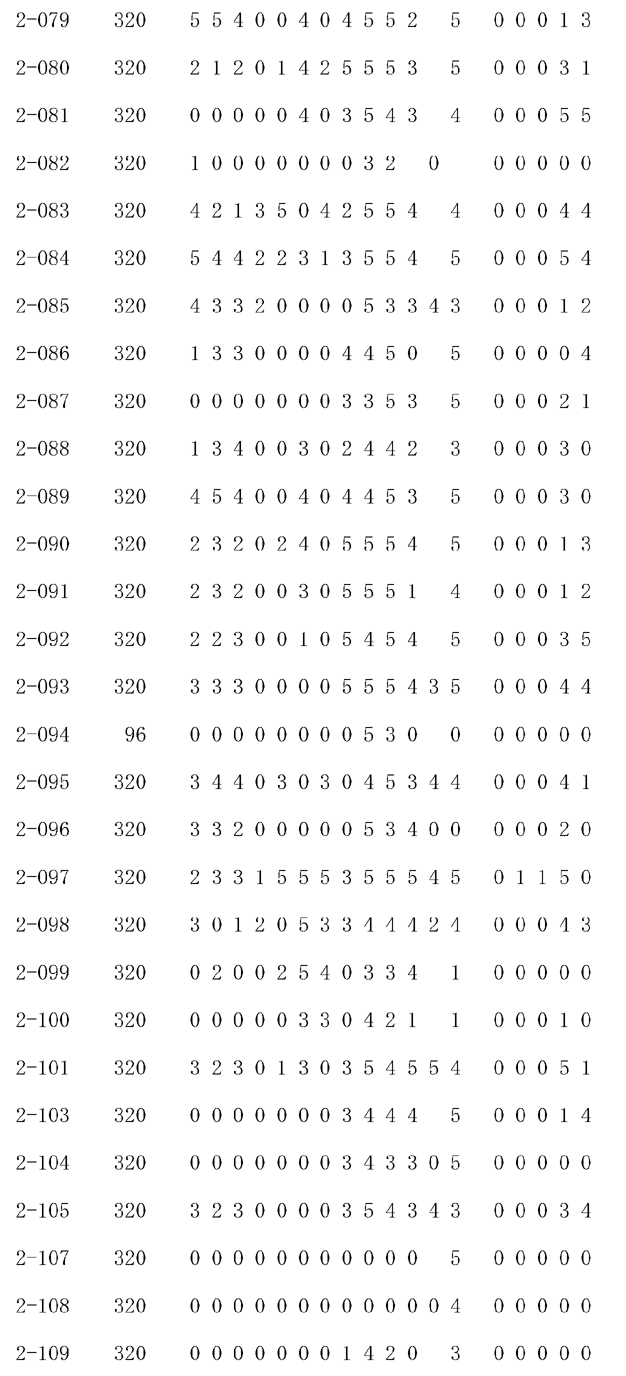

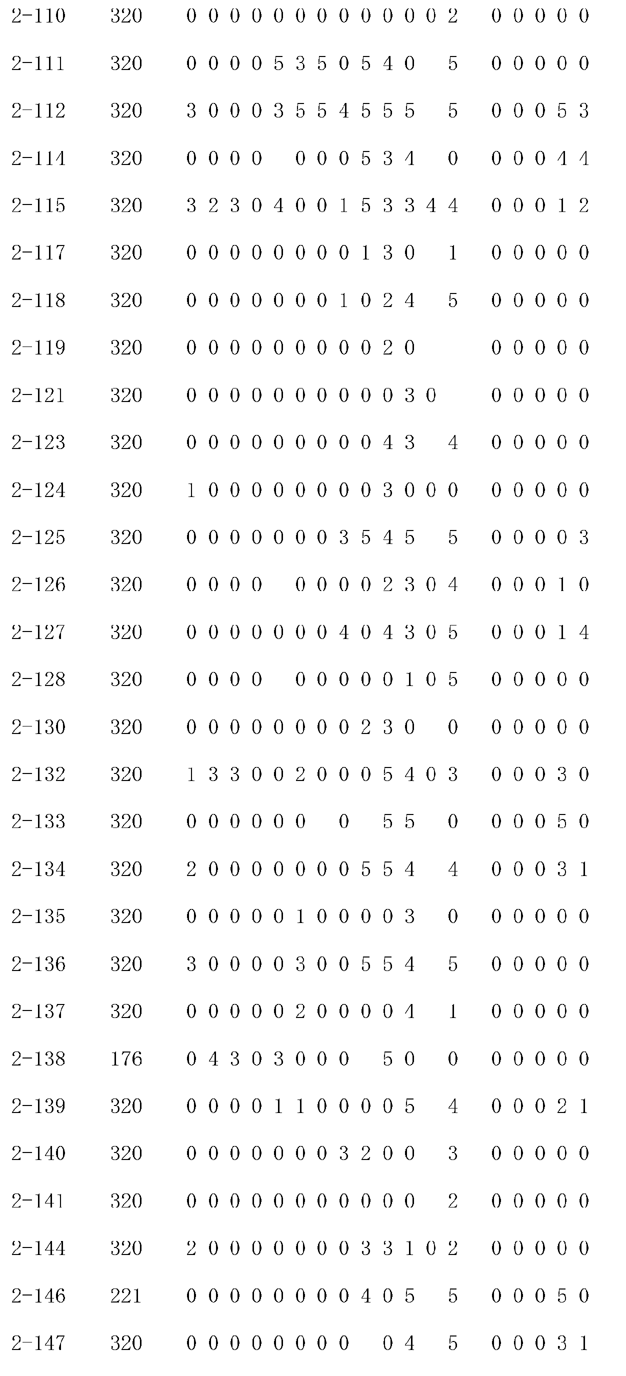

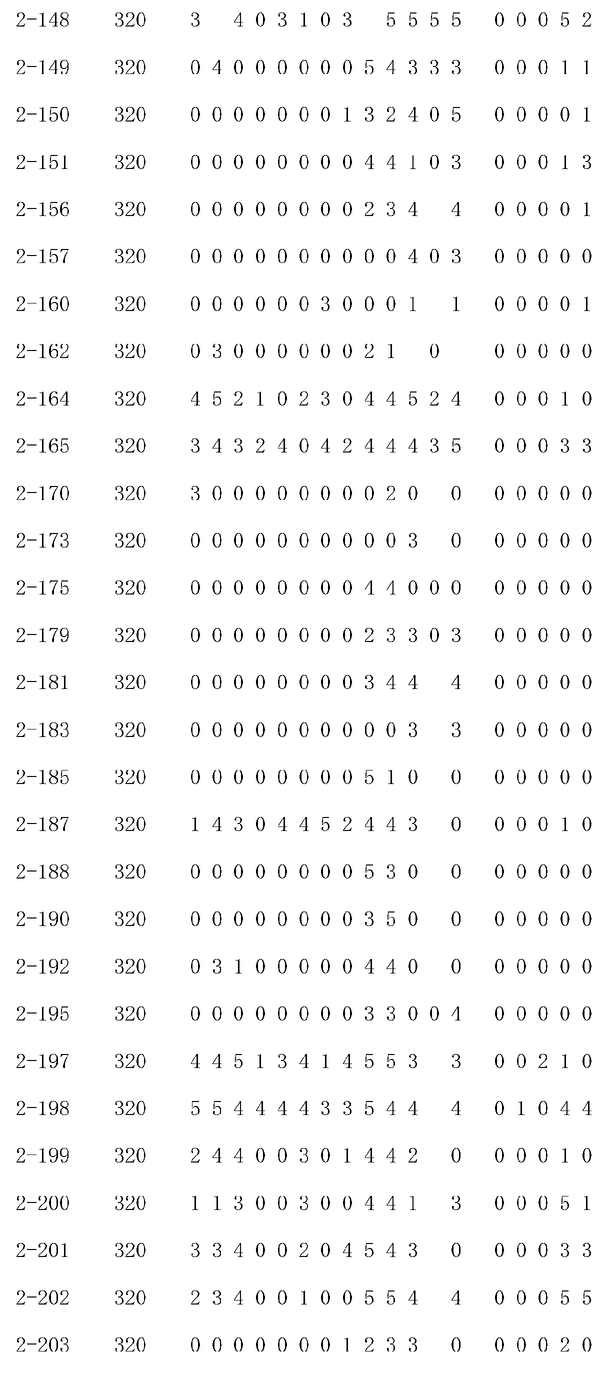

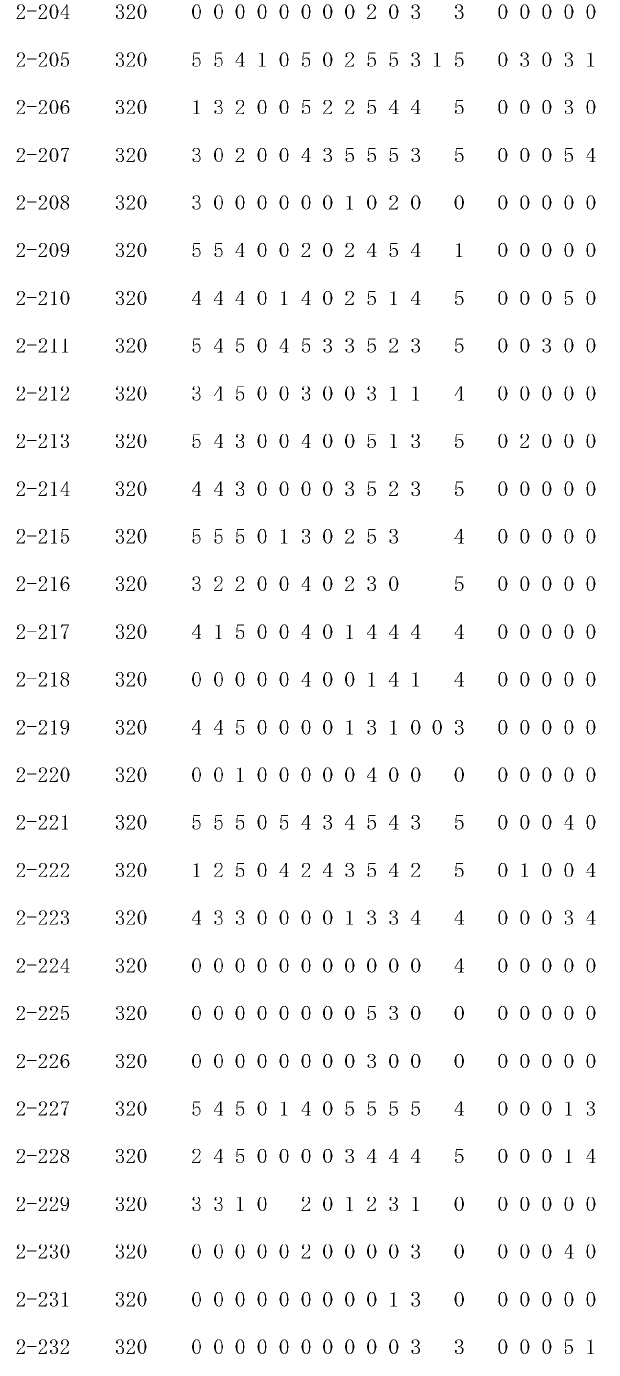

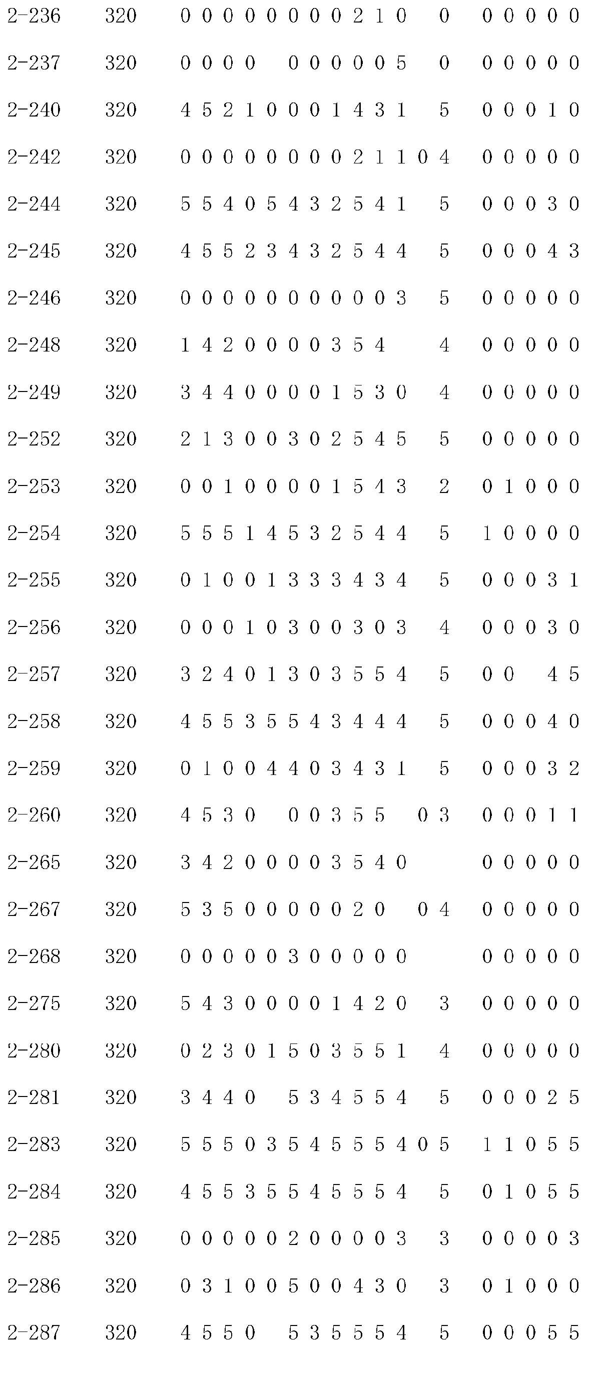

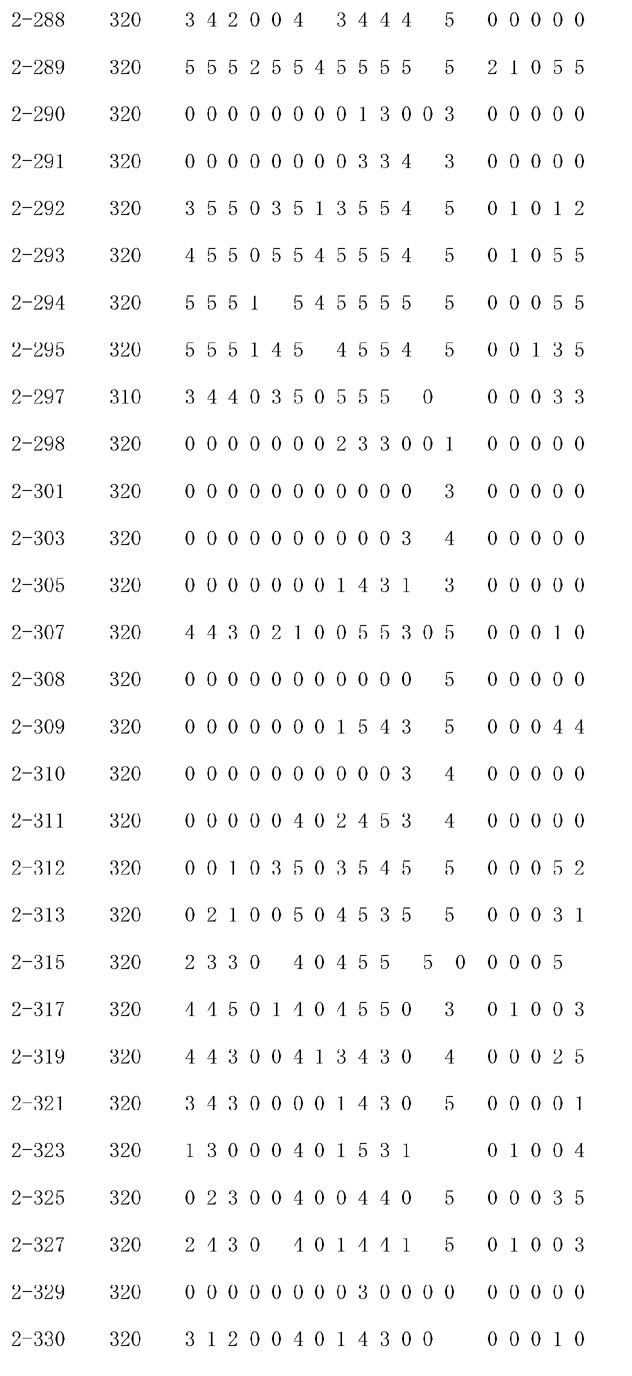

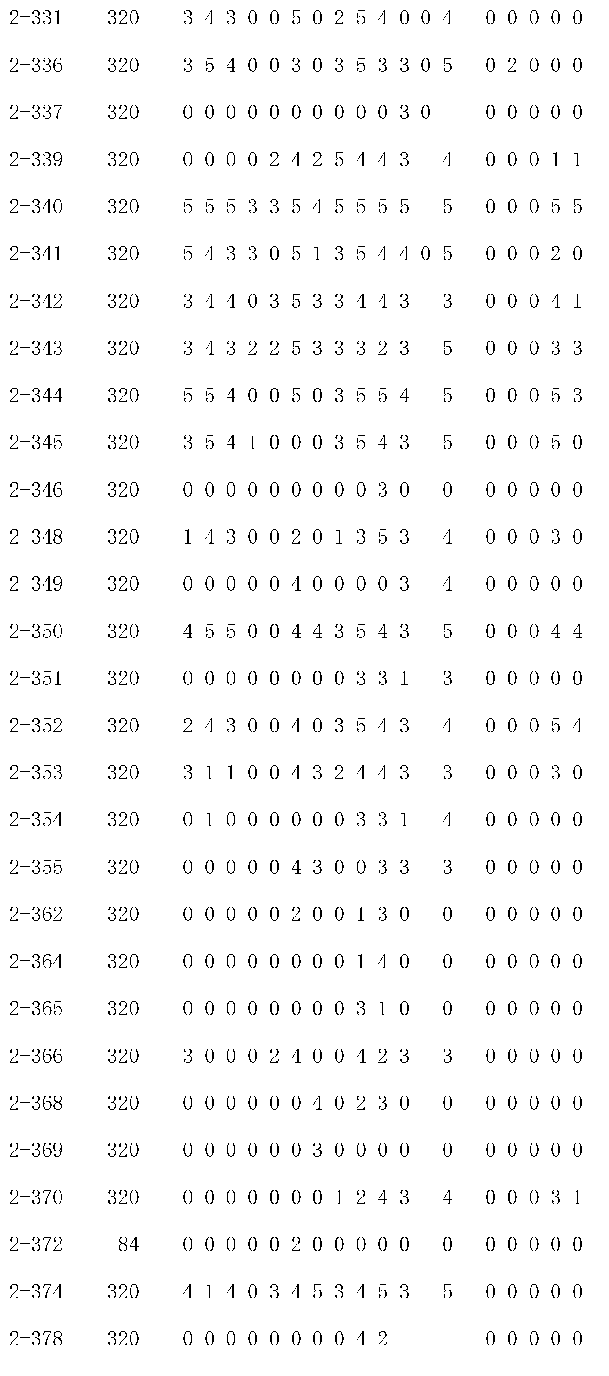

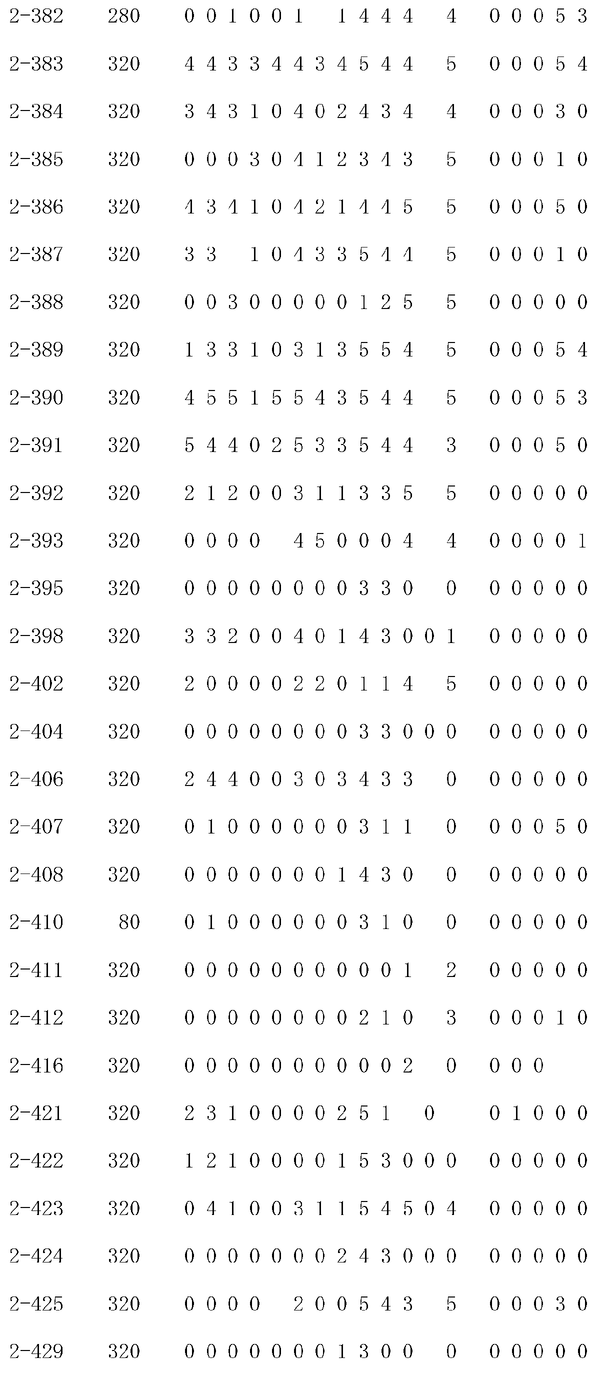

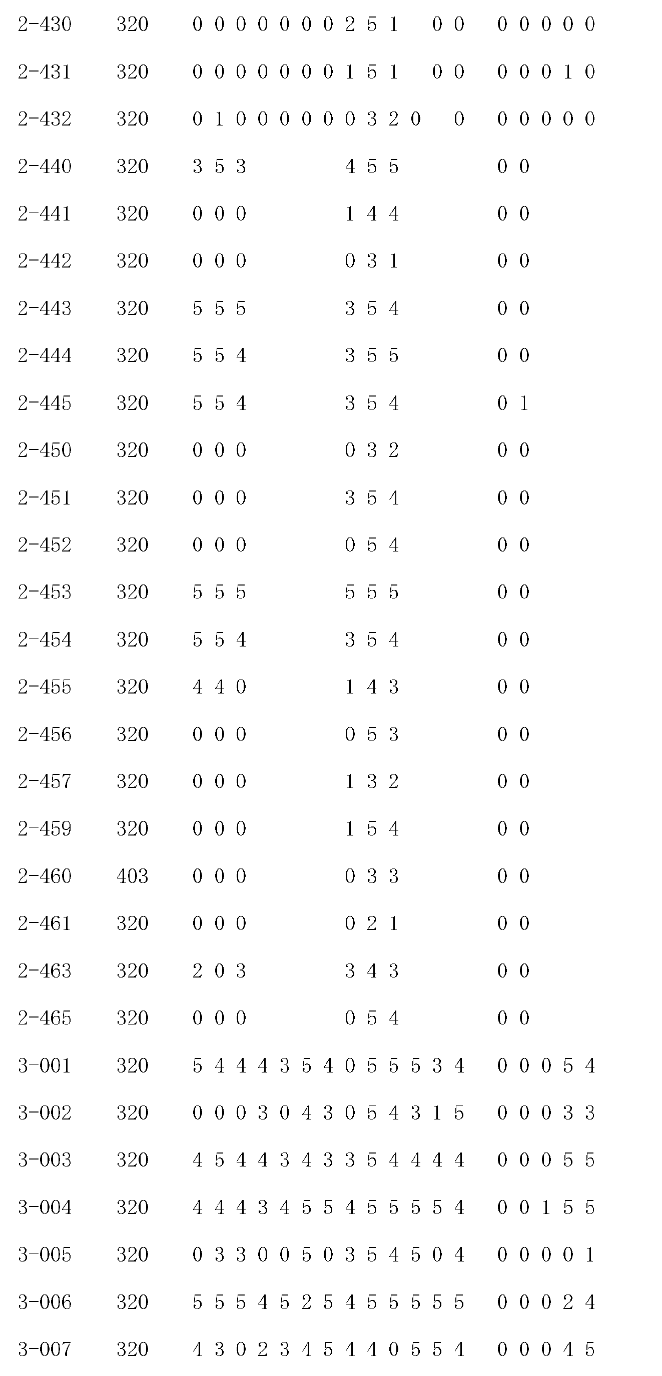

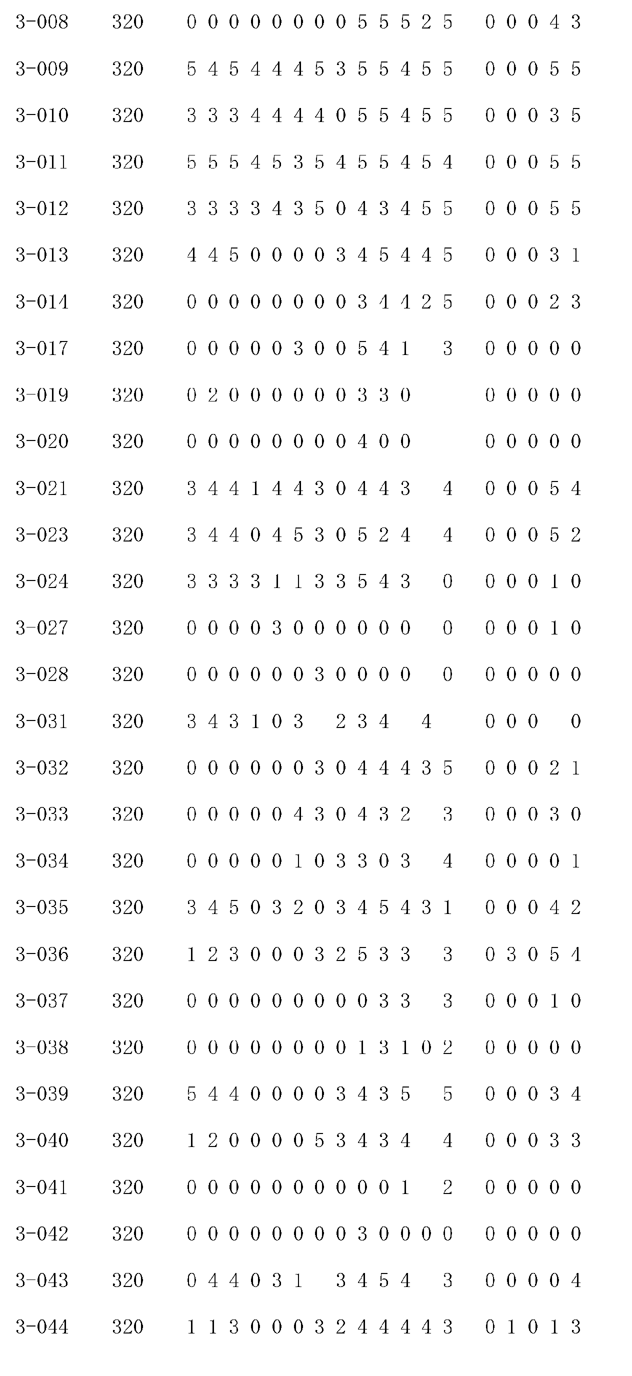

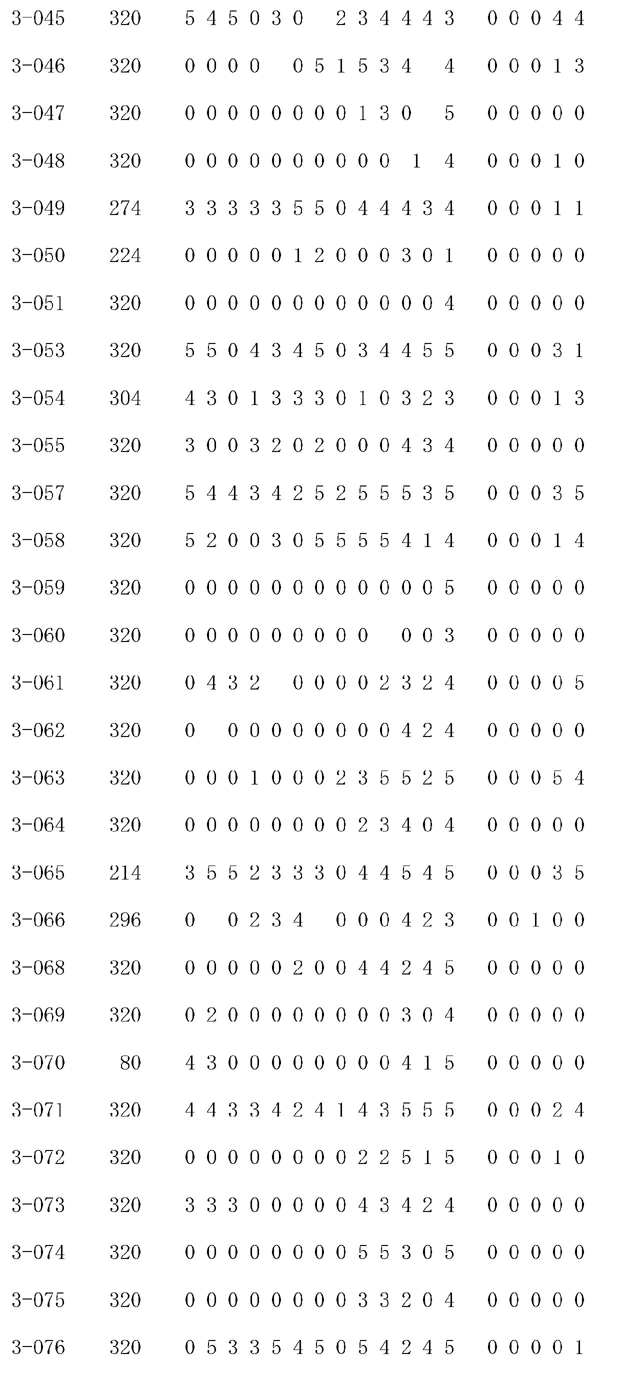

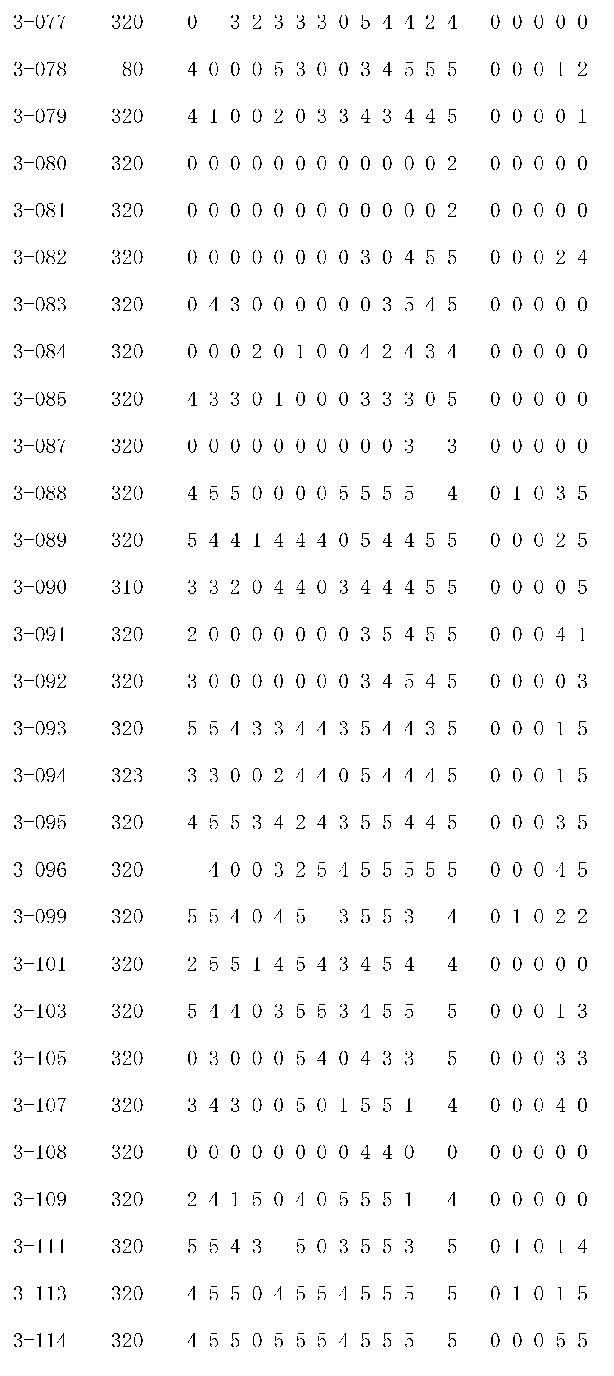

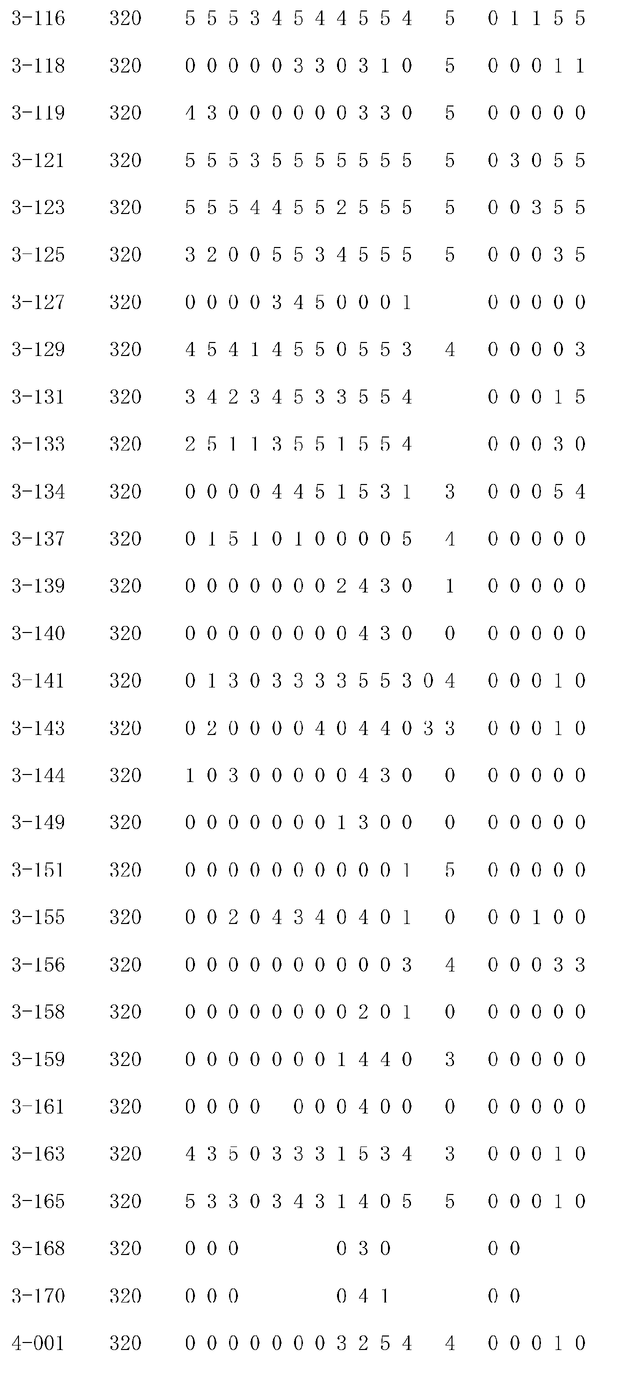

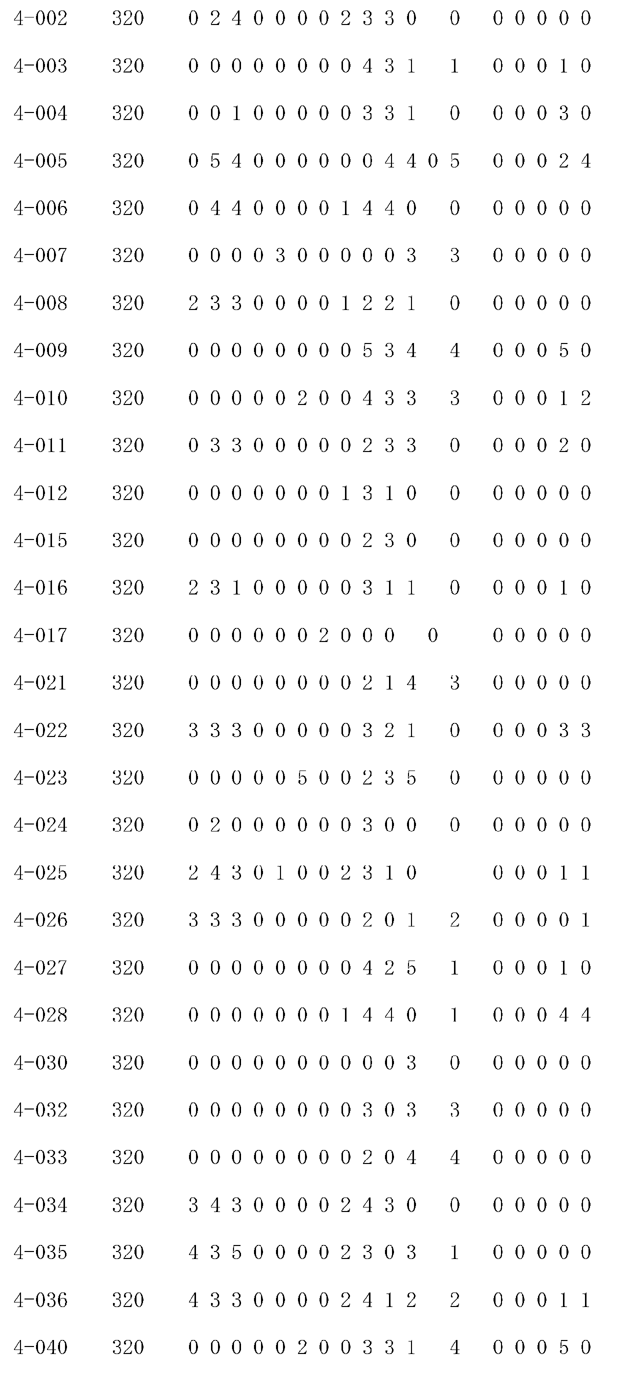

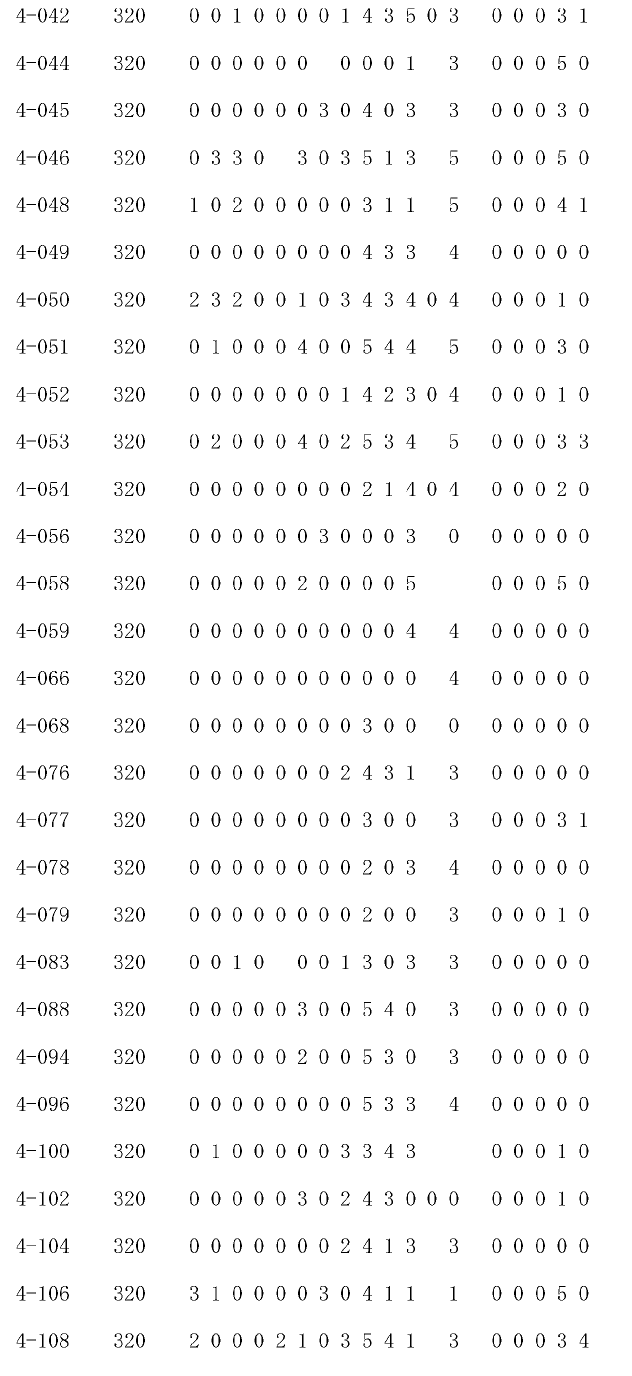

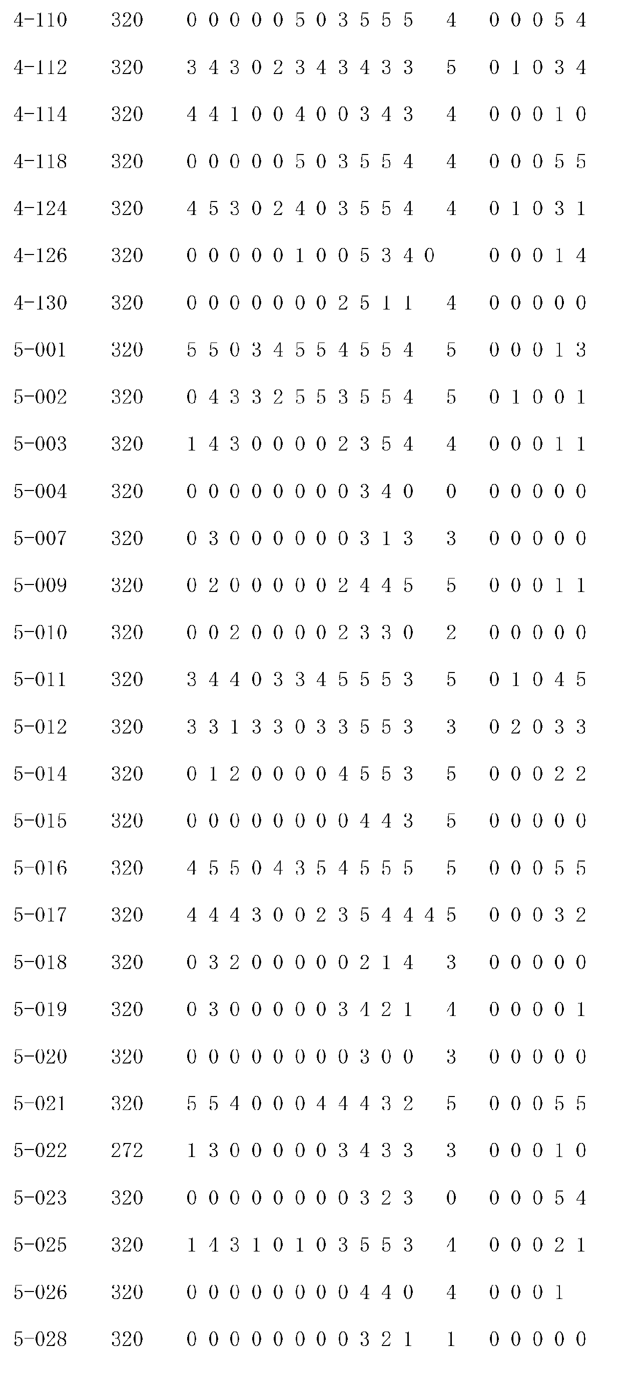

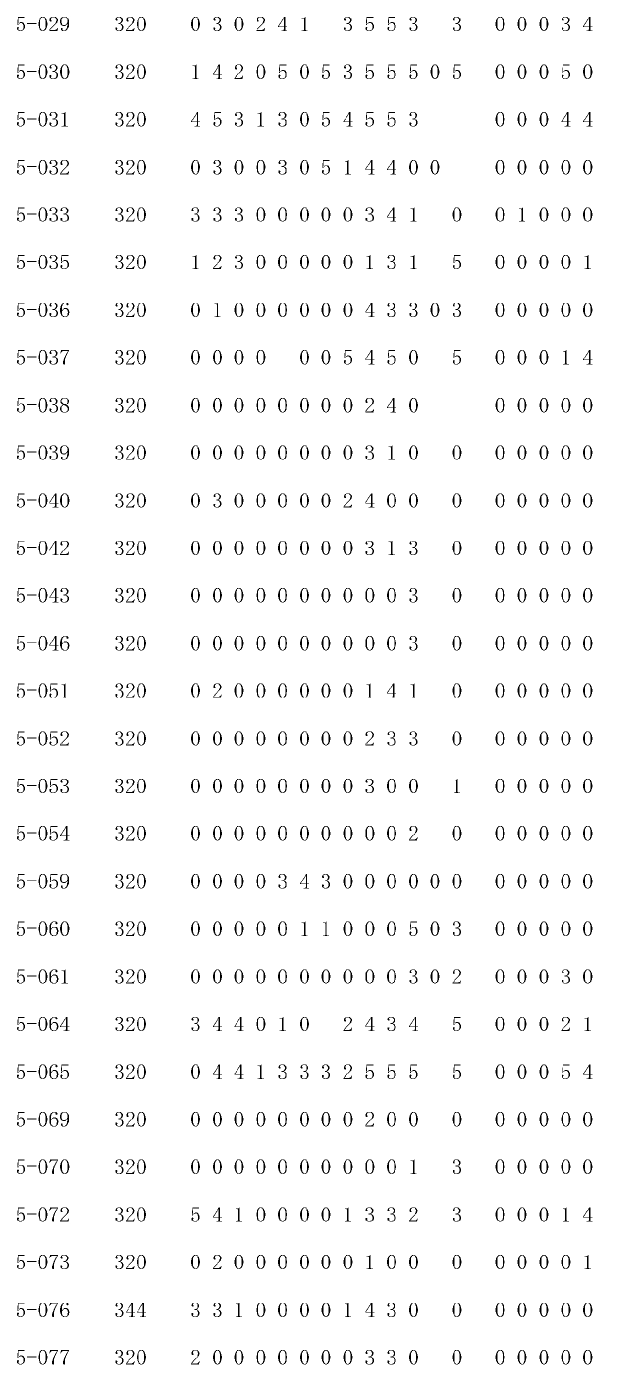

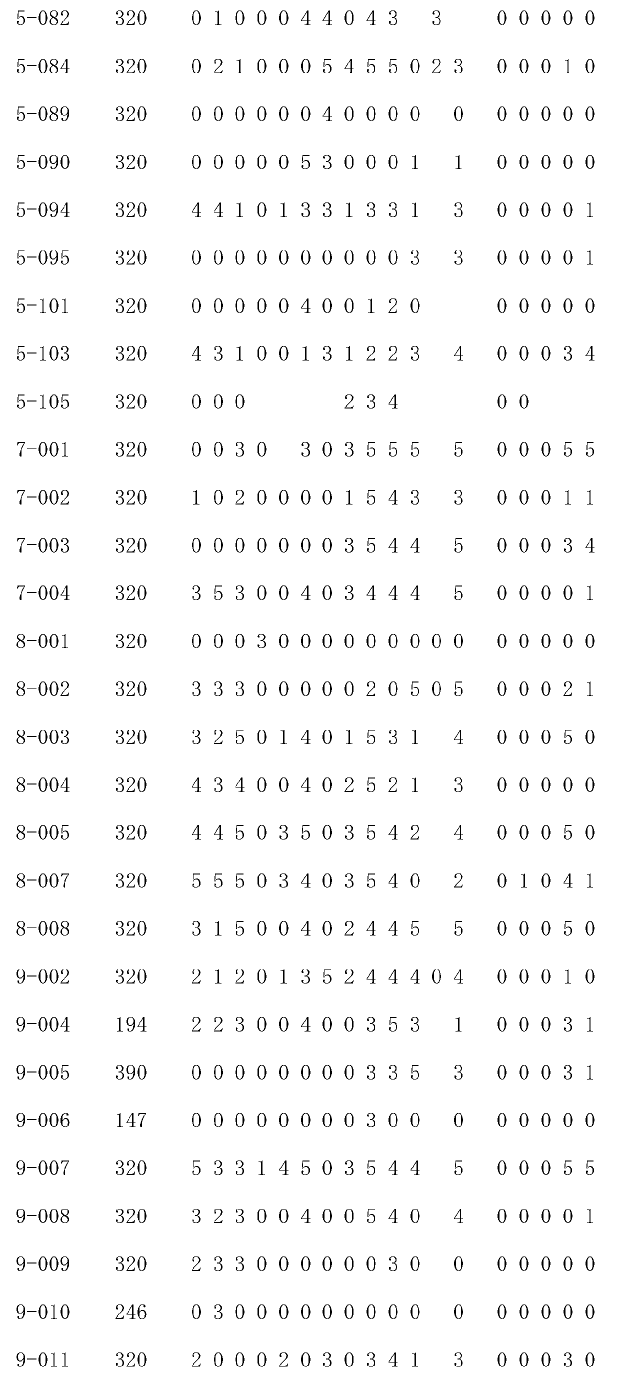

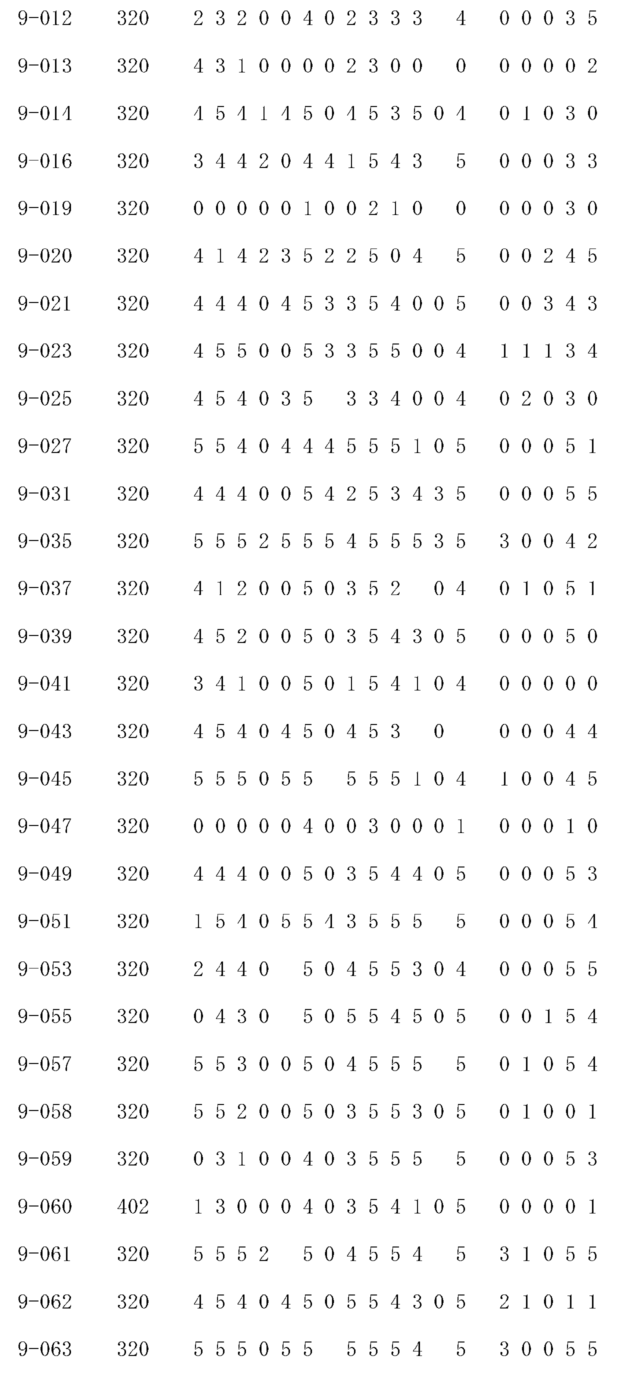

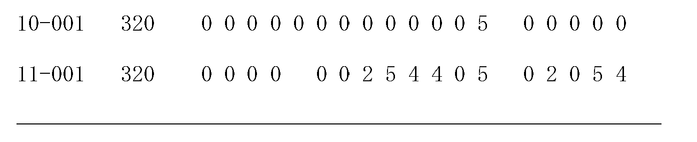

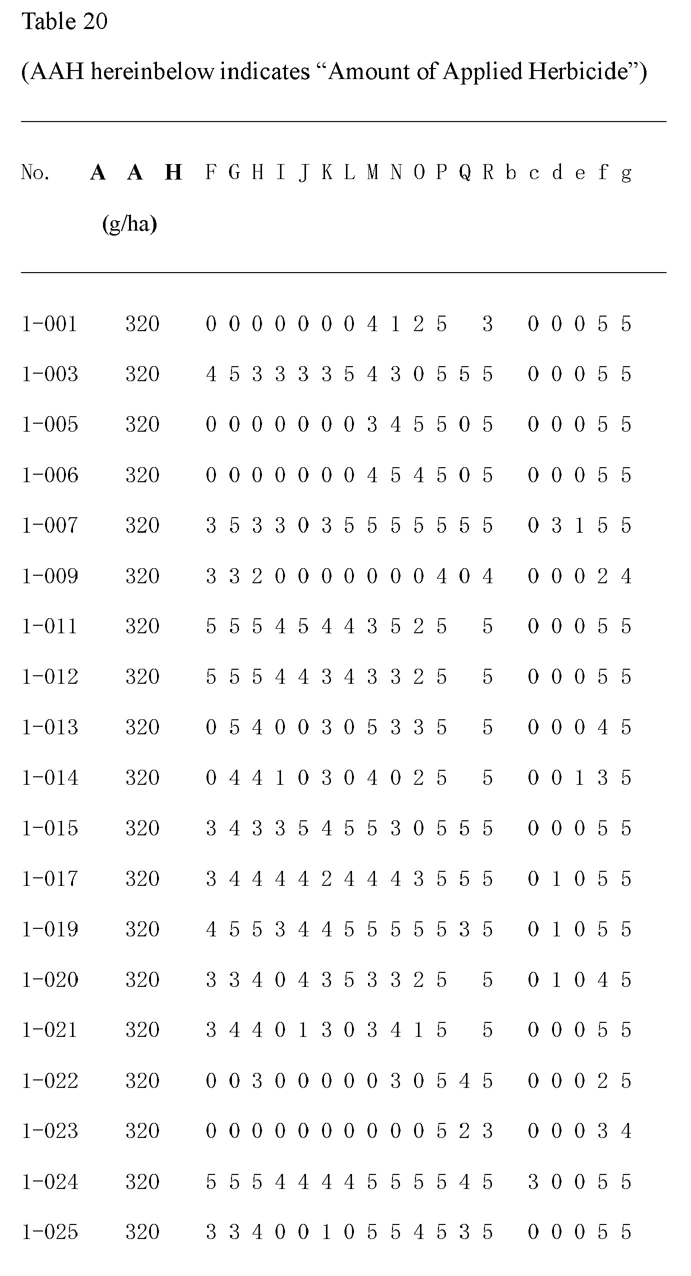

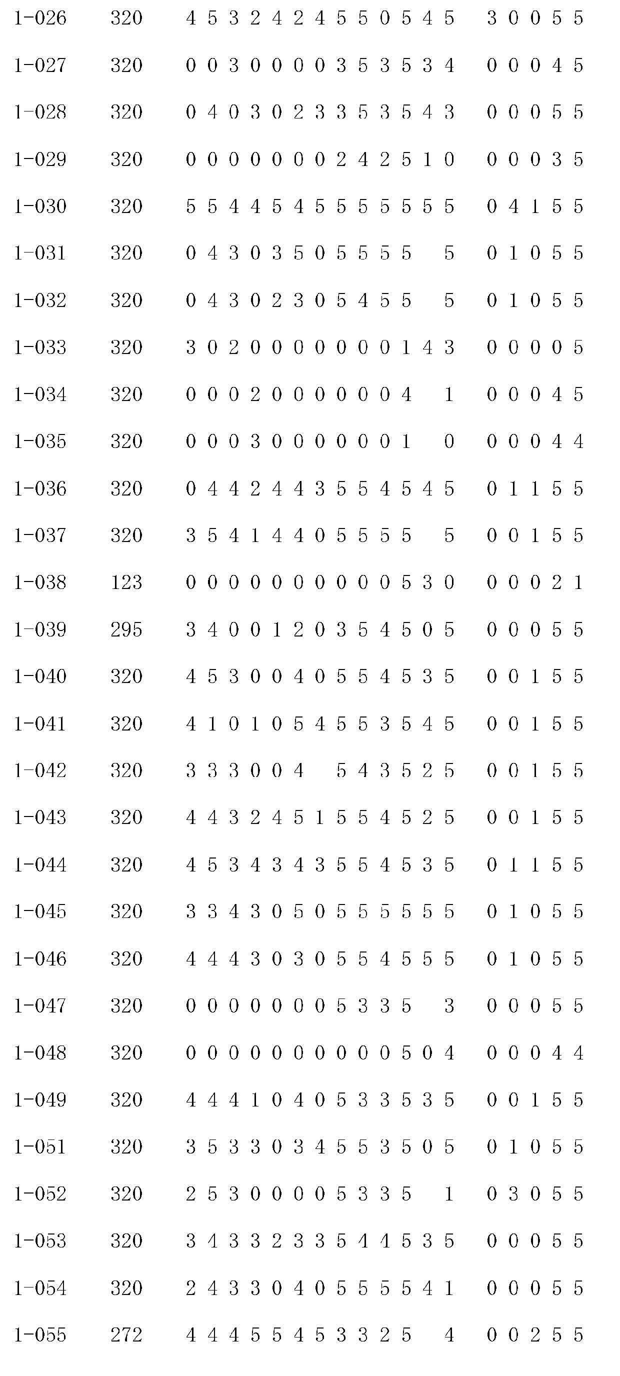

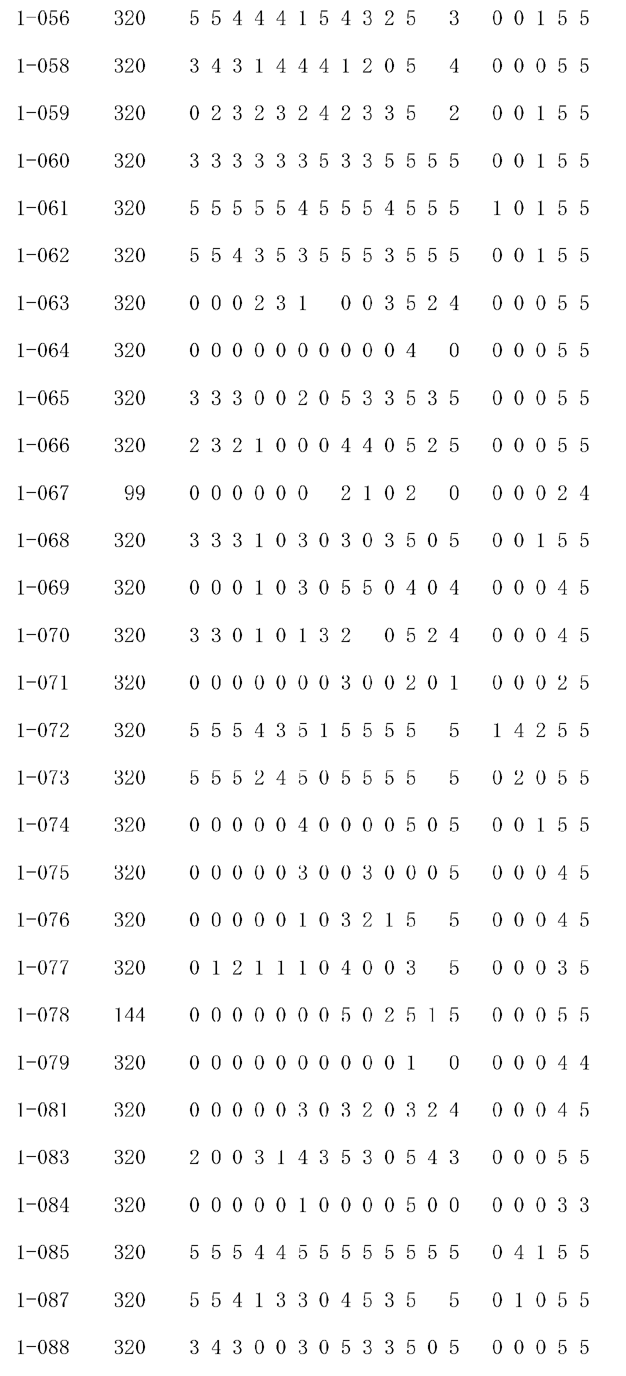

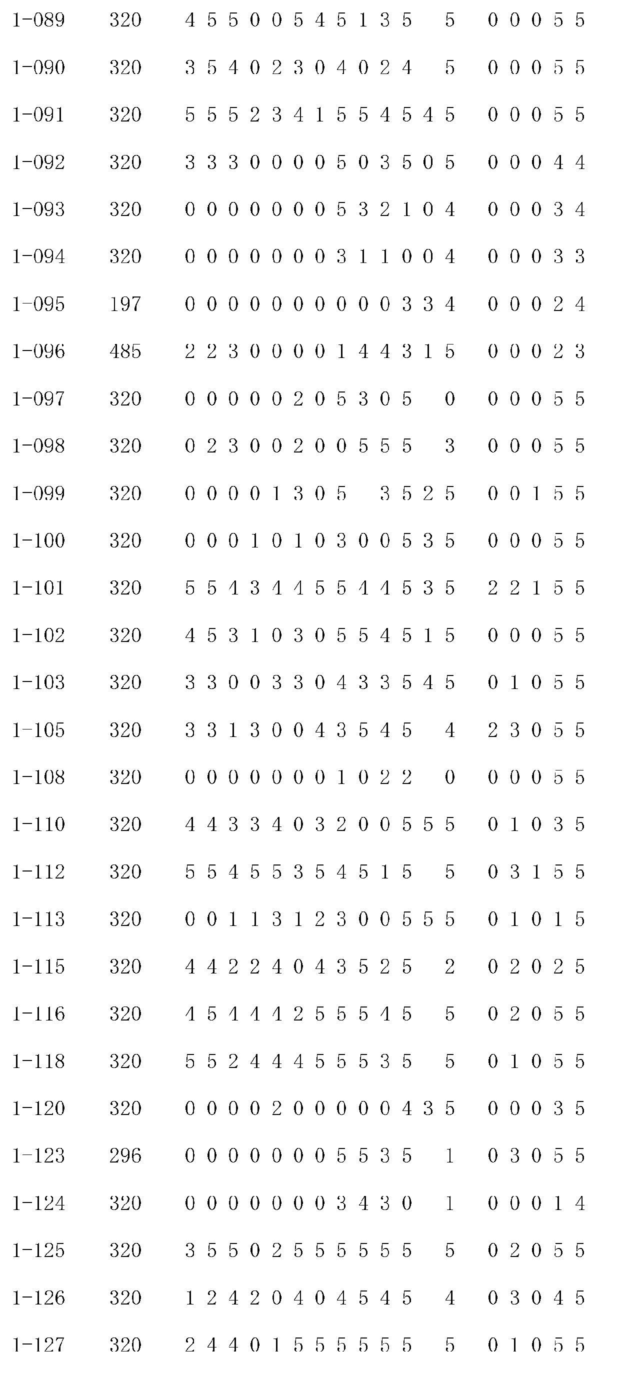

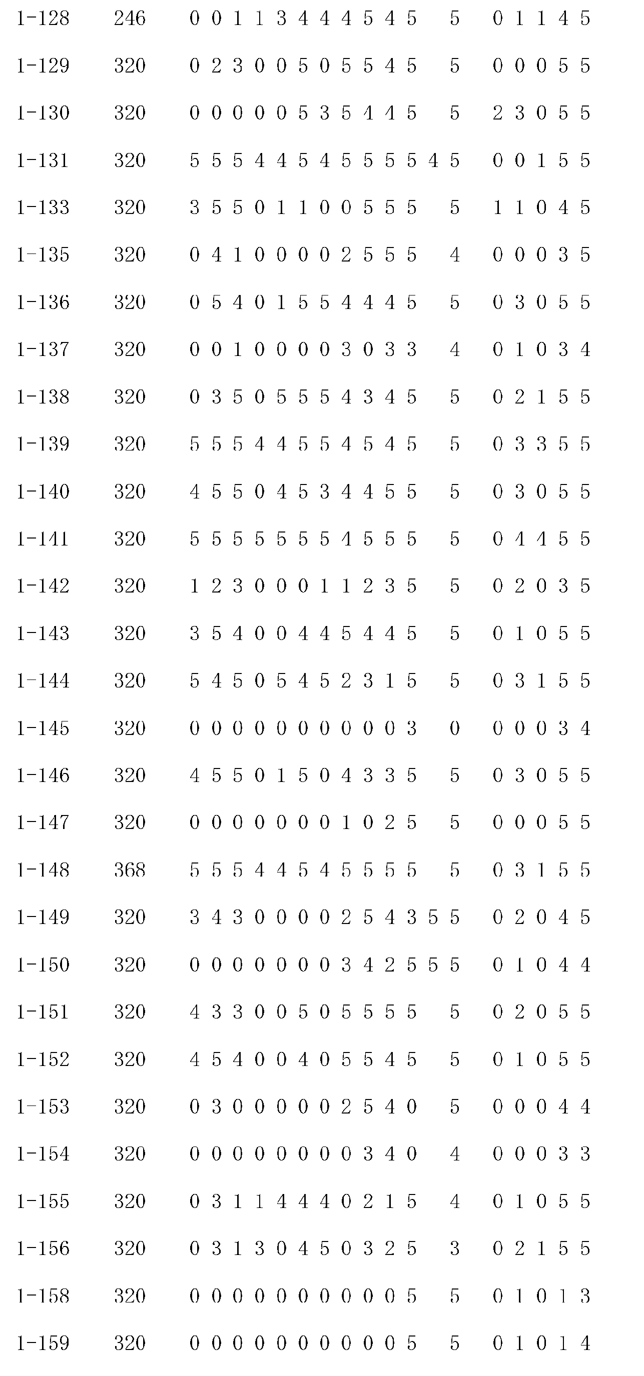

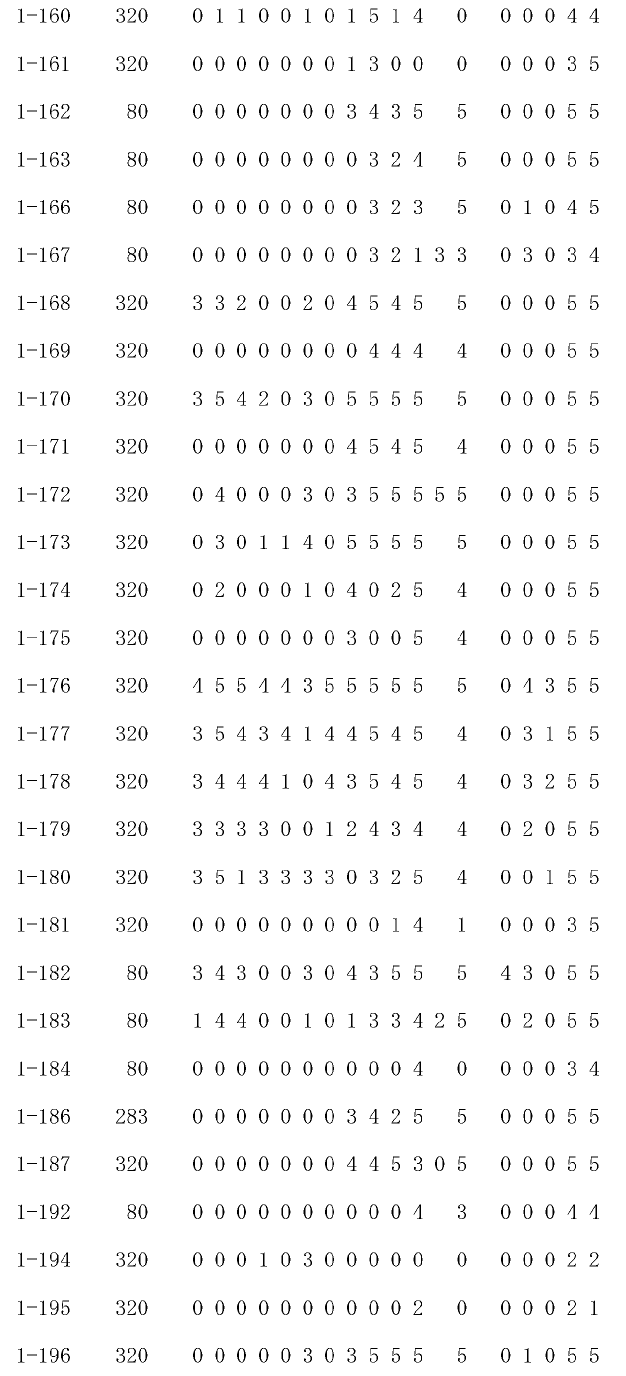

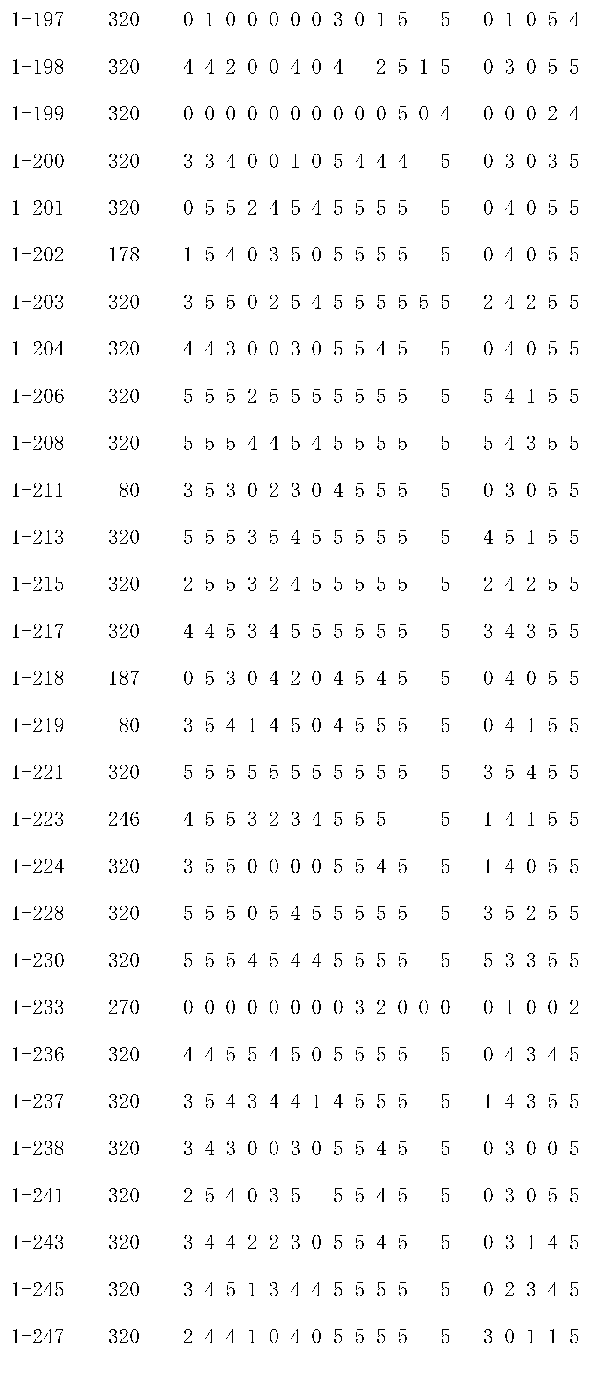

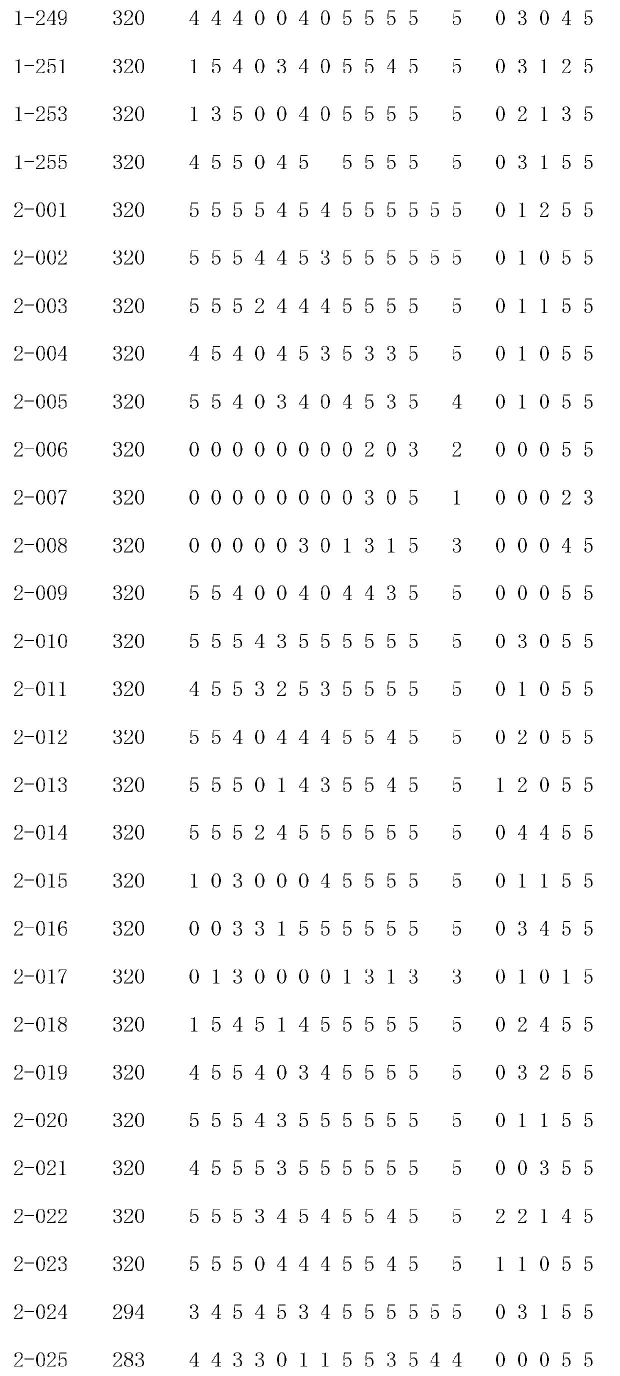

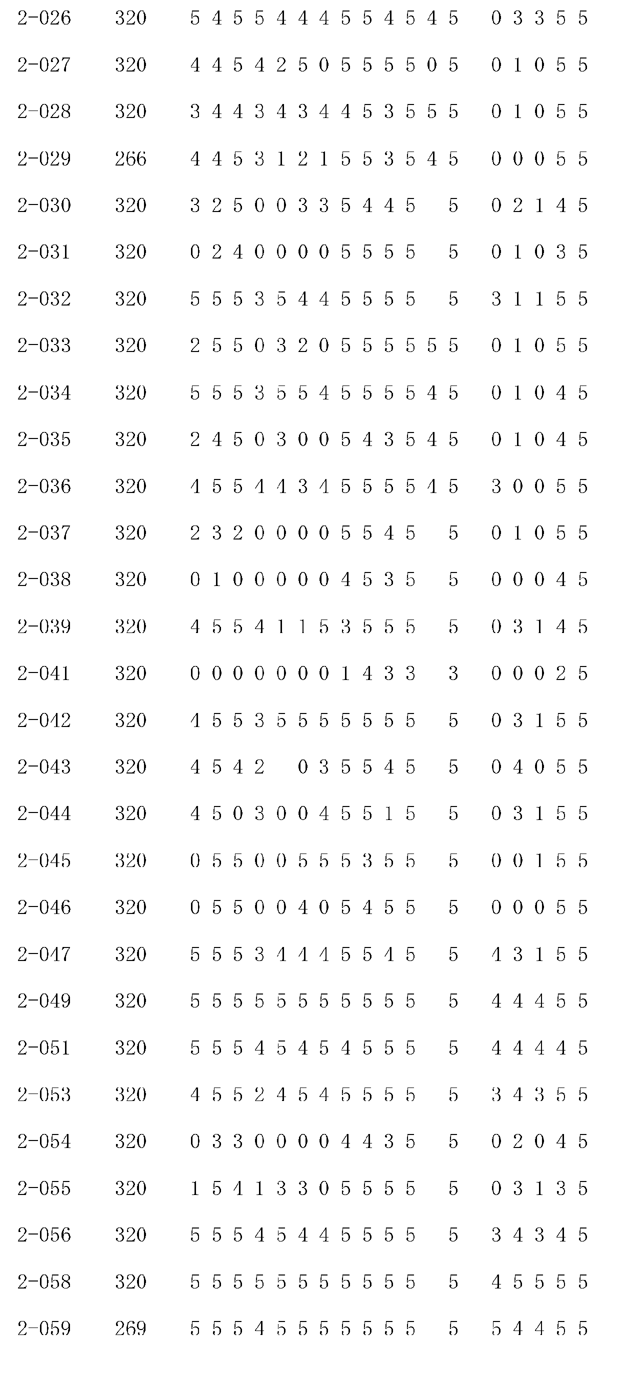

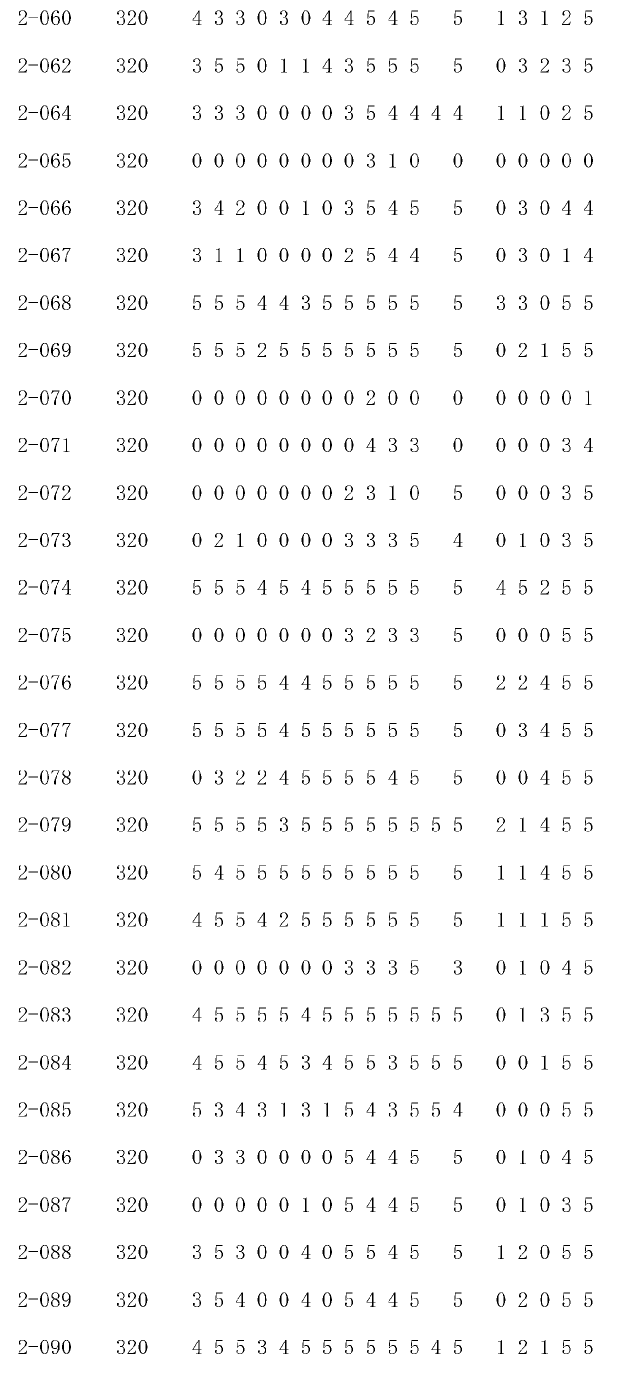

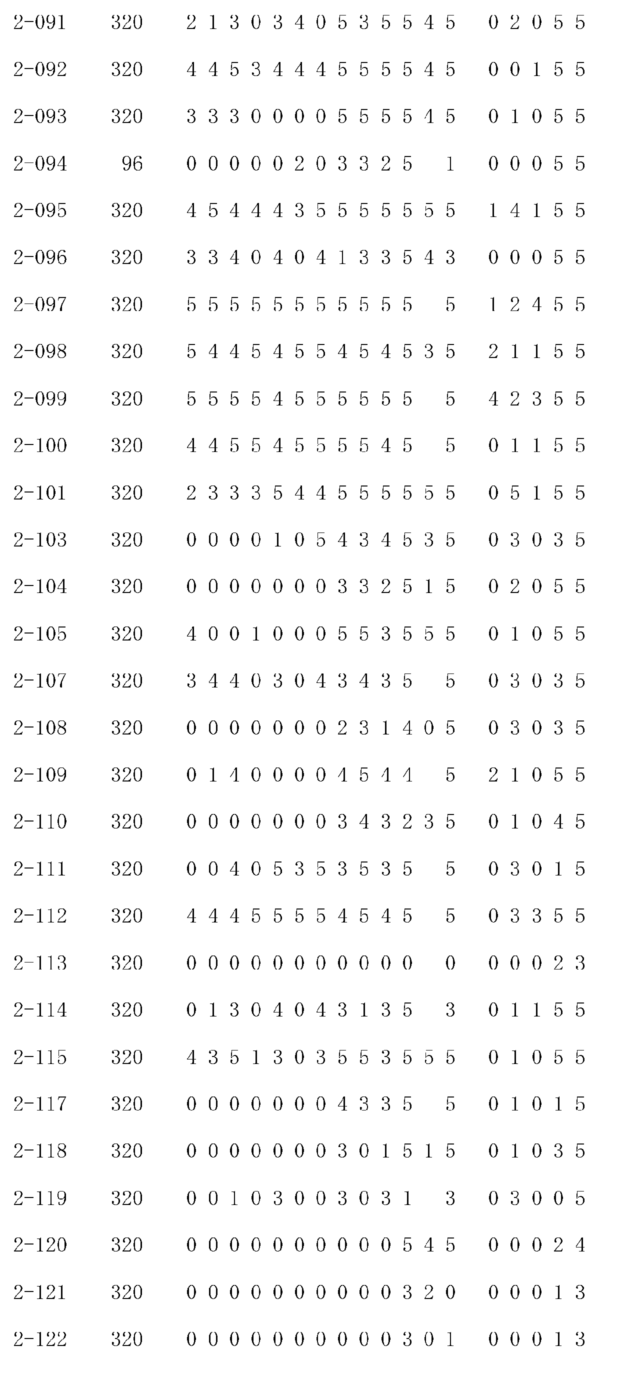

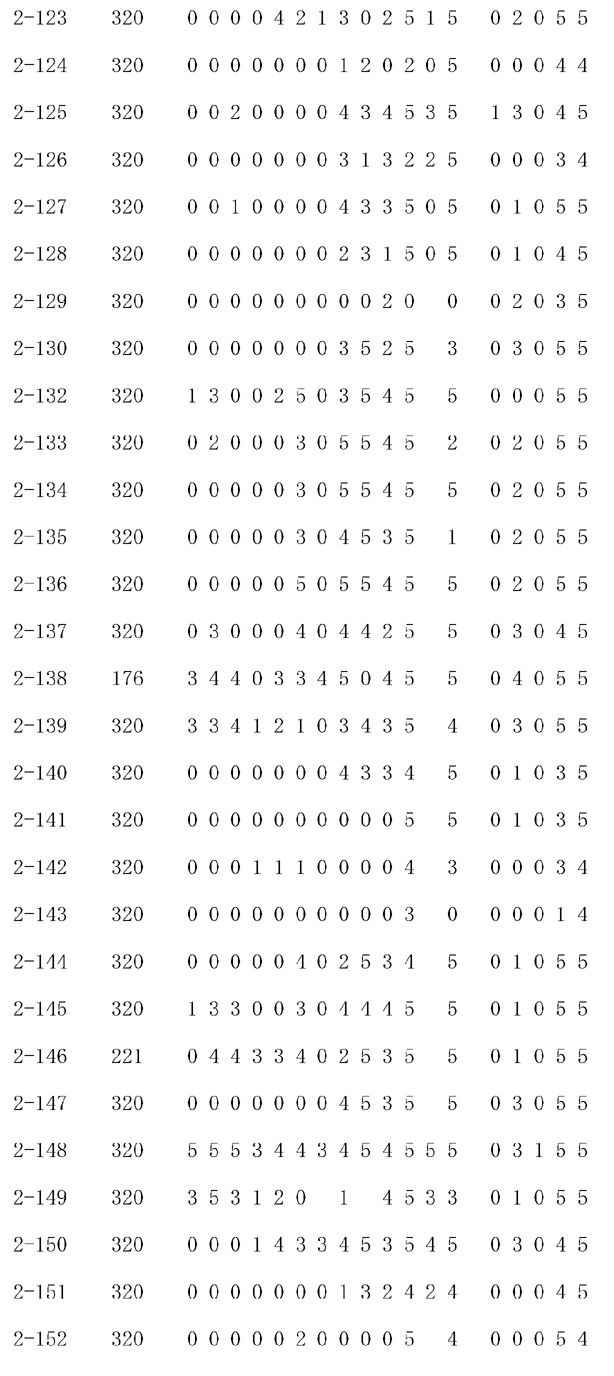

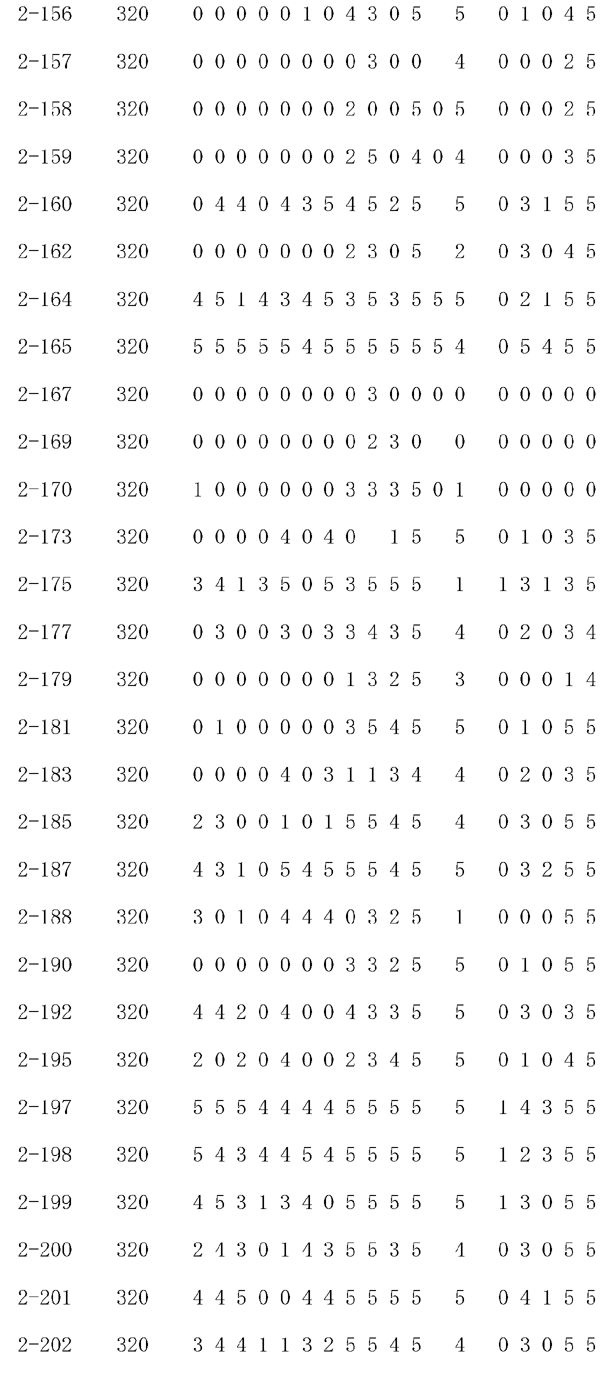

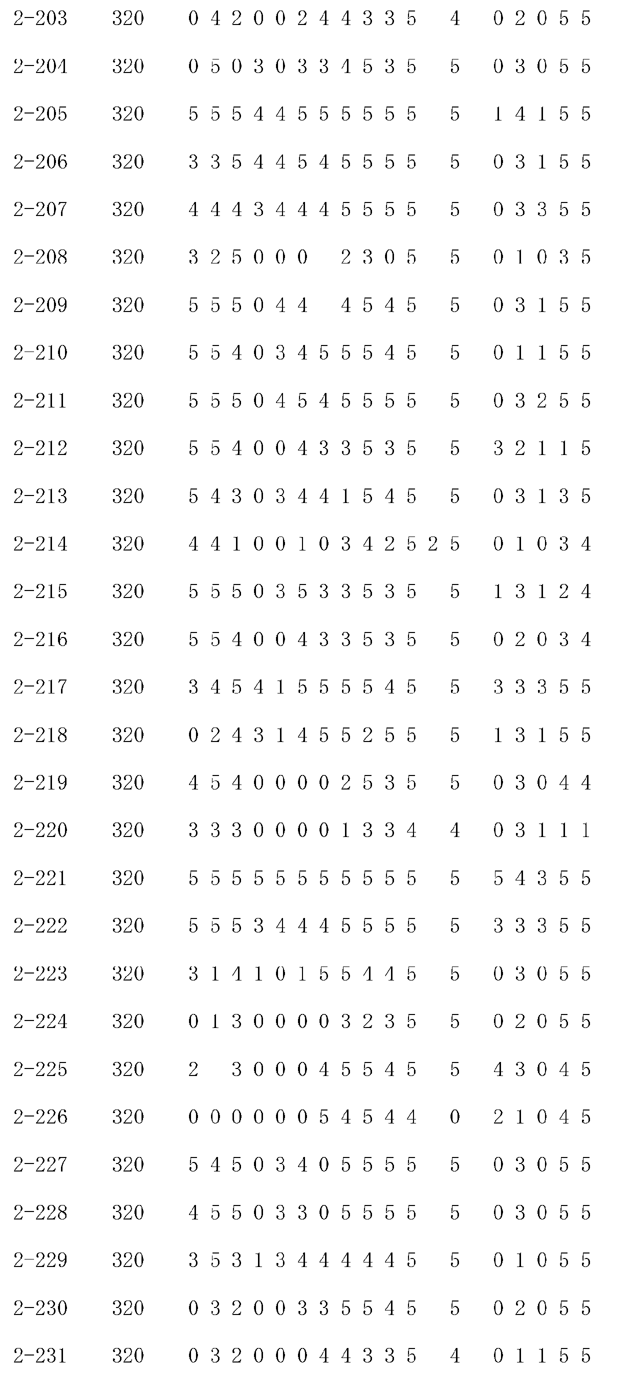

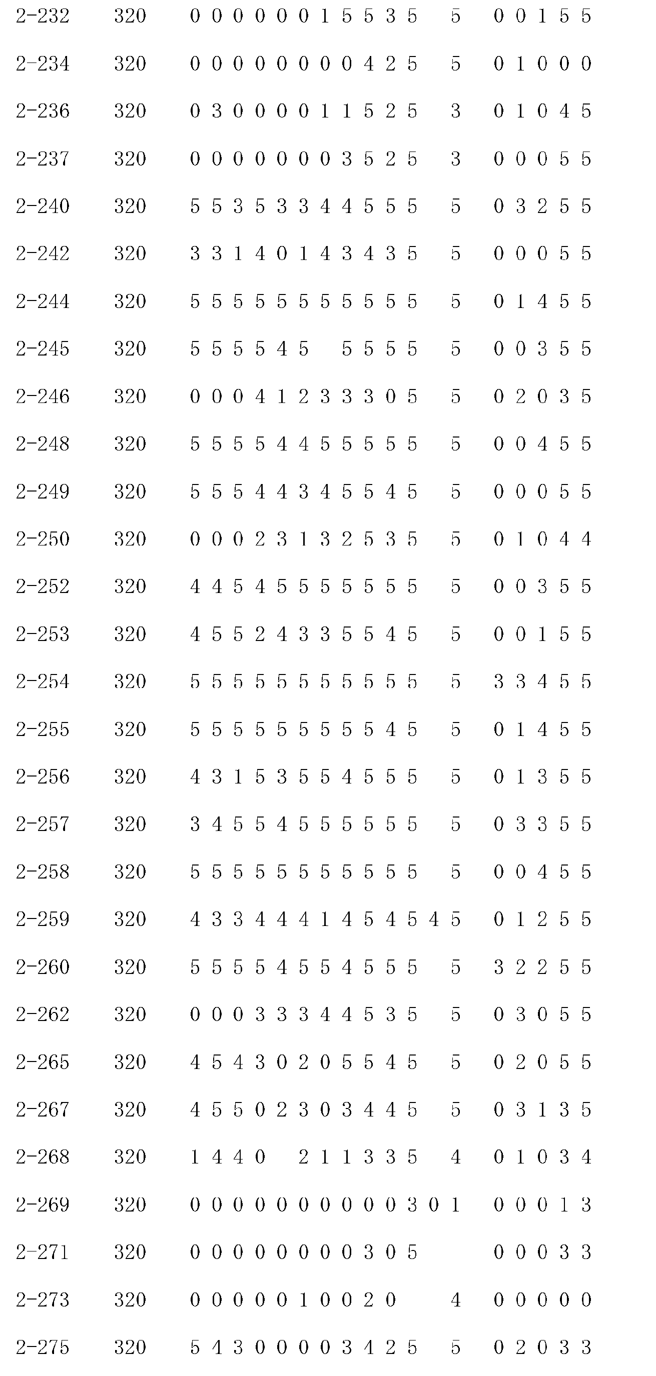

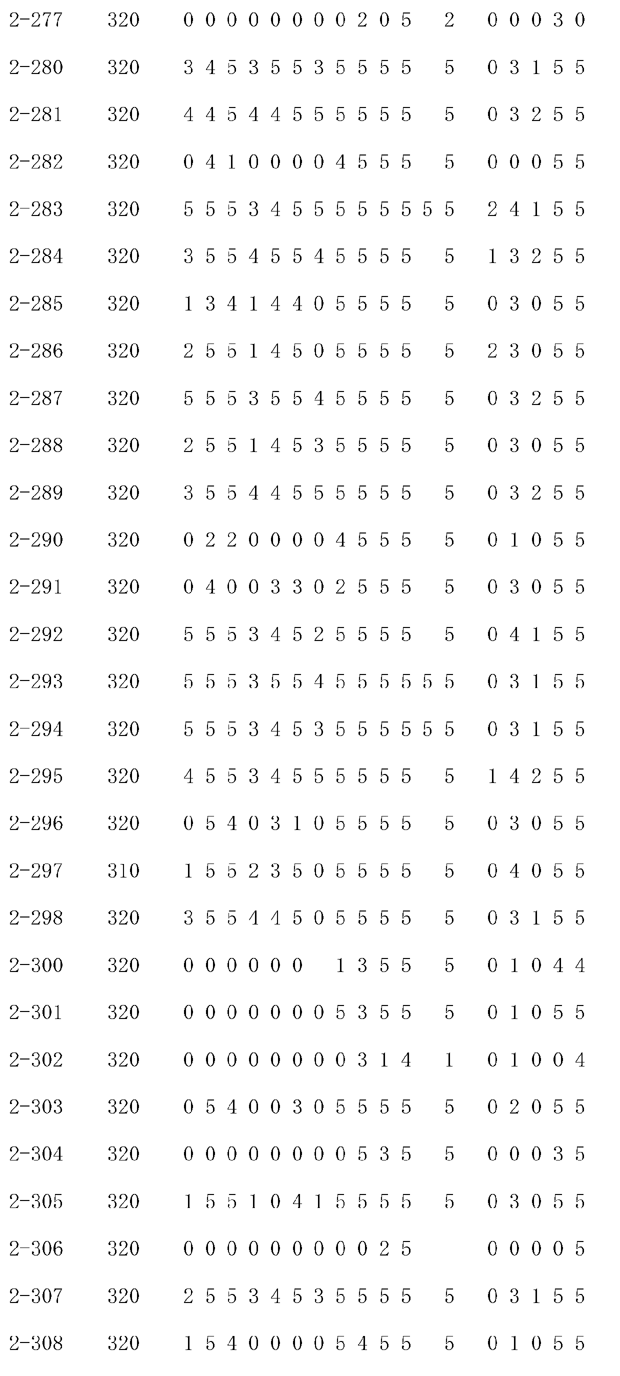

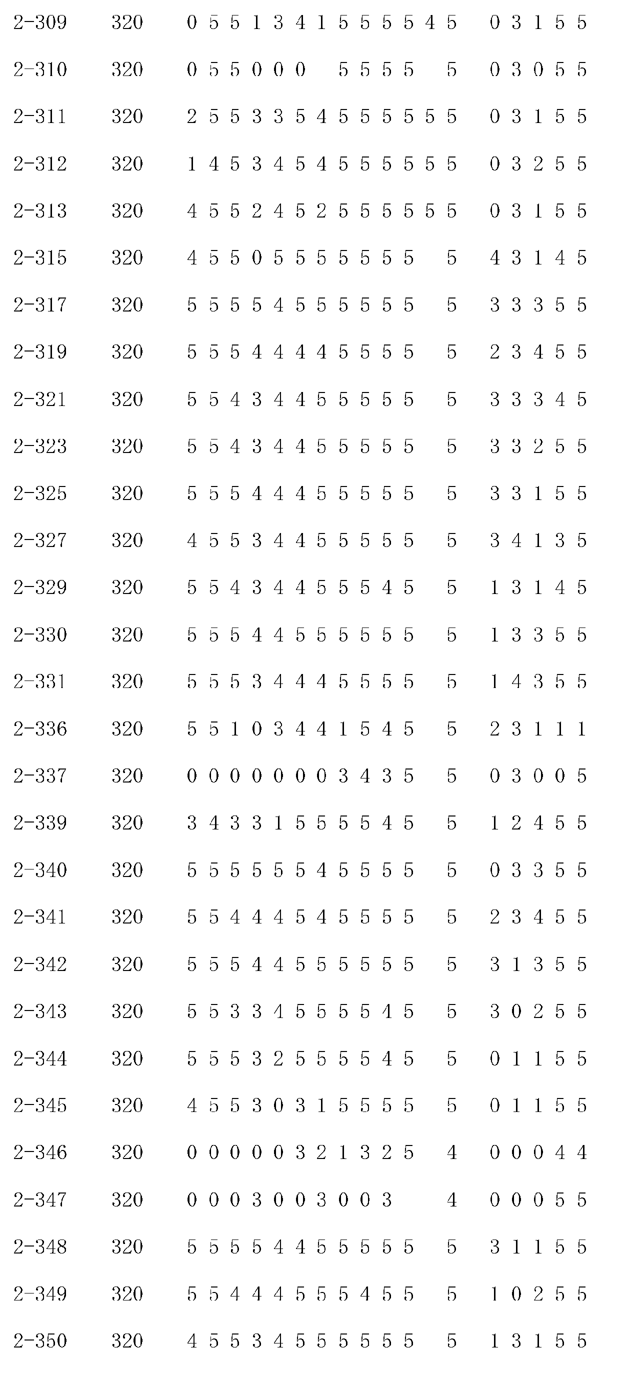

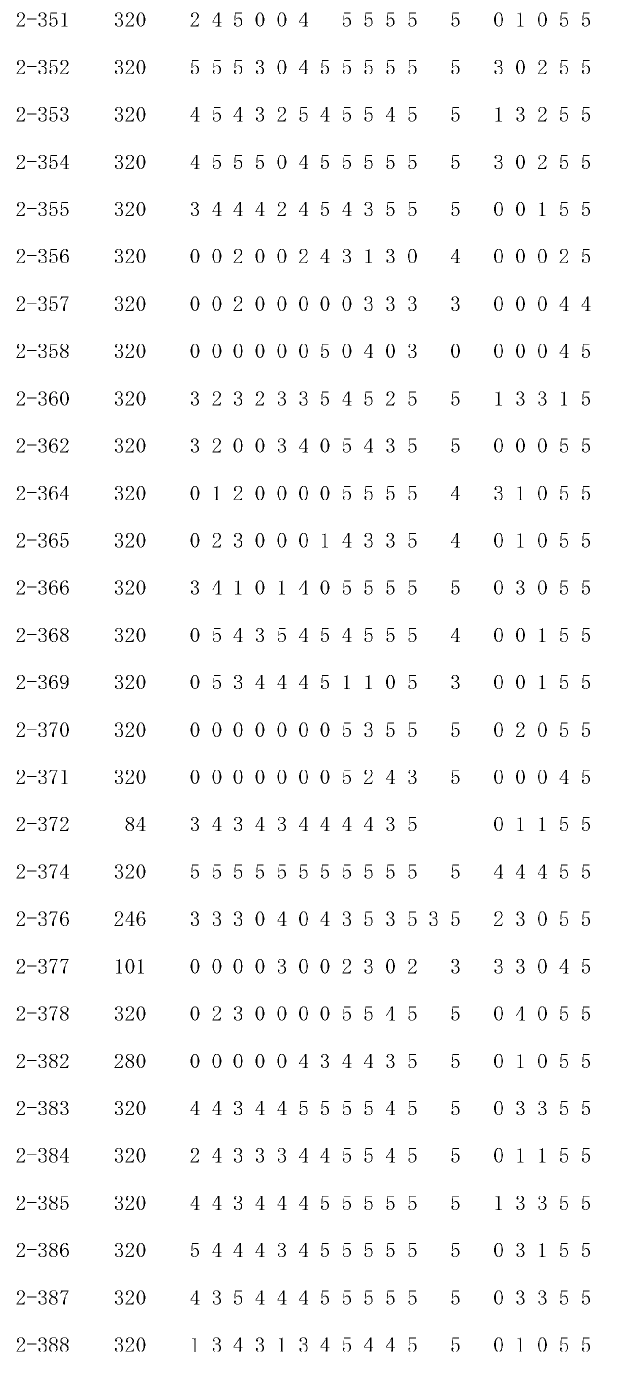

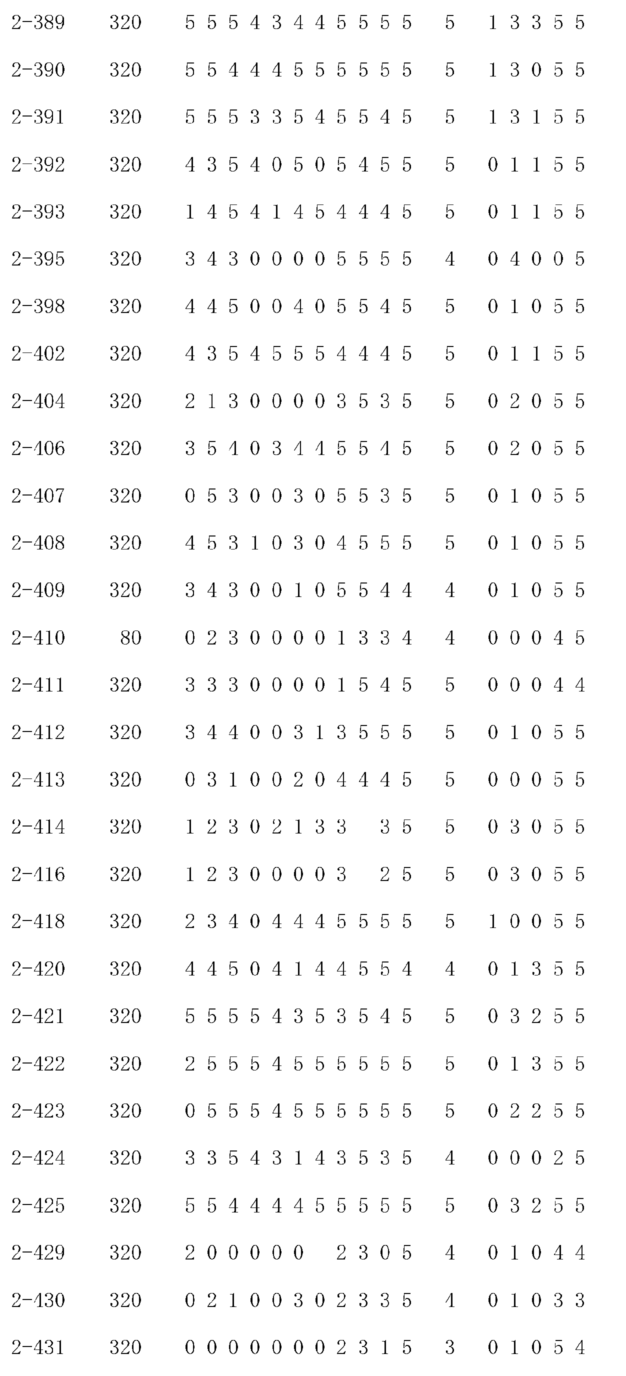

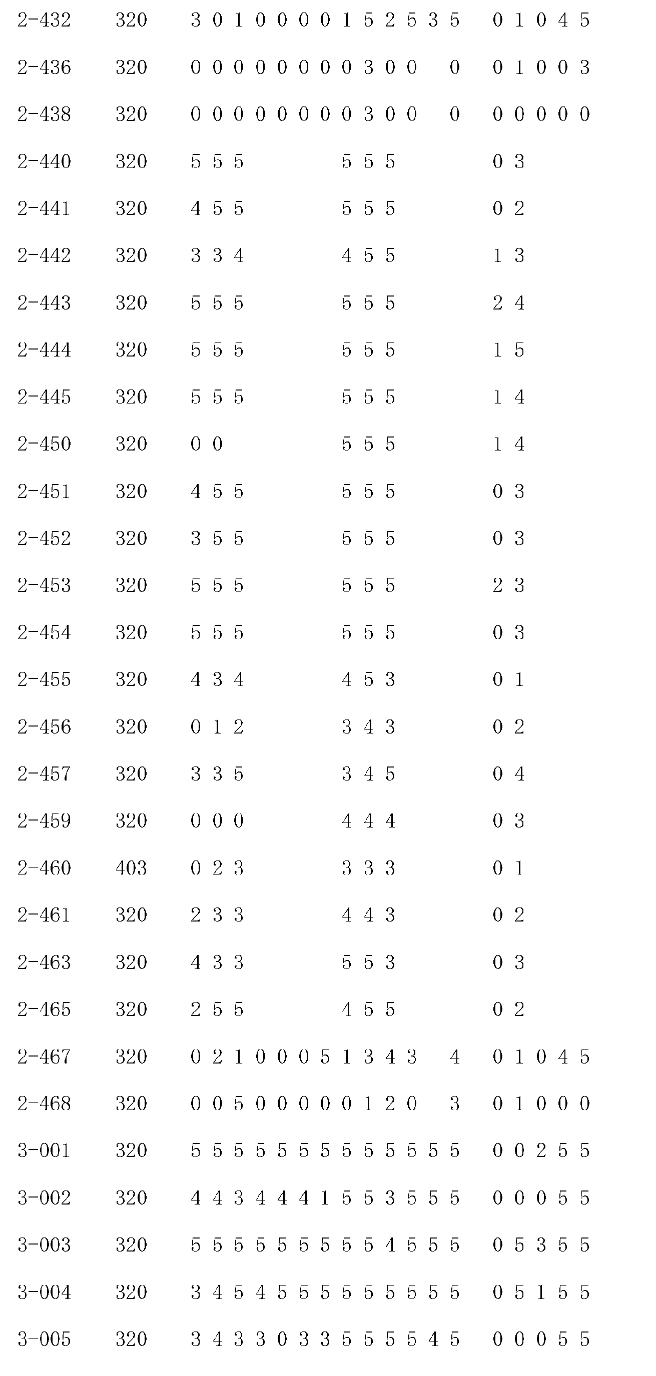

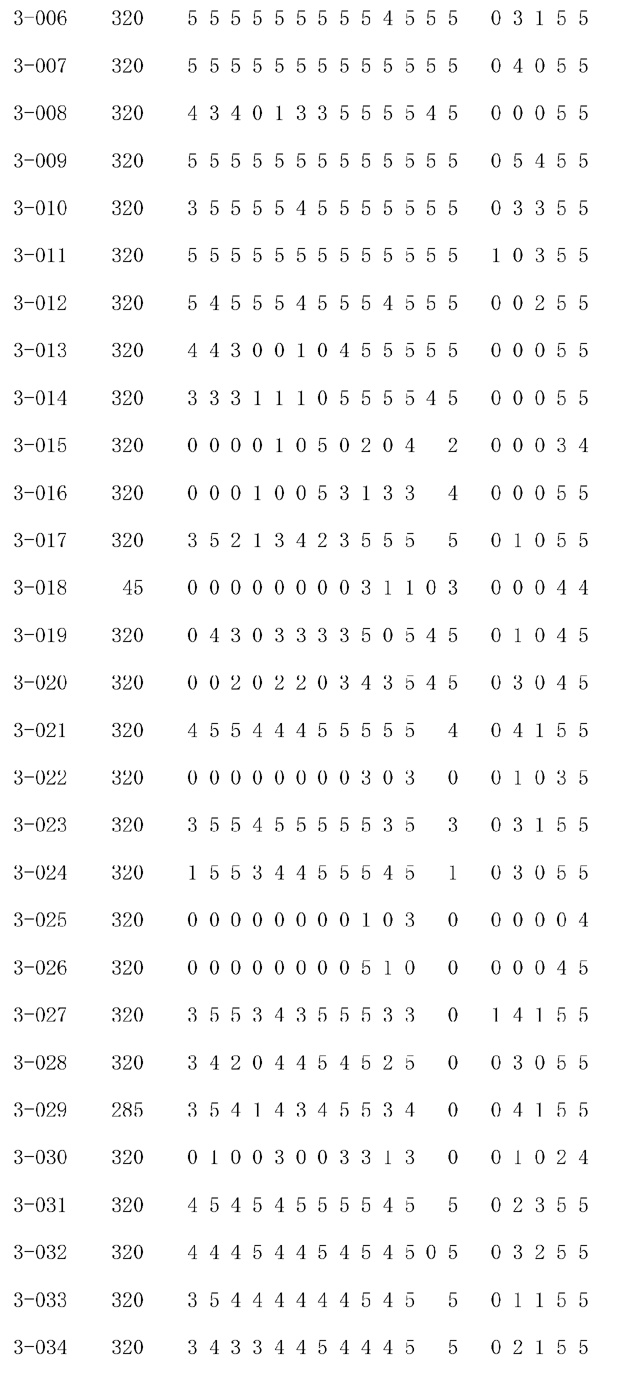

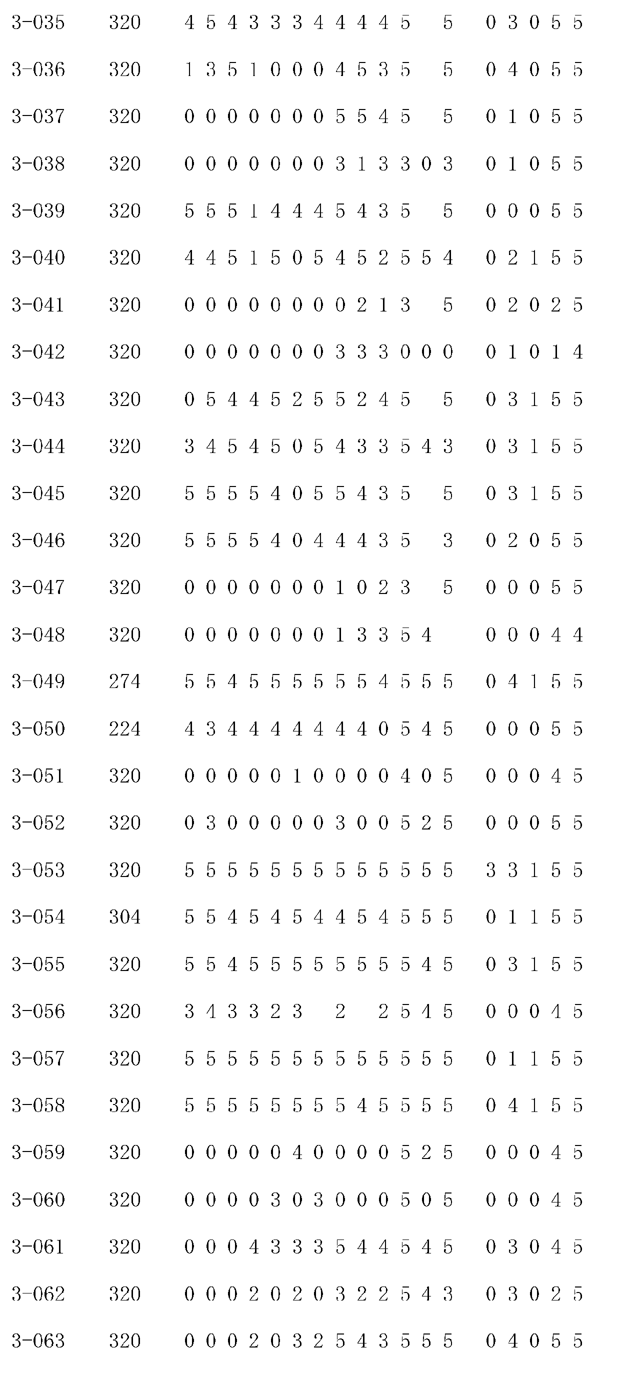

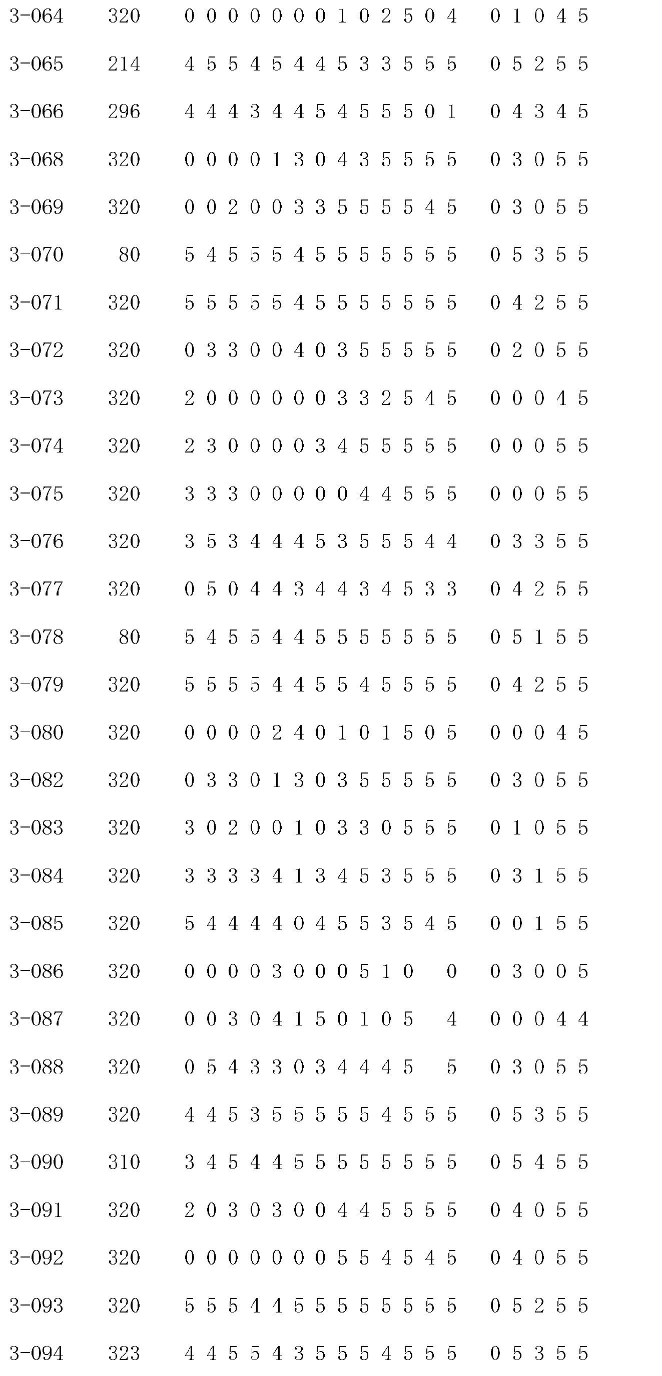

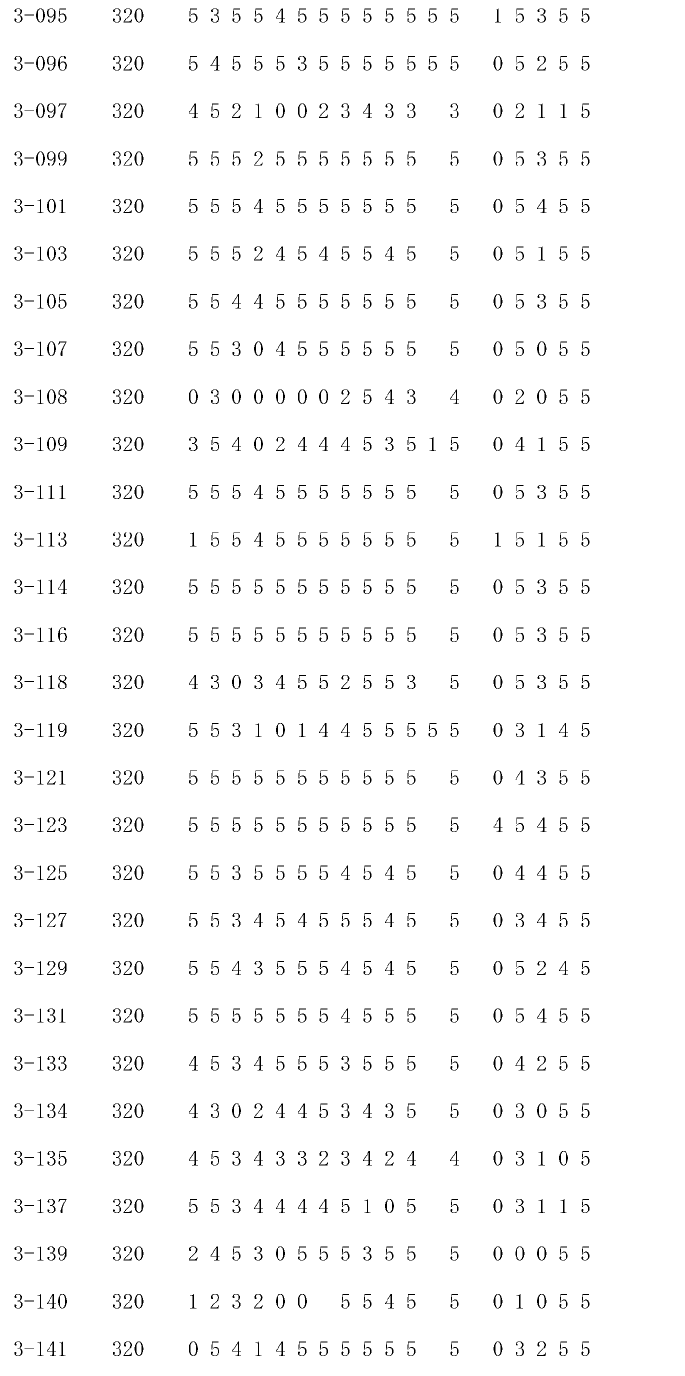

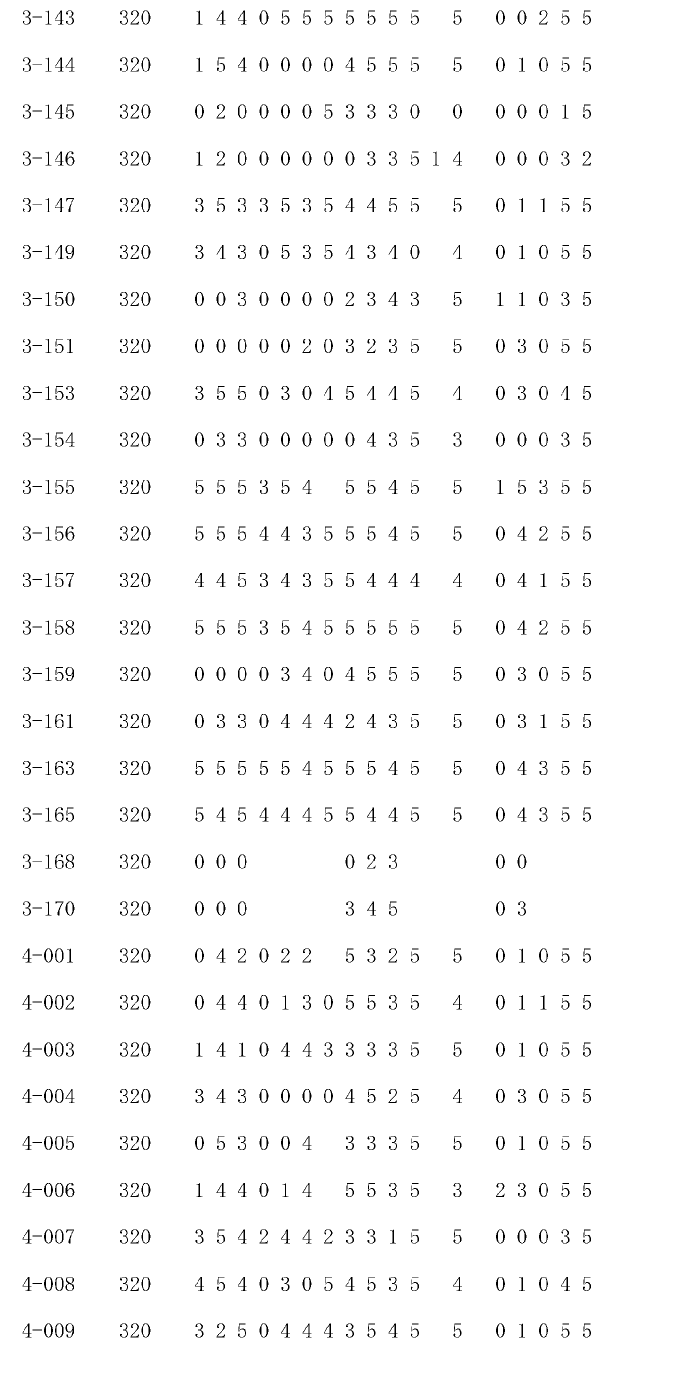

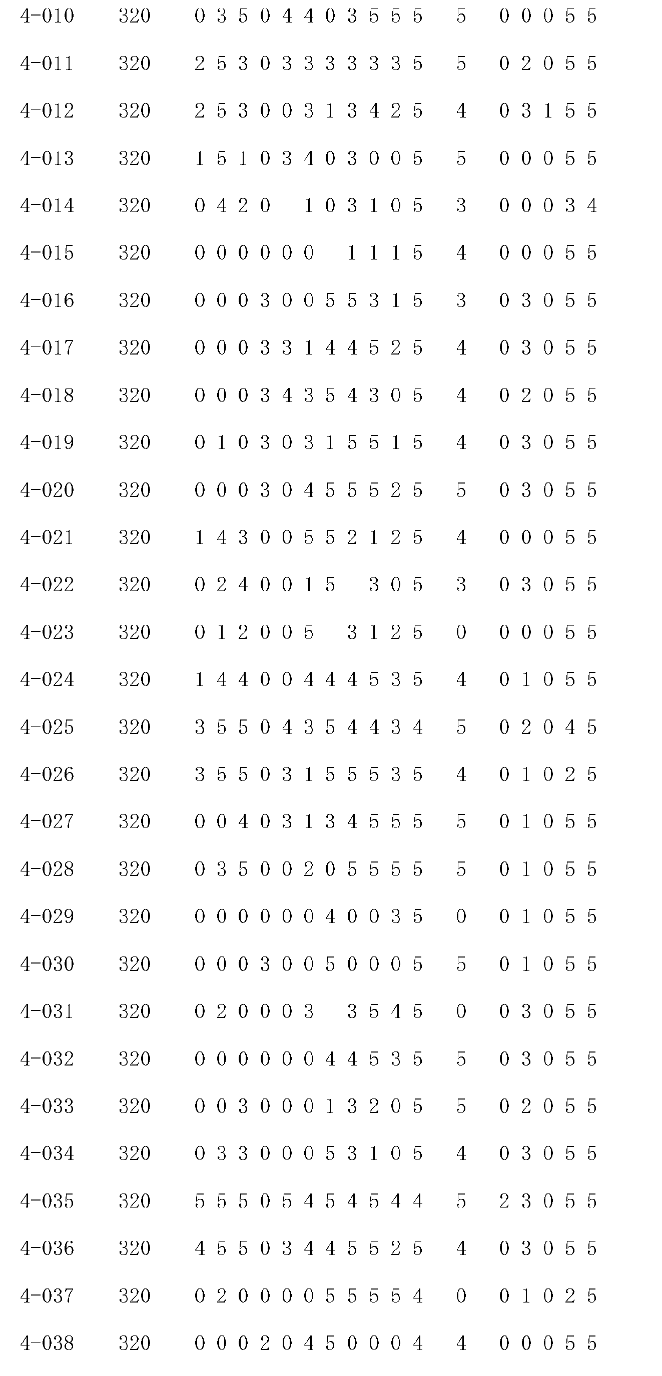

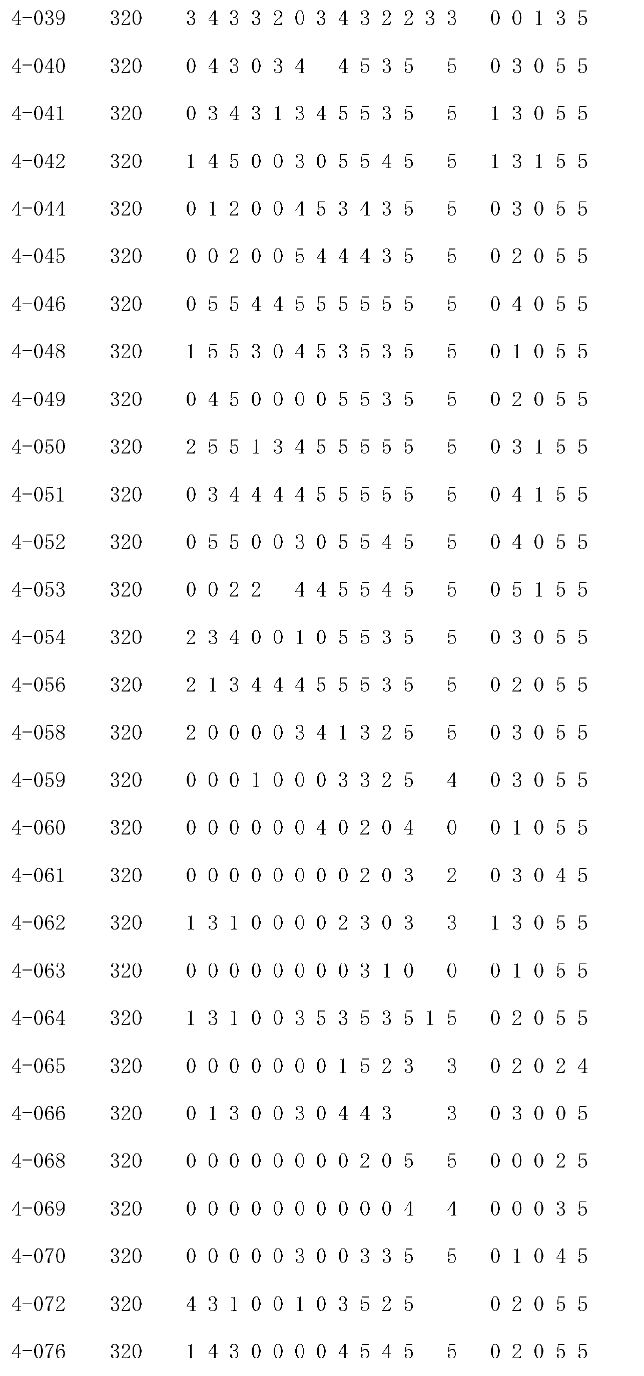

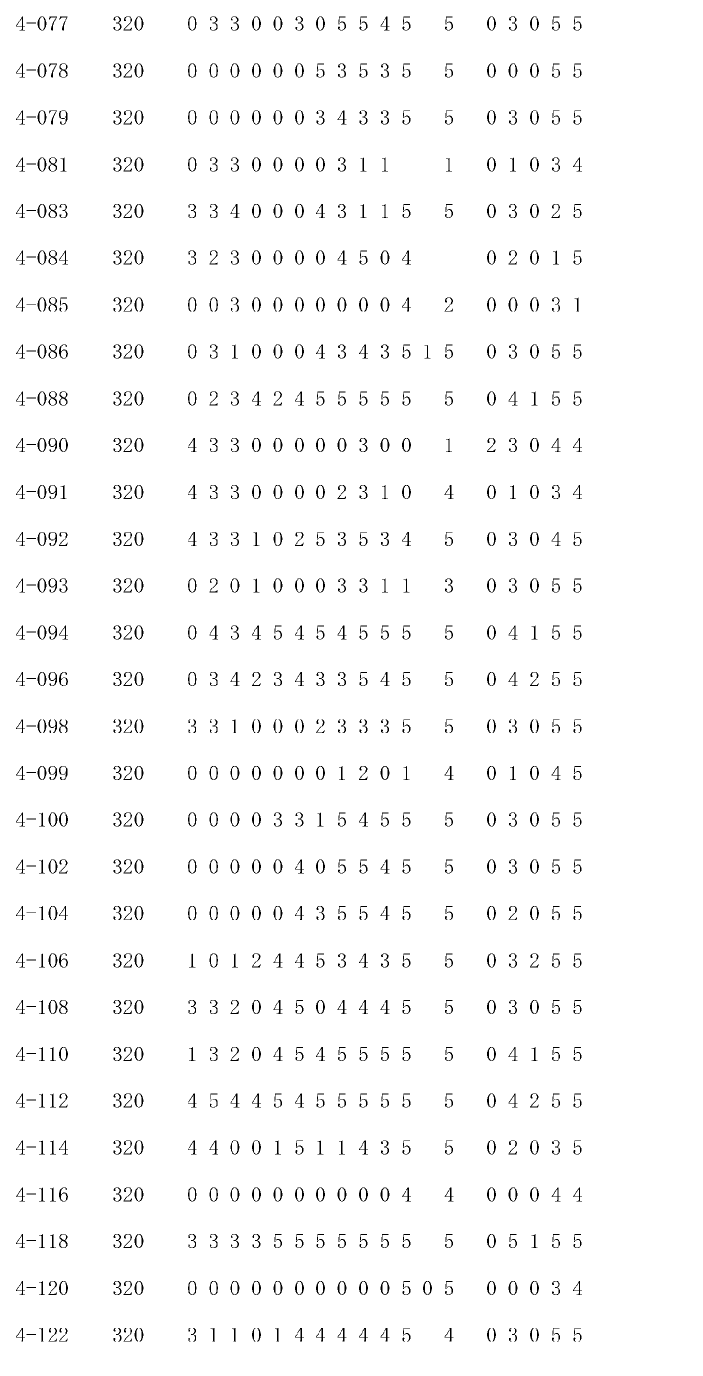

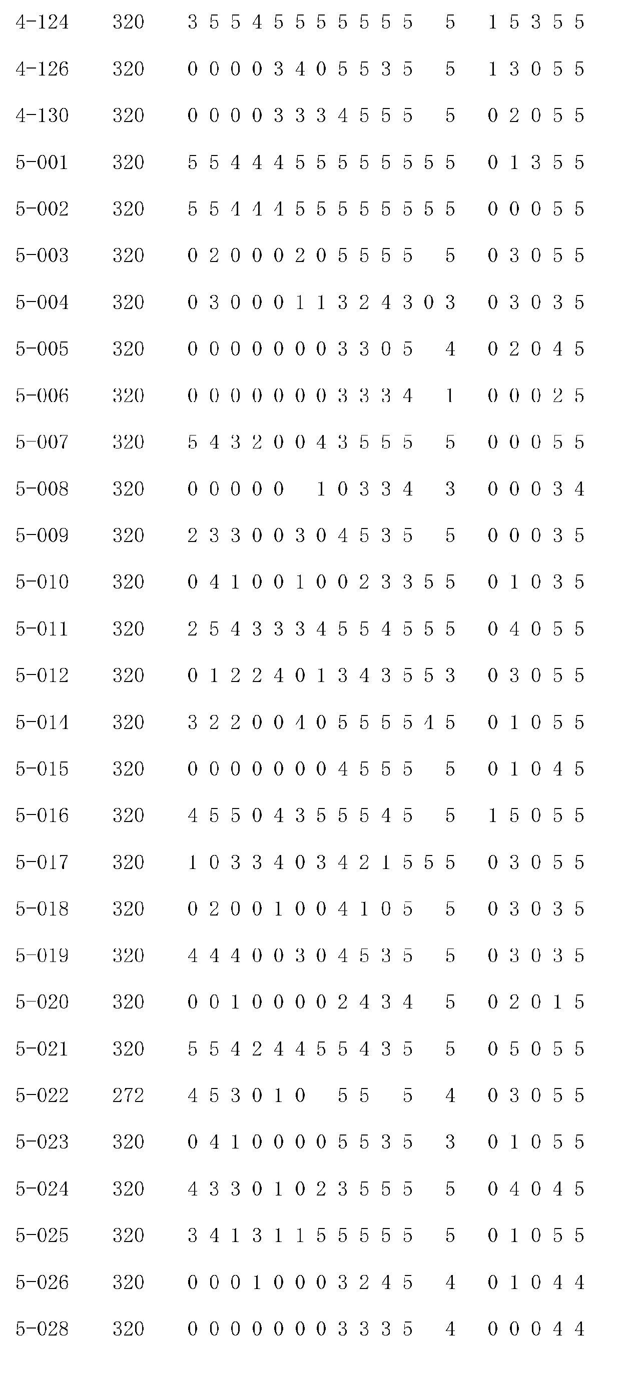

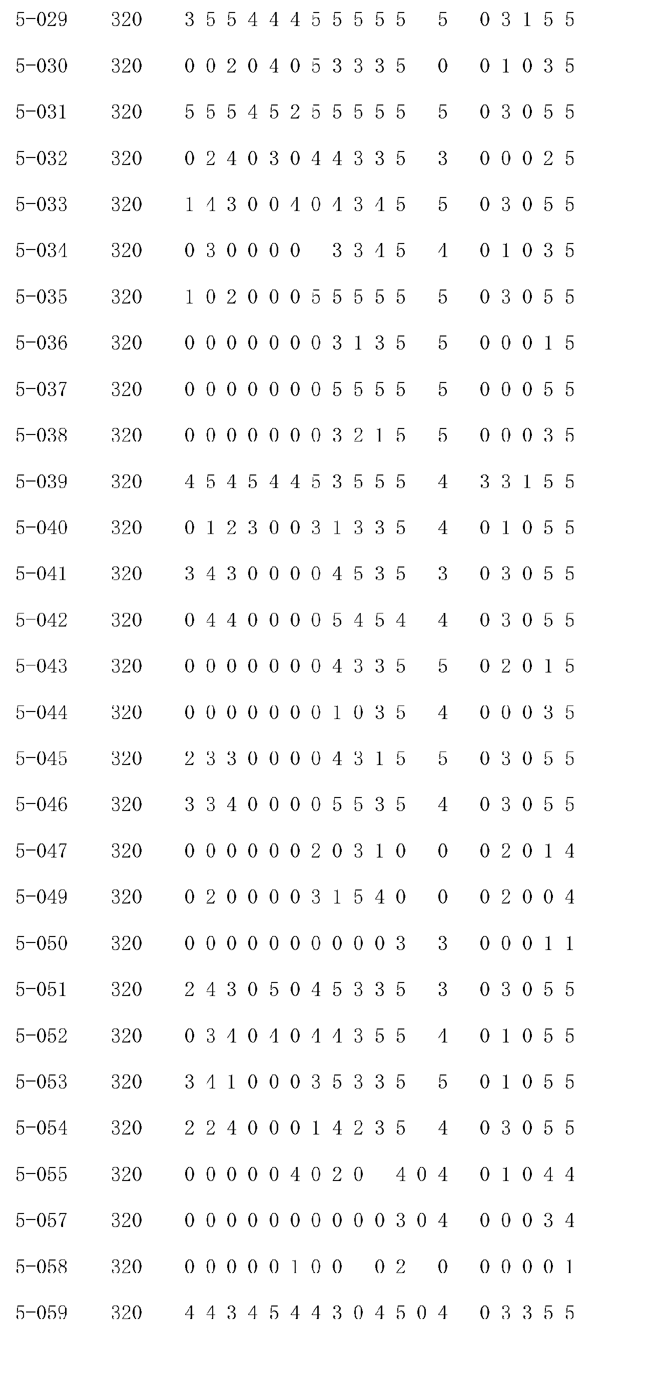

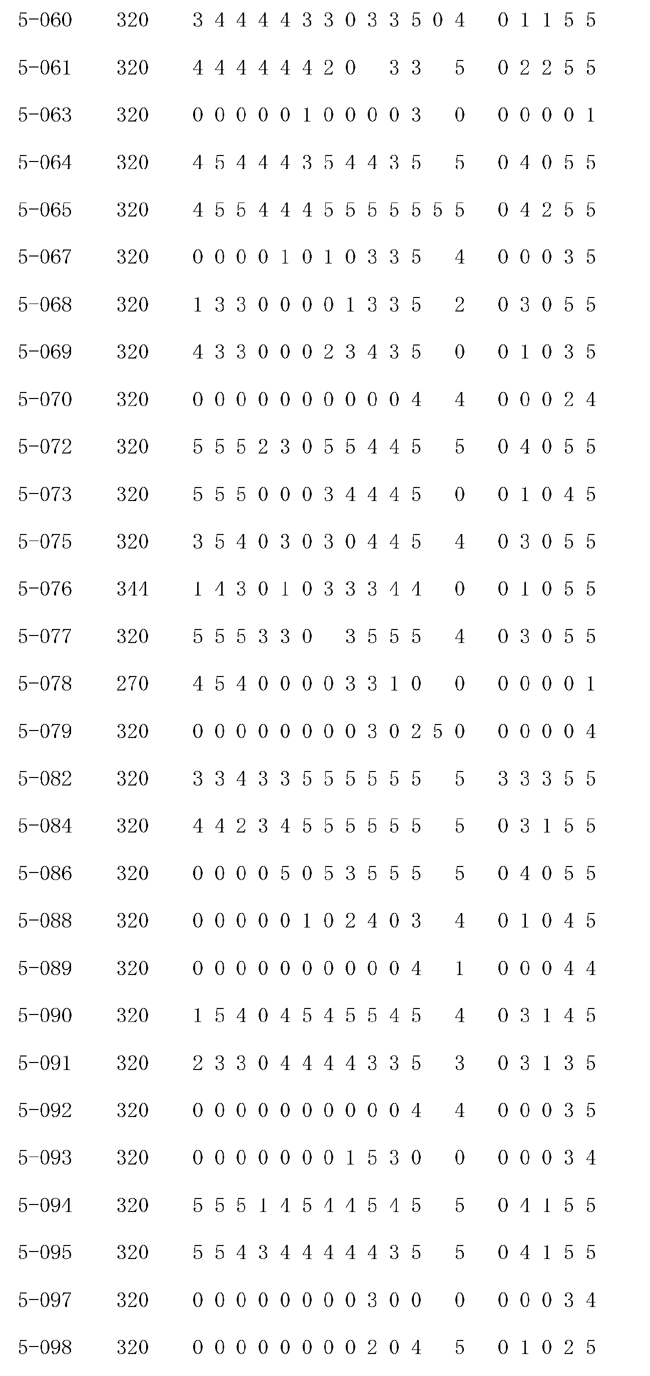

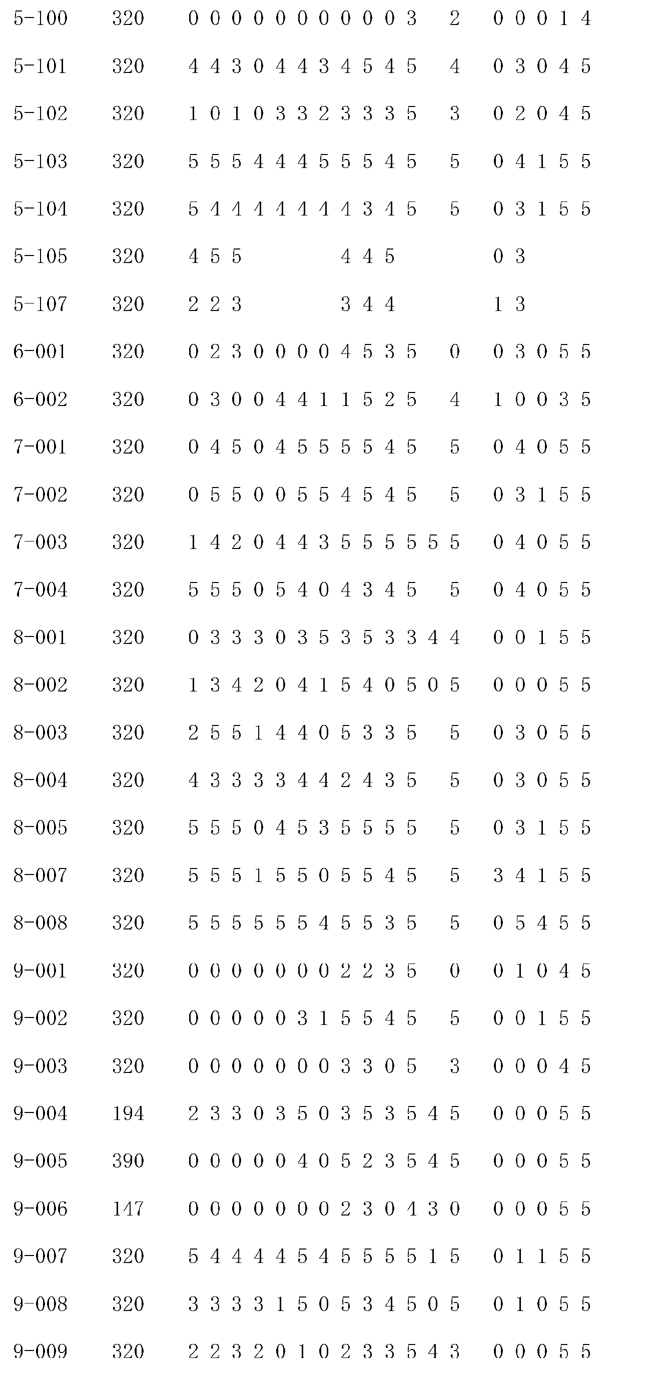

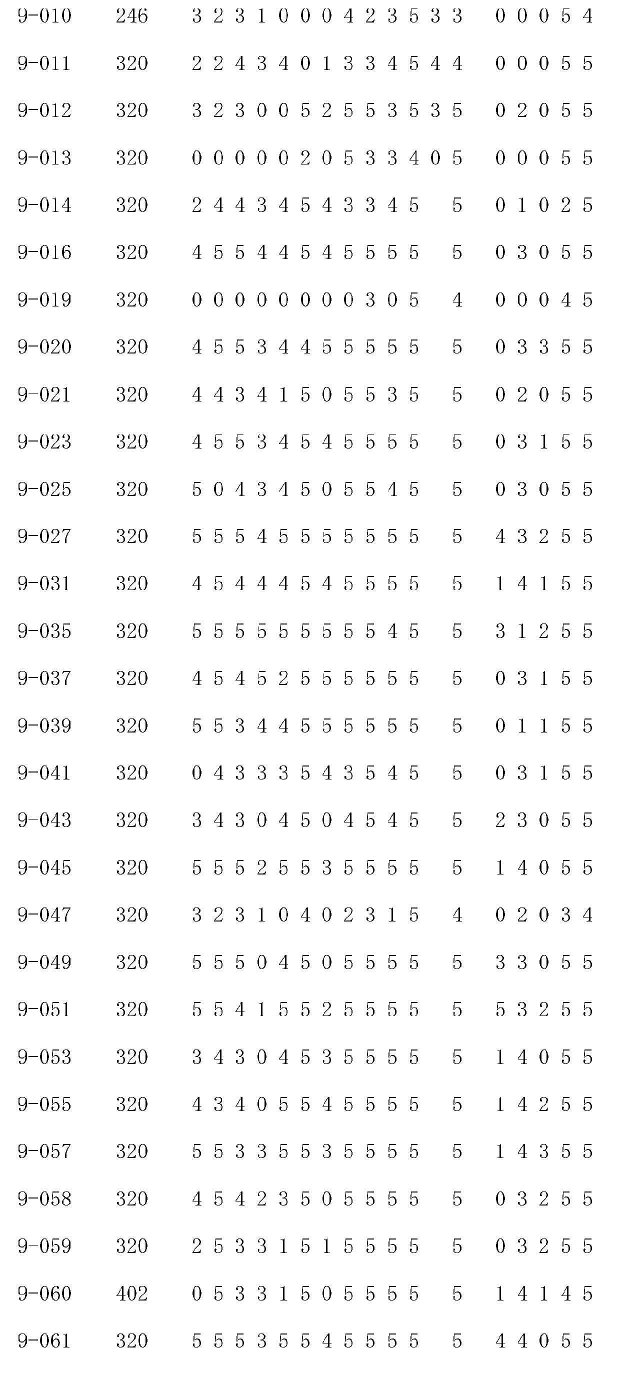

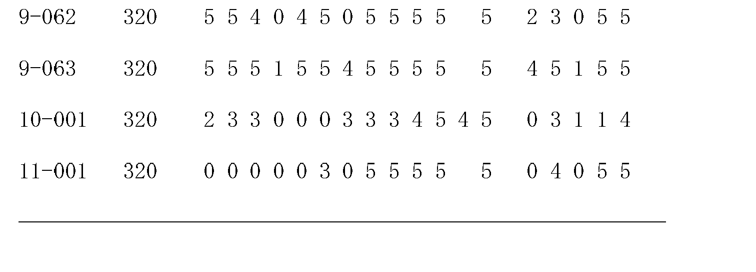

### INDUSTRIAL APPLICABILITY

The pyridazinone compound of the present invention is a novel compound and is very useful as a selective herbicide for rice, corn, soybean, wheat, beet, and rapeseed.

## Claims

1. A pyridazinone compound of the following Formula (1) or a salt thereof: 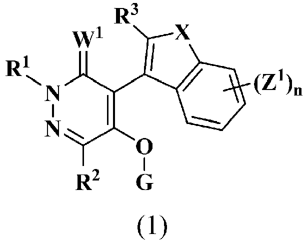 [wherein W¹ is an oxygen atom or a sulfur atom;
X is an oxygen atom or a sulfur atom;
Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, (C₃₋₆) cycloalkyl substituted with R⁴⁴, phenyl, phenyl substituted with (Z⁴)_{p5c}, Q-2, Q-3, Q-4, Q-5, Q-6, Q-7, Q-10, Q-11, Q-12, Q-13, -NR¹⁵R¹⁶, -OR³⁵, -S(O)ᵣ₁R³⁶, -CN, -NO₂, -C(O)OH, -C(=W³)R²⁰, or -N=C(C₆H₅)₂, and when n is an integer of 2 or more, each Z¹ is the same as or different from each other;
G is a hydrogen atom, C₁₋₆ alkyl, (C₁₋₆) alkyl substituted with R⁴, -C(=W⁴)R⁵, or - S(O)₂R⁶;
R¹ is a hydrogen atom, C₁₋₆ alkyl, C₃₋₆ alkenyl, C₃₋₆ alkynyl, or C₁₋₆ alkyl substituted with R³⁴;
R² is a hydrogen atom, a halogen atom, C₁₋₆ alkyl, C₁₋₆ alkoxy, C₃₋₆ cycloalkyl, or - NR³⁰R³¹;
R³ is D-1, D-2, D-3, D-4, D-5, D-6, D-7, D-8, D-9, D-10, D-11, D-12, D-13, D-14, D-15, D-16, D-17, D-18, D-19, D-20, D-21, D-22, D-23, D-24, D-25, D-26, D-27, D-28, D-29, D-30, D-31, D-32, D-33, D-34, D-35, D-36, D-37, D-38, D-39, D-40, D-41, D-42, D-43, D-44, D-45, D-46, D-47, D-48, D-49, D-50, D-51, D-52, D-53, D-54, D-55, or D-56;
D-1, D-2, D-3, D-4, D-5, D-6, D-7, D-8, D-9, D-10, D-11, D-12, D-13, D-14, D-15, D-16, D-17, D-18, D-19, D-20, D-21, D-22, D-23, D-24, D-25, D-26, D-27, D-28, D-29, D-30, D-31, D-32, D-33, D-34, D-35, D-36, D-37, D-38, D-39, D-40, D-41, D-42, D-43, D-44, D-45, D-46, D-47, D-48, D-49, D-50, D-51, D-52, D-53, D-54, D-55, and D-56 are respectively the following structures: 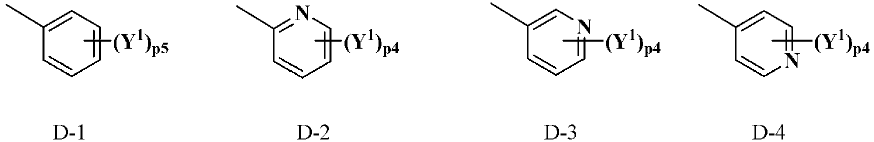 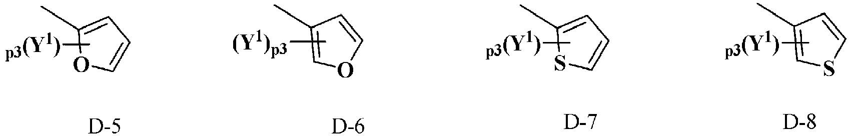 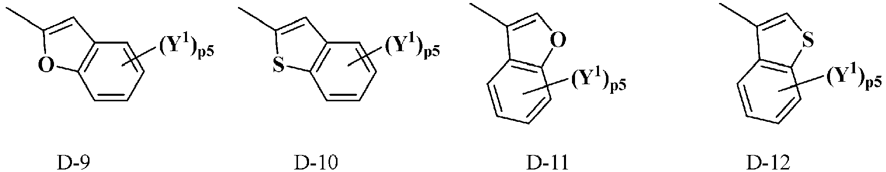 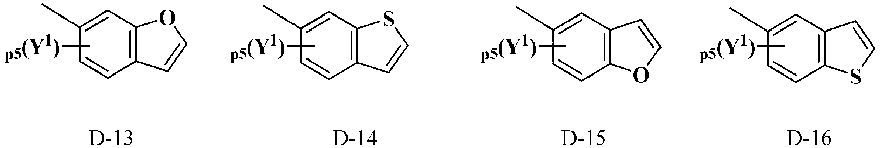 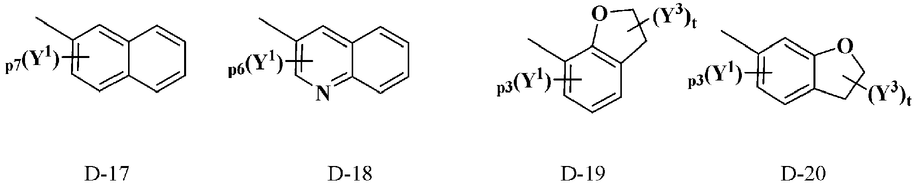 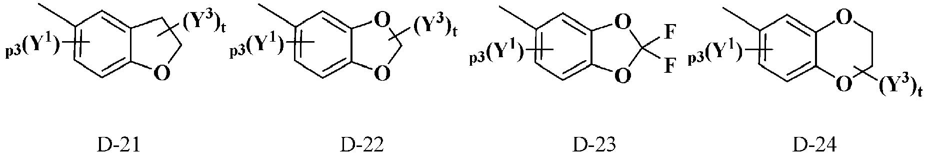 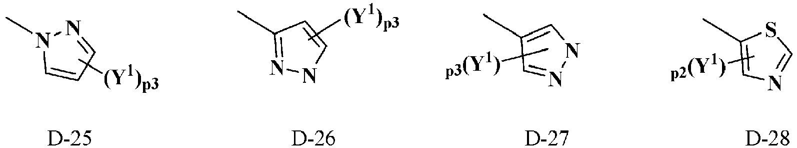 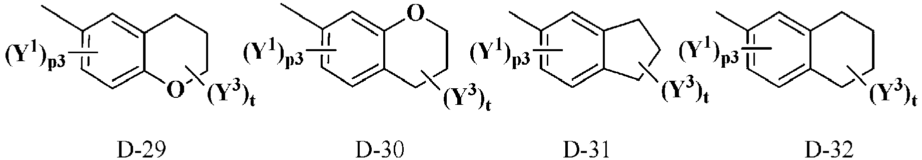 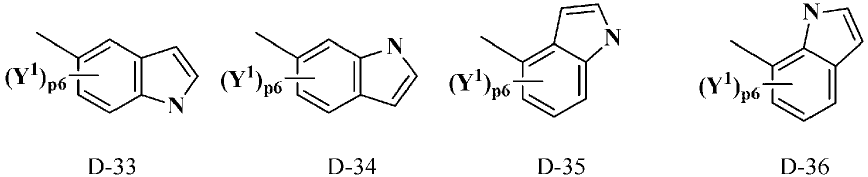 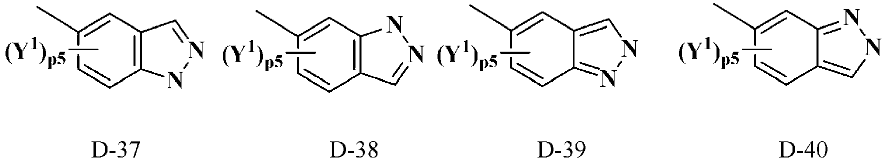 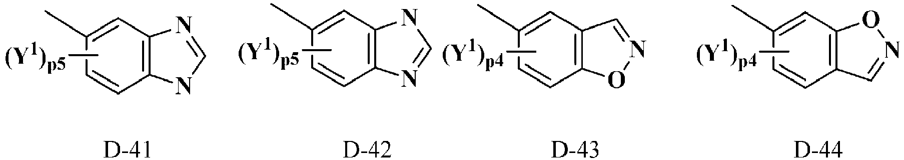 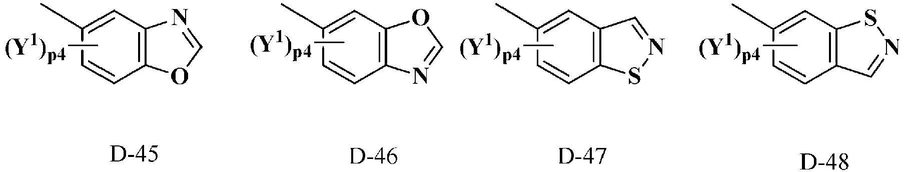 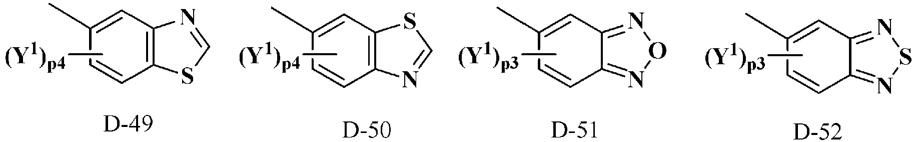 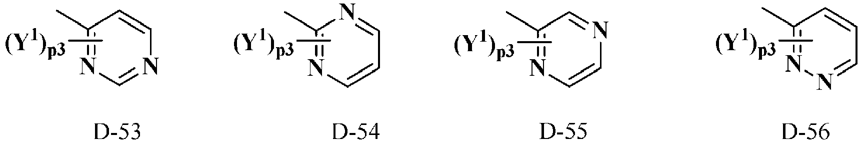
Y¹ is substituted on the aromatic ring of each of D-1 to D-56, and Y³ is substituted on the aliphatic ring of D-19, D-20, D-21, D-22, D-24, D-29, D-30, D-31, or D-32;
Y¹ is a hydrogen atom, a halogen atom, C₁₋₆ alkyl, C₂₋₆ alkenyl, C₂₋₆ alkynyl, C₃₋₆ cycloalkyl, C₂₋₆ haloalkenyl, C₂₋₆ haloalkynyl, (C₁₋₆) alkyl substituted with R¹⁰, C₃₋₆ cycloalkyl substituted with R⁴⁴, -OR¹¹, -S(O)ᵣ₂R³⁸, -NR⁸R⁹, -CN, -NO₂, -C(O)OH, - C(=W²)R¹³, phenyl, phenyl substituted with (Z⁴)_{p5c}, tri(C₁-₆ alkyl)silyl, Q-6, Q-7, Q-10, Q-11, Q-12, or Q-13, and when p7, p6, p5, p4, p3, or p2 is an integer of 2 or more, each Y¹is the same as or different from each other;
R⁴ is a halogen atom, -CN, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁴, -OR²⁴, -S(O)ᵣ₄R⁵⁴, - C(O)R²³, phenyl, phenyl substituted with (Z⁴)_{p5c}, U-1, U-2, U-3, U-4, U-5, U-9, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, or Q-36;
R⁵ is C₁₋₆ alkyl, (C₁₋₆) alkyl substituted with R²⁶, C₂₋₆ alkenyl, C₂₋₆ haloalkenyl, C₂₋₆ alkynyl, C₂₋₆ haloalkynyl, C₁₋₆ alkoxy, -OR⁵⁸, -SR⁵⁹, C₃₋₆ cycloalkyl, (C₃₋₆) cycloalkyl substituted with R⁴⁴, -NR⁵⁶R⁵⁷, U-1, U-2, U-3, U-4, U-5, U-6, U-7, U-8, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-7, Q-8, Q-9, Q-10, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, Q-37, Q-38, Q-39, Q-40, phenyl, phenyl substituted with (Z²)ₚ₅ₐ, or -C(O)R⁵³;
R⁶ is C₁₋₆ alkyl, C₁₋₆ haloalkyl, phenyl, phenyl substituted with (Z²)ₚ₅ₐ, U-6, U-7, U-8, Q-10, or -NR²⁸R²⁹;
R⁷ is a hydrogen atom, C₁₋₆ alkyl, C₃₋₆ alkenyl, C₃₋₆ alkynyl, C₁₋₆ haloalkyl, C₁₋₆ alkoxy (C₁₋₂) alkyl, C₁₋₆ alkylthio (C₁₋₂) alkyl, C₃₋₆ cycloalkyl (C₁₋₂) alkyl, benzyl, or benzyl substituted with (Z⁴)_{p5c};
R⁸ and R⁹ are each independently a hydrogen atom, or C₁₋₆ alkyl;
R¹⁰ is a halogen atom, -OR⁴⁰, -S(O)ᵣ₃R⁴¹, -CN, C₃₋₆ cycloalkyl, (C₃₋₆) cycloalkyl substituted with R⁴⁴, Q-6, Q-7, Q-10, Q-11, Q-12, or Q-13;
R¹¹ is a hydrogen atom, C₁₋₆ alkyl, (C₁₋₆) alkyl substituted with R¹², C₃₋₆ cycloalkyl, (C₃₋₆) cycloalkyl substituted with R⁴⁴, phenyl, phenyl substituted with (Z⁴)_{p5c}, tri(C₁₋₄ alkyl)silyl, C₁₋₆ alkylcarbonyl, C₁₋₆ alkoxycarbonyl, C₁₋₆ alkylsulfonyl, C₁₋₆ haloalkylsulfonyl, - C(=W²)R¹³, -S(O)ᵣ₅R⁴⁹, U-1, U-2, U-3, U-4, U-5, U-12, U-14, U-15, U-16, Q-17, Q-18, Q-19, or Q-20;
R¹² is a halogen atom, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁵, -OR⁴⁸, -S(O)ᵣ₅R⁴⁹, -CN, phenyl, phenyl substituted with (Z³)_{p5b}, -C(=W²)R¹³, U-1, U-2, U-3, U-4, U-5, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, or - ON=CR⁴²R⁴³;
Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, Q-37, Q-38, Q-39, and Q-40 are respectively the following structures: 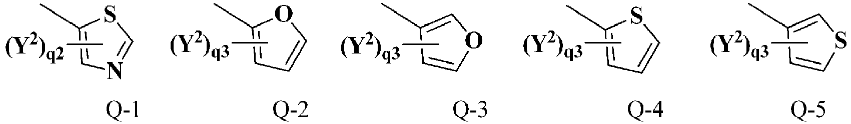 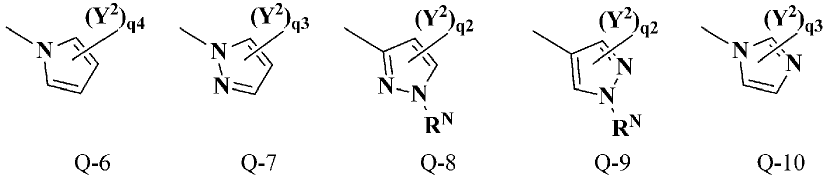 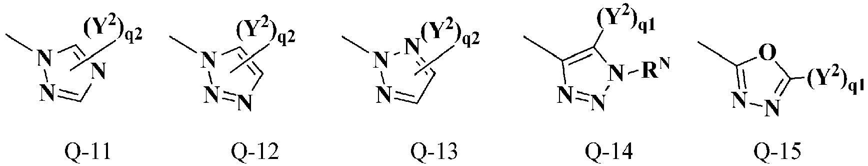 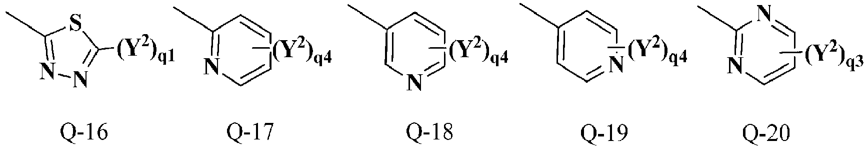 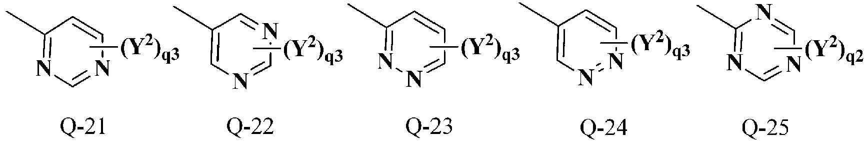 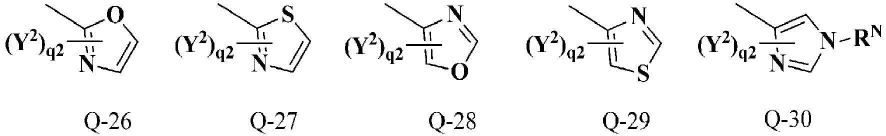 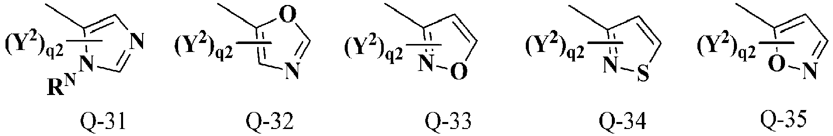 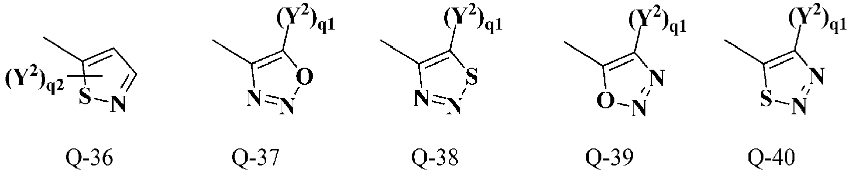
Y² is substituted on the aromatic ring of each of Q-1 to Q-40; U-1, U-2, U-3, U-4, U-5, U-6, U-7, U-8, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, and U-35 are respectively the following structures: 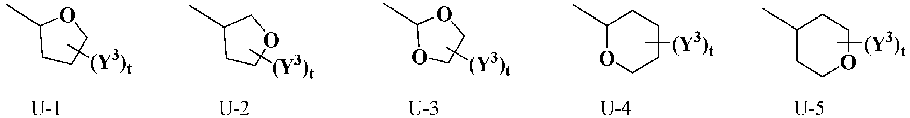 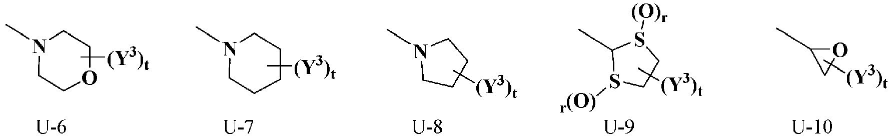 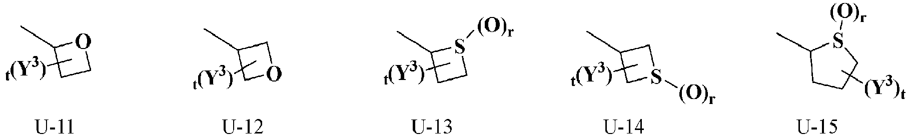 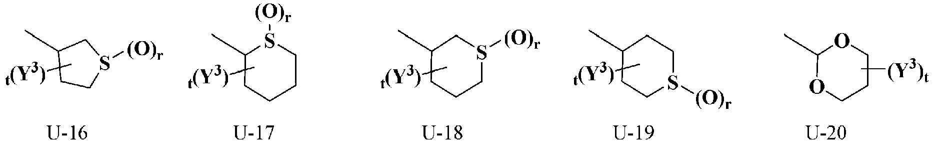 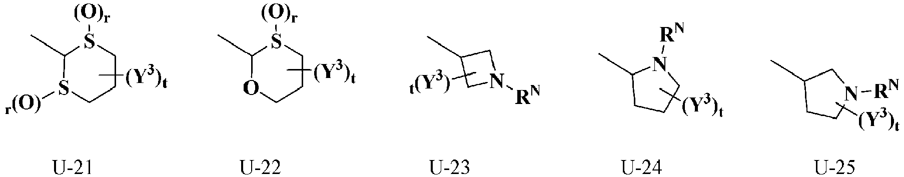 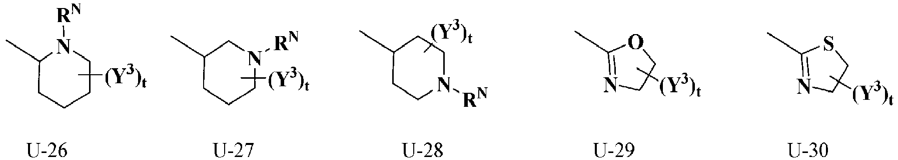 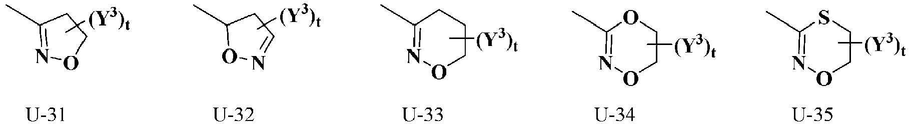
R¹³ is a hydrogen atom, C₁₋₆ alkyl, C₁₋₆ alkoxy, C₁₋₆ alkylamino, di(C₁₋₆) alkylamino, C₁₋₆ haloalkylamino, C₁₋₆ alkylthio, C₁₋₆ haloalkylthio, or -NH₂;
R¹⁴ is a halogen atom, C₂₋₆ alkenyl, C₂₋₆ alkynyl, C₃₋₆ cycloalkyl, C₂₋₆ haloalkenyl, C₂₋₆ haloalkynyl, (C₃₋₆) cycloalkyl substituted with R⁴⁵, C₁₋₆ alkoxy, C₁₋₆ alkylthio, C₁₋₆ alkylsulfinyl, C₁₋₆ alkylsulfonyl, C₁₋₆ haloalkoxy, C₁₋₆ haloalkylthio, C₁₋₆ haloalkylsulfinyl, C₁₋₆ haloalkylsulfonyl, phenyl, phenyl substituted with (Z⁴)_{p5c}, -CN, U-1, U-2, U-3, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, or Q-36;
R¹⁵ and R¹⁶ are each independently a hydrogen atom, C₁₋₆ alkyl, -C(O)R¹⁷, or - S(O)₂R¹⁸;
R¹⁷ is C₁₋₆ alkyl, C₁₋₆ alkoxy, or C₁₋₆ alkoxy (C₁₋₂) alkyl;
R¹⁸ is C₁₋₆ alkyl or C₁₋₆ haloalkyl;
R¹⁹ is C₃₋₆ cycloalkyl or tri(C₁₋₆ alkyl)silyl;
R²⁰ is a hydrogen atom, C₁₋₆ alkyl, or C₁₋₆ alkoxy;
R²¹ is a hydrogen atom, C₁₋₆ alkyl, C₁₋₆ haloalkyl, C₁₋₆ alkoxy (C₁₋₂) alkyl, or C₁₋₆ alkylthio (C₁₋₂) alkyl;
R²² is a halogen atom, -OH, C₁₋₆ alkoxy, C₁₋₆ haloalkoxy, C₁₋₆ alkylthio, C₁₋₆ alkylsulfinyl, C₁₋₆ alkylsulfonyl, C₁₋₆ haloalkylthio, -CN, or Q-7;
R²³ is C₁₋₆ alkyl, C₁₋₆ alkoxy, di(C₁₋₆) alkylamino, phenyl, phenyl substituted with (Z⁴)_{p5c}, U-6, U-7, or U-8;
R²⁴ is C₁₋₆ alkyl, C₁₋₆ haloalkyl, C₃₋₆ alkenyl, C₃₋₆ haloalkenyl, C₃₋₆ alkynyl, C₃₋₆ haloalkynyl, (C₁₋₆) alkoxy (C₁₋₂) alkyl, -C(O)R²⁵, -S(O)₂R³³, phenyl, or phenyl substituted with (Z⁴)_{p5c};
R²⁵ is C₁₋₆ alkyl, C₁₋₆ alkoxy, phenyl, phenyl substituted with (Z⁴)_{p5c}, di(C₁₋₆) alkylamino, U-6, U-7, or U-8;
R²⁶ is a halogen atom, C₁₋₆ alkoxy, C₁₋₆ haloalkoxy, C₁₋₆ alkylthio, C₁₋₆ alkylsulfinyl, C₁₋₆ alkylsulfonyl, C₃₋₆ cycloalkyl, (C₃₋₆) cycloalkyl substituted with R⁴⁴, -CN, -OR³², phenyl, phenyl substituted with (Z⁴)_{p5c}, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, U-1, U-2, U-3, U-4, U-5, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, or U-35;
R²⁷ is a halogen atom, C₂₋₆ alkenyl, C₂₋₆ alkynyl, C₁₋₆ alkoxy, C₁₋₆ haloalkoxy, C₃₋₆ cycloalkyl, C₃₋₆ halocycloalkyl, C₁₋₆ alkylthio, C₁₋₆ haloalkylthio, -CN, phenyl, phenyl substituted with (Z³)_{p5b}, 9-fluorenyl, Q-2, Q-3, Q-4, Q-5, Q-17, U-1, U-2, U-3, U-4, U-5, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, or U-35;
R²⁸ and R²⁹ are each independently a hydrogen atom or C₁₋₆ alkyl;
R³⁰ and R³¹ are each independently a hydrogen atom, C₁₋₆ alkyl, or benzyl;
R³² is phenyl, phenyl substituted with (Z⁴)_{p5c}, or Q-17;
R³³ is C₁₋₆ alkyl, C₁₋₆ haloalkyl, or di(C₁₋₆ alkyl)amino;
R³⁴ is a halogen atom, C₁₋₆ alkoxy, phenyl, or -CN;
R³⁵ is a hydrogen atom, C₁₋₆ alkyl, C₃₋₆ cycloalkyl, (C₁₋₆) alkyl substituted with R¹⁴, (C₁₋₆) cycloalkyl substituted with R⁴⁴, or -S(O)₂R³³;
R³⁶ is C₁₋₆ alkyl, (C₁₋₆) alkyl substituted with R³⁷, C₃₋₆ cycloalkyl, or (C₁₋₆) cycloalkyl substituted with R⁴⁴;
R³⁷ is a halogen atom, C₂₋₆ alkenyl, C₂₋₆ haloalkenyl, C₂₋₆ alkynyl, C₂₋₆ haloalkynyl, C₃₋₆ cycloalkyl, C₃₋₆ halocycloalkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, phenyl, phenyl substituted with (Z⁴)_{p5c}, -CN, U-1, U-2, U-3, U-9, U-10, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, U-34, U-35, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, or C₁₋₁₀ alkoxycarbonyl;
R³⁸ is C₁₋₆ alkyl, C₃₋₆ alkenyl, C₃₋₆ alkynyl, C₃₋₆ cycloalkyl, C₃₋₆ haloalkenyl, C₃₋₆ haloalkynyl, (C₁₋₆) alkyl substituted with R³⁹, (C₃₋₆) cycloalkyl substituted with R⁴⁴, U-1, U-2, U-4, U-5, U-6, U-7, U-8, U-12, U-14, U-15, U-16, or -NR⁶⁰R⁶¹;
R³⁹ is a halogen atom, C₃₋₆ cycloalkyl, (C₃₋₆) cycloalkyl substituted with R⁴⁴, -OR⁵¹, - S(O)ᵣ₆R⁵², -C(=W²)R¹³, -CN, phenyl, phenyl substituted with (Z³)_{p5b}, U-1, U-2, U-3, U-4, U-5, U-9, U-10, U-11, U-12, U-13, U-14, U-15, U-16, U-17, U-18, U-19, U-20, U-21, U-22, U-23, U24, U-25, U-26, U-27, U-28, U-29, U-30, U-31, U-32, U-33, U-34, U-35, Q-1, Q-2, Q-3, Q-4, Q-5, Q-8, Q-9, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, or - ON=CR⁴²R⁴³;
R⁴⁰ is a hydrogen atom, C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, (C₁₋₆) alkyl substituted with R⁴⁶, (C₃₋₆) cycloalkyl substituted with R⁴⁴, or U-4;
R⁴¹ is a hydrogen atom, C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, (C₃₋₆) cycloalkyl substituted with R⁴⁴, or (C₁₋₆) alkyl substituted with R⁴⁷;
R⁴² and R⁴³ are each independently a hydrogen atom, C₁₋₆ alkyl, phenyl, or phenyl substituted with (Z⁴)_{p5c}, or R⁴² and R⁴³ form C₃₋₆ cycloalkyl together with the carbon atom to which R⁴² and R⁴³ are bonded;
R⁴⁴ is a halogen atom, C₁₋₆ alkyl, or -CN;
R⁴⁵ is a halogen atom, C₁₋₆ alkyl, or -CN;
R⁴⁶ is C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₃₋₆ cycloalkyl, (C₃₋₆) cycloalkyl substituted with R⁴⁴, -CN, U-1, U-2, U-3, U-4, U-5, U-9, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, or Q-36;
R⁴⁷ is C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₃₋₆ cycloalkyl, (C₃₋₆) cycloalkyl substituted with R⁴⁴, U-1, U-2, U-3, U-4, U-5, U-9, U-11, U-12, U-13, U-14, U-15, U-16, U-17, U-18, U-19, U-20, U-21, U-22, U-23, U-24, U-25, U-26, U-27, U-28, U-29, U-30, U-31, U-32, U-33, Q-1, Q-2, Q-3, Q-4, Q-5, Q-6, Q-7, Q-8, Q-9, Q-10, Q-11, Q-12, Q-13, Q-14, Q-15, Q-16, Q-17, Q-18, Q-19, Q-20, Q-21, Q-22, Q-23, Q-24, Q-25, Q-26, Q-27, Q-28, Q-29, Q-30, Q-31, Q-32, Q-33, Q-34, Q-35, Q-36, or -CN;
R⁴⁸ is a hydrogen atom, C₁₋₆ alkyl, C₁₋₆ haloalkyl, C₃₋₆ alkenyl, C₃₋₆ haloalkenyl, C₃₋₆ alkynyl, C₃₋₆ haloalkynyl, C₃₋₆ cycloalkyl, (C₃₋₆) cycloalkyl substituted with R⁴⁴, -C(O)R⁵⁰, or -S(O)₂R³³;
R⁴⁹ is C₁₋₆ alkyl, C₁₋₆ haloalkyl, C₃₋₆ alkenyl, C₃₋₆ haloalkenyl, C₃₋₆ alkynyl, C₃₋₆ haloalkynyl, C₃₋₆ cycloalkyl, or (C₃₋₆) cycloalkyl substituted with R⁴⁴;
R⁵⁰ is C₁₋₆ alkyl, C₁₋₆ alkoxy, or di(C₁₋₆ alkyl)amimo;
R⁵¹ is a hydrogen atom, C₁₋₆ alkyl, C₃₋₆ alkenyl, C₃₋₆ alkynyl, C₁₋₆ haloalkyl, C₃₋₆ haloalkenyl, or C₃₋₆ haloalkynyl;
R⁵² is C₁₋₆ alkyl, C₃₋₆ alkenyl, C₃₋₆ alkynyl, C₁₋₆ haloalkyl, C₃₋₆ haloalkenyl, C₃₋₆ haloalkynyl, or C₃₋₆ cycloalkyl;
R⁵³ is C₁₋₆ alkyl, C₁₋₆ alkoxy, phenyl, or phenyl substituted with (Z⁴)_{p5c};
R⁵⁴ is C₁₋₆ alkyl, C₁₋₆ haloalkyl, C₃₋₆ alkenyl, C₃₋₆ alkynyl, phenyl, or phenyl substituted with (Z⁴)_{p5c};
R⁵⁵ is C₁₋₆ alkyl, C₁₋₆ alkoxy, -OH, or NR⁵⁶R⁵⁷;
R⁵⁶ and R⁵⁷ are each independently a hydrogen atom or C₁₋₆ alkyl;
R⁵⁸ is (C₁₋₆) alkyl substituted with R²⁷, C₃₋₆ cycloalkyl, (C₃₋₆) cycloalkyl substituted with R⁴⁴, phenyl, or phenyl substituted with (Z³)_{p5b};
R⁵⁹ is C₁₋₆ alkyl, C₁₋₆ haloalkyl, C₁₋₆ alkenyl, C₁₋₆ alkynyl, C₃₋₆ cycloalkyl, (C₃₋₆) cycloalkyl substituted with R⁴⁴, phenyl, or phenyl substituted with (Z³)_{p5b};
R⁶⁰ and R⁶¹ are each independently a hydrogen atom, C₁₋₆ alkyl, C₁₋₆ haloalkyl, C₁₋₆ alkoxy (C₁₋₆) alkyl, phenyl, phenyl substituted with (Z⁴)_{p5c}, benzyl, or benzyl substituted with (Z⁴)_{p5c};
R^{N} is a hydrogen atom or C₁₋₆ alkyl;
Y² is a halogen atom, C₁₋₆ alkyl, C₁₋₆ haloalkyl, C₁₋₆ alkoxy, C₁₋₆ haloalkoxy, C₁₋₆ alkylthio, C₁₋₆ haloalkylthio, -CN, -NH₂, or -NO₂, and when q4, q3, or q2 is an integer of 2 or more, each Y² is the same as or different from each other;
Y³ is a halogen atom, C₁₋₆ alkyl, C₁₋₆ haloalkyl, C₁₋₆ alkoxy, C₁₋₆ haloalkoxy, C₁₋₆ alkylthio, C₁₋₆ haloalkylthio, C₁₋₆ alkoxycarbonyl, -CN, -C(O)OH, -OH, or -NH₂, and when t is 2, each Y³ is the same as or different from each other;
Z² is a halogen atom, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, - S(O)₂NR¹⁶R⁵⁷, -OH, -NH₂, -CN, -NO₂, or -C(O)R⁵⁵, and when p5a is an integer of 2 or more, each Z² is the same as or different from each other;
Z³ is a halogen atom, C₁₋₆ alkyl, C₁₋₆ haloalkyl, C₁₋₆ alkoxy, C₁₋₆ haloalkoxy, C₁₋₆ alkylthio, C₁₋₆ haloalkylthio, -CN, C₁₋₆ alkoxycarbonyl, or -NO₂, and when p5b is an integer of 2 or more, each Z³ is the same as or different from each other;
Z⁴ is a halogen atom, C₁₋₆ alkyl, C₁₋₆ haloalkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, -CN, -NO₂, C₁₋₆ alkoxycarbonyl, and when p5c is an integer of 2 or more, each Z⁴ is the same as or different from each other;
W² is an oxygen atom or N-OR⁷;
W³ is an oxygen atom or N-OR²¹;
W⁴ is an oxygen atom or a sulfur atom;
r1 is an integer of 0, 1, or 2;
r2 is an integer of 0, 1, or 2;
r3 is an integer of 0, 1, or 2;
r4 is an integer of 0, 1, or 2;
r5 is an integer of 0, 1, or 2;
r6 is an integer of 0, 1, or 2;
n is an integer of 0, 1, 2, 3, or 4;
t is an integer of 0, 1, or 2;
p2 is an integer of 0, 1, or 2;
p3 is an integer of 0, 1, 2, or 3;
p4 is an integer of 0, 1, 2, 3, or 4;
p5 is an integer of 0, 1, 2, 3, 4, or 5;
p6 is an integer of 0, 1, 2, 3, 4, 5, or 6;
p7 is an integer of 0, 1, 2, 3, 4, 5, 6, or 7;
p5a is an integer of 1, 2, 3, 4, or 5;
p5b is an integer of 1, 2, 3, 4, or 5;
p5c is an integer of 1, 2, 3, 4, or 5;
q1 is an integer of 0 or 1;
q2 is an integer of 0, 1, or 2;
q3 is an integer of 0, 1, 2, or 3; and
q4 is an integer of 0, 1, 2, 3, or 4].

2. The pyridazinone compound and a salt thereof according to claim 1, wherein:
W¹ is an oxygen atom;
Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, (C₃₋₆) cycloalkyl substituted with R⁴⁴, phenyl, phenyl substituted with (Z⁴)_{p5c}, Q-3, -NR¹⁵R¹⁶, -OR³⁵, -S(O)ᵣ₁R³⁶, -CN, -NO₂, - C(O)OH, -C(=W³)R²⁰, or -N=C(C₆H₅)₂, and when n is an integer of 2 or more, each Z¹ is the same as or different from each other;
R¹ is a hydrogen atom, C₁₋₆ alkyl, C₃₋₆ alkenyl, C₃₋₆ alkynyl, or C₁₋₆ alkyl substituted with R³⁴;
R² is a hydrogen atom, a halogen atom, C₁₋₆ alkyl, C₁₋₆ alkoxy, or -NR³⁰R³¹;
R³ is D-1, D-2, D-3, D-4, D-5, D-6, D-7, D-8, D-9, D-10, D-13, D-14, D-15, D-16, D-17, D-18, D-19, D-20, D-21, D-22, D-23, D-24, D-25, D-26, D-27, or D-28;
Y¹ is a hydrogen atom, a halogen atom, C₁₋₆ alkyl, C₂₋₆ alkenyl, C₂₋₆ alkynyl, C₃₋₆ cycloalkyl, C₂₋₆ haloalkenyl, C₂₋₆ haloalkynyl, (C₁₋₆) alkyl substituted with R¹⁰, C₃₋₆ cycloalkyl substituted with R⁴⁴, -OR¹¹, -S(O)ᵣ₂R³⁸, -NR⁸R⁹, -CN, -NO₂, -C(O)OH, - C(=W²)R¹³, phenyl, phenyl substituted with (Z⁴)_{p5c}, or tri(C₁₋₆ alkyl)silyl, and when p7, p6, p5, p4, p3, or p2 is an integer of 2 or more, each Y¹ is the same as or different from each other;
R⁴ is a halogen atom, -CN, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁴, -OR²⁴, -S(O)ᵣ₄R⁵⁴, - C(O)R²³, phenyl, or phenyl substituted with (Z⁴)_{p5c};
R⁵ is C₁₋₆ alkyl, (C₁₋₆) alkyl substituted with R²⁶, C₂₋₆ alkenyl, C₂₋₆ alkynyl, C₁₋₆ alkoxy, -OR⁵⁸, -SR⁵⁹, C₃₋₆ cycloalkyl, C₃₋₆ cycloalkyl substituted with R⁴⁴, -NR⁵⁶R⁵⁷, U-1, U-6, U-7, U-8, Q-1, Q-2, Q-3, Q-4, Q-5, Q-7, Q-8, Q-9, Q-10, Q-17, Q-18, Q-19, Q-20, phenyl, phenyl substituted with (Z²)ₚ₅ₐ, or -C(O)R⁵³;
R⁶ is C₁₋₆ alkyl, C₁₋₆ haloalkyl, phenyl, phenyl substituted with (Z²)ₚ₅ₐ, or -NR²⁸R²⁹;
R⁷ is a hydrogen atom, C₁₋₆ alkyl, C₃₋₆ alkenyl, C₃₋₆ alkynyl, C₁₋₆ haloalkyl, C₁₋₆ alkoxy (C₁₋₂) alkyl, or C₁₋₆ alkylthio (C₁₋₂) alkyl;
R¹¹ is a hydrogen atom, C₁₋₆ alkyl, (C₁₋₆) alkyl substituted with R¹², C₃₋₆ cycloalkyl, (C₃₋₆) cycloalkyl substituted with R⁴⁴, phenyl, phenyl substituted with (Z⁴)_{p5c}, C₁₋₆ alkylsulfonyl, C₁₋₆ haloalkylsulfonyl, U-2, U-4, Q-17, Q-18, or Q-19;
R¹² is a halogen atom, C₂₋₆ alkenyl, C₂₋₆ haloalkenyl, C₂₋₆ alkynyl, C₂₋₆ haloalkynyl, C₃₋₆ cycloalkyl, (C₃₋₆) cycloalkyl substituted with R⁴⁵, -OR⁴⁸, -S(O)ᵣ₅R⁴⁹, -CN, phenyl, phenyl substituted with (Z³)_{p5b}, -C(=W²)R¹³, U-1, U-2, U-3, Q-1, Q-2, Q-3, Q-4, Q-5, Q-18, or - ON=CR⁴²R⁴³;
R¹⁴ is a halogen atom, C₂₋₆ alkenyl, C₂₋₆ alkynyl, C₃₋₆ cycloalkyl, C₂₋₆ haloalkenyl, C₂₋₆ haloalkynyl, (C₃₋₆) cycloalkyl substituted with R⁴⁵, C₁₋₆ alkoxy, C₁₋₆ alkylthio, C₁₋₆ alkylsulfinyl, C₁₋₆ alkylsulfonyl, phenyl, phenyl substituted with (Z⁴)_{p5c}, -CN, U-1, U-2, U-3, U-9, Q-17, Q-18, Q-19, or Q-20;
R¹⁹ is tri(C₁₋₆ alkyl)silyl;
R²² is a halogen atom, -OH, C₁₋₆ alkoxy, C₁₋₆ haloalkoxy, C₁₋₆ alkylthio, C₁₋₆ haloalkylthio, or -CN;
R²³ is C₁₋₆ alkyl, C₁₋₆ alkoxy, di(C₁₋₆) alkylamino, phenyl, phenyl substituted with (Z⁴)_{p5c}, U-7, or U-8;
R²⁴ is C₁₋₆ alkyl, C₁₋₆ haloalkyl, C₃₋₆ alkenyl, C₃₋₆ alkynyl, (C₁₋₆) alkoxy (C₁₋₂) alkyl, - C(O)R²⁵, or -S(O)₂R³³;
R²⁵ is C₁₋₆ alkyl, C₁₋₆ alkoxy, or di(C₁₋₆) alkylamino;
R²⁶ is a halogen atom, C₁₋₆ alkoxy, C₁₋₆ haloalkoxy, C₁₋₆ alkylthio, C₁₋₆ alkylsulfinyl, C₁₋₆ alkylsulfonyl, -OR³², phenyl, or phenyl substituted with (Z⁴)_{p5c};
R²⁷ is a halogen atom, C₂₋₆ alkenyl, C₂₋₆ alkynyl, C₁₋₆ alkoxy, or C₁₋₆ alkylthio;
R³⁶ is C₁₋₆ alkyl, (C₁₋₆) alkyl substituted with R³⁷, or C₃₋₆ cycloalkyl;
R³⁷ is a halogen atom, C₂₋₆ alkenyl, C₂₋₆ alkynyl, C₃₋₆ cycloalkyl, C₁₋₆ alkoxy, C₁₋₆ alkylthio, or C₁₋₁₀ alkoxycarbonyl;
R³⁸ is C₁₋₆ alkyl, C₃₋₆ alkenyl, C₃₋₆ alkynyl, C₃₋₆ cycloalkyl, C₃₋₆ haloalkenyl, C₃₋₆ haloalkynyl, (C₁₋₆) alkyl substituted with R³⁹, or (C₃₋₆) cycloalkyl substituted with R⁴⁴;
R³⁹ is a halogen atom, C₃₋₆ cycloalkyl, -OR⁵¹, -S(O)ᵣ₆R⁵², -C(=W²)R¹³, -CN, phenyl, phenyl substituted with (Z³)_{p5b}, U-1, U-3, U-9, Q-1, or Q-18;
R⁴⁰ is a hydrogen atom, C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, (C₁₋₆) alkyl substituted with R⁴⁶, or U-4;
R⁴¹ is C₁₋₆ alkyl, C₃₋₆ alkenyl, C₃₋₆ alkynyl, C₁₋₆ haloalkyl, C₃₋₆ haloalkenyl, C₃₋₆ haloalkynyl, C₃₋₆ cycloalkyl, or (C₁₋₆) alkyl substituted with R⁴⁷;
R⁴⁶ is C₁₋₆ alkoxy, C₁₋₆ haloalkoxy, C₁₋₆ alkylthio, C₁₋₆ haloalkylthio, C₁₋₆ alkylsulfinyl, C₁₋₆ alkylsulfonyl, C₃₋₆ cycloalkyl, -CN, U-1, U-3, or U-9;
R⁴⁷ is C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, or -CN;
R⁴⁸ is a hydrogen atom, C₁₋₆ alkyl, C₁₋₆ haloalkyl, C₃₋₆ alkenyl, C₃₋₆ haloalkenyl, C₃₋₆ alkynyl, C₃₋₆ haloalkynyl, or C₃₋₆ cycloalkyl;
R⁴⁹ is C₁₋₆ alkyl, C₁₋₆ haloalkyl, C₃₋₆ alkenyl, or C₃₋₆ alkynyl;
R⁵¹ is C₁₋₆ alkyl, C₃₋₆ alkenyl, C₃₋₆ alkynyl, or C₁₋₆ haloalkyl;
R⁵² is C₁₋₆ alkyl, C₃₋₆ alkenyl, or C₃₋₆ alkynyl;
R⁵⁴ is C₁₋₆ alkyl or C₃₋₆ alkenyl;
R⁵⁹ is C₁₋₆ alkyl, C₁₋₆ alkenyl, or phenyl;
Y² is a halogen atom, C₁₋₆ alkyl, C₁₋₆ haloalkyl, C₁₋₆ alkoxy, C₁₋₆ haloalkoxy, C₁₋₆ alkylthio, or C₁₋₆ haloalkylthio; and
Z² is a halogen atom, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, C₁₋₆ alkylthio, C₁₋₆ haloalkylthio,-OH, -NH₂, -CN, -NO₂, or -C(O)R⁵⁵, and when p5a is an integer of 2 or more, each Z² is the same as or different from each other.

3. The pyridazinone compound and a salt thereof according to claim 2, wherein:
Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, phenyl, phenyl substituted with (Z⁴)_{p5c}, Q-3, -NR¹⁵R¹⁶, -OR³⁵, -S(O)ᵣ₁R³⁶, -CN, -C(=W³)R²⁰, or -N=C(C₆H₅)₂, and when n is an integer of 2 or more, each Z¹ is the same as or different from each other;
R¹ is a hydrogen atom, C₁₋₆ alkyl, or C₁₋₆ alkyl substituted with R³⁴;
R³ is D-1, D-2, D-3, D-4, D-6, D-7, D-8, D-9, D-10, D-17, D-18, D-19, D-20, D-21, D-22, D-23, D-24, D-25, D-26, or D-28;
Y¹ is a hydrogen atom, a halogen atom, C₁₋₆ alkyl, C₂₋₆ alkenyl, (C₁₋₆) alkyl substituted with R¹⁰, -C(O)OH, -OR¹¹, -S(O)ᵣ₂R³⁸, -NR⁸R⁹, -CN, -NO₂, -C(=W²)R¹³, phenyl, or tri(C₁₋₆ alkyl)silyl, and when p7, p6, p5, p4, p3, or p2 is an integer of 2 or more, each Y¹ is the same as or different from each other;
R⁴ is a halogen atom, -CN, C₂₋₆ alkenyl, C₂₋₆ alkynyl, -OR²⁴, -S(O)ᵣ₄R⁵⁴, -C(O)R²³, or phenyl;
R⁵ is C₁₋₆ alkyl, (C₁₋₆) alkyl substituted with R²⁶, C₂₋₆ alkenyl, C₁₋₆ alkoxy, -OR⁵⁸, -SR⁵⁹, C₃₋₆ cycloalkyl, -NR⁵⁶R⁵⁷, U-1, U-6, Q-2, Q-4, phenyl substituted with (Z²)ₚ₅ₐ, or -C(O)R⁵³;
R⁶ is C₁₋₆ alkyl, phenyl substituted with (Z²)ₚ₅ₐ, or -NR²⁸R²⁹;
R⁷ is a hydrogen atom or C₁₋₆ alkyl;
R⁸ and R⁹ are each independently C₁₋₆ alkyl;
R¹⁰ is a halogen atom, -OR⁴⁰, or -S(O)ᵣ₃R⁴¹;
R¹¹ is a hydrogen atom, C₁₋₆ alkyl, (C₁₋₆) alkyl substituted with R¹², C₃₋₆ cycloalkyl, phenyl, C₁₋₆ alkylsulfonyl, C₁₋₆ haloalkylsulfonyl, U-2, U-4, or Q-17;
R¹² is a halogen atom, C₂₋₆ alkenyl, C₂₋₆ haloalkenyl, C₂₋₆ alkynyl, C₃₋₆ cycloalkyl, (C₃₋₆) cycloalkyl substituted with R⁴⁵, -OR⁴⁸, -S(O)ᵣ₅R⁴⁹, -CN, phenyl, -C(=W²)R¹³, U-3, Q-1, or -ON=CR⁴²R⁴³;
R¹³ is C₁₋₆ alkyl, C₁₋₆ alkoxy, or C₁₋₆ haloalkylamino;
R¹⁴ is a halogen atom, C₂₋₆ alkenyl, C₃₋₆ cycloalkyl, (C₃₋₆) cycloalkyl substituted with R⁴⁵, C₁₋₆ alkoxy, C₁₋₆ alkylthio, C₁₋₆ alkylsulfonyl, phenyl, or U-1;
R¹⁸ is C₁₋₆ alkyl;
R²¹ is a hydrogen atom or C₁₋₆ alkyl;
R²² is a halogen atom, -OH, or C₁₋₆ alkoxy;
R²³ is C₁₋₆ alkyl, C₁₋₆ alkoxy, or phenyl;
R²⁴ is C₁₋₆ alkyl, (C₁₋₆) alkoxy (C₁₋₂) alkyl, or -C(O)R²⁵;
R²⁵ is C₁₋₆ alkoxy;
R²⁶ is C₁₋₆ alkoxy, -OR³², or phenyl;
R²⁷ is a halogen atom, C₂₋₆ alkenyl, or C₁₋₆ alkoxy;
R²⁸ and R²⁹ are each independently C₁₋₆ alkyl;
R³⁰ and R³¹ are each independently a hydrogen atom or benzyl;
R³² is phenyl;
R³³ is C₁₋₆ haloalkyl or di(C₁₋₆) alkylamino;
R³⁴ is C₁₋₆ alkoxy, phenyl, or -CN;
R³⁵ is a hydrogen atom, C₁₋₆ alkyl, C₃₋₆ cycloalkyl, (C₁₋₆) alkyl substituted with R¹⁴, or - S(O)₂R³³;
R³⁶ is C₁₋₆ alkyl or (C₁₋₆) alkyl substituted with R³⁷;
R³⁷ is a halogen atom or (C₁₋₁₀) alkoxycarbonyl;
R³⁸ is C₁₋₆ alkyl, C₃₋₆ alkenyl, C₃₋₆ alkynyl, C₃₋₆ cycloalkyl, C₃₋₆ haloalkenyl, C₃₋₆ haloalkynyl, or (C₁₋₆) alkyl substituted with R³⁹;
R³⁹ is a halogen atom, -OR⁵¹, -S(O)ᵣ₆R⁵², -C(=W²)R¹³, or -CN;
R⁴⁰ is a hydrogen atom, C₁₋₆ alkyl, C₃₋₆ alkenyl, C₁₋₆ haloalkyl, or (C₁₋₆) alkyl substituted with R⁴⁶;
R⁴¹ is C₁₋₆ alkyl, C₃₋₆ alkenyl, C₃₋₆ alkynyl, C₁₋₆ haloalkyl, or (C₁₋₆) alkyl substituted with R⁴⁷;
R⁴² and R⁴³ are each independently C₁₋₆ alkyl;
R⁴⁵ is a halogen atom;
R⁴⁶ is C₁₋₆ alkoxy or C₁₋₆ alkylthio;
R⁴⁷ is C₁₋₆ alkoxy or C₁₋₆ alkylthio;
R⁴⁸ is C₁₋₆ alkyl or C₁₋₆ haloalkyl;
R⁴⁹ is C₁₋₆ alkyl;
R⁵¹ is C₁₋₆ alkyl;
R⁵² is C₁₋₆ alkyl;
R⁵³ is phenyl;
R⁵⁴ is C₁₋₆ alkyl;
R⁵⁶ and R⁵⁷ are each independently C₁₋₆ alkyl;
R⁵⁸ is (C₁₋₆) alkyl substituted with R²⁷ or phenyl;
R⁵⁹ is C₁₋₆ alkyl or phenyl;
Y² is a halogen atom or C₁₋₆ haloalkyl;
Z² is a halogen atom, C₁₋₆ alkyl, or C₁₋₆ alkoxy, and when p5a is an integer of 2 or more, each Z² is the same as or different from each other;
Z⁴ is a halogen atom or C₁₋₆ alkoxy, and when p5c is an integer of 2 or more, each Z⁴ is the same as or different from each other; and
t is 0.

4. The pyridazinone compound and a salt thereof according to claim 3, wherein:
Z¹ is a halogen atom, C₁₋₆ alkyl, C₂₋₆ alkenyl, C₂₋₆ alkynyl, C₃₋₆ cycloalkyl, (C₁₋₆) alkyl substituted with R²², (C₂₋₆) alkynyl substituted with R¹⁹, -NR¹⁵R¹⁶, -OR³⁵, -S(O)ᵣ₁R³⁶, or -CN, and when n is an integer of 2 or more, each Z¹ is the same as or different from each other;
R² is a hydrogen atom, a halogen atom, C₁₋₆ alkyl, or C₁₋₆ alkoxy;
R³ is D-1, D-2, D-3, D-4, D-6, D-7, D-8, D-9, D-10, D-17, D-18, D-20, D-21, D-22, D-23, D-24, or D-28;
Y¹ is a halogen atom, C₁₋₆ alkyl, C₂₋₆ alkenyl, (C₁₋₆) alkyl substituted with R¹⁰, -OR¹¹, - S(O)ᵣ₂R³⁸, -NR⁸R⁹, -CN, -NO₂, or -C(=W²)R¹³, and when p7, p6, p5, p4, p3, or p2 is an integer of 2 or more, each Y¹ is the same as or different from each other;
R¹¹ is a hydrogen atom, C₁₋₆ alkyl, (C₁₋₆) alkyl substituted with R¹², C₃₋₆ cycloalkyl, phenyl, C₁₋₆ alkylsulfonyl, C₁₋₆ haloalkylsulfonyl, U-2, or U-4; and
R³⁷ is a halogen atom.

5. The pyridazinone compound or a salt thereof according to claim 3 or 4, wherein X is a sulfur atom.

6. The pyridazinone compound or a salt thereof according to claim 2, wherein:
X is an oxygen atom;
Z¹ is a halogen atom, C₁₋₆ alkyl, -OR³⁵, or -S(O)ᵣ₁R³⁶, and when n is an integer of 2 or more, each Z¹ is the same as or different from each other;
R¹ is C₁₋₆ alkyl;
R² is a hydrogen atom, a halogen atom, C₁₋₆ alkyl, or C₁₋₆ alkoxy;
R³ is D-1, D-3, D-7, D-20, D-21, D-22, or D-24;
Y¹ is a halogen atom, C₁₋₆ alkyl, (C₁₋₆) alkyl substituted with R¹⁰, -OR¹¹, or -S(O)ᵣ₂R³⁸, and when p7, p6, p5, p4, p3, or p2 is an integer of 2 or more, each Y¹ is the same as or different from each other;
R⁴ is -OR²⁴ or -S(O)ᵣ₄R⁵⁴;
R⁵ is C₁₋₆ alkyl, (C₁₋₆) alkyl substituted with R²⁶, or C₁₋₆ alkoxy;
R⁶ is C₁₋₆ alkyl or -NR²⁸R²⁹;
R¹⁰ is a halogen atom, -OR⁴⁰, or -S(O)ᵣ₃R⁴¹;
R¹¹ is a hydrogen atom, C₁₋₆ alkyl, or (C₁₋₆) alkyl substituted with R¹²;
R¹² is a halogen atom, C₂₋₆ alkenyl, -OR⁴⁸, -S(O)ᵣ₅R⁴⁹, phenyl, or -C(=W²)R¹³;
R¹³ is a hydrogen atom or C₁₋₆ alkyl;
R¹⁴ is a halogen atom, C₂₋₆ alkenyl, C₁₋₆ alkoxy, or C₁₋₆ alkylthio;
R²⁴ is C₁₋₆ alkyl;
R²⁶ is C₁₋₆ alkoxy;
R²⁸ and R²⁹ are each independently C₁₋₆ alkyl;
R³⁴ is C₁₋₆ alkoxy or -CN;
R³⁵ is C₁₋₆ alkyl or (C₁₋₆) alkyl substituted with R¹⁴;
R³⁶ is C₁₋₆ alkyl or (C₁₋₆) alkyl substituted with R³⁷;
R³⁷ is a halogen atom, C₁₋₆ alkoxy, or C₁₋₆ alkylthio;
R³⁸ is C₁₋₆ alkyl, C₃₋₆ alkenyl, C₃₋₆ alkynyl, or (C₁₋₆) alkyl substituted with R³⁹;
R³⁹ is -OR⁵¹ or -S(O)ᵣ₆R⁵²;
R⁴⁰ is C₁₋₆ alkyl, C₃₋₆ alkenyl, or C₃₋₆ alkynyl;
R⁴¹ is C₁₋₆ alkyl, C₃₋₆ alkenyl, C₃₋₆ alkynyl, or (C₁₋₆) alkyl substituted with R⁴⁷;
R⁴⁸ is C₁₋₆ alkyl;
r1 is an integer of 0, 1, or 2;
r4 is an integer of 0, 1, or 2;
r5 is an integer of 0, 1, or 2;
r6 is an integer of 0, 1, or 2;
n is an integer of 0, 1, 2, 3, or 4;
p3 is an integer of 0, 1, 2, or 3;
p4 is an integer of 0, 1, 2, 3, or 4; and
p5 is an integer of 0, 1, 2, 3, 4, or 5.

7. The pyridazinone compound or a salt thereof according to claim 6, wherein:
Z¹ is a halogen atom, C₁₋₆ alkyl, or -OR³⁵;
R¹ is C₁₋₆ alkyl;
R² is C₁₋₆ alkyl or C₁₋₆ alkoxy;
R³ is D-1, D-7, or D-24;
Y¹ is a halogen atom, C₁₋₆ alkyl, (C₁₋₆) alkyl substituted with R¹⁰, -OR¹¹, or -S(O)ᵣ₂R³⁸;
G is a hydrogen atom, C₁₋₆ alkyl, or -C(=W⁴)R⁵;
R⁵ is C₁₋₆ alkyl;
R¹⁰ is a halogen atom or -OR⁴⁰;
R¹² is a halogen atom, C₂₋₆ alkenyl, -OR⁴⁸, -S(O)ᵣ₅R⁴⁹, or phenyl;
R³⁵ is C₁₋₆ alkyl;
R³⁸ is C₁₋₆ alkyl;
R⁴⁰ is C₁₋₆ alkyl;
R⁴⁸ is C₁₋₆ alkyl; and
R⁴⁹ is C₁₋₆ alkyl.

8. An agricultural chemical comprising, as an active ingredient, one or more selected from the pyridazinone compound and a salt thereof according to any one of claims 1 to 7.

9. A herbicide comprising, as an active ingredient, one or more selected from the pyridazinone compound and a salt thereof according to any one of claims 1 to 7.
